# EUROPEAN PATENT APPLICATION

(11) **EP 4 840 467 A1**
(43) Date of publication of application: **18.11.2026**
(21) Application number: 25741660.2
(22) Date of filing: 20.01.2025
(51) Int. Cl.: A61K 47/18, A61K 35/17, A61K 35/28, A61K 31/7088, A61P 43/00, C12N 15/87, A61K 47/20, A61K 47/22, A61K 47/24, A61K 47/26, A61K 47/28, A61K 47/10, A61K 9/51

(54) **USE OF PREFABRICATED CARRIER IN PREPARATION OF PRODUCT FOR IN VITRO DELIVERY OF GENE INTO IMMUNE CELLS AND STEM CELLS**

(30) Priority: 19.01.2024 CN 202410079977
(71) Applicant: Scindy Pharmaceutical Co., Ltd, Suzhou, Jiangsu 215124 (CN)
(72) Inventor: CHEN, Xiaobao, Suzhou, Jiangsu 215124 (CN)
(74) Representative: Elzaburu S.L.P.
(86) International application number: PCT/CN2025/073417
(87) International publication number: WO 2025/153099

(57) **Abstract**

The present application provides use of a prefabricated carrier in preparation of a product for in vitro gene delivery into immune cells and stem cells, belonging to the technical field of biomedicine. The use of the prefabricated carrier in the preparation of the product for in vitro gene delivery into the immune cells and the stem cells comprises a step of mixing the prefabricated carrier with a nucleic acid in a solvent to obtain a prefabricated carrier-based composition. The prefabricated carrier includes the following components: an ionizable lipid, a phospholipid, a steroid or a derivative thereof, and a polyethylene glycol (PEG)-conjugated lipid. The prefabricated carrier can be used as a carrier tool for cell therapy and gene therapy.

## Description

### CROSS-REFERENCE TO RELATED APPLICATIONS

This application claims priority to Chinese Patent Application No. 202410079977.0, filed on January 19, 2024, the entire contents of which are incorporated herein by reference.

### TECHNICAL FIELD

The present application belongs to the technical field of biomedicine, and particularly relates to use of a prefabricated carrier in preparation of a product for in vitro gene delivery into immune cells and stem cells.

### BACKGROUND

Cell and gene therapies boast extremely broad application prospects and are expected to cure a variety of diseases at their sources. The cell therapy refers to a process of harvesting cells with specific functions, subjecting such cells to in vitro manipulation to endow them with capabilities including immune enhancement, pathogen elimination, tumor cell killing, etc., and infusing or implanting the resulting cells into a human body for disease treatment. It encompasses immune cell and stem cell therapies. In contrast, the gene therapy refers to a process of guiding exogenous normal cells into target cells to rectify diseases caused by defective and abnormal genes so that the target genes can be expressed in the cells for disease treatment. The gene therapy is classified into in vivo and in vitro gene therapies.

In the field of biomedicine, researches and application of immune cells, stem cells, and nucleic acids have emerged as cutting-edge frontiers and hot research topics. The immune cells, such as T cells, B cells, and NK cells, play crucial roles in a human immune system, which can recognize and attach foreign pathogens and cancer cells. The stem cells have abilities of self-renewal and multilineage differentiation. The nucleic acids, including DNA and RNA, are carriers of genetic information of organisms, and also function to regulate gene expression. Accordingly, they possess a huge potential in the fields of the gene therapy, research and development of vaccines, and the like.

To deliver biomacromolecules into cells for genome editing, particles containing these biomacromolecules should first overcome extracellular barriers. These barriers include phagocytosis by macrophages, enzymatic degradation or hydrolysis, as well as abilities to trigger immune responses and generate cytokines. If these biomacromolecules and nanoparticles can evade bodily clearance and reach appropriate organs or bloodstream, they still need to enter the target cells. Once entering the cells, the biomacromolecules and the nanoparticles need to escape from endosomes and localize to cytoplasmic mRNA or nuclei to successfully edit the target genes.

At present, the cell and gene therapies mainly rely on chemical-mediated delivery, physical-mediated delivery, and viral-mediated delivery. The chemical-mediated delivery utilizes carrier molecules to coat a nucleic acid, rendering the nucleic acid neutral or positively charged, such as a DEAE-dextran method and a calcium phosphate method, so as to deliver the nucleic acid into the cells for gene transfection and expression. The physical-mediated delivery utilizes physical means such as a micro-injection method, an electroporation method, and a gene gun method to generate an instant pore on a surface of a cytomembrane, so that the nucleic acids enters the cells. The viral-mediated delivery uses genetically engineered viruses such as retroviruses and adenoviruses to transfect non-virus genes into the cells, and such viruses can integrate the nucleic acids into a cellular genome to achieve long-term gene expression. However, the aforementioned methods have respective limitations, such as low delivery efficiency, severe cell damages, high cytotoxicity, large technical difficulty, high operation complexity, and poor reproducibility. Therefore, it is urgent to develop a novel delivery technology to meet the demands of the current cell and gene therapies.

To this end, the present application provides use of a prefabricated carrier in preparation of a product for in vitro gene delivery into immune cells and stem cells for the purpose of achieving safe and effective delivery of a gene editing drug by simply mixing the gene editing drug with the prefabricated carrier in a solution state.

### SUMMARY

Aiming at the problems existing in the prior art, the present application provides use of a prefabricated carrier in preparation of a product for in vitro gene delivery into immune cells and stem cells. The use of the prefabricated carrier in the preparation of the product for in vitro gene delivery into the immune cells and the stem cells according to the present application includes a step of mixing the prefabricated carrier with a nucleic acid in a solvent to obtain a prefabricated carrier-based composition, where the prefabricated carrier includes the following components: an ionizable lipid, a phospholipid, a steroid, and a polyethylene glycol (PEG)-conjugated lipid. By employing the aforementioned prefabricated carrier, the present application enables flexible regulation of a nucleic acid dosage based on a user's demand; and administration through various routes can all yield desirable delivery outcomes.

To achieve the above objective, in a first aspect, the present application provides use of a prefabricated carrier in preparation of a product for in vitro gene delivery into immune cells and stem cells, including a step of mixing the prefabricated carrier with a nucleic acid in a solvent to obtain a prefabricated carrier-based composition, where the prefabricated carrier includes the following components: 10-60 mol% of a first ionizable lipid, 0-30 mol% of a second ionizable lipid, 5-30 mol% of a phospholipid, 15-70 mol% of a steroid or a derivative thereof, and 0-10 mol% of a PEG-conjugated lipid.

In a preferred embodiment, the product is a delivery system or drug.

In a preferred embodiment, the use further includes a step of contacting the prefabricated carrier-based composition with the immune cells or the stem cells.

In a preferred embodiment, an adjuvant is also added to the product for in vitro gene delivery into the immune cells and the stem cells.

In a preferred embodiment, the adjuvant includes at least one of a lipid, a polypeptide, lipopolyamine, or a synthetic polymer.

In a preferred embodiment, the nucleic acid is DNA and/or RNA.

In a preferred embodiment, the nucleic acid includes at least one of siRNA, miRNA, saRNA, sgRNA, dsRNA, shRNA, smRNA, ssRNA, mRNA, circRNA, snRNA, crRNA, IncRNA, snoRNA, piRNA, pDNA, ssDNA, circular or linear DNA, a DNA minicircle, and msDNA.

In a preferred embodiment, an amount of the nucleic acid is 0.5-50% (w/w) of a total amount of the prefabricated carrier and the nucleic acid; and in the prefabricated carrier-based composition, a concentration of the nucleic acid is 5-1000 ng/µl.

In a preferred embodiment, the first ionizable lipid is selected from a compound of General formula (1), or a pharmaceutically acceptable salt, stereoisomer or tautomer thereof; 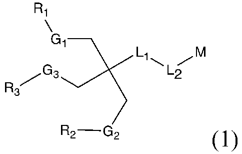
where R₁, R₂, and R₃ are each independently H, C₅₋₄₀ straight-chain or branched alkyl, C₅₋₄₀ straight-chain or branched alkenyl, C₅₋₄₀ straight-chain or branched alkynyl, 3-6 membered saturated or partially unsaturated cyclic hydrocarbyl containing 1-3 side chains, or a 6-10 membered aromatic group containing 1-3 side chains; the side chain is independently selected from C₁₀₋₃₀ straight-chain or branched alkyl, C₁₀₋₃₀ straight-chain or branched alkenyl, and C₁₀₋₃₀ straight-chain or branched alkynyl; provided that at most one of R₁, R₂, and R₃ is H;
M is selected from -NR₄R₅, saturated or partially unsaturated 3-6 membered heterocyclyl containing at least one nitrogen atom, and 6-10 membered heteroaryl containing at least one nitrogen atom, and the heterocyclyl and the heteroaryl are unsubstituted or substituted with one or more -OH, carboxyl, amino, oxo or halogen;
R₄ and R₅ are each independently H, C₁₋₆ straight-chain or branched alkyl, C₂₋₆ straight-chain or branched alkenyl, or C₂₋₆ straight-chain or branched alkynyl, and the C₁₋₆ straight-chain or branched alkyl, the C₂₋₆ straight-chain or branched alkenyl, or the C₂₋₆ straight-chain or branched alkynyl is unsubstituted or substituted with one or more -OH, the carboxyl, the amido, amidino, guanidino or the halogen;
G₁, G₂, and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)-, or -OP(=O)(OR₆)O-; where R₆ and R₁₃ are each independently selected from H, the hydroxyl, C₁₋₃₀ straight-chain or branched alkyl or cycloalkyl, and C₂₋₃₀ straight-chain or branched alkenyl;
L₁ is selected from -X₁- or -(CR₇R₈)m-X₁-, where each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, - SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)-, or -OP(=O)(OR₁₄)O-, where m is an integer of 2-6, R₇ and R₈ are each independently H, the hydroxyl, the halogen, C₁₋₆ straight-chain or branched alkyl or cycloalkyl, or the C₂₋₆ straight-chain or branched alkenyl, and R₁₄ and R₁₅ are each independently selected from H, the C₁₋₃₀ straight-chain or branched alkyl or cycloalkyl, and the C₂₋₃₀ straight-chain or branched alkenyl;
L₂ is -(CR₉R₁₀)ₙ₋ or-(CR₉R₁₀)ₙ-X₂-(CR₁₁R₁₂)ₖ-, wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, - SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, - P(=O)(OR₁₆)O-, - OP(=O)(OR₁₆)-, or -OP(=O)(OR₁₆)O-; n is an integer of 1-6; k is an integer of 1-6; R₉, R₁₀, R₁₁, and R₁₂ are each independently H, the hydroxyl, the halogen, the C₁₋₆ straight-chain or branched alkyl or cycloalkyl, or the C₂₋₆ straight-chain or branched alkenyl, and R₁₆ and R₁₇ are each independently selected from H, the C₁₋₃₀ straight-chain or branched alkyl or cycloalkyl, and the C₂₋₃₀ straight-chain or branched alkenyl;
where the alkyl, the cycloalkyl, and the alkenyl in R₄-R₁₇ are unsubstituted or substituted with one or more groups selected from the hydroxyl, sulfydryl, the amino, the substituted amino, and the halogen; and
the salt does not include quaternary ammonium salts.

In a preferred embodiment, said R₁, R₂, and R₃ are each independently the following groups: 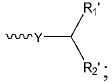 where Y is absent, or the C₁₋₃₀ straight-chain or branched alkyl or cycloalkyl, C₂₋₂₀ straight-chain or branched alkenyl, or C₂₋₂₀ straight-chain or branched alkynyl; R₁' and R₂' are each independently H, the C₁₋₃₀ straight-chain or branched alkyl, the C₂₋₃₀ straight-chain or branched alkenyl, or C₂₋₃₀ straight-chain or branched alkynyl, and a total carbon chain length of Y, R₁' and R₂' is 8-40.

In a preferred embodiment, said R₁, R₂, and R₃ are each independently the following groups: 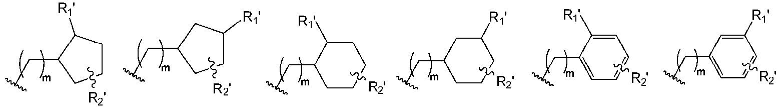 where R₁' and R₂' are each independently H, the C₁₋₃₀ straight-chain or branched alkyl, C₂₋₃₀ straight-chain or branched alkenyl, or the C₂₋₃₀ straight-chain or branched alkynyl, and a total carbon chain length of R₁' and R₂' is 8-30.

In a preferred embodiment, said R₁, R₂, and R₃ are each independently selected from any one of the following groups:

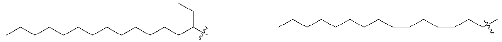

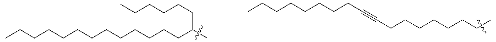

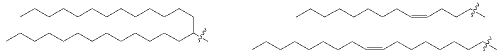

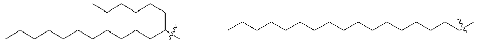

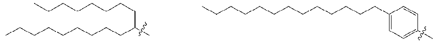

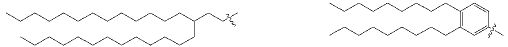

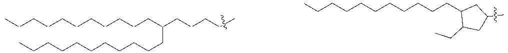

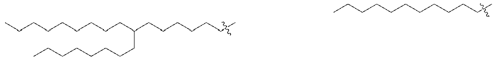

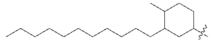

In a preferred embodiment, G₁, G₂, and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

In a preferred embodiment, L₁ is selected from -(CR₇R₈)ₘ-X₁-, where X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

In a preferred embodiment, L₂ is -(CR₉R₁₀)ₙ-X₂-(CR₁₁R₁₂)ₖ-, where X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

In a preferred embodiment, M is selected from the following structures: 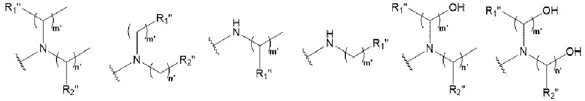 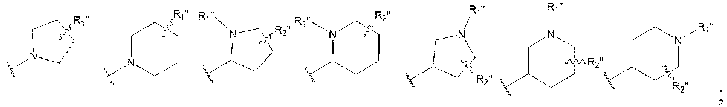 where m' and n' are each independently integers of 0-6; and R₁" and R₂" are each independently H, C₁₋₆ alkyl, C₂₋₆ alkenyl, the guanidino, the amidino, the amido, aliphatic amino, or 3-10 membered nitrogen-containing heterocyclyl; the nitrogen-containing heterocyclyl is selected from pyrrolyl, imidazolyl, pyridyl, pyrazolyl, triazolyl, oxazolyl, isoxazolyl, thienyl, isothiazolyl, pyridazinyl, pyrazinyl, piperazinyl, indolyl, benzimidazolyl, carbazolyl, quinolyl, isoquinolyl, purinyl, pyrimidinyl, and tautomeric forms thereof, and are unsubstituted or optionally substituted with one or more organic groups selected from the hydroxyl, the sulfydryl, the amino, the substituted amino, the halogen, the C₁₋₆ alkyl, the C₂₋₆ alkenyl, C₂₋₆ alkynyl, and C₆₋₁₄ aryl.

In a preferred embodiment, the compound of Formula (1) is selected from compounds of Formula (1A):

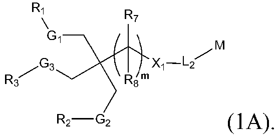

In a preferred embodiment, the compound of Formula (1) is selected from compounds of Formula (1B):

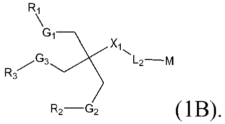

In a preferred embodiment, the compound of Formula (1) is selected from compounds of Formula (1C):

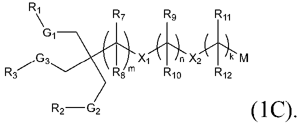

In a preferred embodiment, the compound of Formula (1) is selected from compounds of Formula (1D):

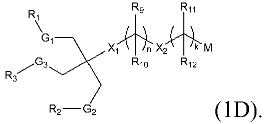

In a preferred embodiment, the compound of Formula (1) is selected from compounds of Formula (1E):

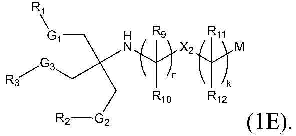

In a preferred embodiment, the compound of Formula (1) is selected from compounds of Formula (1F):

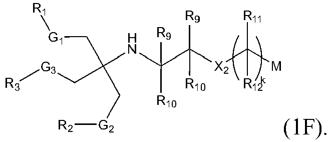

In a preferred embodiment, the compound of Formula (1) is selected from compounds of Formula (1G):

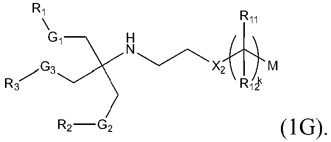

In a preferred embodiment, the compound of Formula (1) is selected from compounds of Formula (1H):

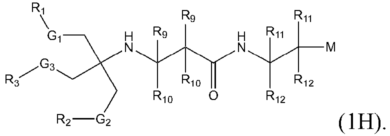

In a preferred embodiment, M is selected from any one of the following groups:

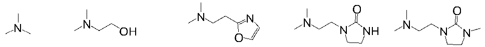

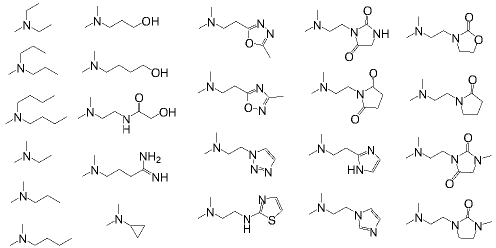

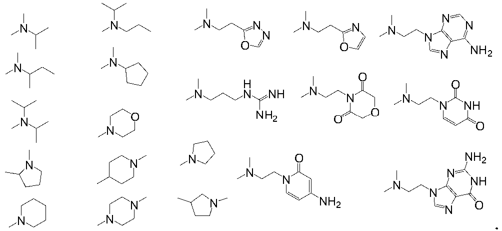

In a preferred embodiment, the compound of Formula (1) is selected from compounds of Formula (1I):

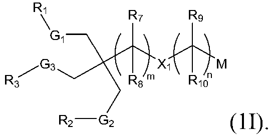

In a preferred embodiment, the compound of Formula (1) is selected from compounds of Formula (1J):

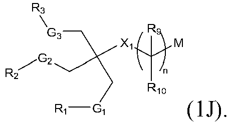

In a preferred embodiment, the compound of Formula (1) is selected from compounds of Formula (1K):

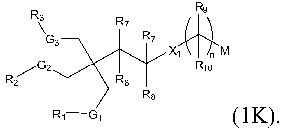

In a preferred embodiment, Y is absent, and the compound of Formula (1) is selected from compounds of Formula (1L): 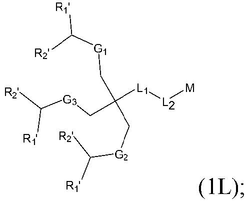 where R₁' and R₂' are independently selected from H, the C₁₋₃₀ straight-chain or branched alkyl, the C₂₋₃₀ straight-chain or branched alkenyl, and the C₂₋₃₀ straight-chain or branched alkynyl, and the total carbon chain length of R₁' and R₂' is 8-40.

In a preferred embodiment, the compound of Formula (1) is selected from compounds of Formula (1M):

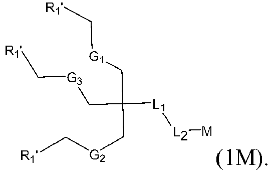

In a preferred embodiment, the compound of Formula (1) is selected from:

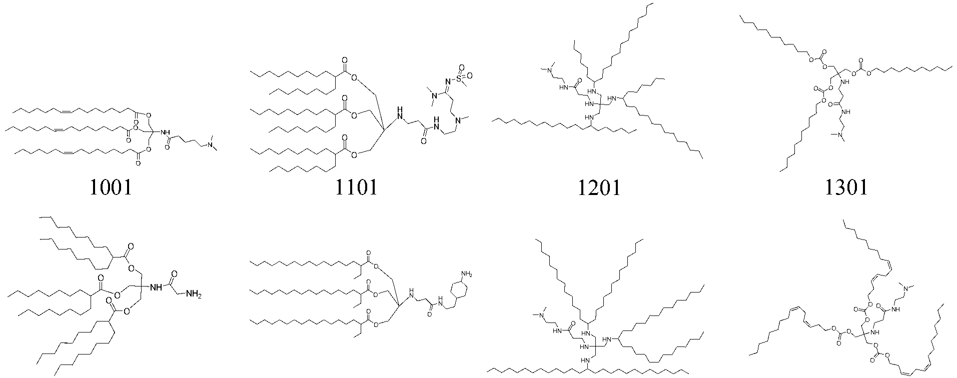

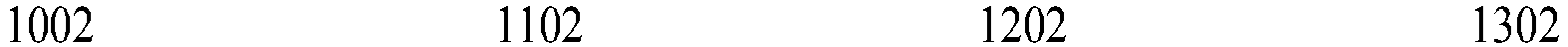

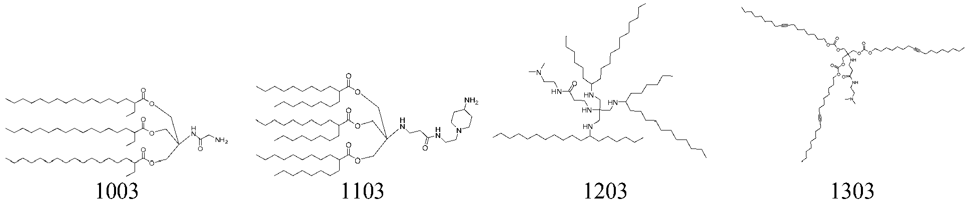

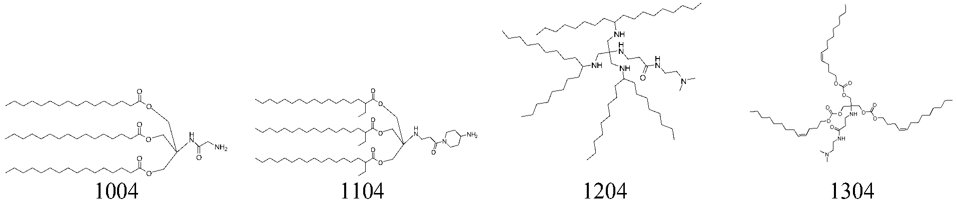

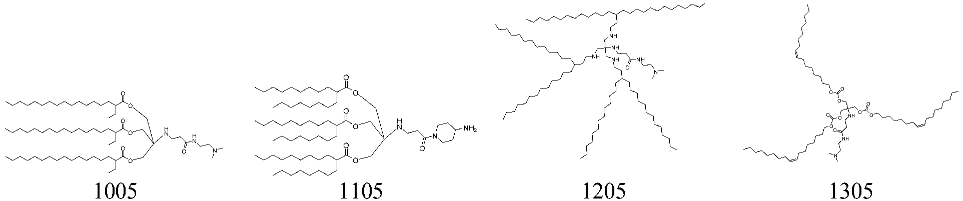

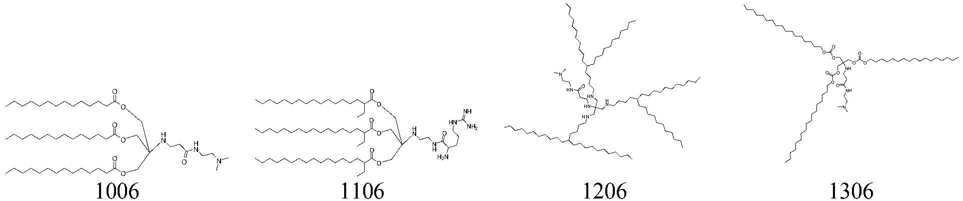

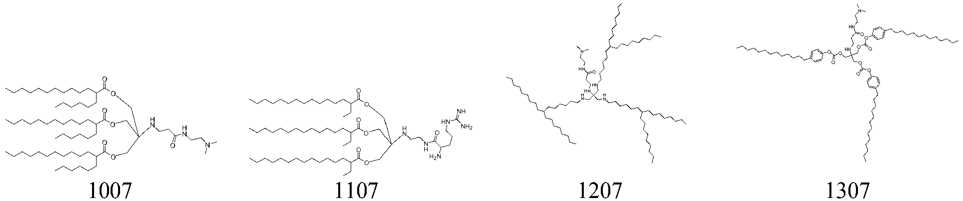

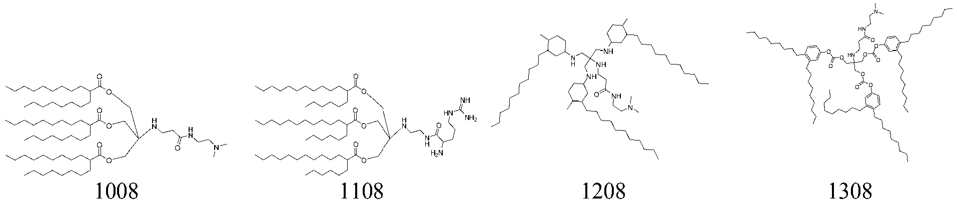

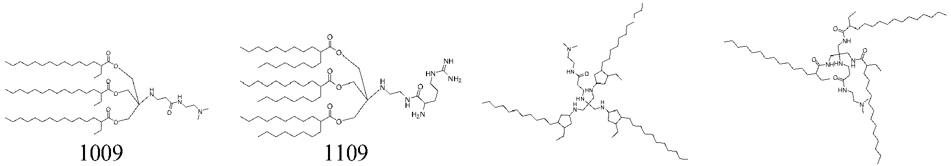

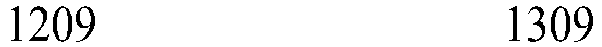

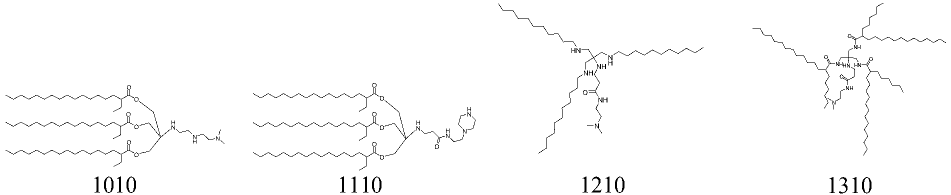

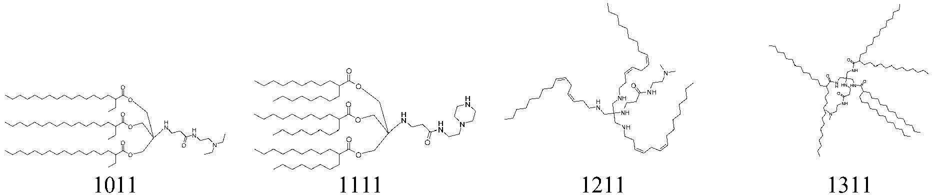

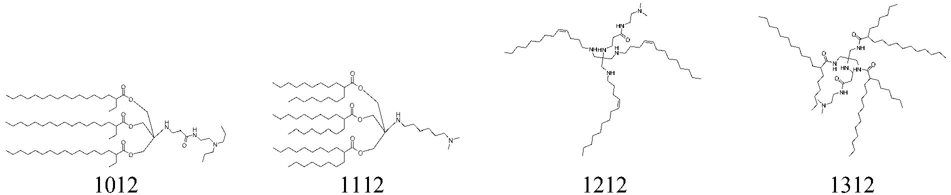

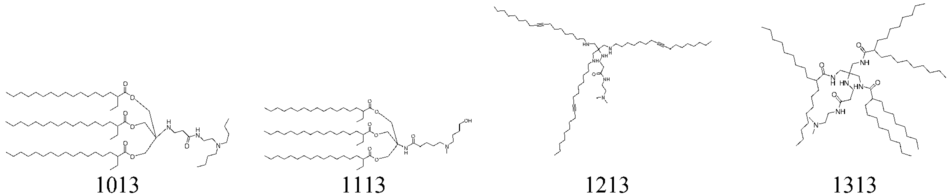

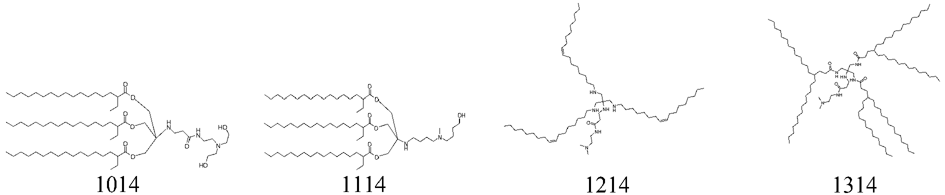

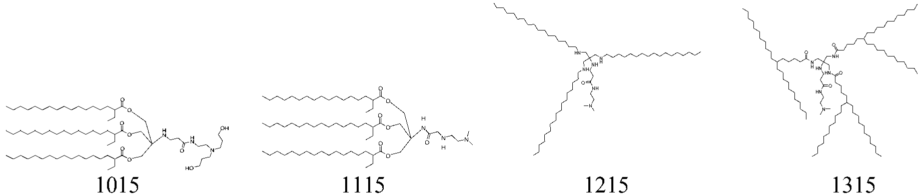

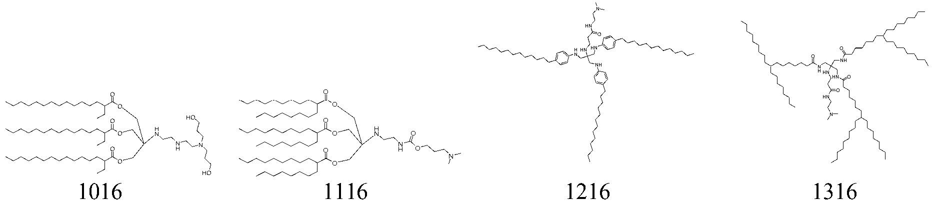

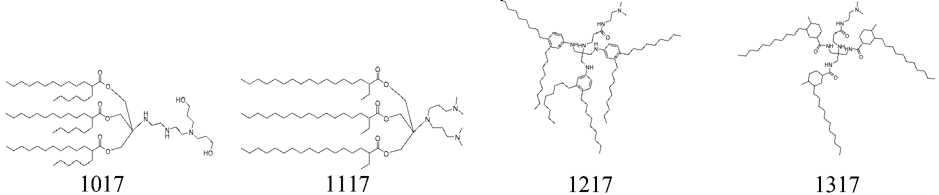

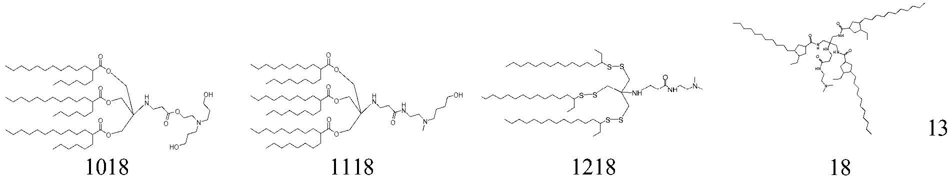

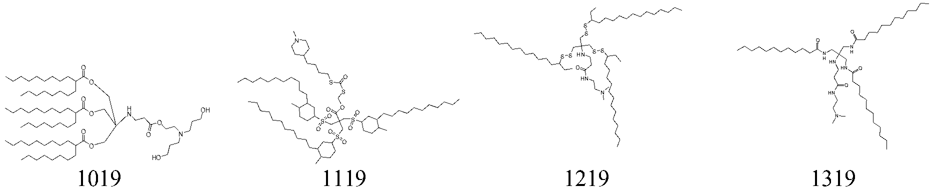

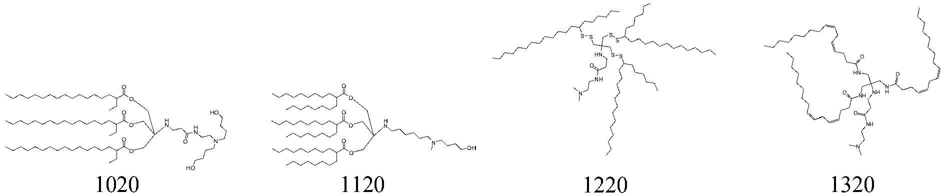

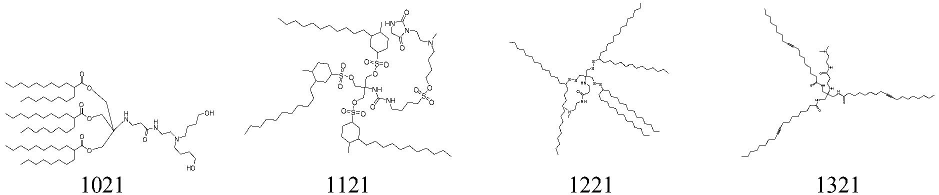

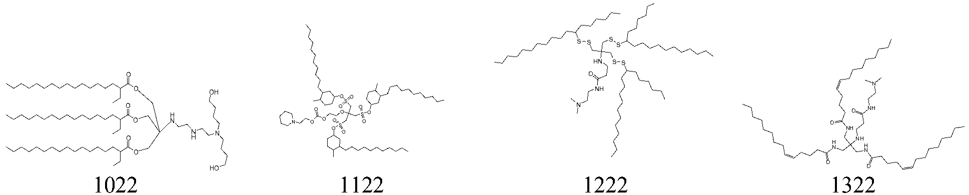

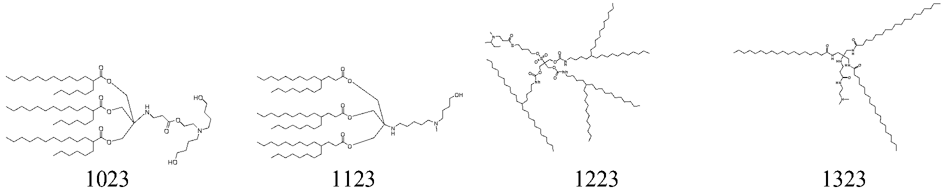

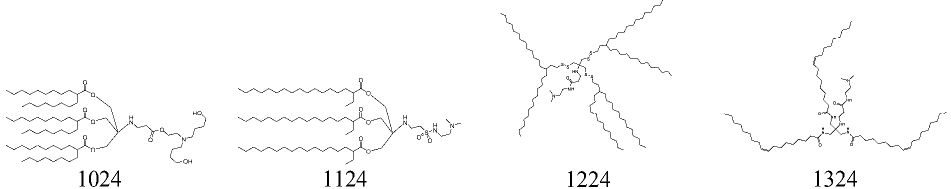

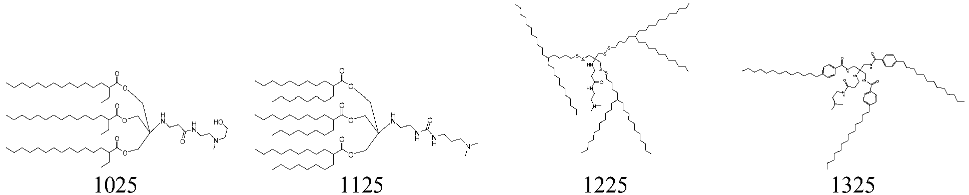

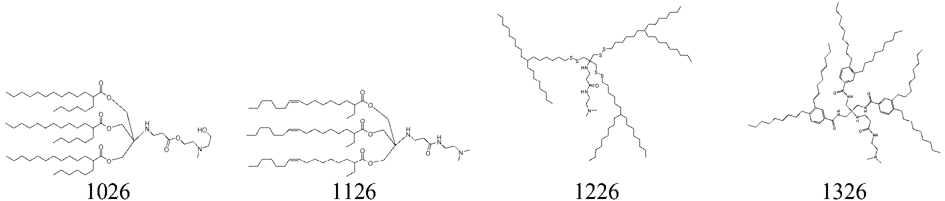

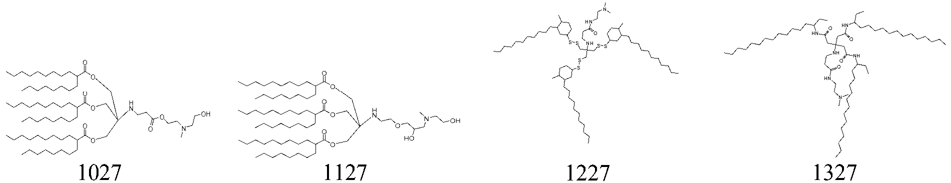

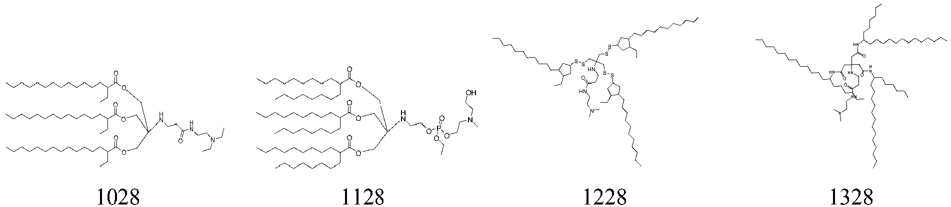

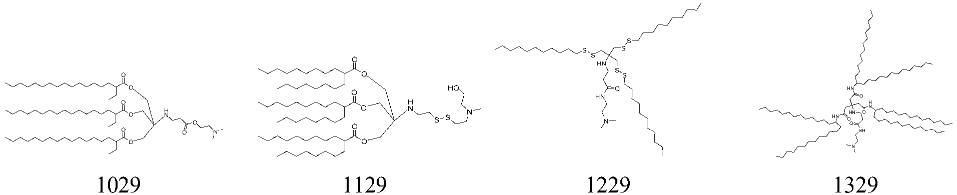

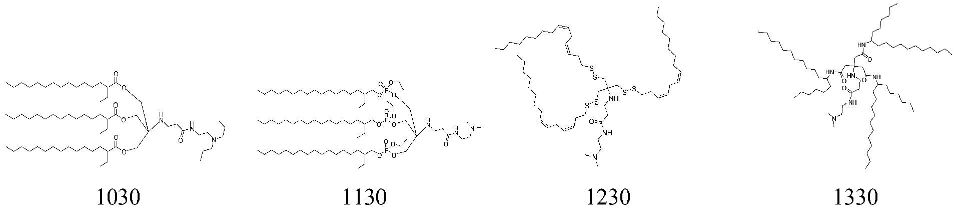

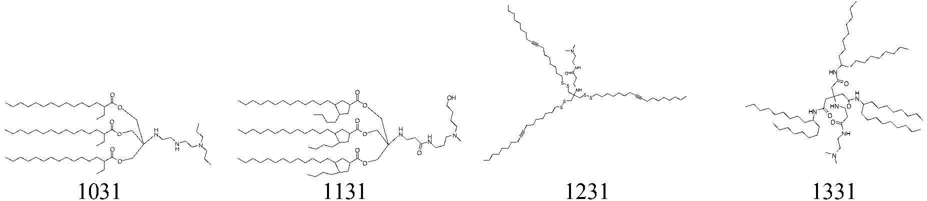

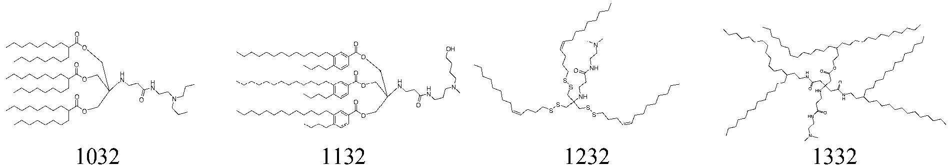

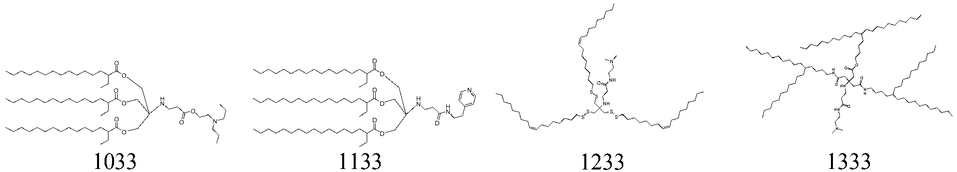

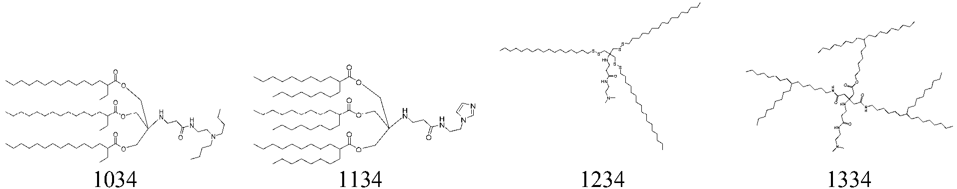

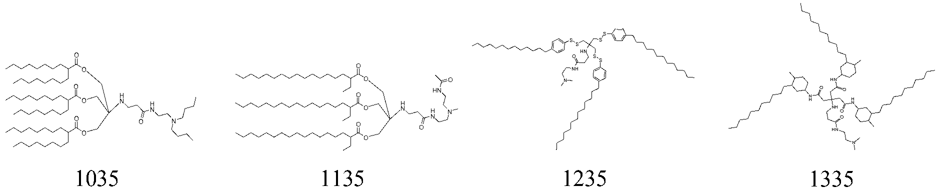

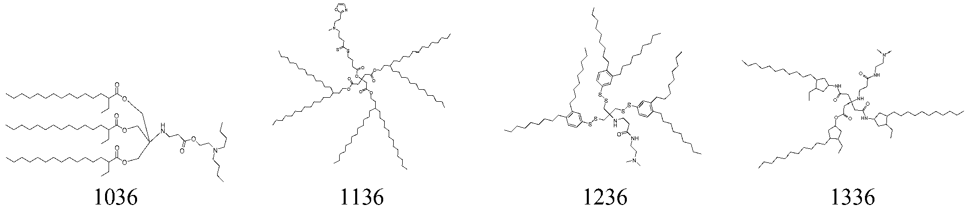

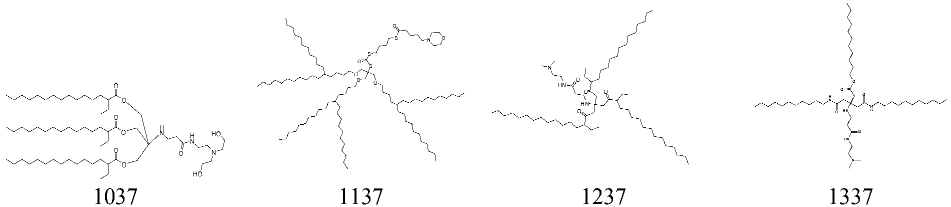

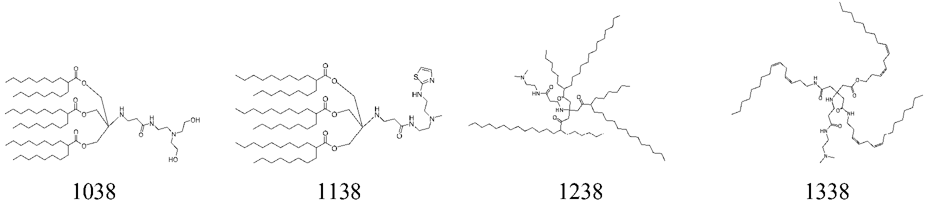

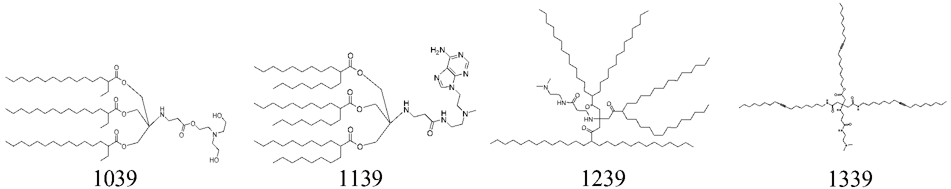

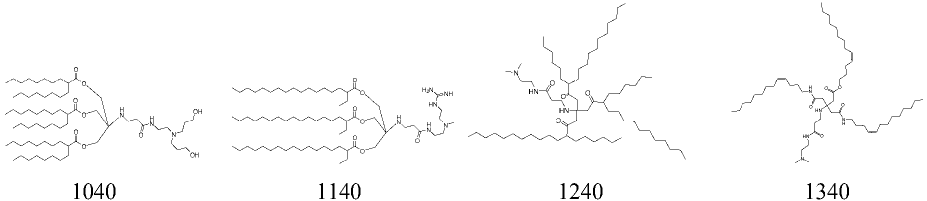

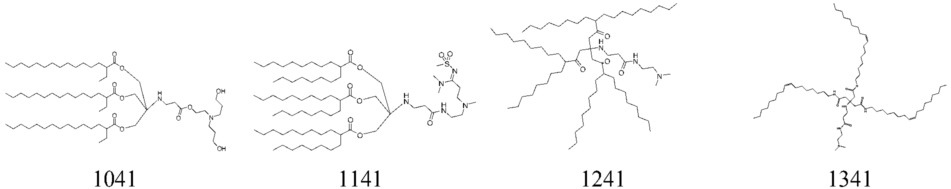

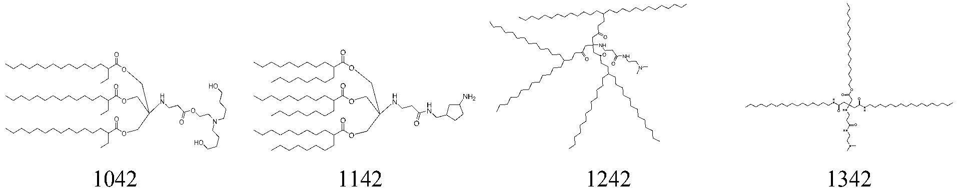

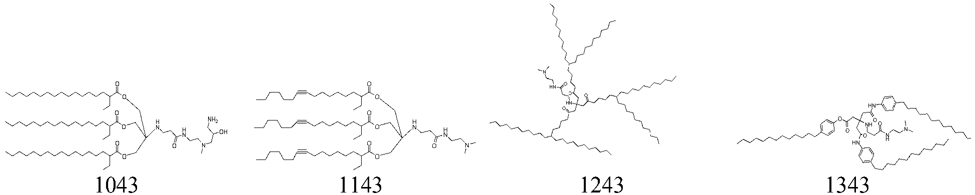

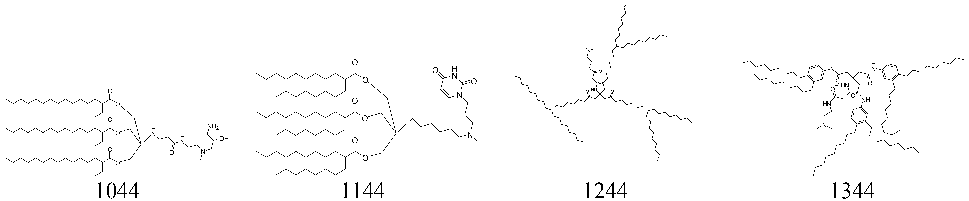

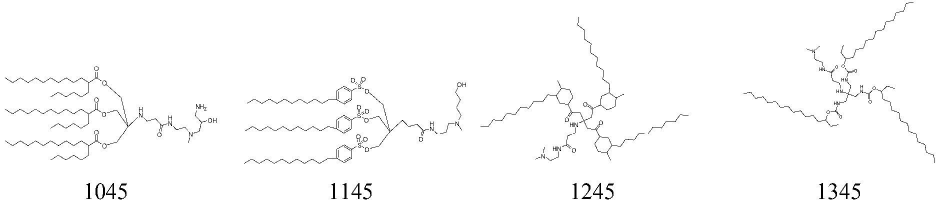

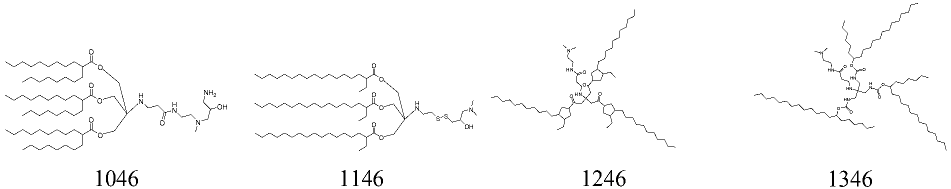

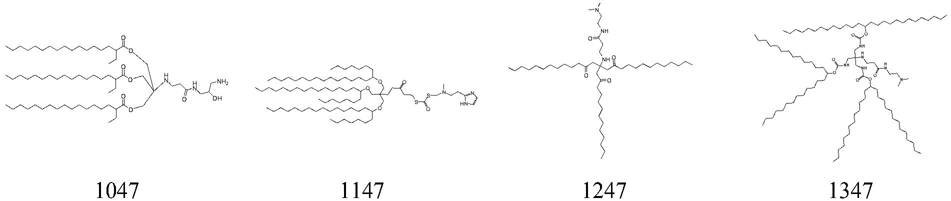

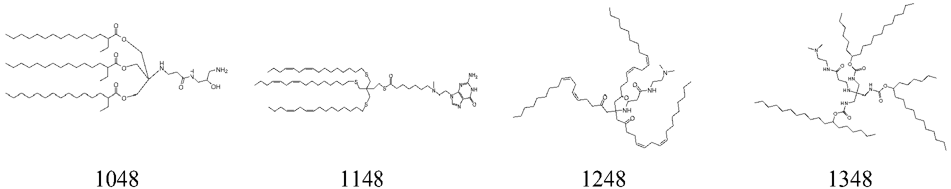

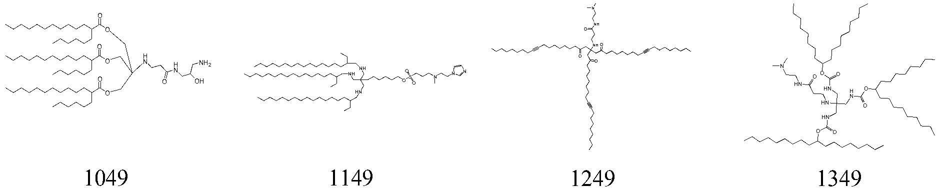

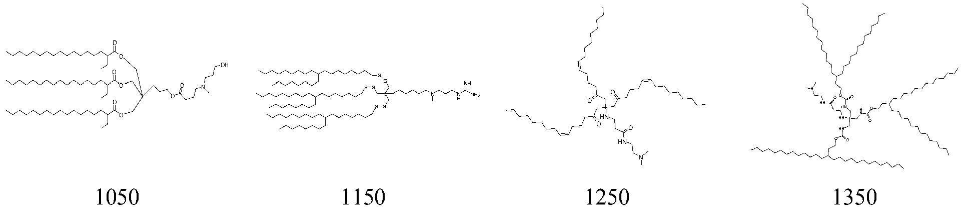

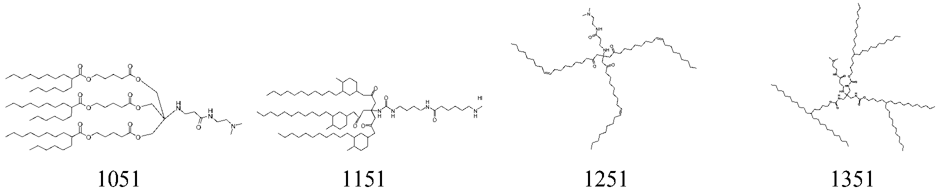

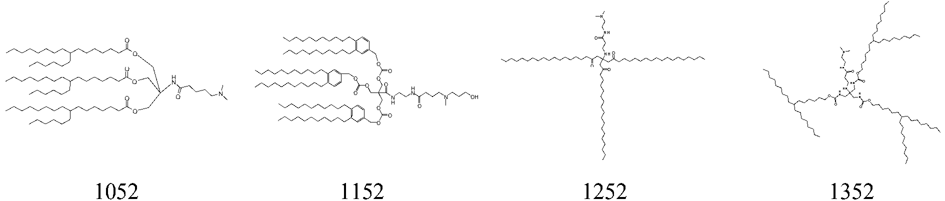

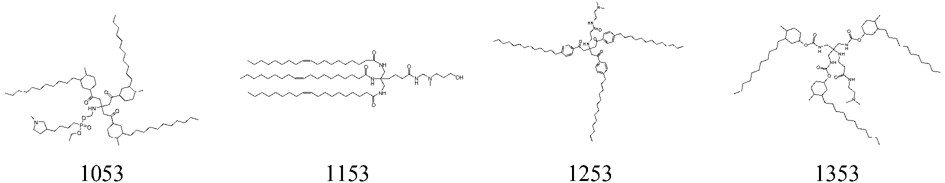

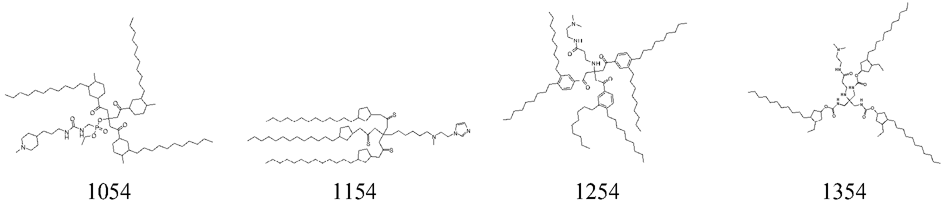

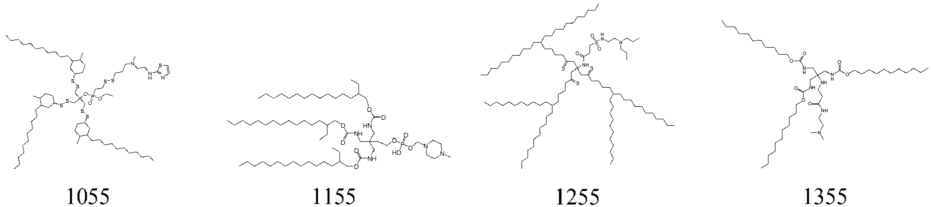

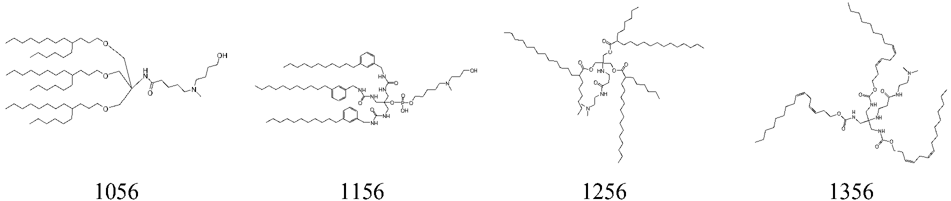

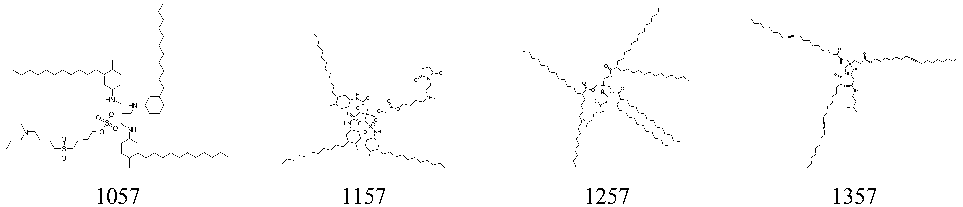

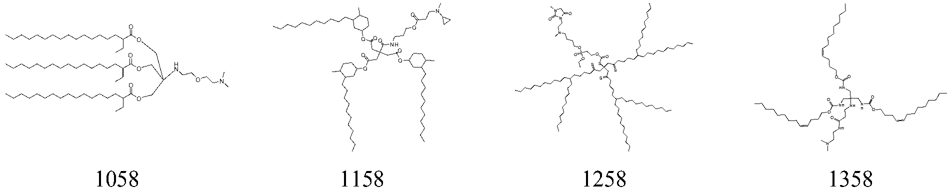

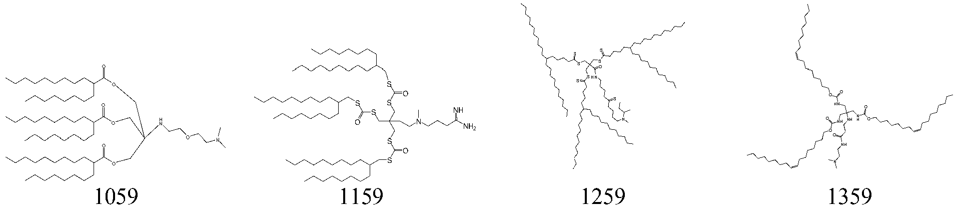

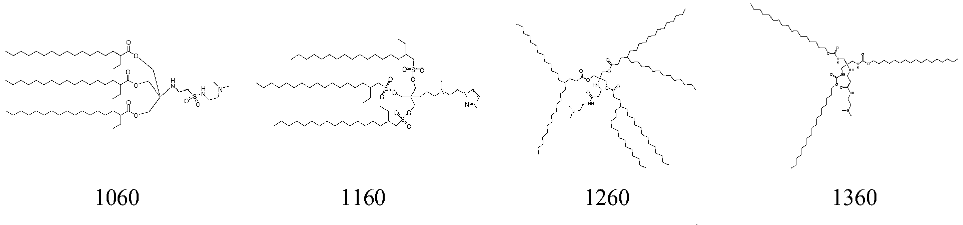

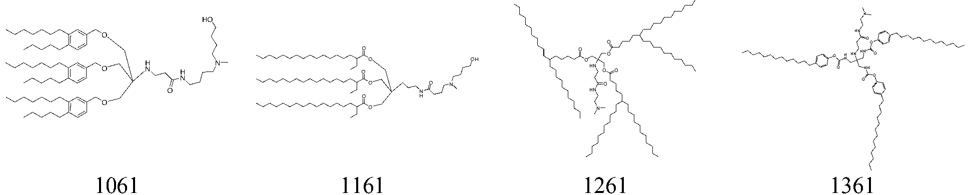

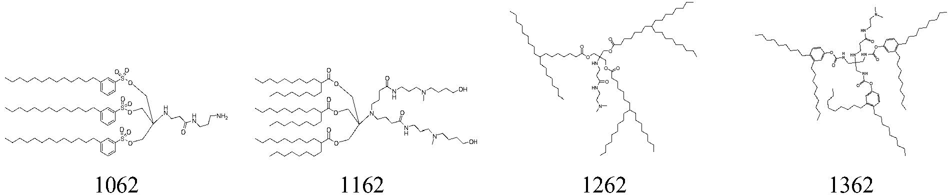

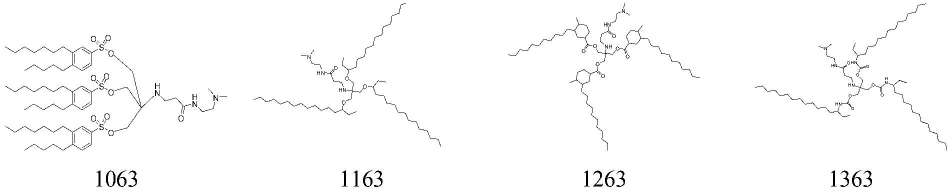

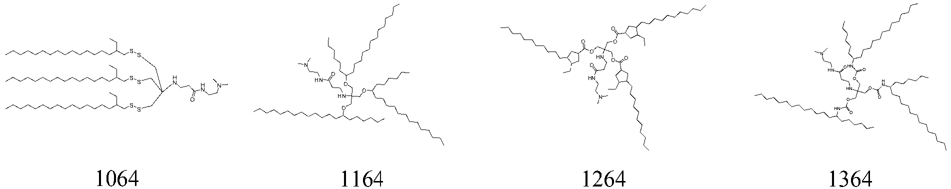

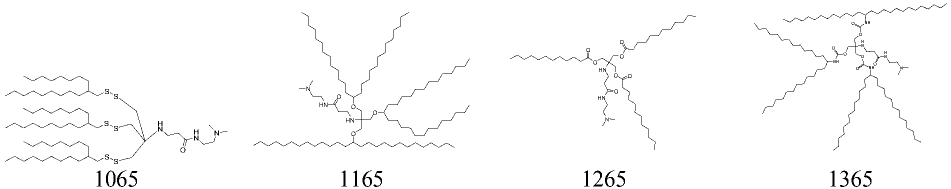

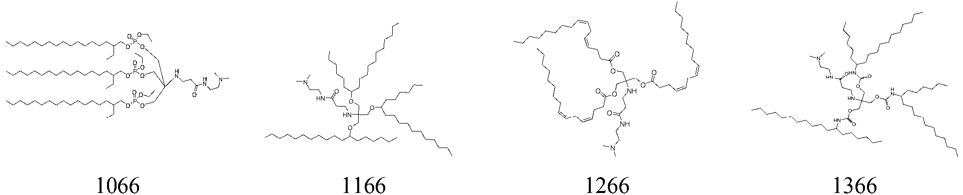

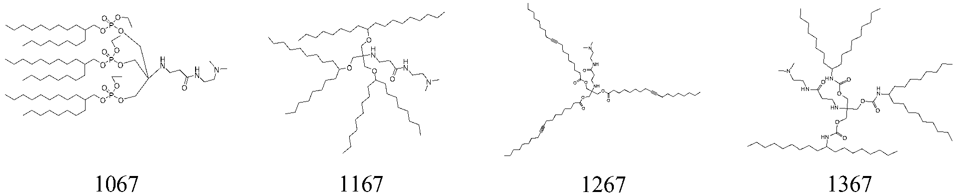

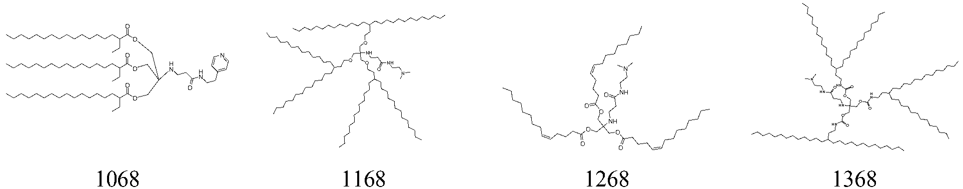

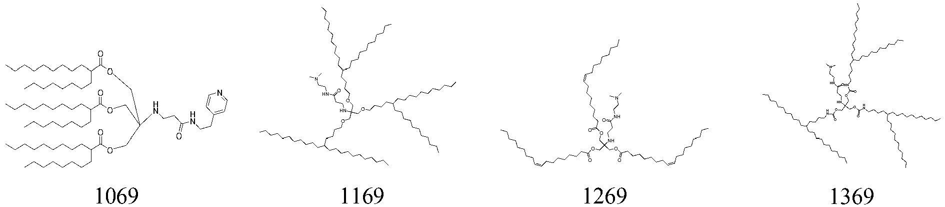

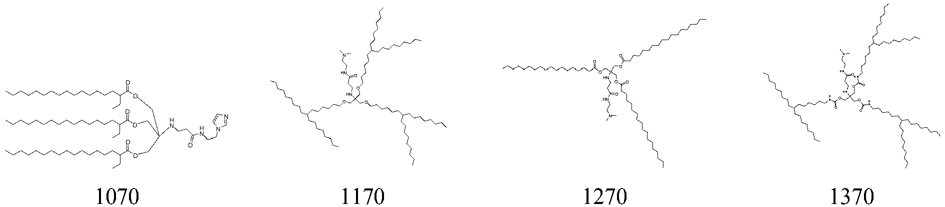

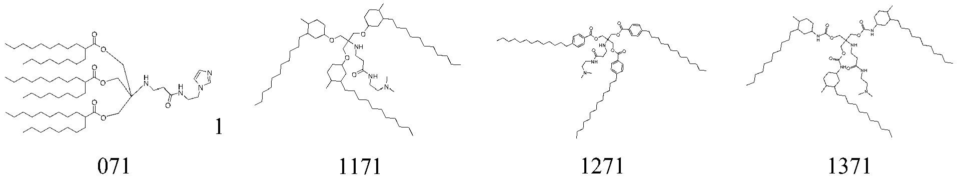

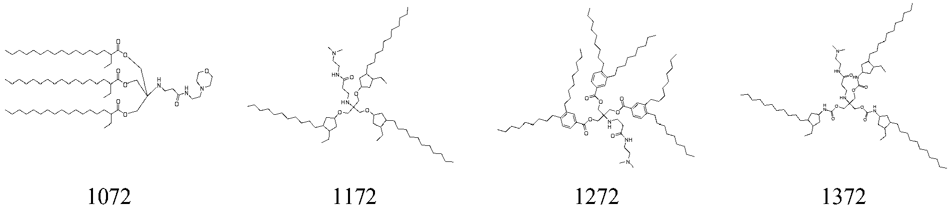

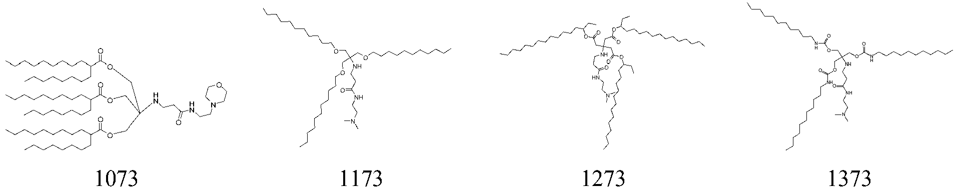

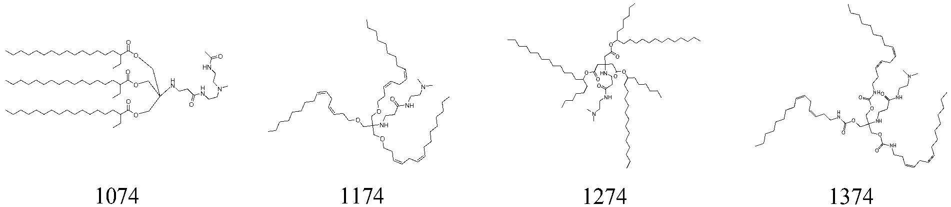

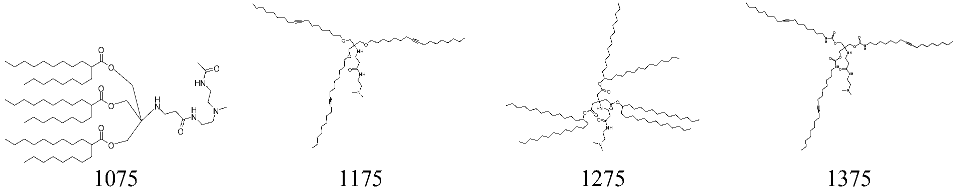

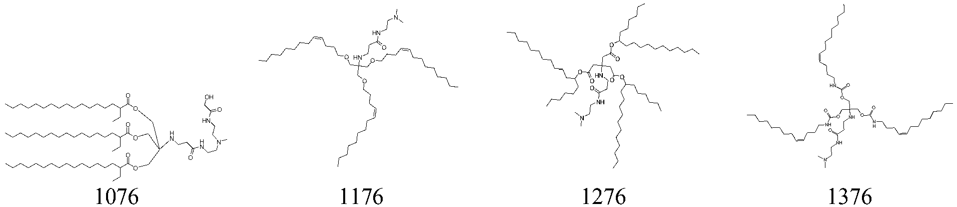

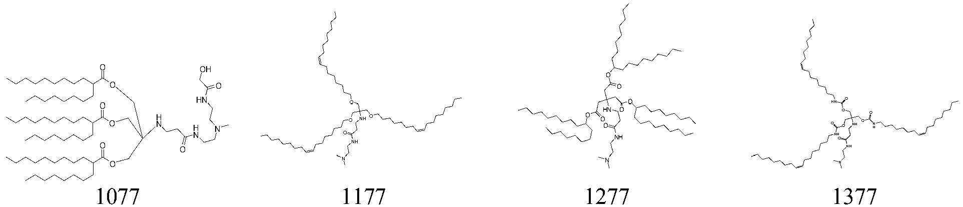

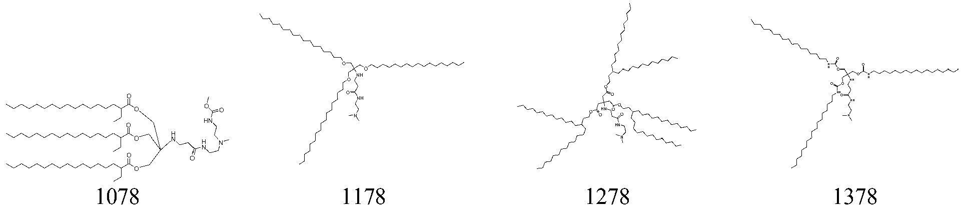

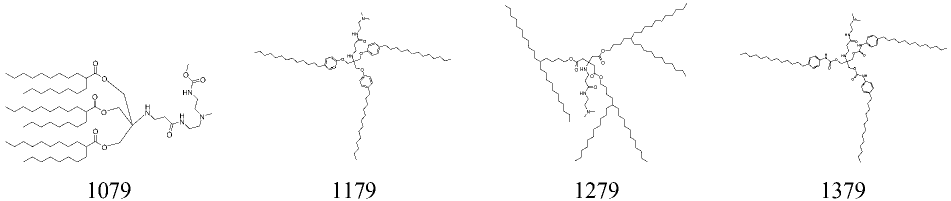

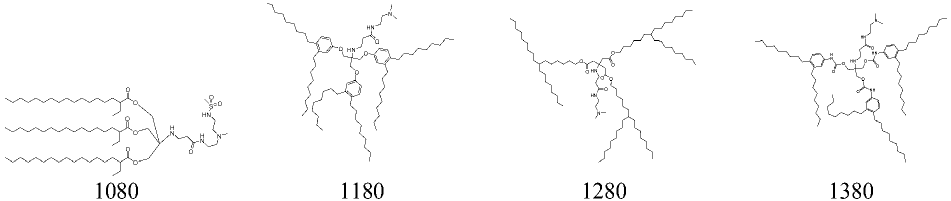

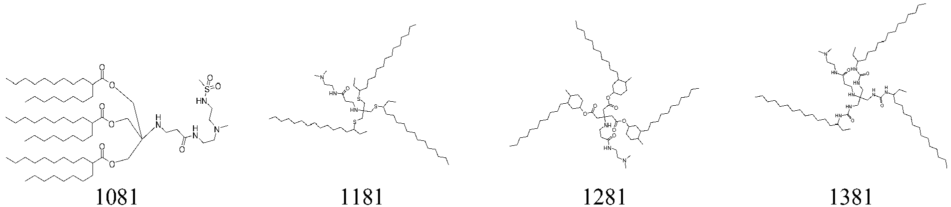

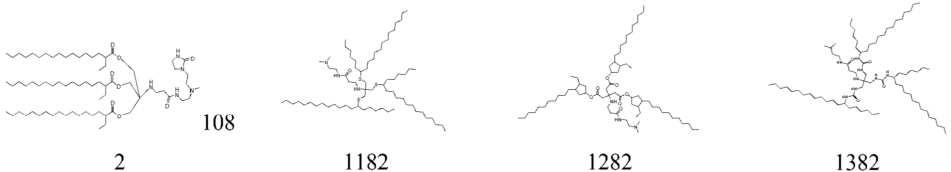

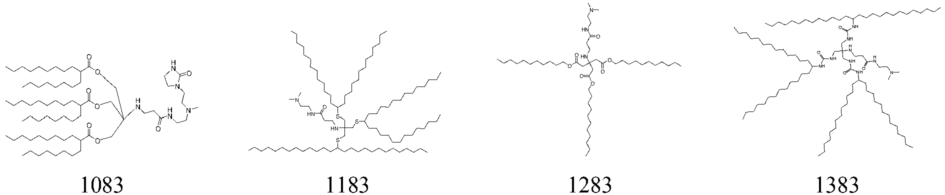

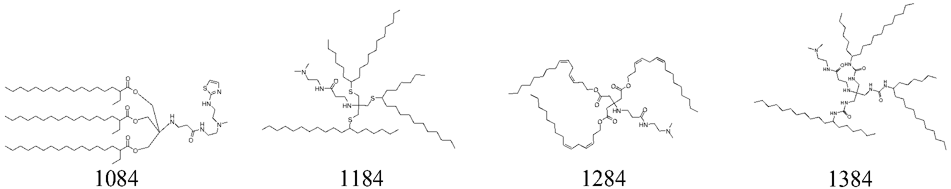

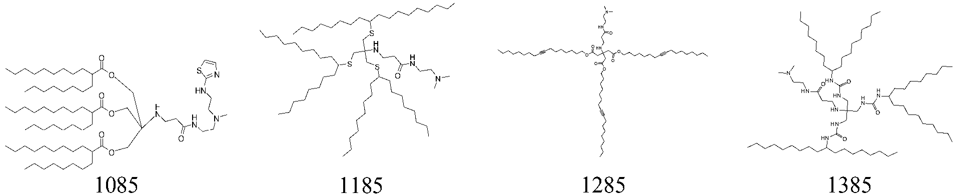

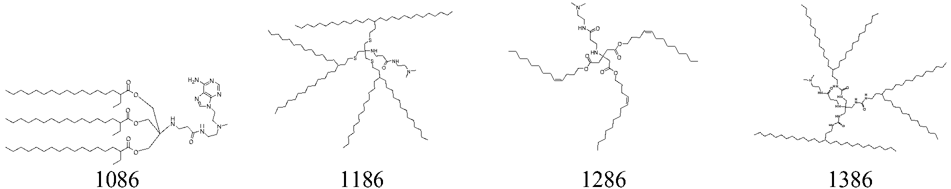

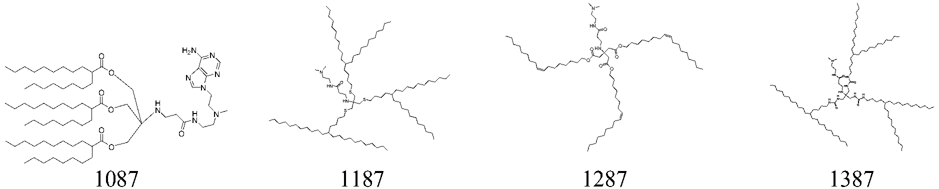

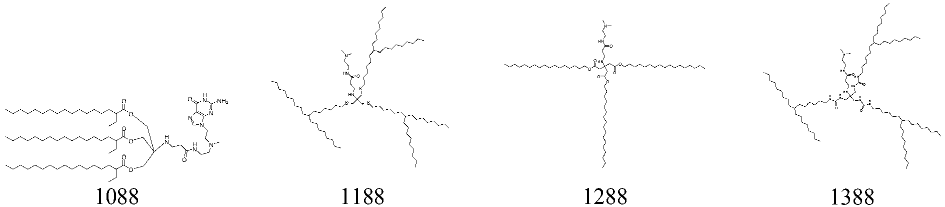

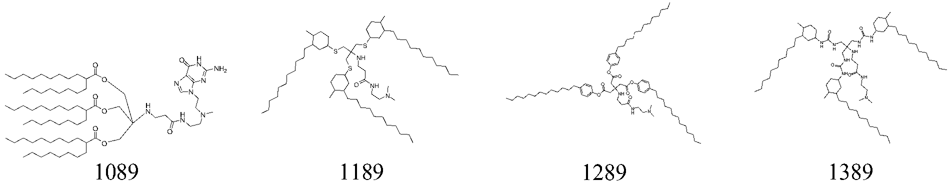

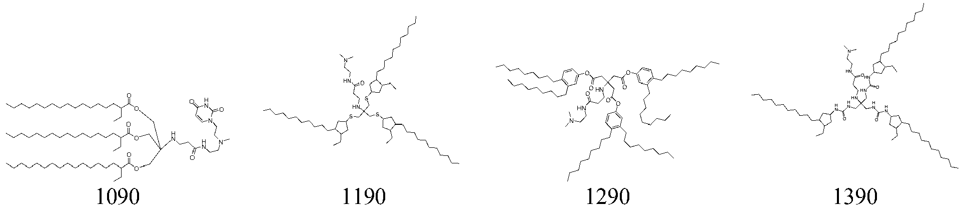

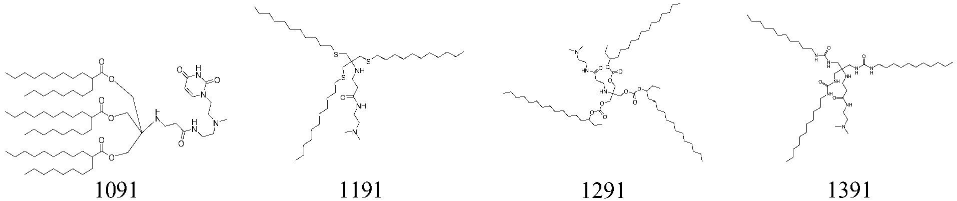

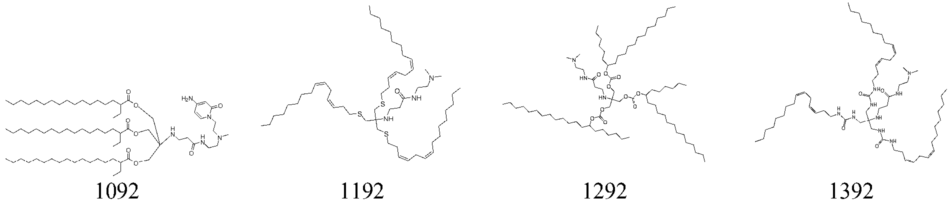

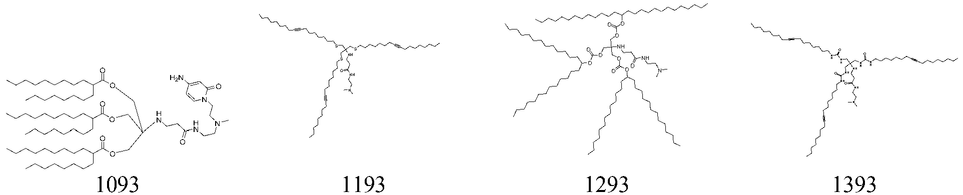

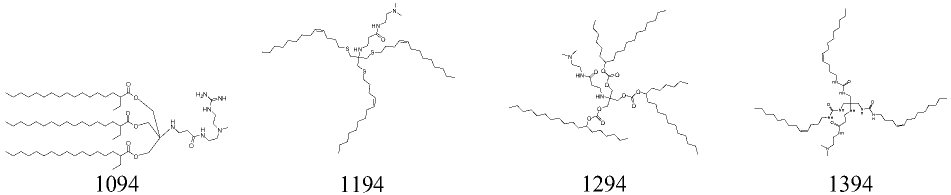

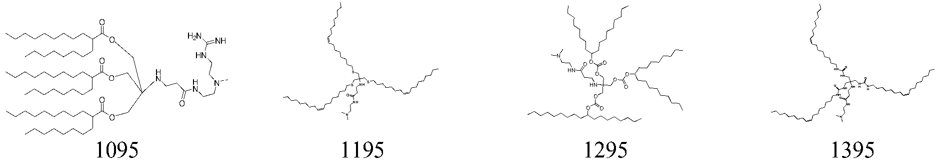

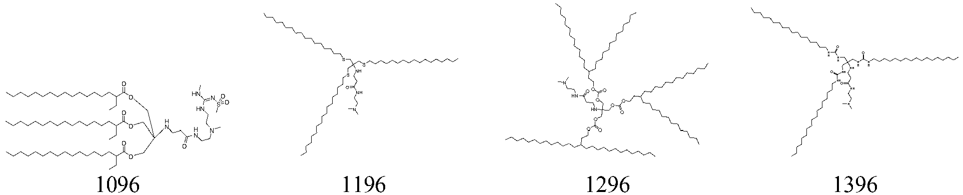

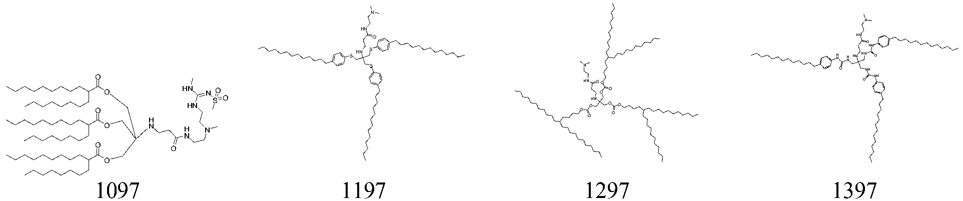

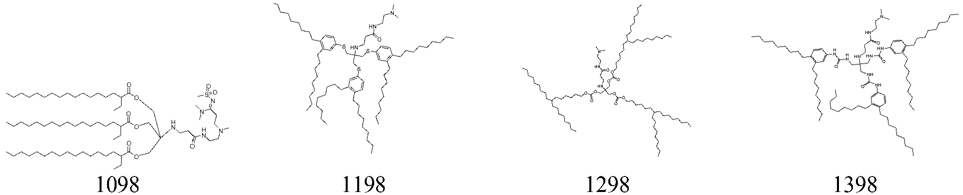

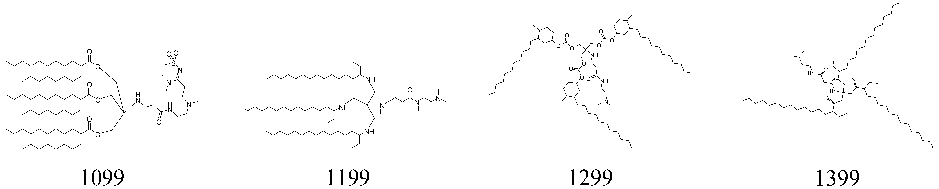

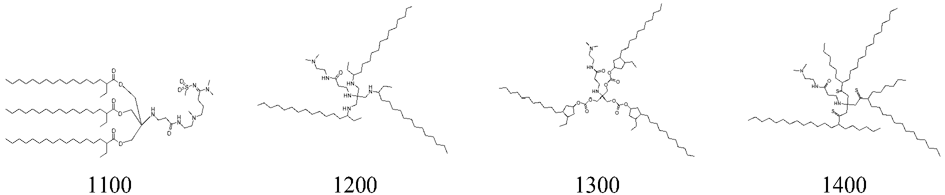

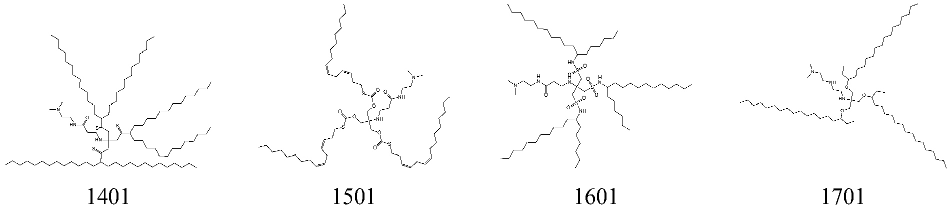

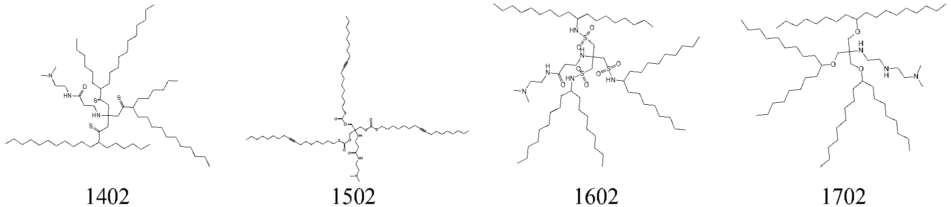

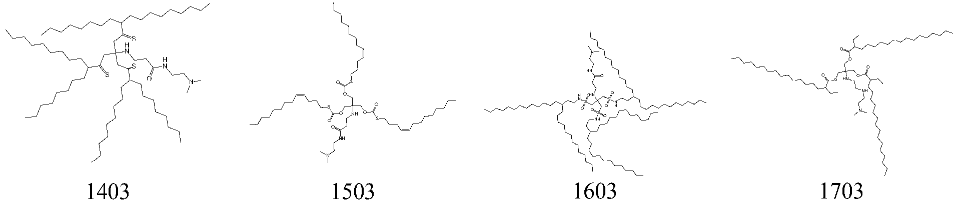

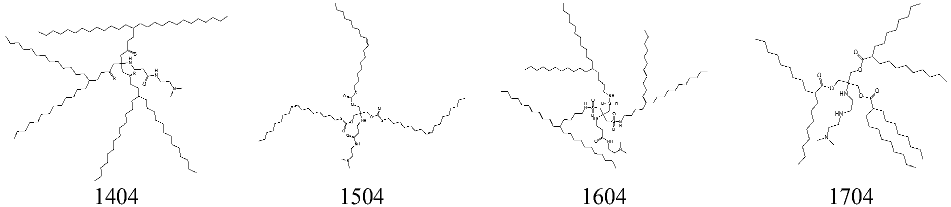

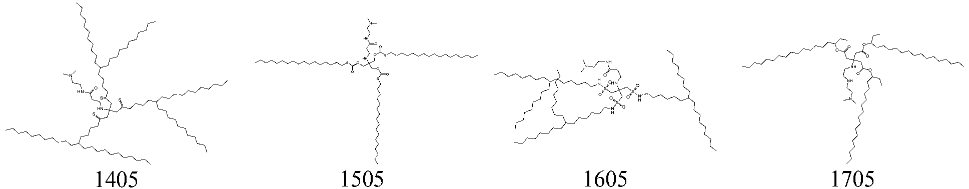

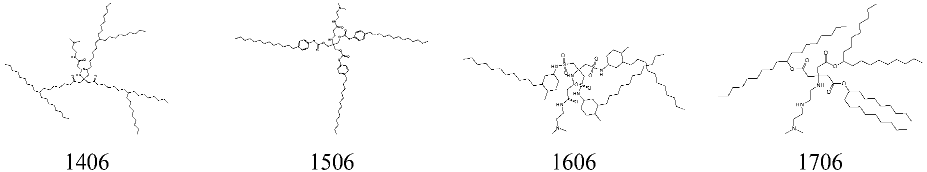

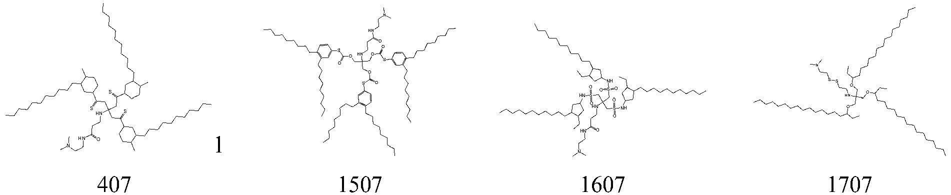

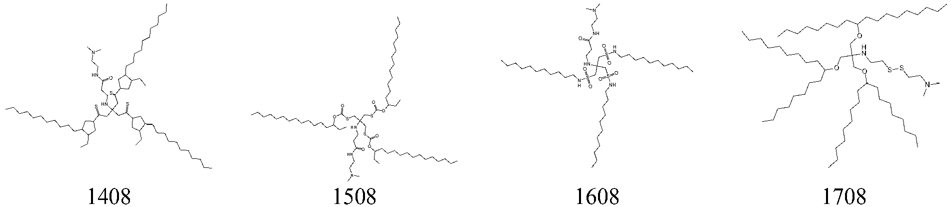

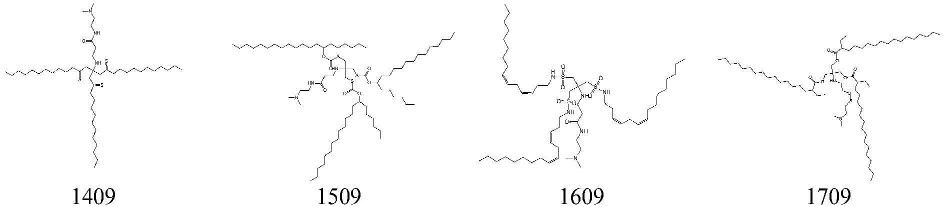

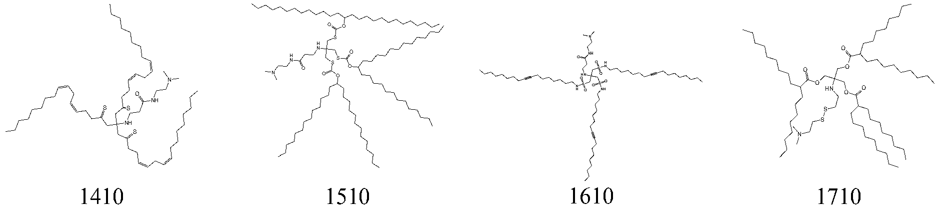

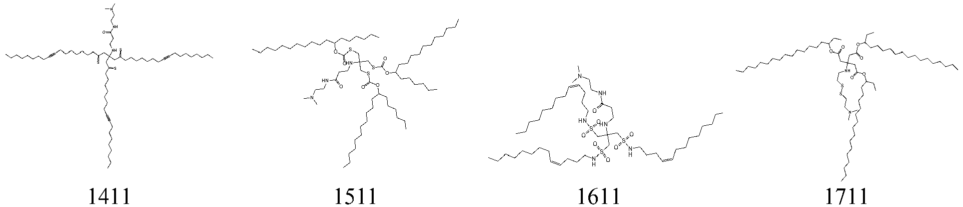

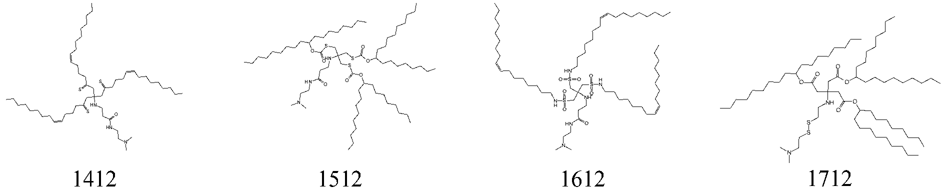

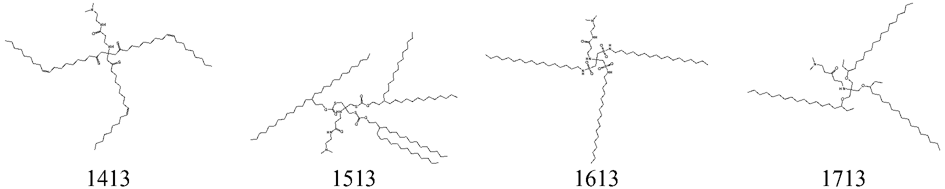

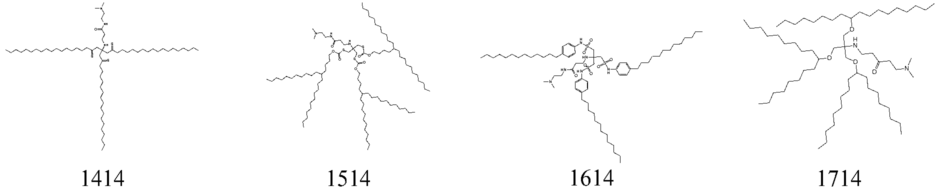

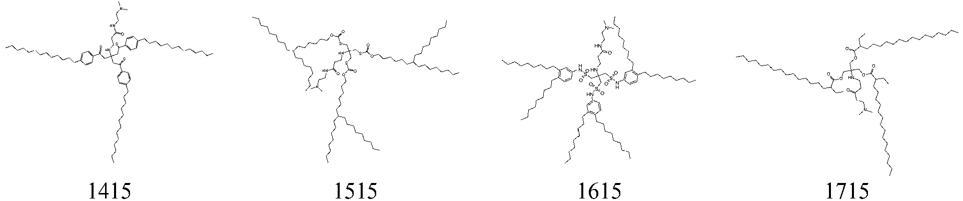

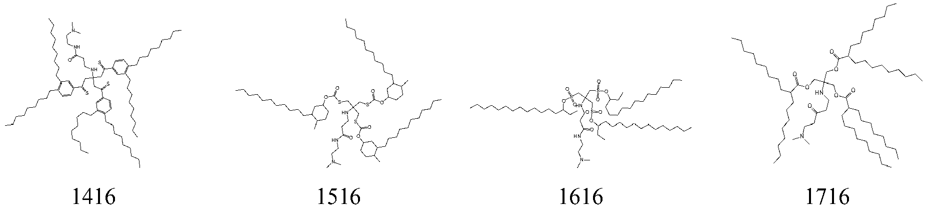

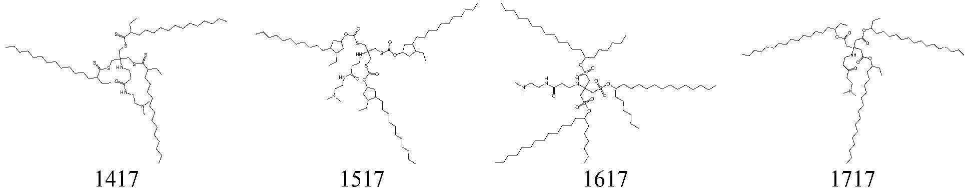

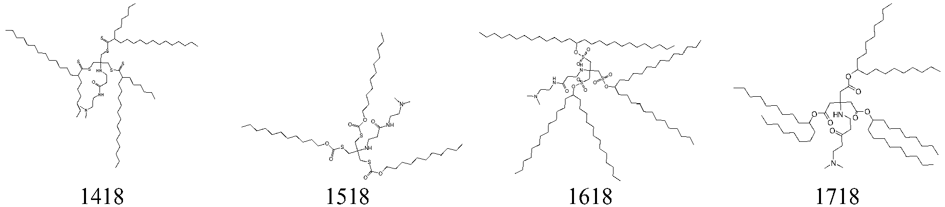

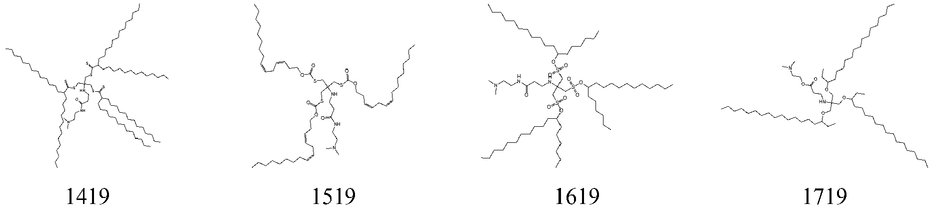

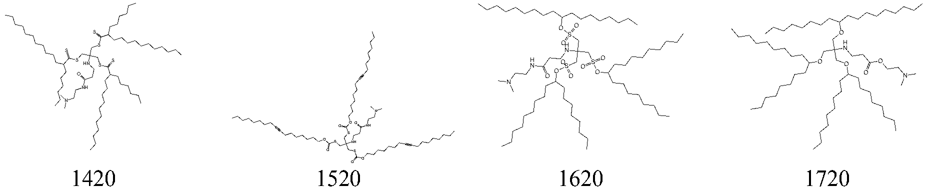

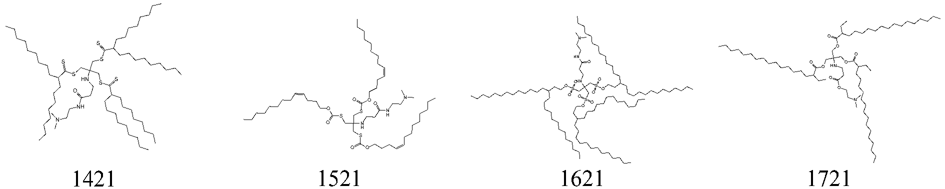

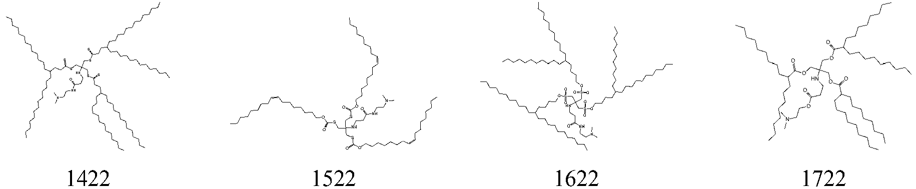

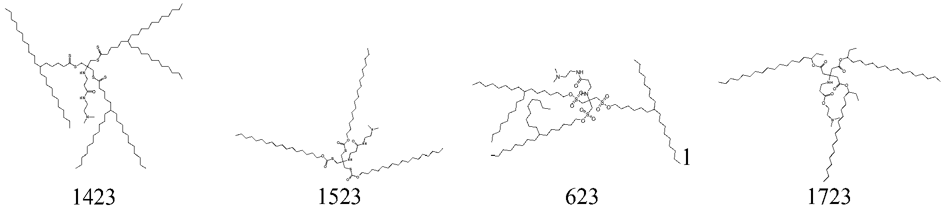

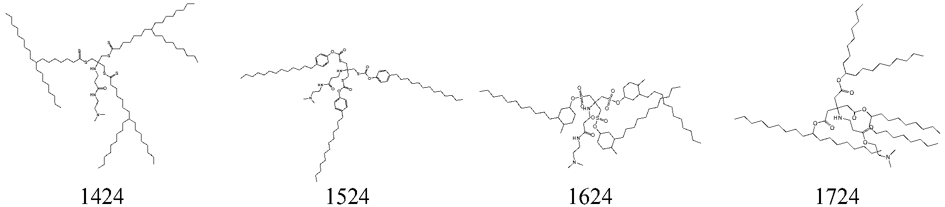

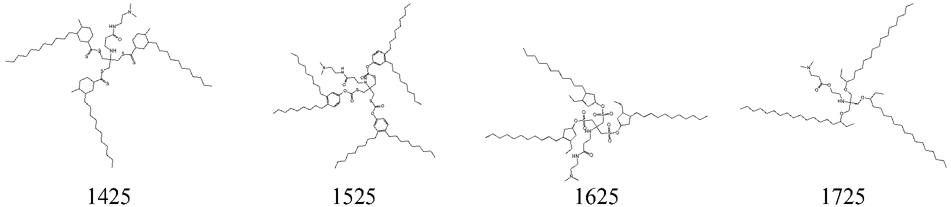

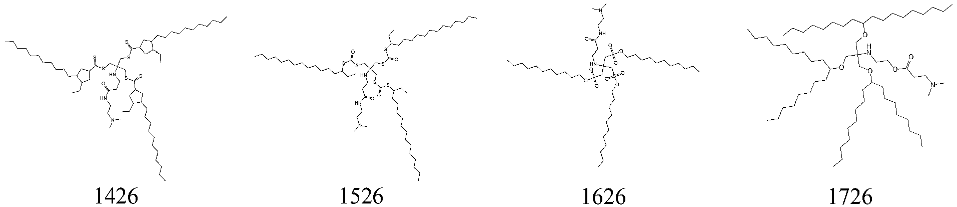

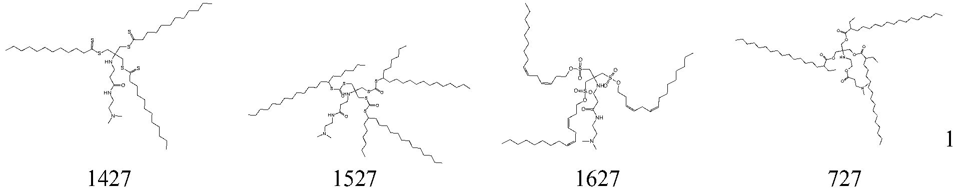

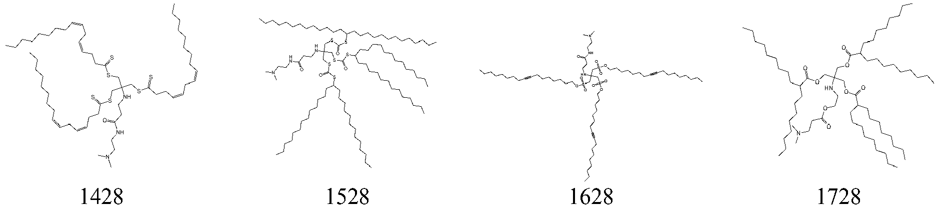

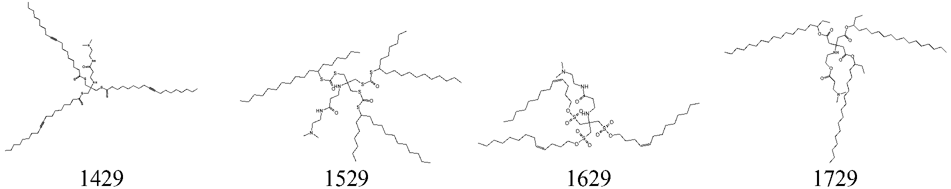

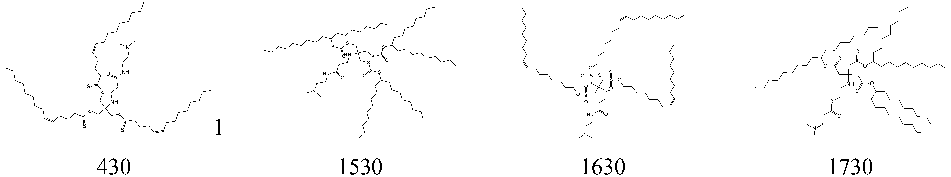

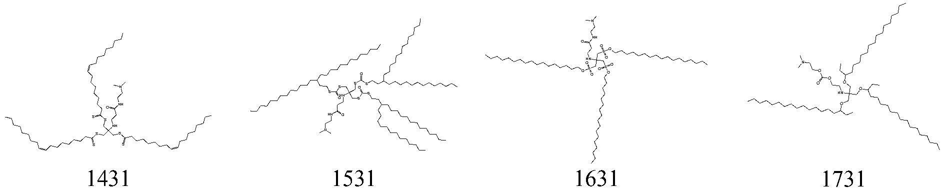

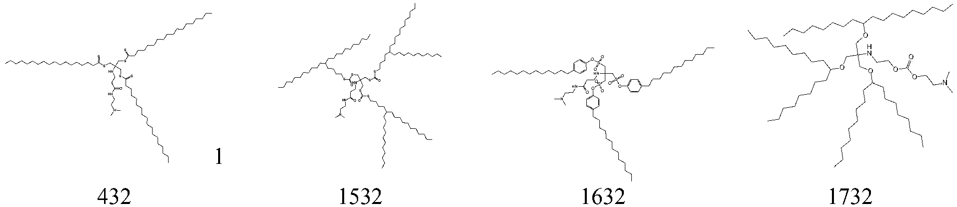

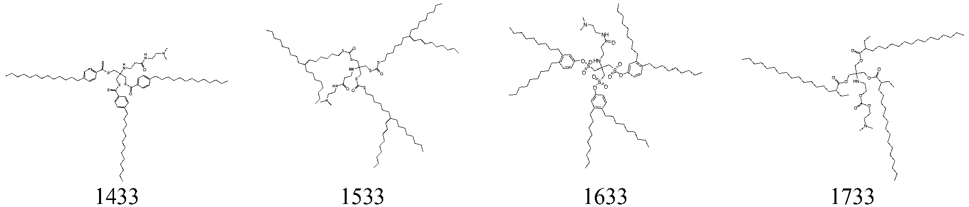

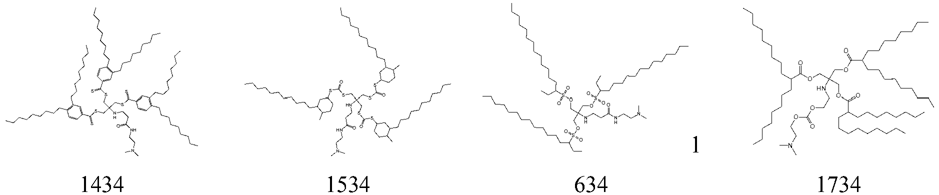

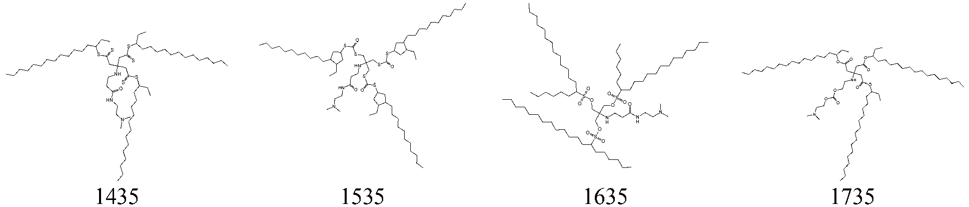

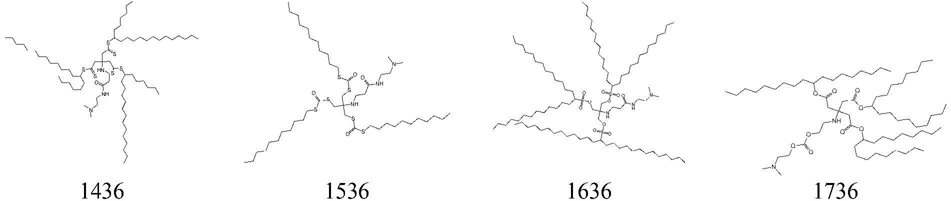

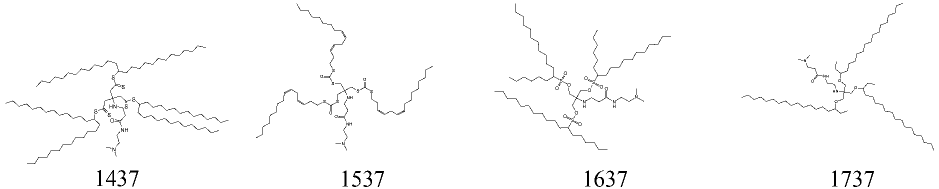

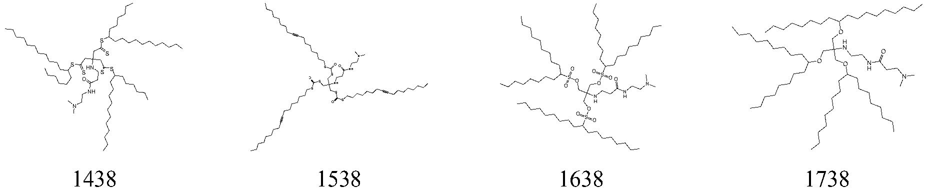

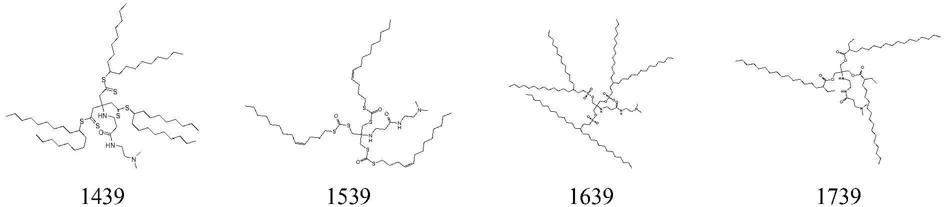

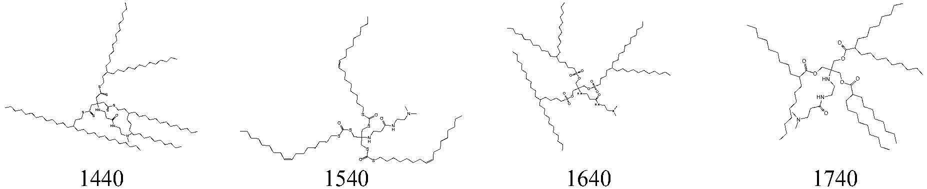

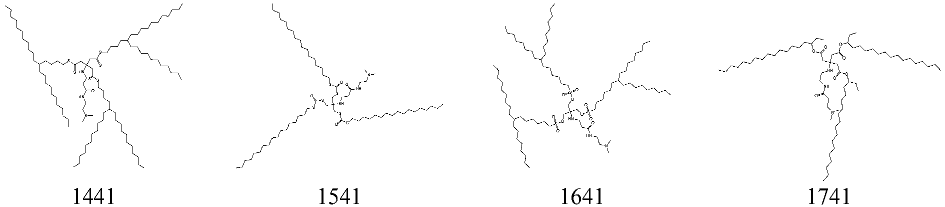

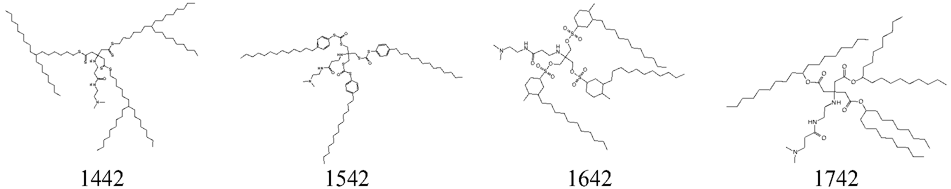

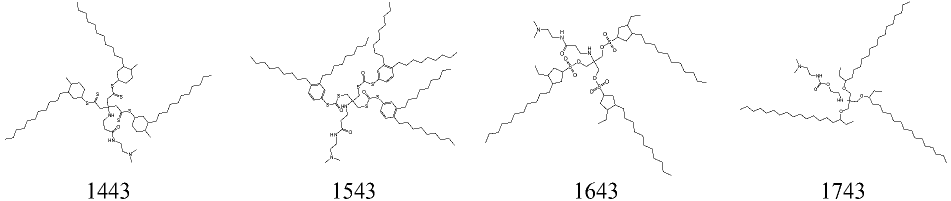

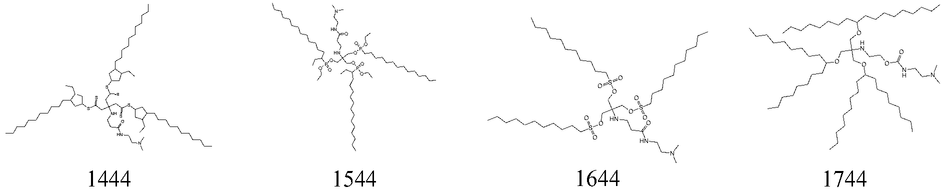

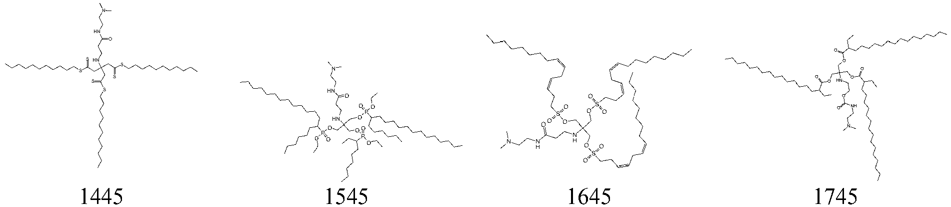

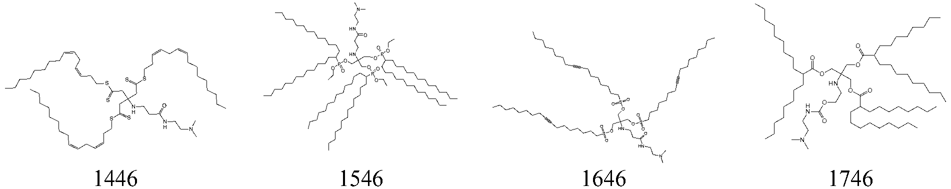

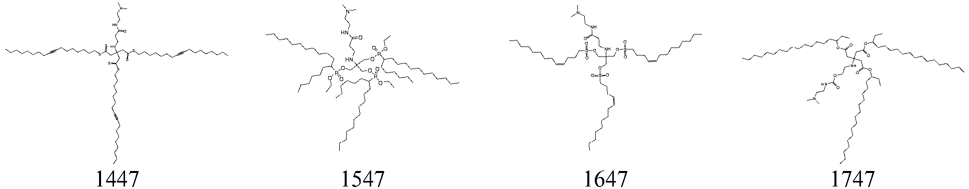

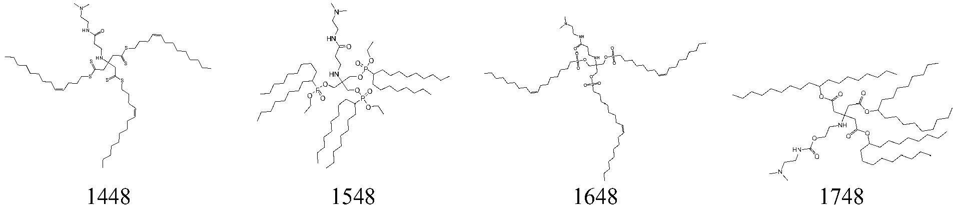

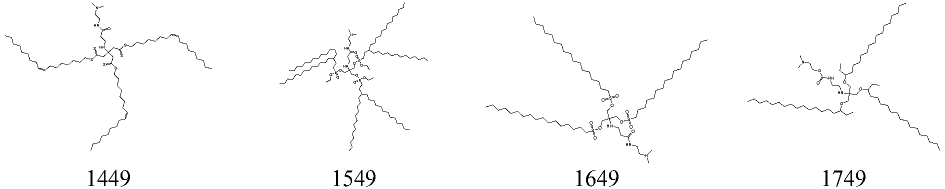

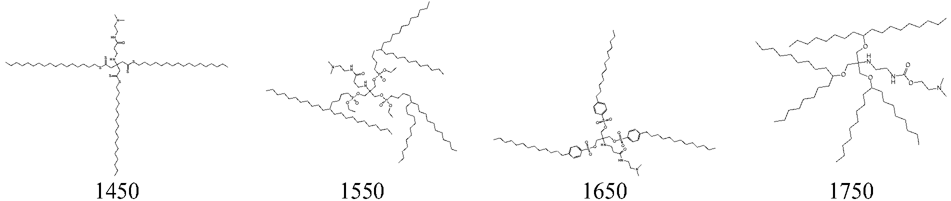

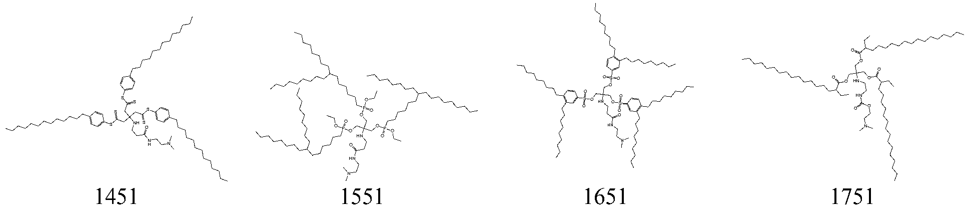

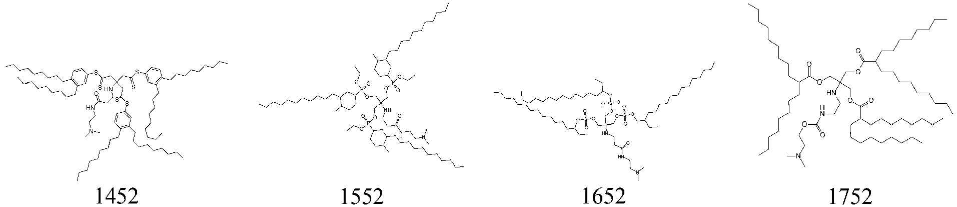

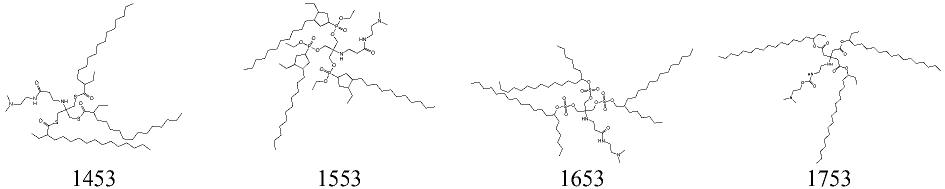

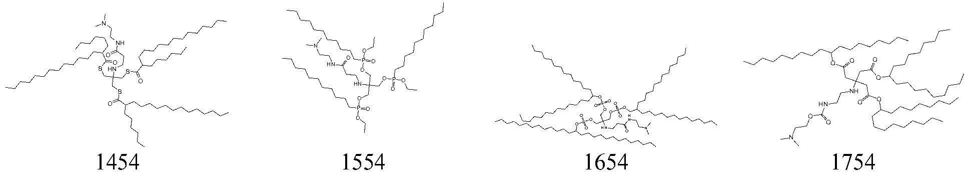

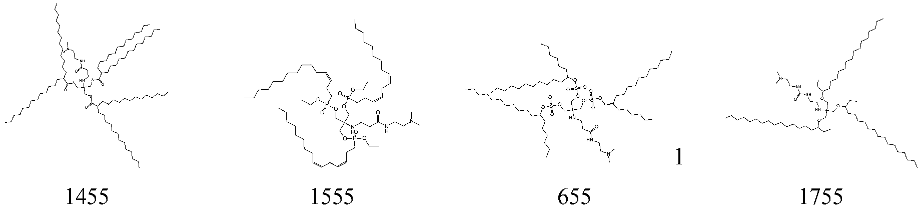

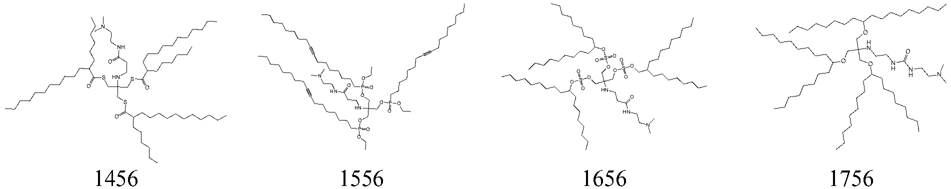

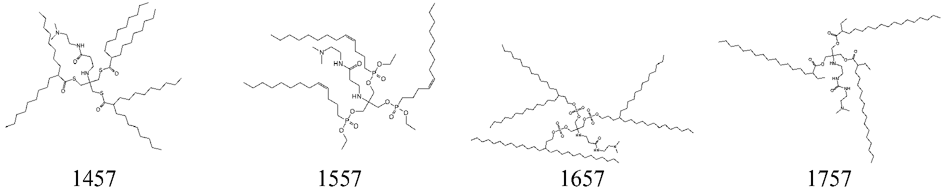

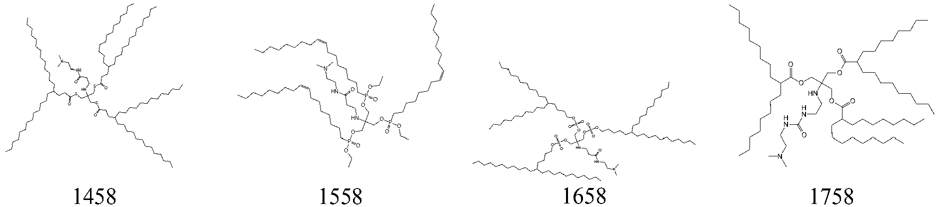

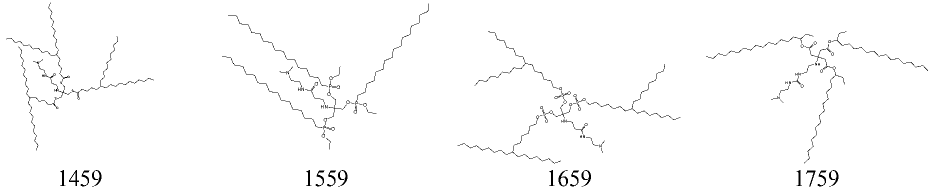

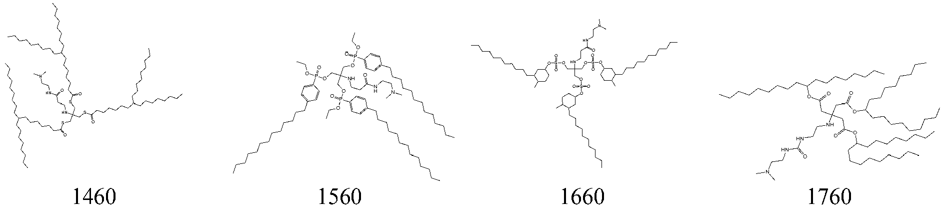

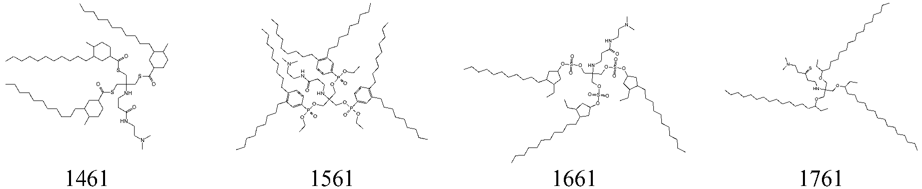

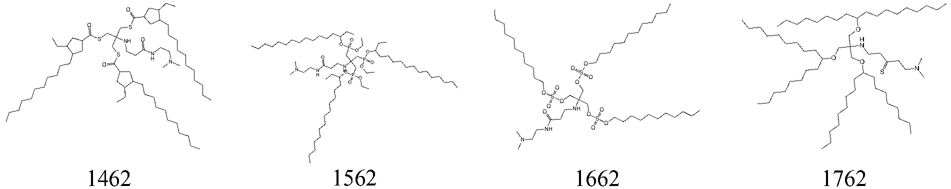

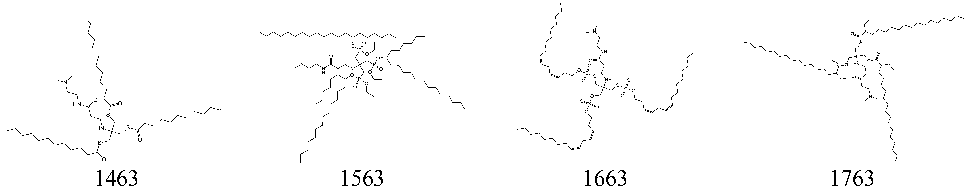

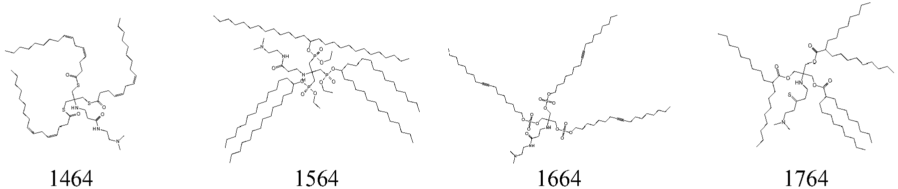

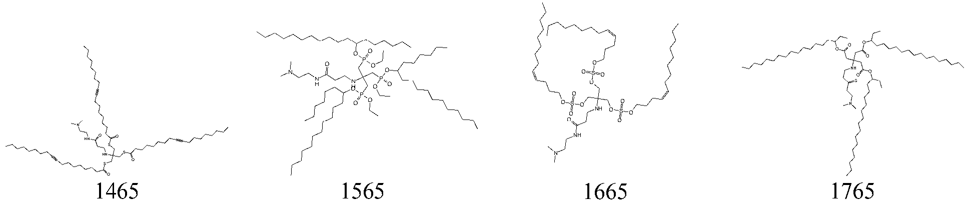

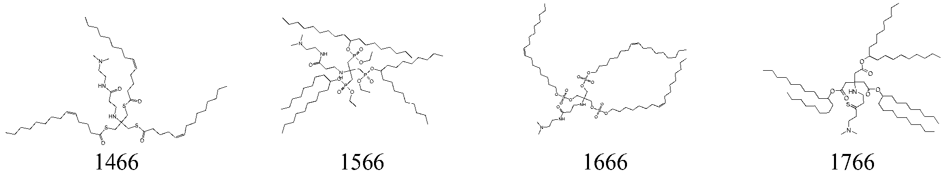

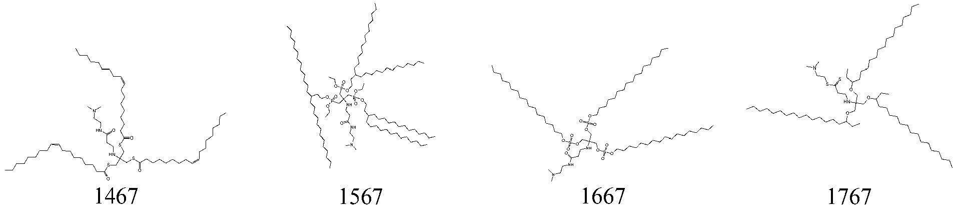

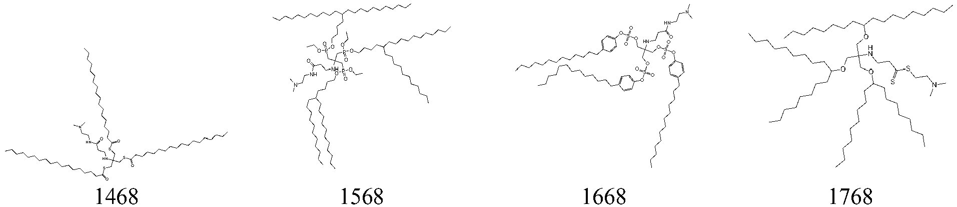

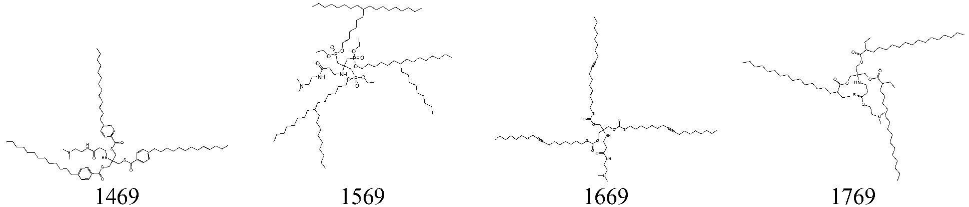

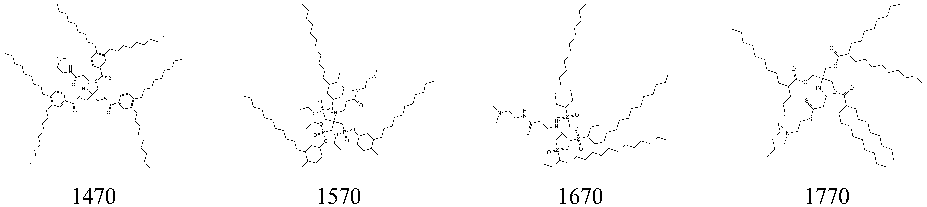

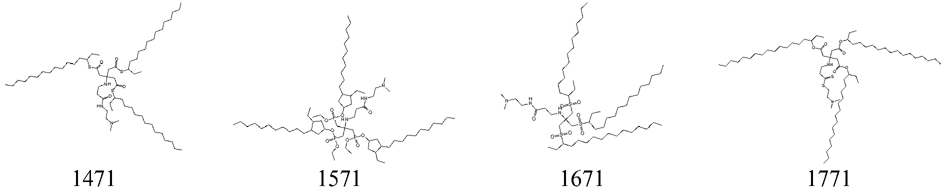

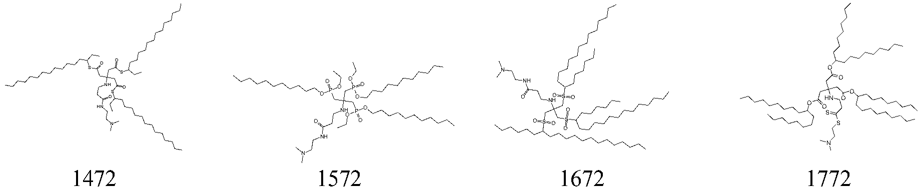

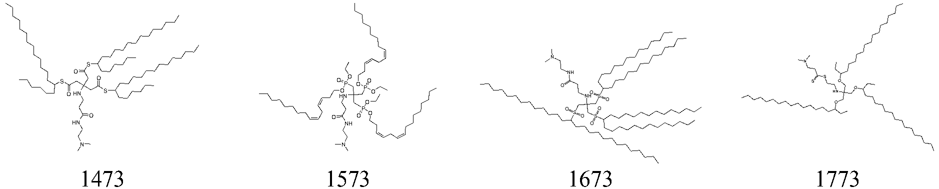

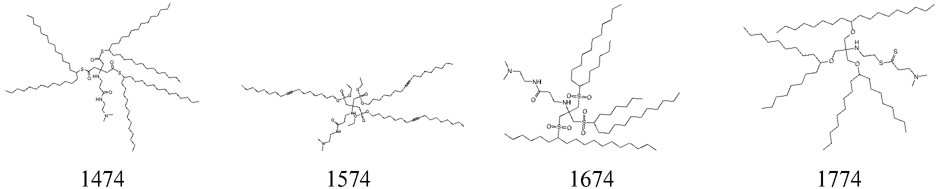

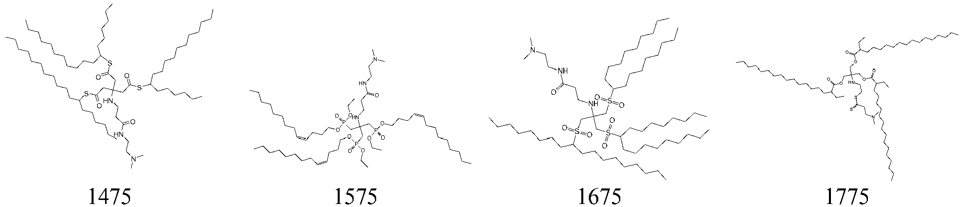

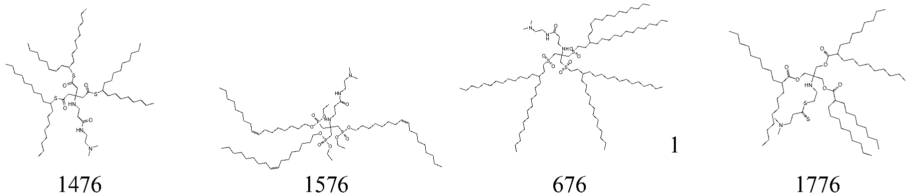

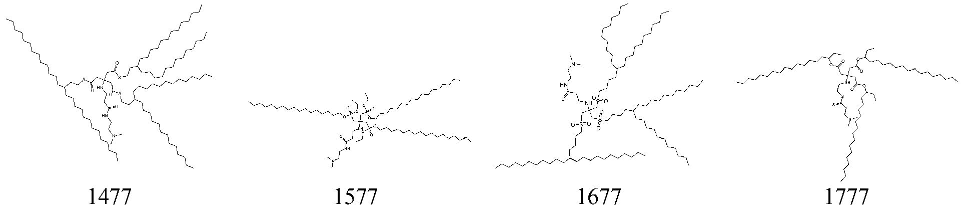

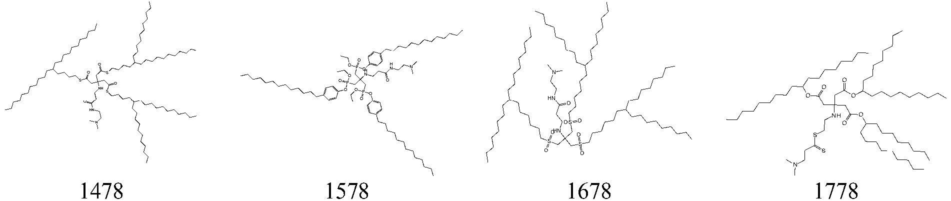

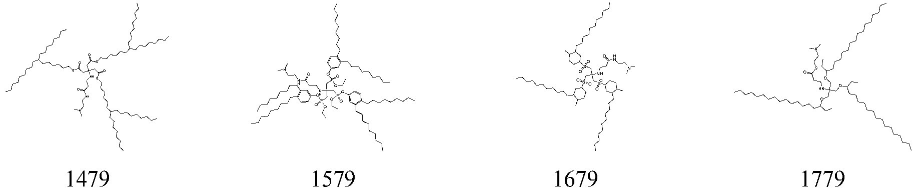

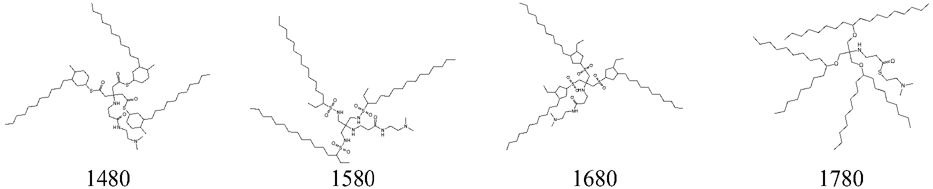

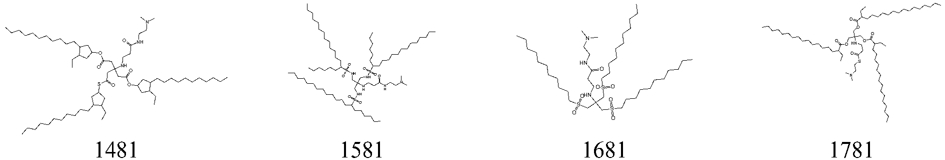

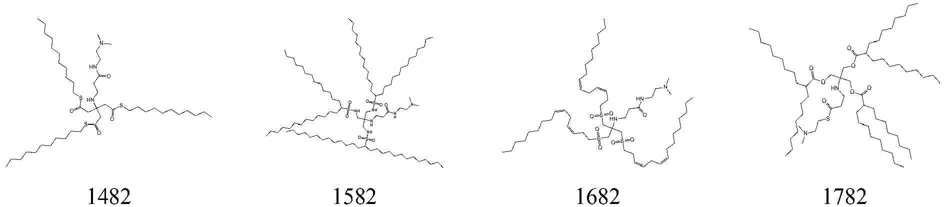

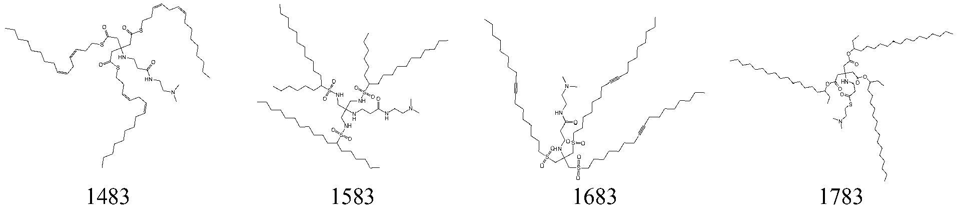

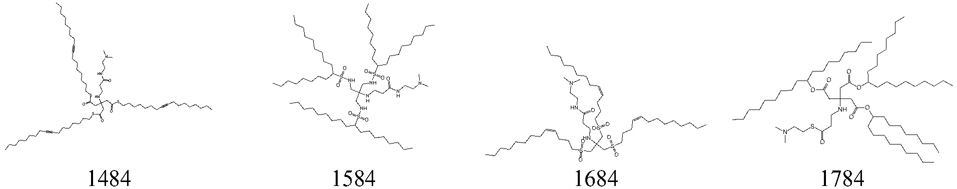

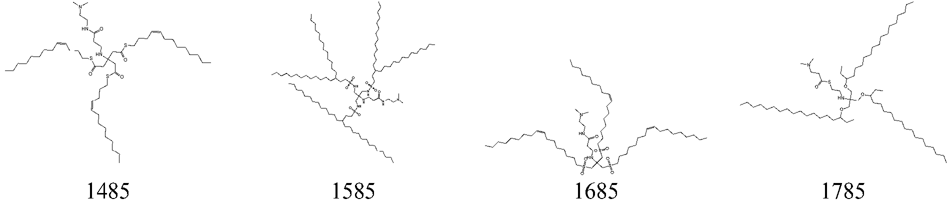

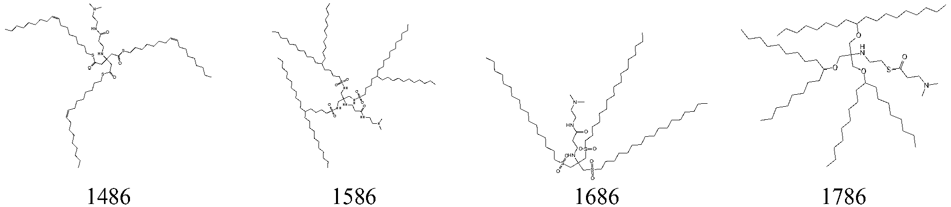

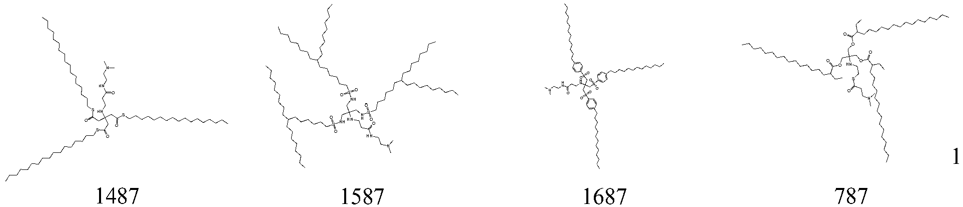

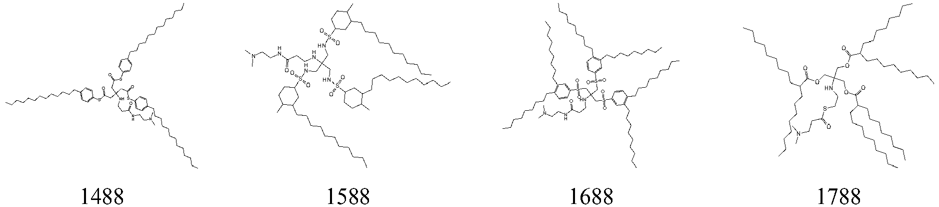

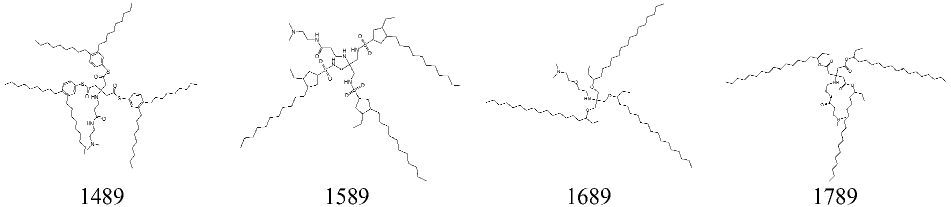

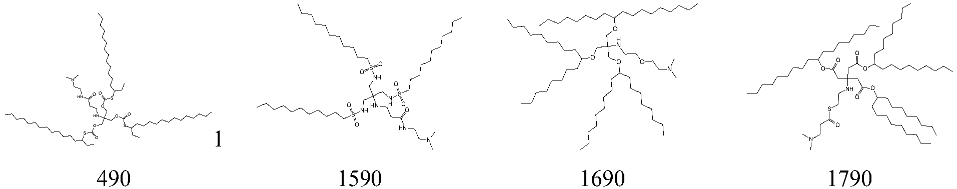

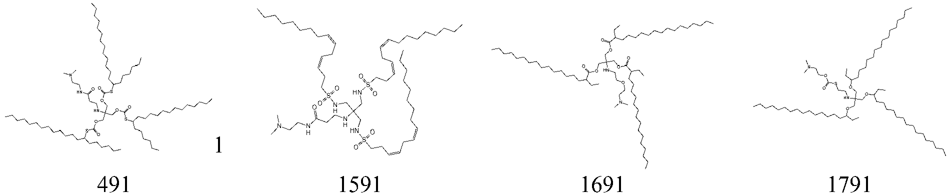

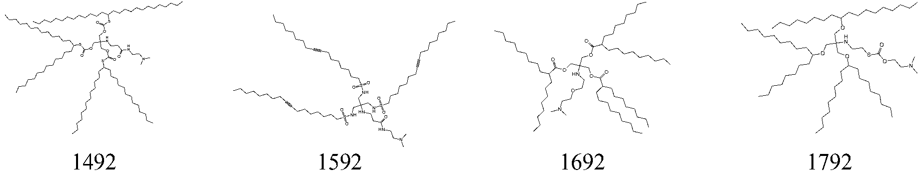

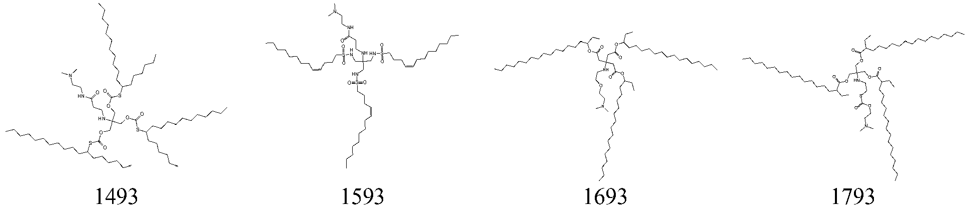

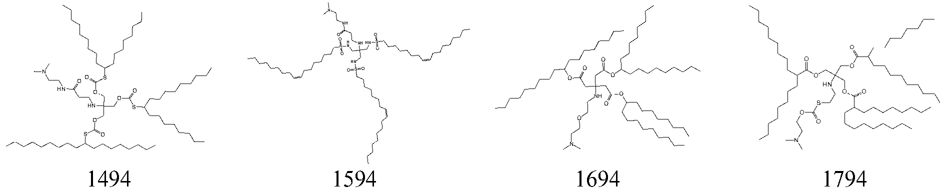

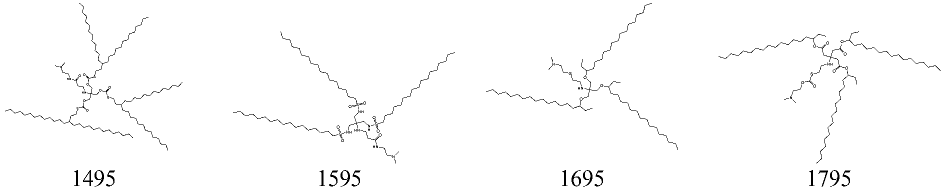

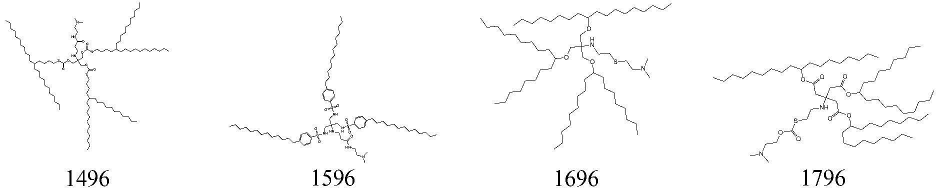

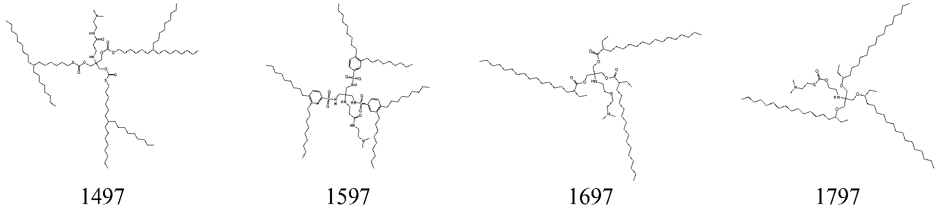

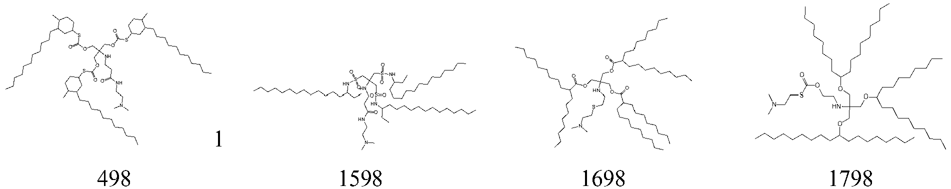

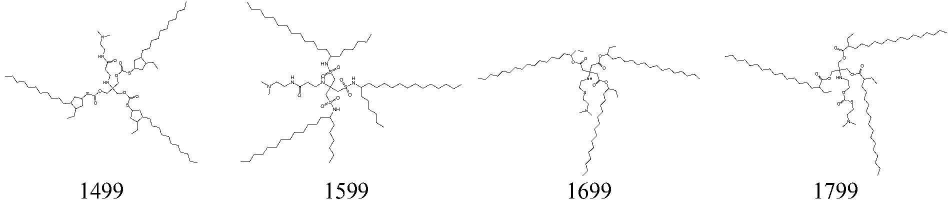

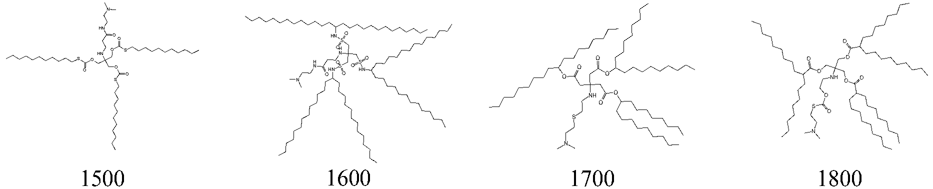

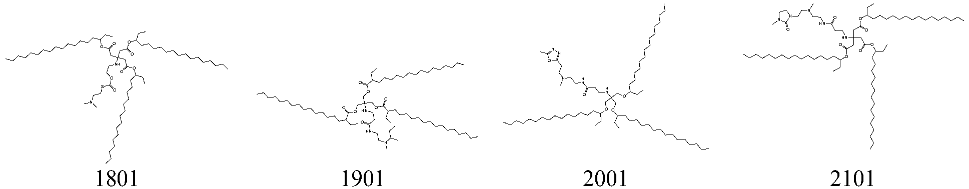

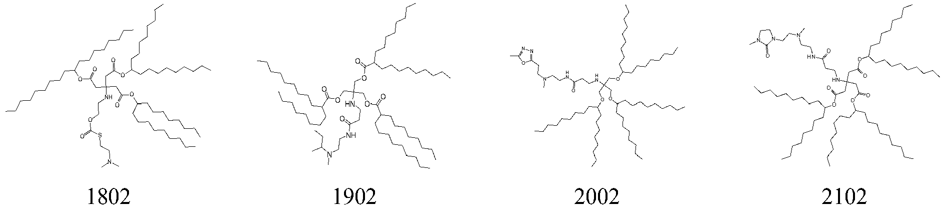

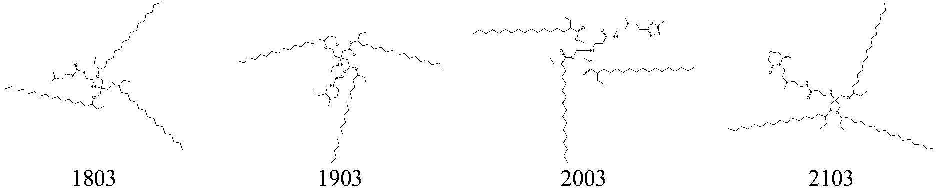

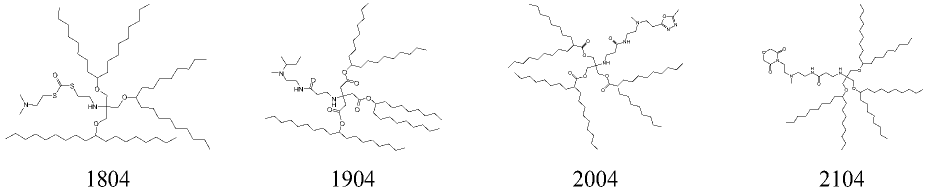

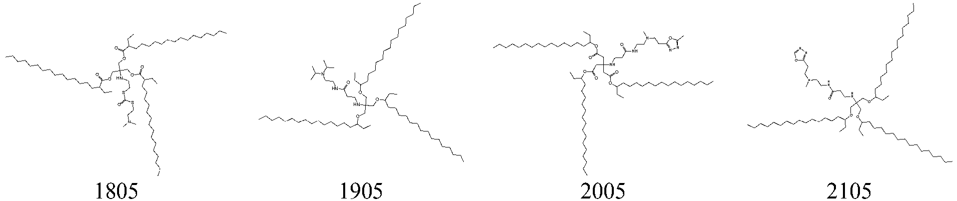

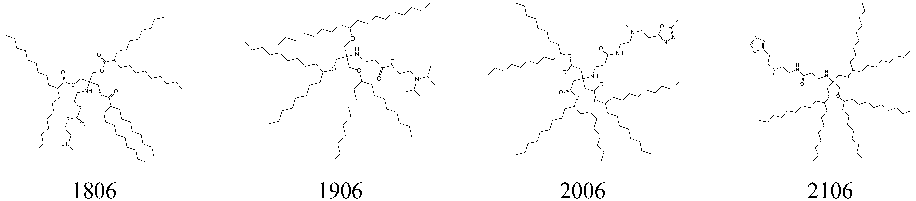

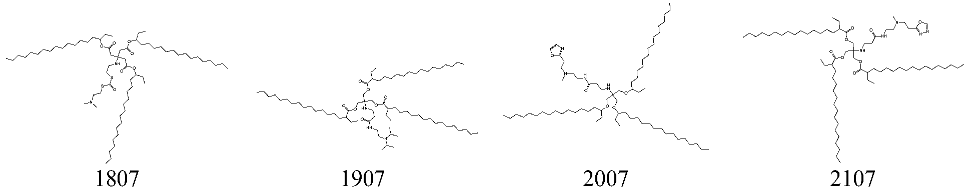

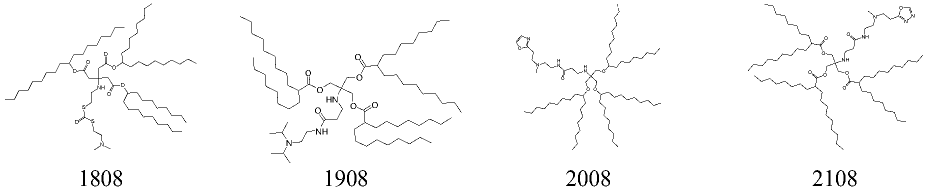

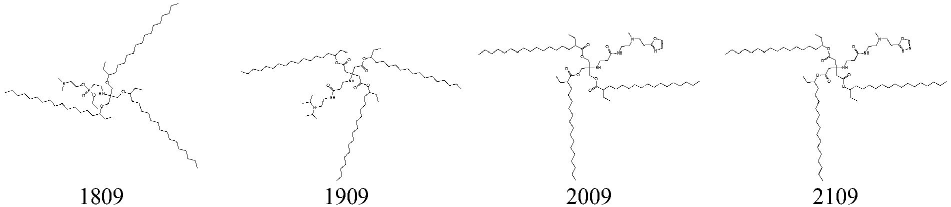

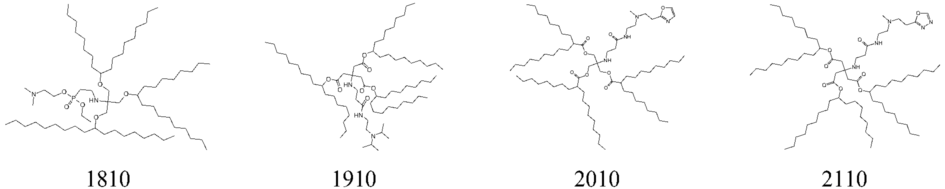

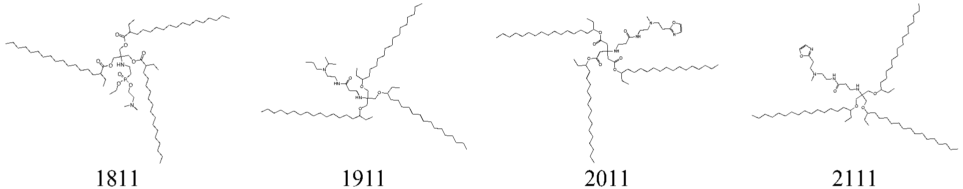

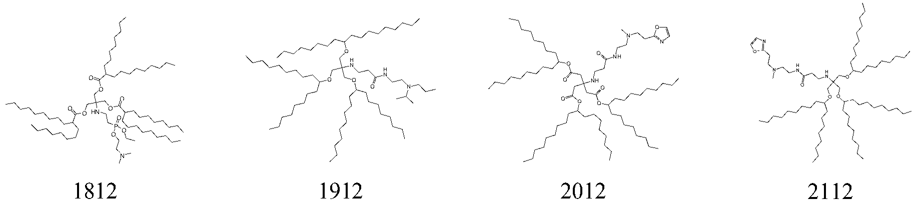

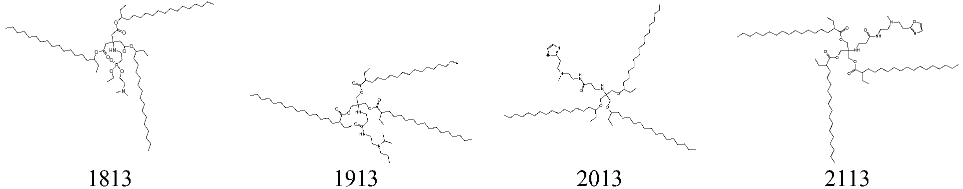

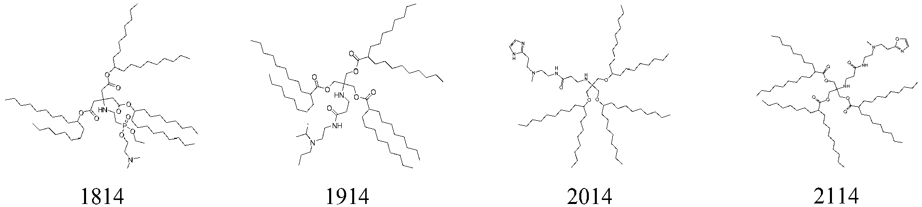

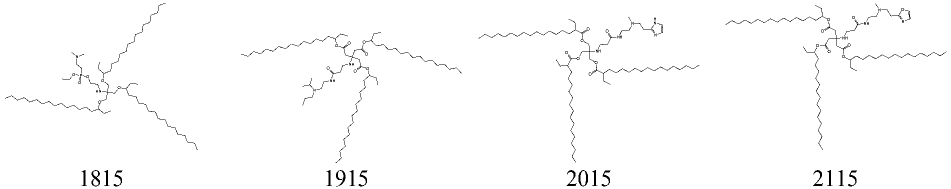

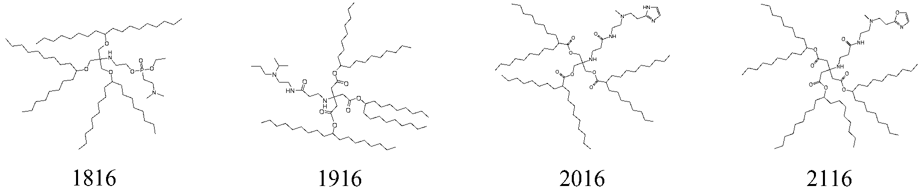

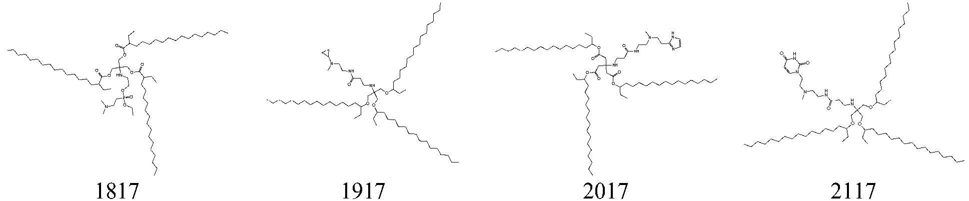

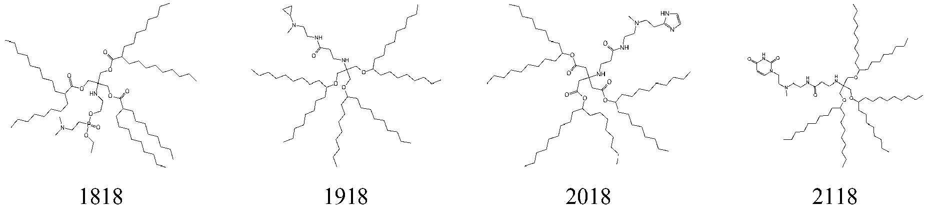

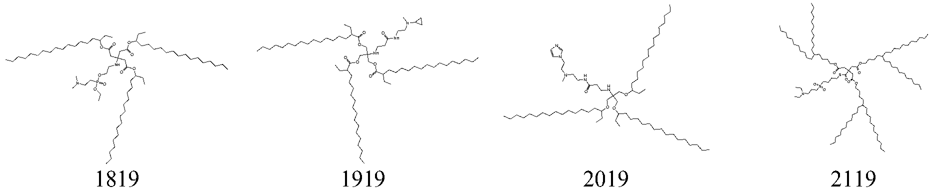

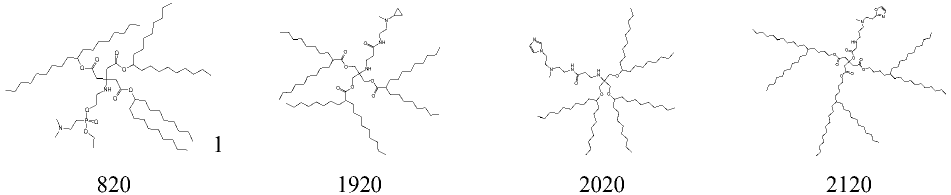

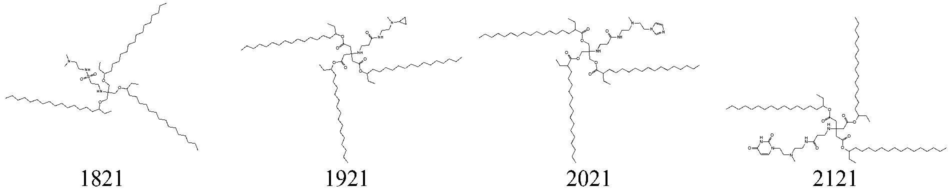

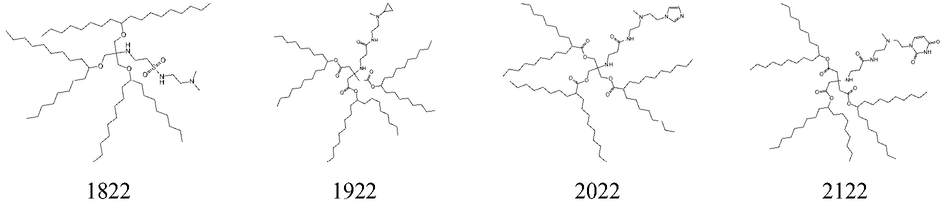

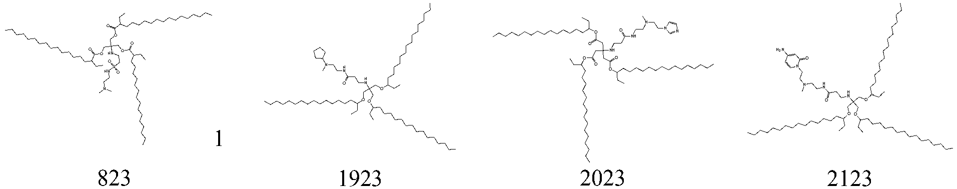

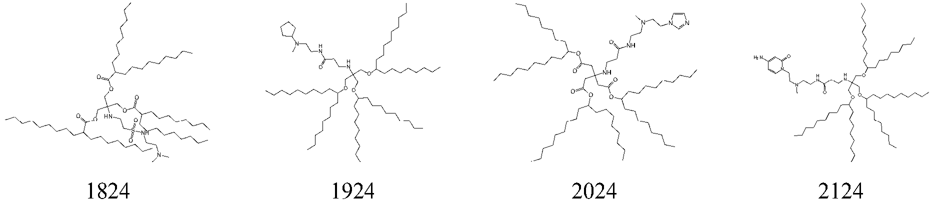

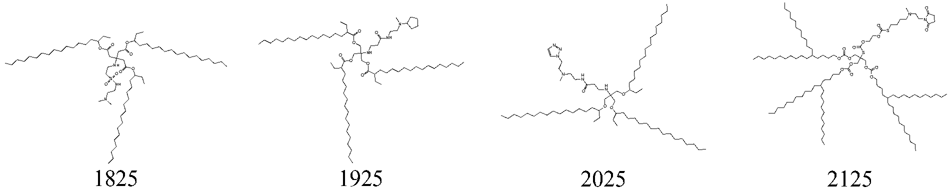

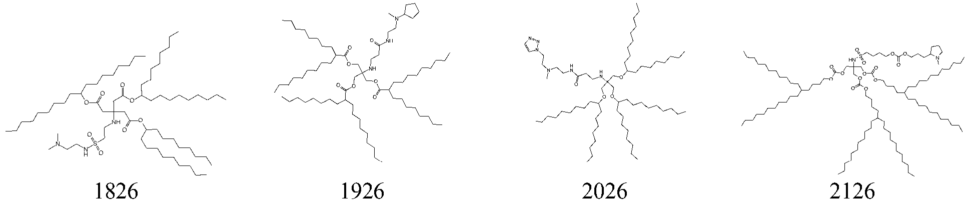

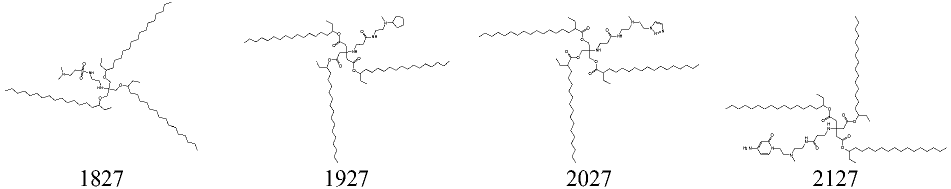

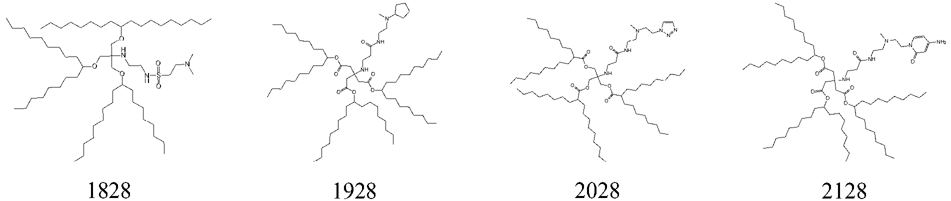

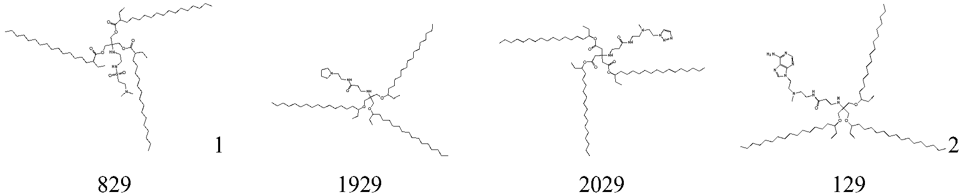

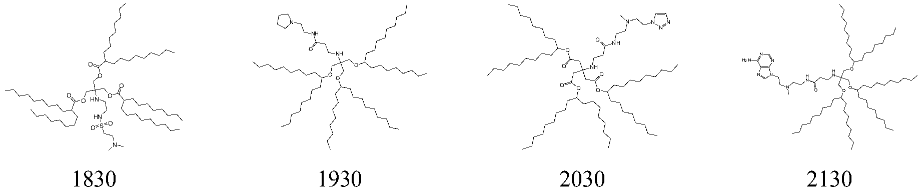

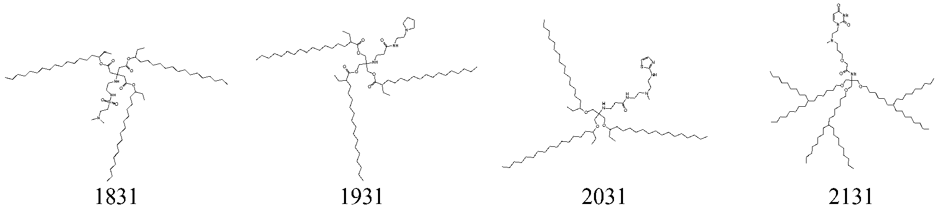

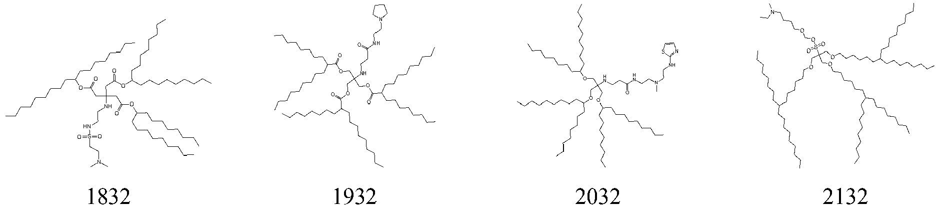

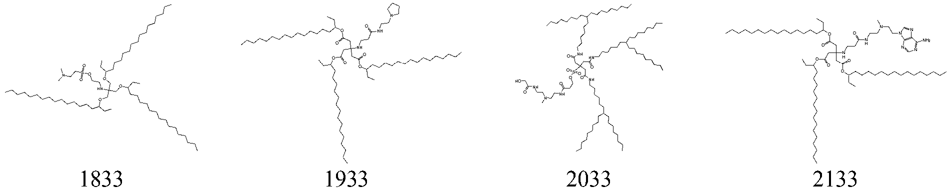

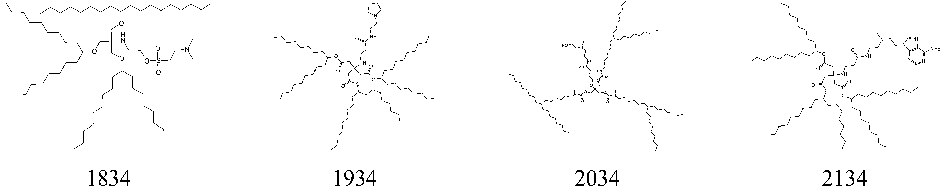

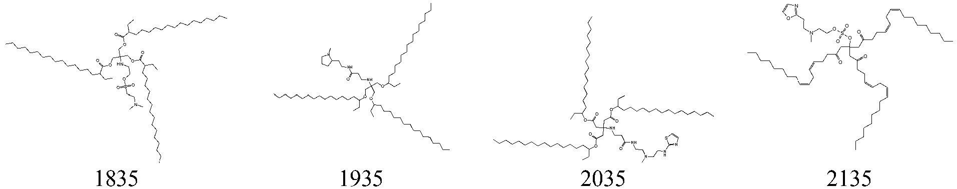

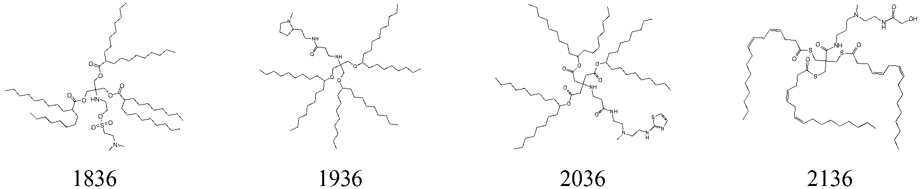

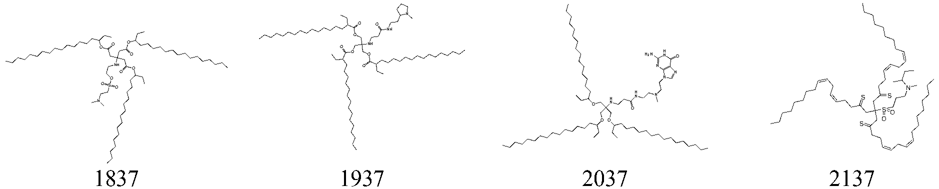

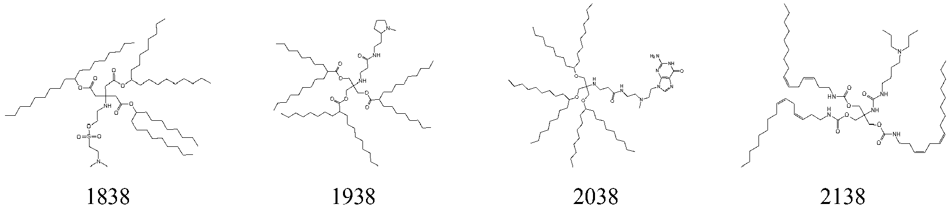

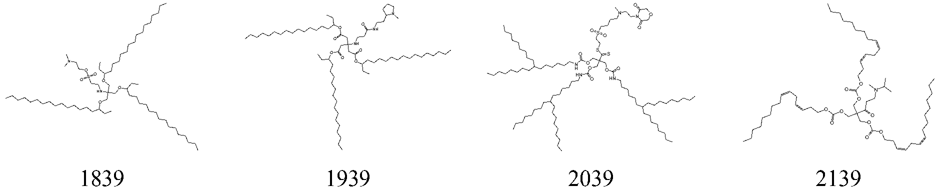

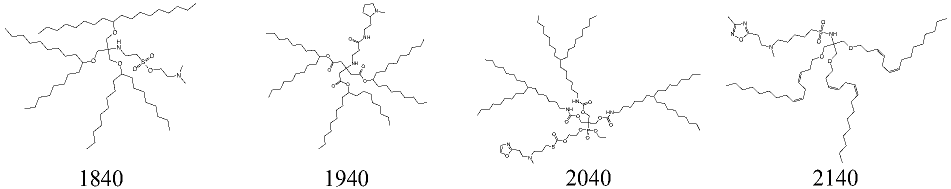

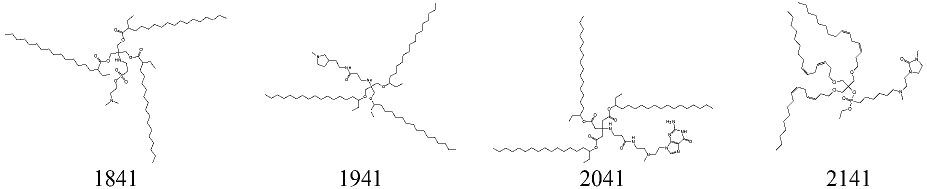

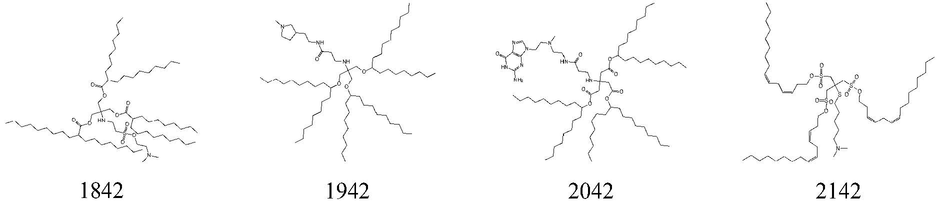

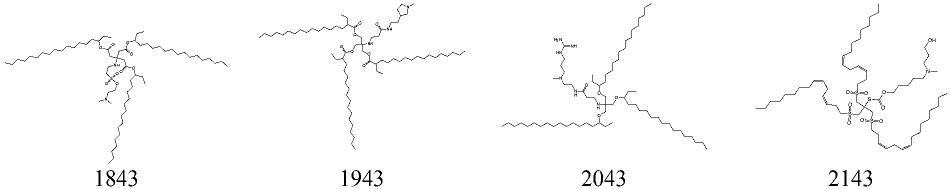

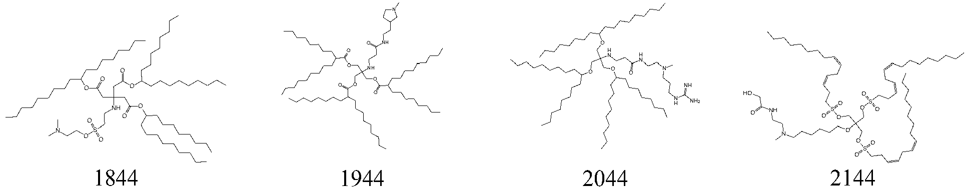

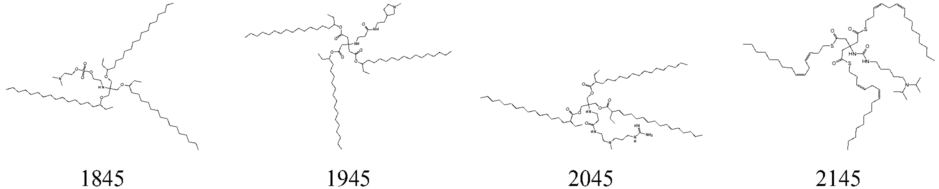

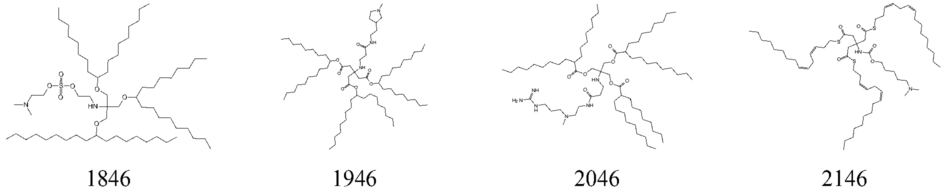

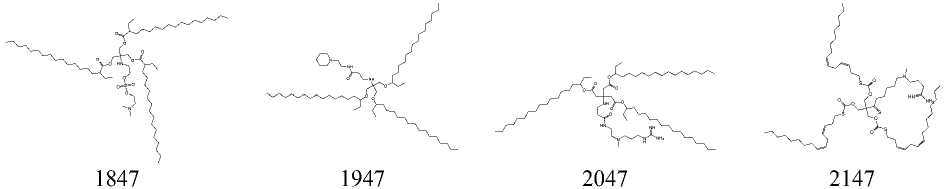

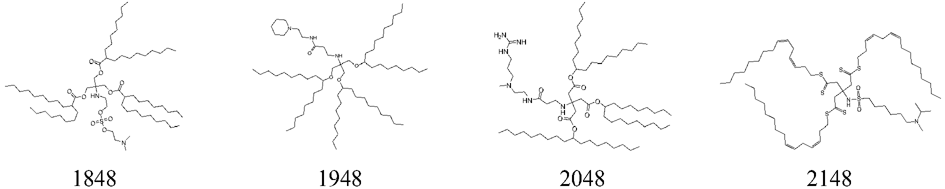

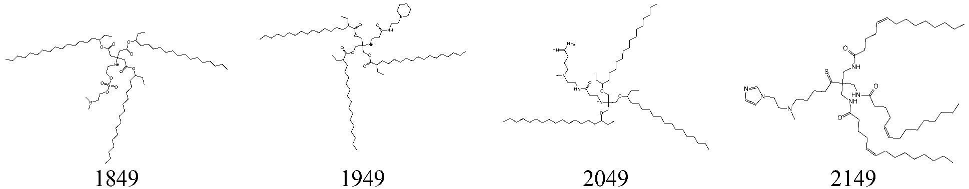

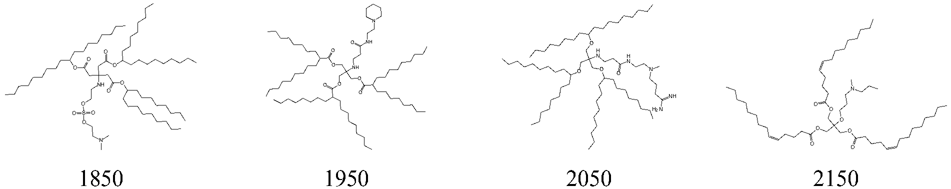

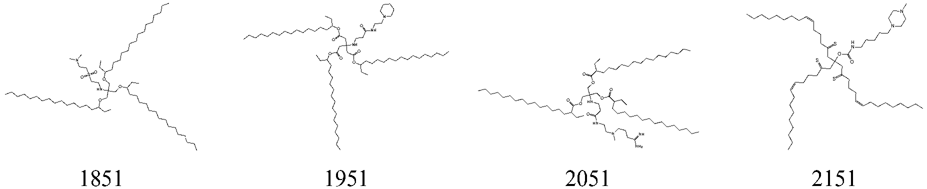

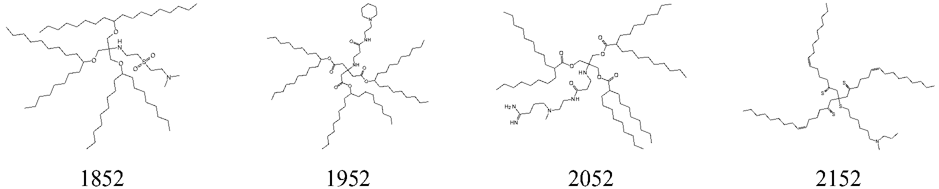

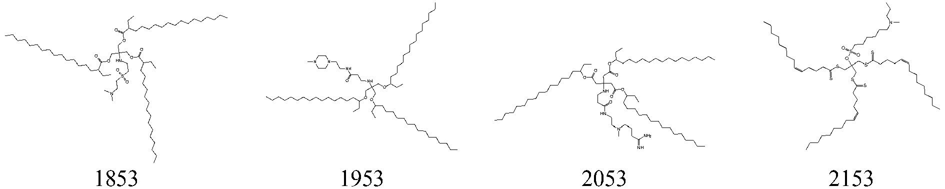

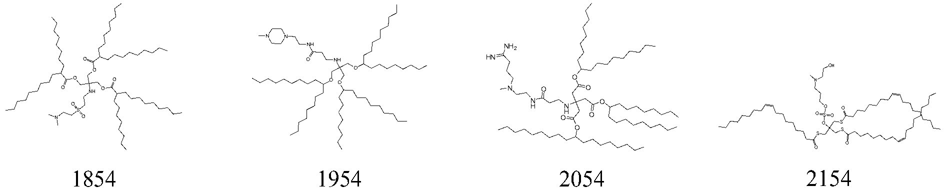

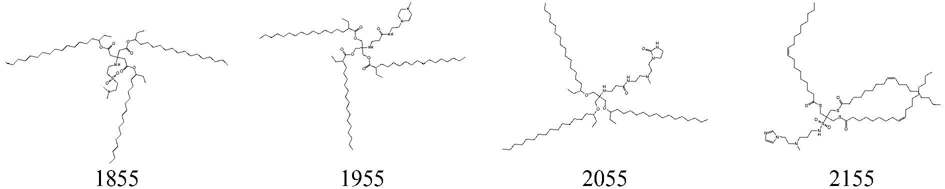

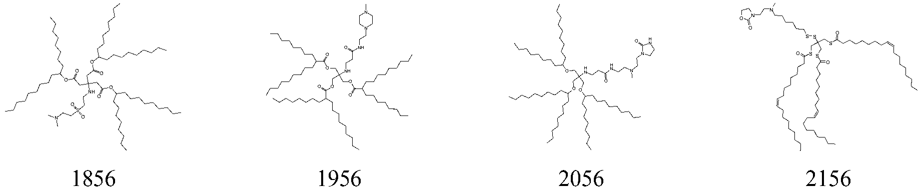

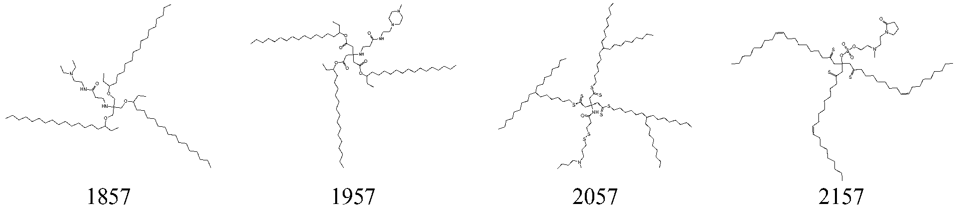

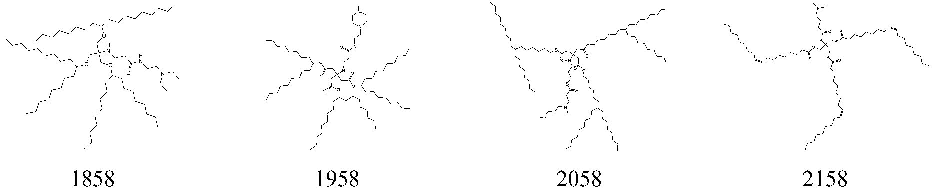

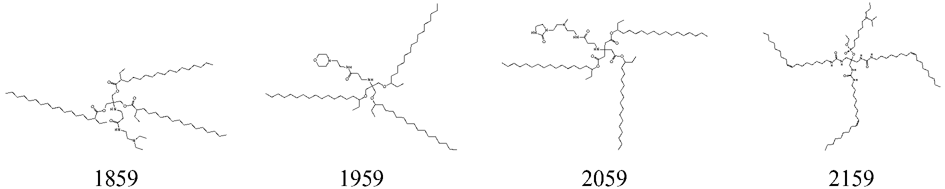

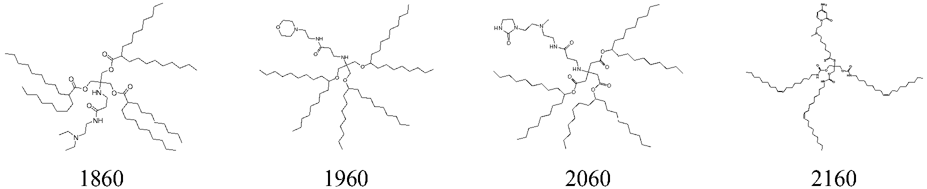

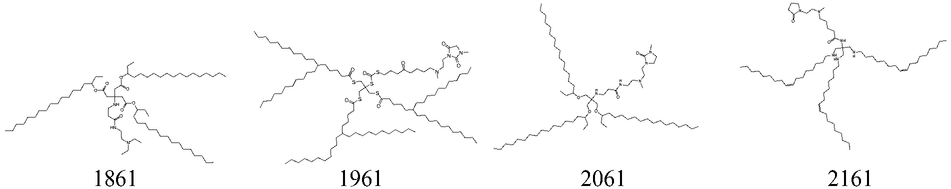

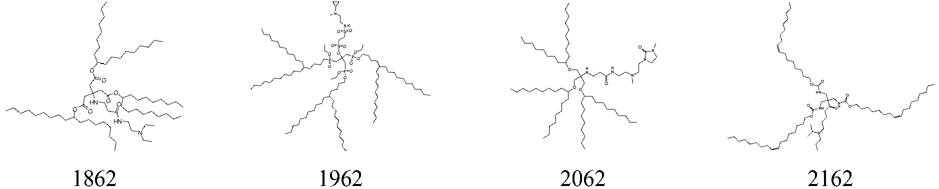

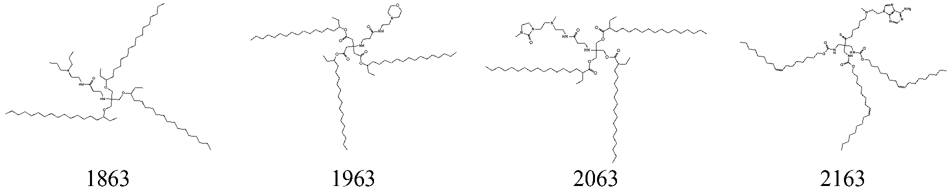

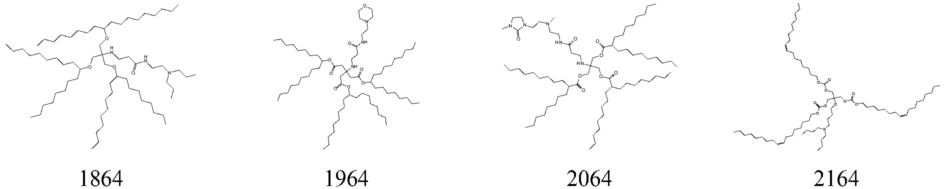

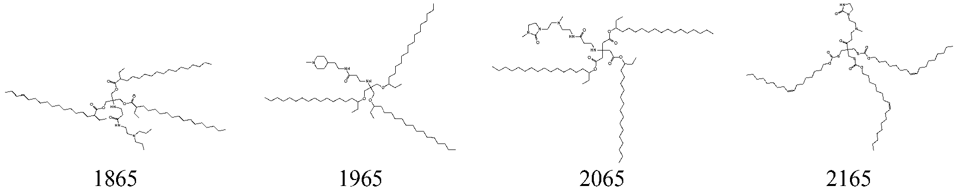

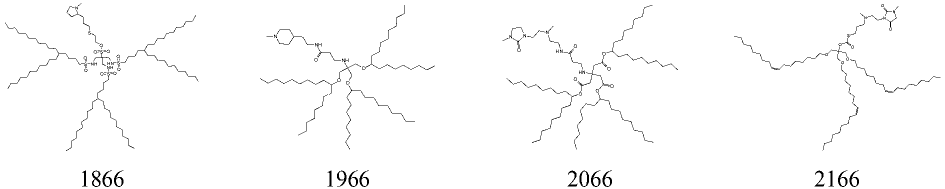

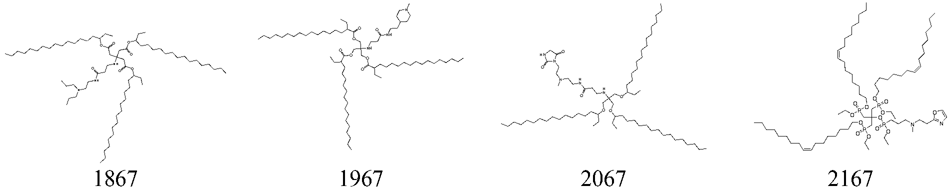

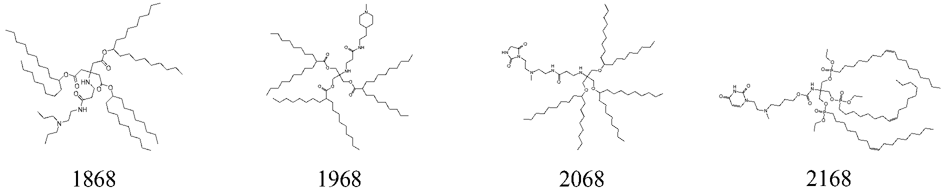

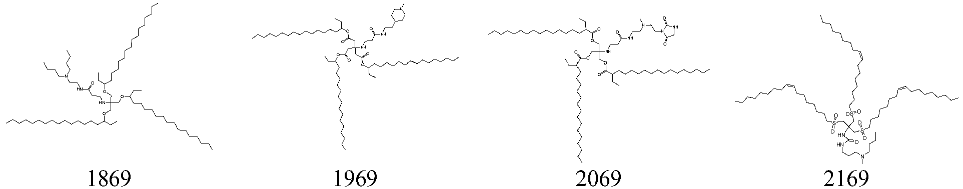

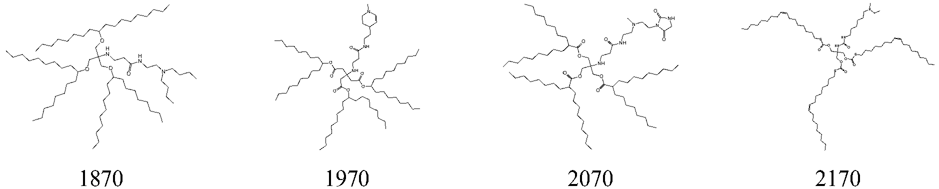

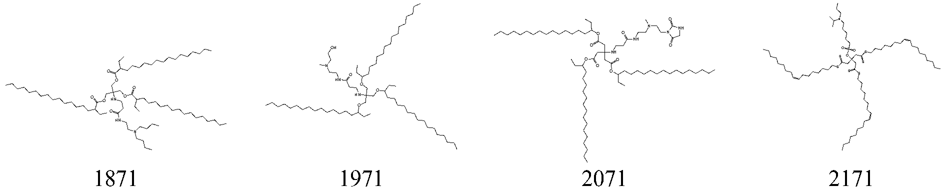

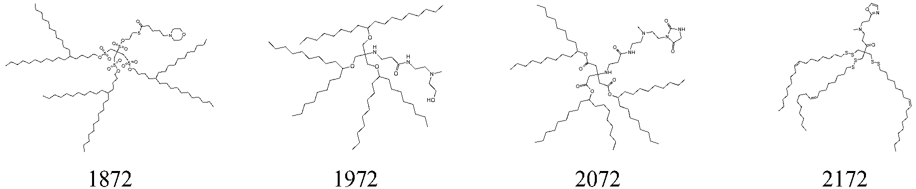

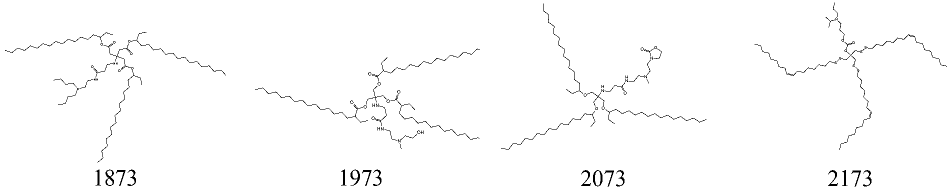

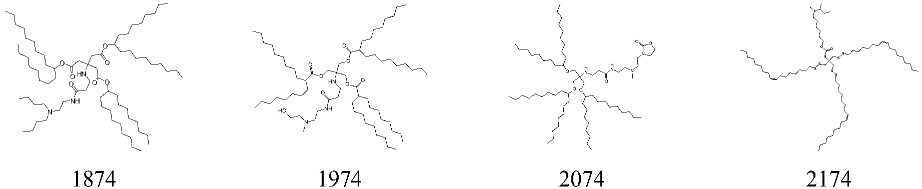

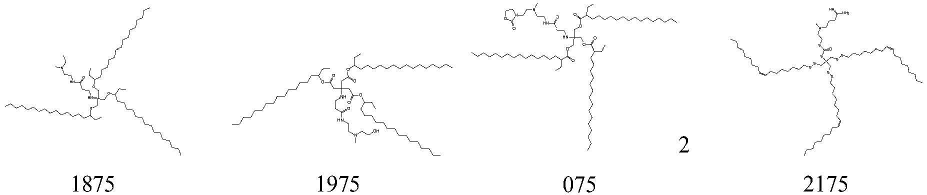

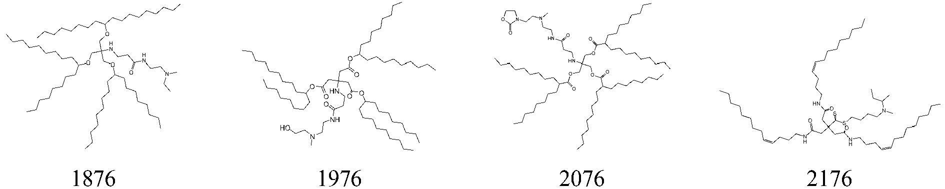

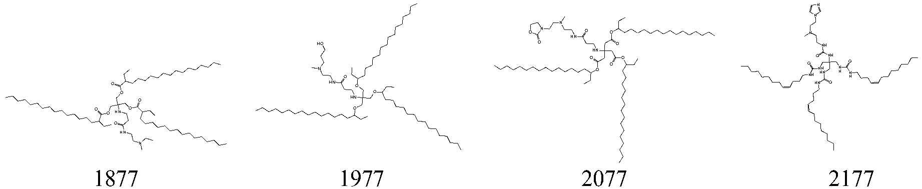

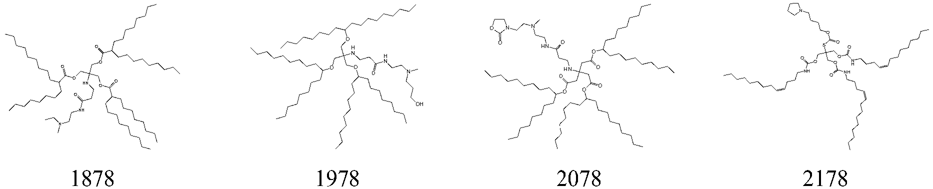

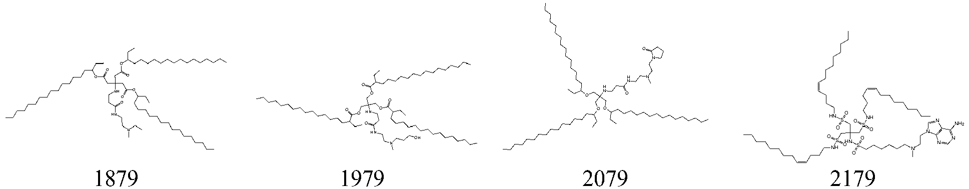

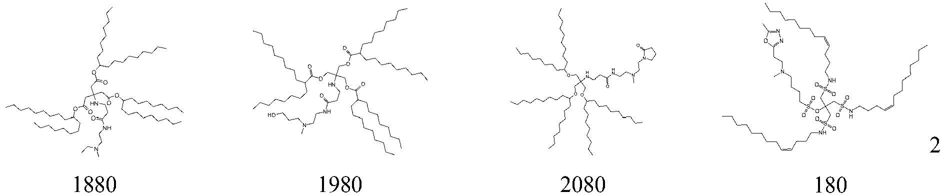

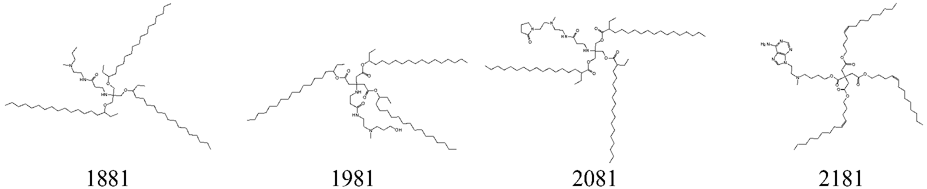

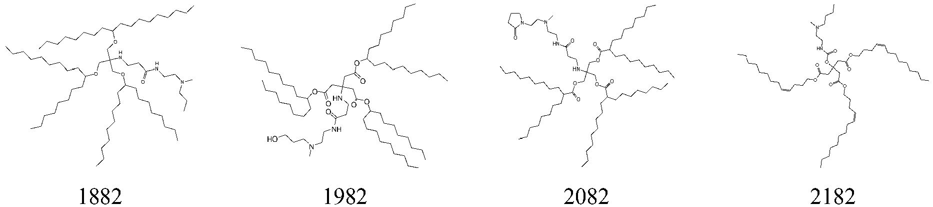

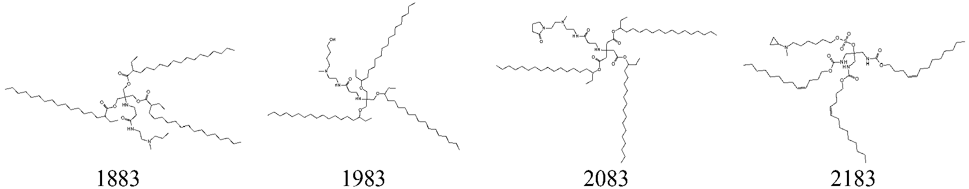

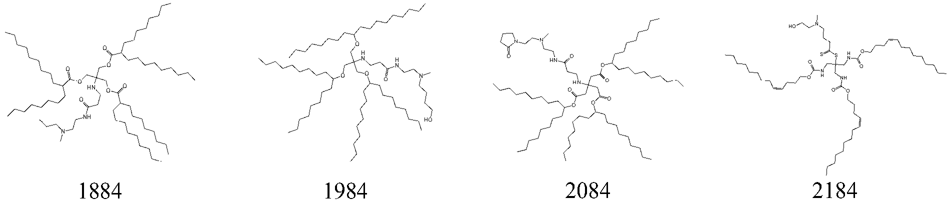

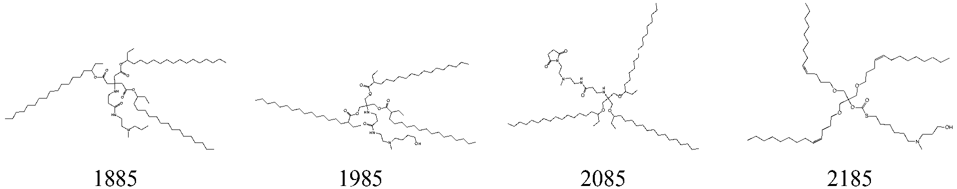

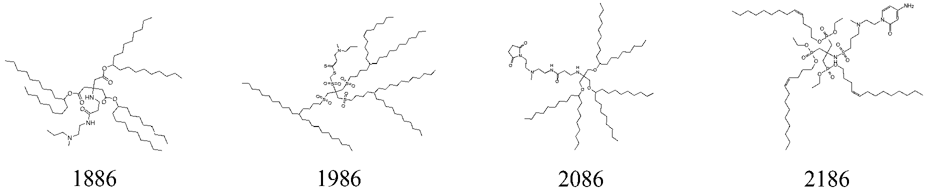

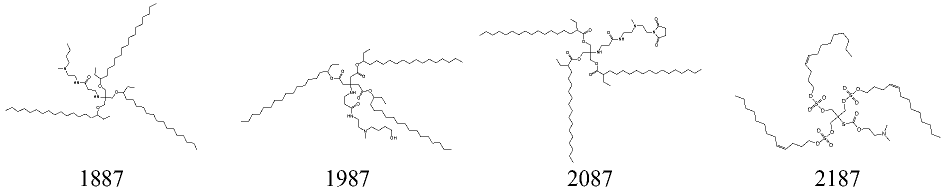

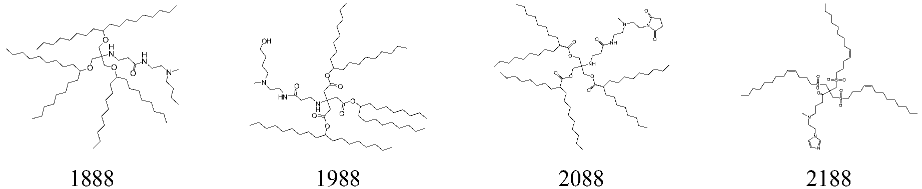

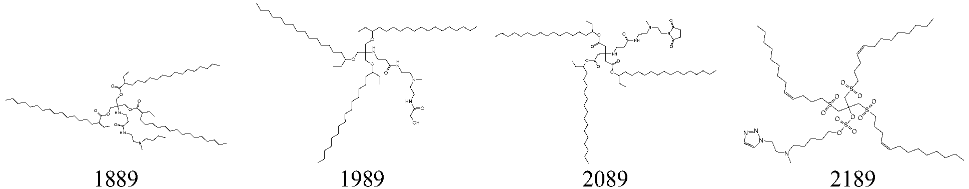

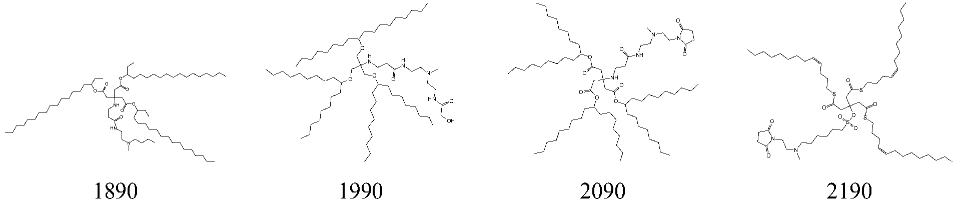

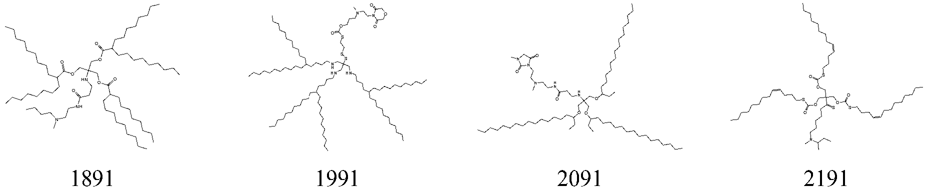

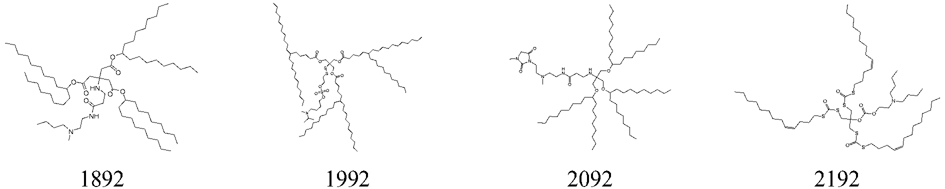

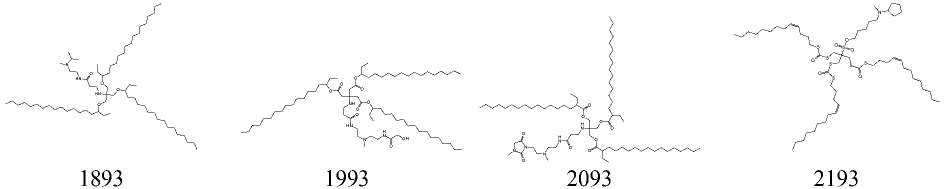

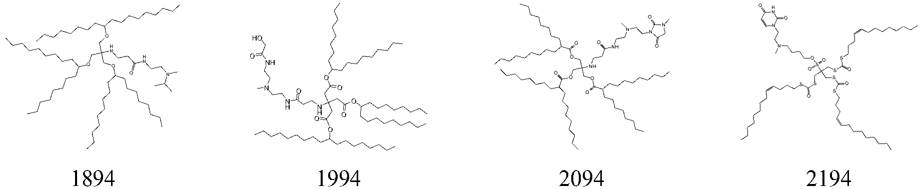

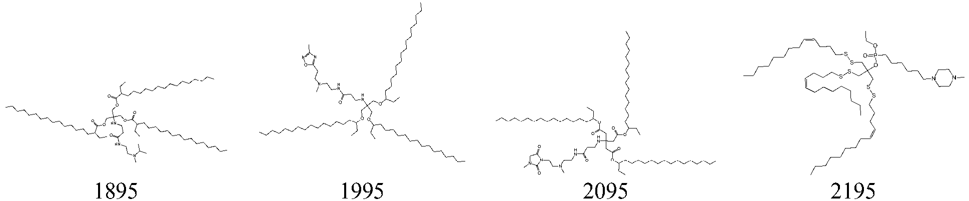

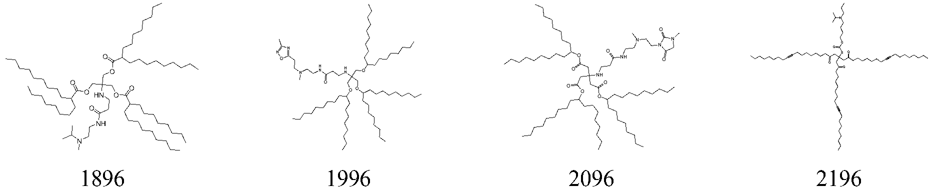

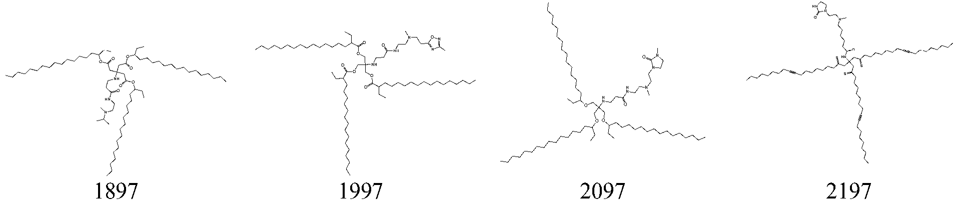

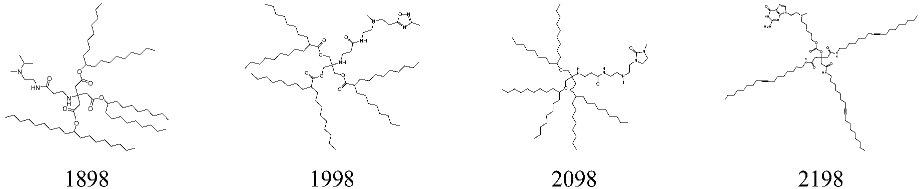

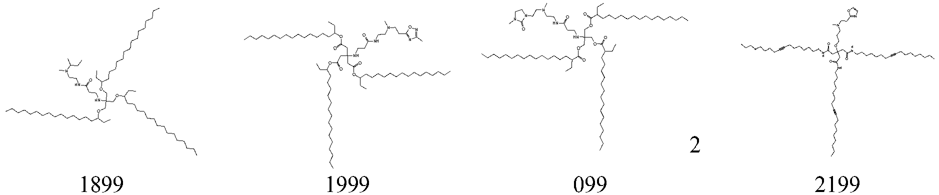

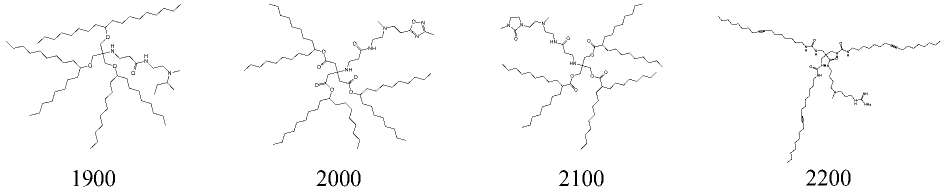

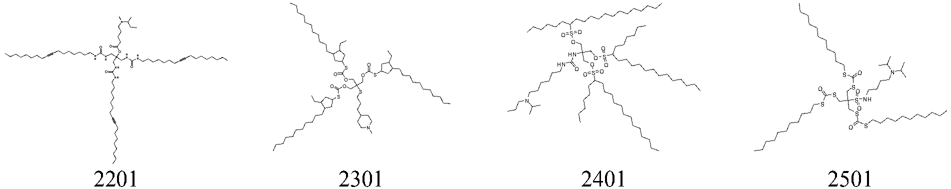

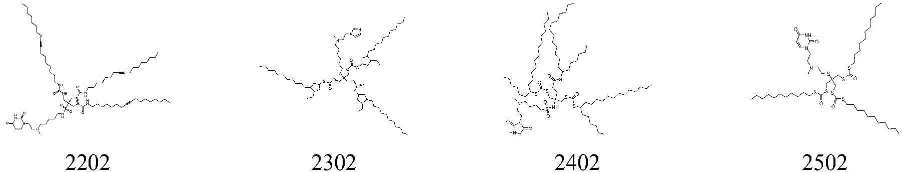

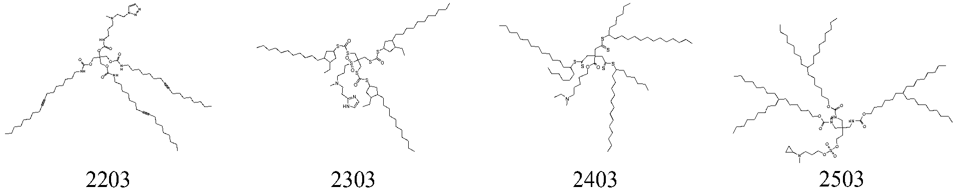

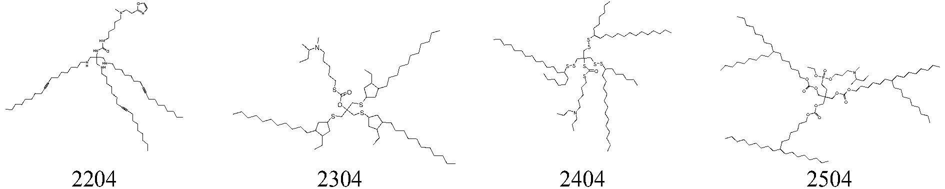

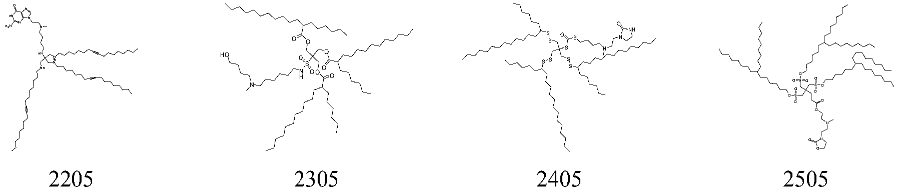

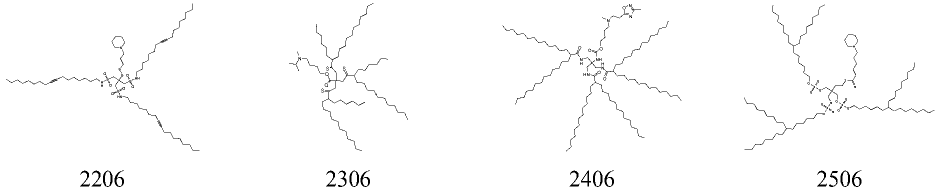

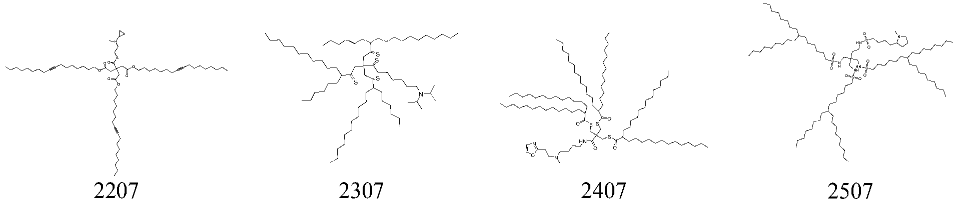

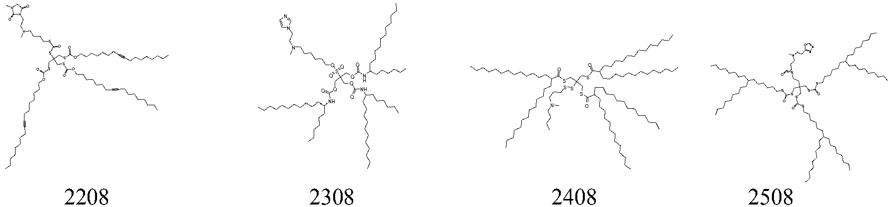

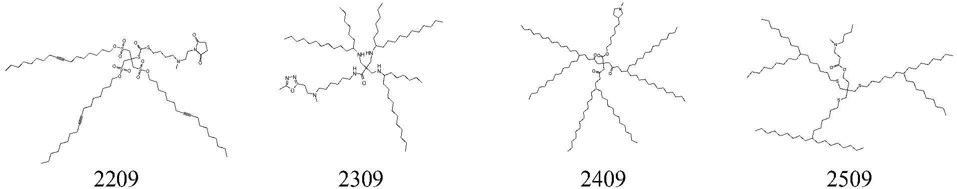

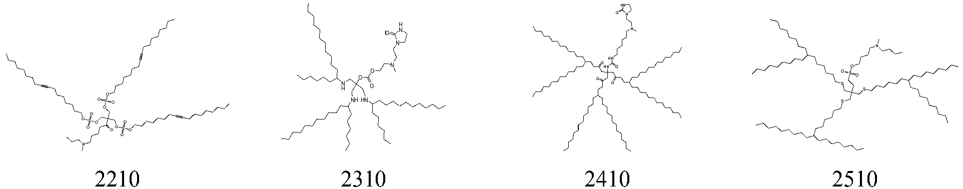

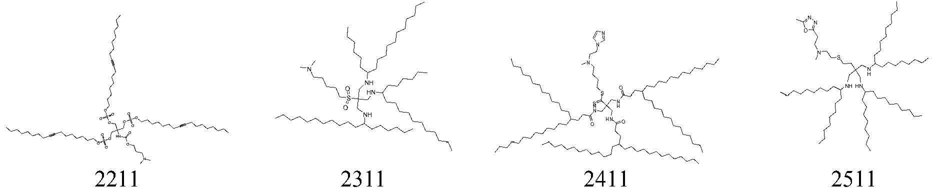

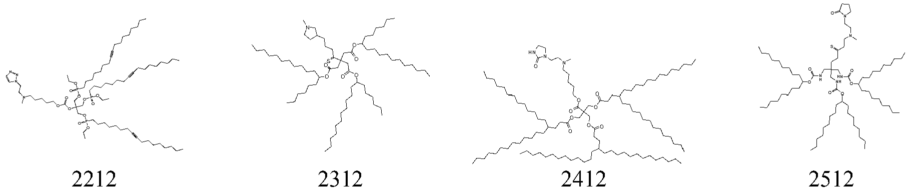

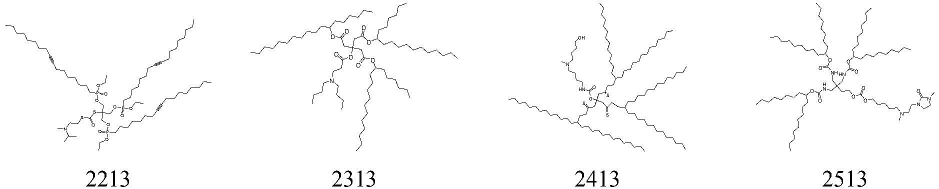

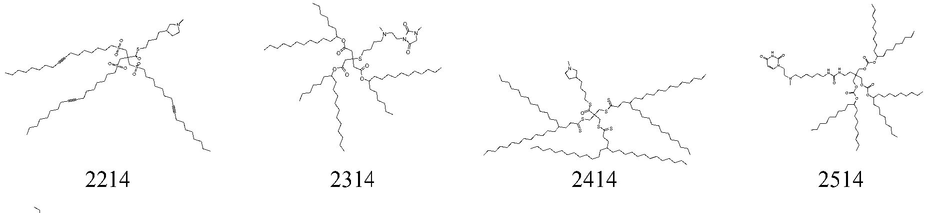

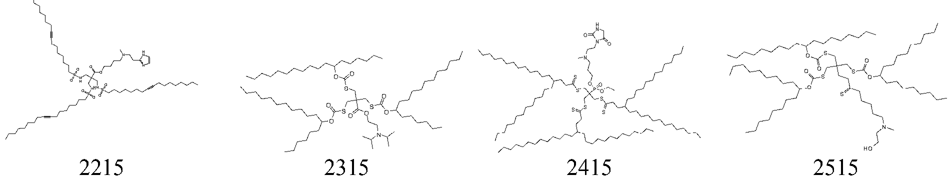

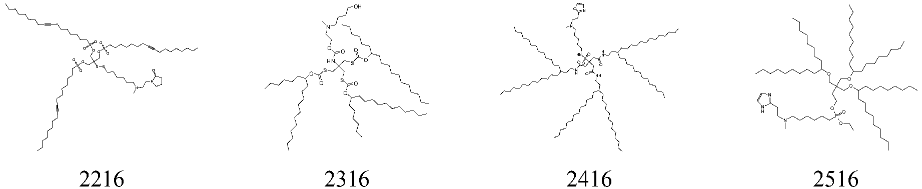

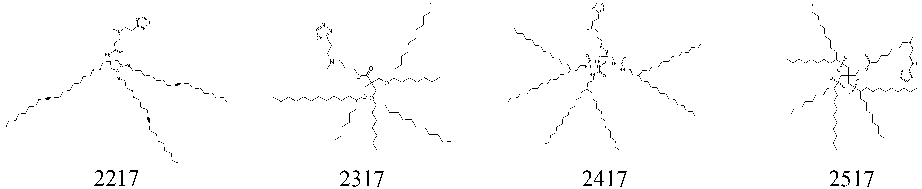

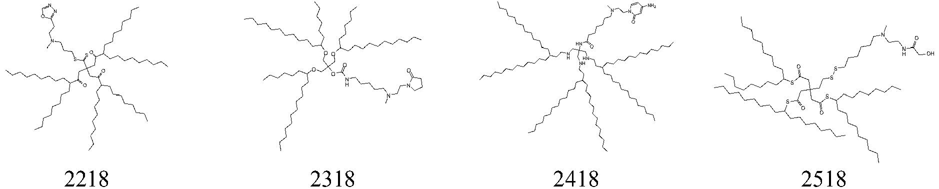

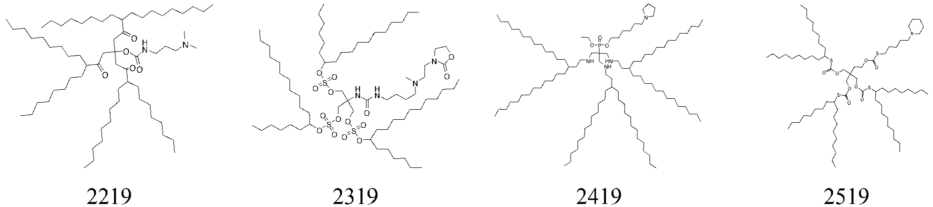

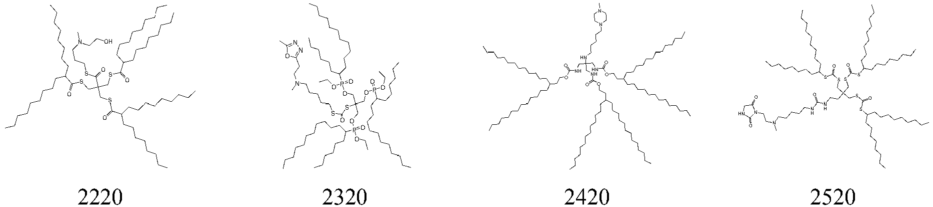

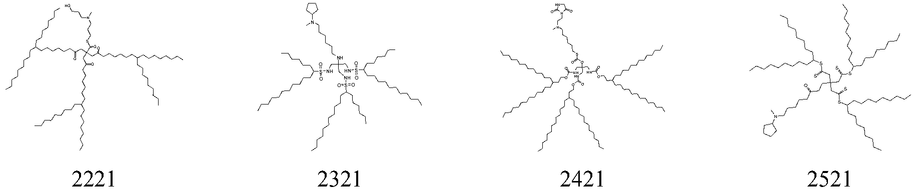

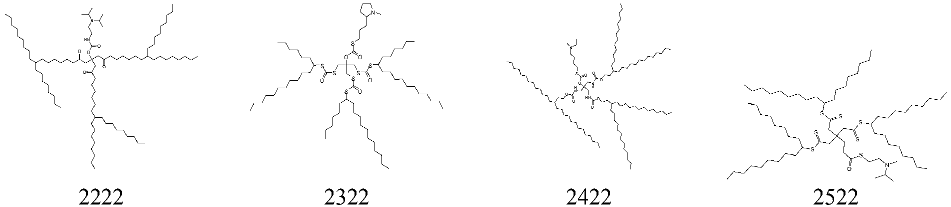

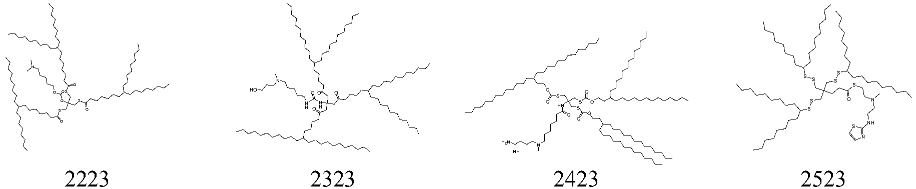

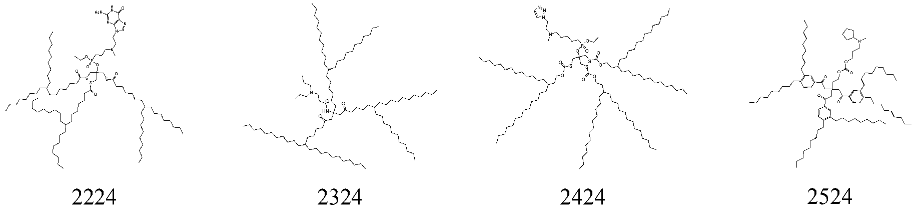

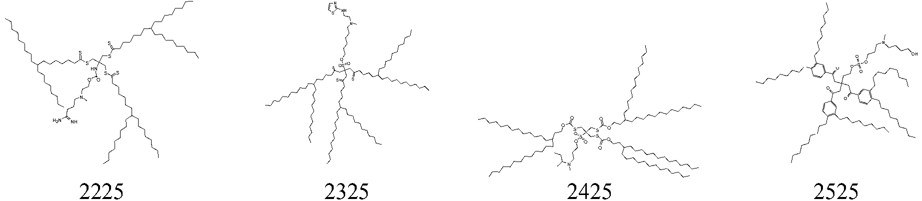

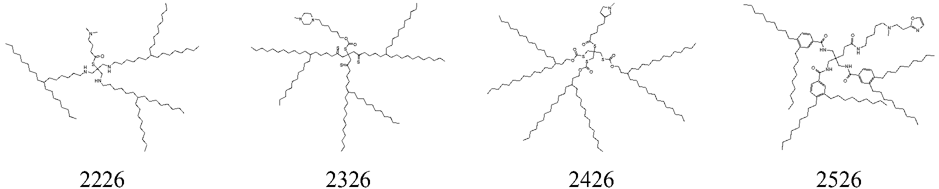

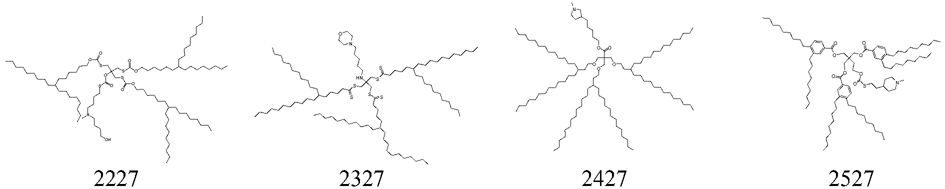

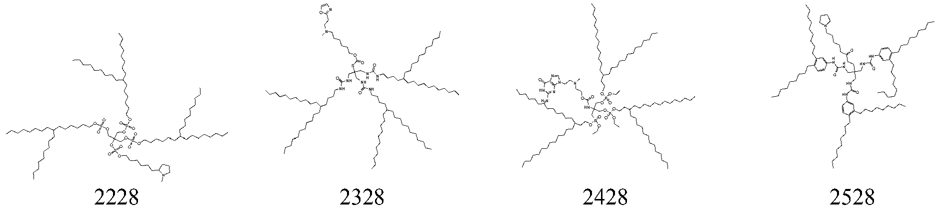

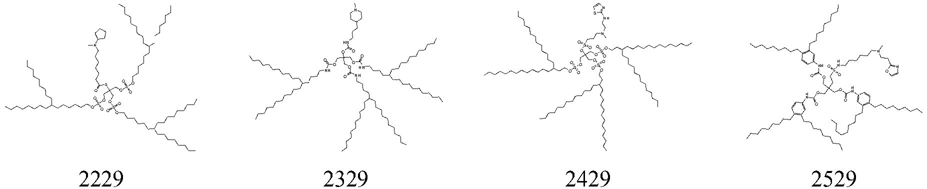

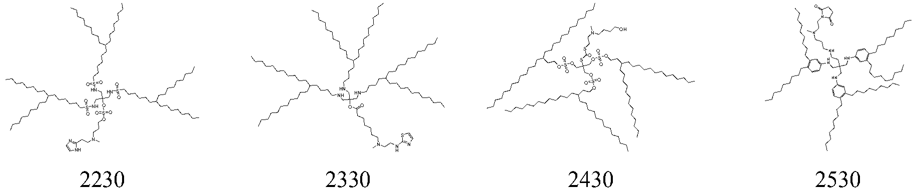

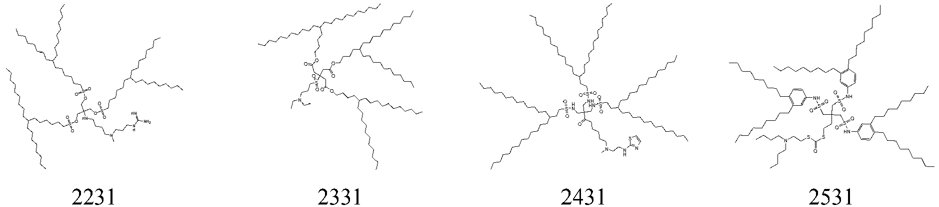

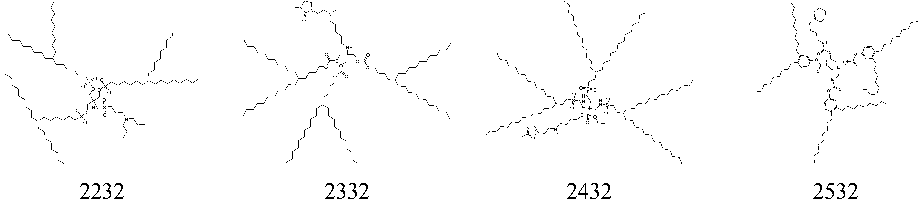

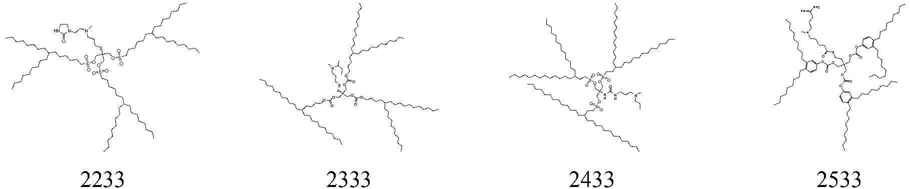

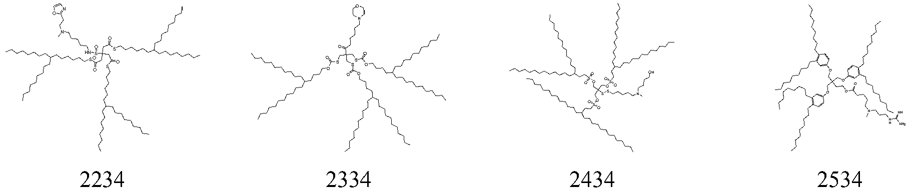

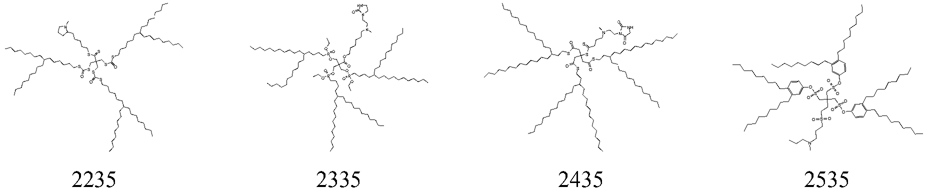

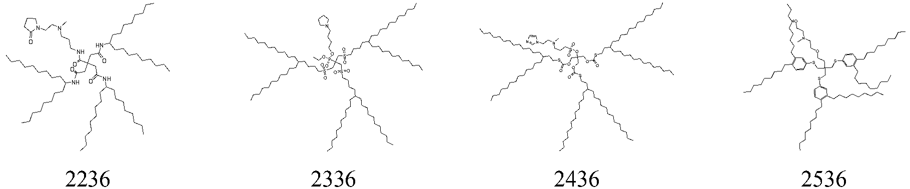

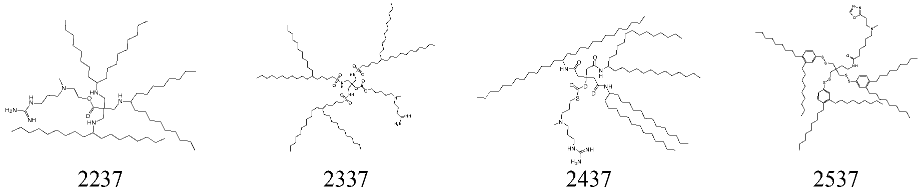

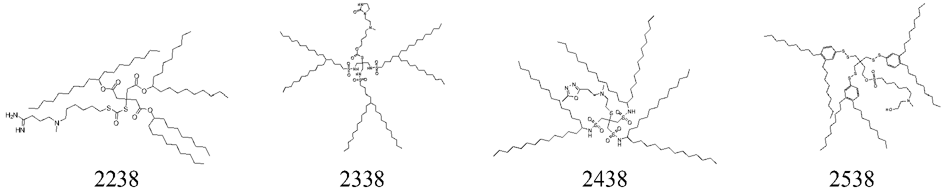

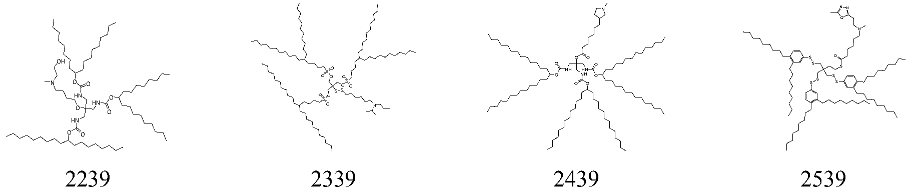

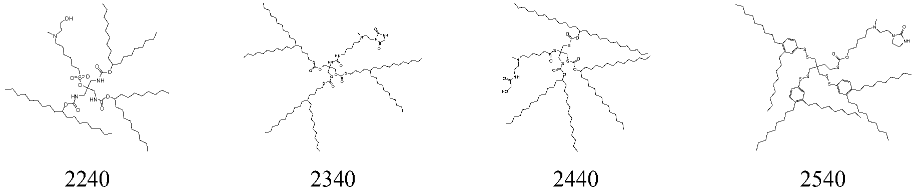

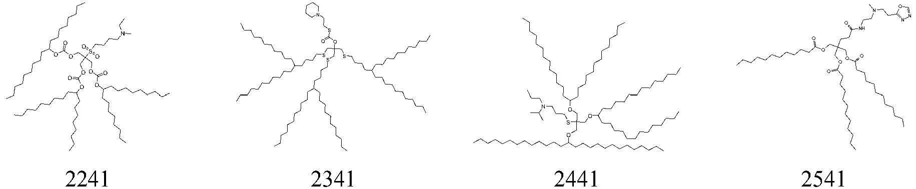

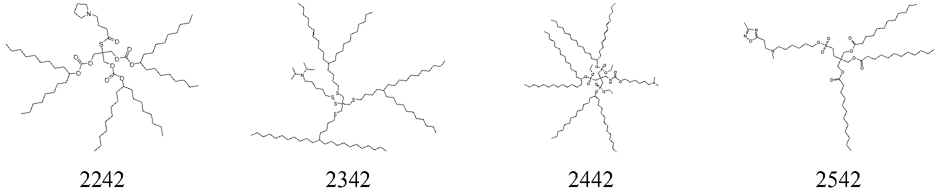

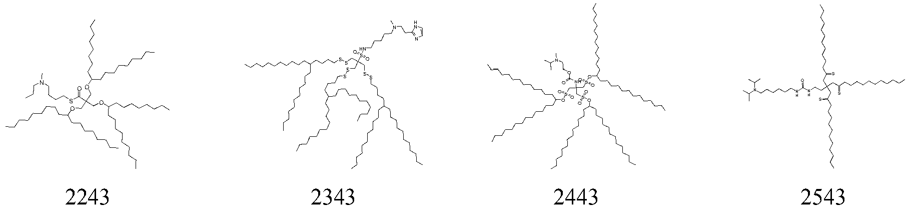

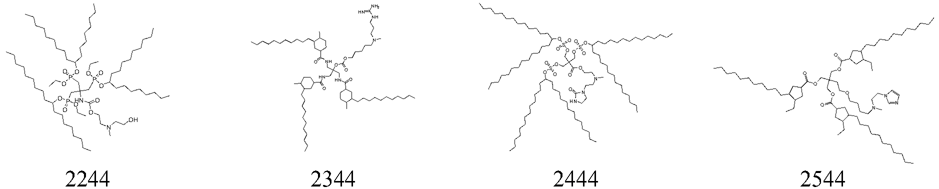

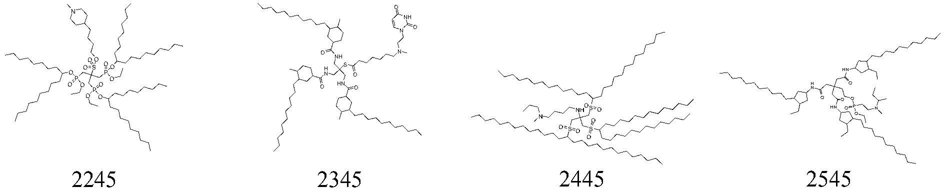

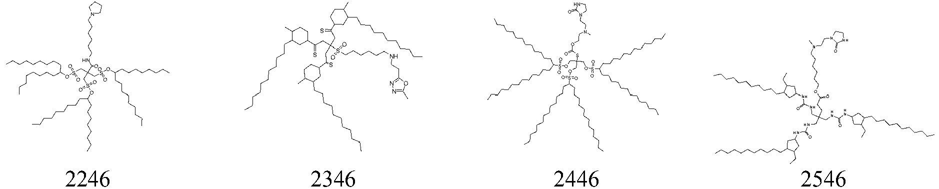

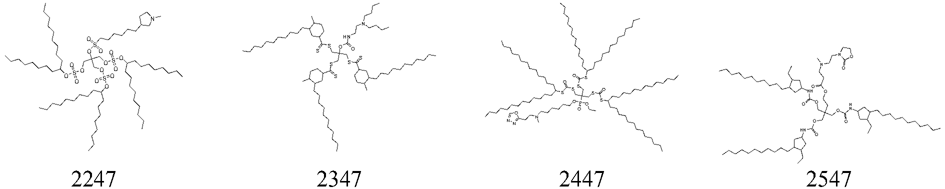

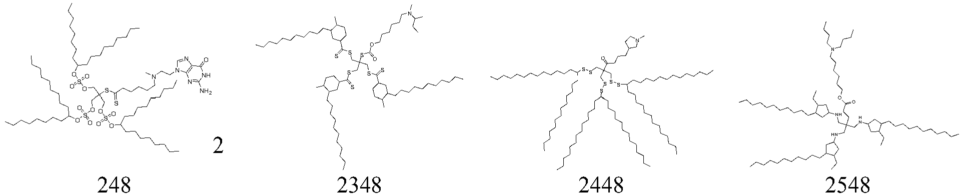

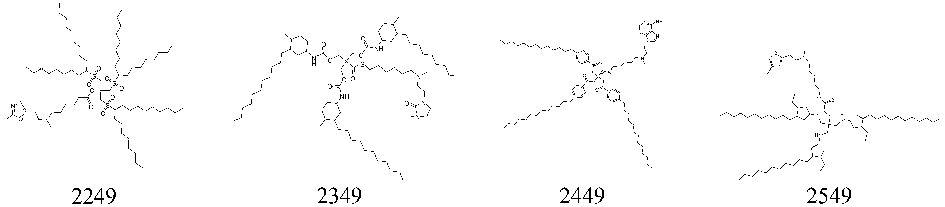

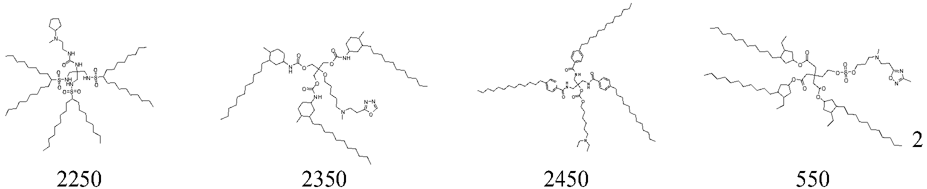

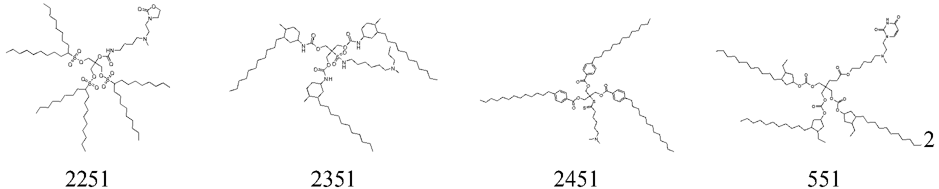

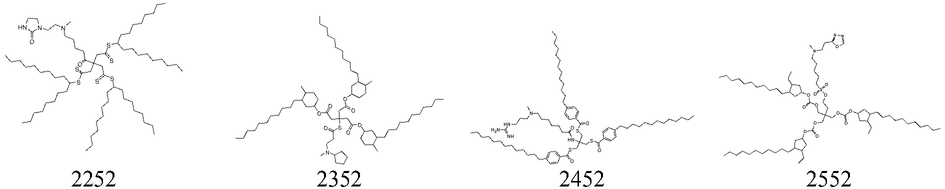

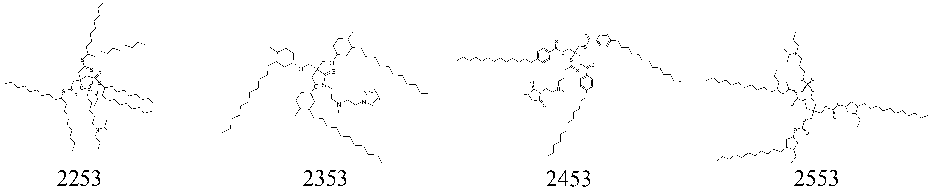

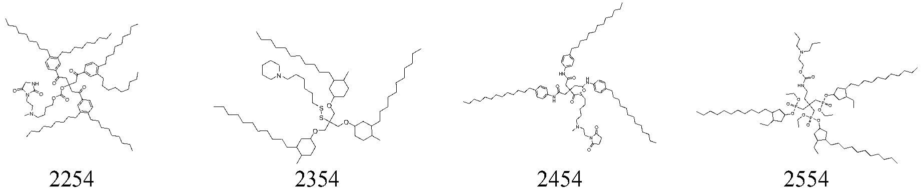

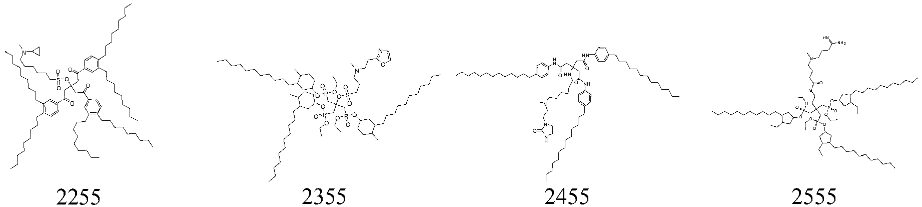

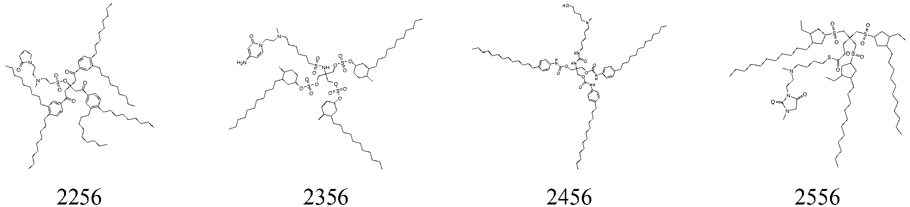

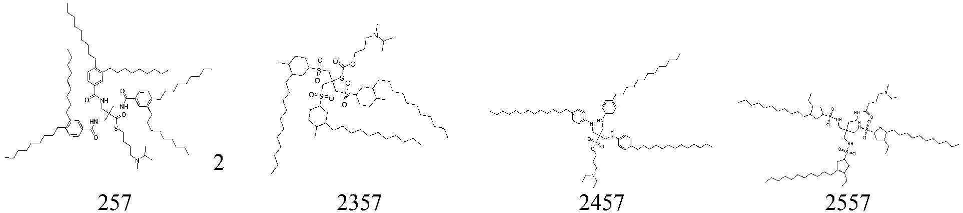

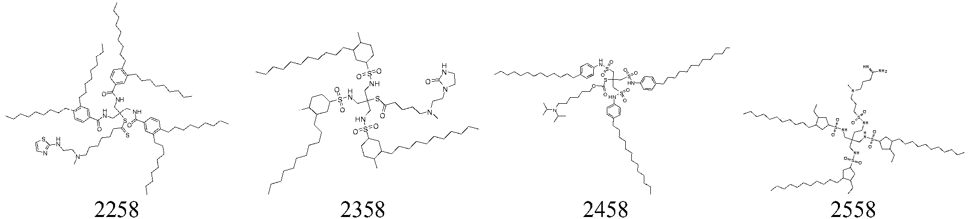

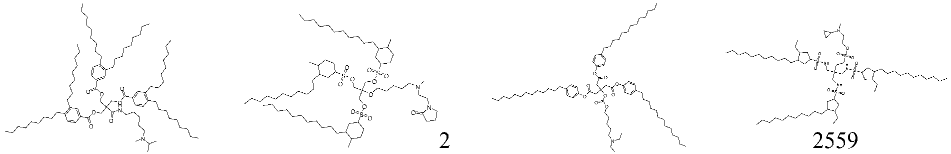

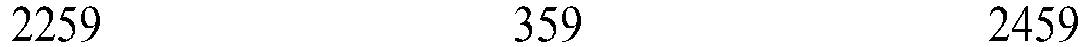

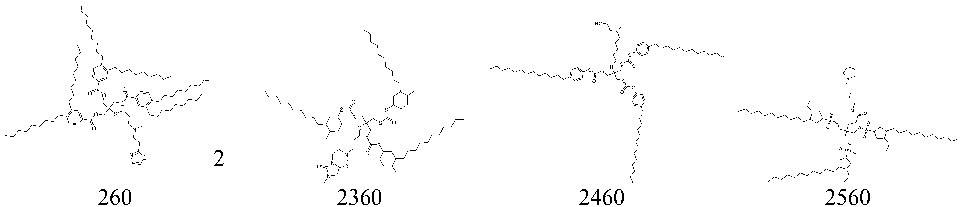

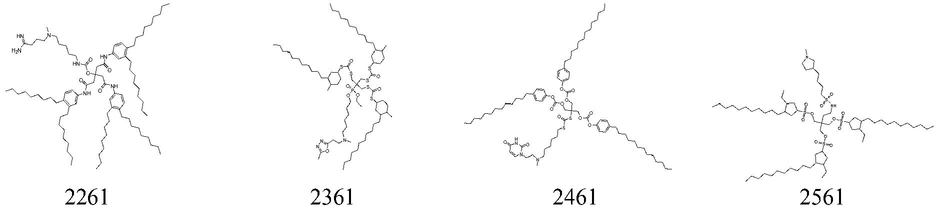

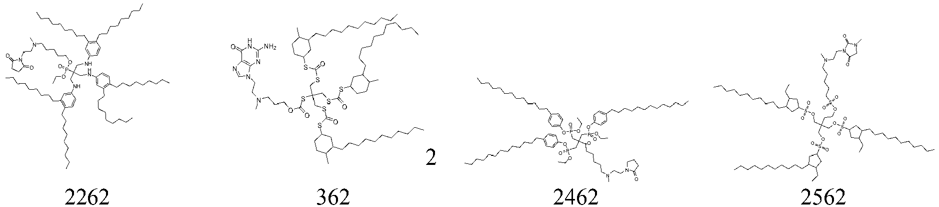

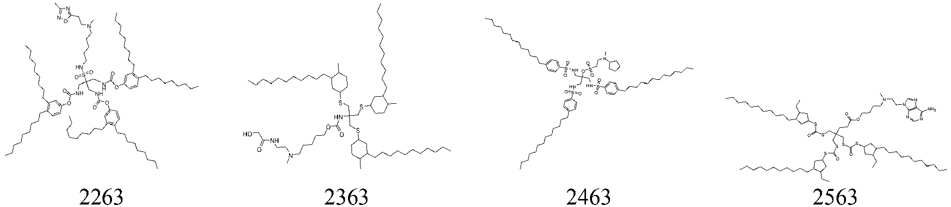

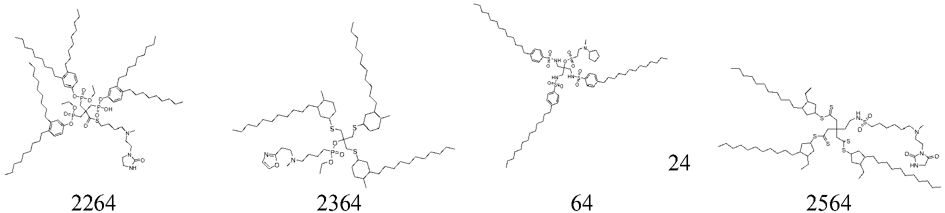

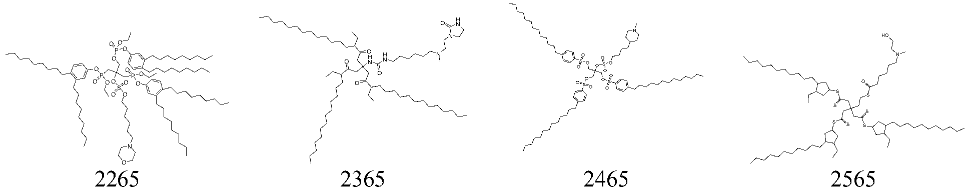

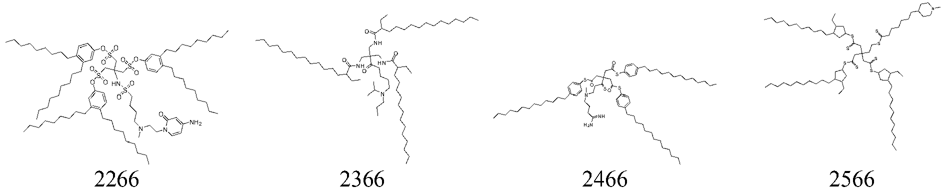

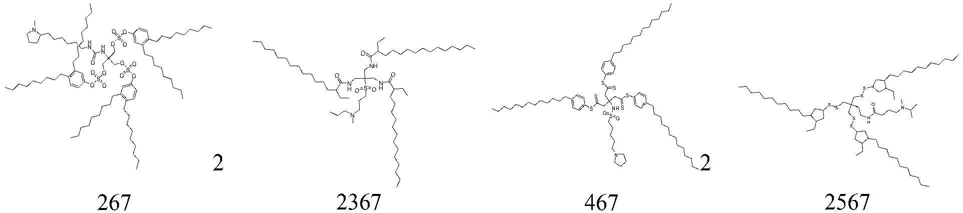

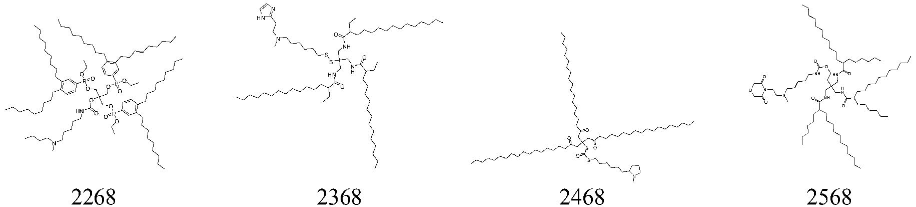

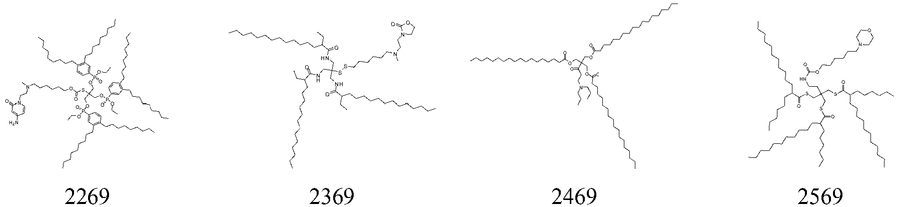

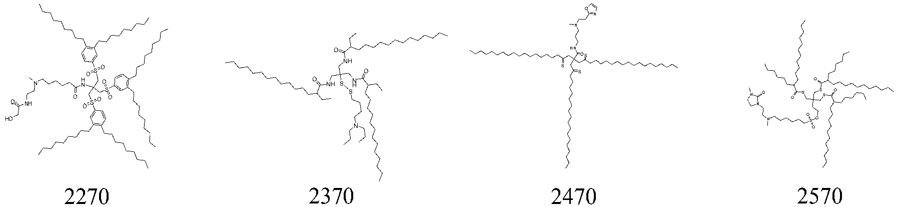

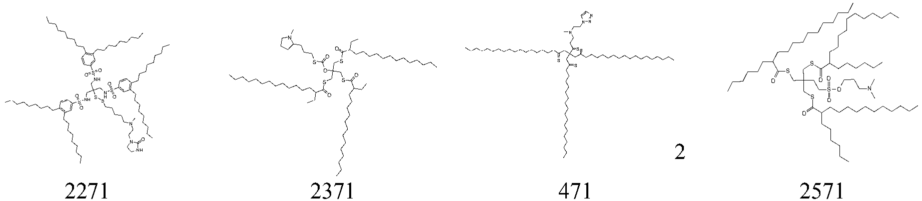

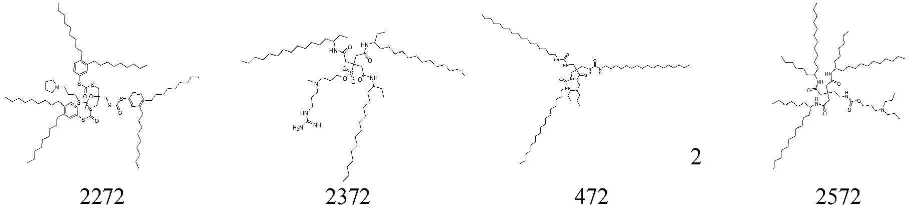

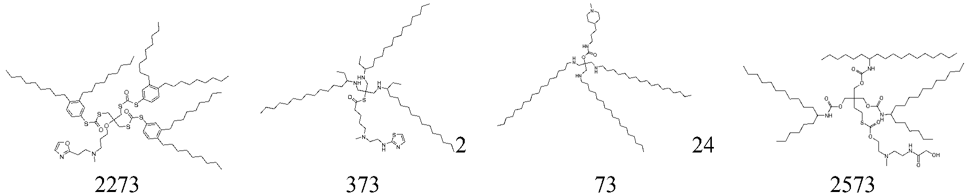

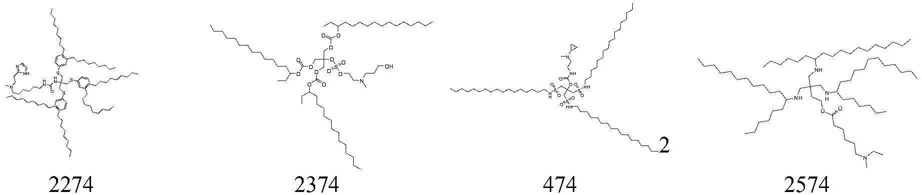

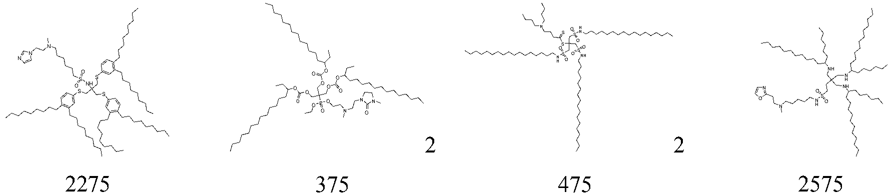

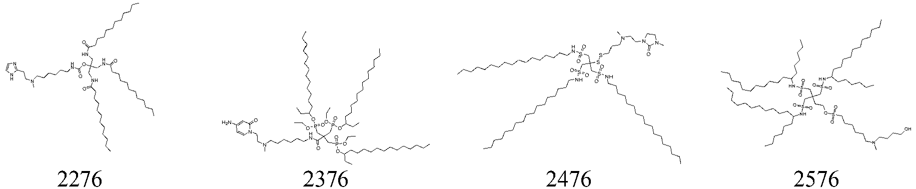

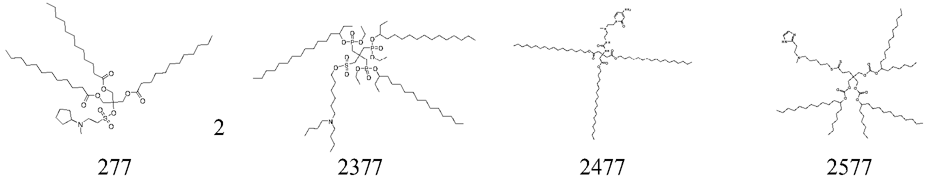

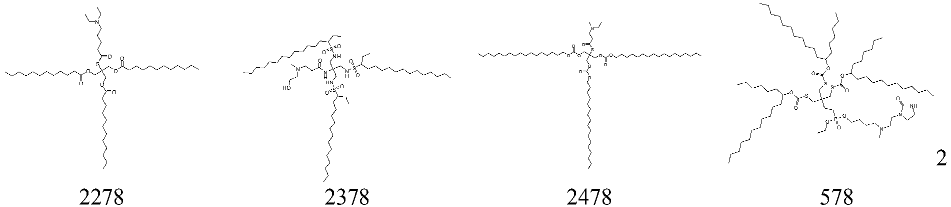

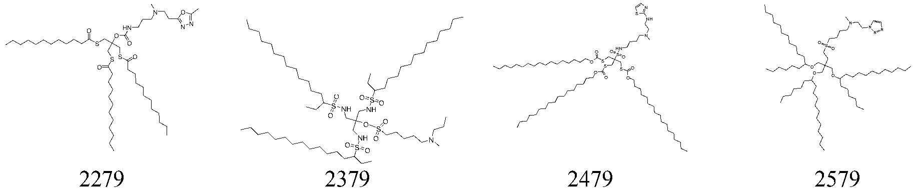

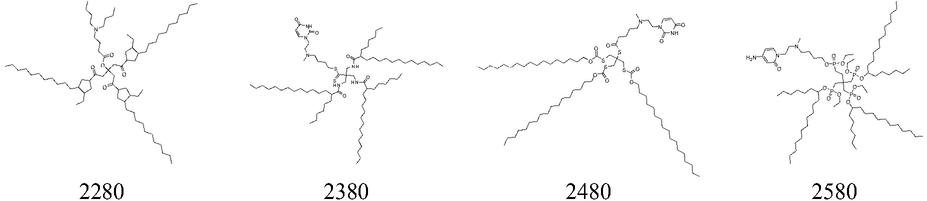

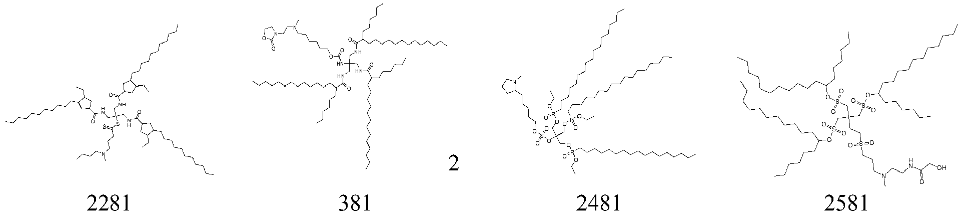

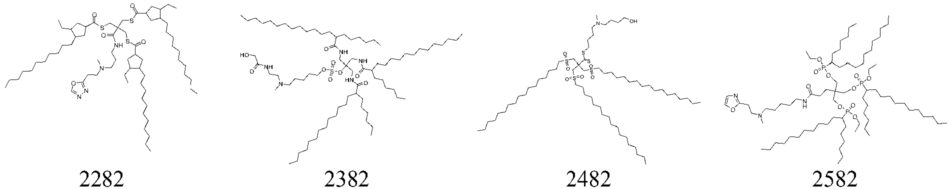

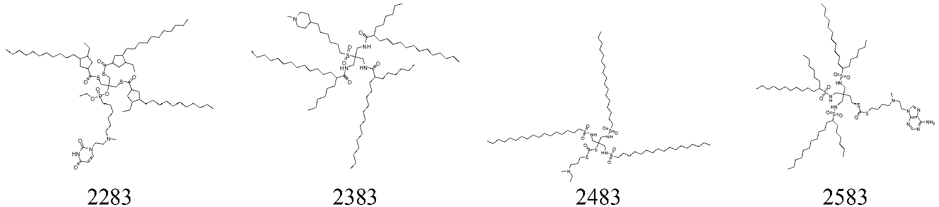

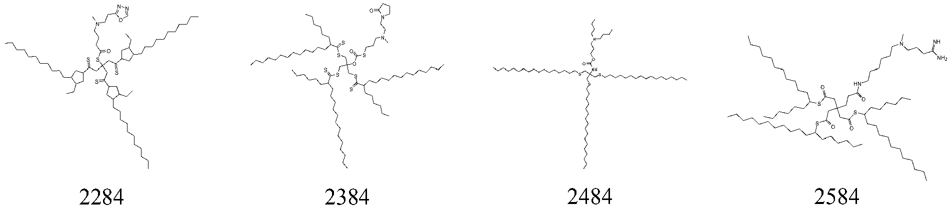

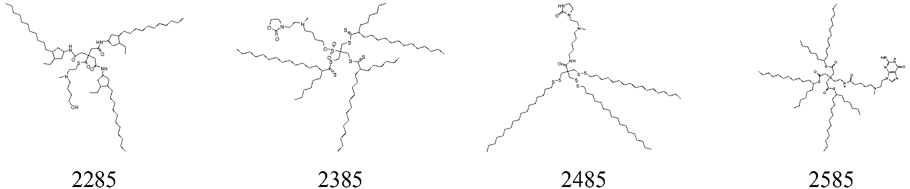

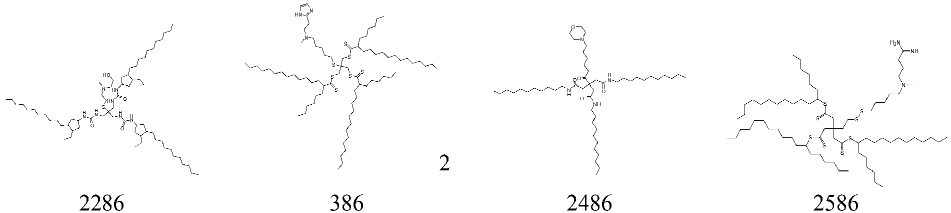

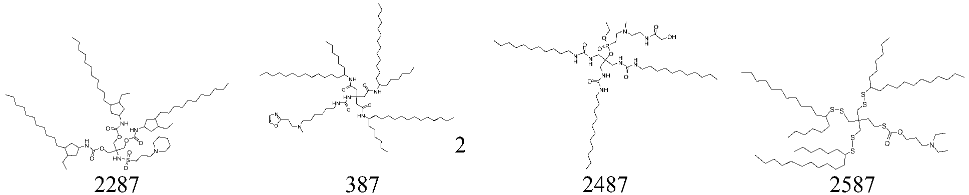

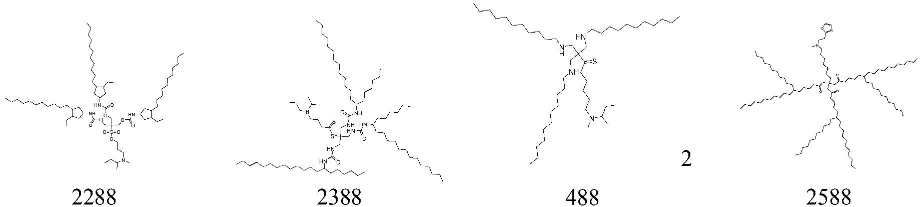

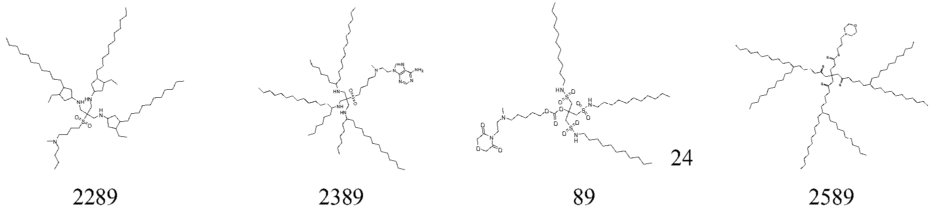

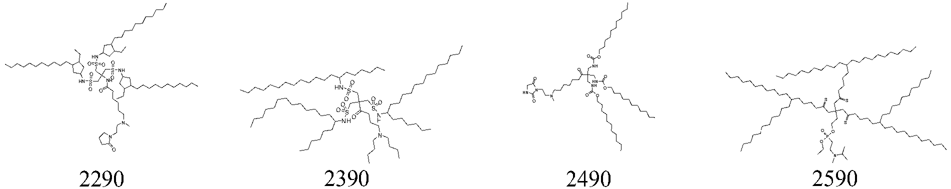

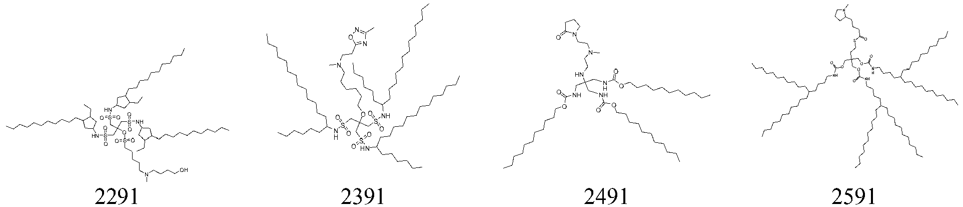

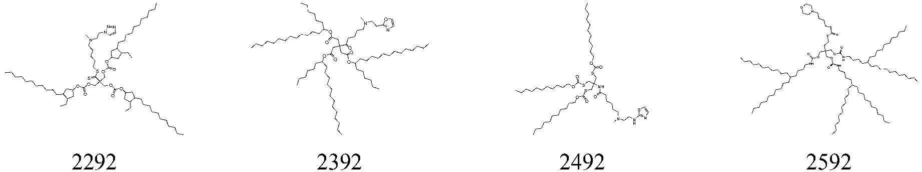

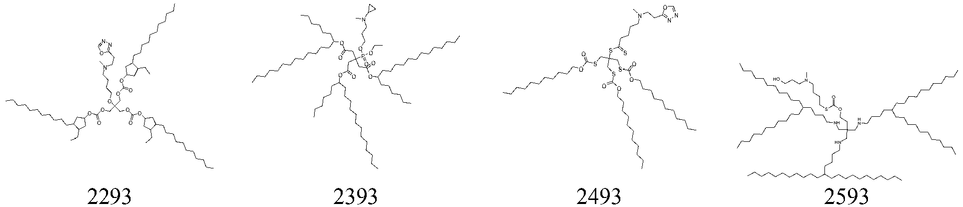

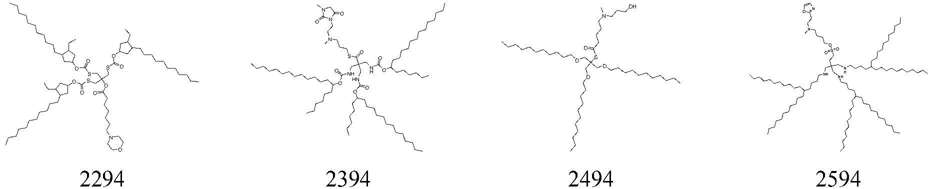

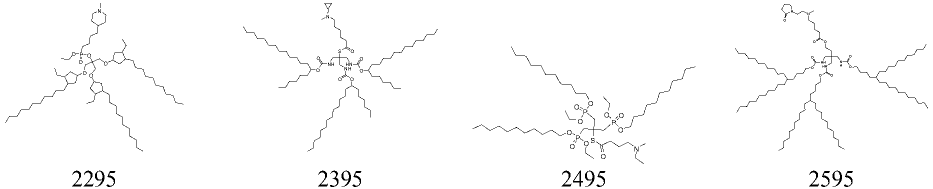

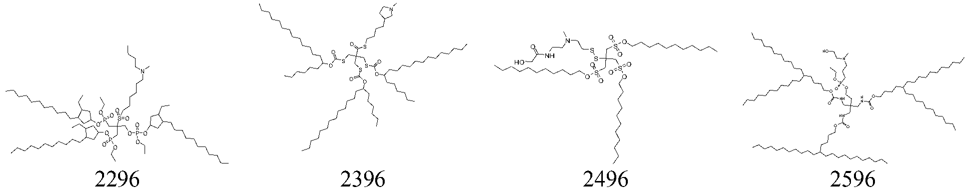

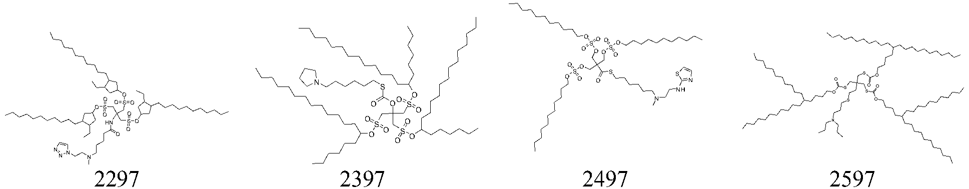

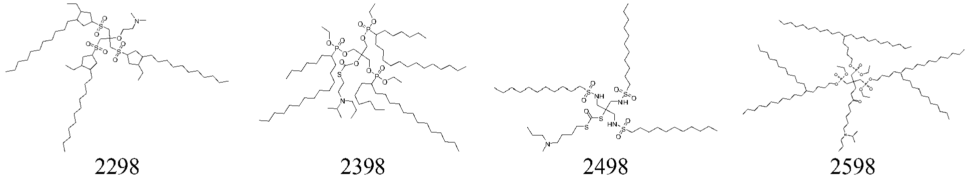

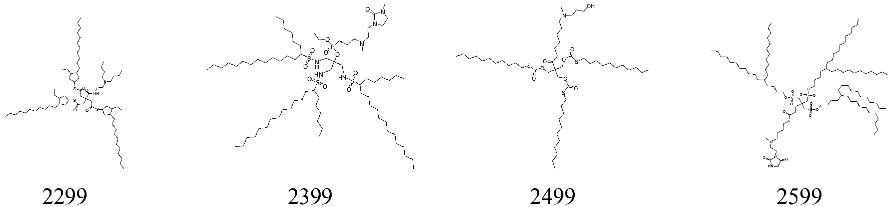

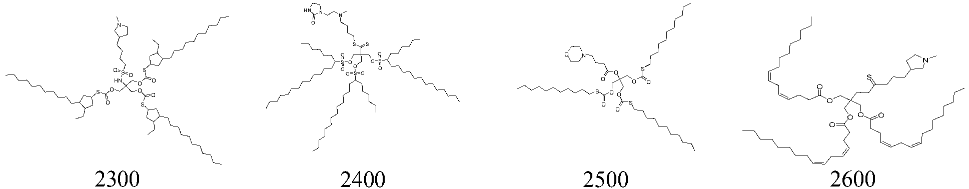

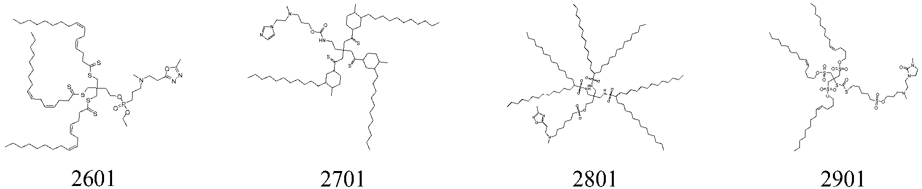

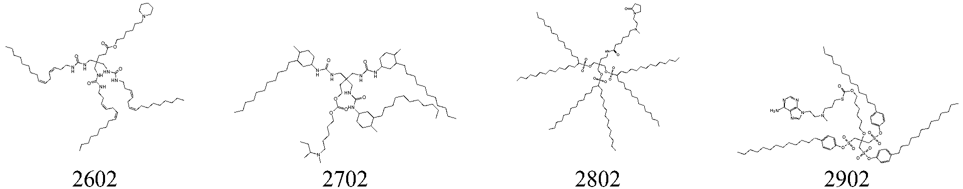

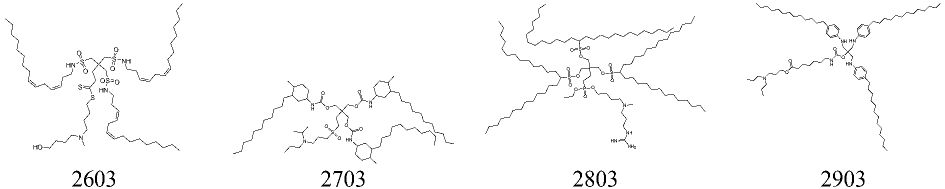

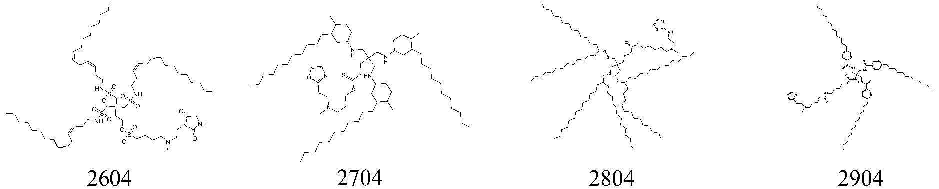

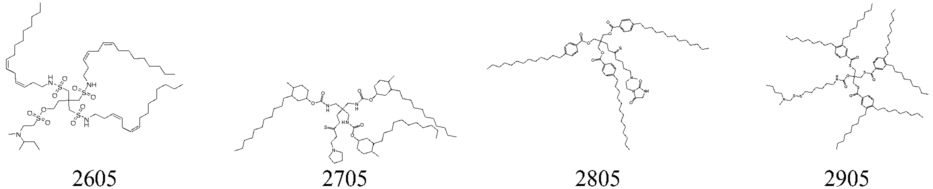

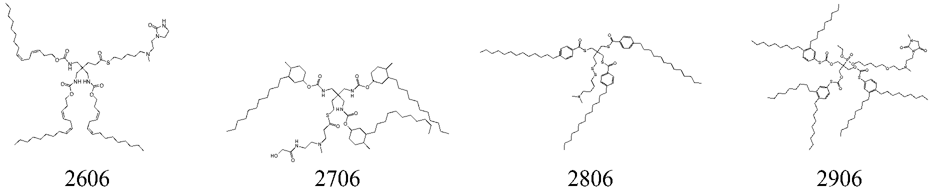

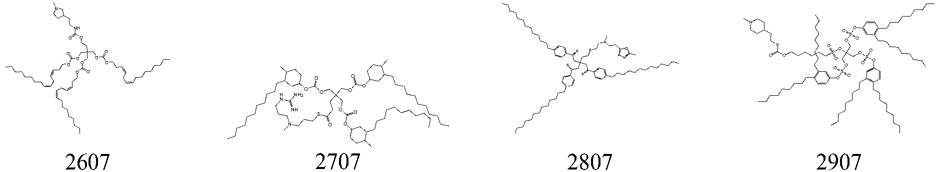

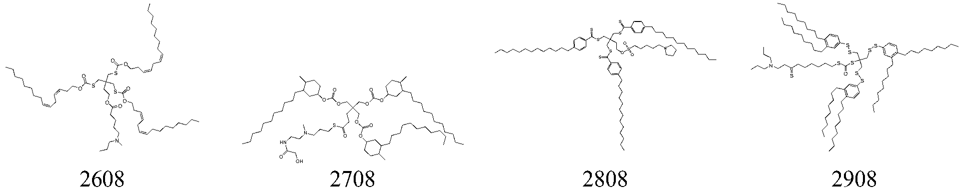

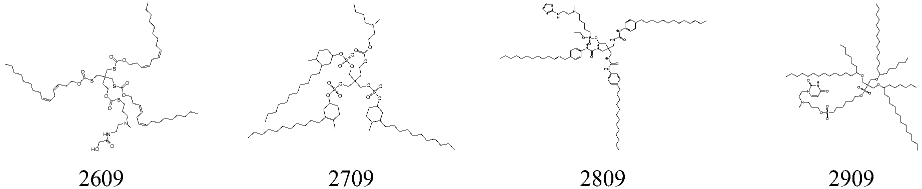

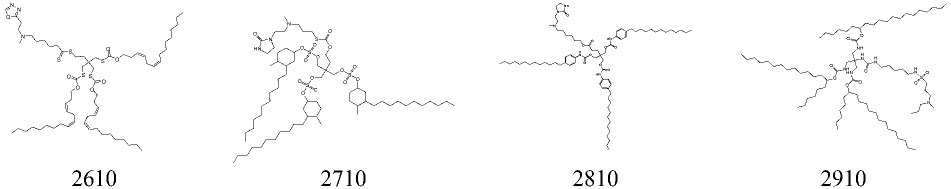

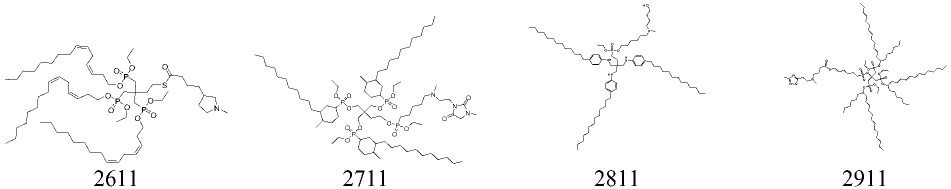

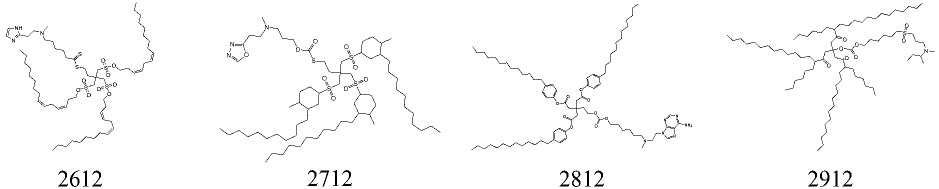

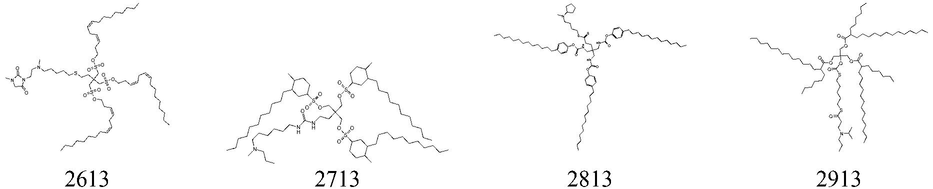

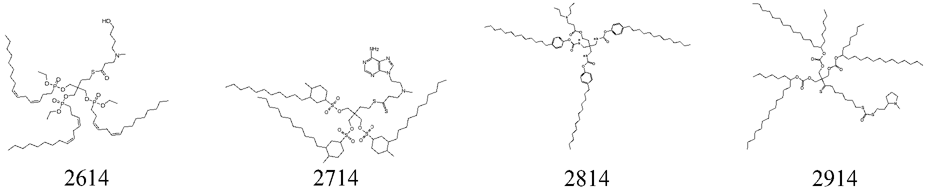

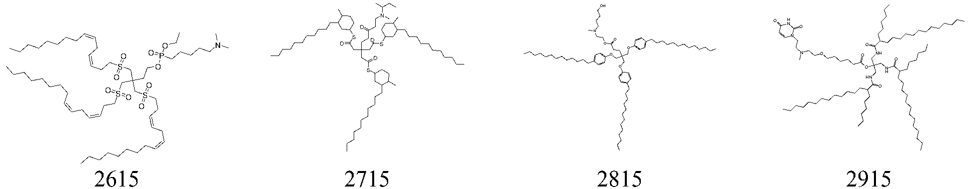

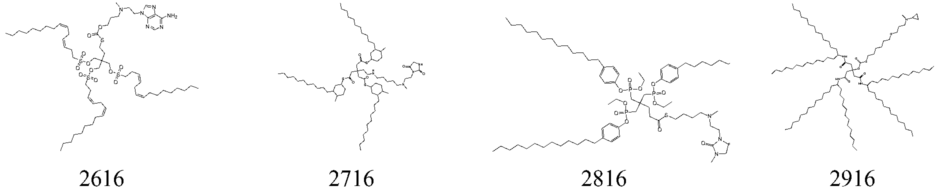

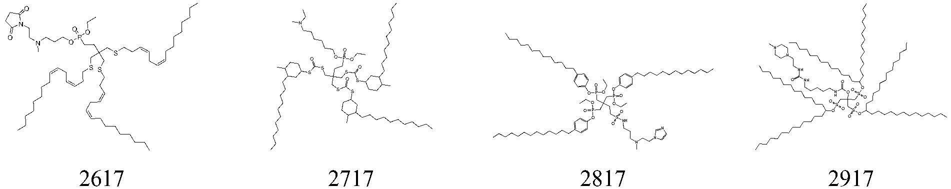

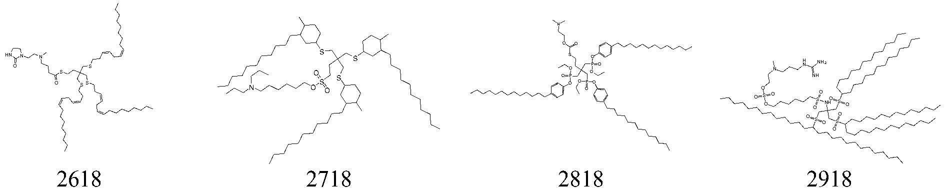

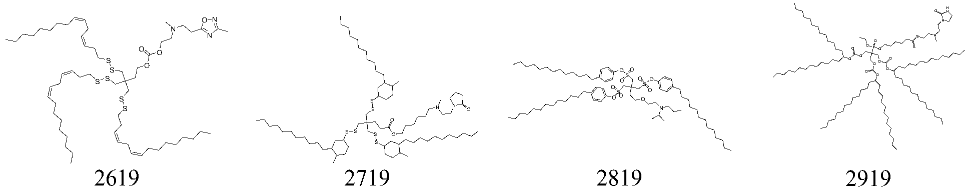

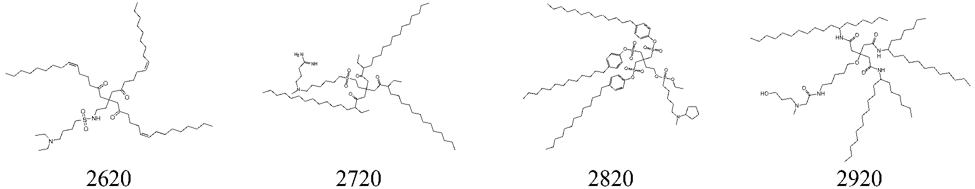

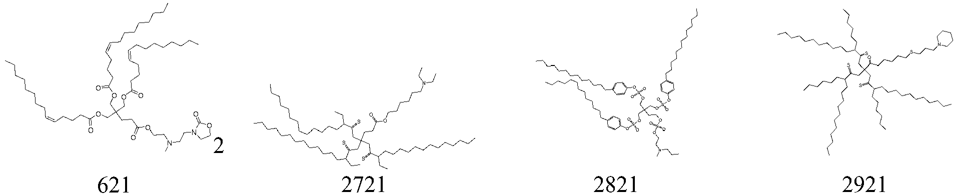

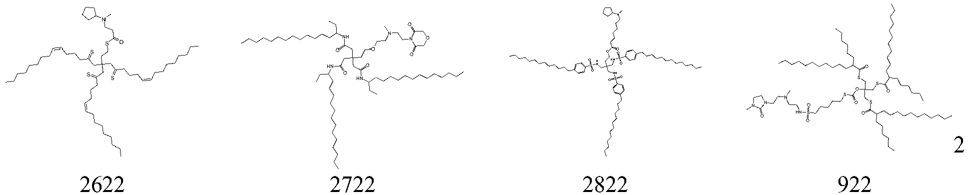

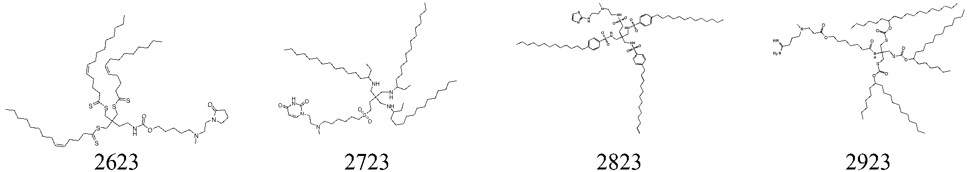

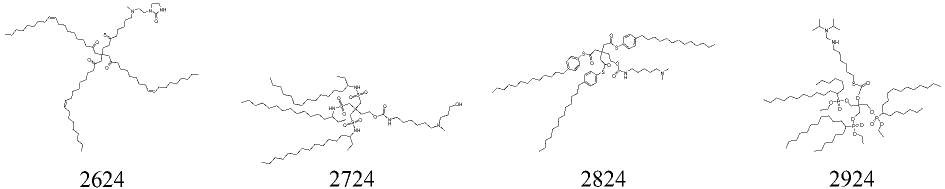

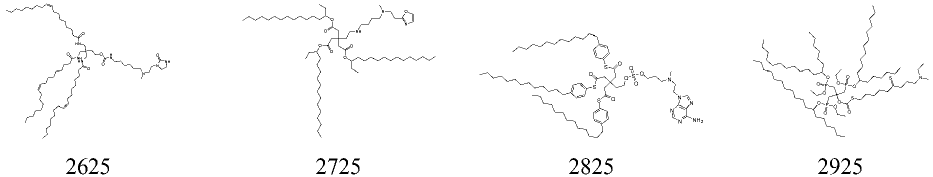

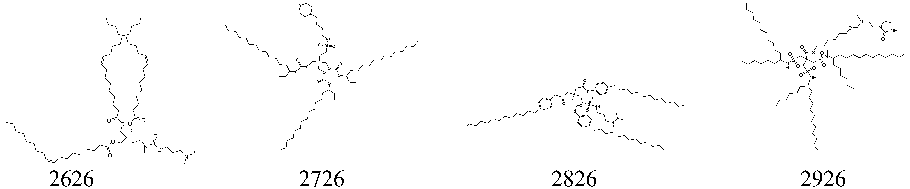

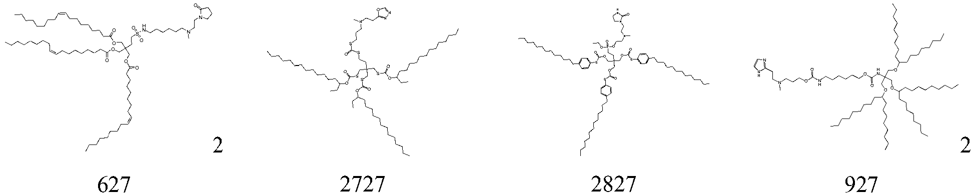

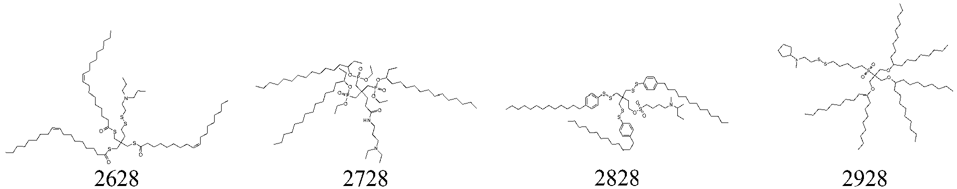

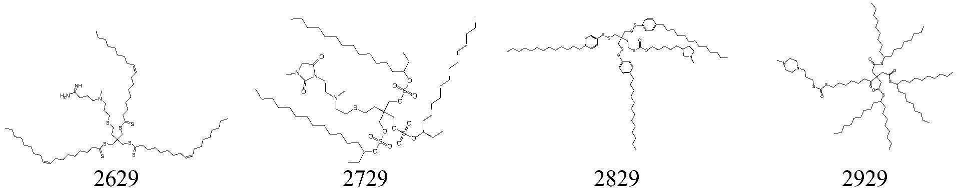

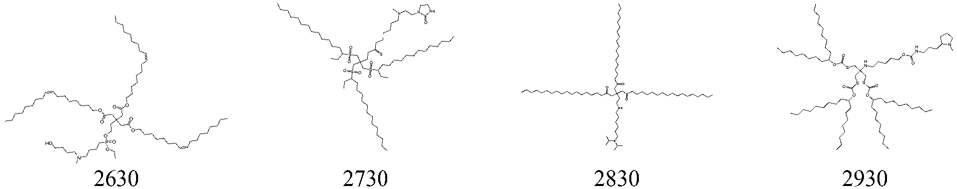

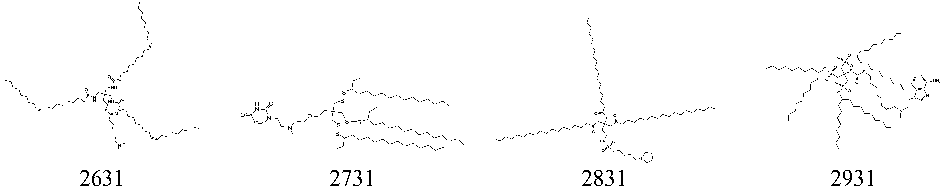

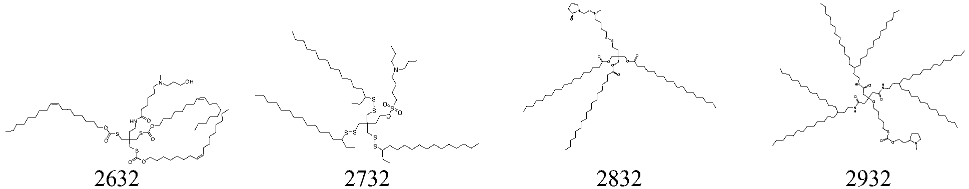

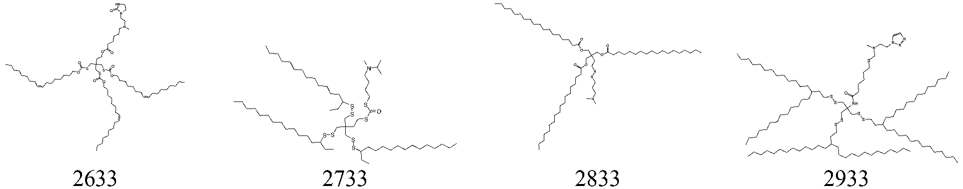

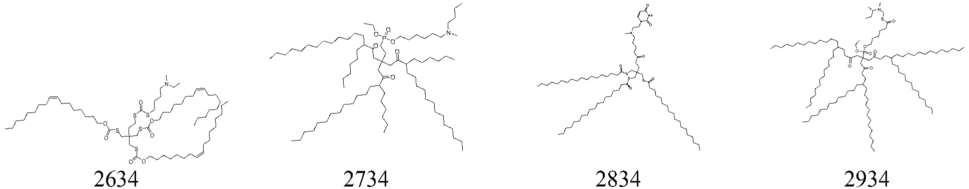

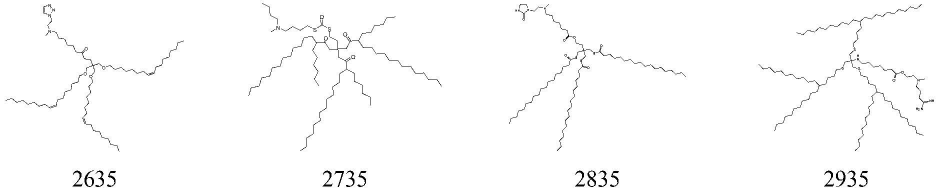

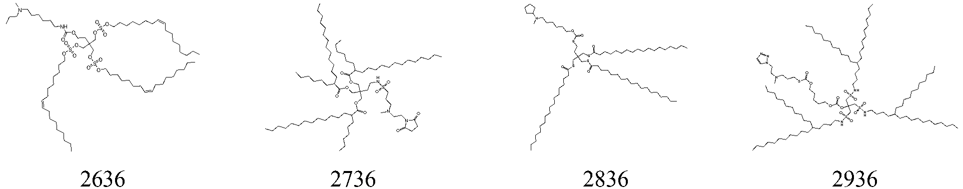

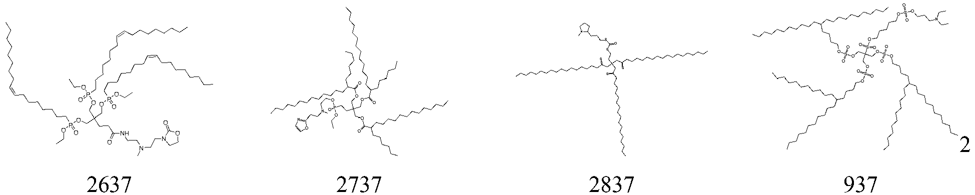

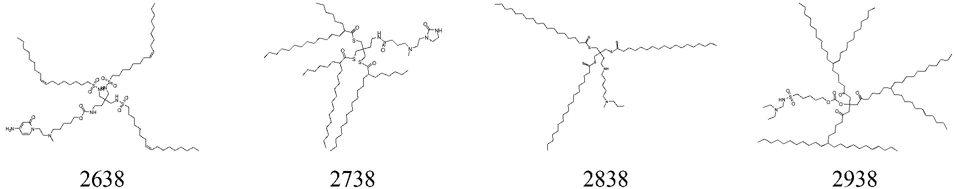

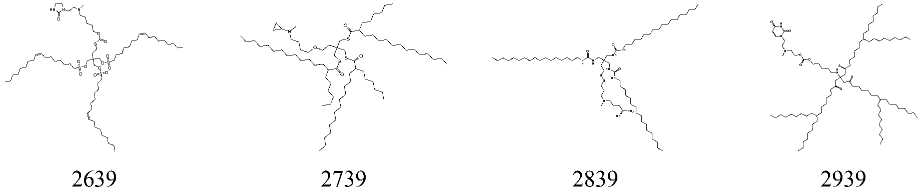

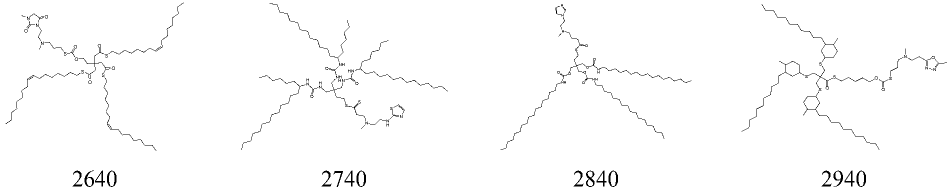

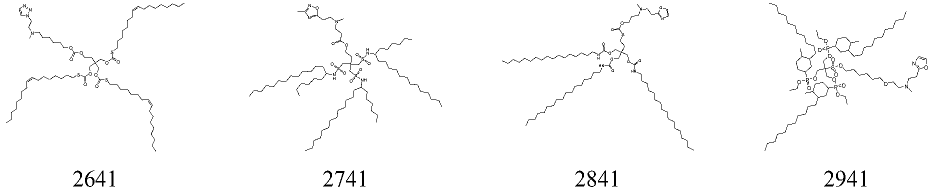

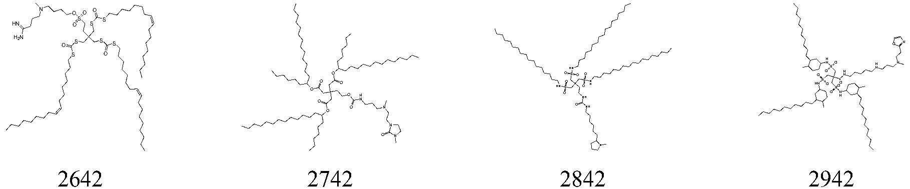

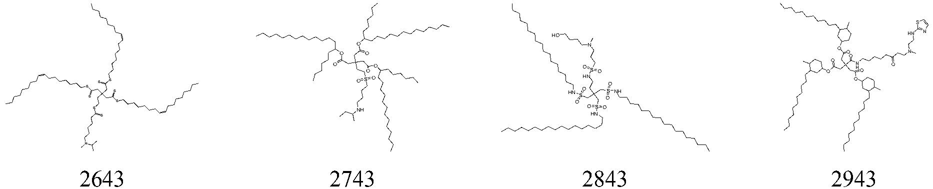

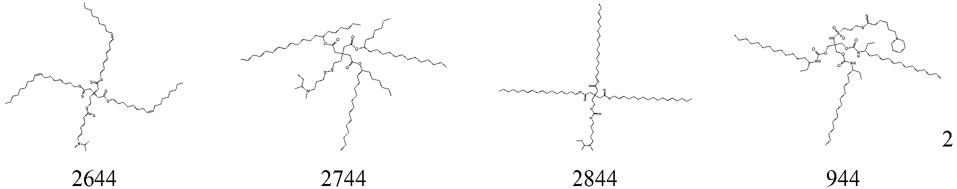

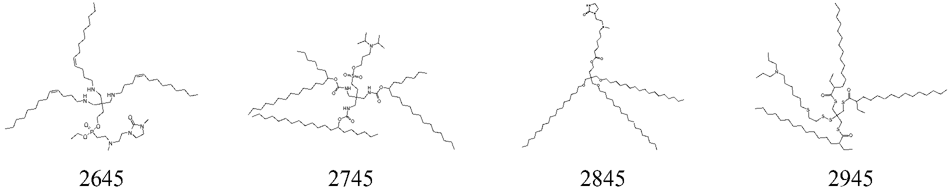

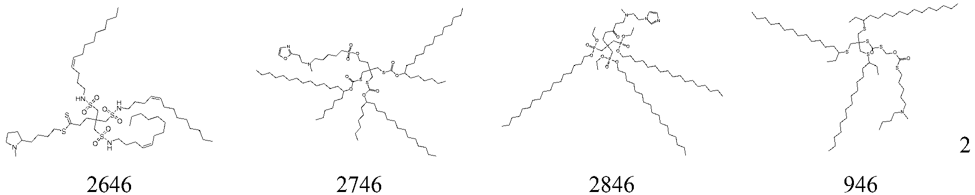

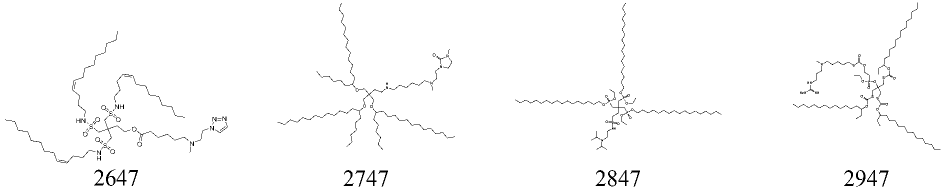

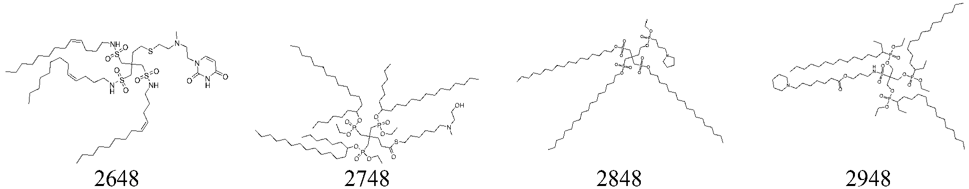

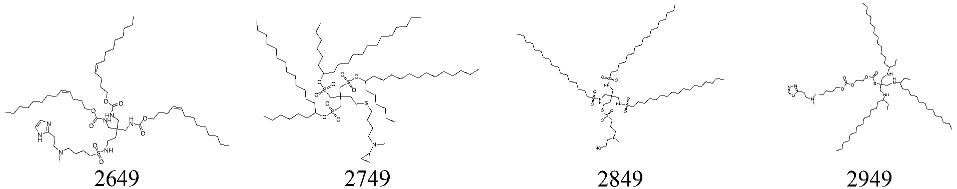

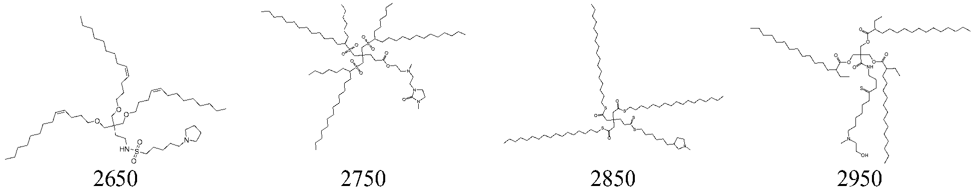

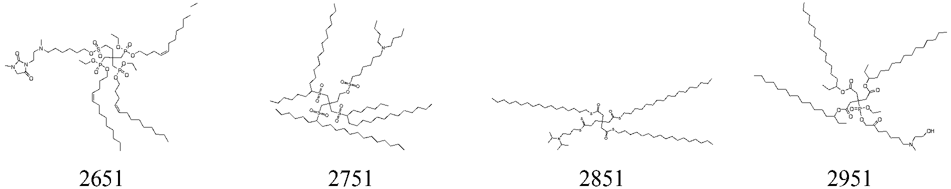

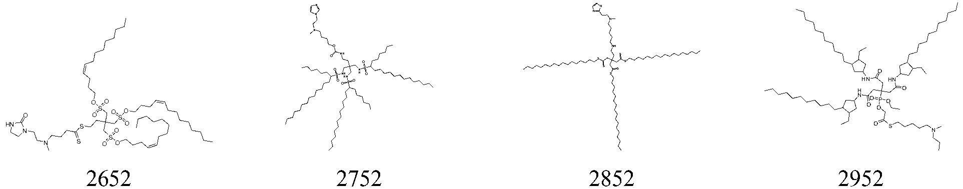

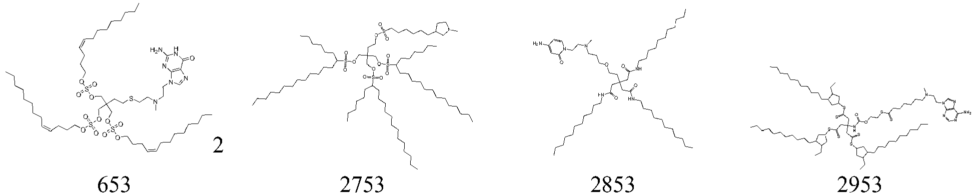

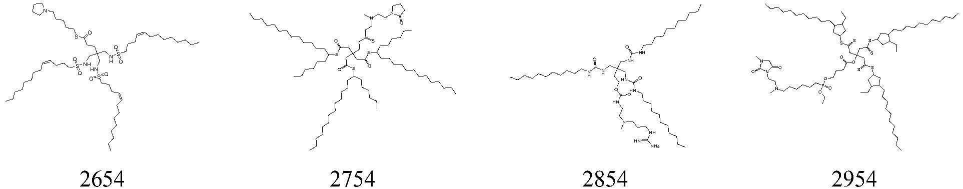

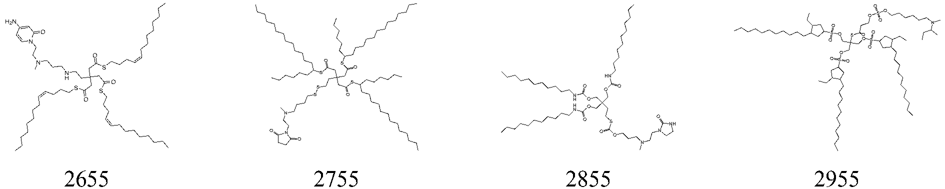

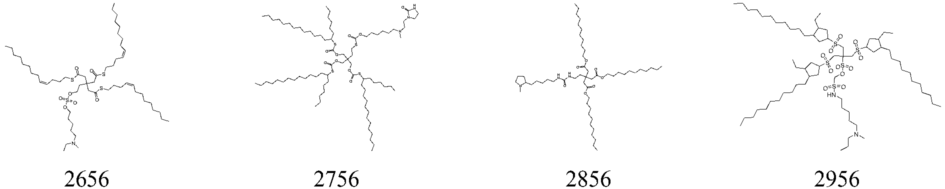

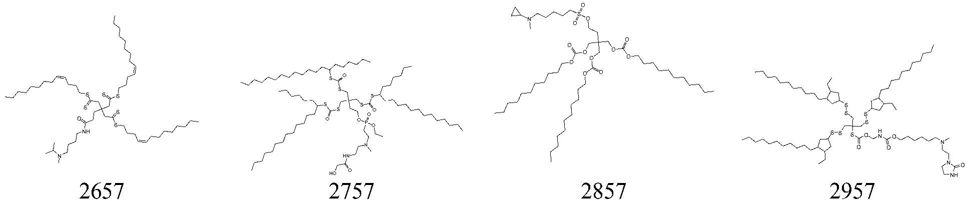

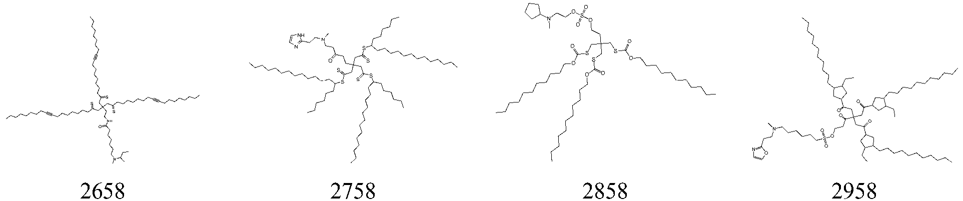

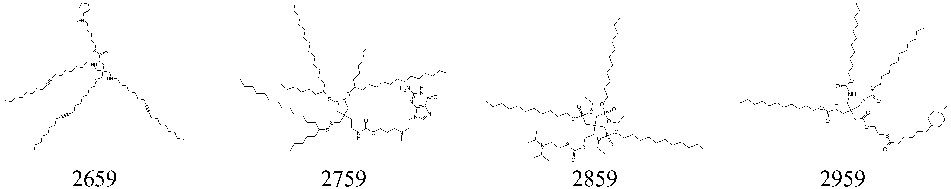

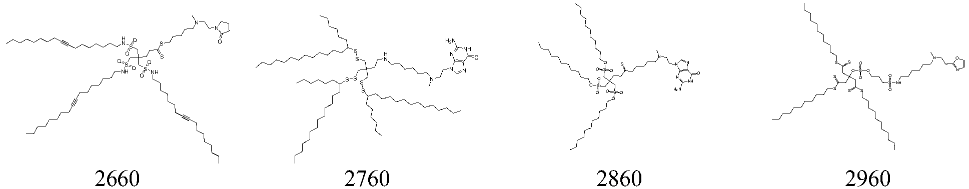

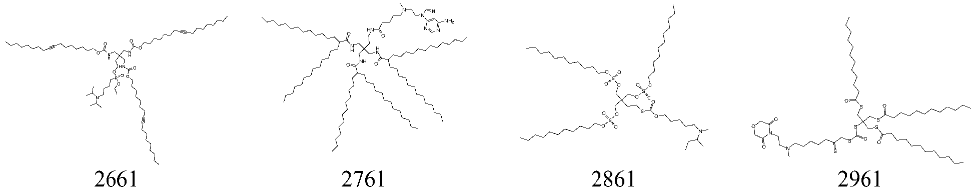

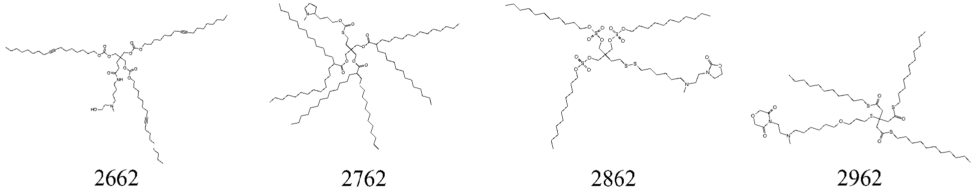

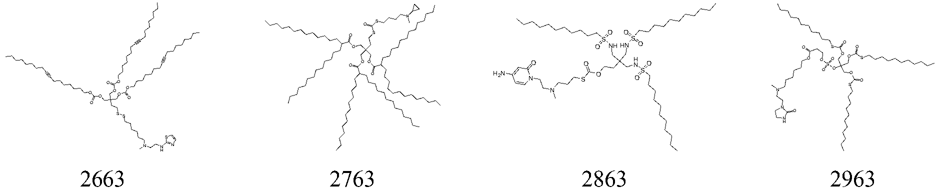

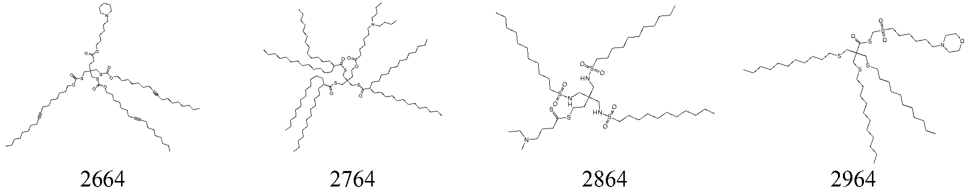

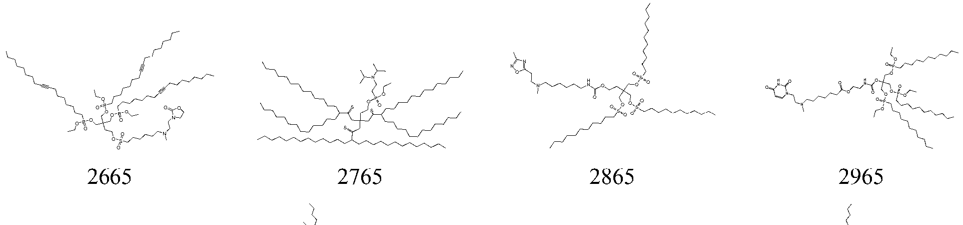

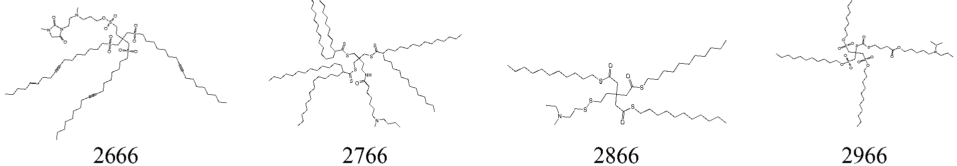

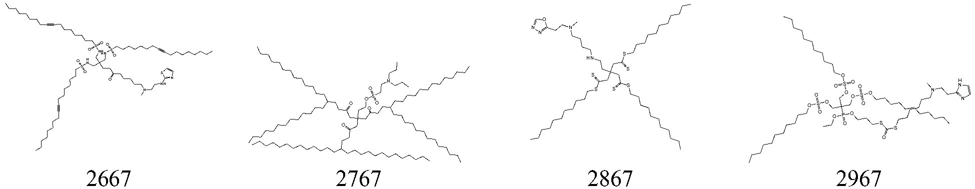

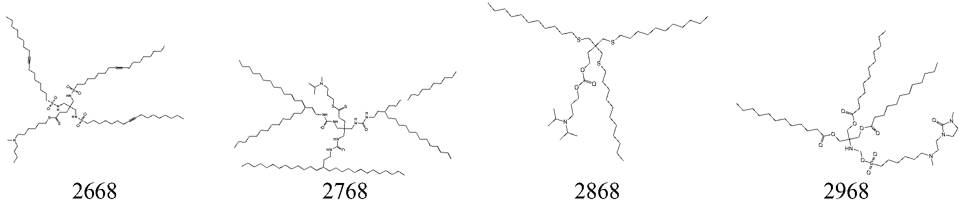

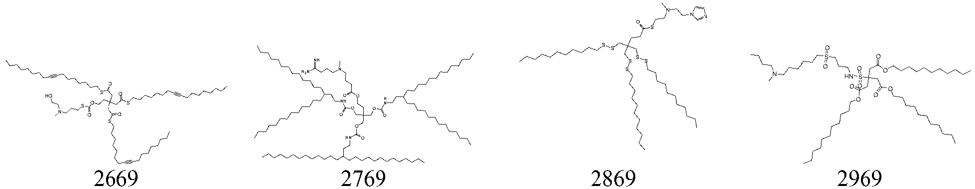

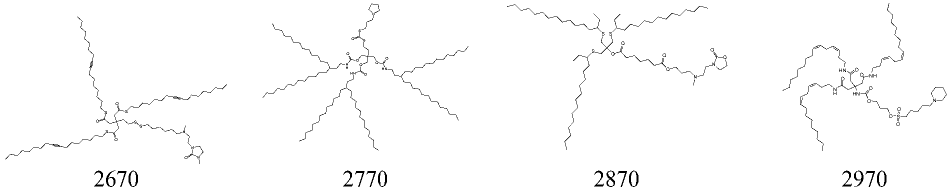

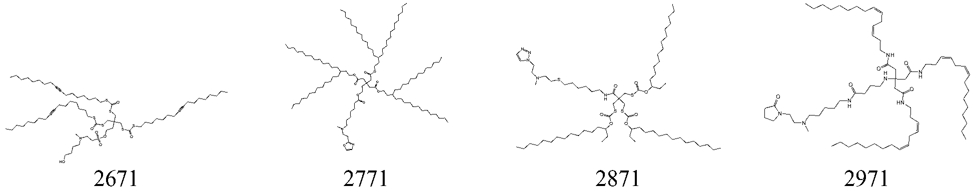

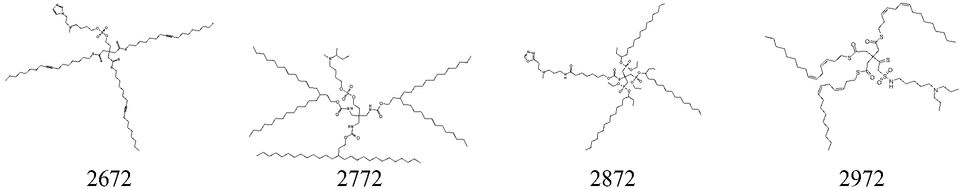

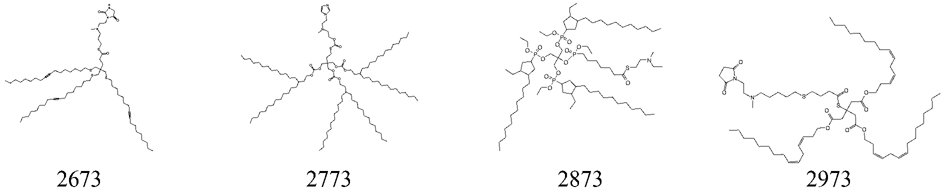

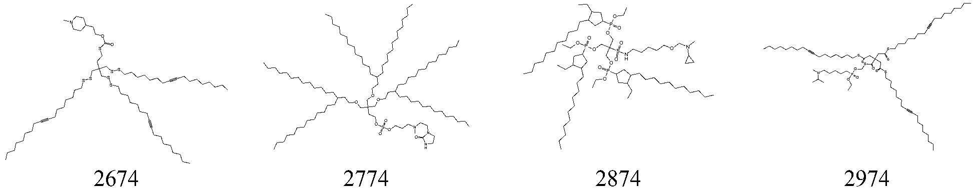

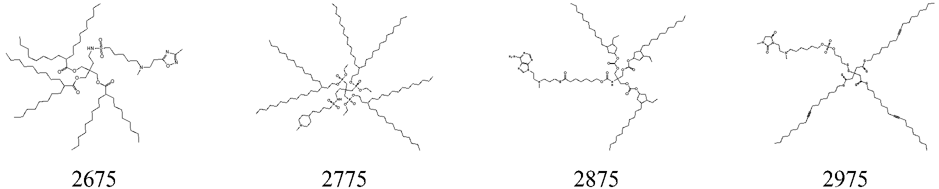

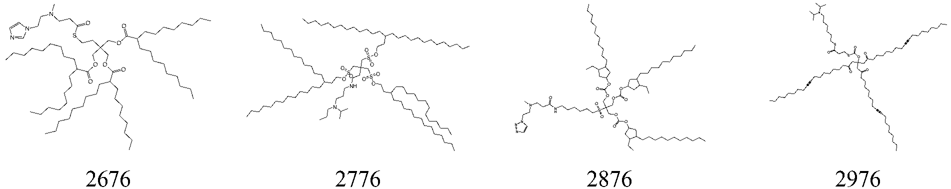

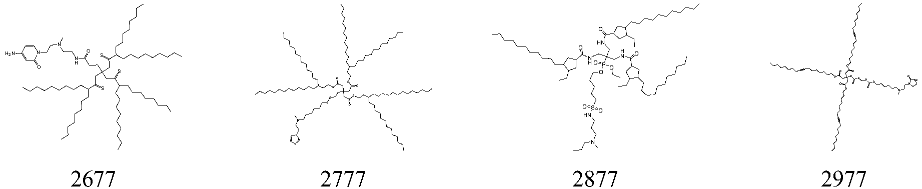

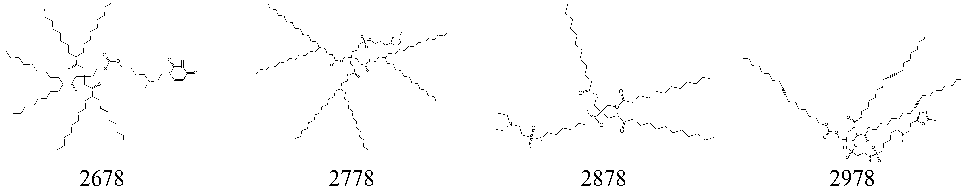

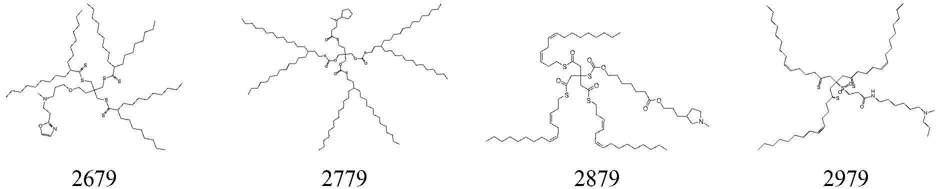

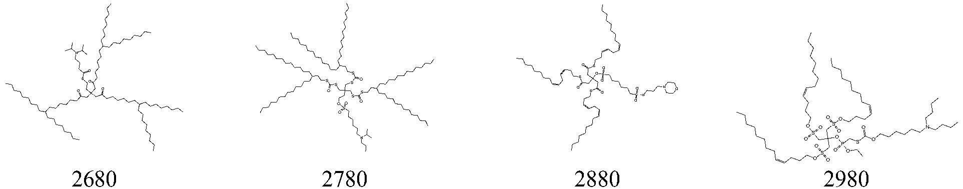

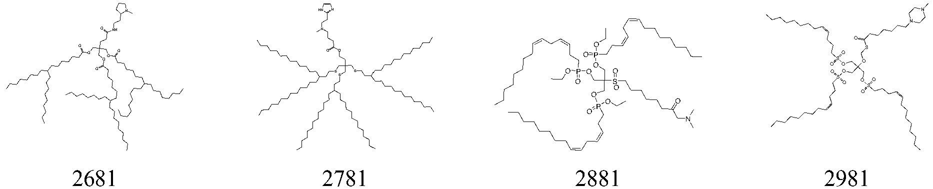

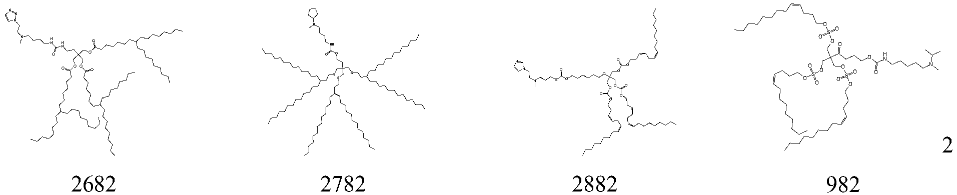

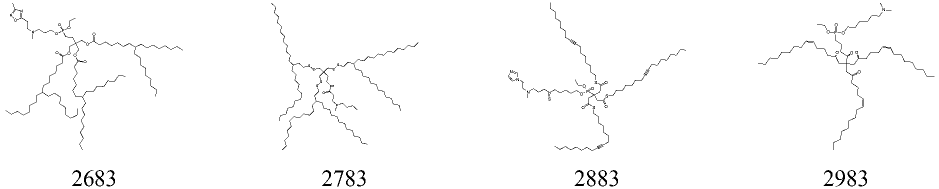

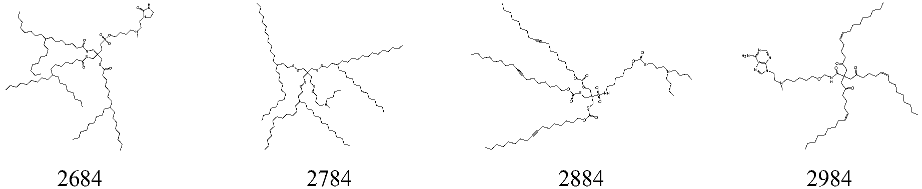

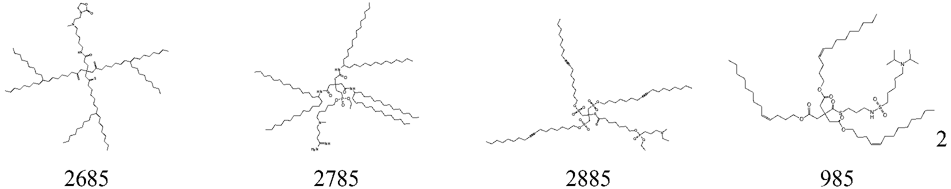

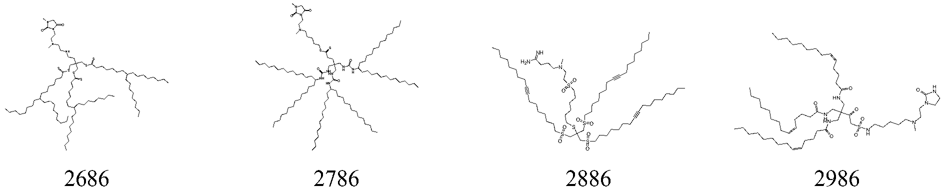

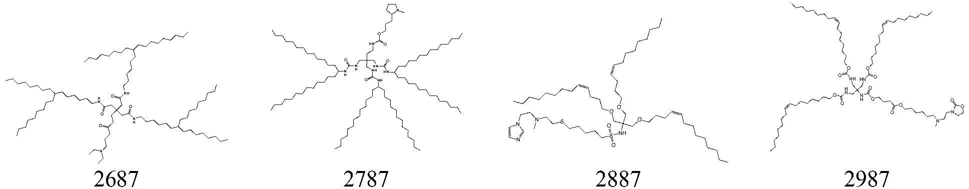

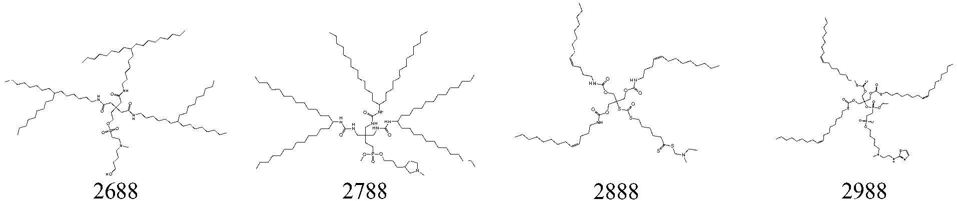

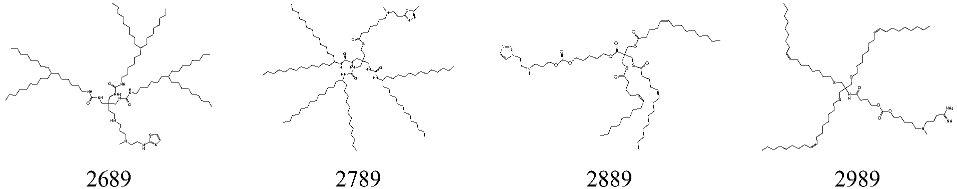

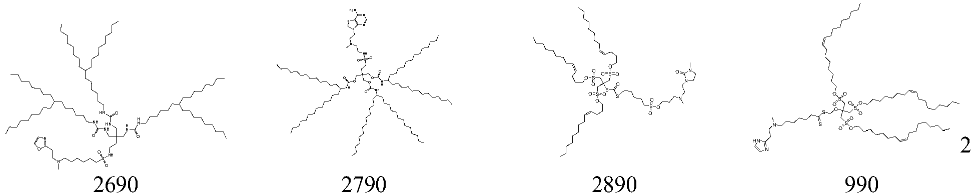

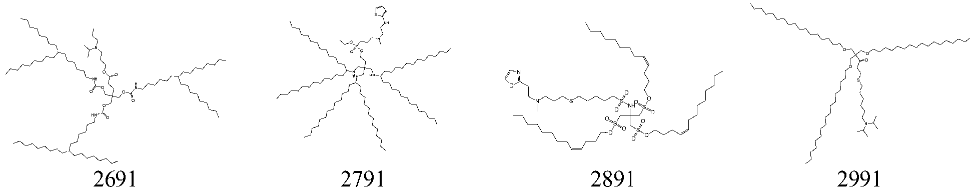

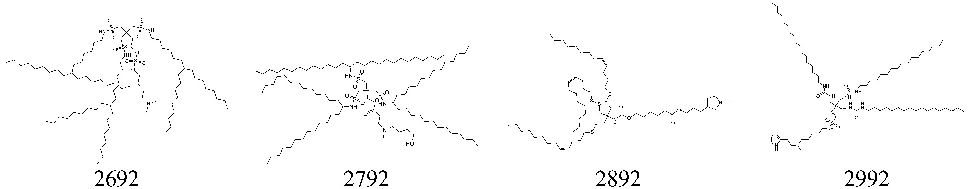

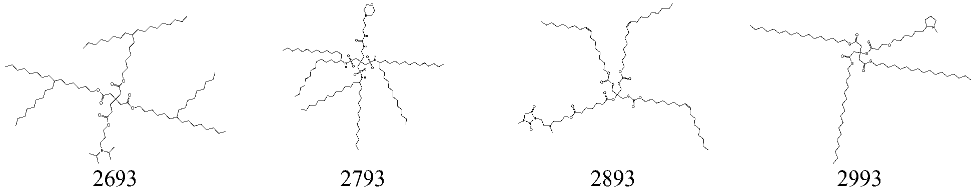

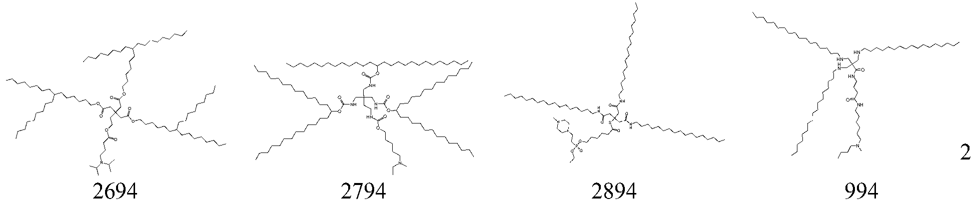

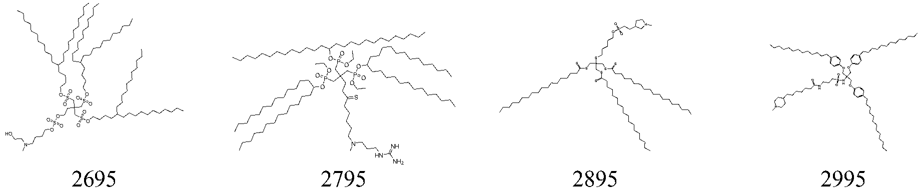

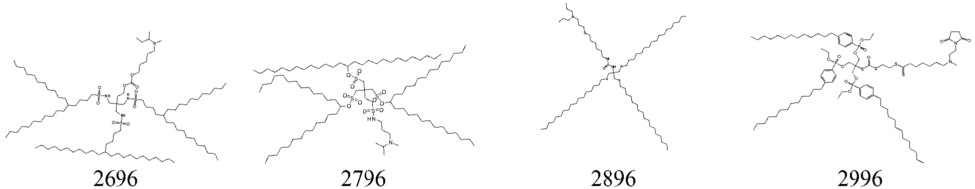

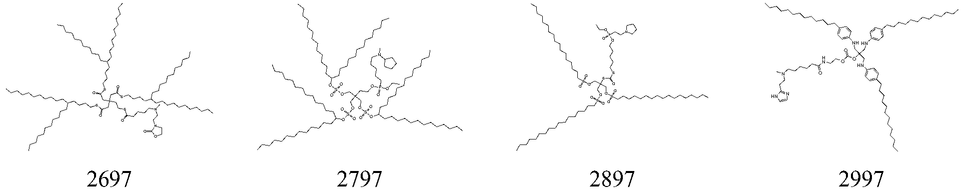

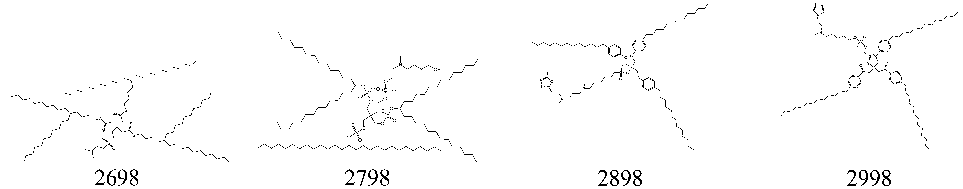

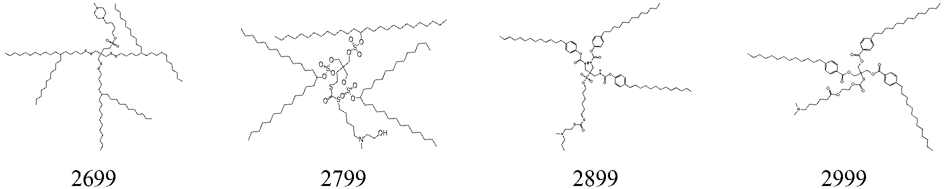

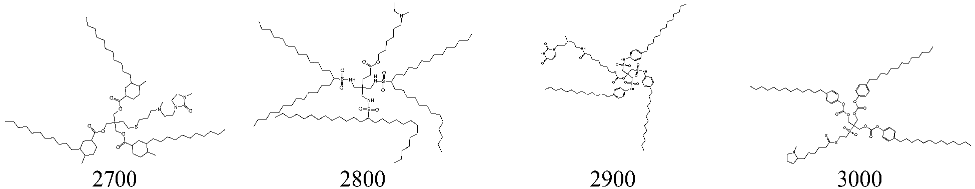

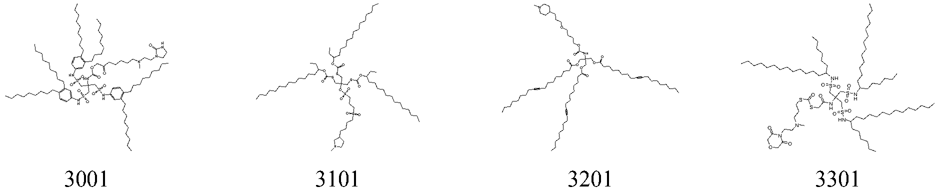

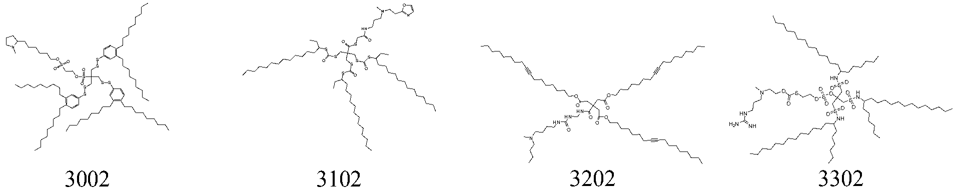

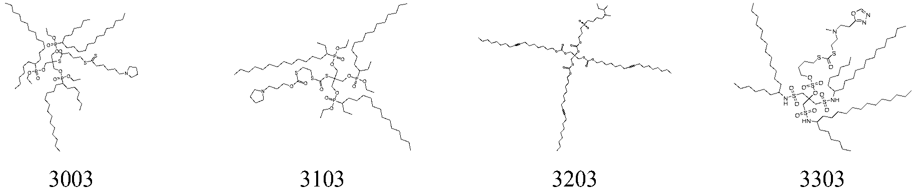

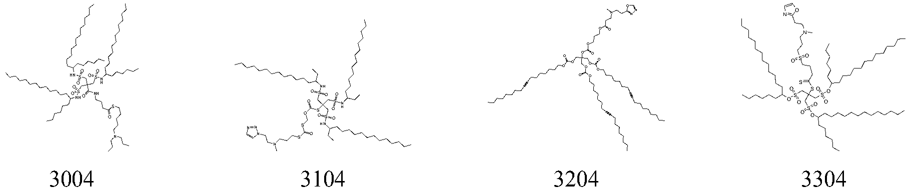

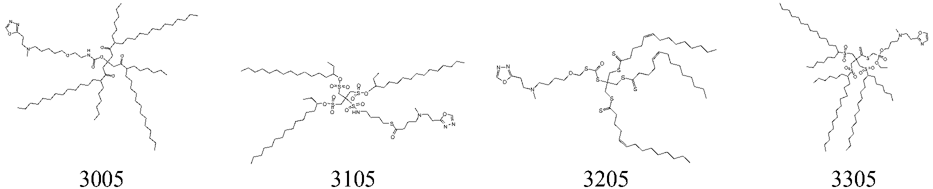

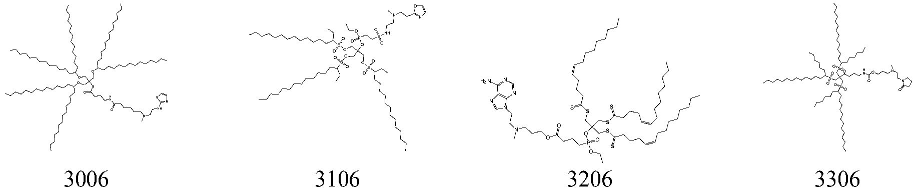

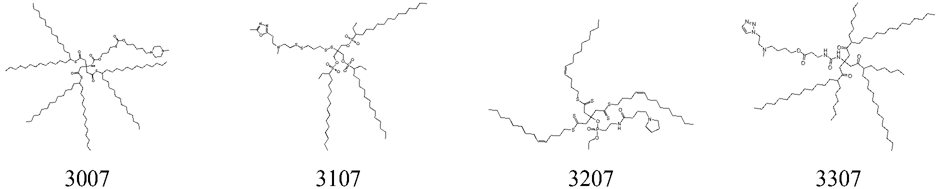

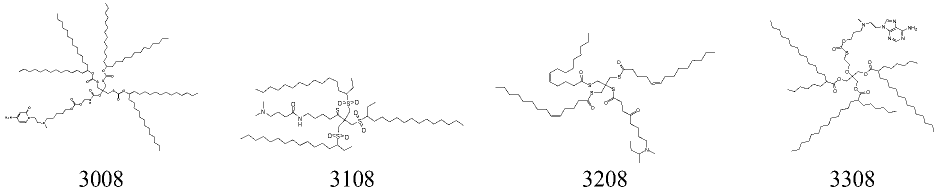

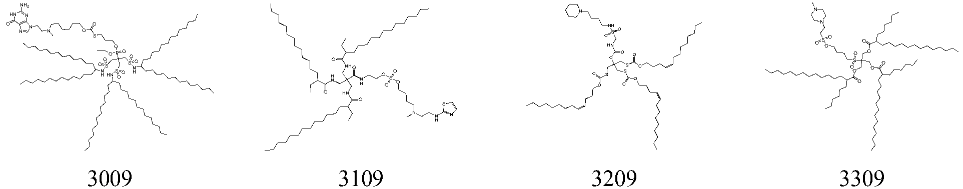

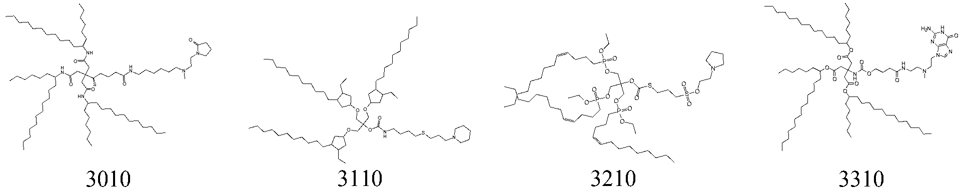

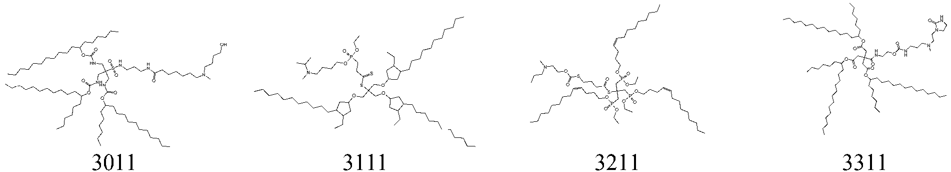

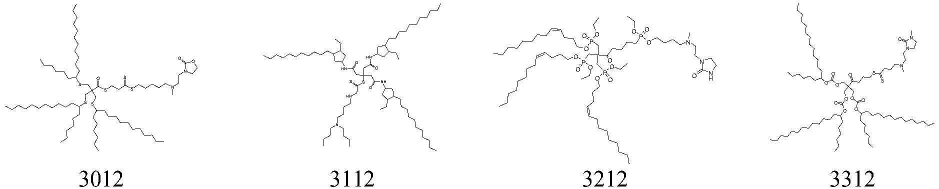

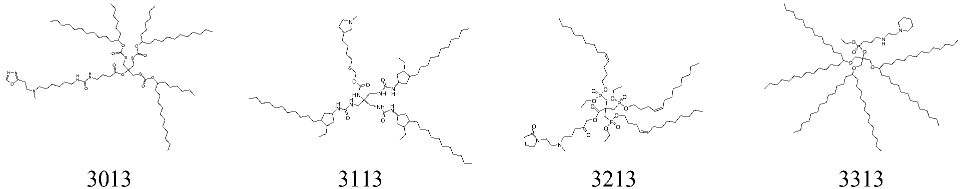

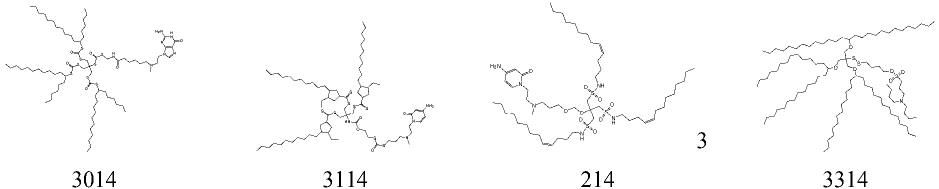

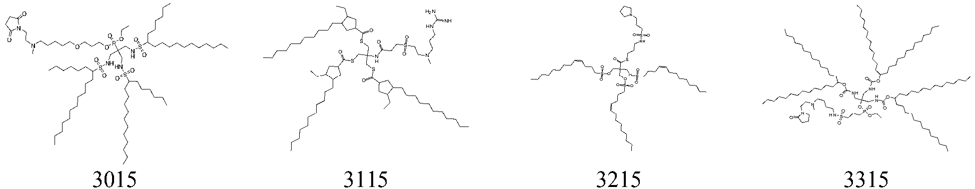

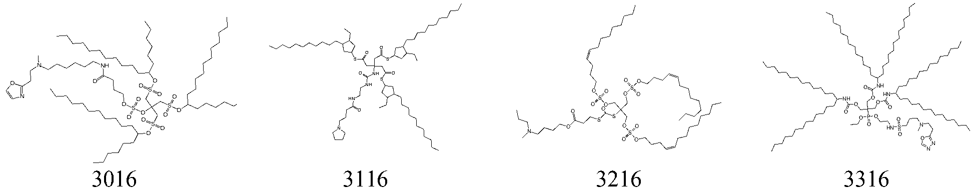

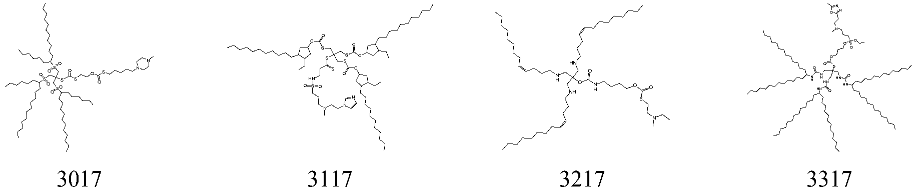

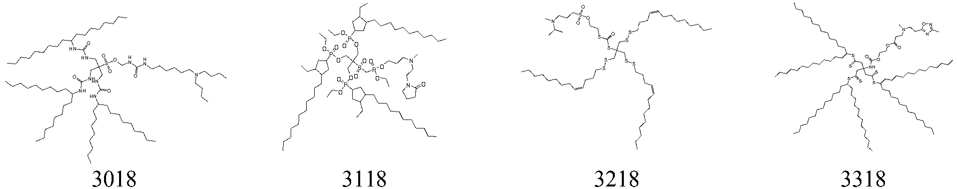

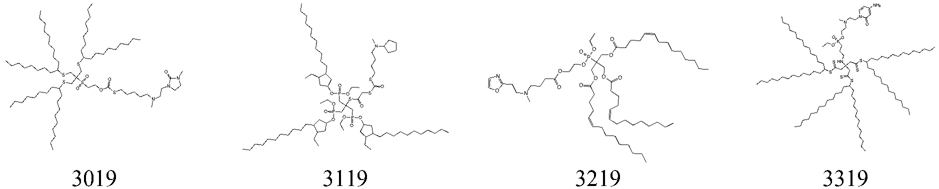

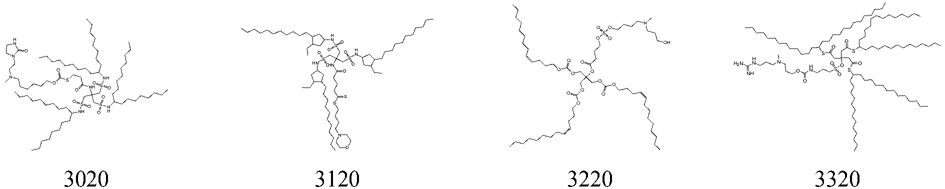

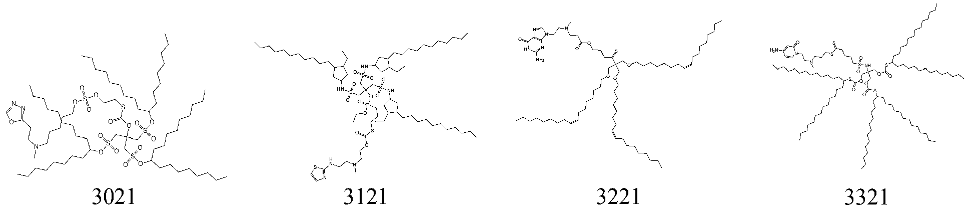

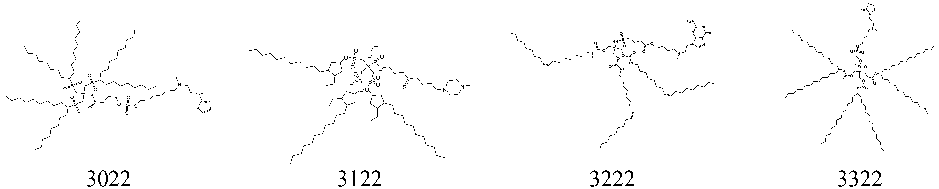

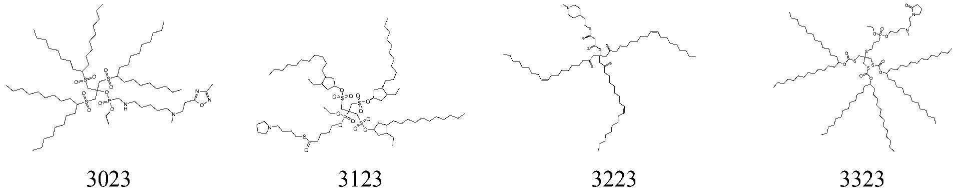

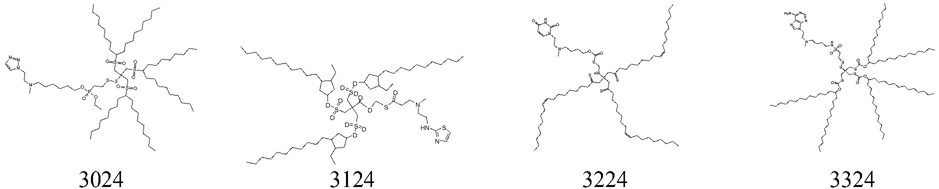

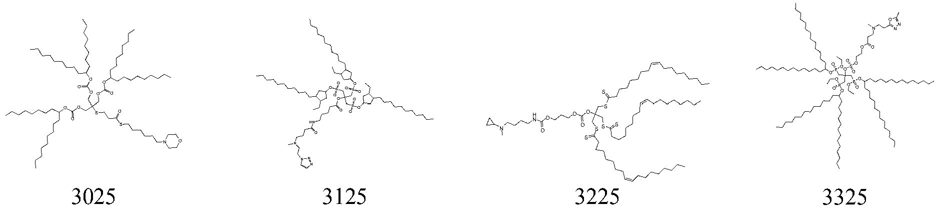

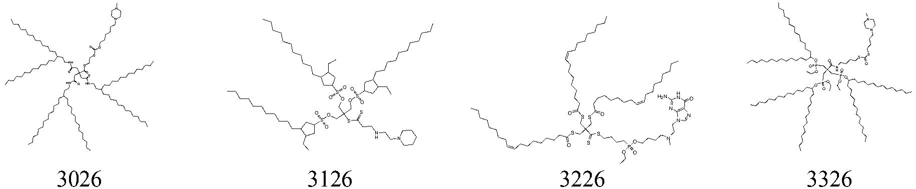

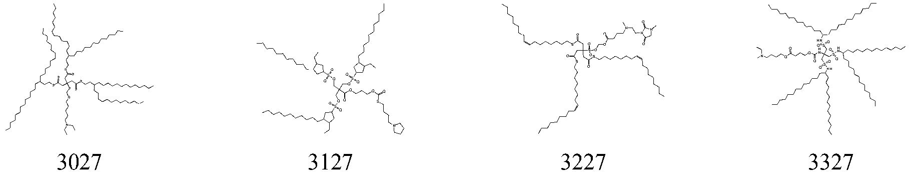

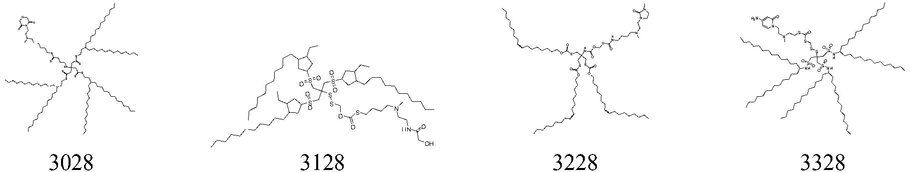

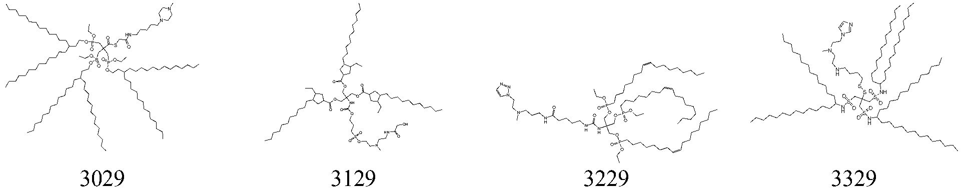

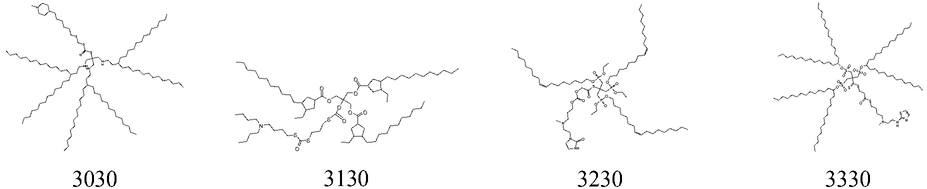

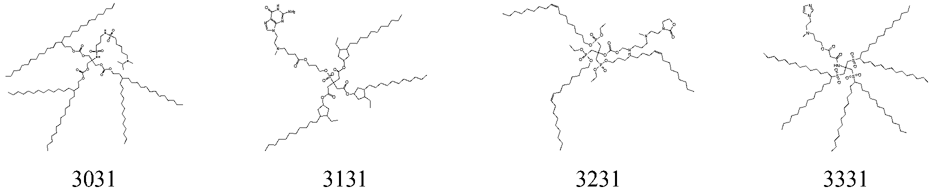

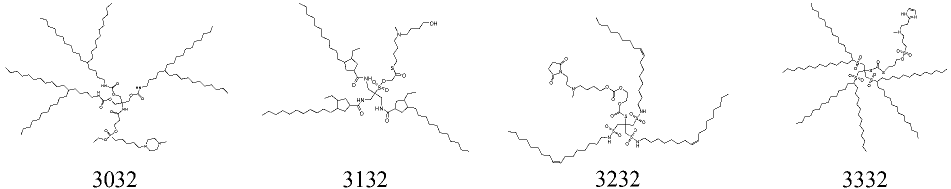

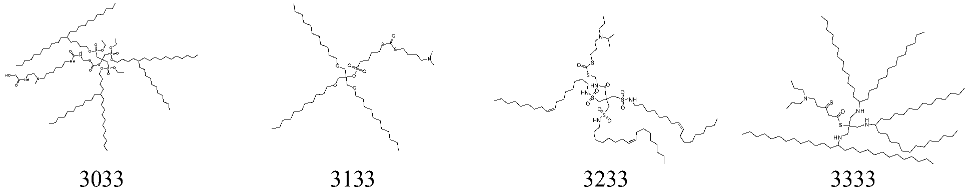

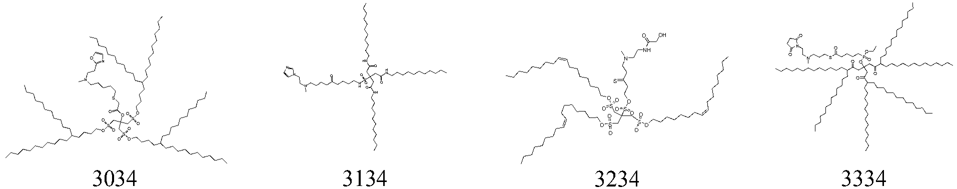

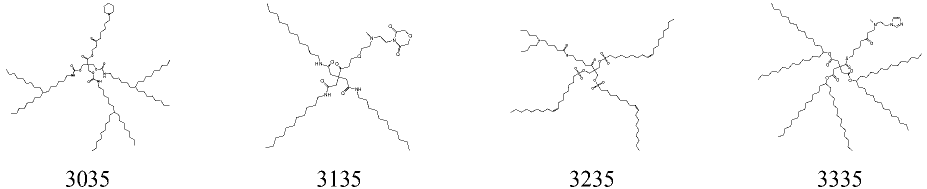

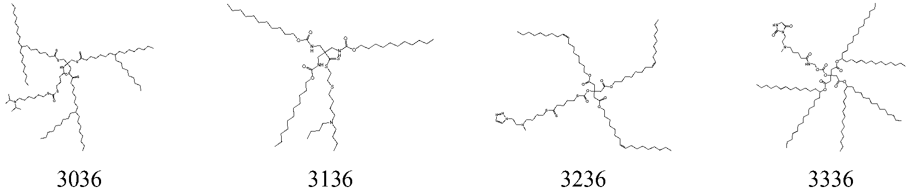

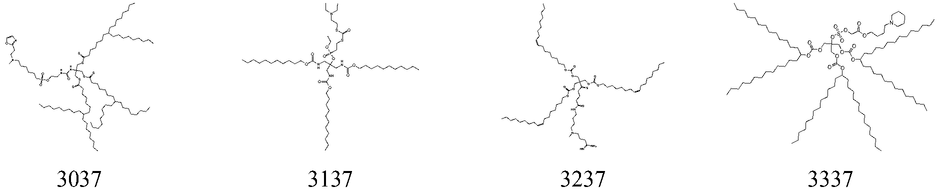

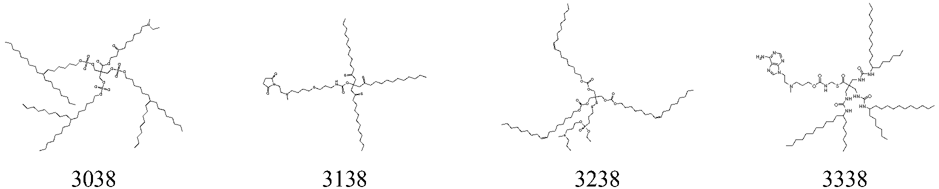

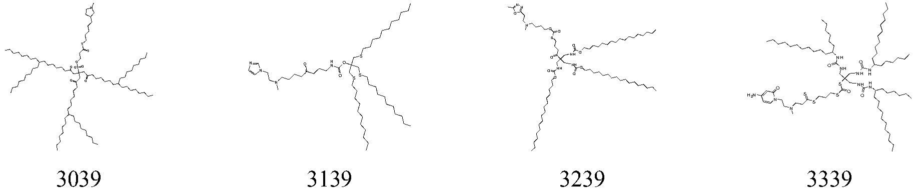

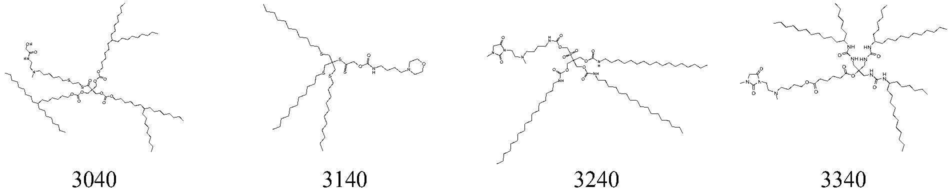

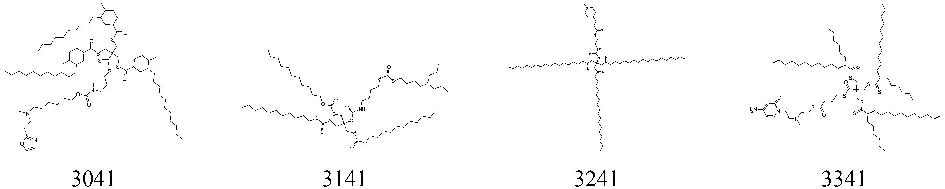

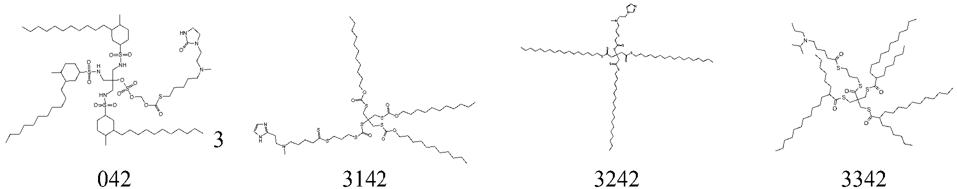

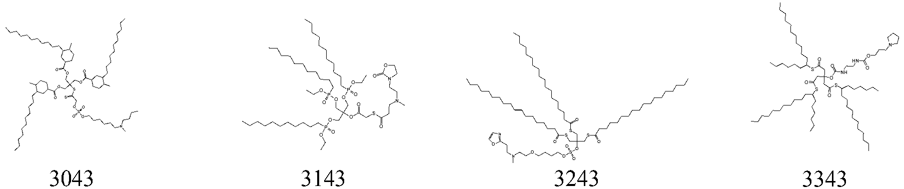

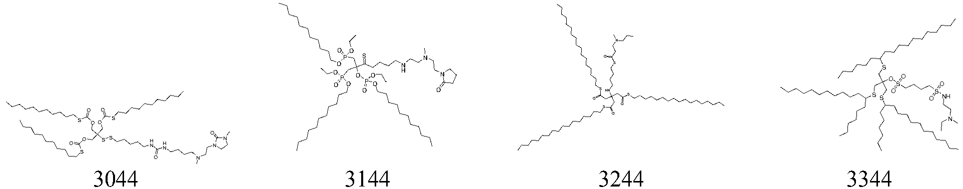

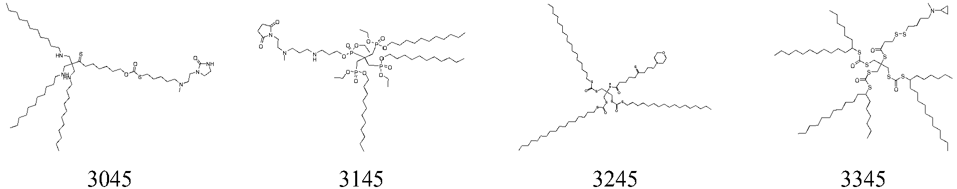

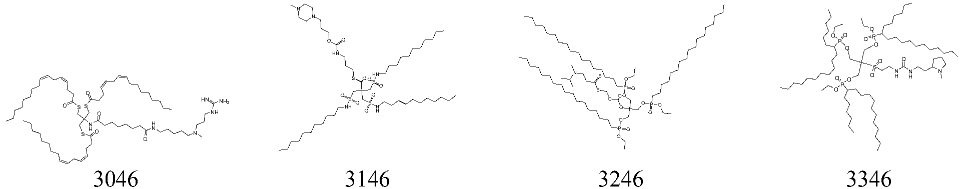

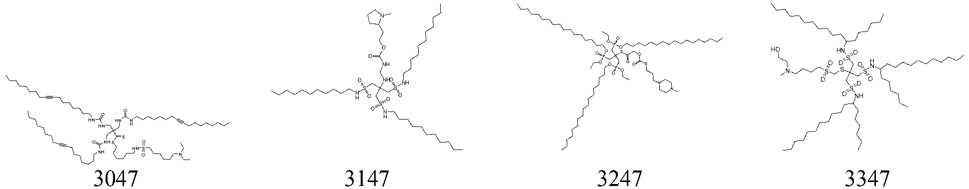

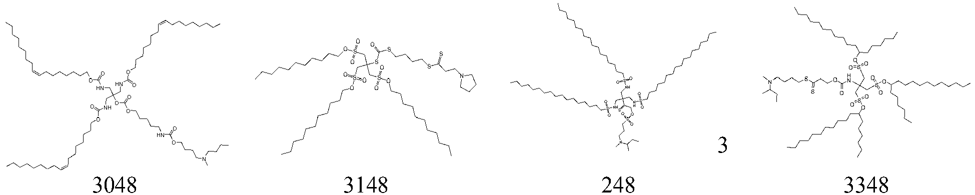

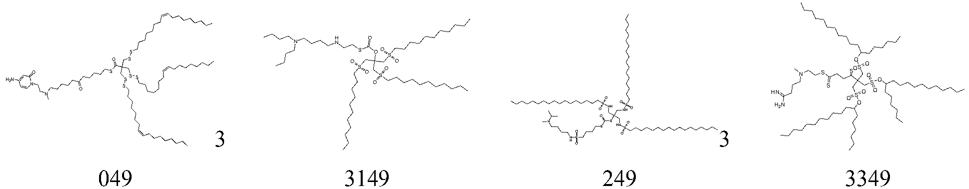

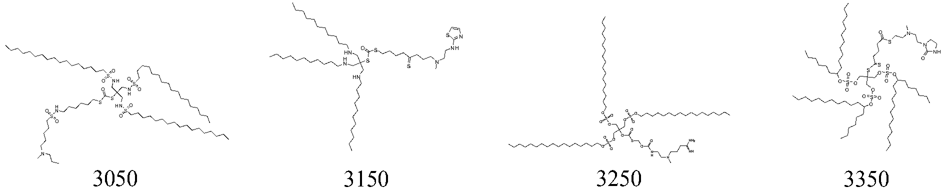

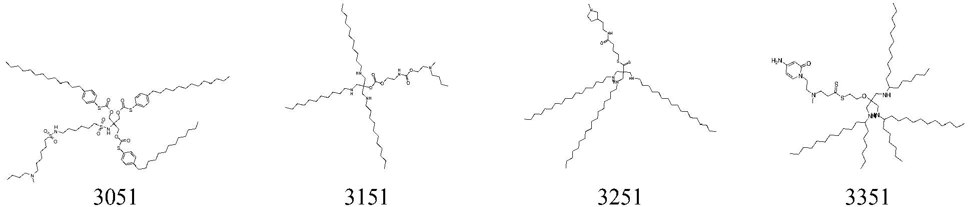

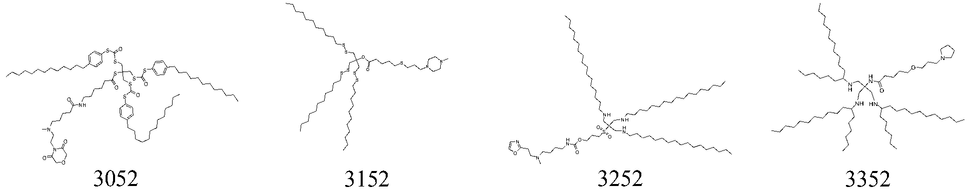

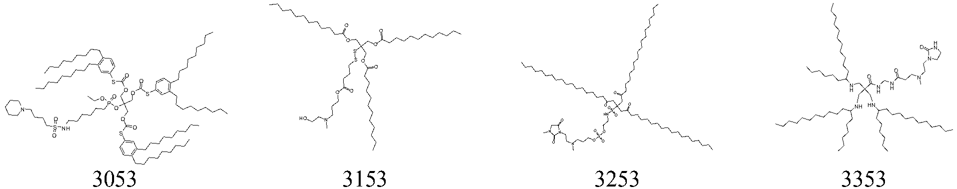

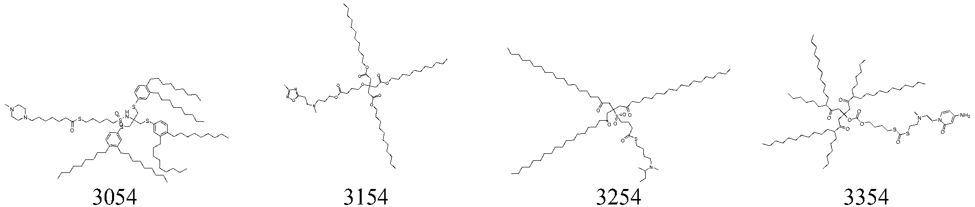

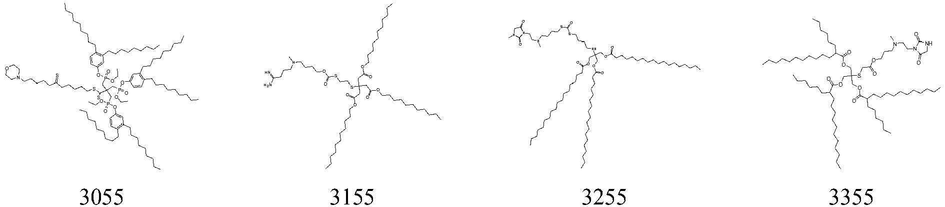

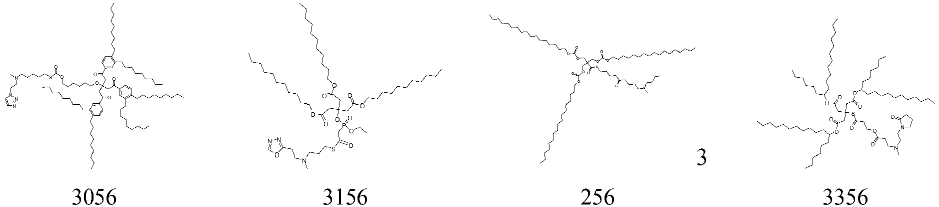

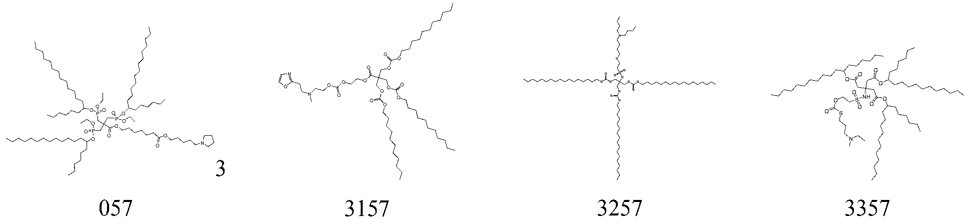

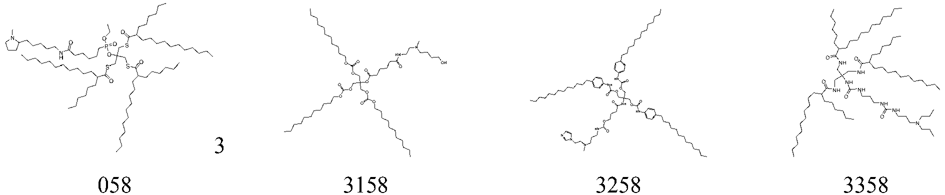

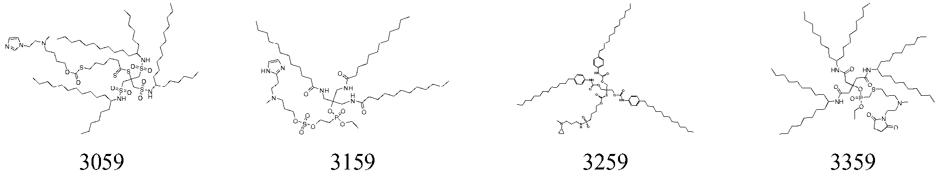

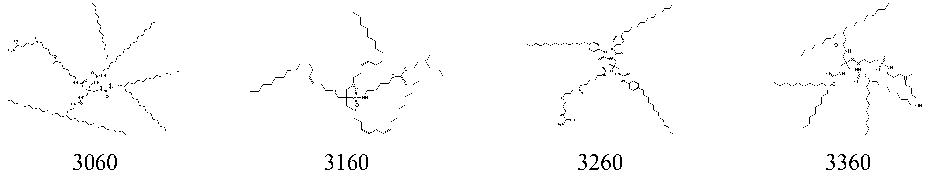

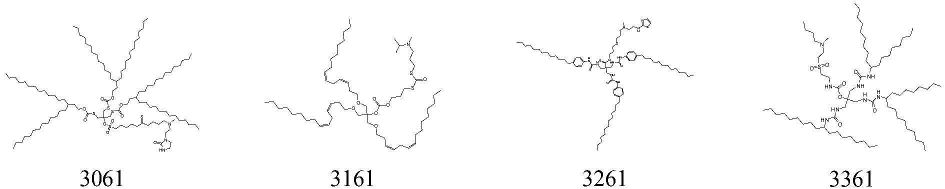

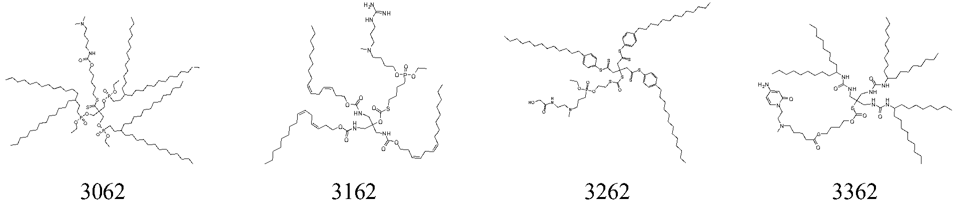

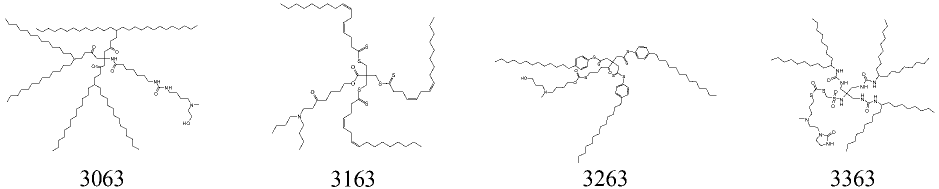

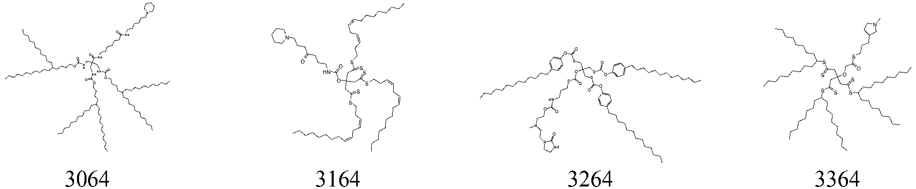

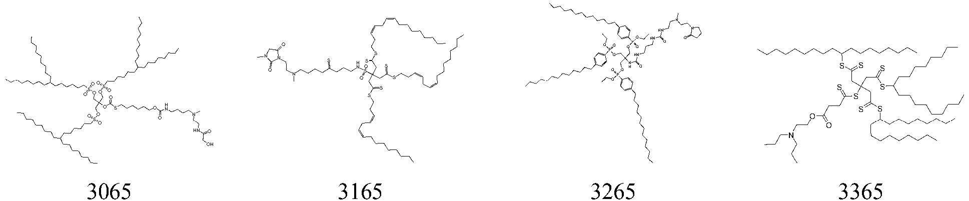

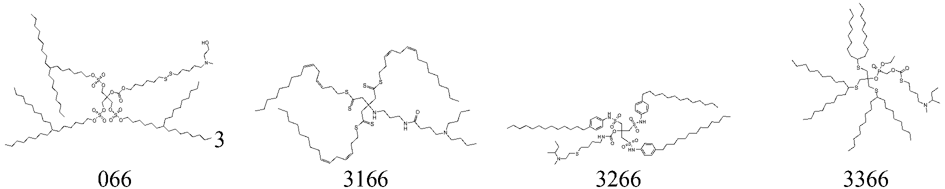

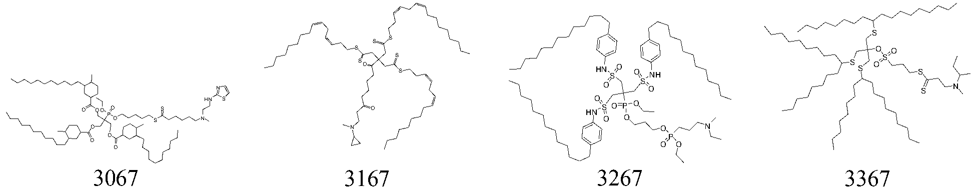

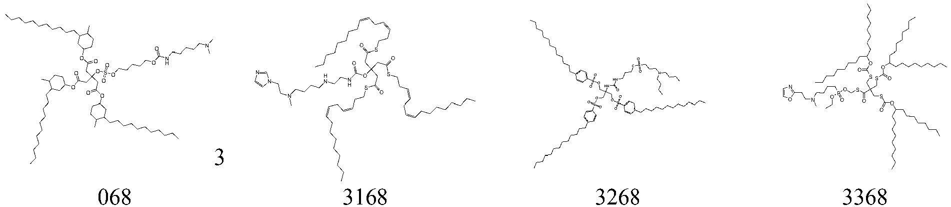

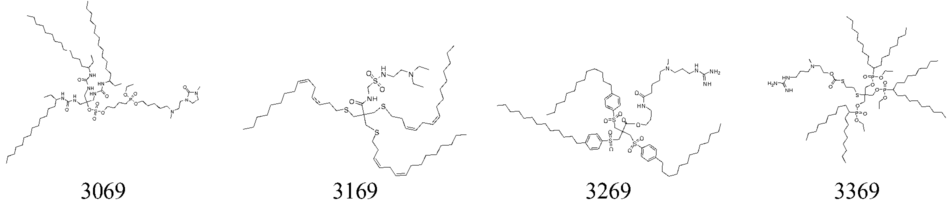

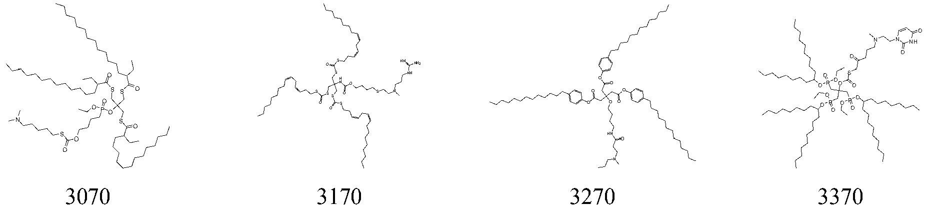

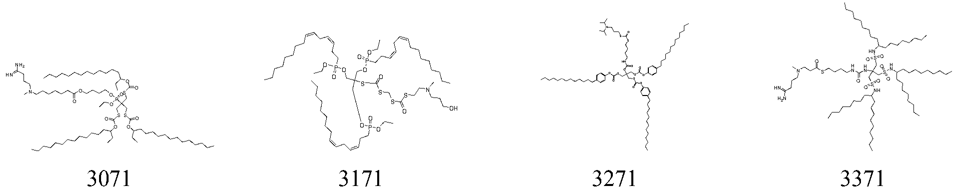

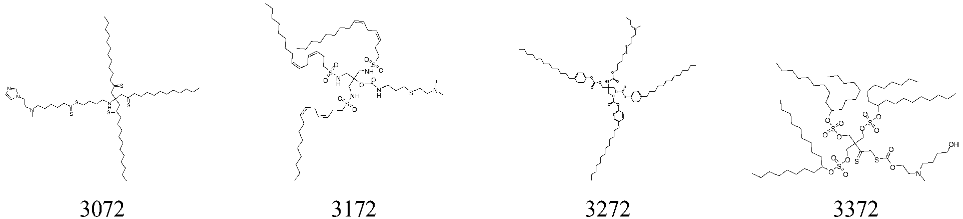

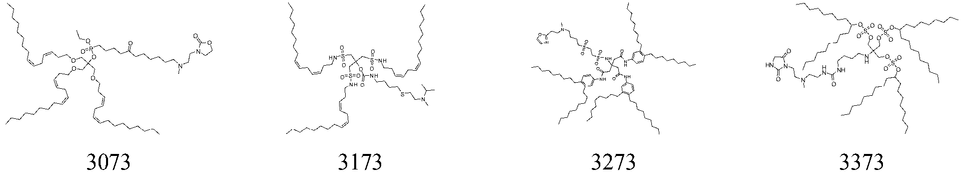

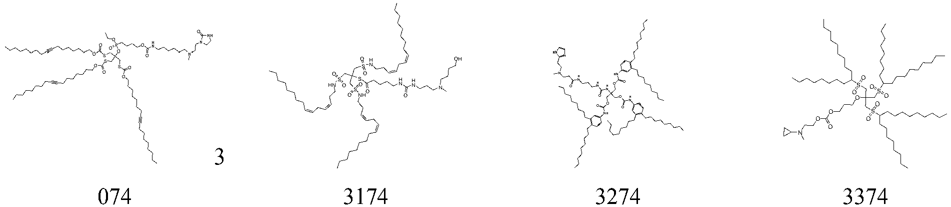

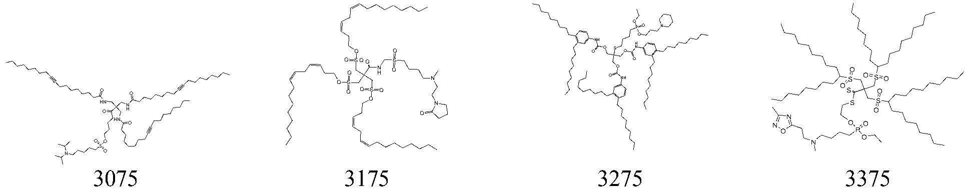

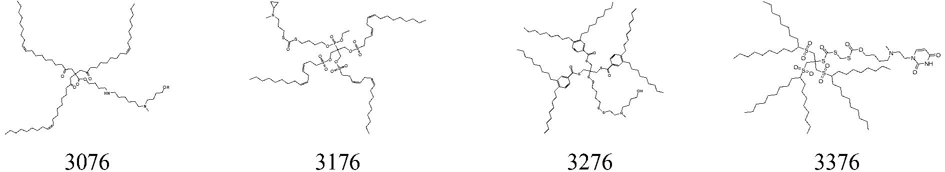

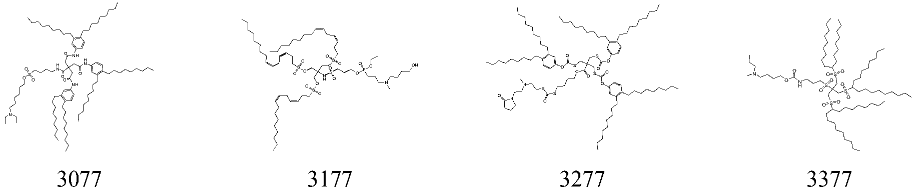

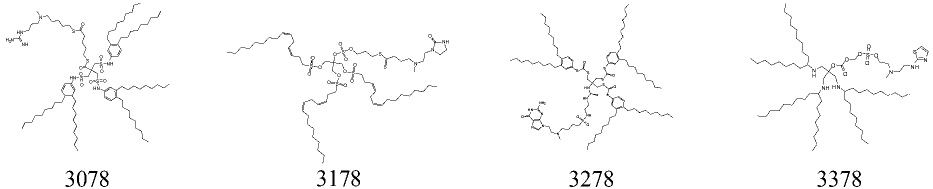

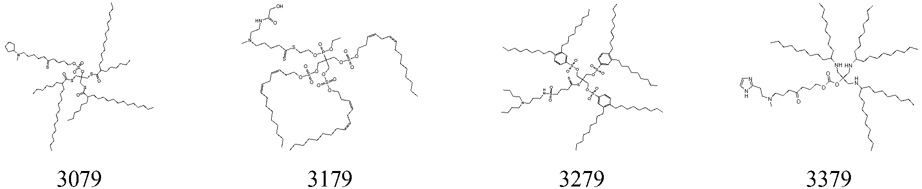

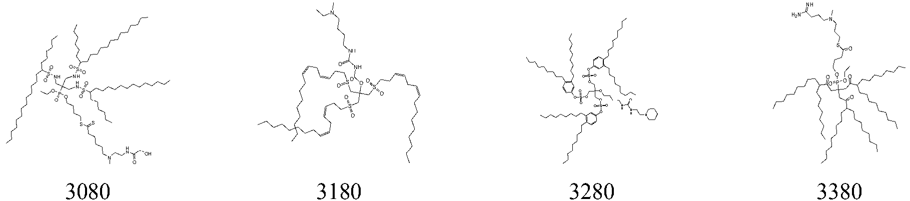

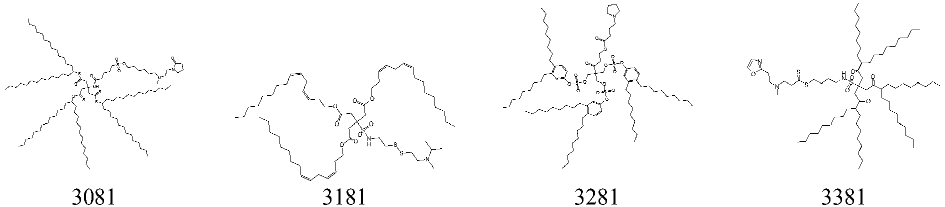

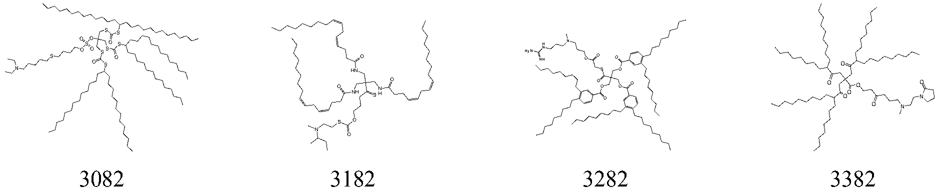

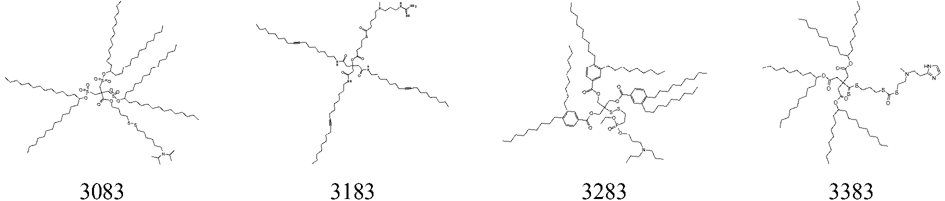

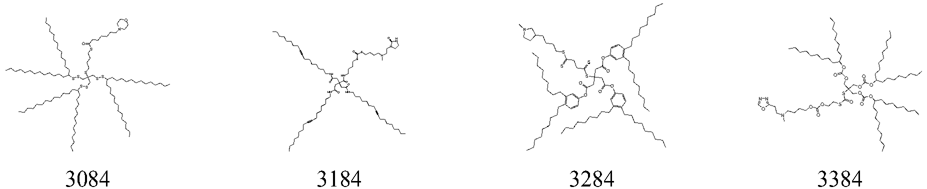

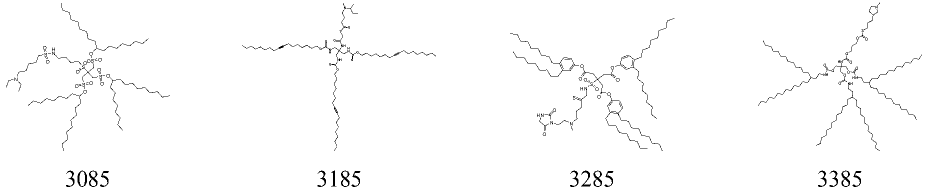

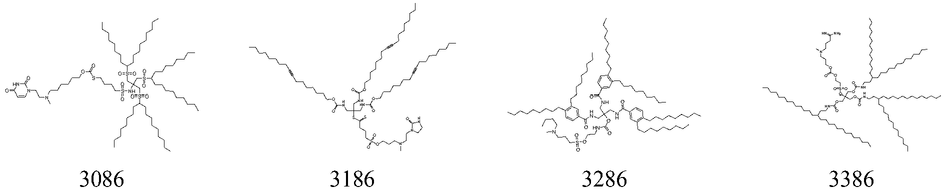

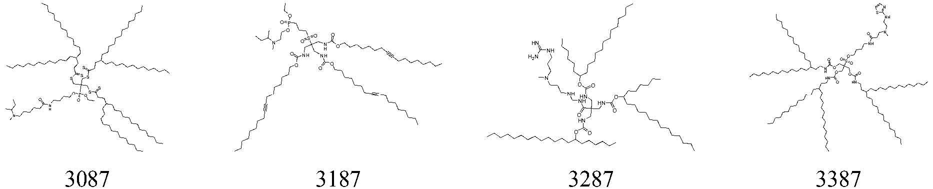

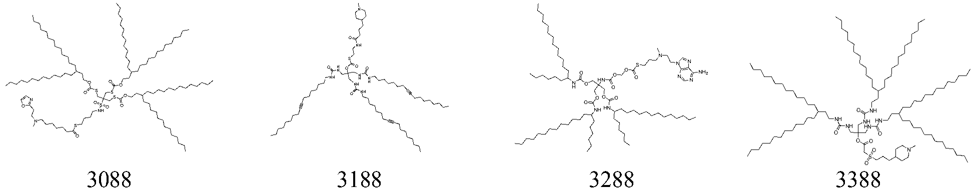

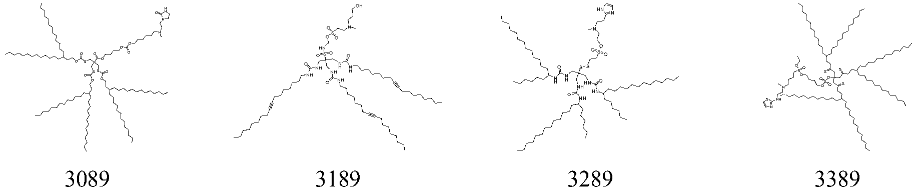

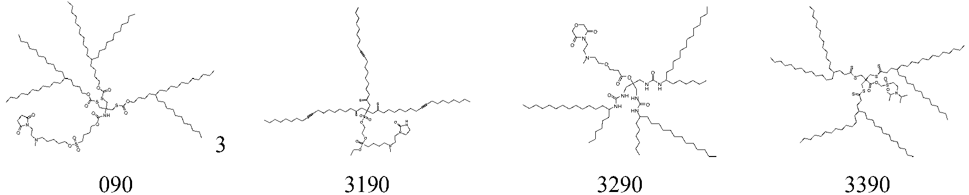

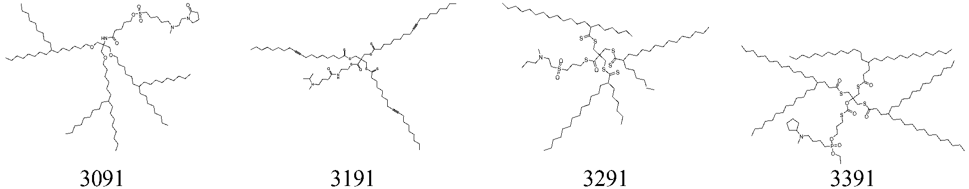

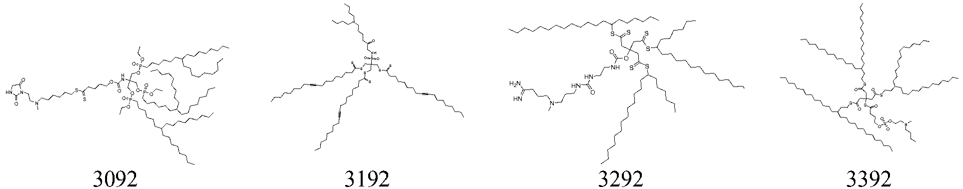

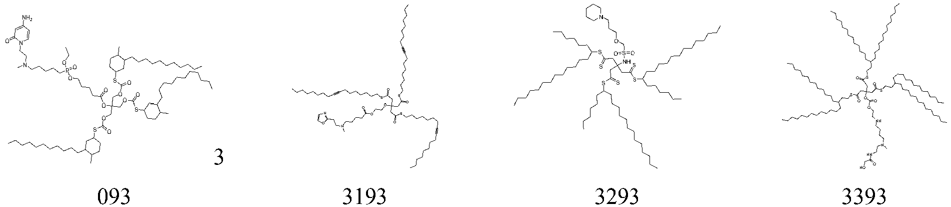

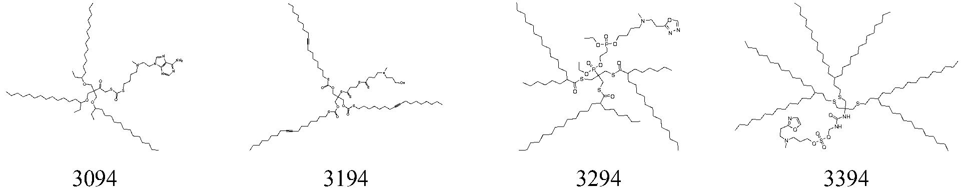

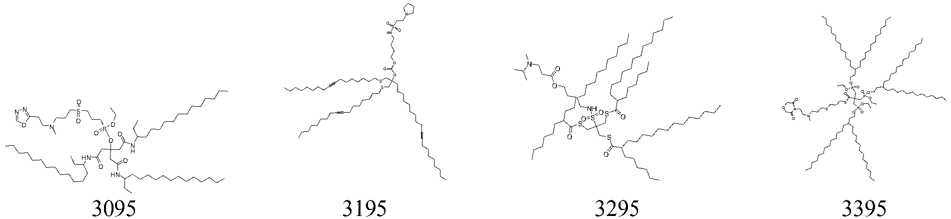

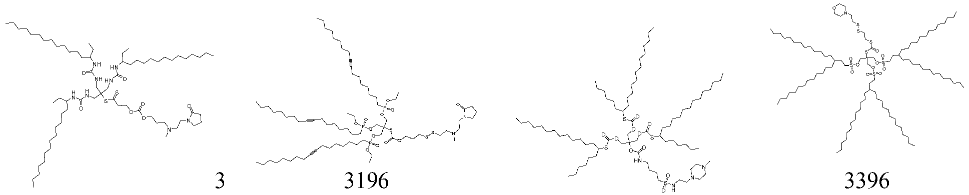

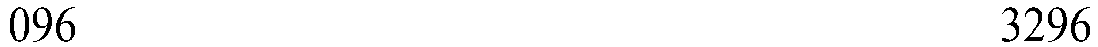

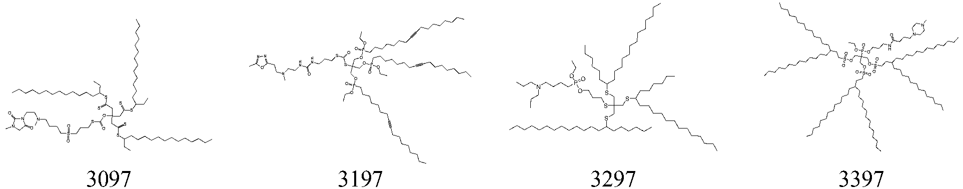

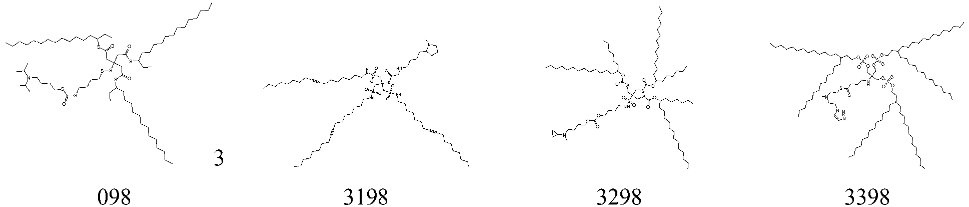

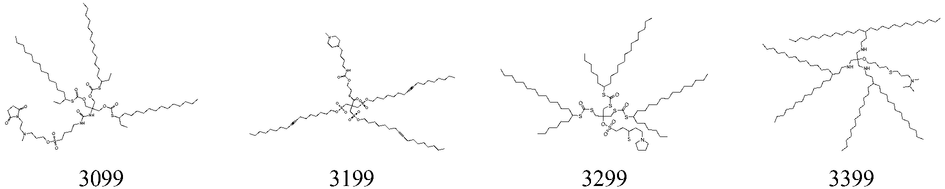

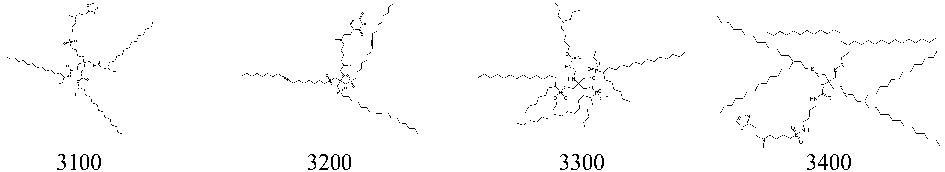

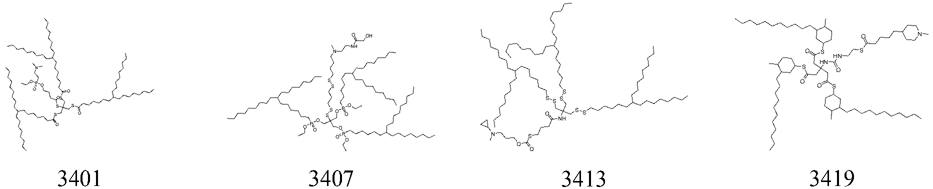

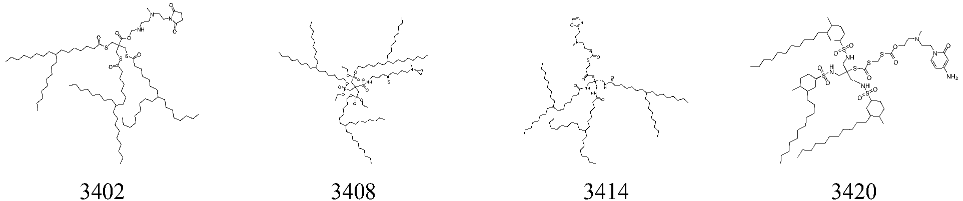

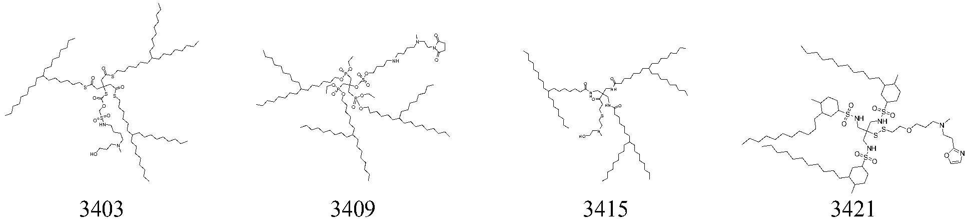

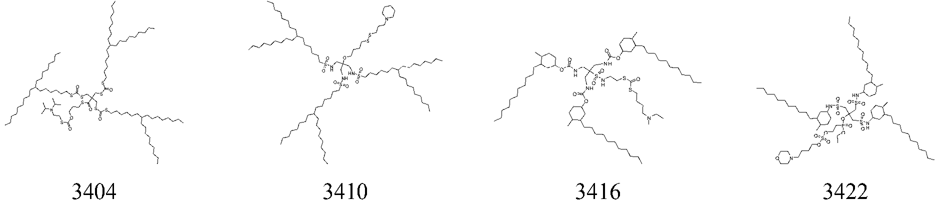

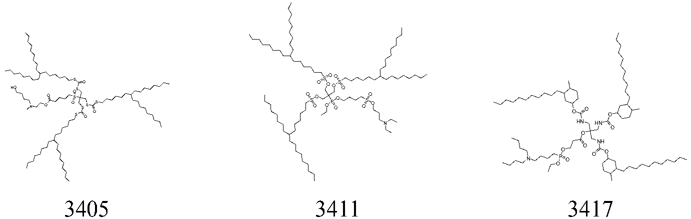

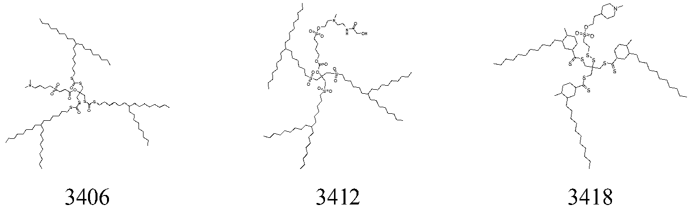

In a preferred embodiment, a method for preparing the first ionizable lipid includes a step of reacting a compound of Formula (II) with a compound of Formula (III): 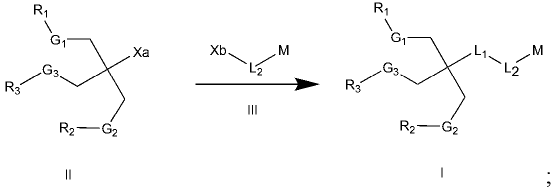 where Xa and Xb are groups containing a leaving group or a nucleophilic group, and Xa and Xb form L₁ via a nucleophilic reaction or a condensation reaction.

In a preferred embodiment, the method for preparing the first ionizable lipid includes a step of reacting a compound of Formula (IV) with a compound of Formula (V): 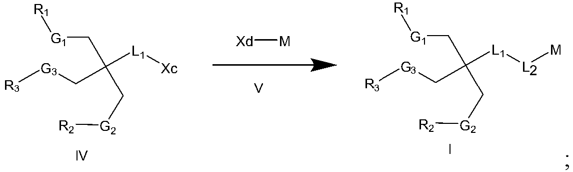 where Xc and Xd are groups containing a leaving group or a nucleophilic group, and Xc and Xd form L₂ via the nucleophilic reaction or the condensation reaction.

In a preferred embodiment, the method for preparing the first ionizable lipid includes the step of reacting a compound of Formula (VI): 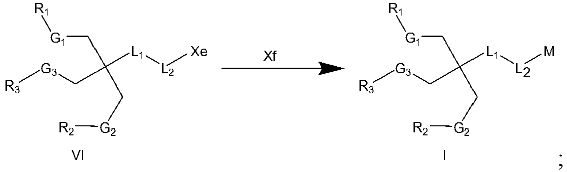 where Xe is a group containing a leaving group or a nucleophilic group, Xf is a group containing a leaving group or a nucleophilic group, and Xe and Xf form M via the nucleophilic reaction or the condensation reaction.

In a preferred embodiment, the method for preparing the first ionizable lipid includes a step of sequentially reacting a compound of Formula (VII) with a compound of Formula (VIII), a compound of Formula (IX), and a compound of Formula (X): 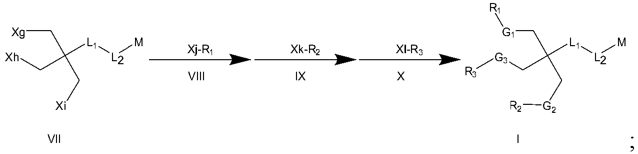 where Xg, Xh, Xi, Xj, Xk, and X1 are groups containing a leaving group or a nucleophilic group, and Xg and Xj form G₁ via the nucleophilic reaction or the condensation reaction, Xh and Xk form G₂ via the nucleophilic reaction or the condensation reaction, and Xi and X1 form G₃ via the nucleophilic reaction or the condensation reaction.

In a preferred embodiment, the method for preparing the first ionizable lipid icludes a step of reacting a compound of Formula (XI) with a compound of Formula (XII): 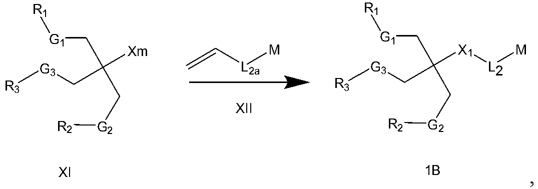 or
a step of reacting a compound of Formula (XIII) with a compound of Formula (XII): 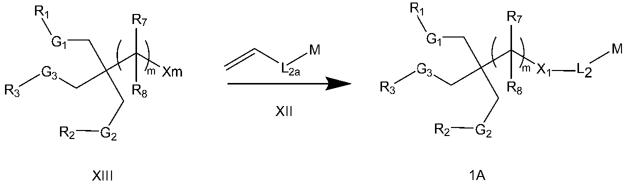
where Xm is a group containing a nucleophilic group, and Xm and C=C-L₂ₐ of the compound of Formula (XII) form X₁-L₂ via an addition reaction.

In a preferred embodiment, R₁, R₂, and R₃ are each independently the following groups: 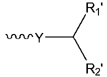
where meanings of Y, R₁' and R₂' are the same as above;
where the method further includes a step of forming tail chains R₁, R₂ and R₃: 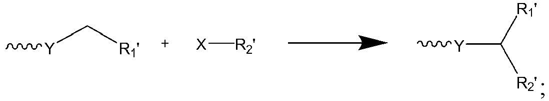
where X is a leaving group.

In a preferred embodiment, the second ionizable lipid is a compound of Formula (2), or a salt, stereoisomer or tautomer thereof; 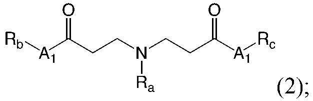
where N₁ is NH or O;
Rₐ is selected from C₆-C₂₄ alkyl, C₆-C₂₄ alkenyl, C₆-C₂₄ cycloalkyl, hydroxyl-substituted C₆-C₂₄ hydrocarbyl, and C₆-C₂₄ short-chain polyoxyethylene; the C₆-C₂₄ alkyl, the C₆-C₂₄ alkenyl, the C₆-C₂₄ cycloalkyl, the hydroxyl-substituted C₆-C₂₄ hydrocarbyl, and the C₆-C₂₄ short-chain polyoxyethylene are of straight-chain or branched structures; R_{b} and R_{c} are each independently selected from C₁-C₁₂ alkyl, C₂-C₁₂ alkenyl, C₁-C₁₂ alkynyl, C₃-C₁₂ cycloalkyl, C₆-C₁₂ aryl, C₁-C₁₂ hydroxyalkyl, C₁-C₁₂ heterocyclyl, and alkylamino;
the alkylamino is 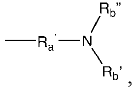 where Ra' is the C₁-C₁₂ alkyl, said R_{b}' and R_{b}" are each independently selected from H, C₁-C₆ alkylamino, 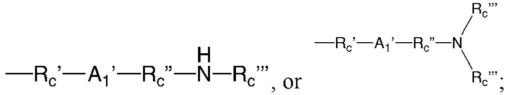 R_{c}" is selected from unsubstituted or amino-substituted C₁-C₆ alkyl, and Rc‴ is H, 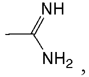 or -R_{c}'-A₁'-R_{c}"-NH₂;
provided that when A₁' is -CO-NH-, -NH-CO- or -CO-O-, R_{c}' is C₁-C₆ alkyl; and when A₁' is -CO-, R_{c}' is absent.

In a preferred embodiment, R_{b} and R_{c} are each independently selected from the C₁-C₁₂ hydroxyalkyl and the alkylamino.

In a preferred embodiment, provided that when Ra is C₆-C₂₄ alkenyl, A₁ is NH, and R_{b} and R_{c} are each independently selected from the C₁-C₁₂ hydroxyalkyl;
when Rₐ is branched C₆-C₂₄ alkyl, A₁ is NH, and R_{b} and R_{c} are each independently selected from the C₁-C₁₂ hydroxyalkyl and the alkylamino;
when Rₐ is straight-chain C₆-C₂₄ alkyl, A₁ is NH or O, and R_{b} and R_{c} are each independently selected from the alkylamino;
when Rₐ is straight-chain C₆-C₂₄ alkyl, A₁ is O, and R_{b} and R_{c} are each independently selected from the C₁-C₁₂ hydroxyalkyl;
when Rₐ is the C₆-C₂₄ short-chain polyoxyethylene, A₁ is NH or O, and R_{b} and R_{c} are each independently selected from the alkylamino;
when Rₐ is C₆-C₂₄ hydroxyalkyl, A₁ is NH or O, and R_{b} and R_{c} are each independently selected from the alkylamino.

In a preferred embodiment, Rₐ is selected from the following compound structures:

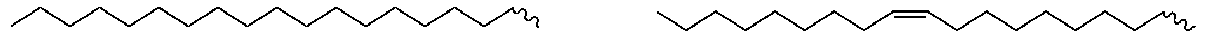

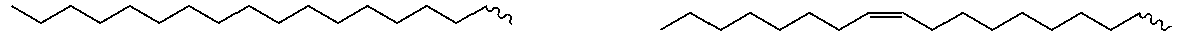

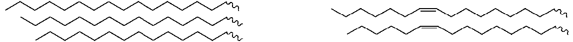

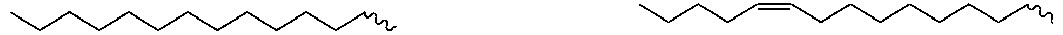

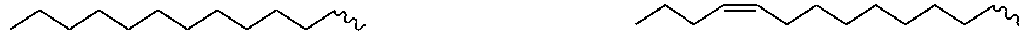

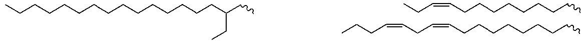

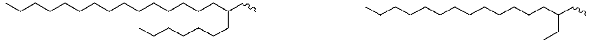

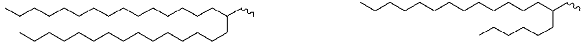

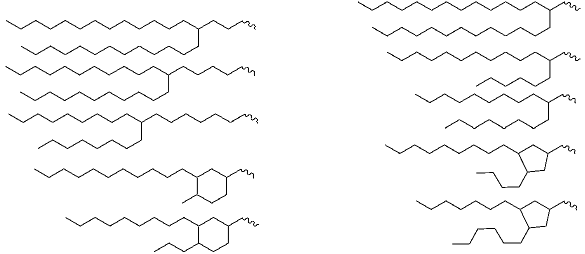

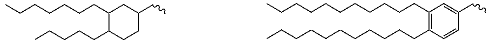

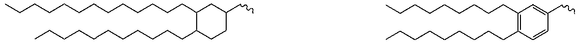

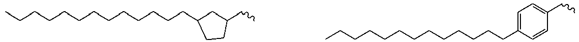

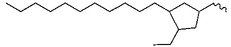

In a preferred embodiment, R_{b} and R_{c} are selected from the following compound structures:

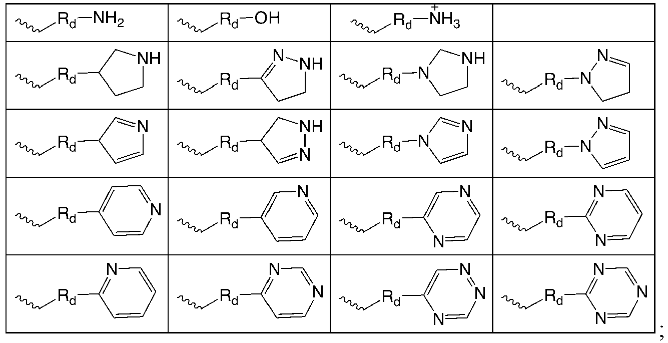

Rₐ is selected from C₁-C₆ alkyl or cycloalkyl.

In a preferred embodiment, the compound of Formula (2) is selected from at least one of the following compounds:

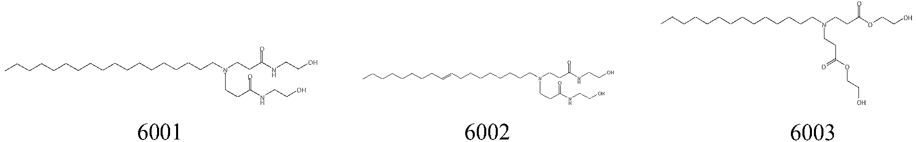

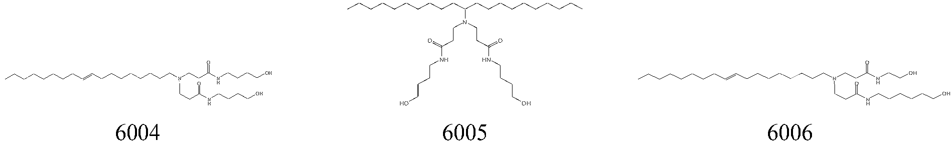

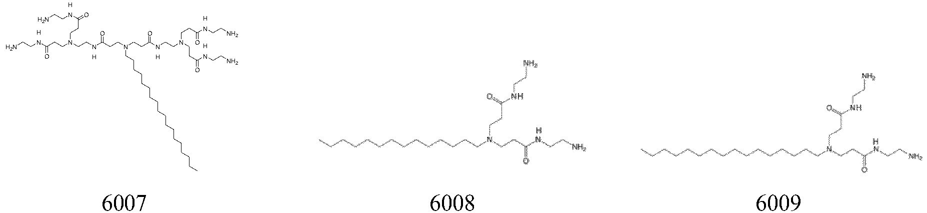

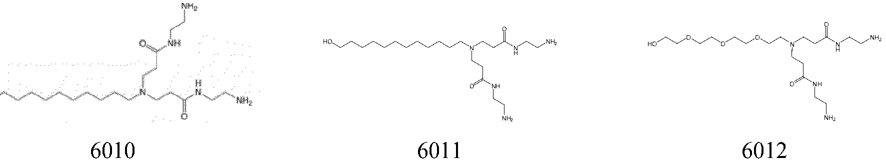

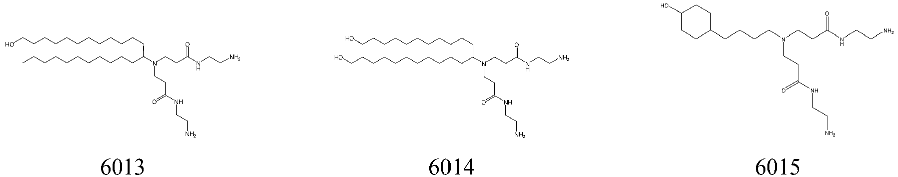

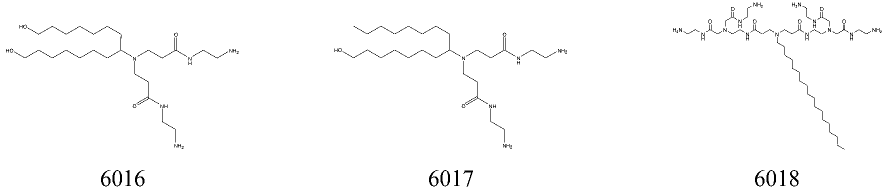

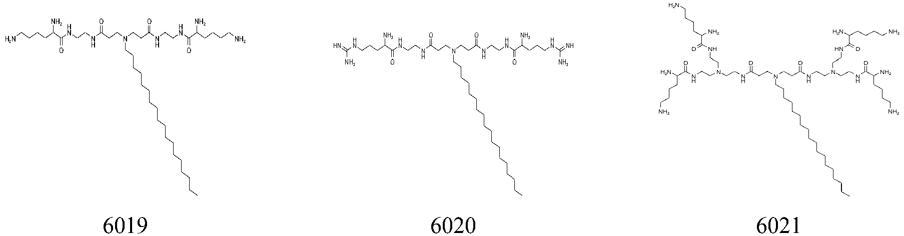

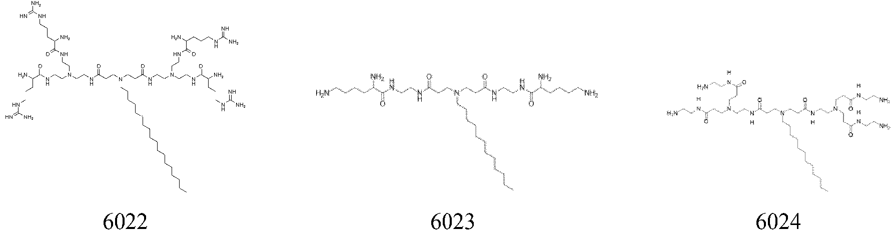

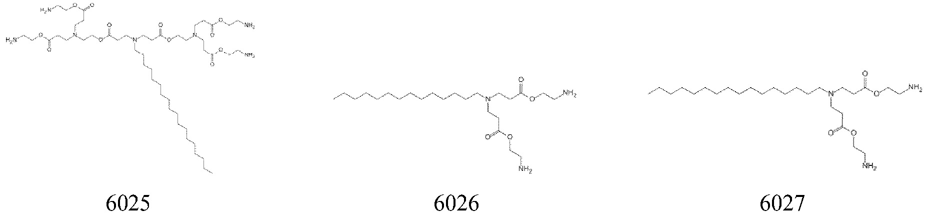

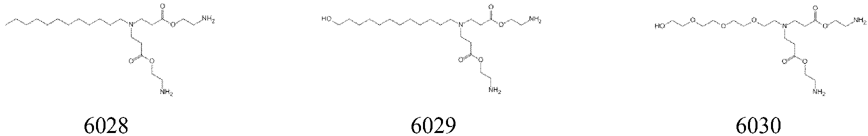

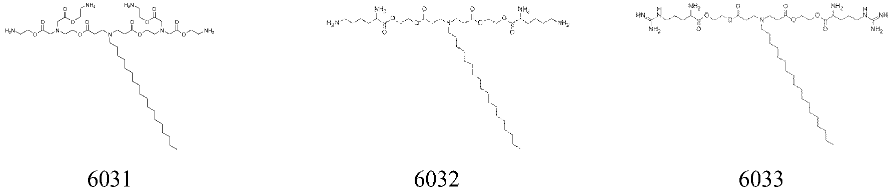

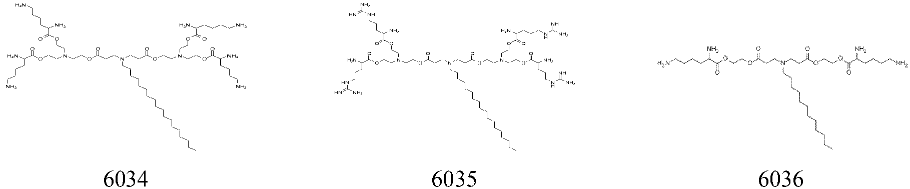

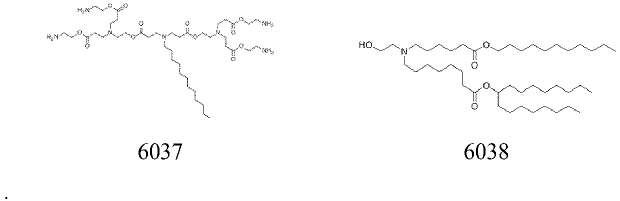

In a preferred embodiment, a method for preparing the second ionizable lipid includes the following reaction steps:
reacting Rₐ"-NH₂ ① with an α,β-unsaturated carbonyl compound ② to generate an ionizable lipid compound ③: 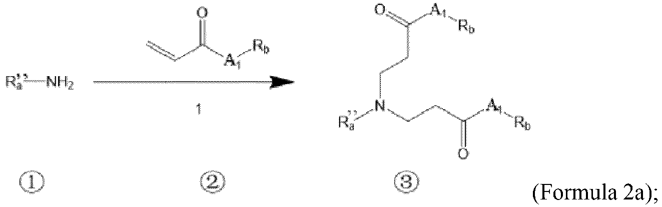
where Rₐ"-NH₂ is Rₐ-NH₂ or 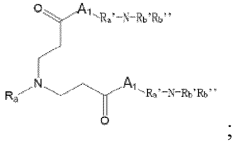
R_{b}' and R_{b}" are H at the same time, or R_{b}' and R_{b}" are C₁-C₆ amino at the same time, or R_{b}' and R_{b}" are -R_{c}'-A₁'-R_{c}"-NH₂ at the same time.

In a preferred embodiment, the method for preparing the second ionizable lipid includes :
1) reacting Rₐ"-NH₂ ① with an α,β-unsaturated carbonyl compound ④ to generate a compound ⑤; and
2) reacting the compound ⑤ with a nucleophile ⑥ to generate an ionizable lipid compound ③; 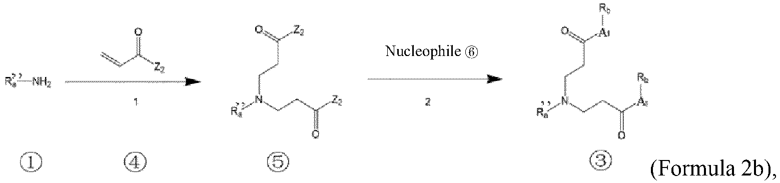
   where the nucleophile ⑥ is R_{b}-NH₂ or R_{b}-OH; Rₐ"-NH₂ is selected from Rₐ-NH₂ or 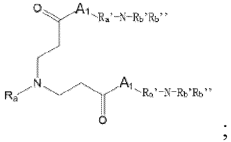
   R_{b}' and R_{b}" are H at the same time, or R_{b}' and R_{b}" are the C₁-C₆ amino at the same time, or R_{b}' and R_{b}" are -R_{c}'-A₁'-R_{c}"-NH₂ at the same time; Z₂ is a leaving group, and Z₂ reacts with NH₂ to obtain A₁.

In a preferred embodiment, the method for preparing the second ionizable lipid includes :
1) reacting Rₐ''-NH₂ ① with the α,β-unsaturated carbonyl compound ② to generate a compound ⑦;
2) reacting the compound (7) with an α,β-unsaturated carbonyl compound ⑧ to generate a compound ⑨; and
3) reacting the compound ⑨ with a nucleophile ⑩ to generate an ionizable lipid compound ⑪; 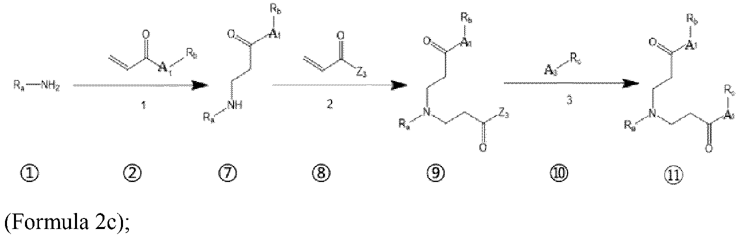 where Rₐ"-NH₂ is Rₐ-NH₂, Z₃ is a leaving group, and A₃ reacts with Z₃ to obtain A₁.

In a preferred embodiment, in Formula 2a-Formula 2c, at least one nucleophile and a carbon atom at position β of at least one α,β-unsaturated carbonyl compound are subjected to a Michael addition reaction to generate the ionizable lipid compound bearing two branches and containing a carbon-carbon bond, a carbon-oxygen bond, a carbon-nitrogen bond, a carbon-sulfur bond, or a carbon-selenium bond.

In a preferred embodiment, the reaction schemes of Formula 2a-Formula 2b further include a step of performing iterative reactions according to Step 1 in Formula 2a or Step 1-Step 2 in Formula 2b by using the compound of Formula (2) with terminal amino groups obtained by the reactions of Formula 2a-Formula 2b as a starting material;
the compound of Formula (2) with the terminal amino groups is 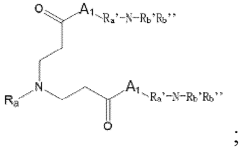
where R_{b}' and R_{b}" are H at the same time, or R_{b}' and R_{b}" are the C₁-C₆ amino at the same time, or R_{b}' and R_{b}" are -R_{c}'-A₁'-R_{c}"-NH₂ at the same time.

In a preferred embodiment, the reaction schemes of Formula 2a-Formula 2c further inlcude a step of reacting the compound of Formula (2) with the terminal amino groups obtained via the reaction schemes of Formula 2a-Formula 2c as a starting material with 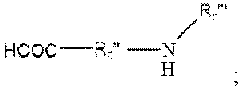
the compound of Formula (2) with the terminal amino groups is 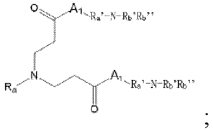
where R_{b}' and R_{b}" are H at the same time, or R_{b}' and R_{b}" are the C₁-C₆ amino at the same time, or R_{b}' and R_{b}" are -R_{c}'-A₁'-R_{c}"-NH₂ at the same time.

The aforementioned leaving groups refer to leaving moieties in the nucleophilic reaction or the condensation reaction, including, but not limited to, H, OH, H₂O, the halogen (e.g., F, Cl, Br, and I), cyanate anions, inorganic acids (e.g., nitric acid, sulfuric acid, and phosphoric acid), carboxylic acids (e.g., acetic acid, trifluoroacetic acid, and benzoic acid), sulfonic acids (e.g., methanesulfonic acid, trifluoromethanesulfonic acid, benzenesulfonic acid, p-toluenesulfonic acid, and p-nitrobenzenesulfonic acid), carbon dioxide (CO₂), nitrogen (N₂), the imidazolyl, the alkoxy (R-O-), the amino (-NHR, where R is alkyl or aryl with H removed), phenoxy, tertiary carbocations (e.g., tert-butyl carbocation), carbocations stabilized by unsaturated systems or heteroatoms, or various protecting groups described above.

The aforementioned nucleophilic groups refer to molecules or ions capable of providing electron pairs to form new chemical bonds in a chemical reaction. Common nucleophilic groups include: hydroxide anion (HO⁻), amino (NH₃), hydroxyamino (NH₂OH), hydrazino (NH₂-NH₂), substituted hydrazino, nucleophilic halogen (e.g., Cl⁻, Br⁻, or I⁻), hydride anion (H⁻), azide anion (N₃⁻), cyanide anion (CN⁻), alcohol moieties or alkoxide anions (e.g., deprotonated alcohol moieties), amino (including primary amino, secondary amino, and tertiary amino) or amide anions, carbanions (e.g., carbanions contained in organometallic reagents such as a Grignard reagent, an organolithium reagent, and a Gilman reagent), sulfydryl or thiolate anions, thioether groups, enol groups or enolate anions, alkenyl ether groups, enamino groups, carboxyl groups or carboxylate anions, alkylphosphino or arylphosphino groups (e.g., a triphenylphosphino group), aromatic heterocyclic groups with lone electron pairs (e.g., pyridyl), and the like.

In a preferred embodiment, starting materials used in a reaction process further contain protecting groups, and a reaction step includes protecting and/or deprotecting steps.

In a preferred embodiment, the prefabricated carrier-based composition further includes 0-60 mol% of other ionizable lipids selected from at least one of the following compounds:

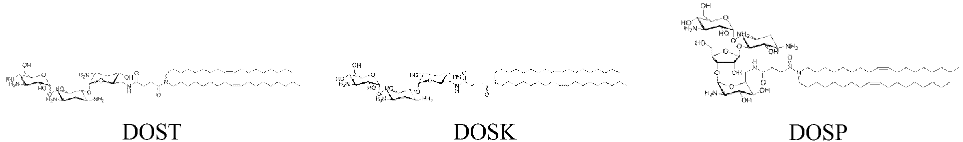

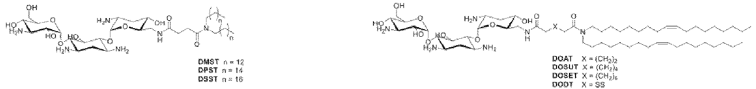

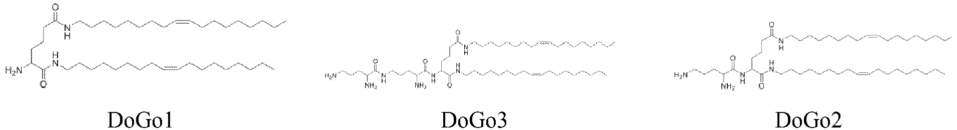

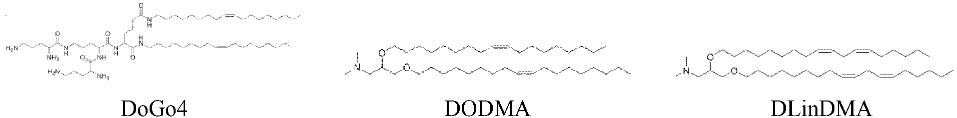

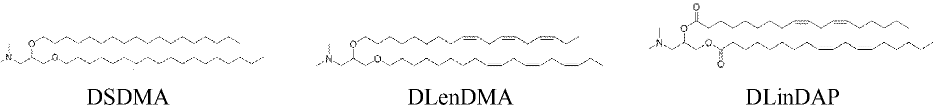

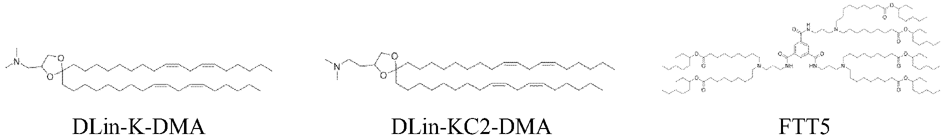

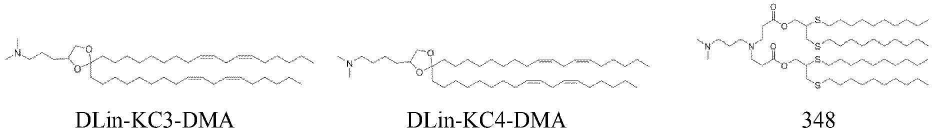

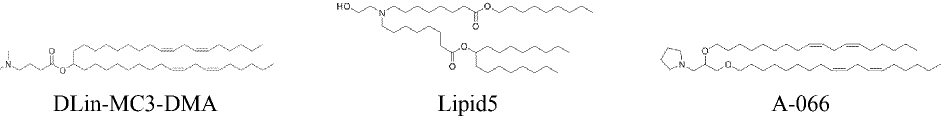

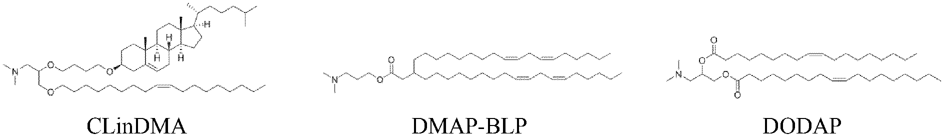

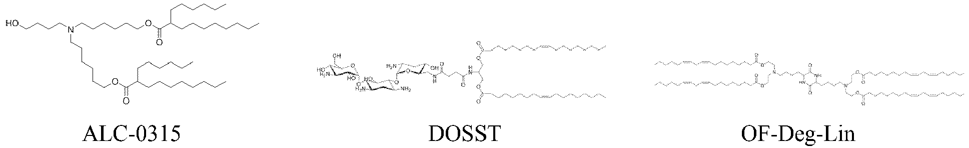

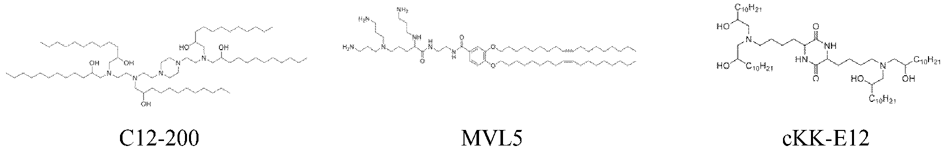

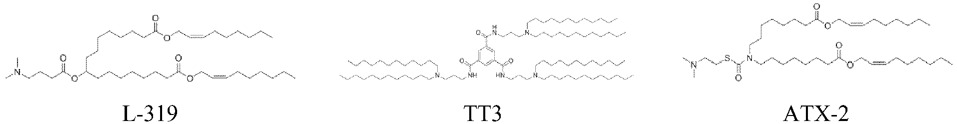

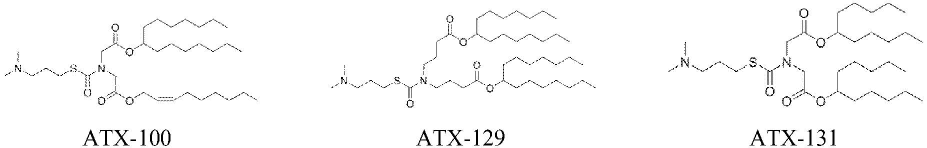

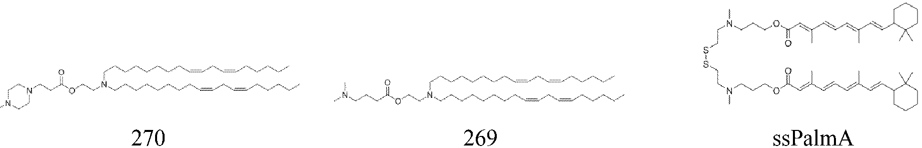

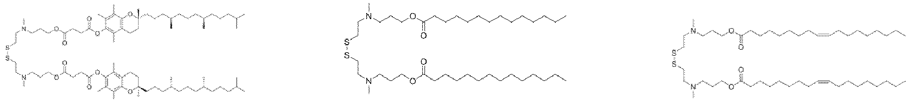

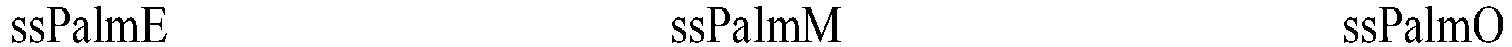

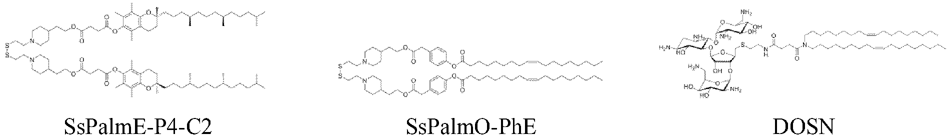

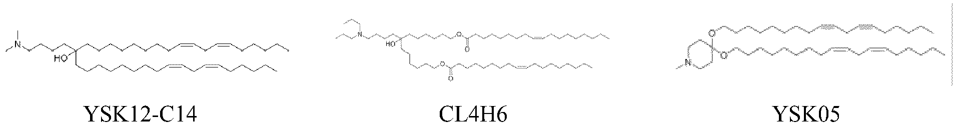

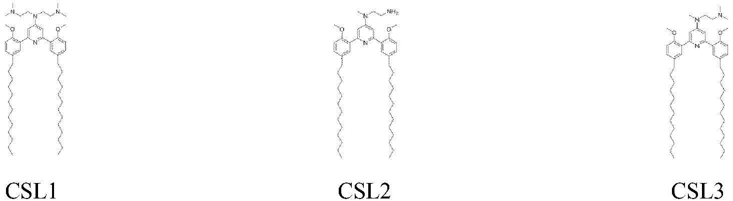

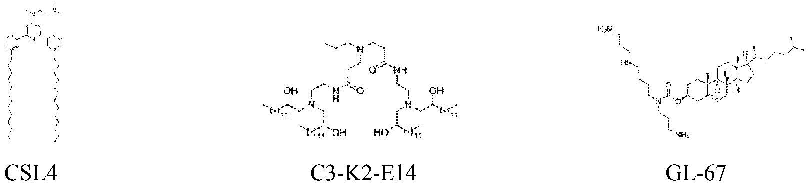

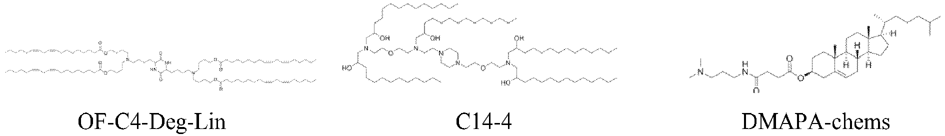

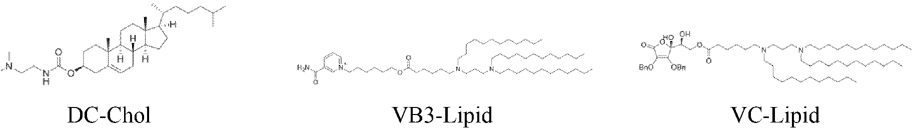

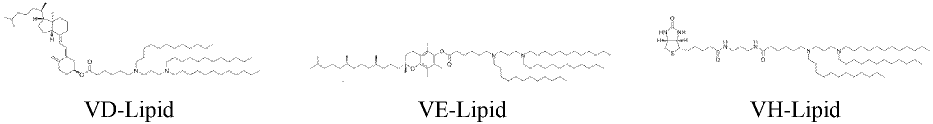

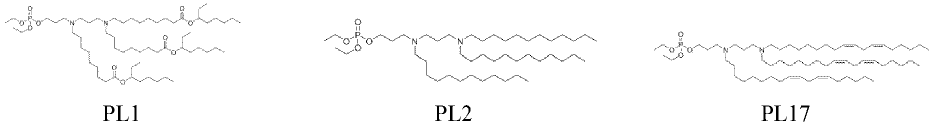

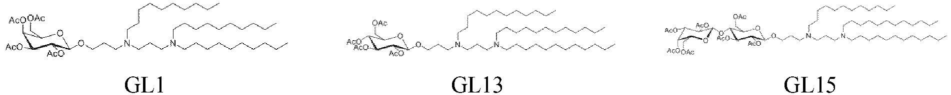

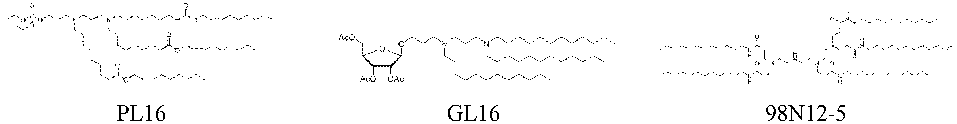

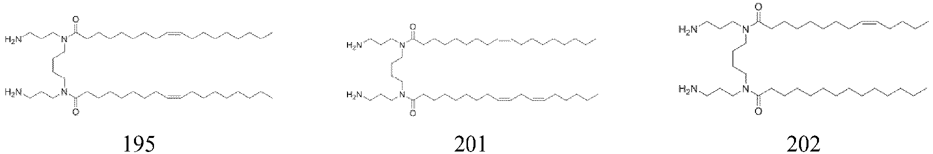

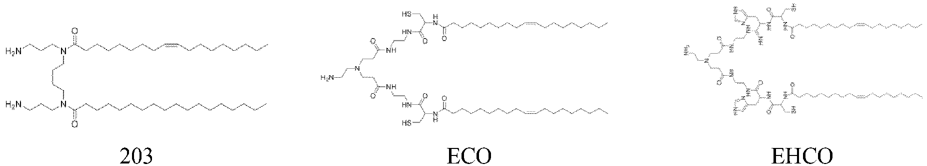

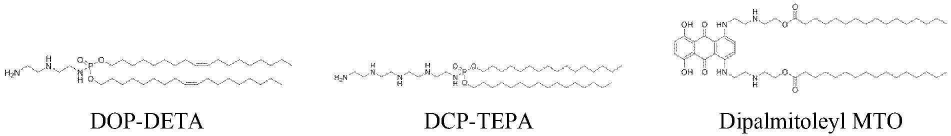

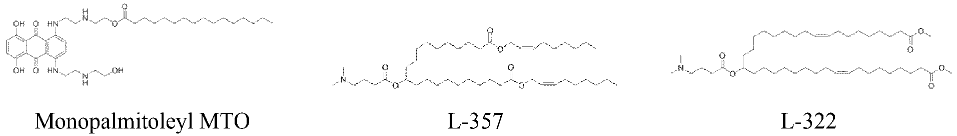

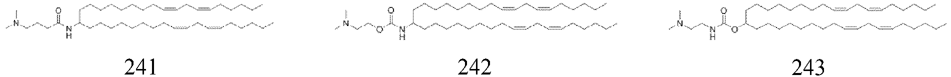

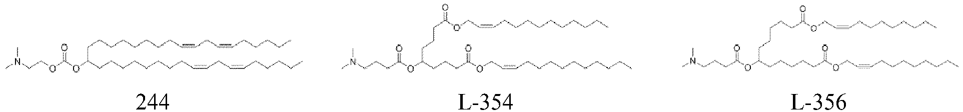

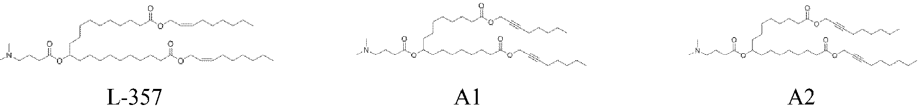

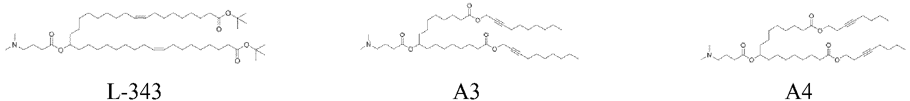

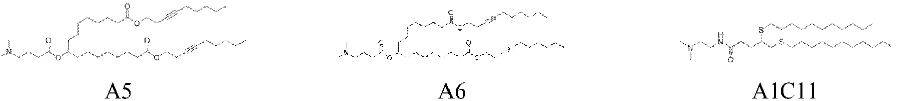

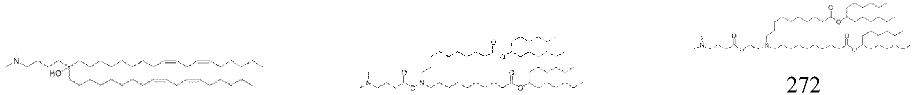

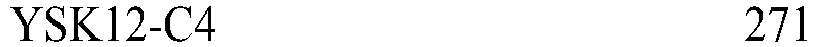

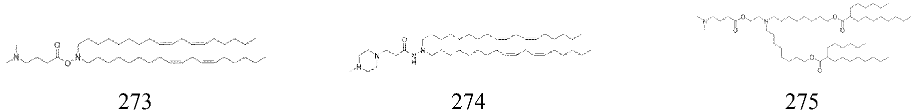

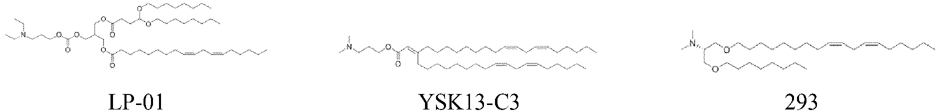

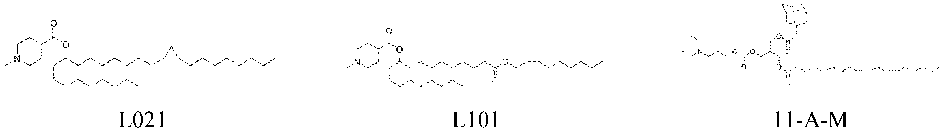

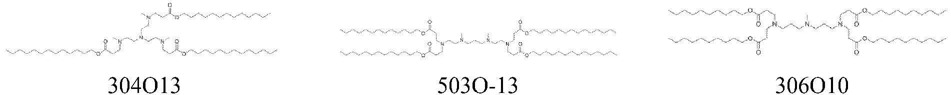

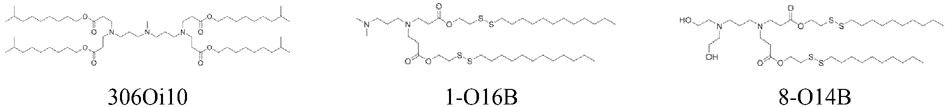

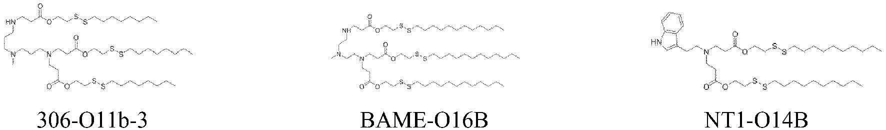

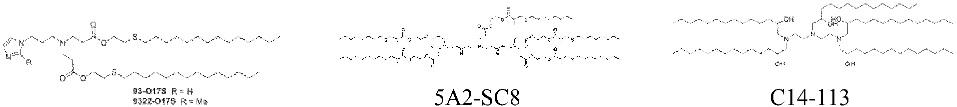

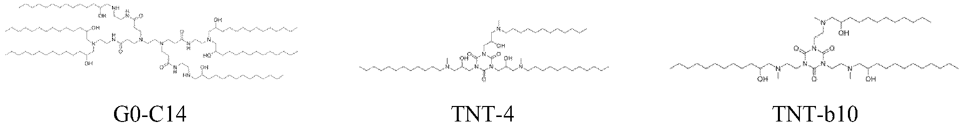

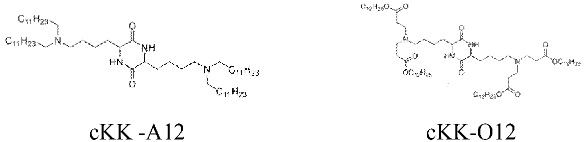

In a preferred embodiment, the phospholipid includes at least one of 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, sodium 1,2-dioleoyl-sn-glycero-3-phospho-rac-(1-glycerol) (DOPG), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphatidylethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, and lysophosphatidylethanolamine (LPE).

In a preferred embodiment, the steroid or the derivative thereof includes at least one of cholesterol, cholesteryl stearate, coprostanol, sitosterol, ergosterol, campesterol, stigmasterol, brassicasterol, dunaliasterol, tomatine, ursolic acid, and α-tocopherol.

In a preferred embodiment, the PEG-conjugated lipid includes at least one of PEGylated phosphatidylethanolamine, PEGylated phosphatidic acid, PEGylated ceramide, PEGylated dialkylamine, PEGylated diacylglycerol, and PEGylated dialkylglycerol.

In a preferred embodiment, the PEG-conjugated lipid includes at least one of PEG-distearyloxypropyl (PEG-DSA), PEG-conjugated dimyristoyl glycerol (PEG-c-DOMG), PEG-dipalmitoylphosphatidylcholine (PEG-DPPC), polyethylene glycol dimethacrylate (PEG-DMA), 1,2-dimethylstyryl-rac-glycero-3-methoxypolyethylene glycol (PEG-PEG), dipalmitoylglycerol-polyethylene glycol (DPG-PEG), 1,2-distearoyl-rac-glycero-3-methoxypolyethylene glycol (DSG-PEG), 2-[(polyethylene glycol)-2000]-N,N-ditetracosylacetamide (ALC-0159), dipalmitoylphosphatidylethanolamine-polyethylene glycol (DPPE-PEG), distearoylphosphatidylethanolamine-polyethylene glycol (PEG-DSPE), dilauroylphosphatidylethanolamine-polyethylene glycol (PEG-DLPE) and dimyristoylphosphatidylethanolamine-polyethylene glycol (PEG-DMPE) lipids.

In a preferred embodiment, in the prefabricated carrier-based composition, the nucleic acid is encapsulated inside the prefabricated carrier and/or adsorbed onto a surface of the prefabricated carrier to form a complex.

In a preferred embodiment, the solvent is at least one of water, an aqueous solution of an organic solvent, and a buffered salt solution.

In a preferred embodiment, a pH value of the buffered salt solution is 1-9, a concentration of the buffered salt solution is 0.1-200 mM, and the aqueous solution of the organic solvent is an alcohol solution with a volume concentration smaller than 50%.

In a preferred embodiment, the alcohol is ethanol.

In a preferred embodiment, the buffered salt solution is selected from at least one of an acitrate solution, an acetate solution, a tartrate solution, a phosphate solution, a carbonate solution, a Tris-HCl solution, and a sodium chloride solution.

In a preferred embodiment, at least one of sugar, glycerol, dimethyl sulfoxide (DMSO), a salt, an antibiotic, and a surfactant is further added to the prefabricated carrier-based composition.

In a second aspect, the present application provides a method for in vitro gene delivery into immune cells and stem cells, including a step of mixing a prefabricated carrier with a nucleic acid in a solvent to obtain a prefabricated carrier-based composition, where the prefabricated carrier inlcudes the following components: 10-60 mol%of a first ionizable lipid, 0-30 mol% of a second ionizable lipid, 5-30 mol% of a phospholipid, 15-70 mol% of a steroid or a derivative thereof, and 0-10 mol% of a PEG-conjugated lipid.

Compared with the prior art, the present application has the following beneficial effects:
1. The use of the prefabricated carrier in the preparation of the product for in vitro delivery to the immune cells and the stem cells according to the present application includes the step of mixing the prefabricated carrier with the nucleic acid in the solvent to obtain the prefabricated carrier-based composition; and the prefabricated carrier includes the ionizable lipid, the phospholipid, the cholesterol, and the PEG-conjugated lipid.
2. The prefabricated carrier used in the present application enables flexible regulation of the nucleic acid dosage according to the user's demand. In addition, it achieves high protein expression efficiency in a variety of cells including human-derived NK cells, human-derived MSC cells, the T cells, and primary T cells, and exhibits desirable delivery performance.
3. The prefabricated carrier of the present application is simple in preparation process, and can be prepared without equipment.

### DETAILED DESCRIPTION OF THE EMBODIMENTS

Synthetic processes of the present application can permit many functional groups, and, therefore, various substituted starting substances can be used. These processes generally afford the desired final compounds at or near the completion of the overall processes; however, in certain instances, the compound may require further conversion to a pharmaceutically acceptable salt thereof. The compounds of the present application can be prepared in a variety of ways using commercially available starting substances, compounds known in literature, or readily prepared intermediates via standard synthetic methods and procedures known to those skilled in the art or readily apparent to those skilled in the art based on the teachings herein. Standard synthetic methods and procedures for preparing organic molecules, as well as functional group conversion and manipulations can be found in relevant scientific literature or standard textbooks in the art. The following descriptions of the synthetic methods are designed to be illustrative rather than limiting of the general procedures for preparing the compounds of the present application.

The compounds of the present application having the various formulas described herein may be prepared from commercially available starting substances or starting substances synthesizable by the literature processes, in accordance with the processes set forth in corresponding general synthetic routes. Variables in each general synthetic route (such as R₁, R₂, and R₃) are as defined herein. Those of ordinary skill in the art will appreciate that an order of certain steps in the reaction procedures and synthetic solutions described herein may be varied, such as introduction and removal of protecting groups.

A variety of stereoisomers may be produced from the reaction solutions described herein. Where no specific stereoisomer is indicated, this should be construed to encompass all stereoisomers obtainable from this reaction. Those of ordinary skill in the art should recognize that said reaction may be optimized to preferentially yield one isomer, or a new solution may be designed to produce a single isomer. If a mixture is produced, the isomers can be separated using technologies such as preparative thin-layer chromatography, preparative HPLC, preparative chiral HPLC, or preparative SFC.

### (1) Preparation example 1 Synthesis of first ionizable lipid

General synthetic routes for a first ionizable lipid are as shown in General synthetic routes 1-5.

### General synthetic route 1

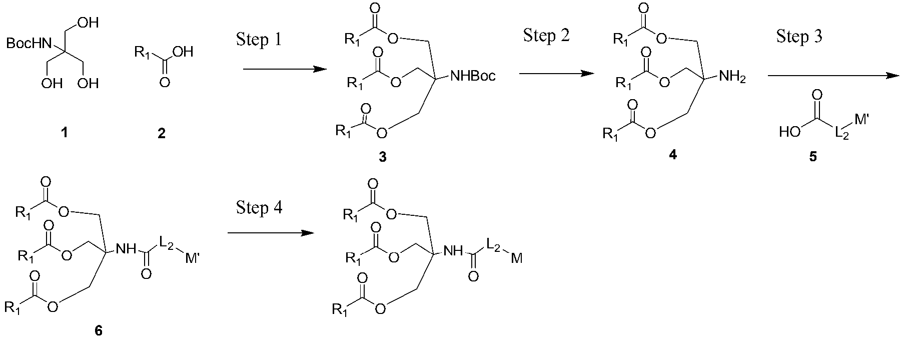

where M' is M or M containing a protecting group.

As illustrated in the above General synthetic route **1,** Boc-aminotris(hydroxymethyl)methane and an acid (compound 2) underwent a condensation reaction to afford compound 3. Step 1 might be carried out in an organic solvent (e.g., dichloromethane (DCM)) in the presence of, for example, 1-ethyl-(3-dimethylaminopropyl)carbodiimide hydrochloride (EDCI) and 4-dimethylaminopyridine (DMAP). Step 1 might be carried out at a room temperature for 24 h.

Subsequently, compound 3 was deprotected to remove a Boc protecting group to afford compound 4. Step 2 might be carried out in an organic solvent (e.g., DCM) under catalysis of an acid (e.g., trifluoroacetic acid).

Subsequently, compound 4 and compound 5 underwent a condensation reaction to afford compound 6. Step 3 might be carried out in an organic solvent (e.g., the DCM or DMF) under catalysis of the EDCI and the DMAP, or dicyclohexylcarbodiimide (DCC).

If the M' group in compound 6 contained the aforementioned protecting group, the protecting group was removed, and a target lipid compound was afforded. Step 4 was carried out under deprotection reaction conditions for the selected protecting group.

### General synthetic route 2

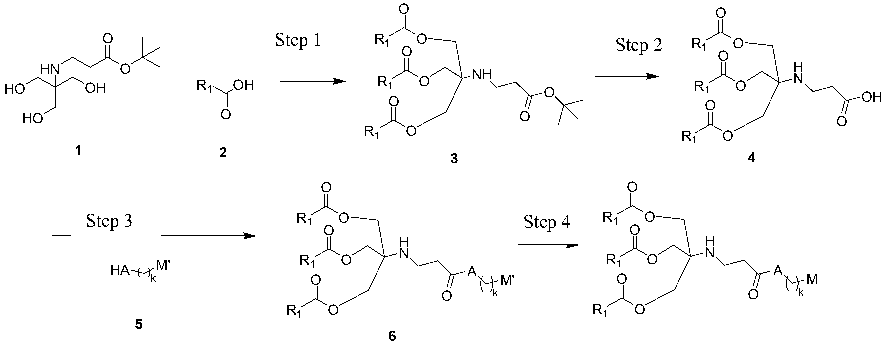

where M' is M or M containing a protecting group; and A is O, NH, or S.

As illustrated in the above General synthetic route 2, compound 1 and compound 2 underwent a condensation reaction to afford compound 3. Step 1 might be carried out in an organic solvent (e.g., the DCM) in the presence of the EDCI and the DMAP.

Subsequently, compound 3 was deprotected to remove the Boc protecting group to afford Compound 4. Step 2 was carried out in an organic solvent (e.g., DCM) in the presence of the acid (e.g., the trifluoroacetic acid) and a cation scavenger (e.g., triisopropylsilane (TiPS)).

Subsequently, compound 4 and compound 5 underwent the condensation reaction to afford compound 6. Step 3 might be carried out in the organic solvent (e.g., the DCM or the DMF) in the presence of the EDCI and the DMAP, or the DCC.

If the M' group in compound 6 contained the aforementioned protecting group, the protecting group was removed, and a target lipid compound was afforded. Step 4 was carried out under the deprotection reaction conditions for the selected protecting group.

### General synthetic route 3

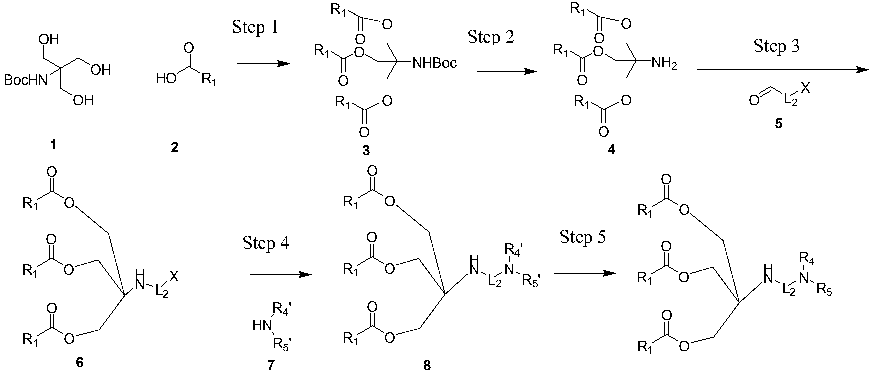

where X is halogen, such as Cl, Br, or I; R₄' is R₄ or R₄ containing a protecting group; and R₅' is R₅ or R₅ containing a protecting group.

As illustrated in the above General synthetic route 3, the Boc-aminotris(hydroxymethyl)methane and compound 2 underwent the condensation reaction to afford compound 3. Step 1 might be carried out in the organic solvent (e.g., the DCM) in the presence of, for example, the EDCI and the DMAP. Step 1 might be carried at the room temperature for 24 h.

Subsequently, compound 3 was deprotected to remove the Boc protecting group to afford compound 4. Step 2 might be carried out in the organic solvent (e.g., the DCM) under catalysis of the acid (e.g., the trifluoroacetic acid).

Subsequently, compound 4 and a halogen-substituted aldehyde (compound 5) underwent the condensation reaction and a reduction reaction to afford compound 6. Step 3 might be carried out in an organic solvent (e.g., dichloroethane (DCE)) in the presence of a reductant (e.g., sodium triacetoxyborohydride (NaBH(OAc)₃)).

Subsequently, compound 6 and an amine (compound 7) underwent a nucleophilic reaction to afford compound 8. Step 4 might be carried out in the organic solvent (e.g., the DMF) in the presence of a base (e.g., a non-nucleophilic organic base (e.g., triethylamine, iPr2EtN) or an inorganic base (e.g., K₂CO₃)) and a catalyst (KI or NaI).

If the R4' and/or R₅' group in Compound 8 contained the aforementioned protecting group, the protecting group was removed, and a target lipid compound was afforded. Step 5 was carried out under the deprotection reaction conditions for the selected protecting group.

### General synthetic route 4

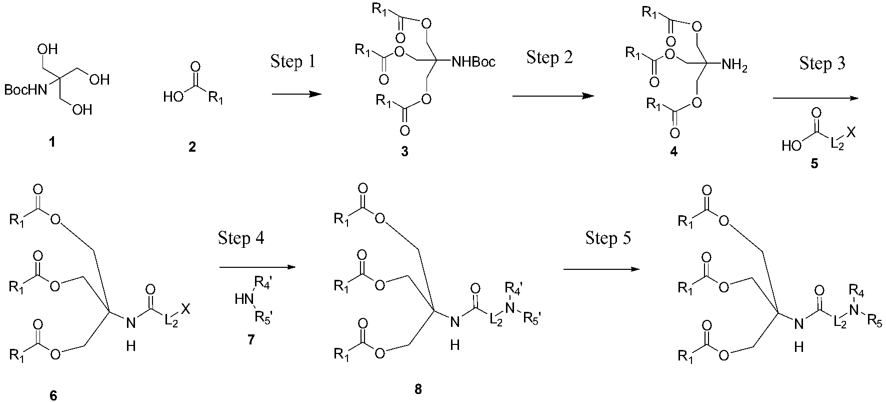

where X is the halogen, such as Cl, Br or I; R₄' is R₄ or R₄ containing a protecting group; and R₅' is R₅ or R₅ containing a protecting group.

As illustrated in the above General synthetic route 4, the Boc-aminotris(hydroxymethyl)methane and compound 2 underwent the condensation reaction to afford compound 3. Step 1 might be carried out in the organic solvent (e.g., the DCM) in the presence of, for example, the EDCI and the DMAP. Step 1 might be carried at the room temperature for 24 h.

Subsequently, compound 3 was deprotected to remove the Boc protecting group to afford compound 4. Step 2 might be carried out in the organic solvent (e.g., the DCM) under catalysis of the acid (e.g., the trifluoroacetic acid).

Subsequently, compound 4 and a halogen-substituted compound 5 underwent the condensation reaction to afford compound 6. Step 3 might be carried out in the organic solvent such as the DCE or the DMF through catalysis of the EDCI and the DMAP, or the DCC.

Subsequently, compound 6 and an amine (Compound 7) underwent the nucleophilic reaction to afford compound 8. Step 4 might be carried out in the organic solvent (e.g., the DMF) in the presence of the base (e.g., the non-nucleophilic organic base (e.g., the triethylamine, iPr2EtN) or the inorganic base (e.g., K₂CO₃)) and the catalyst (KI or NaI).

If the R₄' and/or R₅' group in compound 8 contained the aforementioned protecting group, the protecting group was removed, and a target lipid compound was afforded. Step 5 was carried out under the deprotection reaction conditions for the selected protecting group.

### General synthetic route 5

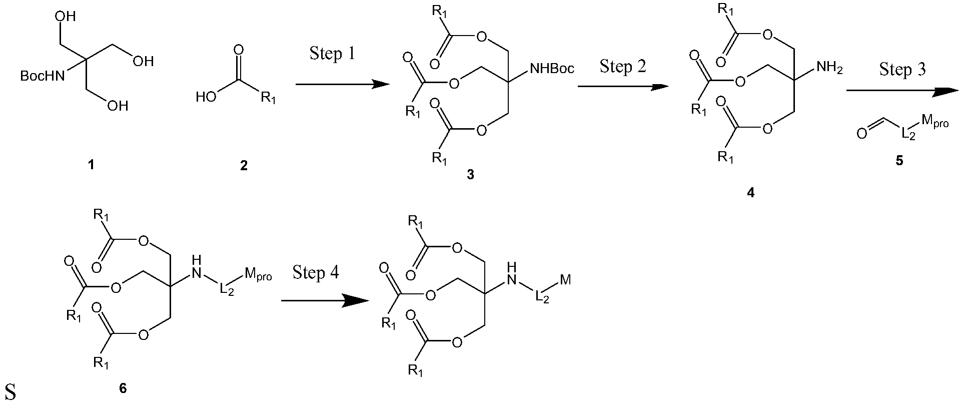

where Mₚᵣₒ is M or M containing a protecting group.

As illustrated in the above General synthetic route 5, the Boc-aminotris(hydroxymethyl)methane and compound 2 underwent the condensation reaction to afford compound 3. Step 1 might be carried out in the organic solvent (e.g., the DCM) in the presence of, for example, the EDCI and the DMAP. Step 1 might be carried at the room temperature for 24 h.

Subsequently, compound 3 was deprotected to remove the Boc protecting group to afford compound 4. Step 2 might be carried out in the organic solvent (e.g., the DCM) in the presence of the acid (e.g., the trifluoroacetic acid).

Subsequently, compound 4 and compound 5 underwent the condensation reaction and the reduction reaction to afford compound 6. Step 3 might be carried out in the organic solvent (e.g., the dichloroethane (DCE)) in the presence of the reductant (e.g., the sodium triacetoxyborohydride (NaBH(OAc)₃)).

If the Mₚᵣₒ group in compound 6 contained the aforementioned protecting group, the protecting group was removed, and a target lipid compound was afforded. Step 4 was carried out under the deprotection reaction conditions for the selected protecting group.

In addition, it should also be understood that any specific embodiment of the present application within the scope of the prior art may be explicitly excluded from any one or more claims. Since these embodiments are deemed known to those of ordinary skill in the art, they may be excluded even if such exclusion is not explicitly set forth herein.

All cited sources, such as references, publications, databases, database entries, and technologies cited herein, are incorporated by reference into the present application, even where such incorporation is not explicitly stated in the citation. In the event of any conflict between the statements in the cited sources and those of the present application, the statements of the present application shall prevail.
**(1)** A compound of Formula (1), (1A), (1B), (1C), (1D), (1E), (1F), (1G), (1H), (1I), (1J), (1K), (1L), or (1M) was synthesized.

### A. General consideration

It is worth noting that all starting materials used in the present application are common commercially available products, and no specific limitations are imposed on their sources.

The process routes described below may be used to synthesize compounds 1001-3422 of the present application.

Abbreviations used herein are as follows:
THF: Tetrahydrofuran
MeCN: acetonitrile
MeOH: methanol
PE: petroleum ether
EA: ethyl acetate
DMF: N,N-dimethylformamide
EDCI: 1-ethyl-(3-dimethylaminopropyl)carbodiimide hydrochloride
LAH: lithium aluminum hydride
DCM: dichloromethane
DMAP: 4-dimethylaminopyridine
LDA: lithium diisopropylamide
rt: room temperature
DCE: 1,2-dichloroethane
n-BuLi: n-butyllithium
i-Pr₂ EtN: N,N-diisopropylethylamine

### B. Synthesis of intermediates

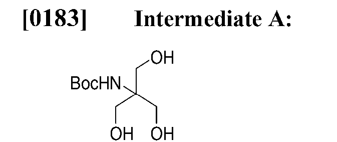

Intermediate A was obtained by using the following synthetic process route:

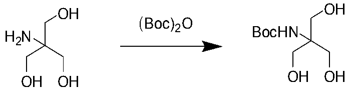

Tris(hydroxymethyl)aminomethane (50.0 g) and di-tert-butyl dicarbonate (Boc₂O) (99.1 g) were dissolved into a mixed solvent of methanol (300 mL)/H₂O (30 mL). A mixture underwent a reaction at the room temperature for 72 h, followed by purification via silica gel column chromatography (DCM: MeOH = (20:1)-(10:1)) to afford a white solid which was intermediate A (N-Boc-tris(hydroxymethyl)aminomethane) (75.0 g). LCMS (ESI) calcd for C₉H₁₉NO₅, [M+H]⁺ m/z 222.13, found 222.25.

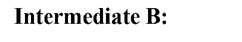

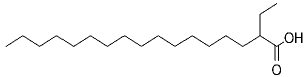

**Intermediate B** was obtained by using the following synthetic process route:

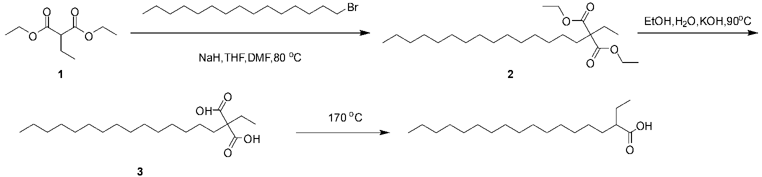

Diethyl 2-ethylpropane-1,3-dioate (29.0 g), THF (90 mL) and DMF (30 mL) were added to a single-neck flask. NaH (3.7 g) was added under an ice bath, and a mixture was stirred at the room temperature under a nitrogen atmosphere for 30 min. Pentadecyl bromide (15.0 g) was then added, and a mixture underwent a reaction at 80°C under a nitrogen atmosphere for 2 h. THF was removed by concentration under reduced pressure. The reaction mixture was added dropwise to ice water, ethyl acetate was added for stirring, and a resulting mixture was stirred and filtered through celite. A filtrate was allowed to stand for phase separation. An organic phase was collected, separated, washed with saturated brine, and then purified via silica gel column chromatography (PE: EA = (100:1)-(50:1)) to afford a compound which was diethyl 2-ethyl-2-pentadecylmalonate (16.5 g). EtOH (50 mL), H₂O (50 mL), and KOH (11.3 g) were added to the diethyl 2-ethyl-2-pentadecylmalonate (8.0 g) for reaction at 90°C for 12 h. The EtOH was removed by concentration under reduced pressure. Dilute hydrochloric acid was added to regulate a pH value of the reaction system to 4-5, followed by addition of water and ethyl acetate. The mixture was stirred and underwent phase separation. The organic phase was washed with saturated brine and separated. The separated organic phase was purified via silica gel column chromatography (PE: EA = (5:1)-(1:1)) to afford 2-ethyl-2-pentadecylmalonic acid (5.2 g). The 2-Ethyl-2-pentadecylmalonic acid (10.0 g) was stirred at 170°C for 6 h under an opening condition, and then cooled to the room temperature. The water and the ethyl acetate were added for stirring and phase separation. The organic phase was washed with saturated brine, separated, and then concentrated. A crude product was purified via silica gel column chromatography (DCM: MeOH = (20:1)-(10:1)) to afford intermediate B (2-ethylheptadecanoic acid) (8.2 g). ¹H NMR (400 MHz, CDCl₃) δ 2.31 (tt, J = 8.6, 5.3 Hz, 1H), 1.71-1.46 (m, 4H), 1.28 (s, 26H), 0.96 (t, J = 7.4 Hz, 3H), 0.90 (t, J = 6.7 Hz, 3H).

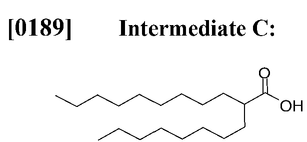

**Intermediate C** was obtained by using the following synthetic process route:

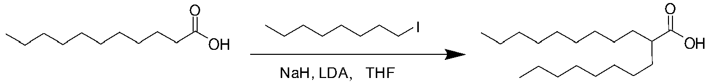

Decanoic acid (50 g) and a solvent (THF) (500 mL) were added to a three-necked flask. The system was cooled to 0°C, and then NaH (23.22 g) as a reactant was slowly added for stirring at 0°C under a nitrogen atmosphere for 1 h; and lithium diisopropylamide (LDA) (62.19 g) was added dropwise slowly for further stirring at 0°C under the nitrogen atmosphere for another 1 h. Finally, nonyl iodide (88.52 g) as a reactant was added dropwise, and a mixture was heated to the room temperature and stirred overnight. The reaction mixture was diluted with 1 L of the DCM, and then washed sequentially with a saturated NH₄Cl solution and the water. An organic layer was dried over anhydrous Na₂SO₄, filtered and concentrated. A crude product was purified via silica gel column chromatography (PE: EA = 50:1) to finally afford intermediate C (2-octylundecanoic acid) (11 g). ¹H NMR (400 MHz, Chloroform-d) δ 2.37 (tt, J = 8.7, 5.1 Hz, 1H), 1.71-1.58 (m, 2H), 1.48 (dt, J = 13.3, 6.7 Hz, 2H), 1.29 (d, J = 9.4 Hz, 25H), 0.90 (t, J = 6.8 Hz, 6H).

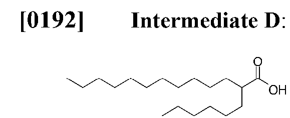

**Intermediate D** was obtained by using the following synthetic process route:

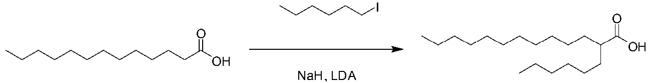

Tridecanoic acid (79.5 g) and a solvent (THF) (800 mL) were added to a three-necked flask. The system was cooled to 0°C, and then NaH (22.25 g) was added slowly. After a reaction mixture was stirred at 0°C under the nitrogen atmosphere for 1 h, the LDA (317.87 g) was added dropwise slowly to the reaction system. The reaction mixture was further stirred at 0°C under the nitrogen atmosphere for another 1 h. n-Hexyl iodide (94.39 g) as a reactant was added dropwise, and then a mixture was heated to the room temperature and stirred overnight. The reaction mixture was diluted with 1 L of the DCM and washed sequentially with a saturated NH₄Cl solution and the water. An organic layer was dried over anhydrous Na₂SO₄, filtered and concentrated. A crude product was purified via silica gel column chromatography (PE: EA = 50:1) to afford intermediate D (2-hexyltridecanoic acid) (26 g). ¹H NMR (400 MHz, Chloroform-d) δ 2.36 (tt, J = 8.7, 5.4 Hz, 1H), 1.63 (ddd, J = 14.3, 8.7, 5.5 Hz, 2H), 1.49 (dq, J = 13.5, 6.6 Hz, 2H), 1.29 (d, J = 10.1 Hz, 26H), 0.90 (t, J = 6.6 Hz, 6H)

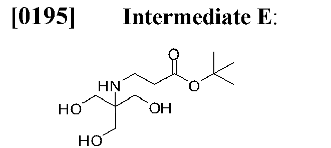

**Intermediate E** was obtained by using the following synthetic process route:

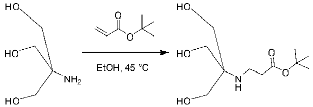

Tris(hydroxymethyl)aminomethane (10.0 g) and tert-butyl acrylate (21.1 g) were dissolved into the EtOH (150 mL). A mixture underwent a reaction at 45°C under the nitrogen atmosphere for 30 h. The EtOH was removed by concentration under reduced pressure. A residue was slurried with a solvent (PE: EA = 20:1, 100 mL × 3) and then filtered to afford a white solid which was intermediate E (11.5 g). ¹H NMR (400 MHz, CDCl₃) δ 3.60 (s, 6H), 2.84 (t, J = 5.9 Hz, 2H), 2.47 (t, J = 5.8 Hz, 2H), 1.48 (s, 9H).

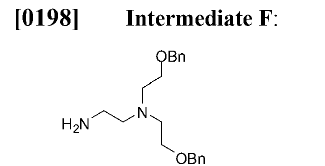

**Intermediate F** was obtained by using the following synthetic process route:

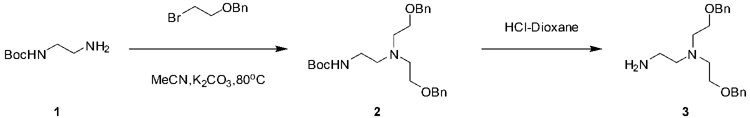

Tert-Butyl (2-aminoethyl)carbamate (2.7 g), MeCN (90 mL), benzyl 2-bromoethyl ether (7.99 g), and K₂CO₃ (11.65 g) were added to a single-neck flask. A mixture underwent a reaction at 80°C overnight. The water and the ethyl acetate were added for stirring and phase separation. An organic phase was washed with the saturated brine, separated and concentrated. A crude product was purified via silica gel column chromatography (PE: EA = (10:1)-(5:1)) to afford compound 2 (5.5 g). Dioxane (30 mL) and a hydrogen chloride in dioxane solution (30 mL) were added to compound 2. A mixture was stirred at room temperature for 3 h, and then concentrated under reduced pressure to afford compound 3, i.e., **intermediate F** (6 g). LCMS (ESI) calcd for C₂₀H₂₈N₂O₂, [M+H]⁺ m/z 329.22, found 329.24.

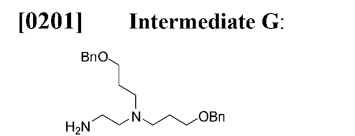

**Intermediate G** was obtained by using the following synthetic process route:

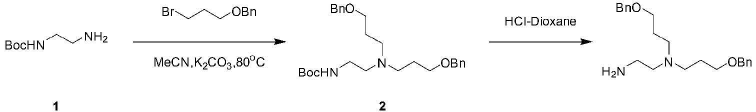

Compound 1 (3.0 g), MeCN (90 mL), 3-benzyloxypropyl bromide (9.45 g), and K₂CO₃ (12.94 g) were added to a single-neck flask. A mixture underwent a reaction at 80°C overnight. The water and the ethyl acetate were added for stirring and phase separation. An organic phase was washed with the saturated brine, separated and concentrated. A crude product was purified via silica gel column chromatography (PE: EA = (10:1)-(5:1)) to afford compound 2 (6.9 g). The dioxane (30 mL) and the hydrogen chloride in dioxane solution (30 mL) were added to compound 2 (6.9 g). A mixture was stirred at the room temperature for 3 h and then concentrated under reduced pressure to afford intermediate G (7.5 g). LCMS (ESI) calcd for C₂₂H₃₂N₂O₂, [M+H]⁺ m/z 357.25, found 357.51.

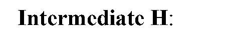

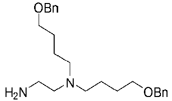

**Intermediate H** was obtained by using the following synthetic process route:

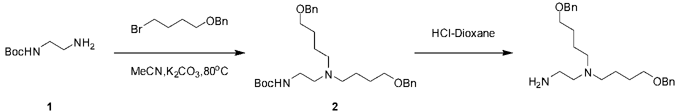

Compound **1** (1.5 g), MeCN (90 mL), benzyl 4-bromobutyl ether (5.02 g), and K₂CO₃ (6.47 g) were added to a single-neck flask. A mixture underwent a reaction at 80°C overnight. The cater and the ethyl acetate were added for stirring and phase separation. An organic phase was washed with the saturated brine, separated and concentrated. A crude product was purified via column chromatography (PE: EA = (20:1)-(10:1)) to afford compound 2 (4.1 g). The dioxane (30 mL) and the hydroxide chloride in dioxane solution (30 mL) were added to compound 2 (4.1 g). A mixture was stirred at the room temperature for 3 h and then concentrated under reduced pressure to afford intermediate H (4.9 g). LCMS (ESI) calcd for C₂₄H₃₆N₂O₂, [M+H]⁺ m/z 385.28, found 385.56.

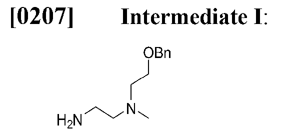

**Intermediate I** was obtained by using the following synthetic process route:

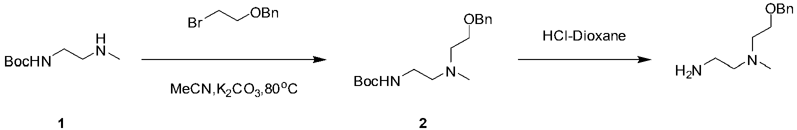

Compound **1** (3.9 g), MeCN (90 mL), benzyl 2-bromoethyl ether (5.78 g), and K₂CO₃ (15.46 g) were added to a single-neck flask. A mixture underwent a reaction at 80°C overnight. The water and the ethyl acetate were added for stirring and phase separation. An organic phase was washed with the saturated brine, separated and concentrated. A crude product was purified via column chromatography (PE: EA = (20:1)-(10:1)) to afford compound **2** (6.8 g). The dioxane (30 mL) and the hydrogen chloride in dioxane solution (30 mL) were added to xompound **2** (6.8 g). A mixture was stirred at the room temperature for 3 h and then concentrated under reduced pressure to afford intermediate I (6.5 g). LCMS (ESI) calcd for C₁₂H₂₀N₂O, [M+H]⁺ m/z 209.16, found 209.31.

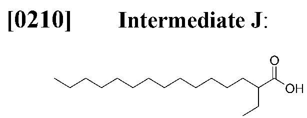

**Intermediate J** was obtained by using the following synthetic process route:

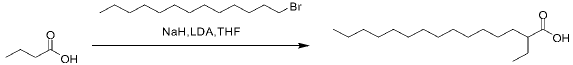

Butyric acid (5.0 g) and THF (100 mL) were added to a single-neck flask. NaH (2.73 g) was added at 0°C, followed by slow dropwise addition of the LDA (56.8 mL). A mixture underwent a reaction at the room temperature for 30 min, 1-bromotridecane was then added for further stirred at the room temperature overnight. Subsequently, ice water and the ethyl acetate were added for stirring and phase separation. An organic phase was washed with the saturated brine, separated and concentrated. A crude product was purified via column chromatography (PE: EA = (10:1)-(5:1)) to afford **intermediate J** (5.0 g). LCMS (ESI) calcd for C₁₇H₃₄O₂, [M+H]⁺ m/z 271.26, found 271.46.

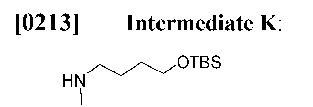

**Intermediate K** was obtained by using the following synthetic process route:

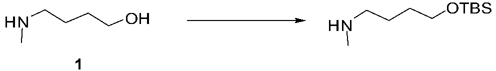

Reactant 1 (24 g), imidazole (19.00 g), and a solvent (the DCM) (200 mL) were added to a three-neck flask. A mixture was cooled to 0°C, and then TBDMSCI (38.57 g) as a reactant was added slowly for stirring at 25°C for 4 h, and a mixture was diluted with 300 mL of the DCM. An organic phase was washed twice with 1 L of the water, dried over anhydrous Na₂SO₄, filtered and concentrated. A resultant was purified via silica gel column chromatography (DCM: MeOH = 1:20). A sample was collected and concentrated to afford **intermediate K** (28.00 g). ¹H NMR (400 MHz, CDCl₃) δ 4.89 (s, 1H), 3.58 (t, J = 6.1 Hz, 2H), 2.66 (t, J = 7.3 Hz, 2H), 2.45 (s, 3H), 1.67-1.40 (m, 4H), 0.84 (s, 9H).

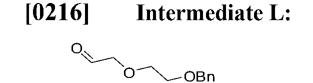

**Intermediate L** was obtained by using the following synthetic process route:

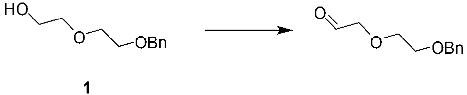

DMSO (3.58 g) and anhydrous DCM (30 mL) were added to a three-neck flask. The system was cooled to -78°C, and then oxalyl chloride (2.91 g) was added slowly. A reaction mixture was stirred at -78°C under a N₂ atmosphere for 10 min, followed by slow dropwise addition of compound 1 (3 g). A mixture was further stirred at -78°C under the N₂ atmosphere for another 1 h, and then TEA (9.28 g) was added dropwise for stirring at -78°C under the N₂ atmosphere for another 0.5 h. A mixture was diluted with 100 mL of the DCM, and then washed sequentially with a saturated NH₄Cl aqueous solution and the water. An organic layer was dried over anhydrous Na₂SO₄, filtered and concentrated to afford **intermediate L** (3.0 g). LCMS (ESI) calcd for C₁₁H₁₄O₃, [M+H]⁺ m/z 195.09, found 195.23.

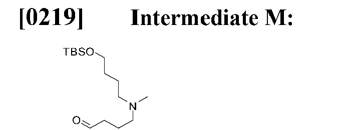

**Intermediate M** was obtained by using the following synthetic process route:

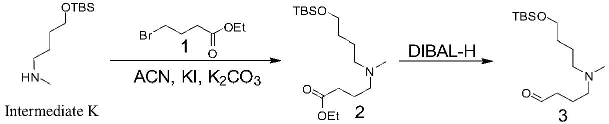

**Intermediate K** (2.0 g), compound 1 (1.79 g), and ACN (20 mL) were added to a sealed tube. K₂CO₃ (3.81 g) and KI (1.52 g) as reactants were added subsequently. for stirring at 70°C overnight under the N₂ atmosphere. A mixture was then diluted with 100 mL of EA, and washed sequentially with the saturated NH₄Cl aqueous solution and the water. An organic phase was dried over anhydrous Na₂SO₄, filtered and concentrated. A crude product was purified via silica gel column chromatography with DCM/MeOH = 15:1. A sample was collected and concentrated to afford compound 2 (2.0 g). Reactant 2 (1.80 g) and a solvent (the DCM) (20 mL) were added to a three-neck flask. The system was purged with nitrogen and then cooled to -78°C. DIBAL-H (1.00 g) was then added dropwise, and a mixture was stirred at -78°C for 4 h. Subsequently, a reaction mixture was quenched by dropwise addition of methanol and a sodium carbonate solution, followed by extraction with the DCM (100 mL). An organic layer was dried over anhydrous sodium sulfate, filtered and concentrated to afford intermediate M (1.36 g). LCMS (ESI) calcd for C₁₅H₃₃NO₂Si, [M+H]⁺ m/z 288.52, found 288.23.

### C. Compound 1003, synthesized via General synthetic route 1

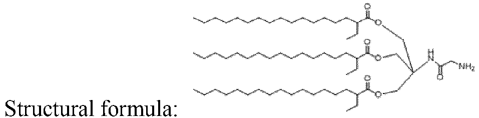

Chemical formula: C₆₃H₁₂₂N₂O₇
Molecular weight: 1019.68

### Step 1: Synthesis of compound 3 in General synthetic route 1

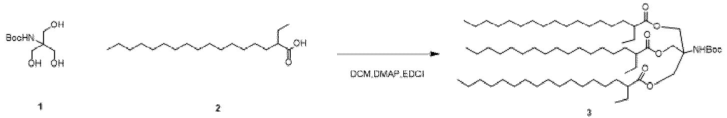

Compound 1 **(intermediate A,** 1.0 g), the DCM (20 mL), the DMAP (2.2 g), and the EDCI (3.4 g) were added to compound 2 **(intermediate B,** 4.3 g). A mixture was stirred at the room temperature under the nitrogen atmosphere for 12 h. The water and dichloromethane were added for stirring and phase separation. An organic phase was washed with the saturated brine, separated, dried over the anhydrous sodium sulfate and concentrated. A crude product was purified via silica gel column chromatography (PE: EA = (40:1)-(30:1)) to afford **compound 3** (2.6 g).

### Step 2: Synthesis of compound 4 in General synthetic route 1

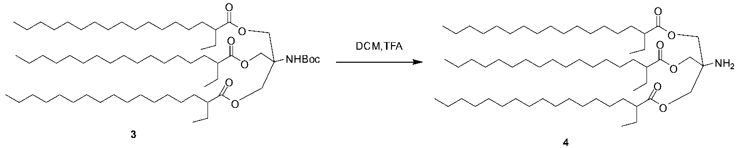

The DCM (15 mL) and the TFA (5 mL) were added to compound 3 (2.6 g) for stirring at the room temperature for 3 h and then concentration. A crude product was purified via silica gel column chromatography (PE: EA = (10:1)-(5:1)) to afford **compound** 4 (3.2 g).

### Step 3: Synthesis of compound 6 in General synthetic route 1

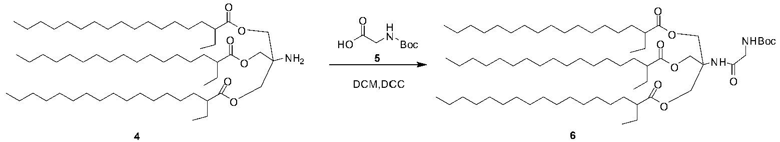

The DCM (20 mL), Boc-glycine (compound 5, 578 mg), and N,N'-dicyclohexylcarbodiimide (the DCC) (6.8 g) were added to **compound 4** (3.2 g) for stirring at the room temperature under the nitrogen atmosphere for 12 h. The water and the ethyl acetate were added for stirring and phase separation. An organic phase was washed with the saturated brine, separated, dried over the anhydrous sodium sulfate and concentrated. A crude product was purified via column chromatography (PE: EA = (5:1)-(2:1) to afford **compound 6** (2.2 g).

### Step 4: Compound 1003

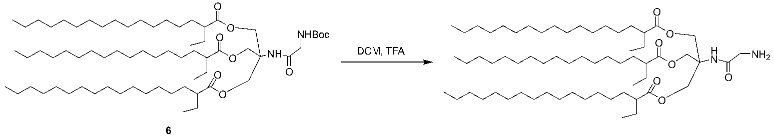

The DCM (15 mL) and the TFA (5 mL) were added to **compound 6** (2.2 g) for stirring at the room temperature for 12 h and then concentration. A crude product was purified via column chromatography (DCM: MeOH = (30:1)-(20:1)) to afford **compound 1003** (1.3 g). ¹H NMR (400 MHz, CDCl₃) δ 4.49 (d, J = 2.6 Hz, 6H), 3.28 (s, 2H), 2.32 (tt, J = 8.6, 5.5 Hz, 3H), 1.62 (q, J = 7.1 Hz, 12H), 1.27 (d, J = 3.4 Hz, 78H), 0.94-0.86 (m, 18H).

### D. Compound 1002, synthesized via General synthetic route 1

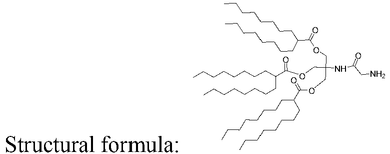

Chemical formula: C₆₀H₁₁₆N₂O₇
Molecular weight: 977.60

Compound 1002 was synthesized via General synthetic route 1, which was similar to a synthetic process of compound 1003, except that **intermediate C** was used as compound 2 in General synthetic route 1. ¹H NMR (400 MHz, CDCl₃) δ 4.48 (s, 6H), 3.27 (s, 2H), 2.37 (tt, J = 8.5, 5.5 Hz, 3H), 1.64 (d, J = 6.9 Hz, 6H), 1.50-1.42 (m, 6H), 1.27 (s, 72H), 0.90 (t, J = 6.7 Hz, 18H).

### E. Compound 1004, synthesized via General synthetic route 1

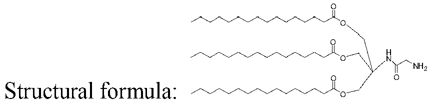

Chemical formula: C₅₄H₁₀₄N₂O₇
Molecular weight: 893.43

Compound 1004 was synthesized via General synthetic route 1, which was similar to the synthetic process of Compound 1003, except that **palmitic acid** was used as compound 2 in General synthetic route 1. ¹H NMR (400 MHz, CDCl₃) δ 7.55 (s, 1H), 4.45 (s, 6H), 3.74 (s, 2H), 2.35 (t, J = 7.6 Hz, 6H), 1.60 (p, J = 6.9 Hz, 6H), 1.27 (s, 72H), 0.90 (t, J = 6.7 Hz, 9H).

### F. Compound 1001, synthesized via General synthetic route 1

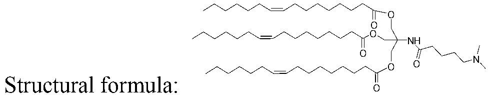

Chemical formula: C₅₉H₁₀₈N₂O₇
Molecular weight: 957.52

Compound 1001 was synthesized via General synthetic route 1, which was similar to the synthetic process of Compound 1003, except that **(9Z)-hexadec-9-enoic acid** was used as compound 2 in General synthetic route 1, and 5-(N,N-dimethylamino)pentanoic acid was used as compound 5 in General synthetic route 1. ¹H NMR (400 MHz, CDCl₃) δ 5.42-5.30 (m, 6H), 4.51-4.38 (m, 6H), 3.19 (dt, J = 11.3, 5.6 Hz, 2H), 2.97 (t, J = 5.9 Hz, 6H), 2.46 (t, J = 6.6 Hz, 1H), 2.35 (td, J = 7.6, 3.1 Hz, 5H), 2.20 (t, J = 7.6 Hz, 2H), 2.03 (d, J = 6.2 Hz, 12H), 1.89-1.81 (m, 3H), 1.74 (q, J = 7.8 Hz, 2H), 1.67-1.51 (m, 6H), 1.31 (d, J = 7.8 Hz, 48H), 0.90 (t, J = 6.7 Hz, 9H).

### G. Compound 1014, synthesized via General synthetic route 2

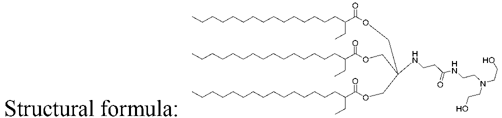

Chemical formula: C₇₀H₁₃₇N₃O₉
Molecular weight: 1164.88

### Step 1: Synthesis of Intermediate 3 in General synthetic route 2

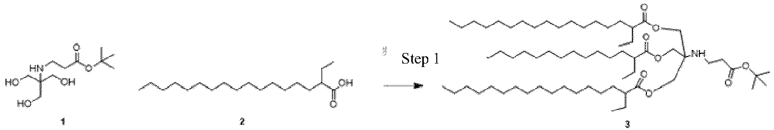

Intermediate 3 was synthesized via the method as shown in Step 1 of General synthetic route 1, except that **intermediate E** was used as compound 1 in General synthetic route 1.

### Step 2: Synthesis of compound 4 in General synthetic route 2

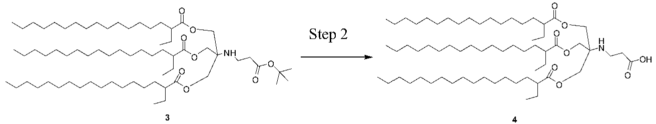

The DCM (15 mL), the TFA (12 mL), and the TiPS (3 mL) were added to compound 3 (4.8 g) for a reaction at the room temperature for 12 h. The water and the dichloromethane were added for stirring and phase separation. An organic phase was washed with the saturated brine, separated, and then concentrated. A crude product was purified via silica gel column chromatography (DCM: MeOH = (20:1)-(10:1)) to afford compound 4 (3.3 g).

### Step 3: Synthesis of compound 6 in General synthetic route 2

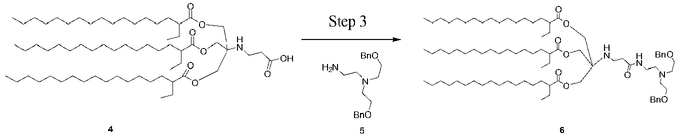

Compound 4 (4.0 g), the DCM (80 mL), the EDCI (1.1 g), 1-hydroxybenzotriazole (HOBt, 0.78 g), and the DIEA (2.5 g) were added to Compound 5 (1.9 g) for stirring at the room temperature for 12 h. The water and the dichloromethane were added for stirring and phase separation. An organic phase was washed with the saturated brine, separated, and then concentrated. A resutlant was purified via silica gel column chromatography (DCM: MeOH = (30:1)-(20:1)) to afford compound 6 (4.0 g).

### Step 4: Compound 1014

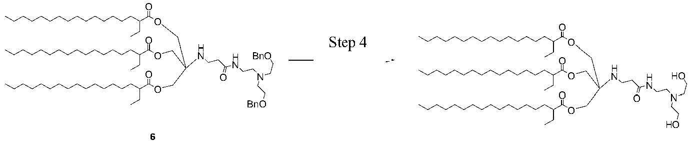

MeOH (30 mL), the DCM (10 mL), and 10% Pd/C (4.73 g) were added to compound 6 (4.0 g) for stirring at the room temperature overnight under a hydrogen atmosphere. A mixture was filtered through celite and concentrated. A crude product was purified via silica gel column chromatography (DCM: MeOH = (30:1)-(15:1)) to afford **compound 1014** (2 g). ¹H (400 MHz, CDCl₃) δ 7.38 (t, J = 5.8 Hz, 1H), 4.14-4.05 (m, 6H), 3.58 (t, J = 4.9 Hz, 4H), 3.31 (q, J = 5.7 Hz, 2H), 2.89 (t, J = 6.3 Hz, 2H), 2.64 (q, J = 5.2 Hz, 6H), 2.29 (dq, J = 10.8, 4.4 Hz, 6H), 1.66-1.37 (m, 12H), 1.24 (d, J = 4.3 Hz, 78H), 0.87 (t, J = 7.1 Hz, 18H)

### H. Compound 1015, synthesized via General synthetic route 2

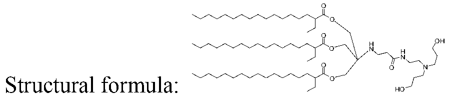

Chemical formula: C₇₂H₁₄₁N₃O₉
Molecular weight: 1192.93

Compound 1015 was synthesized via General synthetic route 2, which was similar to a synthetic process of Compound 1014, except that **intermediate G** was used as compound 5 in General synthetic route 2. ¹H (400 MHz, CDCl₃) δ 7.58 (t, J = 5.7 Hz, 1H), 4.15-4.01 (m, 6H), 3.70 (t, J = 5.4 Hz, 4H), 3.39 (q, J = 5.7 Hz, 2H), 2.86 (t, J = 6.2 Hz, 2H), 2.57 (dt, J = 20.5, 5.8 Hz, 6H), 2.36-2.22 (m, 5H), 1.76-1.67 (m, 4H), 1.62-1.35 (m, 13H), 1.23 (d, J = 4.6 Hz, 78H), 0.92-0.80 (m, 18H).

### I. Compound 1020, synthesized via General synthetic route 2

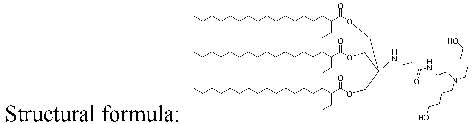

Chemical formula: C₇₄H₁₄₅N₃O₉
Molecular weight: 1220.99

Compound 1020 was synthesized via General synthetic route 2, which was similar to the synthetic process of Compound 1014, except that **intermediate H** was used as compound 5 in General synthetic route 2.

### J. Compound 1025, synthesized via General synthetic route 2

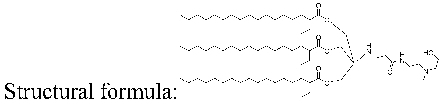

Chemical formula: C₆₉H₁₃₅N₃O₈
Molecular weight: 1134.85

Compound 1025 was synthesized via General synthetic route 2, which was similar to the synthetic process of Compound 1014, except that **intermediate I** was used as compound 5 in General synthetic route 2. ¹H (400 MHz, CDCl₃) δ 6.98 (t, J = 5.7 Hz, 1H), 4.10 (m, J = 3.8 Hz, 6H), 3.63 (t, J = 5.2 Hz, 2H), 3.35 (q, J = 5.8 Hz, 2H), 2.89 (t, J = 6.1 Hz, 2H), 2.57 (q, J = 5.3 Hz, 4H), 2.32 (s, 3H), 2.28 (q, J = 4.1 Hz, 5H), 1.65-1.41 (m, 13H), 1.24 (d, J = 4.4 Hz, 78H), 0.87 (t, J = 7.1 Hz, 18H).

### K. Compound 1005, synthesized via General synthetic route 2

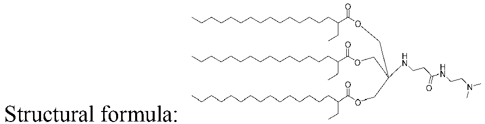

Chemical formula: C₆₈H₁₃₃N₃O₇
Molecular weight: 1104.83

Compound 1005 was synthesized via General synthetic route 2, which was similar to the synthetic process of Compound 1014, except that N,N-dimethylethylenediamine was used as compound 5 in General synthetic route 2. ¹H NMR (400 MHz, CDCl₃) δ 4.19-4.07 (m, 6H), 3.40 (d, J = 5.7 Hz, 2H), 2.95-2.87 (m, 2H), 2.82 (s, 4H), 2.64-2.55 (m, 2H), 2.38 (s, 6H), 2.33-2.29 (m, 3H), 1.63-1.55 (m, 6H), 1.49-1.44 (m, 2H), 1.26 (d, J = 5.4 Hz, 78H), 0.89 (td, J = 7.0, 2.8 Hz, 18H).

### L. Compound 1006, synthesized via General synthetic route 2

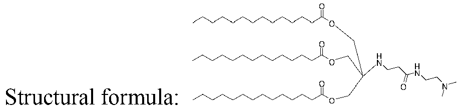

Chemical formula: C₅₃H₁₀₃N₃O₇
Molecular weight: 894.42

Compound 1006 was synthesized via General synthetic route 2, which was similar to the synthetic process of Compound 1014, except that myristic acid was used as compound 2 in General synthetic route 2 and N,N-dimethylethylenediamine was used as compound 5 in General synthetic route 2. ¹H NMR (400 MHz, CDCl₃) δ 7.99 (t, J = 5.8 Hz, 1H), 4.13 (s, 6H), 3.60 (q, J = 5.3 Hz, 2H), 3.38-3.34 (m, 2H), 3.00 (s, 6H), 2.94 (t, J = 5.8 Hz, 2H), 2.45 (t, J = 5.8 Hz, 2H), 2.35 (t, J = 7.6 Hz, 6H), 1.66-1.56 (m, 6H), 1.33-1.23 (m, 60H), 0.90 (t, J = 6.8 Hz, 9H).

### M. Compound 1007, synthesized via General synthetic route 2

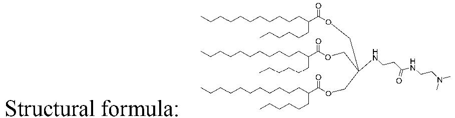

Chemical formula: C₆₈H₁₃₃N₃O₇
Molecular weight: 1104.83

Compound 1007 was synthesized via General synthetic route 2, which was similar to the synthetic process of Compound 1014, except that **intermediate D** was used as compound 2 in General synthetic route 2 and the N,N-dimethylethylenediamine was used as compound 5 in General synthetic route 2. ¹H NMR (400 MHz, Chloroform-d) δ 7.13 (t, J = 5.0 Hz, 1H), 4.13 (s, 6H), 3.33 (q, J = 5.6 Hz, 2H), 2.92 (t, J = 6.0 Hz, 2H), 2.47-2.28 (m, 7H), 2.24 (s, 6H), 1.60 (dq, J = 14.7, 7.2 Hz, 6H), 1.47 (dd, J = 14.2, 6.7 Hz, 6H), 1.27 (d, J = 3.4 Hz, 78H), 0.89 (td, J = 6.7, 2.5 Hz, 18H).

### N. Compound 1008, synthesized via General synthetic route 2

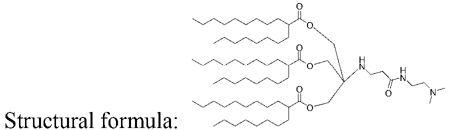

Chemical formula: C₆₈H₁₃₃N₃O₇
Molecular weight: 1104.01

Compound 1008 was synthesized via General synthetic route 2, which was similar to the synthetic process of compound 1014, except that **intermediate C** was used as compound 2 in General synthetic route 2 and the N,N-dimethylethylenediamine was used as compound 5 in General synthetic route 2. ¹H NMR (400 MHz, Chloroform-d) δ 4.13 (s, 6H), 3.33 (q, J = 5.6 Hz, 2H), 2.92 (t, J = 5.9 Hz, 2H), 2.51-2.26 (m, 7H), 2.24 (s, 6H), 1.59 (ddt, J = 14.8, 10.9, 6.4 Hz, 6H), 1.47 (tq, J = 11.0, 5.4 Hz, 6H), 1.26 (s, 77H), 0.89 (t, J = 6.7 Hz, 18H).

### O. Compound 1009, synthesized via General synthetic route 2

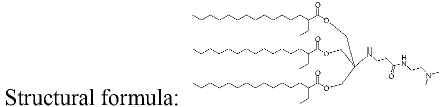

Chemical formula: C₆₂H₁₂₁N₃O₇
Molecular weight: 1020.66

Compound 1009 was synthesized via General synthetic route 2, which was similar to the synthetic process of Compound 1014, except that **intermediate J** was used as compound 2 in General synthetic route 2 and the N,N-dimethylethylenediamine was used as compound 5 in General synthetic route 2. ¹H (400 MHz, CDCl₃) δ 7.04 (t, J = 5.0 Hz, 1H), 4.12 (m, J = 3.3 Hz, 6H), 3.30 (q, J = 5.6 Hz, 2H), 2.91 (t, J = 6.0 Hz, 2H), 2.39 (t, J = 6.0 Hz, 2H), 2.33-2.25 (m, 5H), 2.22 (s, 6H), 1.76-1.54 (m, 12H), 1.24 (m, J = 3.7 Hz, 66H), 0.90-0.84 (m, 18H).

### P. Compound 1011, synthesized via General synthetic route 2

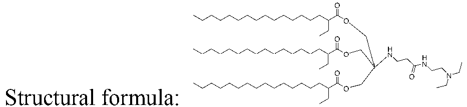

Chemical formula: C₇₀H₁₃₇N₃O₇
Molecular weight: 1132.88

Compound 1011 was synthesized via General synthetic route 2, which was similar to the synthetic process of Compound 1014, except that the N,N-diethylethylenediamine was used as compound 5 in General synthetic route 2. ¹H NMR (400 MHz, CDCl₃) δ 4.19-4.08 (m, 6H), 3.31 (q, J = 5.8 Hz, 2H), 2.92 (t, 2H), 2.57 (t, J = 6.9 Hz, 6H), 2.37-2.26 (m, 5H), 1.62 (ddd, J = 18.3, 9.0, 4.3 Hz, 12H), 1.27 (d, J = 4.7 Hz, 78H), 1.04 (t, J = 7.1 Hz, 6H), 0.94-0.86 (m, 18H).

### Q. Compound 1012, synthesized via General synthetic route 2

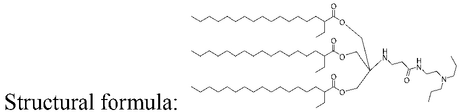

Chemical formula: C₇₂H₁₄₁N₃O₇
Molecular weight: 1160.93

Compound 1012 was synthesized via General synthetic route 2, which was similar to the synthetic process of Compound 1014, except that the N,N-diethylethylenediamine was used as compound 5 in General synthetic route 2. ¹H NMR (400 MHz, CDCl₃) δ 6.59 (s, 1H), 4.18-4.07 (m, 6H), 3.29 (q, J = 5.7 Hz, 2H), 2.91 (t, J = 6.2 Hz, 2H), 2.54 (t, J = 6.1 Hz, 2H), 2.40 (t, J = 7.5 Hz, 4H), 2.35-2.27 (m, 5H), 1.63-1.42 (m, 16H), 1.27 (m, J = 4.5 Hz, 78H), 0.93-0.85 (m, 24H).

### R. Compound 1013, synthesized via General synthetic route 2

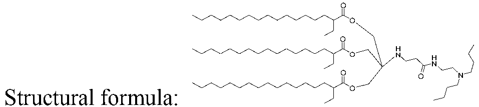

Chemical formula: C₇₄H₁₄₅N₃O₇
Molecular weight: 1188.99

Compound 1013 was synthesized via General synthetic route 2, which was similar to the synthetic process of Compound 1014, except that the N,N-diethylethylenediamine was used as compound 5 in General synthetic route 2. ¹H NMR (400 MHz, CDCl₃) δ 4.10 (d, J = 4.1 Hz, 6H), 3.26 (q, J = 5.8 Hz, 2H), 2.89 (t, J = 6.2 Hz, 2H), 2.52 (d, J = 6.6 Hz, 2H), 2.41 (t, J = 7.4 Hz, 4H), 2.33-2.25 (m, 5H), 1.60 (d, J = 11.8 Hz, 16H), 1.24 (d, J = 4.5 Hz, 82H), 0.96-0.79 (m, 24H).

### S. Compound 1029, synthesized via General synthetic route 2

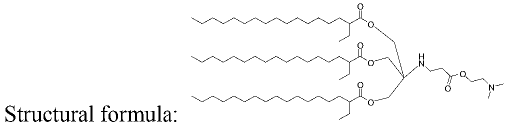

Chemical formula: C₆₈H₁₃₂N₂O₈
Molecular weight: 1105.81

Compound 1029 was synthesized via General synthetic route 2, which was similar to the synthetic process of Compound 1014, except that the N,N-diethyl-2-hydroxyethylamine was used as compound 5 in General synthetic route 2. ¹H NMR (400 MHz, CDCl₃)δ 4.16 (t, J = 5.8 Hz, 2H), 4.13-4.05 (m, 6H), 2.86 (t, J = 6.3 Hz, 2H), 2.55 (t, J = 5.8 Hz, 2H), 2.44 (t, J = 6.3 Hz, 2H), 2.35-2.20 (m, 9H), 1.60-1.39 (m, 11H), 1.24 (d, J = 4.5 Hz, 80H), 1.02-0.79 (m, 18H).

### T. Compound 1111, synthesized via General synthetic route 2

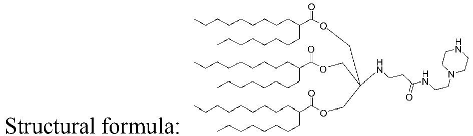

Chemical formula: C₇₀H₁₃₆N₄O₇
Molecular weight: 1145.88

Compound 1111 was synthesized via General synthetic route 2, which was similar to the synthetic process of Compound 1014, except that **intermediate C** was used as compound 2 in General synthetic route 2 and tert-butyl 4-(2-aminoethyl)piperazine-1-carboxylate was used as compound 5 in General synthetic route 2. ¹H NMR (400 MHz, Chloroform-d) δ 4.28 (s, 6H), 3.41 (s, 2H), 3.31 (s, 4H), 3.22 (s, 3H), 2.97-2.80 (m, 5H), 2.70 (s, 5H), 2.46-2.32 (m, 4H), 1.60 (t, J = 7.1 Hz, 6H), 1.54-1.40 (m, 6H), 1.27 (s, 78H), 0.89 (t, J = 6.7 Hz, 18H).

### U. Compound 1118, synthesized via General synthetic route 2

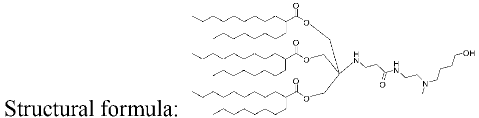

Chemical formula: C₇₁H₁₃₉N₃O₈
Molecular weight: 1162.91

Compound 1118 was synthesized via General synthetic route 2, which was similar to the synthetic process of Compound 1014, except that **intermediate C** was used as compound 2 in General synthetic route 2 and **intermediate K** was used as compound 5 in General synthetic route 2. ¹H NMR (400 MHz, Chloroform-d) δ 4.11 (s, 6H), 3.61 (s, 2H), 3.42 (d, J = 33.5 Hz, 2H), 2.91 (t, J = 6.2 Hz, 3H), 2.36 (dd, J = 9.2, 3.6 Hz, 6H), 1.47 (dd, J = 13.9, 6.2 Hz, 14H), 1.27 (s, 71H), 0.90 (t, J = 6.7 Hz, 18H).

### V. Compound 1010, synthesized via General synthetic route 3

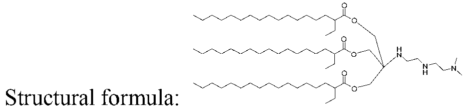

Chemical formula: C₆₇H₁₃₃N₃O₆
Molecular weight: 1076.82

### Step 1 and Step 2: Synthesis of compound 3 and compound 4 in General synthetic route 3

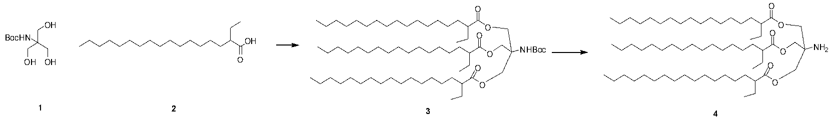

The operations of Step 1 and Step 2 in this general synthetic route followed Step 1 and Step 2 of General synthetic route 1, which were similar to the synthetic process of compound 1003.

### Step 3: Synthesis of compound 6 in General synthetic route 3

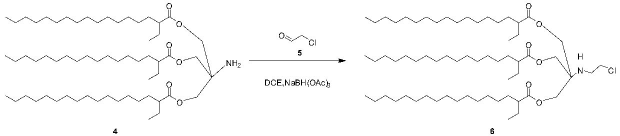

Compound 4 (600 mg), chloroacetaldehyde (compound 5, 242 mg (40%)), and NaBH(OAc)₃ (394 mg) were added to the DCE (60 mL) for a reaction at the room temperature overnight. The water and the dichloromethane were added for stirring and phase separation. An organic phase was washed with the saturated brine, separated and concentrated. A resultnat was purified via silica gel column chromatography (DCM: MeOH = (30:1)-(15:1)) to afford compound 6 (270 mg).

### Step 4: sSynthesis of compound 1010

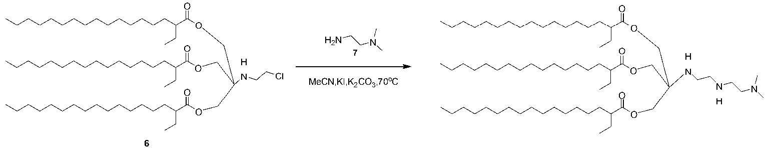

The MeCN (20 mL), the N,N-dimethylethylenediamine (compound 7, 229 mg), KI (43 mg), and K₂CO₃ (179 mg) were added to compound 6 (270 mg) for a reaction at 70°C overnight under the nitrogen atmosphere, A resultant was purified via silica gel column chromatography to afford **compound 1010** (16 mg). ¹H (400 MHz, CDCl₃) δ 4.10 (m, J = 2.9 Hz, 6H), 2.76-2.64 (m, 6H), 2.40 (t, J = 6.2 Hz, 2H), 2.29 (ddd, J = 8.5, 5.6, 2.9 Hz, 3H), 2.22 (s, 6H), 1.97 (s, 6H), 1.50-1.41 (m, 6H), 1.24 (d, J = 4.2 Hz, 78H), 0.87 (t, J = 7.1 Hz, 18H).

### W. Compound 1059, synthesized via General synthetic route 3

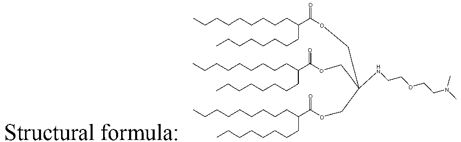

Chemical formula: C₆₇H₁₃₂N₂O₇
Molecular weight: 1077.80

Compound 1059 was synthesized via General synthetic route 3, which was similar to a synthetic process of Compound 1010, except that **intermediate C** and **intermediate L** were respectively used as compound 2 and compound 5 in General synthetic route 3. In addition, compound 6 in General synthetic route 3 was needed to undergo functional group conversion via the following process steps:

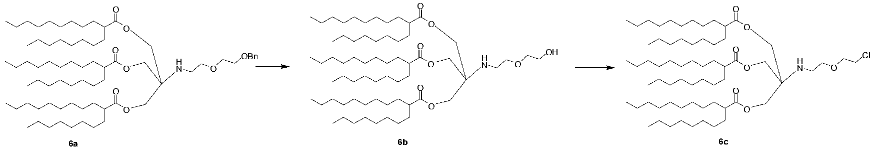

The MeOH (5 mL) and the THF (5 mL) were added to intermediate 6a (1.5 g), followed by addition of 10% Pd(OH)₂/C (10%) (0.46 g) and 10% Pd/C (10%) (0.35 g) as reactants. A mixture underwent a reaction at 25°C overnight under the H₂ atmosphere. A resultant was filtered through the celite, and a filtrate was concentrated and purified via silica gel column chromatography (PE: EA = (30:1)-(10:1)) to afford intermediate 6b (520 mg). The DCM (5 mL) was added to intermediate 6b (470 mg), followed by dropwise addition of SOCl₂ (1064.26 mg, 8.946 mmol) at 0°C. A mixture was heated to the room temperature and underwent a reaction for 3 h. A resultant was extracted with the water and the DCM. An organic phase was concentrated, and purified via silica gel column chromatography (PE: EA = 20:1) to afford intermediate **6c** (181 mg). Dimethylamine (143.39 mg), the DMF (2 Ml), potassium carbonate (43.96 mg), and potassium iodide (26.39 mg) were added to intermediate 6c (170 mg). A reaction mixture was stirred at 70°C, followed by filtration and concentration. A crude product was purified via silica gel column chromatography (DCM: MeOH = 20:1) to afford **compound 1059** (91 mg, yield 53.22%). ¹H NMR (400 MHz, Chloroform-d) δ 4.11 (s, 6H), 3.51 (dt, J = 18.3, 5.6 Hz, 4H), 2.81 (dd, J = 10.3, 5.1 Hz, 2H), 2.50 (t, J = 6.0 Hz, 2H), 2.42-2.32 (m, 3H), 2.28 (s, 6H), 2.08-1.98 (m, 1H), 1.53-1.38 (m, 7H), 1.27 (s, 77H), 0.90 (t, J = 6.7 Hz, 18H).

### X. Compound 1112, synthesized via General synthetic route 3

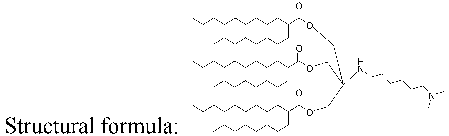

Chemical formula: C₆₉H₁₃₆N₂O₆
Molecular weight: 1089.85

Compound 1112 was synthesized via General synthetic route 3, which was similar to the synthetic process of Compound 1010, except that **Iintermediate C** and **6-bromohexanal** were respectively used as compound 2 and compound 5 in General synthetic route 3. ¹H NMR (400 MHz, Chloroform-d) δ 4.11 (s, 6H), 3.11 (s, 1H), 2.57 (t, J = 6.8 Hz, 2H), 2.44-2.19 (m, 12H), 2.04 (q, J = 6.5 Hz, 2H), 1.53-1.38 (m, 13H), 1.27 (s, 92H), 0.90 (t, J = 6.7 Hz, 20H).

### Y. Compound 1113, synthesized via General synthetic route 4

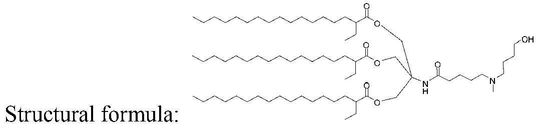

Chemical formula: C₇₁H₁₃₈N₂O₈
Molecular weight: 1147.89

### Step 1 and Step 2: Synthesis of compound 3 and compound 4 in General synthetic route 4

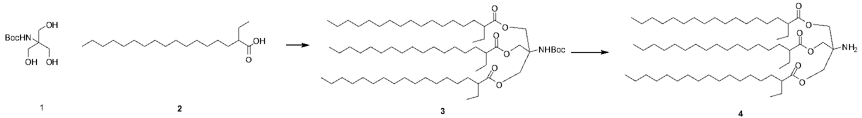

The operations of Step 1 and Step 2 in this general synthetic route followed Step 1 and Step 2 of General synthetic route 1, which were similar to the synthetic process of Compound 1003.

### Step 3: Synthesis of compound 6 in General synthetic route 4

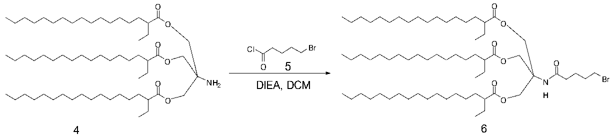

The DIEA (0.30 g) and the DCM (20 mL) were added to compound 4. A mixture was cooled to 0°C under the nitrogen atmosphere, followed by addition of 5-bromopentanoyl chloride (compound 5, 0.27 g). A mixture was stirred at 25°C for 2 h and diluted with 100 mL of the DCM. An organic phase was washed twice with 100 mL of the water, dried over anhydrous Na₂SO₄, filtered and concentration. A crude product was purified via silica gel column chromatography (PE: EA = 20:1) to afford compound 6 (800 mg).

### Step 4: synthesis of compound 8 in General synthetic route 4

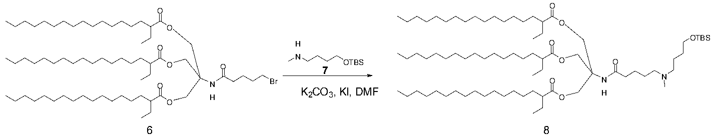

Compound 6 (750.00 mg), **intermediate K** (compound 7, 217.31 mg), and the DMF (20 mL) were added to a three-necked bottle, followed by addition of K₂CO₃ (184.16 mg) and KI (110.60 mg) as reactants. A mixture was stirred at 25°C for 16 h, and then heated to 100°C for further stirring for another 3 h. A reaction mixture was diluted with 200 mL of the EA, and then an organic phase was washed twice with 300 mL of the saturated brine. The organic phase was dried over anhydrous Na₂SO₄, filtered and concentrated. A crude product was purified by silica gel column chromatography (DCM: MeOH = 20:1). A sample was collected and then concentrated to afford compound 8 (700.00 mg).

### Step 5: Synthesis of compound 1113

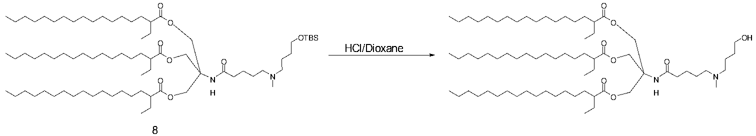

Compound 8 (700.00 mg) and a sovlent (the THF) (5 mL) were added to a three-necked flask, followed by addition of the hydrogen chloride in dioxane solution (1 M, 5.55 mL). A reaction mixture was stirred at 25°C for 2 h, and then diluted with 50 mL of the EA. An organic phase was sequentially washed with a saturated NaHCO₃ aqueous solution and the water, dried over anhydrous Na₂SO₄, filtered and concentrated. A crude product was purified via silica gel column chromatography (MeOH: DCM = 1: 20) to afford compound 1113 (201.1 mg, yield 31.59%). ¹H NMR (400 MHz, CDCl₃) δ 4.50-4.39 (m, 6H), 3.59 (t, J = 4.6 Hz, 2H), 2.49 (s, 3H), 2.35-2.26 (m, 6H), 2.14 (t, J = 7.5 Hz, 2H), 1.75-1.69 (m, 4H), 1.66-1.43 (m, 17H), 1.27 (d, J = 3.7 Hz, 78H), 0.94-0.87 (m, 18H).

### Z. Compound 1115, synthesized via General synthetic route 4

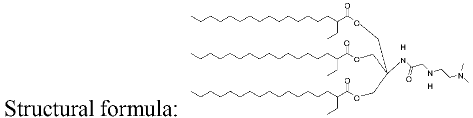

Chemical formula: C₆₇H₁₃₁N₃O₇
Molecular weight: 1090.80

Compound 1115 was synthesized via General synthetic route 4, which was similar to the synthetic process of Compound 1010, except that **2-bromoacetyl chloride** and the **N,N-dimethylethylenediamine** were respectively used as compound 5 and compound 7 in General synthetic route 4. ¹H NMR (400 MHz, CDCl₃) δ 7.42 (s, 1H), 4.48 (tt, J = 11.9, 5.7 Hz, 6H), 3.27 (s, 2H), 3.13 (s, 2H), 3.01 (s, 2H), 2.83 (s, 6H), 2.38-2.25 (m, 3H), 1.55 (dtt, J = 36.3, 14.1, 7.3 Hz, 12H), 1.27 (d, J = 3.3 Hz, 78H), 0.90 (td, J = 7.2, 3.4 Hz, 18H).

### AA. Compound 1114, synthesized via General synthetic route 5

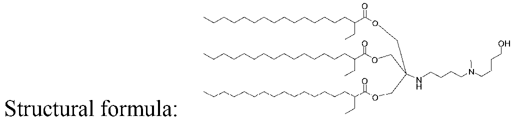

Chemical formula: C₇₀H₁₃₈N₂O₇
Molecular weight: 1119.88

### Step 1 and Step 2: Synthesis of compound 3 and compound 4 in General synthetic route 5

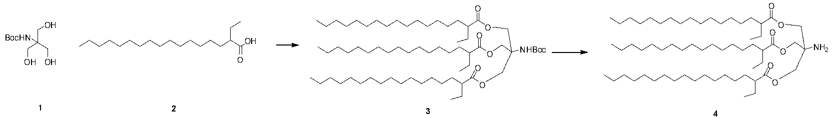

The operations of Step 1 and Step 2 in this general synthetic route followed Step 1 and Step 2 of General synthetic route 1, which was similar to the synthetic process of compound 1003.

### Step 3: Synthesis of compound 6 in General synthetic route 5

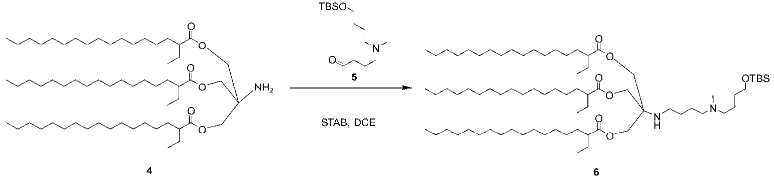

Compound 4 (500 mg), **intermediate M** (compound 5, 298.68 mg), and sodium triacetoxyborohydride (STAB, 328.68 mg) and DCE (10 mL) as reactants were added to a three-necked flask. A reaction mixture was stirred at 25°C for 16 h under the N₂ atmosphere. 100 mL of the water was added to the reaction mixture. A mixture was extracted twice with the EA (100 mL). Organic phases were combined and concentrated. A crude product was purified via silica gel column chromatography (DCM: MeOH = 1:20). A sample was collected and concentrated to afford compound 6 (500 mg).

### Step 4: Synthesis of compound 1114

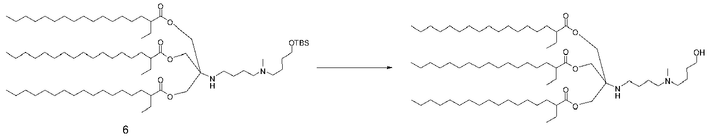

Compound 6 (450 mg) and the solvent (the THF) (5 mL) were added to a three-necked flask, followed by addition of the hydroxide chloride in dioxane solution (1 M) (3.65 mL) for stirring at 25°C for 2 h. A mixture was diluted with 50 mL of the EA. An organic phase was washed with 50 mL of the saturated NaHCO₃ aqueous solution and the water, dried over anhydrous Na₂SO₄, filtered and concentrated. A crude product was purified via silica gel column chromatography (MeOH: DCM = 1:20) to afford compound 1114 (107 mg, yield 24.84%). ¹H NMR (400 MHz, CDCl₃) δ 4.16-4.07 (m, 6H), 3.60 (t, J = 4.8 Hz, 2H), 2.62 (t, J = 7.0 Hz, 2H), 2.49 (s, 3H), 2.31 (tt, J = 8.4, 5.5 Hz, 6H), 1.70 (d, J = 4.6 Hz, 3H), 1.64-1.39 (m, 18H), 1.27 (d, J = 4.0 Hz, 78H), 0.90 (t, J = 7.1 Hz, 18H).

### Z. Compound 1120, synthesized via General synthetic route 5

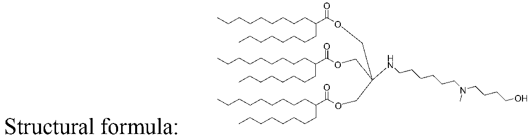

Chemical formula: C₇₂H₁₄₂N₂O₇
Molecular weight: 1147.90

Compound 1120 was synthesized via General synthetic route 5, which was similar to the synthetic process of compound 1114, except that **intermediate C** and the **6-bromohexanal** were respectively used as compound 2 and compound 5 in General synthetic route 5. ¹H NMR (400 MHz, Chloroform-d) δ 4.11 (s, 6H), 3.63 (s, 2H), 2.57 (t, J = 6.8 Hz, 4H), 2.37 (ddd, J = 8.5, 7.1, 4.3 Hz, 5H), 1.67-1.41 (m, 24H), 1.27 (s, 79H), 0.90 (t, J = 6.7 Hz, 18H).

### (2) Preparation example 2 Synthesis of second ionizable lipid

### 1. Preparation of compound 6001

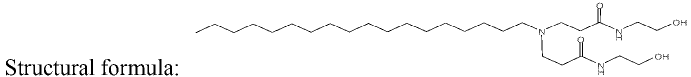

Molecular weight: 499.43

0.8 g of oleylamine and 0.8 g of hydroxyethyl acrylamide were added to 6.4 mL of ethanol, and a mixture was heated to 70°C with stirring. After 3.5 h, 0.8 g of hydroxyethyl acrylamide was supplemented for a further reaction for 16 h. A resultant was then purified by reversed-phase column chromatography to afford compound 6002.

0.7 g of compound 6002, 0.4 g of Pd/C, and 28 mL of tetrahydrofuran were added to a reaction flask for stirring. The system was purged with hydrogen and maintained under hydrogen pressure via a hydrogen balloon. After 16 h, the mixture was filtered, and a filtrate was purified via normal-phase column chromatography (a MeOH-DCM system) to afford 200 mg of **compound 6001.** ¹H NMR (400 MHz, CDCl₃) δ 7.54 (t, *J* = 5.5 Hz, 2H), 3.79-3.68 (m, 4H), 3.42 (dd, *J* = 10.0, 5.4 Hz, 4H), 2.89 (t, *J* = 6.0 Hz, 4H), 2.55 (dt, *J* = 11.8, 6.8 Hz, 6H), 1.54 (s, 2H), 1.27 (d, *J* = 11.2 Hz, 32H), 0.91 (t, *J* = 6.8 Hz, 3H).

### 2. Preparation of compound 6002

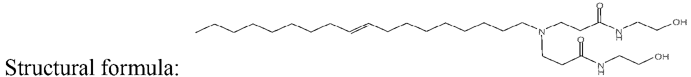

Molecular weight: 497.42

0.8 g of the oleylamine and 0.8 g of the hydroxyethyl acrylamide were added to 6.4 mL of the ethanol, and a mixture was heated to 70°C with stirring. After 3.5 h, 0.8 g of the hydroxyethyl acrylamide was supplemented for a further reaction for another 16 h. An organic solvent was concentrated. A crude product was purified via reversed-phase column chromatography and then lyophilized to afford 400 mg of **compound 6002.** ¹H NMR (400 MHz, CDCl₃) δ 7.31 (s, 2H), 5.38 (dd, *J* = 13.0, 7.4 Hz, 2H), 3.79-3.66 (m, 4H), 3.41 (dd, *J* = 10.1, 5.4 Hz, 4H), 2.82-2.72 (m, 4H), 2.44 (dd, *J* = 15.1, 9.2 Hz, 6H), 2.10-1.99 (m, 4H), 1.46 (s, 2H), 1.29 (s, 24H), 0.90 (t, *J* = 6.8 Hz, 3H).

### 3. Preparation of compound 6003

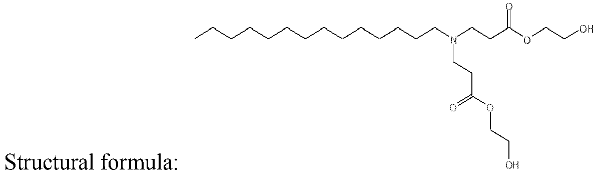

Molecular weight: 445.34

4.00 g of tetradecylamine, 6.53 g of 2-hydroxyethyl acrylate, and 80 mL of tert-butanol were added to a reaction flask, and a misture was heated to 70°C with stirring. After 29 h, an organic solvent was concentrated. A crude product was purified via reversed-phase column chromatography and lyophilized to afford 1.5 g of **compound 6003.** ¹H NMR (400 MHz, CDCl₃) δ 4.29 (t, *J* = 7.5 Hz, 4H), 3.89-3.75 (m, 4H), 2.81 (t, *J* = 6.2 Hz, 4H), 2.50 (ddd, *J* = 23.2, 14.1, 7.1 Hz, 6H), 1.46 (s, 2H), 1.28 (s, 24H), 0.91 (t, *J* = 6.3 Hz, 3H).

### 4. Preparation of compound 6004

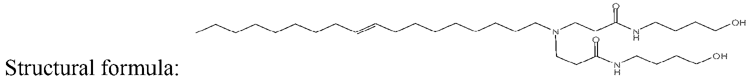

Molecular weight: 553.48

0.8 g of the oleylamine, 1.5 g of n-butyl acrylate ,and 5.6 mL of n-butanol were added to a reaction flask, and a mixture was heated to 100°C with stirring. After 4 h, 1 mL of n-butyl acrylate was supplemented, for a further reaction for 1 h. A resultant was purified via normal-phase column chromatography (a petroleum ether-ethyl acetate system). Then, 0.4 g of sodium hydroxide, 1 mL of the water, and 10 mL of methanol were added, and a mixture was stirred for 30 min to conduct hydrolysis. 1 mL of concentrated hydrochloric acid and 10 mL of the methanol were added to regulate a pH value of the mixed solution to neutral. The resulting neutral solution was subjected to solvent evaporation under reduced pressure, and a residue was dissolved into the DCM, dried over anhydrous magnesium sulfate, followed by filtration and solvent evaporation under reduced pressure. Subsequently, 10 mL of the DCM, 1.06 g of 4-amino-1-butanol, 0.81 g of HOBT, and 2.3 g of the EDCI were added to the residue for a reaction at the room temperature for 18 h. An organic solvent was concentrated. A crude product was purified via reversed-phase column chromatography and lyophilized to afford 240 mg of **compound 6004.** ¹H NMR (400 MHz, CDCl₃) δ 7.58 (d, *J* = 16.6 Hz, 2H), 5.44-5.31 (m, 2H), 3.72 (d, *J* = 16.5 Hz, 4H), 3.31 (d, J = 5.6 Hz, 4H), 3.17 (s, 4H), 2.84 (s, 2H), 2.69 (s, 4H), 2.09-2.01 (m, 4H), 1.67 (d, *J* = 2.7 Hz, 10H), 1.37-1.26 (m, 24H), 0.91 *(t, J* = 6.8 Hz, 3H).

### 5. Preparation of compound 6005

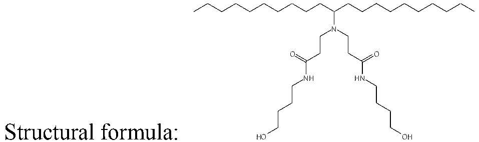

Molecular weight: 597.54

25.00 g of 11-heneicosanone, 62.05 g of ammonium acetate, and 500 mL of the methanol were added to a reaction flask for stirring, followed by addition of 6.55 g of sodium cyanoborohydride. After a reaction for 16 h, 250 mL of the water and 250 mL of the DCM were added to a resultant. A resulting mixture was subjected to phase separation. An aqueous phase was extracted with 50 mL of the DCM, and organic phases were combined and purified via normal-phase column chromatography (methanol-DCM system) to afford 21.00 g of **compound 6005-A.**

21.00 g of **compound 6005-A,** 58 mL of the n-butyl acrylate, and 100 mL of n-butanol were added to a reaction flask. A mixture was heated to 100°C with stirring. 10 mL of the n-butyl acrylate was supplemented. After 16 h, a resutlant was purified via column chromatography (a petroleum ether-ethyl acetate system) to afford 31.00 g of **compound 6005-B.**

A solution prepared from 31.00 g of **compound 6005-B,** 6.55 g of the sodium hydroxide, 310 mL of the methanol, and 31 mL of the water was added to a reaction flask for stirring. After 20 min, 150 mL of the THF was added, and a mixture was heated to 50°C with stirring. After 30 min, a solution prepared from 16.13 g of the concentrated hydrochloric acid and 160 mL of the methanol was added for stirring. After 30 min, a reaction mixture was subjected to solvent evaporation under reduced pressure, and a residue was dissolved into 300 mL of the DCM. A resulting organic solution was dried over anhydrous magnesium sulfate and filtered, followed by solvent evaporation under reduced pressure to afford 27.00 g of **compound 6005-C** as an oily product.

27.00 g of **compound 6005-C,** 25.00 g of the 4-amino-1-butanol, 41.85 g of the EDCI, 14.74 g of the HOBT, and 310 mL of the DCM were added to a reaction flask for stirring. After 18 h, an organic solvent was concentrated. A crude product was purified via column chromatography (a methanol-DCM system) to remove excess 4-amino-1-butanol, affording 6.80 g of compound 6005. ¹H NMR (400 MHz, CDCl₃) δ 3.70 (t, *J* = 5.6 Hz, 4H), 3.28 (t, *J* = 5.6 Hz, 4H), 2.74 (t, *J* = 6.0 Hz, 4H), 2.40-2.45 (m, 1H), 2.34 (t, *J* = 6.4 Hz, 4H), 1.63-1.65 (m, 8H), 1.25-1.35 (m, 36H), 0.91 (t, *J* = 6.4 Hz, 6H).

### 6. Preparation of compound 6006

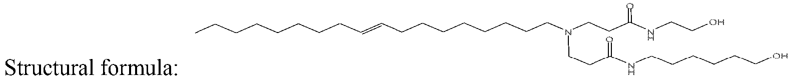

Molecular weight: 553.48

1.00 g of the oleylamine, 0.47 g of the hydroxyethyl acrylamide, and 8 mL of the ethanol were added to a reaction flask, and a mixture was heated to 70°C with stirring. After 13 h, a reaction mixture was subjected to solvent evaporation under reduced pressure. A resutlant was purified via column chromatography (the methanol-DCM system) to afford 800 mg of intermediate 1. 1.5 mL of the n-butyl acrylate and 5 mL of the n-butanol were added, and a mixture was heated to 100°C with stirring. After 3 h, a reaction mixture was subjected to solvent evaporation under reduced pressure. A crude product was purified via column chromatography (the methanol-DCM system). A solution prepared from 0.4 g of the sodium hydroxide, 10 mL of the methanol, and 1 mL of the water was added for stirring for 1 h. Then, a solution prepared from 1 mL of the concentrated hydrochloric acid and 8 mL of the methanol was added, followed by stirring for another 30 min. A reaction mixture was subjected to solvent evaporation under reduced pressure, and a residue was dissolved into 100 mL of the DCM. An resulting organic solution was dried over the anhydrous magnesium sulfate, followed by filtration and solvent evaporation under reduced pressure. 0.45 g of 6-amino-1-hexanol, 0.27 g of the HOBT, 0.78 g of the EDCI, and 10 mL of the DCM were added for stirring. After 14 h, an organic solvent was concentrated. A crude product was purified via column chromatography (the methanol-DCM system) to afford 300 mg of **compound 6006.** ¹H NMR (400 MHz, CDCl₃) δ 7.83 (s, 1H), 6.95 (s, 1H), 5.43-5.28 (m, 2H), 3.74-3.67 (m, 2H), 3.64 (t, *J* = 6.3 Hz, 2H), 3.39 (dd, *J* = 10.0, 5.3 Hz, 2H), 3.24 (dd, *J* = 12.9, 6.7 Hz, 2H), 2.74 (t, *J* = 5.8 Hz, 4H), 2.48-2.42 (m, 2H), 2.41-2.33 (m, 4H), 2.01 (dd, *J* = 13.6, 6.8 Hz, 4H), 1.66-1.17 (m, 34H), 0.89 (t, *J* = 6.7 Hz, 3H).

### 7. Compound 6007

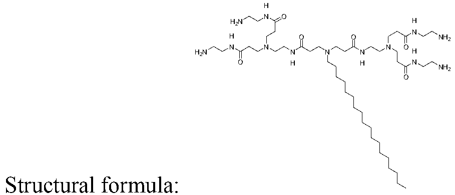

Molecular weight: 953.78

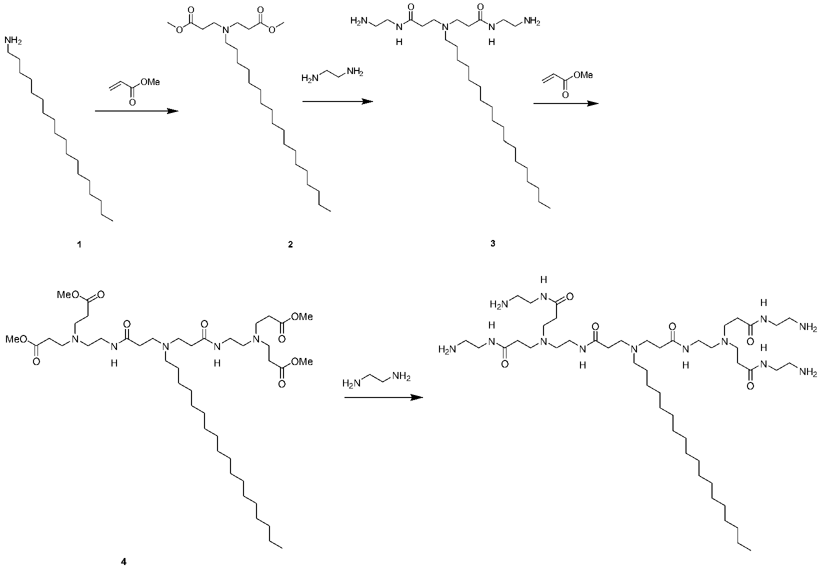

At the room temperature, octadecylamine (50 g) as a compound was added to a three-necked flask, followed by addition of 200 mL of MeOH and then mixing with stirring. The system was cooled to 5°C in an ice-water bath, and then methyl acrylate (31.5 g) was added dropwise slowly with stirring. A mixture was allowed to warm back to the room temperature and underwent a reaction for 4 h at the room temperature. A resulting reaction mixture was concentrated under reduced pressure. A crude material was purified via column chromatography (PE: EA = (20:1)-(10:1)) to afford product 2 (68 g). At the room temperature, product 2 (15.0 g), the MeOH (100 mL), and the ethylenediamine (1.0 g) were added to a single-neck flask. A mixture was heated to 60°C and maintained at this temperature for a reaction overnight. A reaction mixture was concentrated under reduced pressure, and then 30 mL of toluene was added. A resulting suspension was heated to 60°C for dissolution, then slowly cooled to the room temperature, and subsequently subjected to solvent evaporation under reduced pressure to afford compound 3 (15 g). At the room temperature, compound 3 (2 g) and the MeOH (20 mL) were added to a single-neck flask for dissolution with stirring. Methyl acrylate (2.5 g) was then added slowly with stirring. A mixture was heated to 60°C and held overnight. Upon completion of the reaction, a reaction mixture was concentrated under reduced pressure. A crude product was purified via column chromatography (DCM: MeOH = (50:1)-(20:1)) to afford compound 4 (2.5 g). At the room temperature, compound 4 (2.3 g) and the ethylenediamine (20 mL) were added to a single-neck flask, and a mixture was underwent a reaction at the room temperature overnight. Upon completion of the reaction, a reaction mixture was directly concentrated under reduced pressure to afford compound 6007 (24 mg). ¹H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz,4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 8. Compounds 6008-6018

### Synthesis of compound 6009

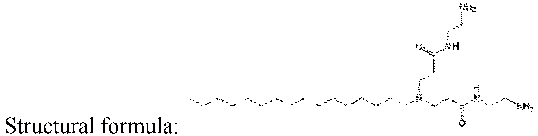

At the room temperature, a solution of freshly recrystallized hexadecylamine (0.03 mol) in methanol (20 mL) was added dropwise to a stirred solution of methyl methacrylate (6 mL) in the methanol (20 mL) under the nitrogen atmosphere, for a reaction overnight. A reaction mixture was subjected to solvent evaporation under reduced pressure at the room temperature. A residue was dissolved into chloroform and washed twice with a 0.1 M NaOH solution. A chloroform solution was collected and dried over anhydrous calcium chloride. Subsequently, a crude product was purified via column chromatography to afford colorless oil. ¹H-NMR (300MHz, CDCl₃): 0.78(t, 3H), 1.16(s, 30H), 2.38(m, 6H), 2.71(t, 4H), 3.57(m, 6H).

Then, a solution of the above colorless oil (11.05 g) in the methanol (20 mL) was added to a vigorously stirred solution of 1,2-ethanediamine (75 g) in the methanol (100 mL) at the room temperature. Upon completion of addition, a mixture was further stirred at the room temperature for another 24 h. A solvent was removed under reduced pressure, with a temperature maintained at no higher than 40°C. The excessive 1,2-ethanediamine was removed using an azeotropic mixture of the toluene and the methanol (9:1). The residual toluene was removed by azeotropic distillation with the methanol. Finally, white powder (10.5 g) was obtained, which was recrystallized repeatedly using the chloroform and cyclohexane to afford a white solid. ¹H-NMR (300MHz, CDCl₃): 0.88 (t, 3H), 1.25 (s, 30H), 1.84 (s, 4H), 2.38 (m, 6H), 2.73 (m, 4H), 2.82 (m, 4H), 3.29 (m, 4H), 7.47 (s, 2H).

The preparation methods for compounds 6008 and 6009 are only different in that an equimolar amount of tetradecylamine was used to replace hexadecylamine. Compounds 6010-6017 were prepared by using an equimolar amount of corresponding Rₐ-NH₂ to replace hexadecylamine with reference to the preparation method of compound 6009.

Compound 6018 was prepared by using an equimolar amount of corresponding Rₐ-NH₂ to replace octadecylamine with reference to the preparation method of compound 6007.

### 9. Compound 6019

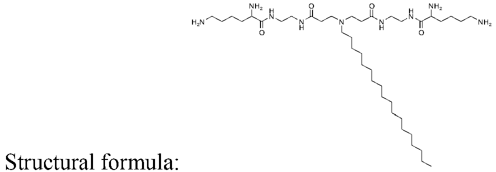

Molecular weight: 753.66

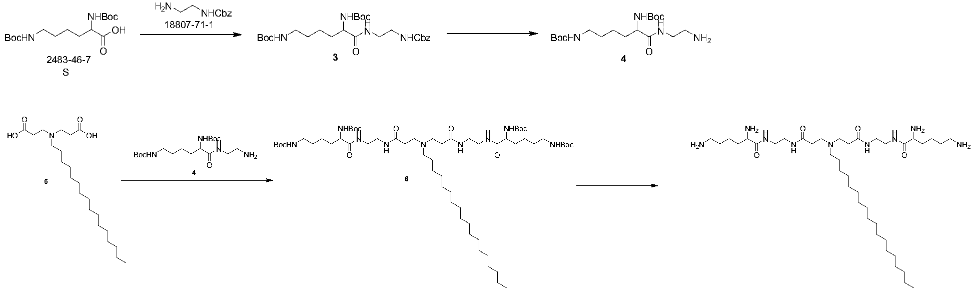

At the room temperature, compound 2483-46-7 (3 g) was added to a single-neck flask, followed by addition of the DCM (50 mL) with stirring and mixing. Compound 18807-71-1 (2.5 g), the DCC (2.7 g), and the DMAP (1.6 g) were then added for mixing with stirring at the room temperature overnight. A reaction mixture was concentrated under reduced pressure. A resutlant was purified via column chromatography (DCM: MeOH = 5:1) to afford product 3 (4 g). At the room temperature, product 3 (5.0 g), the MeOH (50 mL), and Pd/C (1.0 g) were added to a three-neck flask. The system was purged with the hydrogen, heated to 50°C and stirred for 1 h. Upon completion of the reaction, a reaction mixture was cooled to the room temperature and then filtered. A filtrate was subjected to solvent evaporation under reduced pressure to afford product 4 (3.5 g). At the room temperature, starting material 5 (1.2 g) was added to a single-neck flask and dissolved into the DMF (50 mL) with stirring. Product 4 (3.3 g), HATU (3.2 g), and the TEA (0.9 g) were then added, and mixed with stirring at the room temperature overnight. The water (500 mL) and the EA (500 mL) were added, and then a reaction mixture was subjected to phase separation and extraction. An organic phase was dried over anhydrous Na₂SO₄ and concentrated under reduced pressure. A crude product was purified via column chromatography (DCM: MeOH = (20:1)-(10:1)) to afford solid 6 (380 mg). At the room temperature, solid 6 (380 mg) and HCl/Dioxane (4 M) (1.6 mL) were added to a single-neck flask for a reaction at the room temperature for 2 h. A reaction mixture was concentrated under reduced pressure. A crude product was purified to afford compound 6019 (220 mg). ¹H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz,4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 10. Compound 6020

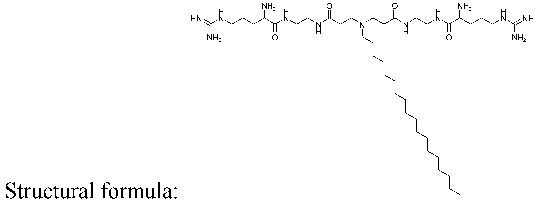

Molecular weight: 810.19

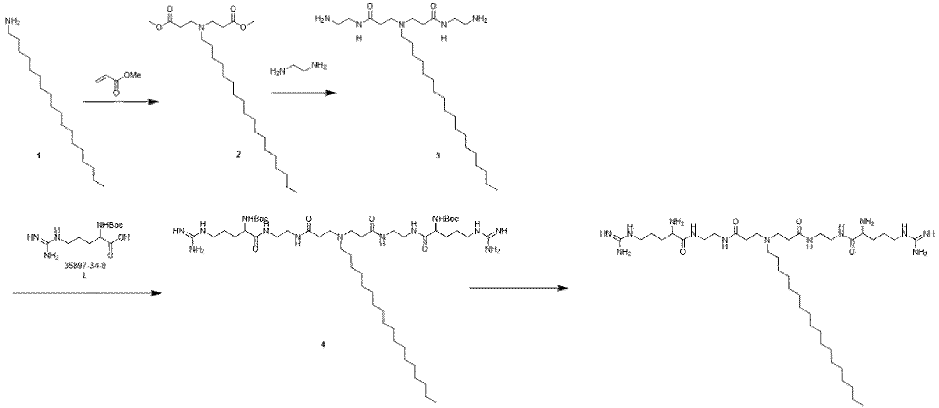

At room temperature, the octadecylamine (50 g) and the MeOH (200 mL) as compounds were added to a three-neck flask for mixing with stirring. A reaction mixture was cooled to 5°C in an ice-water bath, followed by dropwise addition of the methyl acrylate (31.5 g) for mixing with stirring. A reaction mixture was warmed back to the room temperature and underwent a reaction for 4 h. Upon completion of the reaction, the reaction mixture was directly concentrated under reduced pressure. A crude product was purified via column chromatography (PE:EA = (20:1)-(10:1)) to afford product 2 (68 g). At the room temperature, product 2 (15.0 g), the MeOH (100 mL), and the ethylenediamine (1.0 g) were added to a single-neck flask. A mixture was heated to 60°C and ,maintained at this temperature for a reaction overnight. Upon completion of the reaction, a reaction mixture was concentrated under reduced pressure. 30 mL of the toluene was added, and a mixture was heated to 60°C for dissolution, and then slowly cooled to the room temperature. A resultant was filtered, and a filtrate was subjected to solvent evaporation under reduced pressure to afford compound 3 (15 g). At the room temperature, compound 3 (1.3 g) and the DMF (20 mL) were added to a single-neck flask for mixing with stirring. Compound 35897-34-8 (3.6 g), the EDCI (3.0 g), the HOBt (2.1 g), and the DIEA (2.0 g) were then added for mixing with stirring, and a mixture underwent a reaction at the room temperature overnight. The water (200 mL) was added, and the mixture was directly lyophilized under reduced pressure to afford compound 4 (4 g). At the room temperature, compound 4 (100 mg) and the HCl/Dioxane (4M) (2 mL) were added to a single-neck flask for a reaction at the room temperature for 1 h. Upon completion of the reaction, a reaction mixture was concentrated under reduced pressure and purified to afford compound 6020. ¹H NMR (400 MHz, Methanol-d4) δ 3.90 (t, J = 6.4 Hz, 2H), 3.57-3.34 (m, 10H), 3.24 (dt, J = 16.1, 8.0 Hz, 8H), 2.77 (t, J = 6.5 Hz, 4H), 2.03-1.58 (m, 10H), 1.31 (s, 30H), 1.01-0.82 (m, 3H).

### 11. Compound 6021

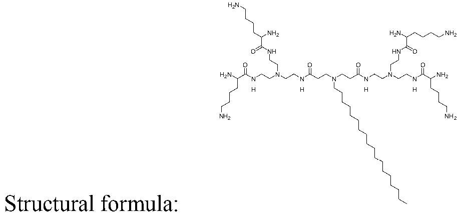

Molecular weight: 1182.79

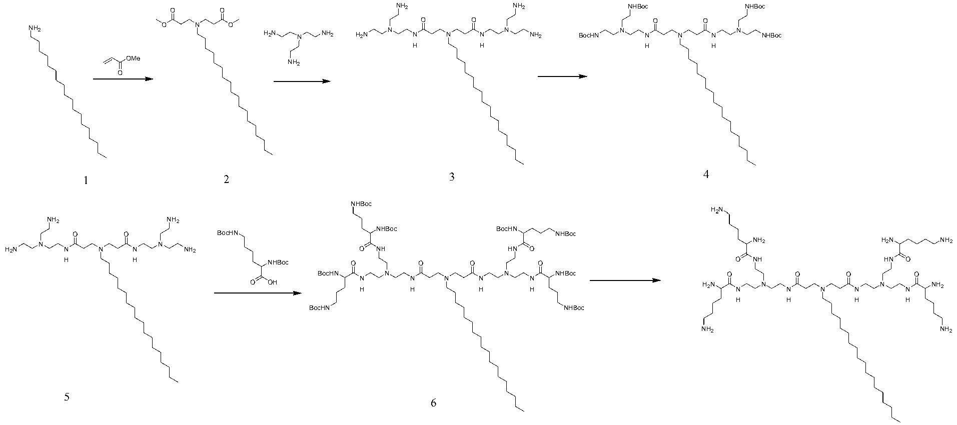

At the room temperature, compound **2** (3.5 g, with the synthetic process being the same as that of product 2 in compound 6020) and the MeOH (20 mL) were added to a single-neck flask for mixing with stirring. Tris(2-aminoethyl)amine (TREN) (23.0 g) was then added, followed by mixing with stirring. A mixture was heated to 60°C and stirred overnight, followed by concentration under reduced pressure. An obtained crude product was lyophilized to afford product 3 (25 g). At the room temperature, product 3 (22.0 g) and the DCM (200 mL) were added to another single-neck flask. A mixture was cooled to 0°C, and then di-tert-butyl dicarbonate ((Boc)₂O, 71.7 g) was added dropwise slowly. After dropwise addition was complete, a mixture was warmed back to the room temperature and underwent a reaction for 3 h. The water (200 mL) was added to quench the reaction, and additional DCM (100 mL) was added for phase separation and extraction. An organic phase was dried over sodium sulfate and concentrated under reduced pressure. A crude product was purified via column chromatography to afford yellow oil 4 (5.8 g). At the room temperature, yellow oil 4 (1.4 g) was added to a single-neck flask. HCl/Dioxane (4 M) (15 mL) was slowly added under an ice-water bath, followed by mixing with stirring. Upon completion of addition, a mixture was allowed to warm back to the room temperature and underwent a reaction for 0.5 h. A reaction mixture was concentrated under reduced pressure to afford compound 5 (800 mg). At the room temperature, compound 5 (1.0 g), (S)-2,6-di(tert-butoxycarbonylamino)hexanoic acid (2.6 g), the EDCI (1.4 g), the HOBt (1.0 g), the DIEA (1.0 g), and the DMF (10 mL) as a solvent were added to a single-neck flask for a reaction at the room temperature overnight. The water (100 mL) and the EA (100 mL) were added, and a mixture was subjected to phase separation and extraction. An EA phase was washed once with 100 mL of the saturated brine, and then concentrated under reduced pressure. A crude product was purified via column chromatography (DCM: MeOH = 10:1) to afford compound 6 (1.1 g). At the room temperature, compound 6 (1.2 g), HCl/Dioxane (4M) (10 mL), and the DCM (10 mL) were added to a single-neck flask for a reaction at the room temperature overnight. A reaction mixture was concentrated under reduced pressure and purified to afford compound 6021 (220 mg). ¹H NMR (400 MHz, Methanol-d4) δ 3.96 (t, J = 6.6 Hz, 4H), 3.75 (dt, J = 14.0, 6.6 Hz, 4H), 3.62-3.45 (m, 12H), 3.36-3.33 (m, 14H), 3.25-3.17 (m, 2H), 3.04-2.90 (m, 8H), 2.83 (t, J = 6.9 Hz, 4H), 2.05-1.82 (m, 8H), 1.73 (p, J = 7.7 Hz, 10H), 1.53 (qd, J = 8.3, 7.7, 4.3 Hz, 8H), 1.30 (s, 28H), 0.97-0.87 (m, 3H).

### 12. Compound 6023

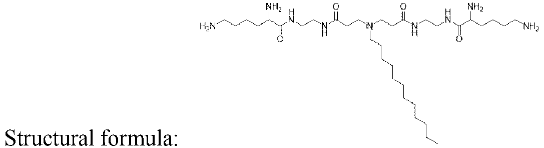

Molecular weight: 670

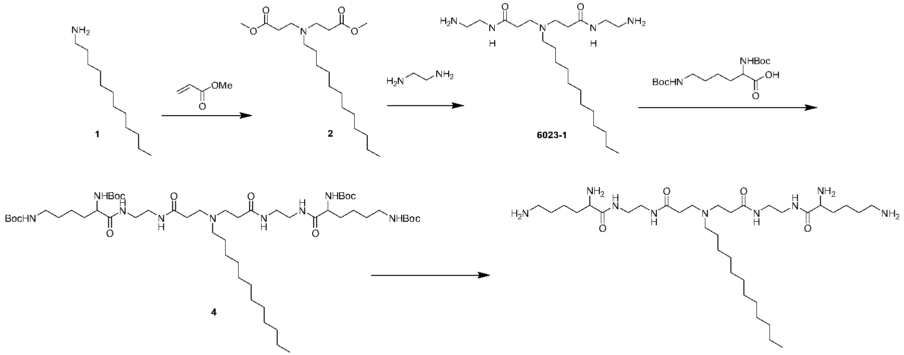

At the room temperature, dodecylamine (10 g) as a compound and the MeOH (100 mL) were added to a three-neck flask for mixing with stirring. A mixture was cooled to 5°C in an ice-water bath, and subsequently the methyl acrylate (10.2 g) was added dropwise slowly, followed by mixing with stirring. A reaction mixture was warmed back to the room temperature for a reaction at the room temperature for 4 h. Upon completion of the reaction, a reaction mixture was concentrated under reduced pressure. A crude product was purified via column chromatography (PE:EA = (20:1)-(10:1)) to afford product 2 (19 g). At the room temperature, product 2 (19.0 g), the MeOH (200 mL), and the ethylenediamine (127.7 g) were added to a single-neck flask. A mixture was heated to 60°C, maintained at this temperature overnight, and then concentrated under reduced pressure to afford compound 3 (20 g). 500 mg of compound 3 was purified and lyophilized to afford compound 6023-1 (218 mg). At the room temperature, starting material **6023-1** (1.4 g) was added to a single-neck flask and dissolved into the DCM (20 mL) with stirring. The (S)-2,6-di(tert-butoxycarbonylamino)hexanoic acid (3.4 g), the EDCI (1.9 g), and the DMAP (1.2 g) were subsequently added for mixing with stirring. A reaction mixture was kept at the room temperature for a reaction overnight, followed by phase separation and extraction. An organic phase was dried and concentrated under reduced pressure to afford compound 4 (3.0 g). At the room temperature, compound 4 (2.8 g) and HCl/1,4-Dioxane (4 M) (20 mL) were added to a single-neck flask, and the mixture underwent a reaction at the room temperature for 2 h. A reaction mixture was concentrated under reduced pressure, purified and lyophilized to afford compound 6023 (123 mg). ¹H NMR (400 MHz, Methanol-d4) δ 3.88 (t, J = 6.6 Hz, 2H), 3.55-3.36 (m, 8H), 3.36-3.33 (m, 2H), 3.31-3.14 (m, 4H), 3.04-2.93 (m, 4H),2.77 (t, J = 6.5 Hz, 4H), 2.00-1.82 (m, 4H), 1.82-1.66 (m, 6H), 1.50 (qd, J = 8.2, 7.8, 4.2 Hz, 4H), 1.45-1.25 (m, 18H), 0.96-0.87 (m, 3H).

### 13. Compound 6024

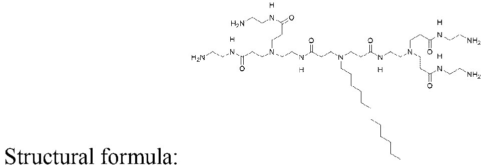

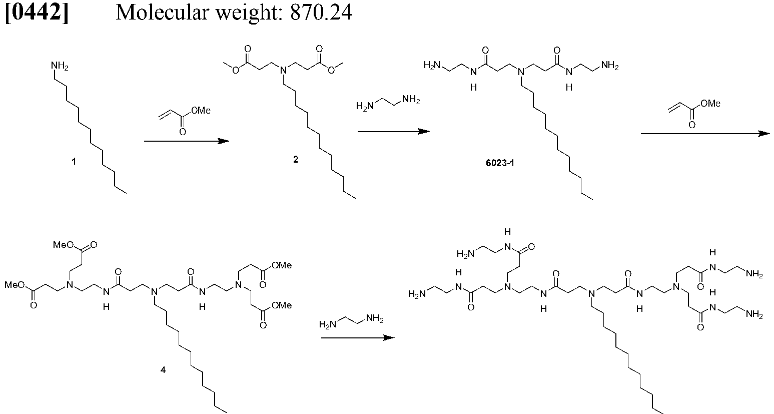

At room temperature, product 6023-1 (1.4 g, with the synthetic method being the same as that of compound 6023-1 described in synthesis of compound 6023) was added to a single-neck flask and dissolved into the MeOH (20 mL) with stirring. The methyl acrylate (2.8 g) was then added slowly with stirring and mixing. A mixture was heated to 60°C for a reaction at this temperature overnight, and a reactant was concentrated under reduced pressure to afford compound 4 (2.0 g). At the room temperature, compound 4 (2.4 g) and the ethanediamine (20 mL) were added to the single-neck flask and underwent a reaction at the room temperature overnight. A reaction mixture was concentrated under reduced pressure and then purified to afford compound 6024 (191 mg). ¹H NMR (400 MHz, Methanol-d4) δ 3.69 (t, J = 6.0 Hz, 4H), 3.60-3.48 (m, 20H), 3.42 (t, J = 6.1 Hz, 4H), 3.25-3.19 (m, 2H),3.12 (t, J = 5.8 Hz, 8H), 2.83 (t, J = 6.5 Hz, 12H), 1.78 (tt, J = 11.0, 6.4 Hz, 2H), 1.51-1.18 (m, 20H), 0.91 (t, J = 6.7 Hz, 3H).

### 14. Compound 6026

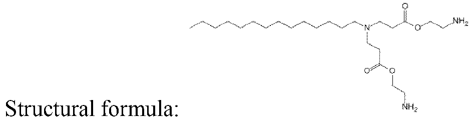

Molecular weight: 443.67

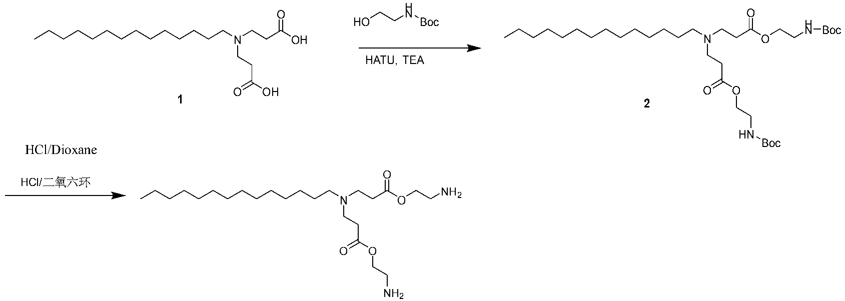

Compound 6026 was synthesized with reference to the synthetic route of compound 6019, only except that an equimolar amount of compound 1 was used to replace starting material 5 for compound 6019, and an equimolar amount of the N-(tert-butoxycarbonyl)ethanolamine was used to replace intermediate 4 of compound 6019. ¹H NMR (300 MHz, DMSO) δ: 4.51 (t, J = 7.3 Hz, 4H), 3.76 (t, J = 6.1 Hz, 4H), 3.18 (t, J = 6.7 Hz, 4H), 3.01 (t, J = 5.8 Hz, 2H), 2.49 (t, J = 7.1 Hz, 4H), 1.36 -1.26 (m, 24H), 0.89 (t, J = 6.2 Hz, 3H).

Compounds 6025-6034 were prepared with reference to the synthetic route of the compound described above, except that the ethanolamine was used to replace corresponding ethylenediamine, the amino in the ethanolamine was protected with a protecting group such as Fmoc or Boc, and the protecting group was removed according to a conventional method after the reaction was completed.

### (3) Exemplary compounds of first ionizable lipid and their properties

The exemplary compounds of the present application and their properties are listed in Table 1, which are synthesized according to the process routes in Preparation example 1.

Calculated c-pKa (molnetwork) and LogP (cLogP driver) values of the exemplary compounds of the present application are generated using a ChemDraw module of ChemOffice.

**Table 1 Properties of Exemplary Compounds of First Ionizable Lipid**

| **Number** | **Molecular formula** | **Molecular weight** | **c-pK a** | **cLog P** | **Nu mbe r** | **Molecular formula** | **Molecular weight** | **c-p Ka** | **cLog P** |
|---|---|---|---|---|---|---|---|---|---|
| 1001 | C₅₉H₁₀₈N₂O₇ | 957.52 | 9.83 | 21.43 | 1201 | C₇₁H₁₄₈N₆O | 1102.01 | 9.01 | 28.48 |
| 1002 | C₆₀H₁₁₆N₂O₇ | 977.60 | 6.97 | 24.49 | 1202 | C₉₂H₁₉₀N₆O | 1396.57 | 9.01 | 39.59 |
| 1003 | C₆₃H₁₂₂N₂O₇ | 1019.68 | 6.97 | 26.07 | 1203 | C₆₅H₁₃₆N₆O | 1017.84 | 9.01 | 25.31 |
| 1004 | C₅₄H₁₀₄N₂O₇ | 893.43 | 6.97 | 21.97 | 1204 | C₆₅H₁₃₆N₆O | 1017.84 | 9.01 | 25.31 |
| 1005 | C₆₈H₁₃₃N₃O₇ | 1104.83 | 9.01 | 26.86 | 1205 | C₉₈H₂₀₂N₆O | 1480.73 | 9.01 | 43.03 |
| 1006 | C₅₃H₁₀₃N₃O₇ | 894.42 | 9.01 | 19.58 | 1206 | C₉₂H₁₉₀N₆O | 1396.57 | 9.01 | 39.86 |
| 1007 | C₆₈H₁₃₃N₃O₇ | 1104.83 | 9.01 | 26.86 | 1207 | C₈₃H₁₇₂N₆O | 1270.33 | 9.01 | 35.10 |
| 1008 | C₆₈H₁₃₃N₃O₇ | 1104.01 | 9.01 | 26.86 | 1208 | C₆₅H₁₃₀N₆O | 1011.80 | 9.01 | 24.06 |
| 1009 | C₆₂H₁₂₁N₃O₇ | 1020.66 | 9.01 | 23.69 | 1209 | C₆₅H₁₃₀N₆O | 1011.80 | 9.01 | 23.97 |
| 1010 | C₆₇H₁₃₃N₃O₆ | 1076.82 | 9.78 | 27.13 | 1210 | C₄₄H₉₄N₆O | 723.28 | 9.01 | 14.86 |
| 1011 | C₇₀H₁₃₇N₃O₇ | 1132.88 | 9.07 | 27.92 | 1211 | C₅₆H₁₀₆N₆O | 879.50 | 9.01 | 18.30 |
| 1012 | C₇₂H₁₄₁N₃O₄ | 1160.93 | 9.10 | 28.98 | 1212 | C₆₂H₁₁₈N₆O | 963.67 | 9.01 | 20.30 |
| 1013 | C₇₄H₁₄₅N₃O₇ | 1188.99 | 9.12 | 30.03 | 1213 | C₅₀H₁₀₀N₆O | 801.39 | 9.01 | 16.58 |
| 1014 | C₇₀H₁₃₇N₃O₉ | 1164.88 | 7.40 | 26.03 | 1214 | C₆₂H₁₂₄N₆O | 969.71 | 9.01 | 22.93 |
| 1015 | C₇₂H₁₄₁N₃O₉ | 1192.93 | 8.33 | 26.61 | 1215 | C₆₂H₁₃₀N₆O | 975.76 | 9.01 | 24.38 |
| 1016 | C₆₅H₁₂₉N₃O₈ | 1080.80 | 9.11 | 23.70 | 1216 | C₆₈H₁₁₈N₆O | 1035.73 | 9.01 | 25.13 |
| 1017 | C₇₁H₁₄₁N₃O₈ | 1164.90 | 9.10 | 26.87 | 1217 | C₈₃H₁₄₈N₆O | 1246.14 | 9.01 | 32.83 |
| 1018 | C₇₂H₁₄₀N₂O₁₀ | 1193.92 | 8.15 | 27.21 | 1218 | C₅₉H₁₂₁N₃OS₆ | 1081.00 | 9.01 | 27.15 |
| 1019 | C₇₂H_{I40}N₂O₁₀ | 1193.92 | 8.15 | 27.21 | 1219 | C₅₉H₁₂₁N₃OS₆ | 1081.00 | 9.01 | 27.15 |
| 1020 | C₇₄H₁₄₅N₃O₉ | 1220.99 | 8.74 | 26.06 | 1220 | C₇₁H₁₄₅N₃OS₆ | 1249.32 | 9.01 | 33.50 |
| 1021 | C₇₄H₁₄₅N₃O₉ | 1221.00 | 8.74 | 26.06 | 1221 | C₉₂H₁₈₇N₃OS₆ | 1543.89 | 9.01 | 44.61 |
| 1022 | C₇₃H₁₄₅N₃O₈ | 1193.00 | 9.51 | 26.33 | 1222 | C₆₅H₁₃₃N₃OS₆ | 1165.16 | 9.01 | 30.32 |
| 1023 | C₇₄H₁₄₄N₂O₁₀ | 1221.97 | 8.55 | 26.66 | 1223 | C₁₀₀H₁₉₈N₄O₁₀S ₂ | 1680.82 | 8.90 | 43.24 |
| 1024 | C₇₄H₁₄₄N₂O₁₀ | 1221.97 | 8.55 | 26.66 | 1224 | C₉₈H₁₉₉N₃OS₆ | 1628.05 | 9.01 | 48.05 |
| 1025 | C₆₉H₁₃₅N₃O₈ | 1134.85 | 8.20 | 26.44 | 1225 | C₉₂H₁₈₇N₃OS₆ | 1543.89 | 9.01 | 44.88 |
| 1026 | C₆₉H₁₃₄N₂O₉ | 1135.84 | 8.02 | 27.05 | 1226 | C₈₃H₁₆₉N₃OS₆ | 1417.64 | 9.01 | 40.11 |
| 1027 | C₆₉H₁₃₄N₂O₉ | 1135.84 | 8.02 | 27.05 | 1227 | C₆₅H₁₂₇N₃OS₆ | 1159.11 | 9.01 | 29.08 |
| 1028 | C₆₄H₁₂₅N₃O₇ | 1048.70 | 9.07 | 24.74 | 1228 | C₆₅H₁₂₇N₃OS₆ | 1159.11 | 9.01 | 28.99 |
| 1029 | C₆₈H₁₃₂N₂O₈ | 1105.81 | 8.83 | 27.46 | 1229 | C₄₄H₉₁N₃OS₆ | 870.59 | 9.01 | 19.87 |
| 1030 | C₆₆H₁₂₉N₃O₇ | 1076.80 | 9.10 | 25.80 | 1230 | C₅₆H₁₀₃N₃OS₆ | 1026.82 | 9.01 | 23.32 |
| 1031 | C₆₅H₁₂₉N₃O₆ | 1048.80 | 9.86 | 26.07 | 1231 | C₆₂H₁₁₅N₃OS₆ | 1110.98 | 9.01 | 25.32 |
| 1032 | C₆₉H₁₃₅N₃O₇ | 1118.90 | 9.10 | 27.39 | 1232 | C₅₀H₉₇N₃OS₆ | 948.71 | 9.01 | 21.60 |
| 1033 | C₆₆H₁₂₈N₂O₈ | 1077.80 | 8.91 | 26.41 | 1233 | C₆₂H₁₂₁N₃OS₆ | 1117.03 | 9.01 | 27.94 |
| 1034 | C₆₈H₁₃₃N₃O₇ | 1104.83 | 9.12 | 26.86 | 1234 | C₆₂H₁₂₇N₃OS₆ | 1123.08 | 9.01 | 29.40 |
| 1035 | C₇₁H₁₃₉N₃O₇ | 1146.91 | 9.12 | 28.45 | 1235 | C₆₈H₁₁₅N₃OS₆ | 1183.05 | 9.01 | 27.86 |
| 1036 | C₆₈H₁₃₂N₂O₈ | 1105.80 | 8.93 | 27.46 | 1236 | C₈₃H₁₄₅N₃OS₆ | 1393.45 | 9.01 | 35.56 |
| 1037 | C₆₄H₁₂₅N₃O₉ | 1080.70 | 7.40 | 22.85 | 1237 | C₆₂H₁₂₁N₃O₄ | 972.67 | 9.01 | 23.48 |
| 1038 | C₆₇H₁₃₁N₃O₉ | 1122.80 | 7.40 | 24.44 | 1238 | C₇₄H₁₄₅N₃O₇ | 1140.99 | 9.01 | 29.83 |
| 1039 | C₆₄H₁₂₄N₂O₁₀ | 1081.70 | 7.21 | 23.46 | 1239 | C₉₅H₁₈₇N₃O₄ | 1435.56 | 9.01 | 40.94 |
| 1040 | C₆₉H₁₃₅N₃O₉ | 1150.90 | 8.33 | 25.02 | 1240 | C₆₈H₁₃₃N₃O₄ | 1056.83 | 9.01 | 26.65 |
| 1041 | C₆₆H₁₂₈N₂O₁₀ | 1109.80 | 8.15 | 24.04 | 1241 | C₆₈H₁₃₃N₃O₄ | 1056.83 | 9.01 | 26.65 |
| 1042 | C₆₈H₁₃₂N₂O₁₀ | 1137.80 | 8.55 | 23.49 | 1242 | C₁₀₁H₁₉₉N₃O₄ | 1519.72 | 9.01 | 43.72 |
| 1043 | C₇₀H₁₃₈N₄O₈ | 1163.89 | 9.40 | 25.69 | 1243 | C₉₅H₁₈₇N₃O₄ | 1435.56 | 9.01 | 40.55 |
| 1044 | C₆₄H₁₂₆N₄O₈ | 1079.73 | 9.40 | 22.52 | 1244 | C₈₆H₁₆₉N₃O₄ | 1309.32 | 9.01 | 35.79 |
| 1045 | C₇₀H₁₃₈N₄O₈ | 1163.89 | 9.40 | 25.69 | 1245 | C₆₈H₁₂₇N₃O₄ | 1050.78 | 9.01 | 24.75 |
| 1046 | C₇₀H₁₃₈N₄O₈ | 1163.89 | 9.40 | 25.69 | 1246 | C₆₈H₁₂₇N₃O₄ | 1050.78 | 9.01 | 24.66 |
| 1047 | C₆₇H₁₃₁N₃O₈ | 1106.80 | 8.95 | 25.14 | 1247 | C₄₇H₉₁N₃O₄ | 762.26 | 9.01 | 15.54 |
| 1048 | C₆₁H₁₁₉N₃O₈ | 1022.64 | 8.95 | 21.96 | 1248 | C₅₉H₁₀₃N₃O₄ | 918.49 | 9.01 | 18.99 |
| 1049 | C₆₇H₁₃₁N₃O₈ | 1106.80 | 8.95 | 25.14 | 1249 | C₆₅H₁₁₅N₃O₄ | 1002.65 | 9.01 | 20.99 |
| 1050 | C₇₂H₁₃₉NO₉ | 1162.90 | 9.10 | 28.52 | 1250 | C₅₃H₉₇N₃O₄ | 840.38 | 9.01 | 17.27 |
| 1051 | C₇₄H₁₃₉N₃O₁₃ | 1278.90 | 9.01 | 23.74 | 1251 | C₆₅H₁₂₁N₃O₄ | 1008.70 | 9.01 | 23.61 |
| 1052 | C₇₇H₁₅₀N₂O₇ | 1216.10 | 9.83 | 32.01 | 1252 | C₆₅H₁₂₇N₃O₄ | 1014.75 | 9.01 | 25.07 |
| 1053 | C₇₃H₁₃₇N₂O₆P | 1169.88 | 9.68 | 25.77 | 1253 | C₇₁H₁₁₅N₃O₄ | 1074.72 | 9.01 | 25.00 |
| 1054 | C₇₄H₁₃₈N₃O₇P | 1212.91 | 9.95 | 26.18 | 1254 | C₈₆H₁₄₅N₃O₄ | 1285.12 | 9.01 | 32.69 |
| 1055 | C₇₁H₁₃₆N₃O₃PS ₉ | 1399.40 | 8.85 | 32.41 | 1255 | C₉₉H₁₉₅N₃O₃S₄ | 1603.91 | 8.68 | 43.96 |
| 1056 | C₆₈H₁₃₈N₂O₅ | 1063.90 | 9.70 | 26.03 | 1256 | C₇₄H₁₄₅N₃O₇ | 1188.99 | 9.01 | 30.03 |
| 1057 | C₇₀H₁₄₀N₄O₆S₂ | 1198.03 | 9.67 | 24.35 | 1257 | C₉₅H₁₈₇N₃O₇ | 1483.55 | 9.01 | 41.14 |
| 1058 | C₆₇H₁₃₂N₂O₇ | 1077.80 | 9.45 | 27.29 | 1258 | C₁₀₄H₂₀₀N₃O₇PS ₄ | 1763.97 | 7.94 | 41.36 |
| 1059 | C₆₇H₁₃₂N₂O₇ | 1077.80 | 9.45 | 27.29 | 1259 | C₁₀₃H₂₀₀N₂OS₇ | 1707.17 | 10.0 9 | 46.14 |
| 1060 | C₆₇H₁₃₃N₃O₈S | 1140.87 | 8.74 | 26.96 | 1260 | C₁₀₁H₁₉₉N₃O₇ | 1567.72 | 9.01 | 44.59 |
| 1061 | C₇₃H₁₂₅N₃O₅ | 1124.82 | 9.77 | 23.41 | 1261 | C₉₅H₁₈₇N₃O₇ | 1483.55 | 9.01 | 41.41 |
| 1062 | C₆₇H₁₁₃N₃O₁₀S ₃ | 1216.83 | 9.73 | 22.69 | 1262 | C₈₆H₁₆₉N₃O₇ | 1357.31 | 9.01 | 36.65 |
| 1063 | C₆₅H₁₀₉N₃O₁₀S ₃ | 1188.78 | 9.01 | 21.48 | 1263 | C₆₈H₁₂₇N₃O₇ | 1098.78 | 9.01 | 25.62 |
| 1064 | C₆₈H₁₃₉N₃OS₆ | 1207.24 | 9.01 | 32.18 | 1264 | C₆₈H₁₂₇N₃O₇ | 1098.78 | 9.01 | 25.53 |
| 1065 | C₆₈H₁₃₉N₃OS₆ | 1207.24 | 9.01 | 32.18 | 1265 | C₄₇H₉₁N₃O₇ | 810.26 | 9.01 | 16.41 |
| 1066 | C₇₄H₁₅₄N₃O₁₃P ₃ | 1386.98 | 9.01 | 26.51 | 1266 | C₅₉H₁₀₃N₃O₇ | 966.49 | 9.01 | 19.85 |
| 1067 | C₇₄H₁₅₄N₃O₁₃P ₃ | 1386.98 | 9.01 | 26.51 | 1267 | C₆₅H₁₁₅N₃O₇ | 1050.65 | 9.01 | 21.86 |
| 1068 | C₇₁H₁₃₁N₃O₇ | 1138.84 | 5.07 | 26.81 | 1268 | C₅₃H₉₇N₃O₇ | 888.37 | 9.01 | 18.13 |
| 1069 | C₇₁H₁₃₁N₃O₇ | 1138.84 | 5.04 | 26.81 | 1269 | C₆₅H₁₂₁N₃O₇ | 1056.70 | 9.01 | 24.48 |
| 1070 | C₆₉H₁₃₀N₄O₇ | 1127.82 | 5.03 | 26.38 | 1270 | C₆₅H₁₂₇N₃O₇ | 1062.74 | 9.01 | 25.93 |
| 1071 | C₆₉H₁₃₀N₄O₇ | 1127.82 | 5.00 | 26.38 | 1271 | C₇₁H₁₁₅N₃O₄ | 1122.71 | 9.01 | 26.17 |
| 1072 | C₇₀H₁₃₅N₃O₈ | 1146.86 | 6.83 | 26.82 | 1272 | C₈₆H₁₄₅N₃O₇ | 1333.12 | 9.01 | 33.87 |
| 1073 | C₇₀H₁₃₅N₃O₈ | 1146.86 | 6.83 | 26.82 | 1273 | C₆₂H₁₂₁N₃O₇ | 1020.66 | 9.01 | 23.69 |
| 1074 | C₇₁H₁₃₈N₄O₈ | 1175.91 | 7.89 | 26.41 | 1274 | C₇₄H₁₄₅N₃O₇ | 1188.99 | 9.01 | 30.03 |
| 1075 | C₇₁H₁₃₈N₄O₈ | 1175.91 | 7.89 | 26.41 | 1275 | C₉₅H₁₈₇N₃O₇ | 1483.55 | 9.01 | 41.14 |
| 1076 | C₇₁H₁₃₈N₄O₉ | 1191.90 | 7.80 | 26.25 | 1276 | C₆₈H₁₃₃N₃O₇ | 1104.83 | 9.01 | 26.86 |
| 1077 | C₇₁H₁₃₈N₄O₉ | 1191.90 | 7.80 | 26.25 | 1277 | C₆₈H₁₃₃N₃O₇ | 1104.83 | 9.01 | 26.86 |
| 1078 | C₇₁H₁₃₈N₄O₉ | 1191.90 | 7.75 | 27.09 | 1278 | C₁₀₁H₁₉₉N₃O₇ | 1567.72 | 9.01 | 44.59 |
| 1079 | C₇₁H₁₃₈N₄O₉ | 1191.90 | 7.75 | 27.09 | 1279 | C₉₅H₁₈₇N₃O₇ | 1483.55 | 9.01 | 41.41 |
| 1080 | C₇₀H₁₃₈N₄O₉S | 1211.95 | 7.61 | 26.56 | 1280 | C₈₆H₁₆₉N₃O₇ | 1357.31 | 9.01 | 36.65 |
| 1081 | C₇₀H₁₃₈N₄O₉S | 1211.95 | 7.61 | 26.56 | 1281 | C₆₈H₁₂₇N₃O₇ | 1098.78 | 9.01 | 25.62 |
| 1082 | C₇₂H₁₃₉N₅O₈ | 1202.93 | 7.82 | 26.46 | 1282 | C₆₈H₁₂₇N₃O₇ | 1098.78 | 9.01 | 25.53 |
| 1083 | C₇₂H₁₃₉N₅O₈ | 1202.93 | 7.82 | 26.46 | 1283 | C₄₇H₉₁N₃O₇ | 810.26 | 9.01 | 16.41 |
| 1084 | C₇₂H₁₃₇N₅O₇S | 1216.98 | 7.95 | 28.08 | 1284 | C₅₉H₁₀₃N₃O₇ | 966.49 | 9.01 | 19.85 |
| 1085 | C₇₂H₁₃₇N₅O₇S | 1216.98 | 7.95 | 28.08 | 1285 | C₆₅H₁₁₅N₃O₇ | 1050.65 | 9.01 | 21.86 |
| 1086 | C₇₄H₁₃₈N₈O₇ | 1251.97 | 5.88 | 25.00 | 1286 | C₅₃H₉₇N₃O₇ | 888.37 | 9.01 | 18.13 |
| 1087 | C₇₄H₁₃₈N₈O₇ | 1251.97 | 5.88 | 25.00 | 1287 | C₆₅H₁₂₁N₃O₇ | 1056.70 | 9.01 | 24.48 |
| 1088 | C₇₄H₁₃₈N₈O₈ | 1267.97 | 7.59 | 25.58 | 1288 | C₆₅H₁₂₇N₃O₇ | 1062.74 | 9.01 | 25.93 |
| 1089 | C₇₄H₁₃₈N₈O₈ | 1267.97 | 7.59 | 25.58 | 1289 | C₇₁H₁₁₅N₃O₇ | 1122.71 | 9.01 | 24.80 |
| 1090 | C₇₃H₁₃₇N₅O₉ | 1228.93 | 7.07 | 26.15 | 1290 | C₈₆H₁₄₅N₃O₇ | 1333.12 | 9.01 | 32.49 |
| 1091 | C₇₃H₁₃₇N₅O₉ | 1228.93 | 7.07 | 26.15 | 1291 | C₆₂H₁₂₁N₃O₁₀ | 1068.66 | 9.01 | 23.51 |
| 1092 | C₇₄H₁₃₉N₅O₈ | 1226.95 | 7.47 | 26.45 | 1292 | C₇₄H₁₄₅N₃O₁₀ | 1236.98 | 9.01 | 29.86 |
| 1093 | C₇₄H₁₃₉N₅O₈ | 1226.95 | 7.47 | 26.45 | 1293 | C₉₅H₁₈₇N₃O₁₀ | 1531.55 | 9.01 | 40.96 |
| 1094 | C₇₀H₁₃₈N₆O₇ | 1175.91 | 8.12 | 25.90 | 1294 | C₆₈H₁₃₃N₃O₁₀ | 1152.82 | 9.01 | 26.68 |
| 1095 | C₇₀H₁₃₈N₆O₇ | 1175.91 | 8.12 | 25.90 | 1295 | C₆₈H₁₃₃N₃O₁₀ | 1152.82 | 9.01 | 26.68 |
| 1096 | C₇₂H₁₄₂N₆O₉S | 1268.02 | 7.63 | 26.40 | 1296 | C₁₀₁H₁₉₉N₃O₁₀ | 1615.71 | 9.01 | 44.41 |
| 1097 | C₇₂H₁₄₂N₆O₉S | 1268.02 | 7.63 | 26.40 | 1297 | C₉₅H₁₈₇N₃O₁₀ | 1531.55 | 9.01 | 41.23 |
| 1098 | C₇₃H₁₄₃N₅O₉S | 1267.03 | 7.51 | 27.09 | 1298 | C₈₆H₁₆₉N₃O₁₀ | 1405.31 | 9.01 | 36.47 |
| 1099 | C₇₃H₁₄₃N₅O₉S | 1267.03 | 7.51 | 27.09 | 1299 | C₆₈H₁₂₇N₃O₁₀ | 1146.77 | 9.01 | 25.44 |
| 1100 | C₇₄H₁₄₅N₅O₉S | 1281.06 | 8.31 | 27.25 | 1300 | C₆₈H₁₂₇N₃O₁₀ | 1146.77 | 9.01 | 25.35 |
| 1101 | C₇₃H₁₄₃N₅O₉S | 1267.03 | 7.51 | 27.09 | 1301 | C₄₇H₉₁N₃O₁₀ | 858.26 | 9.01 | 16.23 |
| 1102 | C₇₂H₁₃₉N₃O₇ | 1158.92 | 10.26 | 27.41 | 1302 | C₅₉H₁₀₃N₃O₁₀ | 1014.48 | 9.01 | 19.68 |
| 1103 | C₇₁H₁₃₈N₄O₇ | 1159.91 | 9.78 | 26.04 | 1303 | C₆₅H₁₁₅N₃O₁₀ | 1098.65 | 9.01 | 21.68 |
| 1104 | C₆₉H₁₃₃N₃O₇ | 1116.84 | 9.32 | 25.52 | 1304 | C₅₃H₉₇N₃O₁₀ | 936.37 | 9.01 | 17.95 |
| 1105 | C₆₉H₁₃₃N₃O₇ | 1116.84 | 9.32 | 25.52 | 1305 | C₆₅H₁₂₁N₃O₁₀ | 1104.69 | 9.01 | 24.30 |
| 1106 | C₆₉H₁₃₆N₆O₇ | 1161.88 | 7.46 | 24.33 | 1306 | C₆₅H₁₂₇N₃O₁₀ | 1110.74 | 9.01 | 25.75 |
| 1107 | C₆₃H₁₂₄N₆O₇ | 1077.72 | 7.46 | 21.15 | 1307 | C₇₁H₁₁₅N₃O₁₀ | 1170.71 | 9.01 | 24.31 |
| 1108 | C₆₉H₁₃₆N₆O₇ | 1161.88 | 7.46 | 24.33 | 1308 | C₈₆H₁₄₅N₃O₁₀ | 1381.12 | 9.01 | 32.00 |
| 1109 | C₆₉H₁₃₆N₆O₇ | 1161.88 | 7.46 | 24.33 | 1309 | C₆₂H₁₂₄N₆O₄ | 1017.71 | 9.01 | 20.89 |
| 1110 | C₇₀H₁₃₆N₄O₇ | 1145.88 | 8.82 | 26.41 | 1310 | C₇₄H₁₄₈N₆O₄ | 1186.04 | 9.01 | 27.24 |
| 1111 | C₇₀H₁₃₆N₄O₇ | 1145.88 | 8.82 | 26.41 | 1311 | C₉₅H₁₉₀N₆O₄ | 1480.60 | 9.01 | 38.35 |
| 1112 | C₆₉H₁₃₆N₂O₆ | 1089.85 | 10.19 | 28.16 | 1312 | C₆₈H₁₃₆N₆O₄ | 1101.87 | 9.01 | 24.06 |
| 1113 | C₇₁H₁₃₈N₂O₈ | 1147.89 | 9.69 | 26.58 | 1313 | C₆₈H₁₃₆N₆O₄ | 1101.87 | 9.01 | 24.06 |
| 1114 | C₇₀H₁₃₈N₂O₇ | 1119.88 | 9.84 | 26.71 | 1314 | C₁₀₁H₂₀₂N₆O₄ | 1564.76 | 9.01 | 41.79 |
| 1115 | C₆₇H₁₃₁N₃O₇ | 1090.80 | 9.50 | 26.91 | 1315 | C₉₅H₁₉₀N₆O₄ | 1480.60 | 9.01 | 38.62 |
| 1116 | C₆₉H₁₃₅N₃O₈ | 1134.85 | 9.52 | 27.58 | 1316 | C₈₆H₁₇₂N₆O₄ | 1354.36 | 9.01 | 33.85 |
| 1117 | C₇₁H₁₄₁N₃o₆ | 1132.92 | 9.88 | 29.03 | 1317 | C₆₈H₁₃₀N₆O₄ | 1095.83 | 9.01 | 22.82 |
| 1118 | C₇₁H₁₃₉N₃O₈ | 1162.91 | 8.87 | 26.46 | 1318 | C₆₈H₁₃₀N₆O₄ | 1095.83 | 9.01 | 22.73 |
| 1119 | C₇₁H₁₃₃NO₉S₅ | 1305.14 | 9.96 | 25.12 | 1319 | C₄₇H₉₄N₆O₄ | 807.31 | 9.01 | 13.61 |
| 1120 | C₇₂H₁₄₂N₂O₇ | 1147.90 | 10.05 | 27.76 | 1320 | C₅₉H₁₀₆N₆O₄ | 963.53 | 9.01 | 17.06 |
| 1121 | C₇₃H₁₃₉N₅O₁₅S ₄ | 1455.17 | 7.80 | 23.62 | 1321 | C₅₉H₁₀₆N₆O₄ | 963.53 | 9.01 | 17.06 |
| 1122 | C₆₈H₁₂₉NO₁₃S₃ | 1264.95 | 8.49 | 25.91 | 1322 | C₅₃H₁₀₀N₆O₄ | 885.42 | 9.01 | 15.34 |
| 1123 | C₇₇H₁₅₂N₂O₇ | 1218.07 | 9.99 | 30.68 | 1323 | C₆₅H₁₂₄N₆O₄ | 1053.74 | 9.01 | 21.68 |
| 1124 | C₆₇H₁₃₃N₃O₈S | 1140.87 | 8.74 | 26.96 | 1324 | C₆₅H₁₃₀N₆O₄ | 1059.79 | 9.01 | 23.14 |
| 1125 | C₆₉H₁₃₆N₄O₇ | 1133.87 | 9.61 | 27.37 | 1325 | C₇₁H₁₁₈N₆O₄ | 1119.76 | 9.01 | 23.40 |
| 1126 | C₆₅H₁₂₁N₃O₇ | 1056.70 | 9.01 | 23.82 | 1326 | C₈₆H₁₄₈N₆O₄ | 1330.17 | 9.01 | 31.09 |
| 1127 | C₆₉H₁₃₆N₂O₉ | 1137.85 | 8.31 | 26.05 | 1327 | C₆₂H₁₂₄N₆O₄ | 1017.71 | 9.01 | 20.89 |
| 1128 | C₇₀H₁₃₉N₂O₁₁P | 1215.86 | 7.77 | 26.53 | 1328 | C₇₄H₁₄₈N₆O₄ | 1186.04 | 9.01 | 27.24 |
| 1129 | C₆₈H₁₃₄N₂O₇S₂ | 1155.95 | 8.84 | 27.80 | 1329 | C₉₅H₁₉₀N₆O₄ | 1480.60 | 9.01 | 38.35 |
| 1130 | C₇₄H₁₅₄N₃O₁₃P ₃ | 1386.98 | 9.01 | 26.51 | 1330 | C₆₈H₁₃₆N₆O₄ | 1101.87 | 9.01 | 24.06 |
| 1131 | C₈₄H₁₅₉N₃O₈ | 1339.21 | 9.46 | 31.70 | 1331 | C₆₈H₁₃₆N₆O₄ | 1101.87 | 9.01 | 24.06 |
| 1132 | C₈₇H₁₄₇N₃O₈ | 1363.15 | 9.46 | 32.11 | 1332 | C₁₀₁H₂₀₁N₅O₅ | 1565.75 | 9.01 | 42.72 |
| 1133 | C₇₁H₁₃₁N₃O₇ | 1138.84 | 5.07 | 26.81 | 1333 | C₉₅H₁₈₉N₅O₅ | 1481.59 | 9.01 | 39.55 |
| 1134 | C₆₉H₁₃₀N₄O₇ | 1127.82 | 5.00 | 26.38 | 1334 | C₈₆H₁₇₁N₅O₅ | 1355.34 | 9.01 | 34.79 |
| 1135 | C₇₁H₁₃₈N₄O₈ | 1175.91 | 7.89 | 26.41 | 1335 | C₆₈H₁₃₀N₆O₄ | 1095.83 | 9.01 | 22.82 |
| 1136 | C₁₀₈H₂₀₄N₂O₉S ₂ | 1738.94 | 8.56 | 46.05 | 1336 | C₆₈H₁₂₉N₅O₅ | 1096.81 | 9.01 | 23.66 |
| 1137 | C₉₉H₁₉₅NO₆S₃ | 1591.83 | 7.68 | 42.68 | 1337 | C₄₇H₉₃N₅O₅ | 808.29 | 9.01 | 14.55 |
| 1138 | C₇₂H₁₃₇N₅O₇S | 1216.98 | 7.95 | 28.08 | 1338 | C₅₉H₁₀₅N₅O₅ | 964.52 | 9.01 | 17.99 |
| 1139 | C₇₄H₁₃₈N₈O₇ | 1251.97 | 5.88 | 25.00 | 1339 | C₆₅H₁₁₇N₅O₅ | 1048.68 | 9.01 | 19.99 |
| 1140 | C₇₀H₁₃₈N₆O₇ | 1175.91 | 8.12 | 25.90 | 1340 | C₅₃H₉₉N₅O₅ | 886.40 | 9.01 | 16.27 |
| 1141 | C₇₃H₁₄₃N₅O₉S | 1267.03 | 7.51 | 27.09 | 1341 | C₆₅H₁₂₃N₅O₅ | 1054.73 | 9.01 | 22.62 |
| 1142 | C₇₀H₁₃₅N₃O₇ | 1130.86 | 9.98 | 26.32 | 1342 | C₆₅H₁₂₉N₅O₅ | 1060.78 | 9.01 | 24.07 |
| 1143 | C₆₅H₁₁₅N₃O₇ | 1050.60 | 9.01 | 21.20 | 1343 | C₇₁H₁₁₇N₅O₅ | 1120.75 | 9.01 | 24.31 |
| 1144 | C₇₅H₁₄₁N₃O₈ | 1212.97 | 9.18 | 28.86 | 1344 | C₈₆H₁₄₈N₆O₄ | 1330.17 | 9.01 | 31.76 |
| 1145 | C₇₀H₁₁₈N₂O₁₁S ₃ | 1259.90 | 9.49 | 22.27 | 1345 | C₆₂H₁₂₄N₆O₇ | 1065.71 | 9.01 | 22.93 |
| 1146 | C₆₈H₁₃₄N₂O₇S₂ | 1155.95 | 9.14 | 27.39 | 1346 | C₇₄H₁₄₈N₆O₇ | 1234.03 | 9.01 | 29.28 |
| 1147 | C₈₆H₁₆₇N₃O₅S₂ | 1387.40 | 6.75 | 35.10 | 1347 | C₉₅H₁₉₀N₆O₇ | 1528.60 | 9.01 | 40.39 |
| 1148 | C₆₉H₁₂₀N₆O₂S₄ | 1194.00 | 8.63 | 24.08 | 1348 | C₆₈H₁₃₆N₆O₇ | 1149.87 | 9.01 | 26.10 |
| 1149 | C₇₆H₁₅₄N₆O₃S | 1232.20 | 7.65 | 30.39 | 1349 | C₆₈H₁₃₆N₆O₇ | 1149.87 | 9.01 | 26.10 |
| 1150 | C₉₁H₁₈₆N₄S₆ | 1528.90 | 9.77 | 43.45 | 1350 | C₁₀₁H₂₀₂N₆O₇ | 1612.76 | 9.01 | 43.83 |
| 1151 | C₇₈H₁₄₂N₆O₅ | 1244.00 | NA | 27.09 | 1351 | C₉₅H₁₉₀N₆O₇ | 1528.60 | 9.01 | 40.66 |
| 1152 | C₁₀₅H₁₈₁N₃O₁₂ | 1677.60 | 9.16 | 40.21 | 1352 | C₈₆H₁₇₂N₆O₇ | 1402.36 | 9.01 | 35.90 |
| 1153 | C₆₉H₁₃₁N₅O₅ | 1110.80 | 7.41 | 22.95 | 1353 | C₆₈H₁₃₀N₆O₇ | 1143.82 | 9.01 | 24.86 |
| 1154 | C₇₆H₁₃₉N₃S₃ | 1191.10 | 8.91 | 30.35 | 1354 | C₆₈H₁₃₀N₆O₇ | 1143.82 | 9.01 | 24.77 |
| 1155 | C₆₆H₁₃₂N₅O₁₀P | 1186.80 | 8.65 | 24.15 | 1355 | C₄₇H₉₄N₆O₇ | 855.30 | 9.01 | 15.66 |
| 1156 | C₇₀H₁₂₀N₇O₈P | 1218.70 | 9.55 | 21.96 | 1356 | C₅₉H₁₀₆N₆O₇ | 1011.53 | 9.01 | 19.10 |
| 1157 | C₇₂H₁₃₅N₅O₁₂S ₃ | 1359.08 | 7.99 | 23.39 | 1357 | C₆₅H₁₁₈N₆O₇ | 1095.69 | 9.01 | 21.10 |
| 1158 | C₇₂H₁₃₀N₂O₉ | 1167.84 | 8.05 | 25.65 | 1358 | C₅₃H₁₀₀N₆O₇ | 933.42 | 9.01 | 17.38 |
| 1159 | C₇₁H₁₃₉N₃O₃S₆ | 1275.30 | 9.04 | 31.51 | 1359 | C₆₅H₁₂₄N₆O₇ | 1101.74 | 9.01 | 23.73 |
| 1160 | C₆₉H₁₃₈N₄O₉S₃ | 1264.10 | 7.48 | 28.35 | 1360 | C₆₅H₁₃₀N₆O₇ | 1107.79 | 9.01 | 25.18 |
| 1161 | C₇₃H₁₄₂N₂O₈ | 1175.90 | 9.39 | 27.24 | 1361 | C₇₁H₁₁₈N₆O₇ | 1167.76 | 9.01 | 24.18 |
| 1162 | C₈₅H₁₆₇N₅O₁₀ | 1419.30 | 9.49 | 27.91 | 1362 | C₈₆H₁₄₈N₆O₇ | 1378.16 | 9.01 | 31.87 |
| 1163 | C₅₉H₁₂₁N₃O₄ | 936.63 | 9.01 | 22.86 | 1363 | C₆₂H₁₂₄N₆O₇ | 1065.71 | 9.01 | 22.93 |
| 1164 | C₇₁H₁₄₅N₃O₄ | 1104.96 | 9.01 | 29.21 | 1364 | C₇₄H₁₄₈N₆O₇ | 1234.03 | 9.01 | 29.28 |
| 1165 | C₉₂H₁₈₇N₃O₄ | 1399.53 | 9.01 | 40.32 | 1365 | C₉₅H₁₉₀N₆O₇ | 1528.60 | 9.01 | 40.39 |
| 1166 | C₆₅H₁₃₃N₃O₄ | 1020.80 | 9.01 | 26.04 | 1366 | C₆₈H₁₃₆N₆O₇ | 1149.87 | 9.01 | 26.10 |
| 1167 | C₆₅H₁₃₃N₃O₄ | 1020.80 | 9.01 | 26.04 | 1367 | C₆₈H₁₃₆N₆O₇ | 1149.87 | 9.01 | 26.10 |
| 1168 | C₉₈H₁₉₉N₃O₄ | 1483.69 | 9.01 | 43.77 | 1368 | C₁₀₁H₂₀₂N₆O₇ | 1612.76 | 9.01 | 43.83 |
| 1169 | C₉₂H₁₈₇N₃O₄ | 1399.53 | 9.01 | 40.59 | 1369 | C₉₅H₁₉₀N₆O₇ | 1528.60 | 9.01 | 40.66 |
| 1170 | C₈₃H₁₆₉N₃O₄ | 1273.28 | 9.01 | 35.83 | 1370 | C₈₆H₁₇₂N₆O₇ | 1402.36 | 9.01 | 35.90 |
| 1171 | C₆₅H₁₂₇N₃O₄ | 1014.75 | 9.01 | 24.80 | 1371 | C₆₈H₁₃₀N₆O₇ | 1143.82 | 9.01 | 24.86 |
| 1172 | C₆₅H₁₂₇N₃O₄ | 1014.75 | 9.01 | 24.71 | 1372 | C₆₈H₁₃₀N₆O₇ | 1143.82 | 9.01 | 24.77 |
| 1173 | C₄₄H₉₁N₃O₄ | 726.23 | 9.01 | 15.59 | 1373 | C₄₇H₉₄N₆O₇ | 855.30 | 9.01 | 15.66 |
| 1174 | C₅₆H₁₀₃N₃O₄ | 882.46 | 9.01 | 19.03 | 1374 | C₅₉H₁₀₆N₆O₇ | 1011.53 | 9.01 | 19.10 |
| 1175 | C₆₂H₁₁₅N₃O₄ | 966.62 | 9.01 | 21.04 | 1375 | C₆₅H₁₁₈N₆O₇ | 1095.69 | 9.01 | 21.10 |
| 1176 | C₅₀H₉₇N₃O₄ | 804.34 | 9.01 | 17.31 | 1376 | C₅₃H₁₀₀N₆O₇ | 933.42 | 9.01 | 17.38 |
| 1177 | C₆₂H₁₂₁N₃O₄ | 972.67 | 9.01 | 23.66 | 1377 | C₆₅H₁₂₄N₆O₇ | 1101.74 | 9.01 | 23.73 |
| 1178 | C₆₂H₁₂₇N₃O₄ | 978.71 | 9.01 | 25.11 | 1378 | C₆₅H₁₃₀N₆O₇ | 1107.79 | 9.01 | 25.18 |
| 1179 | C₆₈H₁₁₅N₃O₄ | 1038.68 | 9.01 | 26.06 | 1379 | C₇₁H₁₁₈N₆O₇ | 1167.76 | 9.01 | 25.06 |
| 1180 | C₈₃H₁₄₅N₃O₄ | 1249.09 | 9.01 | 33.76 | 1380 | C₈₆H₁₄₈N₆O₇ | 1378.16 | 9.01 | 32.76 |
| 1181 | C₅₉H₁₂₁N₃OS₃ | 984.82 | 9.01 | 25.15 | 1381 | C₆₂H₁₂₇N₉O₄ | 1062.76 | 9.01 | 22.06 |
| 1182 | C₇₁H₁₄₅N₃OS₃ | 1153.14 | 9.01 | 31.50 | 1382 | C₇₄H₁₅₁N₉O₄ | 1231.08 | 9.01 | 28.41 |
| 1183 | C₉₂H₁₈₇N₃OS₃ | 1447.71 | 9.01 | 42.61 | 1383 | C₉₅H₁₉₃N₉O₄ | 1525.65 | 9.01 | 39.52 |
| 1184 | C₆₅H₁₃₃N₃OS₃ | 1068.98 | 9.01 | 28.32 | 1384 | C₆₈H₁₃₉N₉O₄ | 1146.92 | 9.01 | 25.24 |
| 1185 | C₆₅H₁₃₃N₃OS₃ | 1068.98 | 9.01 | 28.32 | 1385 | C₆₈H₁₃₉N₉O₄ | 1146.92 | 9.01 | 25.24 |
| 1186 | C₉₈H₁₉₉N₃OS₃ | 1531.87 | 9.01 | 46.05 | 1386 | C₁₀₁H₂₀₅N₉O₄ | 1609.81 | 9.01 | 42.97 |
| 1187 | C₉₂H₁₈₇N₃OS₃ | 1447.71 | 9.01 | 42.88 | 1387 | C₉₅H₁₉₃N₉O₄ | 1525.65 | 9.01 | 39.79 |
| 1188 | C₈₃H₁₆₉N₃OS₃ | 1321.46 | 9.01 | 38.12 | 1388 | C₈₆H₁₇₅N₉O₄ | 1399.41 | 9.01 | 35.03 |
| 1189 | C₆₅H₁₂₇N₃OS₃ | 1062.93 | 9.01 | 27.08 | 1389 | C₆₈H₁₃₃N₉O₄ | 1140.87 | 9.01 | 24.00 |
| 1190 | C₆₅H₁₂₇N₃OS₃ | 1062.93 | 9.01 | 26.99 | 1390 | C₆₈H₁₃₃N₉O₄ | 1140.87 | 9.01 | 23.91 |
| 1191 | C₄₄H₉₁N₃OS₃ | 774.41 | 9.01 | 17.88 | 1391 | C₄₇H₉₇N₉O₄ | 852.35 | 9.01 | 14.79 |
| 1192 | C₅₆H₁₀₃N₃OS₃ | 930.64 | 9.01 | 21.32 | 1392 | C₅₉H₁₀₉N₉O₄ | 1008.58 | 9.01 | 18.23 |
| 1193 | C₆₂H₁₁₅N₃OS₃ | 1014.80 | 9.01 | 23.32 | 1393 | C₆₅H₁₂₁N₉O₄ | 1092.74 | 9.01 | 20.24 |
| 1194 | C₅₀H₉₇N₃OS₃ | 852.53 | 9.01 | 19.60 | 1394 | C₅₃H₁₀₃N₉O₄ | 930.47 | 9.01 | 16.51 |
| 1195 | C₆₂H₁₂₁N₃OS₃ | 1020.85 | 9.01 | 25.95 | 1395 | C₆₅H₁₂₇N₉O₄ | 1098.79 | 9.01 | 22.86 |
| 1196 | C₆₂H₁₂₇N₃OS₃ | 1026.90 | 9.01 | 27.40 | 1396 | C₆₅H₁₃₃N₉O₄ | 1104.84 | 9.01 | 24.31 |
| 1197 | C₆₈H₁₁₅N₃OS₃ | 1086.87 | 9.01 | 27.48 | 1397 | C₇₁H₁₂₁N₉O₄ | 1164.81 | 9.01 | 23.93 |
| 1198 | C₈₃H₁₄₅N₃OS₃ | 1297.27 | 9.01 | 35.18 | 1398 | C₈₆H₁₅₁N₉O₄ | 1375.21 | 9.01 | 31.63 |
| 1199 | C₅₉H₁₂₄N₆O | 933.68 | 9.01 | 22.13 | 1399 | C₆₂H₁₂₁N₃OS₃ | 1020.85 | 9.01 | 24.00 |
| 1200 | C₅₉H₁₂₄N₆O | 933.68 | 9.01 | 22.13 | 1400 | C₇₄H₁₄₅N₃OS₃ | 1189.17 | 9.01 | 30.34 |
| 1401 | C₉₅H₁₈₇N₃OS₃ | 1483.74 | 9.01 | 41.45 | 1601 | C₆₅H₁₃₆N₆O₇S₃ | 1210.04 | 9.01 | 24.53 |
| 1402 | C₆₈H₁₃₃N₃OS₃ | 1105.01 | 9.01 | 27.17 | 1602 | C₆₅H₁₃₆N₆O₇S₃ | 1210.04 | 9.01 | 24.53 |
| 1403 | C₆₈H₁₃₃N₃OS₃ | 1105.01 | 9.01 | 27.17 | 1603 | C₉₈H₂₀₂N₆O₇S₃ | 1672.93 | 9.01 | 42.26 |
| 1404 | C₁₀₁H₁₉₉N₃OS₃ | 1567.90 | 9.01 | 44.90 | 1604 | C₉₂H₁₉₀N₆O₇S₃ | 1588.77 | 9.01 | 39.08 |
| 1405 | C₉₅H₁₈₇N₃OS₃ | 1483.74 | 9.01 | 41.72 | 1605 | C₈₃H₁₇₂N₆O₇S₃ | 1462.52 | 9.01 | 34.32 |
| 1406 | C₈₆H₁₆₉N₃OS₃ | 1357.50 | 9.01 | 36.96 | 1606 | C₆₅H₁₃₀N₆O₇S₃ | 1203.99 | 9.01 | 23.29 |
| 1407 | C₆₈H₁₂₇N₃OS₃ | 1098.96 | 9.01 | 25.93 | 1607 | C₆₅H₁₃₀N₆O₇S₃ | 1203.99 | 9.01 | 23.20 |
| 1408 | C₆₈H₁₂₇N₃OS₃ | 1098.96 | 9.01 | 25.84 | 1608 | C₄₄H₉₄N₆O₇S₃ | 915.47 | 9.01 | 14.08 |
| 1409 | C₄₇H₉₁N₃OS₃ | 810.44 | 9.01 | 16.72 | 1609 | C₅₆H₁₀₆N₆O₇S₃ | 1071.70 | 9.01 | 17.52 |
| 1410 | C₅₉H₁₀₃N₃OS₃ | 966.67 | 9.01 | 20.17 | 1610 | C₆₂H₁₁₈N₆O₇S₃ | 1155.86 | 9.01 | 19.53 |
| 1411 | C₆₅H₁₁₅N₃OS₃ | 1050.83 | 9.01 | 22.17 | 1611 | C₅₀H₁₀₀N₆O₇S₃ | 993.59 | 9.01 | 15.80 |
| 1412 | C₅₃H₉₇N₃OS₃ | 888.56 | 9.01 | 18.44 | 1612 | C₆₂H₁₂₄N₆O₇S₃ | 1161.91 | 9.01 | 22.15 |
| 1413 | C₆₅H₁₂₁N₃OS₃ | 1056.88 | 9.01 | 24.79 | 1613 | C₆₂H₁₃₀N₆O₇S₃ | 1167.96 | 9.01 | 23.60 |
| 1414 | C₆₄H₁₂₇N₃OS₃ | 1062.93 | 9.01 | 26.24 | 1614 | C₆₈H₁₁₈N₆O₇S₃ | 1227.93 | 9.01 | 23.60 |
| 1415 | C₇₁H₁₁₅N₃OS₃ | 1122.90 | 9.01 | 25.38 | 1615 | C₈₃H₁₄₈N₆O₇S₃ | 1438.33 | 9.01 | 31.29 |
| 1416 | C₈₆H₁₄₅N₃OS₃ | 1333.31 | 9.01 | 33.07 | 1616 | C₅₉H₁₂₁N₃O₁₀S₃ | 1128.83 | 9.01 | 22.22 |
| 1417 | C₆₂H₁₂₁N₃OS₆ | 1117.03 | 9.01 | 26.24 | 1617 | C₇₁H₁₄₅N₃O₁₀S₃ | 1297.15 | 9.01 | 28.57 |
| 1418 | C₇₄H₁₄₅N₃OS₆ | 1285.35 | 9.01 | 32.59 | 1618 | C₉₂H₁₈₇N₃O₁₀S₃ | 1591.72 | 9.01 | 39.68 |
| 1419 | C₉₅H₁₈₇N₃OS₆ | 1579.92 | 9.01 | 43.70 | 1619 | C₆₅H₁₃₃N₃O₁₀S₃ | 1212.99 | 9.01 | 25.39 |
| 1420 | C₆₈H₁₃₃N₃OS₆ | 1201.19 | 9.01 | 29.41 | 1620 | C₆₅H₁₃₃N₃O₁₀S₃ | 1212.99 | 9.01 | 25.39 |
| 1421 | C₆₈H₁₃₃N₃OS₆ | 1201.19 | 9.01 | 29.41 | 1621 | C₉₈H₁₉₉N₃O₁₀S₃ | 1675.88 | 9.01 | 43.12 |
| 1422 | C₁₀₁H₁₉₉N₃OS₆ | 1664.08 | 9.01 | 47.14 | 1622 | C₉₂H₁₈₇N₃O₁₀S₃ | 1591.72 | 9.01 | 39.95 |
| 1423 | C₉₅H₁₈₇N₃OS₆ | 1579.92 | 9.01 | 43.97 | 1623 | C₈₃H₁₆₉N₃O₁₀S₃ | 1465.48 | 9.01 | 35.19 |
| 1424 | C₈₆H₁₆₉N₃OS₆ | 1453.68 | 9.01 | 39.20 | 1624 | C₆₅H₁₂₇N₃O₁₀S₃ | 1206.94 | 9.01 | 24.15 |
| 1425 | C₆₈H₁₂₇N₃OS₆ | 1195.14 | 9.01 | 28.17 | 1625 | C₆₅H₁₂₇N₃O₁₀S₃ | 1206.94 | 9.01 | 24.06 |
| 1426 | C₆₈H₁₂₇N₃OS₆ | 1195.14 | 9.01 | 28.08 | 1626 | C₄₄H₉₁N₃O₁₀S₃ | 918.42 | 9.01 | 14.95 |
| 1427 | C₄₇H₉₁N₃OS₆ | 906.62 | 9.01 | 18.96 | 1627 | C₅₆H₁₀₃N₃O₁₀S₃ | 1074.65 | 9.01 | 18.39 |
| 1428 | C₅₉H₁₀₃N₃OS₆ | 1062.85 | 9.01 | 22.41 | 1628 | C₆₂H₁₁₅N₃O₁₀S₃ | 1158.81 | 9.01 | 20.39 |
| 1429 | C₆₅H₁₁₅N₃OS₆ | 1147.01 | 9.01 | 24.41 | 1629 | C₅₀H₉₇N₃O₁₀S₃ | 996.54 | 9.01 | 16.67 |
| 1430 | C₅₃H₉₇N₃OS₆ | 984.74 | 9.01 | 20.69 | 1630 | C₆₂H₁₂₁N₃O₁₀S₃ | 1164.86 | 9.01 | 23.02 |
| 1431 | C₆₅H₁₂₁N₃OS₆ | 1153.06 | 9.01 | 27.03 | 1631 | C₆₂H₁₂₇N₃O₁₀S₃ | 1170.91 | 9.01 | 24.47 |
| 1432 | C₆₅H₁₂₇N₃OS₆ | 1159.11 | 9.01 | 28.49 | 1632 | C₆₈H₁₁₅N₃O₁₀S₃ | 1230.88 | 9.01 | 24.27 |
| 1433 | C₇₁H₁₁₅N₃O₂S₅ | 1203.02 | 9.01 | 26.60 | 1633 | C₈₃H₁₄₅N₃O₁₀S₃ | 1441.28 | 9.01 | 31.96 |
| 1434 | C₈₆H₁₄₅N₃OS₆ | 1429.49 | 9.01 | 34.45 | 1634 | C₅₉H₁₂₁N₃O₁₀S₃ | 1128.83 | 9.01 | 22.22 |
| 1435 | C₆₂H₁₂₁N₃OS₆ | 1117.03 | 9.01 | 26.24 | 1635 | C₇₁H₁₄₅N₃O₁₀S₃ | 1297.15 | 9.01 | 28.57 |
| 1436 | C₇₄H₁₄₅N₃OS₆ | 1285.35 | 9.01 | 32.59 | 1636 | C₉₂H₁₈₇N₃O₁₀S₃ | 1591.72 | 9.01 | 39.68 |
| 1437 | C₉₅H₁₈₇N₃OS₆ | 1579.92 | 9.01 | 43.70 | 1637 | C₆₅H₁₃₃N₃O₁₀S₃ | 1212.99 | 9.01 | 25.39 |
| 1438 | C₆₈H₁₃₃N₃OS₆ | 1201.19 | 9.01 | 29.41 | 1638 | C₆₅H₁₃₃N₃O₁₀S₃ | 1212.99 | 9.01 | 25.39 |
| 1439 | C₆₈H₁₃₃N₃OS₆ | 1201.19 | 9.01 | 29.41 | 1639 | C₉₈H₁₉₉N₃O₁₀S₃ | 1675.88 | 9.01 | 43.12 |
| 1440 | C₁₀₁H₁₉₉N₃OS₆ | 1664.08 | 9.01 | 47.14 | 1640 | C₉₂H₁₈₇N₃O₁₀S₃ | 1591.72 | 9.01 | 39.95 |
| 1441 | C₉₅H₁₈₇N₃OS₆ | 1579.92 | 9.01 | 43.97 | 1641 | C₈₃H₁₆₉N₃O₁₀S₃ | 1465.48 | 9.01 | 35.19 |
| 1442 | C₈₆H₁₆₉N₃OS₆ | 1453.68 | 9.01 | 39.20 | 1642 | C₆₅H₁₂₇N₃O₁₀S₃ | 1206.94 | 9.01 | 24.15 |
| 1443 | C₆₈H₁₂₇N₃OS₆ | 1195.14 | 9.01 | 28.17 | 1643 | C₆₅H₁₂₇N₃O₁₀S₃ | 1206.94 | 9.01 | 24.06 |
| 1444 | C₆₈H₁₂₇N₃OS₆ | 1195.14 | 9.01 | 28.08 | 1644 | C₄₄H₉₁N₃O₁₀S₃ | 918.42 | 9.01 | 14.95 |
| 1445 | C₄₇H₉₁N₃OS₆ | 906.62 | 9.01 | 18.96 | 1645 | C₅₆H₁₀₃N₃O₁₀S₃ | 1074.65 | 9.01 | 18.39 |
| 1446 | C₅₉H₁₀₃N₃OS₆ | 1062.85 | 9.01 | 22.41 | 1646 | C₆₂H₁₁₅N₃O₁₀S₃ | 1158.81 | 9.01 | 20.39 |
| 1447 | C₆₅H₁₁₅N₃OS₆ | 1147.01 | 9.01 | 24.41 | 1647 | C₅₀H₉₇N₃O₁₀S₃ | 996.54 | 9.01 | 16.67 |
| 1448 | C₅₃H₉₇N₃OS₆ | 984.74 | 9.01 | 20.69 | 1648 | C₆₂H₁₂₁N₃O₁₀S₃ | 1164.86 | 9.01 | 23.02 |
| 1449 | C₆₅H₁₂₁N₃OS₆ | 1153.06 | 9.01 | 27.03 | 1649 | C₆₂H₁₂₇N₃O₁₀S₃ | 1170.91 | 9.01 | 24.47 |
| 1450 | C₆₅H₁₂₇N₃OS₆ | 1159.11 | 9.01 | 28.49 | 1650 | C₆₈H₁₁₅N₃O₁₀S₃ | 1230.88 | 9.01 | 23.31 |
| 1451 | C₇₁H₁₁₅N₃OS₆ | 1219.08 | 9.01 | 27.44 | 1651 | C₈₃H₁₄₅N₃O₁₀S₃ | 1441.28 | 9.01 | 31.01 |
| 1452 | C₈₆H₁₄₅N₃OS₆ | 1429.49 | 9.01 | 35.13 | 1652 | C₆₀H₁₂₃N₃O₁₃S₃ | 1190.85 | 9.01 | 23.97 |
| 1453 | C₆₂H₁₂₁N₃O₄S₃ | 1068.85 | 9.01 | 25.13 | 1653 | C₇₂H₁₄₇N₃O₁₃S₃ | 1359.18 | 9.01 | 30.32 |
| 1454 | C₇₄H₁₄₅N₃O₄S₃ | 1237.17 | 9.01 | 31.48 | 1654 | C₉₃H₁₈₉N₃O₁₃ S₃ | 1653.74 | 9.01 | 41.43 |
| 1455 | C₉₅H₁₈₇N₃O₄S₃ | 1531.74 | 9.01 | 42.59 | 1655 | C₆₆H₁₃₅N₃O₁₃S₃ | 1275.01 | 9.01 | 27.15 |
| 1456 | C₆₈H₁₃₃N₃O₄S₃ | 1153.01 | 9.01 | 28.30 | 1656 | C₆₆H₁₃₅N₃O₁₃S₃ | 1275.01 | 9.01 | 27.15 |
| 1457 | C₆₈H₁₃₃N₃O₄S₃ | 1153.01 | 9.01 | 28.30 | 1657 | C₉₉H₂₀₁N₃O₁₃S₃ | 1737.91 | 9.01 | 44.78 |
| 1458 | C₁₀₁H₁₉₉N₃O₄S ₃ | 1615.90 | 9.01 | 46.03 | 1658 | C₉₃H₁₈₉N₃O₁₃S₃ | 1653.74 | 9.01 | 41.61 |
| 1459 | C₉₅H₁₈₇N₃O₄S₃ | 1531.74 | 9.01 | 42.86 | 1659 | C₈₄H₁₇₁N₃O₁₃S₃ | 1527.50 | 9.01 | 36.85 |
| 1460 | C₈₆H₁₁₆N₃O₄S₃ | 1405.50 | 9.01 | 38.09 | 1660 | C₆₅H₁₂₇N₃O₁₃S₃ | 1254.94 | 9.01 | 25.28 |
| 1461 | C₆₈H₁₂₇N₃O₄S₃ | 1146.96 | 9.01 | 27.06 | 1661 | C₆₅H₁₂₇N₃O₁₃S₃ | 1254.94 | 9.01 | 25.19 |
| 1462 | C₆₈H₁₂₇N₃O₄S₃ | 1146.96 | 9.01 | 26.97 | 1662 | C₄₅H₉₃N₃O₁₃S₃ | 980.45 | 9.01 | 16.61 |
| 1463 | C₄₇H₉₁N₃O₄S₃ | 858.44 | 9.01 | 17.85 | 1663 | C₅₆H₁₀₃N₃O₁₃S₃ | 1122.65 | 9.01 | 19.52 |
| 1464 | C₅₉H₁₀₃N₃O₄S₃ | 1014.67 | 9.01 | 21.30 | 1664 | C₆₂H₁₁₅N₃O₁₃S₃ | 1206.81 | 9.01 | 21.53 |
| 1465 | C₆₅H₁₁₅N₃O₄S₃ | 1098.83 | 9.01 | 23.30 | 1665 | C₅₀H₉₇N₃O₁₃S₃ | 1044.53 | 9.01 | 17.80 |
| 1466 | C₅₃H₉₇N₃O₄S₃ | 936.56 | 9.01 | 19.58 | 1666 | C₆₂H₁₂₁N₃O₁₃S₃ | 1212.86 | 9.01 | 24.15 |
| 1467 | C₆₅H₁₂₁N₃O₄S₃ | 1104.88 | 9.01 | 25.92 | 1667 | C₆₂H₁₂₇N₃O₁₃S₃ | 1218.91 | 9.01 | 25.60 |
| 1468 | C₆₅H₁₂₇N₃O₄S₃ | 1110.93 | 9.01 | 27.38 | 1668 | C₆₉H₁₁₅N₃O₁₃S₃ | 1278.88 | 9.01 | 25.80 |
| 1469 | C₇₁H₁₁₅N₃O₄S₃ | 1170.90 | 9.01 | 26.31 | 1669 | C₈₃H₁₄₅N₃O₁₃S₃ | 1489.28 | 9.01 | 33.49 |
| 1470 | C₈₆H₁₄₅N₃O₄S₃ | 1381.30 | 9.01 | 34.00 | 1670 | C₅₉H₁₂₁N₃O₇S₃ | 1080.83 | 9.01 | 19.96 |
| 1471 | C₆₂H₁₂₁N₃O₄S₃ | 1068.85 | 9.01 | 25.13 | 1671 | C₅₉H₁₂₁N₃O₇S₃ | 1080.83 | 9.01 | 19.96 |
| 1472 | C₆₂H₁₂₁N₃O₄S₃ | 1068.85 | 9.01 | 25.13 | 1672 | C₇₁H₁₄₅N₃O₇S₃ | 1249.16 | 9.01 | 26.30 |
| 1473 | C₇₄H₁₄₅N₃O₄S₃ | 1237.17 | 9.01 | 31.48 | 1673 | C₉₂H₁₈₇N₃O₇S₃ | 1543.72 | 9.01 | 37.41 |
| 1474 | C₉₅H₁₈₇N₃O₄S₃ | 1531.74 | 9.01 | 42.59 | 1674 | C₆₅H₁₃₃N₃O₇S₃ | 1164.99 | 9.01 | 23.13 |
| 1475 | C₆₈H₁₃₃N₃O₄S₃ | 1153.01 | 9.01 | 28.30 | 1675 | C₆₅H₁₃₃N₃O₇S₃ | 1164.99 | 9.01 | 23.13 |
| 1476 | C₆₈H₁₃₃N₃O₄S₃ | 1153.01 | 9.01 | 28.30 | 1676 | C₉₈H₁₉₉N₃O₇S₃ | 1627.88 | 9.01 | 40.86 |
| 1477 | C₁₀₁H₁₉₉N₃O₄S ₃ | 1615.90 | 9.01 | 46.03 | 1677 | C₉₂H₁₈₇N₃O₇S₃ | 1543.72 | 9.01 | 37.68 |
| 1478 | C₉₅H₁₈₇N₃O₄S₃ | 1531.74 | 9.01 | 42.86 | 1678 | C₈₃H₁₆₉N₃O₇S₃ | 1417.48 | 9.01 | 32.92 |
| 1479 | C₈₆H₁₆₉N₃O₄S₃ | 1405.50 | 9.01 | 38.09 | 1679 | C₆₅H₁₂₇N₃O₇S₃ | 1158.95 | 9.01 | 21.89 |
| 1480 | C₆₈H₁₂₇N₃O₄S₃ | 1146.96 | 9.01 | 27.06 | 1680 | C₆₅H₁₂₇N₃O₇S₃ | 1158.95 | 9.01 | 21.80 |
| 1481 | C₆₈H₁₂₇N₃O₆S | 1114.84 | 9.01 | 26.01 | 1681 | C₄₄H₉₁N₃O₇S₃ | 870.43 | 9.01 | 12.68 |
| 1482 | C₄₇H₉₁N₃O₄S₃ | 858.46 | 9.01 | 17.85 | 1682 | C₅₆H₁₀₃N₃O₇S₃ | 1026.65 | 9.01 | 16.12 |
| 1483 | C₅₉H₁₀₃N₃O₄S₃ | 1014.69 | 9.01 | 21.30 | 1683 | C₆₂H₁₁₅N₃O₇S₃ | 1110.82 | 9.01 | 18.13 |
| 1484 | C₆₅H₁₁₅N₃O₄S₃ | 1098.85 | 9.01 | 23.30 | 1684 | C₅₀H₉₇N₃O₇S₃ | 948.54 | 9.01 | 14.40 |
| 1485 | C₅₃H₉₇N₃O₄S₃ | 936.58 | 9.01 | 19.58 | 1685 | C₆₂H₁₂₁N₃O₇S₃ | 1116.86 | 9.01 | 20.75 |
| 1486 | C₆₅H₁₂₁N₃O₄S₃ | 1104.90 | 9.01 | 25.92 | 1686 | C₆₂H₁₂₇N₃O₇S₃ | 1122.91 | 9.01 | 22.20 |
| 1487 | C₆₅H₁₂₇N₃O₄S₃ | 1110.95 | 9.01 | 27.38 | 1687 | C₆₈H₁₁₅N₃O₇S₃ | 1182.88 | 9.01 | 22.60 |
| 1488 | C₇₁H₁₁₅N₃O₄S₃ | 1170.92 | 9.01 | 26.44 | 1688 | C₈₃H₁₄₅N₃O₇S₃ | 1393.29 | 9.01 | 30.30 |
| 1489 | C₈₆H₁₄₅N₃O₄S₃ | 1381.32 | 9.01 | 34.14 | 1689 | C₆₄H₁₃₂N₂O₄ | 993.77 | 9.45 | 26.47 |
| 1490 | C₆₂H₁₂₁N₃O₇S₃ | 1116.86 | 9.01 | 26.05 | 1690 | C₆₄H₁₃₂N₂O₄ | 993.77 | 9.45 | 26.47 |
| 1491 | C₇₄H₁₄₅N₃O₇S₃ | 1285.19 | 9.01 | 32.39 | 1691 | C₆₇H₁₃₂N₂O₇ | 1077.80 | 9.45 | 27.29 |
| 1492 | C₉₅H₁₈₇N₃O₇S₃ | 1579.76 | 9.01 | 43.50 | 1692 | C₆₇H₁₃₂N₂O₇ | 1077.80 | 9.45 | 27.29 |
| 1493 | C₆₈H₁₃₃N₃O₇S₃ | 1201.03 | 9.01 | 29.22 | 1693 | C₆₇H₁₃₂N₂O₇ | 1077.80 | 9.46 | 27.29 |
| 1494 | C₆₈H₁₃₃N₃O₇S₃ | 1201.03 | 9.01 | 29.22 | 1694 | C₆₇H₁₃₂N₂O₇ | 1077.80 | 9.46 | 27.29 |
| 1495 | C₁₀₁H₁₉₉N₃O₇S ₃ | 1663.92 | 9.01 | 46.95 | 1695 | C₆₄H₁₃₂N₂O₃S | 1009.83 | 9.80 | 26.96 |
| 1496 | C₉₅H₁₈₇N₃O₇S₃ | 1579.76 | 9.01 | 43.77 | 1696 | C₆₄H₁₃₂N₂O₃S | 1009.83 | 9.80 | 26.96 |
| 1497 | C₈₆H₁₆₉N₃O₇S₃ | 1453.51 | 9.01 | 39.01 | 1697 | C₆₇H₁₃₂N₂O₆S | 1093.86 | 9.79 | 27.78 |
| 1498 | C₆₈H₁₂₇N₃O₇S₃ | 1194.98 | 9.01 | 27.98 | 1698 | C₆₇H₁₃₂N₂O₆S | 1093.86 | 9.79 | 27.78 |
| 1499 | C₆₈H₁₂₇N₃O₇S₃ | 1194.98 | 9.01 | 27.89 | 1699 | C₆₇H₁₃₂N₂O₆S | 1093.86 | 9.81 | 27.78 |
| 1500 | C₄₇H₉₁N₃O₇S₃ | 906.46 | 9.01 | 18.77 | 1700 | C₆₇H₁₃₂N₂O₆S | 1093.86 | 9.81 | 27.78 |
| 1501 | C₅₉H₁₀₃N₃O₇S₃ | 1062.69 | 9.01 | 22.21 | 1701 | C₆₄H₁₃₃N₃O₃ | 992.79 | 9.78 | 26.31 |
| 1502 | C₆₅H₁₁₅N₃O₇S₃ | 1146.85 | 9.01 | 24.22 | 1702 | C₆₄H₁₃₃N₃O₃ | 992.79 | 9.78 | 26.31 |
| 1503 | C₅₃H₉₇N₃O₇S₃ | 984.57 | 9.01 | 20.49 | 1703 | C₆₇H₁₃₃N₃O₆ | 1076.82 | 9.78 | 27.13 |
| 1504 | C₆₅H₁₂₁N₃O₇S₃ | 1152.90 | 9.01 | 26.84 | 1704 | C₆₇H₁₃₃N₃O₆ | 1076.82 | 9.78 | 27.13 |
| 1505 | C₆₅H₁₂₇N₃O₇S₃ | 1158.95 | 9.01 | 28.29 | 1705 | C₆₇H₁₃₃N₃O₆ | 1076.82 | 9.79 | 27.13 |
| 1506 | C₇₁H₁₁₅N₃O₇S₃ | 1218.92 | 9.01 | 27.44 | 1706 | C₆₇H₁₃₃N₃O₆ | 1076.82 | 9.79 | 27.13 |
| 1507 | C₈₆H₁₄₅N₃O₇S₃ | 1429.32 | 9.01 | 35.13 | 1707 | C₆₄H₁₃₂N₂O₃S₂ | 1041.89 | 9.65 | 27.39 |
| 1508 | C₆₂H₁₂₁N₃O₇S₃ | 1116.86 | 9.01 | 26.05 | 1708 | C₆₄H₁₃₂N₂O₃S₂ | 1041.89 | 9.65 | 27.39 |
| 1509 | C₇H₁₄₅N₃O₇S₃ | 1285.19 | 9.01 | 32.39 | 1709 | C₆₇H₁₃₂N₂O₆S₂ | 1125.92 | 9.65 | 28.21 |
| 1510 | C₉₅H₁₈₇N₃O₇S₃ | 1579.76 | 9.01 | 43.50 | 1710 | C₆₇H₁₃₂N₂O₆S₂ | 1125.92 | 9.65 | 28.21 |
| 1511 | C₆₈H₁₃₃N₃O₇S₃ | 1201.03 | 9.01 | 29.22 | 1711 | C₆₇H₁₃₂N₂O₆S₂ | 1125.92 | 9.65 | 28.21 |
| 1512 | C₆₈H₁₃₃N₃O₇S₃ | 1201.03 | 9.01 | 29.22 | 1712 | C₆₇H₁₃₂N₂O₆S₂ | 1125.92 | 9.65 | 28.21 |
| 1513 | C₁₀₁H₁₉₉N₃O₇S ₃ | 1663.92 | 9.01 | 46.95 | 1713 | C₆₅H₁₃₂N₂O₄ | 1005.78 | 8.95 | 26.46 |
| 1514 | C₉₅H₁₈₇N₃O₇S₃ | 1579.76 | 9.01 | 43.77 | 1714 | C₆₅H₁₃₂N₂O₄ | 1005.78 | 8.95 | 26.46 |
| 1515 | C₈₆H₁₆₉N₃O₇S₃ | 1453.51 | 9.01 | 39.01 | 1715 | C₆₈H₁₃₂N₂O₇ | 1089.81 | 8.95 | 27.28 |
| 1516 | C₆₈H₁₂₇N₃O₇S₃ | 1194.98 | 9.01 | 27.98 | 1716 | C₆₈H₁₃₂N₂O₇ | 1089.81 | 8.95 | 27.28 |
| 1517 | C₆₈H₁₂₇N₃O₇S₃ | 1194.98 | 9.01 | 27.89 | 1717 | C₆₈H₁₃₂N₂O₇ | 1089.81 | 8.96 | 27.28 |
| 1518 | C₄₇H₉₁N₃O₇S₃ | 906.46 | 9.01 | 18.77 | 1718 | C₆₈H₁₃₂N₂O₇ | 1089.81 | 8.96 | 27.28 |
| 1519 | C₅₉H₁₀₃N₃O₇S₃ | 1062.69 | 9.01 | 22.21 | 1719 | C₆₅H₁₃₂N₂O₅ | 1021.78 | 8.83 | 26.64 |
| 1520 | C₆₅H₁₁₅N₃O₇S₃ | 1146.85 | 9.01 | 24.22 | 1720 | C₆₅H₁₃₂N₂O₅ | 1021.78 | 8.83 | 26.64 |
| 1521 | C₅₃H₉₇N₃O₇S₃ | 984.57 | 9.01 | 20.49 | 1721 | C₆₈H₁₃₂N₂O₈ | 1105.81 | 8.83 | 27.46 |
| 1522 | C₆₅H₁₂₁N₃O₇S₃ | 1152.90 | 9.01 | 26.84 | 1722 | C₆₈H₁₃₂N₂O₈ | 1105.81 | 8.83 | 27.46 |
| 1523 | C₆₅H₁₂₇N₃O₇S₃ | 1158.95 | 9.01 | 28.29 | 1723 | C₆₈H₁₃₂N₂O₈ | 1105.81 | 8.83 | 27.46 |
| 1524 | C₇₁H₁₁₅N₃O₇S₃ | 1218.92 | 9.01 | 25.64 | 1724 | C₆₈H₁₃₂N₂O₈ | 1105.81 | 8.83 | 27.46 |
| 1525 | C₈₆H₁₄₅N₃O₇S₃ | 1429.32 | 9.01 | 33.34 | 1725 | C₆₅H₁₃₂N₂O₅ | 1021.78 | 8.67 | 26.64 |
| 1526 | C₆₂H₁₂₁N₃O₄S₆ | 1165.07 | 9.01 | 27.16 | 1726 | C₆₅H₁₃₂N₂O₅ | 1021.78 | 8.67 | 26.64 |
| 1527 | C₇₄H₁₄₅N₃O₄S₆ | 1333.39 | 9.01 | 33.50 | 1727 | C₆₈H₁₃₂N₂O₈ | 1105.81 | 8.67 | 27.46 |
| 1528 | C₉₅H₁₈₇N₃O₄S₆ | 1627.96 | 9.01 | 44.61 | 1728 | C₆₈H₁₃₂N₂O₈ | 1105.81 | 8.67 | 27.46 |
| 1529 | C₆₈H₁₃₃N₃O₄S₆ | 1249.23 | 9.01 | 30.33 | 1729 | C₆₈H₁₃₂N₂O₈ | 1105.81 | 8.67 | 27.46 |
| 1530 | C₆₈H₁₃₃N₃O₄S₆ | 1249.23 | 9.01 | 30.33 | 1730 | C₆₈H₁₃₂N₂O₈ | 1105.81 | 8.67 | 27.46 |
| 1531 | C₁₀₁H₁₉₉N₃O₄S ₆ | 1712.12 | 9.01 | 48.06 | 1731 | C₆₅H₁₃₂N₂O₆ | 1037.78 | 8.66 | 26.60 |
| 1532 | C₉₅H₁₈₇N₃O₄S₆ | 1627.96 | 9.01 | 44.88 | 1732 | C₆₅H₁₃₂N₂O₆ | 1037.78 | 8.66 | 26.60 |
| 1533 | C₈₆H₁₆₉N₃O₄S₆ | 1501.72 | 9.01 | 40.12 | 1733 | C₆₈H₁₃₂N₂O₉ | 1121.81 | 8.66 | 27.43 |
| 1534 | C₆₈H₁₂₇N₃O₄S₆ | 1243.18 | 9.01 | 29.09 | 1734 | C₆₈H₁₃₂N₂O₉ | 1121.81 | 8.66 | 27.43 |
| 1535 | C₆₈H₁₂₇N₃O₄S₆ | 1243.18 | 9.01 | 29.00 | 1735 | C₆₈H₁₃₂N₂O₉ | 1121.81 | 8.66 | 27.43 |
| 1536 | C₄₇H₉₁N₃O₄S₆ | 954.66 | 9.01 | 19.88 | 1736 | C₆₈H₁₃₂N₂O₉ | 1121.81 | 8.66 | 27.43 |
| 1537 | C₅₉H₁₀₃N₃O₄S₆ | 1110.89 | 9.01 | 23.32 | 1737 | C₆₅H₁₃₃N₃O₄ | 1020.80 | 8.84 | 26.04 |
| 1538 | C₆₅H₁₁₅N₃O₄S₆ | 1195.05 | 9.01 | 25.33 | 1738 | C₆₅H₁₃₃N₃O₄ | 1020.80 | 8.84 | 26.04 |
| 1539 | C₅₃H₉₇N₃O₄S₆ | 1032.78 | 9.01 | 21.60 | 1739 | C₆₈H₁₃₃N₃O₇ | 1104.83 | 8.83 | 26.86 |
| 1540 | C₆₅H₁₂₁N₃O₄S₆ | 1201.10 | 9.01 | 27.95 | 1740 | C₆₈H₁₃₃N₃O₇ | 1104.83 | 8.84 | 26.86 |
| 1541 | C₆₅H₁₂₇N₃O₄S₆ | 1207.15 | 9.01 | 29.40 | 1741 | C₆₈H₁₃₃N₃O₇ | 1104.83 | 8.84 | 26.86 |
| 1542 | C₇₁H₁₁₅N₃O₄S₆ | 1267.12 | 9.01 | 28.55 | 1742 | C₆₈H₁₃₃N₃O₇ | 1104.83 | 8.84 | 26.86 |
| 1543 | C₈₆H₁₄₅N₃O₄S₆ | 1477.52 | 9.01 | 36.24 | 1743 | C₆₅H₁₃₃N₃O₅ | 1036.79 | 8.85 | 26.48 |
| 1544 | C₆₅H₁₃₆N₃O₁₀P ₃ | 1212.74 | 9.01 | 21.98 | 1744 | C₆₅H₁₃₃N₃O₅ | 1036.79 | 8.85 | 26.48 |
| 1545 | C₇₇H₁₆₀N₃O₁₀P ₃ | 1381.06 | 9.01 | 28.33 | 1745 | C₆₈H₁₃₃N₃O₈ | 1120.82 | 8.85 | 27.30 |
| 1546 | C₉₈H₂₀₂N₃O₁₀P ₃ | 1675.63 | 9.01 | 39.44 | 1746 | C₆₈H₁₃₃N₃O₈ | 1120.82 | 8.85 | 27.30 |
| 1547 | C₇₁H₁₄₈N₃O₁₀P ₃ | 1296.90 | 9.01 | 25.16 | 1747 | C₆₈H₁₃₃N₃O₈ | 1120.82 | 8.85 | 27.30 |
| 1548 | C₇₁H₁₄₈N₃O₁₀P ₃ | 1296.90 | 9.01 | 25.16 | 1748 | C₆₈H₁₃₃N₃O₈ | 1120.82 | 8.85 | 27.30 |
| 1549 | C₁₀₄H₂₁₄N₃O₁₀ P₃ | 1759.79 | 9.01 | 42.88 | 1749 | C₆₅H₁₃₃N₃O₅ | 1036.79 | 8.75 | 26.48 |
| 1550 | C₉₈H₂₀₂N₃O₁₀P₃ | 1675.63 | 9.01 | 39.71 | 1750 | C₆₅H₁₃₃N₃O₅ | 1036.79 | 8.75 | 26.48 |
| 1551 | C₈₉H₁₈₄N₃O₁₀P ₃ | 1549.38 | 9.01 | 34.95 | 1751 | C₆₈H₁₃₃N₃O₈ | 1120.82 | 8.75 | 27.30 |
| 1552 | C₇₁H₁₄₂N₃O₁₀P ₃ | 1290.85 | 9.01 | 23.91 | 1752 | C₆₈H₁₃₃N₃O₈ | 1120.82 | 8.75 | 27.30 |
| 1553 | C₇₁H₁₄₂N₃O₁₀P ₃ | 1290.85 | 9.01 | 23.82 | 1753 | C₆₈H₁₃₃N₃O₈ | 1120.82 | 8.75 | 27.30 |
| 1554 | C₅₀H₁₀₆N₃O₁₀P ₃ | 1002.33 | 9.01 | 14.71 | 1754 | C₆₈H₁₃₃N₃O₈ | 1120.82 | 8.75 | 27.30 |
| 1555 | C₆₂H₁₁₈N₃O₁₀P ₃ | 1158.56 | 9.01 | 18.15 | 1755 | C₆₅H₁₃₄N₄O₄ | 1035.81 | 8.95 | 26.29 |
| 1556 | C₆₈H₁₃₀N₃O₁₀P ₃ | 1242.72 | 9.01 | 20.16 | 1756 | C₆₅H₁₃₄N₄O₄ | 1035.81 | 8.95 | 26.29 |
| 1557 | C₅₆H₁₁₂N₃O₁₀P ₃ | 1080.44 | 9.01 | 16.43 | 1757 | C₆₈H₁₃₄N₄O₇ | 1119.84 | 8.95 | 27.11 |
| 1558 | C₆₈H₁₃₆N₃O₁₀P ₃ | 1248.77 | 9.01 | 22.78 | 1758 | C₆₈H₁₃₄N₄O₇ | 1119.84 | 8.95 | 27.11 |
| 1559 | C₆₈H₁₄₂N₃O₁₀P ₃ | 1254.82 | 9.01 | 24.23 | 1759 | C₆₈H₁₃₄N₄O₇ | 1119.84 | 8.95 | 27.11 |
| 1560 | C₇₄H₁₃₀N₃O₁₀P ₃ | 1314.79 | 9.01 | 23.74 | 1760 | C₆₈H₁₃₄N₄O₇ | 1119.84 | 8.95 | 27.11 |
| 1561 | C₈₉H₁₆₀N₃O₁₀P ₃ | 1525.19 | 9.01 | 31.43 | 1761 | C₆₅H₁₃₂N₂O₃S | 1021.84 | 9.29 | 26.71 |
| 1562 | C₆₅H₁₃₆N₃O₁₀P ₃ | 1212.74 | 9.01 | 21.98 | 1762 | C₆₅H₁₃₂N₂O₃S | 1021.84 | 9.29 | 26.71 |
| 1563 | C₇₇H₁₆₀N₃O₁₀P ₃ | 1381.06 | 9.01 | 28.33 | 1763 | C₆₈H₁₃₂N₂O₆S | 1105.87 | 9.29 | 27.53 |
| 1564 | C₉₈H₂₀₂N₃O₁₀P ₃ | 1675.63 | 9.01 | 39.44 | 1764 | C₆₈H₁₃₂N₂O₆S | 1105.87 | 9.29 | 27.53 |
| 1565 | C₇₁H₁₄₈N₃O₁₀P ₃ | 1296.90 | 9.01 | 25.16 | 1765 | C₆₈H₁₃₂N₂O₆S | 1105.87 | 9.31 | 27.53 |
| 1566 | C₇₁H₁₄₈N₃O₁₀P ₃ | 1296.90 | 9.01 | 25.16 | 1766 | C₆₈H₁₃₂N₂O₆S | 1105.87 | 9.31 | 27.53 |
| 1567 | C₁₀₄H₂₁₄N₃O₁₀ P₃ | 1759.79 | 9.01 | 42.88 | 1767 | C₆₅H₁₃₂N₂O₃S₂ | 1053.90 | 9.35 | 27.19 |
| 1568 | C₉₈H₂₀₂N₃O₁₀P ₃ | 1675.63 | 9.01 | 39.71 | 1768 | C₆₅H₁₃₂N₂O₃S₂ | 1053.90 | 9.35 | 27.19 |
| 1569 | C₈₉H₁₈₄N₃O₁₀P ₃ | 1549.38 | 9.01 | 34.95 | 1769 | C₆₈H₁₃₂N₂O₆S₂ | 1137.93 | 9.35 | 28.02 |
| 1570 | C₇₁H₁₄₂N₃O₁₀P ₃ | 1290.85 | 9.01 | 23.91 | 1770 | C₆₈H₁₃₂N₂O₆S₂ | 1137.93 | 9.35 | 28.02 |
| 1571 | C₇₁H₁₄₂N₃O₁₀P ₃ | 1290.85 | 9.01 | 23.82 | 1771 | C₆₈H₁₃₂N₂O₆S₂ | 1137.93 | 9.35 | 28.02 |
| 1572 | C₅₀H₁₀₆N₃O₁₀P ₃ | 1002.33 | 9.01 | 14.71 | 1772 | C₆₈H₁₃₂N₂O₆S₂ | 1137.93 | 9.35 | 28.02 |
| 1573 | C₆₂H₁₁₈N₃O₁₀P ₃ | 1158.56 | 9.01 | 18.15 | 1773 | C₆₅H₁₃₂N₂O₃S₂ | 1053.90 | 9.13 | 27.19 |
| 1574 | C₆₈H₁₃₀N₃O₁₀P ₃ | 1242.72 | 9.01 | 20.16 | 1774 | C₆₅H₁₃₂N₂O₃S₂ | 1053.90 | 9.13 | 27.19 |
| 1575 | C₅₆H₁₁₂N₃O₁₀P ₃ | 1080.44 | 9.01 | 16.43 | 1775 | C₆₈H₁₃₂N₂O₆S₂ | 1137.93 | 9.13 | 28.02 |
| 1576 | C₆₈H₁₃₆N₃O₁₀P ₃ | 1248.77 | 9.01 | 22.78 | 1776 | C₆₈H₁₃₂N₂O₆S₂ | 1137.93 | 9.13 | 28.02 |
| 1577 | C₆₈H₁₄₂N₃O₁₀P ₃ | 1254.82 | 9.01 | 24.23 | 1777 | C₆₈H₁₃₂N₂O₆S₂ | 1137.93 | 9.14 | 28.02 |
| 1578 | C₇₄H₁₃₀N₃O₁₀P ₃ | 1314.79 | 9.01 | 24.34 | 1778 | C₆₈H₁₃₂N₂O₆S₂ | 1137.93 | 9.14 | 28.02 |
| 1579 | C₈₉H₁₆₀N₃O₁₀P ₃ | 1525.19 | 9.01 | 32.03 | 1779 | C₆₅H₁₃₂N₂O₄S | 1037.84 | 9.20 | 26.95 |
| 1580 | C₅₉H₁₂₄N₆O₇S₃ | 1125.88 | 9.01 | 21.35 | 1780 | C₆₅H₁₃₂N₂O₄S | 1037.84 | 9.20 | 26.95 |
| 1581 | C₇₁H₁₄₈N₆O₇S₃ | 1294.20 | 9.01 | 27.70 | 1781 | C₆₈H₁₃₂N₂O₇S | 1121.87 | 9.20 | 27.78 |
| 1582 | C₉₂H₁₉₀N₆O₇S₃ | 1588.77 | 9.01 | 38.81 | 1782 | C₆₈H₁₃₂N₂O₇S | 1121.87 | 9.20 | 27.78 |
| 1583 | C₆₅H₁₃₆N₆O₇S₃ | 1210.04 | 9.01 | 24.53 | 1783 | C₆₈H₁₃₂N₂O₇S | 1121.87 | 9.20 | 27.78 |
| 1584 | C₆₅H₁₃₆N₆O₇S₃ | 1210.04 | 9.01 | 24.53 | 1784 | C₆₈H₁₃₂N₂O₇S | 1121.87 | 9.20 | 27.78 |
| 1585 | C₉₈H₂₀₂N₆O₇S₃ | 1672.93 | 9.01 | 42.26 | 1785 | C₆₅H₁₃₂N₂O₄S | 1037.84 | 8.80 | 26.95 |
| 1586 | C₉₂H₁₉₀N₆O₇S₃ | 1588.77 | 9.01 | 39.08 | 1786 | C₆₅H₁₃₂N₂O₄S | 1037.84 | 8.80 | 26.95 |
| 1587 | C₈₃H₁₇₂N₆O₇S₃ | 1462.52 | 9.01 | 34.32 | 1787 | C₆₈H₁₃₂N₂O₇S | 1121.87 | 8.80 | 27.78 |
| 1588 | C₆₅H₁₃₀N₆O₇S₃ | 1203.99 | 9.01 | 23.29 | 1788 | C₆₈H₁₃₂N₂O₇S | 1121.87 | 8.80 | 27.78 |
| 1589 | C₆₅H₁₃₀N₆O₇S₃ | 1203.99 | 9.01 | 23.20 | 1789 | C₆₈H₁₃₂N₂O₇S | 1121.87 | 8.80 | 27.78 |
| 1590 | C₄₄H₉₄N₆O₇S₃ | 915.47 | 9.01 | 14.08 | 1790 | C₆₈H₁₃₂N₂O₇S | 1121.87 | 8.80 | 27.78 |
| 1591 | C₅₆H₁₀₆N₆O₇S₃ | 1071.70 | 9.01 | 17.52 | 1791 | C₆₅H₁₃₂N₂O₅S | 1053.84 | 8.70 | 27.15 |
| 1592 | C₆₂H₁₁₈N₆O₇S₃ | 1155.86 | 9.01 | 19.53 | 1792 | C₆₅H₁₃₂N₂O₅S | 1053.84 | 8.70 | 27.15 |
| 1593 | C₅₀H₁₀₀N₆O₇S₃ | 993.59 | 9.01 | 15.80 | 1793 | C₆₈H₁₃₂N₂O₈S | 1137.87 | 8.70 | 27.97 |
| 1594 | C₆₂H₁₂₄N₆O₇S₃ | 1161.91 | 9.01 | 22.15 | 1794 | C₆₈H₁₃₂N₂O₈S | 1137.87 | 8.70 | 27.97 |
| 1595 | C₆₂H₁₃₀N₆O₇S₃ | 1167.96 | 9.01 | 23.60 | 1795 | C₆₈H₁₃₂N₂O₈S | 1137.87 | 8.70 | 27.97 |
| 1596 | C₆₈H₁₁₈N₆O₇S₃ | 1227.93 | 9.01 | 23.53 | 1796 | C₆₈H₁₃₂N₂O₈S | 1137.87 | 8.70 | 27.97 |
| 1597 | C₈₃H₁₄₈N₆O₇S₃ | 1438.33 | 9.01 | 31.23 | 1797 | C₆₅H₁₃₂N₂O₅S | 1053.84 | 9.00 | 27.15 |
| 1598 | C₅₉H₁₂₄N₆O₇S₃ | 1125.88 | 9.01 | 21.35 | 1798 | C₆₅H₁₃₂N₂O₅S | 1053.84 | 9.00 | 27.15 |
| 1599 | C₇₁H₁₄₈N₆O₇S₃ | 1294.20 | 9.01 | 27.70 | 1799 | C₆₈H₁₃₂N₂O₈S | 1137.87 | 9.00 | 27.97 |
| 1600 | C₉₂H₁₉₀N₆O₇S₃ | 1588.77 | 9.01 | 38.81 | 1800 | C₆₈H₁₃₂N₂O₈S | 1137.87 | 9.00 | 27.97 |
| 1801 | C₆₈H₁₃₂N₂O₈S | 1137.87 | 9.00 | 27.97 | 2001 | C₆₉H₁₃₇N₅O₅ | 1116.88 | 7.65 | 24.63 |
| 1802 | C₆₈H₁₃₂N₂O₈S | 1137.87 | 9.00 | 27.97 | 2002 | C₆₉H₁₃₇N₅O₅ | 1116.88 | 7.65 | 24.63 |
| 1803 | C₆₅H₁₃₂N₂O₄S₂ | 1069.90 | 9.06 | 27.39 | 2003 | C₇₂H₁₃₇N₅O₈ | 1200.91 | 7.65 | 25.45 |
| 1804 | C₆₅H₁₃₂N₂O₄S₂ | 1069.90 | 9.06 | 27.39 | 2004 | C₇₂H₁₃₇N₅O₈ | 1200.91 | 7.65 | 25.45 |
| 1805 | C₆₈H₁₃₂N₂O₇S₂ | 1153.93 | 9.06 | 28.21 | 2005 | C₇₂H₁₃₇N₅O₈ | 1200.91 | 7.65 | 25.45 |
| 1806 | C₆₈H₁₃₂N₂O₇S₂ | 1153.93 | 9.06 | 28.21 | 2006 | C₇₂H₁₃₇N₅O₈ | 1200.91 | 7.65 | 25.45 |
| 1807 | C₆₈H₁₃₂N₂O₇S₂ | 1153.93 | 9.06 | 28.21 | 2007 | C₆₉H₁₃₆N₄O₅ | 1101.87 | 7.90 | 25.58 |
| 1808 | C₆₈H₁₃₂N₂O₇S₂ | 1153.93 | 9.06 | 28.21 | 2008 | C₆₉H₁₃₆N₄O₅ | 1101.87 | 7.90 | 25.58 |
| 1809 | C₆₆H₁₃₇N₂O₆P | 1085.80 | 8.79 | 26.39 | 2009 | C₇₂H₁₃₆N₄O₈ | 1185.90 | 7.90 | 26.41 |
| 1810 | C₆₆H₁₃₇N₂O₆P | 1085.80 | 8.79 | 26.39 | 2010 | C₇₂H₁₃₆N₄O₈ | 1185.90 | 7.90 | 26.41 |
| 1811 | C₆₉H₁₃₇N₂O₉P | 1169.83 | 8.79 | 27.21 | 2011 | C₇₂H₁₃₆N₄O₈ | 1185.90 | 7.90 | 26.41 |
| 1812 | C₆₉H₁₃₇N₂O₉P | 1169.83 | 8.79 | 27.21 | 2012 | C₇₂H₁₃₆N₄O₈ | 1185.90 | 7.90 | 26.41 |
| 1813 | C₆₉H₁₃₇N₂O₉P | 1169.83 | 8.79 | 27.21 | 2013 | C₆₉H₁₃₇N₅O₄ | 1100.89 | 8.23 | 25.73 |
| 1814 | C₆₉H₁₃₇N₂O₉P | 1169.83 | 8.79 | 27.21 | 2014 | C₆₉H₁₃₇N₅O₄ | 1100.89 | 8.23 | 25.73 |
| 1815 | C₆₆H₁₃₇N₂O₆P | 1085.80 | 8.41 | 26.39 | 2015 | C₇₂H₁₃₇N₅O₇ | 1184.92 | 8.23 | 26.56 |
| 1816 | C₆₆H₁₃₇N₂O₆P | 1085.80 | 8.41 | 26.39 | 2016 | C₇₂H₁₃₇N₅O₇ | 1184.92 | 8.23 | 26.56 |
| 1817 | C₆₉H₁₃₇N₂O₉P | 1169.83 | 8.41 | 27.21 | 2017 | C₇₂H₁₃₇N₅O₇ | 1184.92 | 8.23 | 26.56 |
| 1818 | C₆₉H₁₃₇N₂O₉P | 1169.83 | 8.41 | 27.21 | 2018 | C₇₂H₁₃₇N₅O₇ | 1184.92 | 8.23 | 26.56 |
| 1819 | C₆₉H₁₃₇N₂O₉P | 1169.83 | 8.41 | 27.21 | 2019 | C₆₉H₁₃₇N₅O₄ | 1100.89 | 7.75 | 26.13 |
| 1820 | C₆₉H₁₃₇N₂O₉P | 1169.83 | 8.41 | 27.21 | 2020 | C₆₉H₁₃₇N₅O₄ | 1100.89 | 7.75 | 26.13 |
| 1821 | C₆₄H₁₃₃N₃O₅S | 1056.84 | 8.74 | 26.14 | 2021 | C₇₂H₁₃₇N₅O₇ | 1184.92 | 7.75 | 26.95 |
| 1822 | C₆₄H₁₃₃N₃O₅S | 1056.84 | 8.74 | 26.14 | 2022 | C₇₂H₁₃₇N₅O₇ | 1184.92 | 7.75 | 26.95 |
| 1823 | C₆₇H₁₃₃N₃O₈S | 1140.87 | 8.74 | 26.96 | 2023 | C₇₂H₁₃₇N₅O₇ | 1184.92 | 7.75 | 26.95 |
| 1824 | C₆₇H₁₃₃N₃O₈S | 1140.87 | 8.74 | 26.96 | 2024 | C₇₂H₁₃₇N₅O₇ | 1184.92 | 7.75 | 26.95 |
| 1825 | C₆₇H₁₃₃N₃O₈S | 1140.87 | 8.74 | 26.96 | 2025 | C₆₈H₁₃₆N₆O₄ | 1101.87 | 7.19 | 25.85 |
| 1826 | C₆₇H₁₃₃N₃O₈S | 1140.87 | 8.74 | 26.96 | 2026 | C₆₈H₁₃₆N₆O₄ | 1101.87 | 7.19 | 25.85 |
| 1827 | C₆₄H₁₃₃N₃O₅S | 1056.84 | 7.78 | 26.14 | 2027 | C₇₁H₁₃₆N₆O₇ | 1185.90 | 7.19 | 26.67 |
| 1828 | C₆₄H₁₃₃N₃O₅S | 1056.84 | 7.78 | 26.14 | 2028 | C₇₁H₁₃₆N₆O₇ | 1185.90 | 7.19 | 26.67 |
| 1829 | C₆₇H₁₃₃N₃O₈S | 1140.87 | 7.78 | 26.96 | 2029 | C₇₁H₁₃₆N₆O₇ | 1185.90 | 7.19 | 26.67 |
| 1830 | C₆₇H₁₃₃N₃O₈S | 1140.87 | 7.78 | 26.96 | 2030 | C₇₁H₁₃₆N₆O₇ | 1185.90 | 7.19 | 26.67 |
| 1831 | C₆₇H₁₃₃N₃O₈S | 1140.87 | 7.78 | 26.96 | 2031 | C₆₉H₁₃₇N₅O₄S | 1132.95 | 7.95 | 27.26 |
| 1832 | C₆₇H₁₃₃N₃O₈S | 1140.87 | 7.78 | 26.96 | 2032 | C₆₉H₁₃₇N₅O₄S | 1132.95 | 7.95 | 27.26 |
| 1833 | C₆₄H₁₃₂N₂O₆S | 1057.83 | 7.60 | 26.33 | 2033 | C₈₉H₁₇₆N₆O₉S | 1506.48 | 7.74 | 33.24 |
| 1834 | C₆₄H₁₃₂N₂O₆S | 1057.83 | 7.60 | 26.33 | 2034 | C₈₈H₁₇₅N₅O₉ | 1447.39 | 8.17 | 35.73 |
| 1835 | C₆₇H₁₃₂N₂O₉S | 1141.86 | 7.60 | 27.15 | 2035 | C₇₂H₁₃₇N₅O₇S | 1216.98 | 7.96 | 28.08 |
| 1836 | C₆₇H₁₃₂N₂O₉S | 1141.86 | 7.60 | 27.15 | 2036 | C₇₂H₁₃₇N₅O₇S | 1216.98 | 7.96 | 28.08 |
| 1837 | C₆₇H₁₃₂N₂O₉S | 1141.86 | 7.60 | 27.15 | 2037 | C₇₁H₁₃₈N₈O₅ | 1183.94 | 7.59 | 24.76 |
| 1838 | C₆₇H₁₃₂N₂O₉S | 1141.86 | 7.60 | 27.15 | 2038 | C₇₁H₁₃₈N₈O₅ | 1183.94 | 7.59 | 24.76 |
| 1839 | C₆₄H₁₃₂N₂O₆S | 1057.83 | 8.32 | 26.33 | 2039 | C₉₃H₁₇₉N₅O₁S₃ | 1639.66 | 7.56 | 35.27 |
| 1840 | C₆₄H₁₃₂N₂O₆S | 1057.83 | 8.32 | 26.33 | 2040 | C₉₃H₁₅₀N₅O₁₂PS | 1623.52 | 8.52 | 37.28 |
| 1841 | C₆₇H₁₃₂N₂O₉S | 1141.86 | 8.32 | 27.15 | 2041 | C₇₄H₁₃₈N₈O₈ | 1267.97 | 7.59 | 25.58 |
| 1842 | C₆₇H₁₃₂N₂O₉S | 1141.86 | 8.32 | 27.15 | 2042 | C₇₄H₁₃₈N₈O₈ | 1267.97 | 7.59 | 25.58 |
| 1843 | C₆₇H₁₃₂N₂O₉S | 1141.86 | 8.32 | 27.15 | 2043 | C₆₈H₁₄₀N₆O₄ | 1105.91 | 8.62 | 25.32 |
| 1844 | C₆₇H₁₃₂N₂O₉S | 1141.86 | 8.32 | 27.15 | 2044 | C₆₈H₁₄₀N₆O₄ | 1105.91 | 8.62 | 25.32 |
| 1845 | C₆₄H₁₃₂N₂O₇S | 1073.83 | 8.15 | 26.57 | 2045 | C₇₁H₁₄₀N₆O₇ | 1189.94 | 8.62 | 26.14 |
| 1846 | C₆₄H₁₃₂N₂O₇S | 1073.83 | 8.15 | 26.57 | 2046 | C₇₁H₁₄₀N₆O₇ | 1189.94 | 8.62 | 26.14 |
| 1847 | C₆₇H₁₃₂N₂O₁₀S | 1157.86 | 8.15 | 27.39 | 2047 | C₇₁H₁₄₀N₆O₇ | 1189.94 | 8.62 | 26.14 |
| 1848 | C₆₇H₁₃₂N₂O₁₀S | 1157.86 | 8.15 | 27.39 | 2048 | C₇₁H₁₄₀N₆O₇ | 1189.94 | 8.62 | 26.14 |
| 1849 | C₆₇H₁₃₂N₂O₁₀S | 1157.86 | 8.15 | 27.39 | 2049 | C₆₈H₁₃₉N₅O₄ | 1090.89 | 8.61 | 25.54 |
| 1850 | C₆₇H₁₃₂N₂O₁₀S | 1157.86 | 8.15 | 27.39 | 2050 | C₆₈H₁₃₉N₅O₄ | 1090.89 | 8.61 | 25.54 |
| 1851 | C₆₄H₁₃₂N₂O₅S | 1041.83 | 7.83 | 25.84 | 2051 | C₇₁H₁₃₉N₅O₇ | 1174.92 | 8.61 | 26.36 |
| 1852 | C₆₄H₁₃₂N₂O₅S | 1041.83 | 7.83 | 25.84 | 2052 | C₇₁H₁₃₉N₅O₇ | 1174.92 | 8.61 | 26.36 |
| 1853 | C₆₇H₁₃₂N₂O₈S | 1125.86 | 7.83 | 26.66 | 2053 | C₇₁H₁₃₉N₅O₇ | 1174.92 | 8.61 | 26.36 |
| 1854 | C₆₇H₁₃₂N₂O₈S | 1125.86 | 7.83 | 26.66 | 2054 | C₇₁H₁₃₉N₅O₇ | 1174.92 | 8.61 | 26.36 |
| 1855 | C₆₇H₁₃₂N₂O₈S | 1125.86 | 7.84 | 26.66 | 2055 | C₆₉H₁₃₉N₅O₅ | 1118.90 | 7.82 | 25.64 |
| 1856 | C₆₇H₁₃₂N₂O₈S | 1125.86 | 7.84 | 26.66 | 2056 | C₆₉H₁₃₉N₅O₅ | 1118.90 | 7.82 | 25.64 |
| 1857 | C₆₇H₁₃₇N₃O₄ | 1048.85 | 9.07 | 27.10 | 2057 | C₉₀H₁₇₆N₂OS₈ | 1558.89 | 9.96 | 42.56 |
| 1858 | C₆₇H₁₃₇N₃O₄ | 1048.85 | 9.07 | 7.10 | 2058 | C₈₉H₁₇₄N₂OS₈ | 1544.86 | 8.77 | 40.62 |
| 1859 | C₇₀H₁₃₇N₃O₇ | 1132.88 | 9.07 | 27.92 | 2059 | C₇₂H₁₃₉N₅O₈ | 1202.93 | 7.82 | 26.46 |
| 1860 | C₇₀H₁₃₇N₃O₇ | 1132.88 | 9.07 | 27.92 | 2060 | C₇₂H₁₃₉N₅O₈ | 1202.93 | 7.82 | 26.46 |
| 1861 | C₇₀H₁₃₇N₃O₇ | 1132.88 | 9.07 | 27.92 | 2061 | C₇₀H₁₄₁N₅O₅ | 1132.93 | 7.84 | 26.35 |
| 1862 | C₇₀H₁₃₇N₃O₇ | 1132.88 | 9.07 | 27.92 | 2062 | C₇₀H₁₄₁N₅O₅ | 1132.93 | 7.84 | 26.35 |
| 1863 | C₆₉H₁₄₁N₃O₄ | 1076.90 | 9.10 | 28.15 | 2063 | C₇₃H₁₄₁N₅O₈ | 1216.96 | 7.84 | 27.17 |
| 1864 | C₆₉H₁₄₁N₃O₄ | 1076.90 | 9.09 | 28.15 | 2064 | C₇₃H₁₄₁N₅O₈ | 1216.96 | 7.84 | 27.17 |
| 1865 | C₇₂H₁₄₁N₃O₇ | 1160.93 | 9.10 | 28.98 | 2065 | C₇₃H₁₄₁N₅O₈ | 1216.96 | 7.84 | 27.17 |
| 1866 | C₉₅H₁₉₄N₄O₉S₅ | 1696.92 | 9.86 | 40.86 | 2066 | C₇₃H₁₄₁N₅O₈ | 1216.96 | 7.84 | 27.17 |
| 1867 | C₇₂H₁₄₁N₃O₇ | 1160.93 | 9.10 | 28.98 | 2067 | C₆₉H₁₃₇N₅O₆ | 1132.88 | 7.19 | 25.38 |
| 1868 | C₇₂H₁₄₁N₃O₇ | 1160.93 | 9.10 | 28.98 | 2068 | C₆₉H₁₃₇N₅O₆ | 1132.88 | 7.19 | 25.38 |
| 1869 | C₇₁H₁₄₅N₃O₄ | 1104.96 | 9.12 | 29.21 | 2069 | C₇₂H₁₃₇N₅O₉ | 1216.91 | 7.06 | 26.20 |
| 1870 | C₇₁H₁₄₅N₃O₄ | 1104.96 | 9.12 | 29.21 | 2070 | C₇₂H₁₃₇N₅O₉ | 1216.91 | 7.06 | 26.20 |
| 1871 | C₇₄H₁₄₅N₃O₇ | 1188.99 | 9.12 | 30.03 | 2071 | C₇₂H₁₃₇N₅O₉ | 1216.91 | 7.06 | 26.20 |
| 1872 | C₉₆H₁₉₁NO₁₄S₅ | 1743.88 | 7.67 | 41.25 | 2072 | C₇₂H₁₃₇N₅O₉ | 1216.91 | 7.06 | 26.20 |
| 1873 | C₇₄H₁₄₅N₃O₇ | 1188.99 | 9.12 | 30.03 | 2073 | C₆₉H₁₃₈N₄O₆ | 1119.88 | 7.66 | 26.29 |
| 1874 | C₇₄H₁₄₅N₃O₇ | 1188.99 | 9.12 | 30.03 | 2074 | C₆₉H₁₃₈N₄O₆ | 1119.88 | 7.66 | 26.29 |
| 1875 | C₆₆H₁₃₅N₃O₄ | 1034.82 | 9.04 | 26.57 | 2075 | C₇₂H₁₃₈N₄O₉ | 1203.91 | 7.66 | 27.11 |
| 1876 | C₆₆H₁₃₅N₃O₄ | 1034.82 | 9.04 | 26.57 | 2076 | C₇₂H₁₃₈N₄O₉ | 1203.91 | 7.66 | 27.11 |
| 1877 | C₆₉H₁₃₅N₃O₇ | 1118.85 | 9.04 | 27.39 | 2077 | C₇₂H₁₃₈N₄O₉ | 1203.91 | 7.66 | 27.11 |
| 1878 | C₆₉H₁₃₅N₃O₇ | 1118.85 | 9.04 | 27.39 | 2078 | C₇₂H₁₃₈N₄O₉ | 1203.91 | 7.66 | 27.11 |
| 1879 | C₆₉H₁₃₅N₃O₇ | 1118.85 | 9.04 | 27.39 | 2079 | C₇₀H₁₄₀N₄O₅ | 1117.91 | 7.94 | 26.15 |
| 1880 | C₆₉H₁₃₅N₃O₇ | 1118.85 | 9.04 | 27.39 | 2080 | C₇₀H₁₄₀N₄O₅ | 1117.91 | 7.93 | 26.15 |
| 1881 | C₆₇H₁₃₇N₃O₄ | 1048.85 | 9.05 | 27.10 | 2081 | C₇₃H₁₄₀N₄O₈ | 1201.94 | 7.94 | 26.97 |
| 1882 | C₆₇H₁₃₇N₃O₄ | 1048.85 | 9.05 | 27.10 | 2082 | C₇₃H₁₄₀N₄O₈ | 1201.94 | 7.93 | 26.97 |
| 1883 | C₇₀H₁₃₇N₃O₇ | 1132.88 | 9.05 | 27.92 | 2083 | C₇₃H₁₄₀N₄O₈ | 1201.94 | 7.94 | 26.97 |
| 1884 | C₇₀H₁₃₇N₃O₇ | 1132.88 | 9.05 | 27.92 | 2084 | C₇₃H₁₄₀N₄O₈ | 1201.94 | 7.94 | 26.97 |
| 1885 | C₇₀H₁₃₇N₃O₇ | 1132.88 | 9.05 | 27.92 | 2085 | C₇₀H₁₃₈N₄O₆ | 1131.90 | 7.19 | 25.85 |
| 1886 | C₇₀H₁₃₇N₃O₇ | 1132.88 | 9.05 | 27.92 | 2086 | C₇₀H₁₃₈N₄O₆ | 1131.90 | 7.19 | 25.85 |
| 1887 | C₆₈H₁₃₉N₃O₄ | 1062.88 | 9.06 | 27.63 | 2087 | C₇₃H₁₃₈N₄O₉ | 1215.93 | 7.17 | 26.67 |
| 1888 | C₆₈H₁₃₉N₃O₄ | 1062.88 | 9.06 | 27.63 | 2088 | C₇₃H₁₃₈N₄O₉ | 1215.93 | 7.17 | 26.67 |
| 1889 | C₇₁H₁₃₉N₃O₇ | 1146.91 | 9.06 | 28.45 | 2089 | C₇₃H₁₃₈N₄O₉ | 1215.93 | 7.17 | 26.67 |
| 1890 | C₇₁H₁₃₉N₃O₇ | 1146.91 | 9.06 | 28.45 | 2090 | C₇₃H₁₃₈N₄O₉ | 1215.93 | 7.17 | 26.67 |
| 1891 | C₇₁H₁₃₉N₃O₇ | 1146.91 | 9.06 | 28.45 | 2091 | C₇₀H₁₃₉N₅O₆ | 1146.91 | 7.19 | 25.65 |
| 1892 | C₇₁H₁₃₉N₃O₇ | 1146.91 | 9.06 | 28.45 | 2092 | C₇₀H₁₃₉N₅O₆ | 1146.91 | 7.19 | 25.65 |
| 1893 | C₆₇H₁₃₇N₃O₄ | 1048.85 | 9.11 | 26.88 | 2093 | C₇₃H₁₃₉N₅O₉ | 1230.94 | 7.08 | 26.47 |
| 1894 | C₆₇H₁₃₇N₃O₄ | 1048.85 | 9.11 | 26.88 | 2094 | C₇₃H₁₃₉N₅O₉ | 1230.94 | 7.08 | 26.47 |
| 1895 | C₇₀H₁₃₇N₃O₇ | 1132.88 | 9.11 | 27.70 | 2095 | C₇₃H₁₃₉N₅O₉ | 1230.94 | 7.08 | 26.47 |
| 1896 | C₇₀H₁₃₇N₃O₇ | 1132.88 | 9.11 | 27.70 | 2096 | C₇₃H₁₃₉N₅O₉ | 1230.94 | 7.08 | 26.47 |
| 1897 | C₇₀H₁₃₇N₃O₇ | 1132.88 | 9.11 | 27.70 | 2097 | C₇₀H₁₄₁N₅O₅ | 1132.93 | 7.84 | 26.35 |
| 1898 | C₇₀H₁₃₇N₃O₇ | 1132.88 | 9.11 | 27.70 | 2098 | C₇₀H₁₄₁N₅O₅ | 1132.93 | 7.84 | 26.35 |
| 1899 | C₆₈H₁₃₉N₃O₄ | 1062.88 | 9.12 | 27.41 | 2099 | C₇₃H₁₄₁N₅O₈ | 1216.96 | 7.84 | 27.17 |
| 1900 | C₆₈H₁₃₉N₃O₄ | 1062.88 | 9.12 | 27.41 | 2100 | C₇₃H₁₄₁N₅O₈ | 1216.96 | 7.84 | 27.17 |
| 1901 | C₇₁H₁₃₉N₃O₇ | 1146.91 | 9.12 | 28.23 | 2101 | C₇₃H₁₄₁N₅O₈ | 1216.96 | 7.84 | 27.17 |
| 1902 | C₇₁H₁₃₉N₃O₇ | 1146.91 | 9.12 | 28.23 | 2102 | C₇₃H₁₄₁N₅O₈ | 1216.96 | 7.84 | 27.17 |
| 1903 | C₇₁H₁₃₉N₃O₇ | 1146.91 | 9.12 | 28.23 | 2103 | C₇₀H₁₃₈N₄O₇ | 1147.89 | 7.19 | 26.06 |
| 1904 | C₇₁H₁₃₉N₃O₇ | 1146.91 | 9.12 | 28.23 | 2104 | C₇₀H₁₃₈N₄O₇ | 1147.89 | 7.19 | 26.06 |
| 1905 | C₆₉H₁₄₁N₃O₄ | 1076.90 | 9.20 | 27.71 | 2105 | C₆₈H₁₃₅N₅O₅ | 1102.86 | 7.65 | 24.36 |
| 1906 | C₆₉H₁₄₁N₃O₄ | 1076.90 | 9.20 | 27.71 | 2106 | C₆₈H₁₃₅N₅O₅ | 1102.86 | 7.65 | 24.36 |
| 1907 | C₇₂H₁₄₁N₃O₇ | 1160.93 | 9.20 | 28.54 | 2107 | C₇₁H₁₃₅N₅O₈ | 1186.89 | 7.65 | 25.18 |
| 1908 | C₇₂H₁₄₁N₃O₇ | 1160.93 | 9.20 | 28.54 | 2108 | C₇₁H₁₃₅N₅O₈ | 1186.89 | 7.65 | 25.18 |
| 1909 | C₇₂H₁₄₁N₃O₇ | 1160.93 | 9.20 | 28.54 | 2109 | C₇₁H₁₃₅N₅O₈ | 1186.89 | 7.65 | 25.18 |
| 1910 | C₇₂H₁₄₁N₃O₇ | 1160.93 | 9.20 | 28.54 | 2110 | C₇₁H₁₃₅N₅O₈ | 1186.89 | 7.65 | 25.18 |
| 1911 | C₆₉H₁₄₁N₃O₄ | 1076.90 | 9.15 | 27.93 | 2111 | C₆₉H₁₃₆N₄O₅ | 1101.87 | 7.90 | 25.58 |
| 1912 | C₆₉H₁₄₁N₃O₄ | 1076.90 | 9.15 | 27.93 | 2112 | C₆₉H₁₃₆N₄O₅ | 1101.87 | 7.90 | 25.58 |
| 1913 | C₇₂H₁₄₁N₃O₇ | 1160.93 | 9.15 | 28.76 | 2113 | C₇₂H₁₃₆N₄O₈ | 1185.90 | 7.90 | 26.41 |
| 1914 | C₇₂H₁₄₁N₃O₇ | 1160.93 | 9.15 | 28.76 | 2114 | C₇₂H₁₃₆N₄O₈ | 1185.90 | 7.90 | 26.41 |
| 1915 | C₇₂H₁₄₁N₃O₇ | 1160.93 | 9.15 | 28.76 | 2115 | C₇₂H₁₃₆N₄O₈ | 1185.90 | 7.90 | 26.41 |
| 1916 | C₇₂H₁₄₁N₃O₇ | 1160.93 | 9.15 | 28.76 | 2116 | C₇₂H₁₃₆N₄O₈ | 1185.90 | 7.90 | 26.41 |
| 1917 | C₆₇H₁₃₅N₃O₄ | 1046.83 | 8.42 | 26.62 | 2117 | C₇₀H₁₃₇N₅O₆ | 1144.89 | 7.19 | 25.33 |
| 1918 | C₆₇H₁₃₅N₃O₄ | 1046.83 | 8.42 | 26.62 | 2118 | C₇₀H₁₃₇N₅O₆ | 1144.89 | 7.19 | 25.33 |
| 1919 | C₇₀H₁₃₅N₃O₇ | 1130.86 | 8.42 | 27.44 | 2119 | C₁₀₀H₁₉₅NO₉S₂ | 1619.78 | 9.12 | 42.45 |
| 1920 | C₇₀H₁₃₅N₃O₇ | 1130.86 | 8.42 | 27.44 | 2120 | C₉₉H₁₈₉N₃O₉ | 1565.61 | 8.77 | 41.14 |
| 1921 | C₇₀H₁₃₅N₃O₇ | 1130.86 | 8.42 | 27.44 | 2121 | C₇₃H₁₃₇N₅O₉ | 1228.92 | 7.07 | 26.15 |
| 1922 | C₇₀H₁₃₅N₃O₇ | 1130.86 | 8.42 | 27.44 | 2122 | C₇₃H₁₃₇N₅O₉ | 1228.92 | 7.07 | 26.15 |
| 1923 | C₆₉H₁₃₉N₃O₄ | 1074.89 | 8.99 | 27.51 | 2123 | C₇₁H₁₃₉N₅O₅ | 1142.92 | 7.47 | 25.63 |
| 1924 | C₆₉H₁₃₉N₃O₄ | 1074.89 | 8.99 | 27.51 | 2124 | C₇₁H₁₃₉N₅O₅ | 1142.92 | 7.47 | 25.63 |
| 1925 | C₇₂H₁₃₉N₃O₇ | 1158.92 | 8.99 | 28.33 | 2125 | C₁₀₃H₁₉₄N₂O₁₅S ₂ | 1764.80 | 8.09 | 42.63 |
| 1926 | C₇₂H₁₃₉N₃O₇ | 1158.92 | 8.99 | 28.33 | 2126 | C₁₀₁H₁₉₆N₂O₁₄S | 1694.74 | 9.65 | 42.49 |
| 1927 | C₇₂H₁₃₉N₃O₇ | 1158.92 | 9.00 | 28.33 | 2127 | C₇₄H₁₃₉N₅O₈ | 1226.95 | 7.47 | 26.45 |
| 1928 | C₇₂H₁₃₉N₃O₇ | 1158.92 | 9.00 | 28.33 | 2128 | C₇₄H₁₃₉N₅O₈ | 1226.95 | 7.47 | 26.45 |
| 1929 | C₆₇H₁₃₅N₃O₄ | 1046.83 | 8.77 | 26.65 | 2129 | C₇₁H₁₃₈N₈O₄ | 1167.94 | 7.58 | 25.74 |
| 1930 | C₆₇H₁₃₅N₃O₄ | 1046.83 | 8.77 | 26.65 | 2130 | C₇₁H₁₃₈N₈O₄ | 1167.94 | 7.58 | 25.74 |
| 1931 | C₇₀H₁₃₅N₃O₇ | 1130.86 | 8.77 | 27.47 | 2131 | C₈₈H₁₇₂N₄O₇ | 1398.36 | 7.81 | 35.59 |
| 1932 | C₇₀H₁₃₅N₃O₇ | 1130.86 | 8.77 | 27.47 | 2132 | C₈₄H₁₇₁NO₇S | 1339.35 | 10.0 2 | 36.46 |
| 1933 | C₇₀H₁₃₅N₃O₇ | 1130.86 | 8.77 | 27.47 | 2133 | C₇₄H₁₃₈N₈O₇ | 1251.97 | 7.58 | 26.56 |
| 1934 | C₇₀H₁₃₅N₃O₇ | 1130.86 | 8.77 | 27.47 | 2134 | C₇₄H₁₃₈N₈O₇ | 1251.97 | 7.58 | 26.56 |
| 1935 | C₆₈H₁₃₇N₃O₄ | 1060.86 | 9.40 | 26.63 | 2135 | C₆₀H₁₀₀N₂O₈S | 1009.53 | 7.01 | 18.88 |
| 1936 | C₆₈H₁₃₇N₃O₄ | 1060.86 | 9.40 | 26.63 | 2136 | C₆₁H₁₀₅N₃O₆S₃ | 1072.71 | 8.34 | 21.30 |
| 1937 | C₇₁H₁₃₇N₃O₇ | 1144.89 | 9.40 | 27.45 | 2137 | C₆₀H₁₀₅NO₂S₄ | 1000.75 | 8.96 | 22.13 |
| 1938 | C₇₁H₁₃₇N₃O₇ | 1144.89 | 9.40 | 27.45 | 2138 | C₆₃H₁₁₄N₆O₇ | 1067.64 | 10.0 0 | 21.45 |
| 1939 | C₇₁H₁₃₇N₃O₇ | 1144.89 | 9.40 | 27.45 | 2139 | C₅₉H₁₀₁NO₁₀ | 984.45 | 8.35 | 21.09 |
| 1940 | C₇₁H₁₃₇N₃O₇ | 1144.89 | 9.40 | 27.45 | 2140 | C₆₀H₁₀₈N₄O₆S | 1013.61 | 8.40 | 19.22 |
| 1941 | C₆₈H₁₃₇N₃O₄ | 1060.86 | 9.62 | 25.87 | 2141 | C₆₄H₁₁₈N₃O₇P | 1072.64 | 8.79 | 20.87 |
| 1942 | C₆₈H₁₃₇N₃O₄ | 1060.86 | 9.62 | 25.87 | 2142 | C₅₆H₁₀₃NO₉S₄ | 1062.68 | 10.1 3 | 19.46 |
| 1943 | C₇₁H₁₃₇N₃O₇ | 1144.89 | 9.62 | 26.69 | 2143 | C₅₉H₁₀₇NO₉S₄ | 1102.74 | 9.62 | 17.16 |
| 1944 | C₇₁H₁₃₇N₃O₇ | 1144.89 | 9.62 | 26.69 | 2144 | C₆₀H₁₁₀N₂O₁₂S₃ | 1147.72 | 8.95 | 19.15 |
| 1945 | C₇₁H₁₃₇N₃O₇ | 1144.89 | 9.62 | 26.69 | 2145 | C₆₄H₁₁₃N₃O₄S₃ | 1084.80 | 10.1 7 | 23.74 |
| 1946 | C₇₁H₁₃₇N₃O₇ | 1144.89 | 9.62 | 26.69 | 2146 | C₆₀H₁₀₄N₂O₅S₃ | 1029.68 | 9.96 | 22.15 |
| 1947 | C₆₈H₁₃₇N₃O₄ | 1060.86 | 8.87 | 27.21 | 2147 | C₆₃H₁₀₉N₃O₆S₄ | 1132.82 | 9.65 | 22.68 |
| 1948 | C₆₈H₁₃₇N₃O₄ | 1060.86 | 8.87 | 27.21 | 2148 | C₆₂H₁₁₀N₂O₂S₇ | 1139.99 | 10.0 3 | 24.47 |
| 1949 | C₇₁H₁₃₇N₃O₇ | 1144.89 | 8.87 | 28.03 | 2149 | C₅₇H₁₀₂N₆O₃S | 951.54 | 8.64 | 15.85 |
| 1950 | C₇₁H₁₃₇N₃O₇ | 1144.89 | 8.87 | 28.03 | 2150 | C₅₃H₉₇NO₇ | 860.36 | 9.79 | 19.82 |
| 1951 | C₇₁H₁₃₇N₃O₇ | 1144.89 | 8.87 | 28.03 | 2151 | C₅₇H₁₀₃N₃O₂S₃ | 958.65 | 8.96 | 18.52 |
| 1952 | C₇₁H₁₃₇N₃O₇ | 1144.89 | 8.87 | 28.03 | 2152 | C₅₆H₁₀₃NS₄ | 918.69 | 10.2 1 | 21.10 |
| 1953 | C₆₈H₁₃₈N₄O₄ | 1075.88 | 8.77 | 25.07 | 2153 | C₅₆H₁₀₃NO₃S₇ | 1062.86 | 9.96 | 23.06 |
| 1954 | C₆₈H₁₃₈N₄O₄ | 1075.88 | 8.77 | 25.07 | 2154 | C₆₄H₁₁₉NO₈S₄ | 1158.89 | 8.40 | 26.14 |
| 1955 | C₇₁H₁₃₈N₄O₇ | 1159.91 | 8.77 | 25.89 | 2155 | C₆₇H₁₂₂N₄O₅S₄ | 1191.98 | 8.15 | 26.63 |
| 1956 | C₇₁H₁₃₈N₄O₇ | 1159.91 | 8.77 | 25.89 | 2156 | C₇₀H₁₂₈N₂O₅S₅ | 1238.10 | 8.80 | 28.64 |
| 1957 | C₇₁H₁₃₈N₄O₇ | 1159.91 | 8.77 | 25.89 | 2157 | C₆₇H₁₂₂N₂O₅S₄ | 1163.96 | 7.06 | 25.24 |
| 1958 | C₇₁H₁₃₈N₄O₇ | 1159.91 | 8.77 | 25.89 | 2158 | C₆₄H₁₁₇NOS₇ | 1141.07 | 9.43 | 28.02 |
| 1959 | C₆₇H₁₃₅N₃O₅ | 1062.83 | 7.19 | 26.00 | 2159 | C₇₂H₁₄₂N₇O₆P | 1232.94 | 10.1 0 | 26.28 |
| 1960 | C₆₇H₁₃₅N₃O₅ | 1062.83 | 7.19 | 26.00 | 2160 | C₇₂H₁₃₀N₆O₇S₂ | 1255.99 | 8.52 | 24.50 |
| 1961 | C₁₀₄H₁₉₇N₃O₇S ₅ | 1762.03 | 7.97 | 43.84 | 2161 | C₆₇H₁₃₀N₆O₂ | 1051.82 | 8.75 | 23.16 |
| 1962 | C₉₉H₂₀₃N₂O₁₄P ₃S₂ | 1802.75 | 7.87 | 40.39 | 2162 | C₆₉H₁₃₀N₄O₇ | 1127.82 | 9.95 | 26.16 |
| 1963 | C₇₀H₁₃₅N₃O₈ | 1146.86 | 6.84 | 26.82 | 2163 | C₇₂H₁₂₉N₉O₆S | 1248.94 | 8.63 | 24.39 |
| 1964 | C₇₀H₁₃₅N₃O₈ | 1146.86 | 6.83 | 26.82 | 2164 | C₇₀H₁₃₁NO₁₀ | 1146.82 | 10.0 1 | 27.80 |
| 1965 | C₆₉H₁₃₉N₃O₄ | 1074.89 | 9.80 | 26.43 | 2165 | C₆₇H₁₂₁N₃O₈S₃ | 1192.90 | 7.17 | 27.01 |
| 1966 | C₆₉H₁₃₉N₃O₄ | 1074.89 | 9.80 | 26.43 | 2166 | C₆₆H₁₂₃N₃O₇S | 1102.78 | 7.67 | 23.92 |
| 1967 | C₇₂H₁₃₉N₃O₇ | 1158.92 | 9.80 | 27.25 | 2167 | C₇₂H₁₄₀N₂O₁₃P₄ | 1365.81 | 8.13 | 23.27 |
| 1968 | C₇₂H₁₃₉N₃O₇ | 1158.92 | 9.80 | 27.25 | 2168 | C₇₃H₁₃₉N₄O₁₃P₃ | 1373.85 | 7.94 | 22.39 |
| 1969 | C₇₂H₁₃₉N₃O₇₊₊ | 1158.92 | 9.80 | 27.25 | 2169 | C₆₄H₁₂₅N₃O₇S₃ | 1144.90 | 9.63 | 22.96 |
| 1970 | C₇₂H₁₃₉N₃O₇ | 1158.92 | 9.80 | 27.25 | 2170 | C₆₈H₁₂₇N₃O₇S₃ | 1194.96 | 10.1 8 | 28.67 |
| 1971 | C₆₆H₁₃₅N₃O₅ | 1050.82 | 8.20 | 25.62 | 2171 | C₆₉H₁₂₉NO₃S₇ | 1245.21 | 10.0 2 | 29.71 |
| 1972 | C₆₆H₁₃₅N₃O₅ | 1050.82 | 8.20 | 25.62 | 2172 | C₆₄H₁₁₈N₂O₂S₆ | 1140.02 | 7.72 | 28.06 |
| 1973 | C₆₉H₁₃₅N₃O₈ | 1134.85 | 8.20 | 26.44 | 2173 | C₆₅H₁₂₅NO₃S₆ | 1161.08 | 9.59 | 30.57 |
| 1974 | C₆₉H₁₃₅N₃O₈ | 1134.85 | 8.20 | 26.44 | 2174 | C₆₇H₁₂₉NO₂S₇ | 1205.19 | 10.2 5 | 31.99 |
| 1975 | C₆₉H₁₃₅N₃O₈ | 1134.85 | 8.20 | 26.44 | 2175 | C₆₃H₁₂₁N₃OS₈ | 1193.16 | 8.62 | 29.09 |
| 1976 | C₆₉H₁₃₅N₃O₈ | 1134.85 | 8.20 | 26.44 | 2176 | C₅₆H₁₀₄N₄O₃S₂ | 945.59 | 10.0 8 | 17.35 |
| 1977 | C₆₇H₁₃₇N₃O₅ | 1064.85 | 8.67 | 25.91 | 2177 | C₅₅H₁₀₃N₁₁O₄ | 982.50 | 7.63 | 16.96 |
| 1978 | C₆₇H₁₃₇N₃O₅ | 1064.85 | 8.67 | 25.91 | 2178 | C₅₅H₁₀₀N₄O₈S | 977.48 | 9.61 | 18.82 |
| 1979 | C₇₀H₁₃₇N₃O₈ | 1148.88 | 8.67 | 26.73 | 2179 | C₅₇H₁₀₈N₁₀O₈S₄ | 1189.79 | 8.50 | 16.73 |
| 1980 | C₇₀H₁₃₇N₃O₈ | 1148.88 | 8.67 | 26.73 | 2180 | C₅₃H₁₀₂N₆O₁₀S₄ | 1111.67 | 8.18 | 14.64 |
| 1981 | C₇₀H₁₃₇N₃O₈ | 1148.88 | 8.67 | 26.73 | 2181 | C₅₉H₁₀₀N₆O₈ | 1021.48 | 8.42 | 18.23 |
| 1982 | C₇₀H₁₃₇N₃O₈ | 1148.88 | 8.67 | 26.73 | 2182 | C₅₄H₉₈N₂O₈ | 903.38 | 8.85 | 20.07 |
| 1983 | C₆₈H₁₃₉N₃O₅ | 1078.88 | 8.87 | 25.64 | 2183 | C₅₆H₁₀₄N₄O₁₀S | 1025.53 | 9.53 | 19.39 |
| 1984 | C₆₈H₁₃₉N₃O₅ | 1078.88 | 8.87 | 25.64 | 2184 | C₅₃H₉₈N₄O₇S₂ | 967.51 | 8.78 | 17.62 |
| 1985 | C₇₁H₁₃₉N₃O₈ | 1162.91 | 8.87 | 26.46 | 2185 | C₅₄H₁₀₃NO₆S | 894.48 | 9.81 | 18.88 |
| 1986 | C₉₃H₁₈₇NO₈S₆ | 1639.88 | 8.87 | 39.61 | 2186 | C₆₀H₁₁₅N₄O₁₂P₃ S | 1209.58 | 7.38 | 16.63 |
| 1987 | C₇₁H₁₃₉N₃O₈ | 1162.91 | 8.87 | 26.46 | 2187 | ₄₈H₉₁NO₁₄S₄ | 1034.49 | 8.57 | 18.09 |
| 1988 | C₇₁H₁₃₉N₃O₈ | 1162.91 | 8.87 | 26.46 | 2188 | C₅₃H₉₇N₃O₇S₃ | 984.55 | 7.65 | 15.34 |
| 1989 | C₆₈H₁₃₈N₄O₆ | 1107.87 | 7.80 | 25.43 | 2189 | C₅₃H₁₀₀N₄O₁₀S₄ | 1081.64 | 8.02 | 15.11 |
| 1990 | C₆₈H₁₃₈N₄O₆ | 1107.87 | 7.80 | 25.43 | 2190 | C₅₉H₁₀₄N₂O₈S₄ | 1097.73 | 8.07 | 20.70 |
| 1991 | C₉₈H₁₉₅N₅O₅S₃ | 1619.85 | 7.45 | 41.60 | 2191 | C₅₇H₁₀₃NO₆S₄ | 1026.69 | 10.1 6 | 22.82 |
| 1992 | C₉₈H₁₉₃NO₁₀S₃ | 1641.80 | 9.82 | 42.89 | 2192 | C₅₇H₁₀₃NO₆S₆ | 1090.81 | 8.72 | 25.21 |
| 1993 | C₇₁H₁₃₈N₄O₉ | 1191.90 | 7.80 | 26.25 | 2193 | C₅₇H₁₀₃NO₆S₇ | 1122.87 | 9.87 | 23.86 |
| 1994 | C₇₁H₁₃₈N₄O₉ | 1191.90 | 7.80 | 26.25 | 2194 | C₅₇H₉₉N₃O₈S₇ | 1178.85 | 7.78 | 21.15 |
| 1995 | C₆₉H₁₃₇N₅O₅ | 1116.88 | 7.49 | 25.53 | 2195 | C₅₆H₁₀₉N₂O₃PS₆ | 1081.83 | 8.96 | 22.34 |
| 1996 | C₆₉H₁₃₇N₅O₅ | 1116.88 | 7.49 | 25.53 | 2196 | C₆₉H₁₂₁NO₅S | 1076.79 | 9.98 | 23.52 |
| 1997 | C₇₂H₁₃₇N₅O₈ | 1200.91 | 7.49 | 26.35 | 2197 | C₇₀H₁₂₂N₄O₂S | 1147.95 | 8.85 | 22.42 |
| 1998 | C₇₂H₁₃₇N₅O₈ | 1200.91 | 7.49 | 26.35 | 2198 | C₇₂H₁₂₃N₉O₇ | 1226.83 | 8.52 | 18.69 |
| 1999 | C₇₂H₁₃₇N₅O₈ | 1200.91 | 7.49 | 26.35 | 2199 | C₆₇H₁₁₇N₅O₅ | 1072.70 | 8.36 | 19.23 |
| 2000 | C₇₂H₁₃₇N₅O₈ | 1200.91 | 7.49 | 26.35 | 2200 | C₆₈H₁₂₇N₁₁O₄ | 1162.84 | 9.49 | 19.64 |
| 2201 | C₆₈H₁₂₅N₇O₅ | 1120.79 | 9.91 | 22.00 | 2401 | C₇₇H₁₅₇N₃O₁₀S₃ | 1381.31 | 10.2 9 | 31.76 |
| 2202 | C₇₀H₁₂₆N₁₀O₇S | 1251.90 | 7.99 | 20.23 | 2402 | C₇₇H₁₄₈N₄O₇S₇ | 1466.52 | 7.62 | 33.01 |
| 2203 | C₆₇H₁₁₈N₈O₈ | 1163.73 | 7.71 | 21.54 | 2403 | C₇₆H₁₄₇NO₂S₆ | 1299.42 | 10.0 2 | 34.04 |
| 2204 | C₆₇H₁₂₃N₇O₂ | 1058.77 | 8.87 | 20.62 | 2404 | C₇₆H₁₅₃NOS₈ | 1353.60 | 10.0 9 | 38.26 |
| 2205 | C₆₇H₁₂₂N₁₀O | 1083.78 | 8.58 | 19.27 | 2405 | C₇₅H₁₄₉N₃O₂S₈ | 1381.57 | 8.73 | 35.21 |
| 2206 | C₆₃H₁₁₈N₄O₆S₅ | 1187.96 | 9.68 | 21.42 | 2406 | C₉₉H₁₉₃N₇O₆ | 1577.68 | 8.36 | 38.16 |
| 2207 | C₆₅H₁₁₁NO₇S | 1050.66 | 8.10 | 22.75 | 2407 | C₉₉H₁₈₉N₃O₅S₃ | 1597.82 | 8.77 | 42.25 |
| 2208 | C₇₀H₁₁₉N₃O₉S₅ | 1307.03 | 7.99 | 25.56 | 2408 | C₉₆H₁₈₉NO₃S₅ | 1565.91 | 9.91 | 45.50 |
| 2209 | C₆₇H₁₁₈N₂O₁₂S ₅ | 1303.98 | 8.08 | 20.95 | 2409 | C₁₀₆H₂₀₅NO₅ | 1573.81 | 9.90 | 46.33 |
| 2210 | C₆₄H₁₁₇NO₁₃S₃ | 1204.81 | 9.78 | 23.12 | 2410 | C₁₀₇H₂₀₀N₅O₅ | 1645.88 | 8.96 | 44.62 |
| 2211 | C₆₁H₁₁₂N₂O₁₄S ₃ | 1193.75 | 9.49 | 22.15 | 2411 | C₁₀₅H₂₀₄N₆O₃S₂ | 1662.96 | 8.70 | 42.53 |
| 2212 | C₇₂H₁₃₃N₄O₁₂P ₃ | 1339.79 | 8.12 | 20.87 | 2412 | C₁₀₆H₂₀₅N₃O₉ | 1665.82 | 8.80 | 45.11 |
| 2213 | C₆₈H₁₂₈NO₁₀P₃ S₂ | 1276.81 | 9.04 | 21.27 | 2413 | C₁₀₄H₂₀₄N₂O₃S₃ | 1626.99 | 9.75 | 44.82 |
| 2214 | C₆₅H₁₁₇NO₇S₄ | 1152.89 | 9.86 | 19.82 | 2414 | C₁₀₄H₂₀₁NOS₇ | 1706.23 | 9.86 | 49.18 |
| 2215 | C₆₆H₁₂₀N₆O₈S₃ | 1221.90 | 9.07 | 19.62 | 2415 | C₁₀₅H₂₀₄N₃O₅PS ₆ | 1812.18 | 7.51 | 47.43 |
| 2216 | C₆₈H₁₂₄N₂O₁₀S ₅ | 1290.04 | 9.08 | 22.31 | 2416 | C₁₀₅H₂₀₆N₆O₆S | 1680.91 | 8.82 | 42.03 |
| 2217 | C₆₃H₁₁₂N₄O₂S₆ | 1149.97 | 7.37 | 24.03 | 2417 | C₁₀₃H₂₀₄N₃O₄S₂ | 1682.95 | 8.74 | 43.18 |
| 2218 | C₇₀H₁₃₁N₃O₄S₂ | 1142.95 | 8.36 | 25.63 | 2418 | C₁₀₆H₂₁₁N₇O₂ | 1615.90 | 8.51 | 43.80 |
| 2219 | C₆₇H₁₃₀N₂O₅ | 1043.79 | 9.54 | 27.28 | 2419 | C₁₀₃H₂₁₁N₄O₃P | 1584.82 | 9.91 | 45.36 |
| 2220 | C₆₈H₁₃₁NO₅S₄ | 1171.04 | 8.83 | 28.53 | 2420 | C₁₀₄H₂₀₈N₆O₆ | 1638.84 | 8.96 | 43.83 |
| 2221 | C₈₇H₁₆₉NO₅S | 1341.37 | 9.31 | 36.31 | 2421 | C₁₀₇H₂₀₈N₆O₁₀S | 1770.94 | 8.19 | 44.86 |
| 2222 | C₈₈H₁₇₂N₂O₅ | 1338.35 | 9.01 | 37.81 | 2422 | C₁₀₂H₂₀₂N₄O₈S | 1644.82 | 9.95 | 44.99 |
| 2223 | C₈₇H₁₆₉NO₆S₃ | 1421.49 | 9.94 | 39.01 | 2423 | C₁₀₆H₂₀₈N₄O₇S₃ | 1747.05 | 9.64 | 47.63 |
| 2224 | C₉₂H₁₇₅N₆O₇PS ₃ | 1604.60 | 7.83 | 37.76 | 2424 | C₁₀₆H₂₀₇N₄O₉PS ₃ | 1809.02 | 8.18 | 47.75 |
| 2225 | C₈₇H₁₇₀N₄O₂S₆ | 1496.70 | 8.30 | 39.06 | 2425 | C₁₀₂H₂₀₁NO₉S₄ | 1714.00 | 9.33 | 48.93 |
| 2226 | C₈₂H₁₆₈N₄OS | 1258.33 | 9.43 | 35.75 | 2426 | C₁₀₄H₂₀₁NO₇S₄ | 1706.02 | 9.76 | 48.92 |
| 2227 | C₈₉H₁₇₃NO₉S₄ | 1529.60 | 9.78 | 39.84 | 2427 | C₁₀₂H₂₀₃NO₅ | 1523.75 | 9.86 | 45.85 |
| 2228 | C₈₇H₁₇₅NO₁₆S₄ | 1619.59 | 9.94 | 38.90 | 2428 | C₁₀₈H₂₁₄N₇O₁₂P ₃ | 1895.86 | 7.23 | 41.61 |
| 2229 | C₈₉H₁₇₇NO₁₃S₄ | 1597.63 | 10.04 | 39.39 | 2429 | C₁₀₀H₁₉₉N₃O₁₅S ₅ | 1844.03 | 7.71 | 46.10 |
| 2230 | C₈₅H₁₇₂N₆O₁₀S ₄ | 1566.58 | 8.43 | 34.65 | 2430 | C₉₉H₁₉₉NO₁₄S₅ | 1788.01 | 8.79 | 44.17 |
| 2231 | C₈₅H₁₇₅N₅O₉S₃ | 1507.54 | 9.58 | 34.71 | 2431 | C₁₀₃H₂₀₆N₆O₇S₄ | 1769.08 | 8.96 | 44.54 |
| 2232 | C₈₅H₁₇₄N₂O₁₁S ₄ | 1528.60 | 8.94 | 37.92 | 2432 | C₁₀₃H₂₀₉N₆O₁₀P S₃ | 1819.01 | 8.49 | 41.31 |
| 2233 | C₈₅H₁₇₁N₃O₁₀S ₄ | 1523.58 | 8.67 | 35.43 | 2433 | C₉₉H₂₀₁N₃O₁₀S₃ | 1689.91 | 9.61 | 44.80 |
| 2234 | C₉₀H₁₇₃N₃O₆S₄ | 1521.66 | 8.82 | 38.34 | 2434 | C₁₀₁H₂₀₅NO₁₀S₅ | 1754.08 | 9.99 | 43.99 |
| 2235 | C₉₀H₁₇₃NO₃S₈ | 1573.91 | 9.95 | 43.51 | 2435 | C₁₀₄H₁₉₉N₃O₅S₅ | 1732.09 | 7.64 | 46.37 |
| 2236 | C₇₂H₁₄₀N₆O₅ | 1169.95 | 8.47 | 24.78 | 2436 | C₁₀₃H₁₉₉N₃O₉S₄ | 1752.00 | 7.51 | 47.66 |
| 2237 | C₆₆H₁₃₇N₇O₂ | 1060.87 | 8.33 | 24.92 | 2437 | C₉₇H₁₉₃N₇O₅S | 1569.72 | 9.22 | 38.28 |
| 2238 | C₇₃H₁₄₁N₃O₇S₂ | 1237.08 | 9.74 | 28.32 | 2438 | C₉₃H₁₈₈N₆O₇S₄ | 1630.83 | 7.70 | 37.71 |
| 2239 | C₆₈H₁₃₆N₄O₈ | 1137.86 | 9.16 | 26.01 | 2439 | C₁₀₀H₁₉₆N₄O₈ | 1582.69 | 9.91 | 43.00 |
| 2240 | C₇₀H₁₂₀N₄O₁₀S | 1229.97 | 9.11 | 27.00 | 2440 | C₉₉H₁₉₂N₂O₉S₄ | 1682.90 | 8.81 | 44.24 |
| 2241 | C₆₈H₁₃₃NO₁₁S | 1172.87 | 9.51 | 27.24 | 2441 | C₉₄H₁₉₁NO₃S | 1415.63 | 10.1 0 | 42.86 |
| 2242 | C₆₉H₁₃₁NO₁₀S | 1166.87 | 9.12 | 27.95 | 2442 | C₁₀₀H₂₀₅N₂O₁₁P ₃ | 1704.66 | 10.1 4 | 43.84 |
| 2243 | C₆₇H₁₃₅NO₄S | 1050.89 | 9.96 | 27.64 | 2443 | C₉₂H₁₈₆N₂O₁₁S₃ | 1592.70 | 8.75 | 40.86 |
| 2244 | C₇₀H₁₄₅N₂O₁₂P ₃ | 1299.85 | 7.89 | 25.09 | 2444 | C₉₄H₁₈₇N₃O₁₅S₃ | 1695.74 | 7.43 | 40.74 |
| 2245 | C₇₄H₁₅₂NO₁₂P₃ S | 1373.01 | 9.94 | 27.12 | 2445 | C₉₃H₁₉₀N₂O₆S₃ | 1528.75 | 10.0 6 | 38.72 |
| 2246 | C₆₉H₁₃₈N₂O₁₀S ₃ | 1252.07 | 9.92 | 27.38 | 2446 | C₉₄H₁₈₇N₃O₁₂S₄ | 1679.81 | 7.37 | 39.57 |
| 2247 | C₆₉H₁₃₉NO₁₅S₄ | 1351.13 | 9.90 | 28.99 | 2447 | C₁₀₁H₁₉₆N₃O₇PS ₆ | 1788.07 | 8.79 | 44.46 |
| 2248 | C₇₂H₁₃₈N₆O₁₃S ₅ | 1456.26 | 8.64 | 26.43 | 2448 | C₉₄H₁₈₇NOS₆ | 1539.94 | 9.73 | 45.76 |
| 2249 | C₇₀H₁₃₇N₃O₉S₃ | 1261.08 | 8.66 | 22.65 | 2449 | C₇₇H₁₂₀N₆O₃S₂ | 1241.98 | 8.70 | 26.41 |
| 2250 | C₆₇H₁₃₈N₆O₇S₃ | 1236.08 | 8.87 | 26.36 | 2450 | C₇₅H₁₂₄N₄O₆ | 1177.84 | 10.2 0 | 25.90 |
| 2251 | C₆₉H₁₃₇N₃O₁₃S ₃ | 1313.06 | 8.54 | 25.97 | 2451 | C₇₂H₁₁₅NO₆S₂ | 1154.85 | 10.0 6 | 27.89 |
| 2252 | C₇₂H₁₃₇N₃O₂S₆ | 1269.31 | 8.65 | 29.55 | 2452 | C₇₆H₁₂₅N₅O₄S₃ | 1269.07 | 9.66 | 26.77 |
| 2253 | C₇₄H₁₄₆NO₃PS₆ | 1321.36 | 10.13 | 32.30 | 2453 | C₇₅H₁₁₇N₃O₂S₈ | 1349.32 | 7.66 | 27.75 |
| 2254 | C₉₀H₁₄₇N₃O₈ | 1399.18 | 7.90 | 32.42 | 2454 | C₇₇H₁₂₃N₅O₆S | 1246.93 | 8.19 | 24.95 |
| 2255 | C₈₉H₁₄₉NO₆S | 1361.24 | 9.32 | 34.59 | 2455 | C₇₆H₁₂₇N₇O₄ | 1202.90 | 9.00 | 24.97 |
| 2256 | C₈₈H₁₄₆N₂O₇S | 1376.21 | 6.19 | 33.17 | 2456 | C₇₅H₁₂₆N₆O₈ | 1239.87 | 9.84 | 25.43 |
| 2257 | C₈₈H₁₅₀N₄O₄S | 1360.26 | 10.02 | 32.47 | 2457 | C₆₈H₁₁₅N₄O₃S | 1071.78 | 9.27 | 26.81 |
| 2258 | C₉₂H₁₅₄N₆O₃S₃ | 1488.48 | 9.03 | 34.03 | 2458 | C₇₄H₁₂₈N₄O₈S₄ | 1330.13 | 10.3 3 | 26.97 |
| 2259 | C₈₉H₁₅₀N₂O₇ | 1360.19 | 10.09 | 35.36 | 2459 | C₇₄H₁₁₉NO₉ | 1166.76 | 10.0 0 | 26.77 |
| 2260 | C₈₈H₁₄₄N₂O₇S | 1374.19 | 8.78 | 34.21 | 2460 | C₇₂H₁₁₈N₂O₁₀ | 1171.74 | 9.32 | 24.67 |
| 2261 | C₉₀H₁₅₅N₇O₅ | 1415.27 | 9.54 | 32.12 | 2461 | C₇₈H₁₂₁N₃O₁₂S₂ | 1356.97 | 8.21 | 25.60 |
| 2262 | C₉₀H₁₅₈N₅O₅P | 1421.26 | 8.10 | 33.64 | 2462 | C₈₀H₁₃₇N₂O₁₁P₃ | 1395.90 | 8.95 | 25.51 |
| 2263 | C₉₀H₁₅₃N₇O₉S | 1509.32 | 8.41 | 32.06 | 2463 | C₇₇H₁₃₅N₄O₁₀P₃ S | 1401.93 | 7.50 | 25.37 |
| 2264 | C₉₃H₁₆₄N₃O₁₁P ₃S | 1625.33 | 8.74 | 32.17 | 2464 | C₆₉H₁₁₈N₄O₉S₄ | 1275.99 | 7.50 | 25.37 |
| 2265 | C₉₂H₁₆₄NO₁₄P₃ S | 1633.31 | 7.95 | 33.25 | 2465 | C₇₁H₁₁₉NO₁₃S₄ | 1322.99 | 9.94 | 25.39 |
| 2266 | C₈₈H₁₅₀N₄O₁₂S ₄ | 1584.45 | 8.03 | 31.72 | 2466 | C₇₂H₁₁₅N₃O₃S₄ | 1199.00 | 8.42 | 26.27 |
| 2267 | C₈₈H₁₅₃N₃O₁₃S ₃ | 1557.40 | 9.96 | 35.94 | 2467 | C₇₃H₁₁₈N₂O₂S₇ | 1280.23 | 9.68 | 28.94 |
| 2268 | C₉₃H₁₆₇N₂O₁₁P ₃ | 1582.28 | 10.00 | 33.87 | 2468 | C₇₀H₁₃₃NO₄S₂ | 1116.97 | 9.97 | 28.02 |
| 2269 | C₉₇H₁₆₈N₃O₁₂P ₃S | 1693.41 | 8.60 | 32.71 | 2469 | C₆₇H₁₂₉NO₇ | 1060.77 | 8.51 | 28.62 |
| 2270 | C₈₇H₁₅₁N₃O₉S₃ | 1479.38 | 8.83 | 30.34 | 2470 | C₆₈H₁₂₇N₃O₂S₃ | 1114.98 | 8.46 | 26.40 |
| 2271 | C₈₇H₁₅₄N₆O₇S₅ | 1556.56 | 8.94 | 32.10 | 2471 | C₆₆H₁₂₄N₄S₄ | 1102.01 | 7.24 | 26.38 |
| 2272 | C₈₇H₁₄₃NO₅S₇ | 1507.57 | 9.36 | 38.83 | 2472 | C₆₇H₁₃₅N₇O₃S | 1118.93 | 8.80 | 26.76 |
| 2273 | C₈₈H₁₄₄N₂O₅S₆ | 1502.53 | 8.66 | 36.59 | 2473 | C₆₅H₁₃₃N₅O₂ | 1016.81 | 9.94 | 25.89 |
| 2274 | C₈₉H₁₅₃N₅OS₃ | 1405.44 | 9.38 | 36.17 | 2474 | C₆₂H₁₂₇N₅O₈S₃ | 1166.93 | 8.18 | 24.53 |
| 2275 | C₈₈H₁₅₂N₄O₂S₄ | 1426.48 | 8.67 | 36.49 | 2475 | C₆₇H₁₃₈N₄O₆S₅ | 1256.20 | 9.66 | 27.43 |
| 2276 | C₅₃H₁₀₁N₇O₅ | 916.43 | 9.37 | 14.69 | 2476 | C₆₅H₁₃₄N₆O₇S₅ | 1272.16 | 8.65 | 24.58 |
| 2277 | C₄₈H₉₁NO₉S | 858.32 | 7.45 | 18.24 | 2477 | C₆₉H₁₂₉N₅O₈ | 1156.82 | 7.35 | 25.88 |
| 2278 | C₄₉H₉₃NO₇S | 840.35 | 9.87 | 18.01 | 2478 | C₆₄H₁₂₃NO₁₀S | 1098.75 | 8.66 | 26.59 |
| 2279 | C₅₀H₉₂N₄O₆S₃ | 941.51 | 8.18 | 17.07 | 2479 | C₆₈H₁₃₀N₄O₈S₅ | 1292.14 | 8.76 | 29.68 |
| 2280 | C₇₃H₁₃₅NO₅ | 1106.88 | 9.49 | 28.47 | 2480 | C₇₀H₁₂₉N₃O₉S₄ | 1285.08 | 7.88 | 28.40 |
| 2281 | C₇₀H₁₃₂N₄O₃S₂ | 1141.98 | 9.64 | 24.97 | 2481 | C₇₁H₁₄₆NO₁₃P₃S | 1346.93 | 9.92 | 26.28 |
| 2282 | C₇₀H₁₂₆N₄O₅S₃ | 1200.00 | 8.18 | 25.90 | 2482 | C₆₅H₁₃₁NO₇S₅ | 1199.10 | 9.82 | 22.46 |
| 2283 | C₇₆H₁₃₃N₃O₈PS ₃ | 1349.13 | 8.02 | 27.79 | 2483 | C₆₂H₁₂₈N₄O₇S₅ | 1202.06 | 9.66 | 24.80 |
| 2284 | C₇₀H₁₂₅N₃O₂S₄ | 1169.06 | 8.09 | 24.80 | 2484 | C₆₇H₁₃₆N₂O₂S₃ | 1098.04 | 9.60 | 31.20 |
| 2285 | C₆₉H₁₃₀N₄O₅S | 1127.89 | 8.62 | 22.64 | 2485 | C₆₇H₁₃₄N₄O₂S₆ | 1220.24 | 8.81 | 29.65 |
| 2286 | C₆₆H₁₂₉N₇O₄S | 1116.87 | 8.42 | 23.80 | 2486 | C₅₀H₉₆N₄O₅ | 833.34 | 7.85 | 14.83 |
| 2287 | C₆₉H₁₃₁N₅O₈S | 1190.90 | 8.72 | 26.48 | 2487 | C₄₉H₁₀₁N₈O₈P | 961.37 | 7.10 | 14.90 |
| 2288 | C₆₉H₁₃₂N₄O₉S | 1193.90 | 9.26 | 26.76 | 2488 | C₄₈H₁₀₀N₄S | 765.42 | 10.1 7 | 17.18 |
| 2289 | C₆₇H₁₃₄N₄O₂S | 1059.90 | 9.61 | 25.59 | 2489 | C₅₀H₉₉N₅O₁₂S₃ | 1058.57 | 7.89 | 15.01 |
| 2290 | C₇₁H₁₃₈N₆O₈S₃ | 1300.12 | 8.97 | 23.96 | 2490 | C₅₃H₁₀₀N₆O₉ | 965.42 | 8.13 | 16.59 |
| 2291 | C₆₈H₁₃₆N₄O₁₀S ₄ | 1298.12 | 9.75 | 23.58 | 2491 | C₄₉H₉₆N₆O₇ | 881.34 | 8.08 | 16.13 |
| 2292 | C₇₁H₁₃₀N₄O₉S₂ | 1259.98 | 8.25 | 26.34 | 2492 | C₅₂H₉₆N₄O₇S₄ | 1017.63 | 8.99 | 20.64 |
| 2293 | C₇₀H₁₂₇N₃O₁₁ | 1186.80 | 8.54 | 23.99 | 2493 | C₅₀H₉₁N₃O₇S₅ | 1006.63 | 8.54 | 18.41 |
| 2294 | C₇₂H₁₃₁NO₉S₃ | 1251.04 | 7.85 | 29.49 | 2494 | C₄₆H₉₃NO₅S | 772.32 | 9.48 | 16.00 |
| 2295 | C₇₀H₁₃₆NO₆P | 1118.83 | 9.96 | 26.88 | 2495 | C₄₉H₁₀₂NO₁₀P₃S ₂ | 1022.41 | 9.45 | 15.88 |
| 2296 | C₇₅H₁₅₀NO₁₁P₃ S | 1367.01 | 10.01 | 26.33 | 2496 | C₄₄H₉₀N₂O₁₁S₅ | 983.54 | 7.61 | 14.61 |
| 2297 | C₆₈H₁₂₉N₅O₁₀S ₃ | 1273.01 | 8.02 | 23.99 | 2497 | C₅₀H₉₇N₃O₁₃S₅ | 1108.67 | 9.10 | 18.90 |
| 2298 | C₆₂H₁₂₁NO₇S₃ | 1088.85 | 8.69 | 22.15 | 2498 | C₄₆H₉₆N₄O₇S₅ | 977.63 | 9.97 | 15.88 |
| 2299 | C₇₂H₁₃₄N₂O₄S₃ | 1188.07 | 8.69 | 30.53 | 2499 | C₅₁H₉₇NO₈S₃ | 948.54 | 9.74 | 20.24 |
| 2300 | C₇₀H₁₃₀N₂O₈S₄ | 1256.08 | 9.65 | 29.21 | 2500 | C₄₈H₈₉NO₉S₃ | 920.44 | 7.12 | 19.34 |
| 2301 | C₇₀H₁₂₉NO₆S₄ | 1209.07 | 9.97 | 29.90 | 2501 | C₅₀H₉₈N₂O₅S₇ | 1031.81 | 10.0 1 | 21.72 |
| 2302 | C₇₃H₁₃₃N₃O₆S₄ | 1277.15 | 8.91 | 29.86 | 2502 | C₄₉H₈₉N₃O₅S₇ | 1024.74 | 7.38 | 20.15 |
| 2303 | C₇₀H₁₂₇N₃O₅S₇ | 1315.27 | 8.15 | 29.29 | 2503 | C₈₈H₁₇₄N₄O₁₀S | 1480.44 | 8.64 | 37.76 |
| 2304 | C₆₉H₁₃₃NO₂S₄ | 1137.10 | 10.08 | 29.80 | 2504 | C₉₁H₁₈₀NO₁₂P | 1511.41 | 9.64 | 38.49 |
| 2305 | C₇₂H₁₄₂N₂O₉S | 1212.00 | 10.03 | 27.69 | 2505 | C₈₇H₁₇₂N₂O₁₃S₃ | 1550.51 | 7.44 | 36.68 |
| 2306 | C₇₀H₁₃₅NO₂S₃ | 1119.06 | 9.94 | 28.41 | 2506 | C₈₉H₁₇₇NO₁₃S₄ | 1597.63 | 9.89 | 39.95 |
| 2307 | C₇₄H₁₄₃NS₄ | 1175.23 | 10.31 | 30.33 | 2507 | C₈₇H₁₇₉N₅O₈S₄ | 1551.66 | 9.72 | 35.89 |
| 2308 | C₇₃H₁₄₂N₆O₉S | 1280.04 | 8.87 | 27.51 | 2508 | C₉₁H₁₇₃N₃O₅S₇ | 1613.82 | 8.49 | 39.46 |
| 2309 | C₇₁H₁₄₃N₇O₂ | 1126.97 | 8.80 | 25.07 | 2509 | C₉₆H₁₇₃NO₂S₃ | 1349.52 | 8.69 | 40.39 |
| 2310 | C₆₇H₁₃₆N₆O₄ | 1089.86 | 7.38 | 25.24 | 2510 | C₈₇H₁₇₇NO₃S₄ | 1413.62 | 9.80 | 39.79 |
| 2311 | C₆₅H₁₃₆N₄O₂S | 1037.90 | 9.89 | 25.86 | 2511 | C₆₈H₁₃₈N₆OS | 1087.95 | 8.34 | 25.64 |
| 2312 | C₇₀H₁₃₃NO₆S | 1116.90 | 9.78 | 27.91 | 2512 | C₇₃H₁₄₁N₅O₇S | 1233.02 | 8.19 | 27.42 |
| 2313 | C₇₂H₁₃₉NO₈ | 1146.90 | 8.29 | 30.37 | 2513 | C₇₆H₁₄₈N₆O₁₀ | 1306.05 | 8.77 | 28.04 |
| 2314 | C₇₂H₁₃₇N₃O₈S | 1204.97 | 8.10 | 27.01 | 2514 | C₇₇H₁₄₅N₅O₁₂ | 1333.03 | 8.22 | 27.36 |
| 2315 | C₇₀H₁₃₅NO₈S₃ | 1215.05 | 8.87 | 31.23 | 2515 | C₇₂H₁₃₉NO₇S₄ | 1259.14 | 9.28 | 30.05 |
| 2316 | C₆₉H₁₃₄N₂O₉S₃ | 1232.04 | 8.56 | 29.17 | 2516 | C₇₄H₁₄₈N₃O₆P | 1206.99 | 9.19 | 27.09 |
| 2317 | C₆₇H₁₃₁N₃O₆ | 1074.80 | 8.05 | 24.99 | 2517 | C₇₃H₁₄₃N₃O₇S₅ | 1335.26 | 9.00 | 27.79 |
| 2318 | C₇₁H₁₄₁N₃O₆ | 1132.92 | 8.87 | 27.00 | 2518 | C₇₄H₁₄₄N₂O₅S₅ | 1302.27 | 8.94 | 31.06 |
| 2319 | C₆₉H₁₃₈N₄O₁₅S ₃ | 1360.08 | 8.57 | 27.02 | 2519 | C₇₄H₁₄₁NO₈S₄ | 1301.18 | 9.84 | 32.75 |
| 2320 | C₇₆H₁₅₂N₃O₁₁P ₃S₂ | 1441.12 | 8.65 | 25.02 | 2520 | C₇₅H₁₄₃N₅O₆S₆ | 1403.36 | 8.02 | 29.84 |
| 2321 | C₇₀H₁₄₅N₅O₆S₃ | 1249.16 | 10.17 | 27.30 | 2521 | C₇₅H₁₄₃NOS₆ | 1267.34 | 10.0 3 | 31.57 |
| 2322 | C₇₀H₁₃₃NO₅S₇ | 1293.30 | 9.72 | 32.57 | 2522 | C₆₉H₁₃₅N₃OS₈ | 1231.28 | 9.26 | 31.21 |
| 2323 | C₉₈H₁₉₃N₃O₅ | 1493.64 | 9.64 | 41.19 | 2523 | C₆₉H₁₃₅N₃OS₈ | 1279.34 | 8.11 | 32.33 |
| 2324 | C₉₇H₁₉₂N₂O₃ | 1434.61 | 9.81 | 43.28 | 2524 | C₉₁H₁₅₁NO₆ | 1355.21 | 9.47 | 35.21 |
| 2325 | C₁₀₀H₁₉₃N₃O₃S ₅ | 1646.00 | 9.08 | 44.26 | 2525 | C₈₉H₁₅₁NO₈S | 1395.25 | 9.10 | 33.28 |
| 2326 | C₁₀₀H₁₉₄N₂O₂S ₄ | 1584.93 | 8.96 | 42.63 | 2526 | C₉₃H₁₅₆N₆O₅ | 1438.31 | 8.90 | 31.09 |
| 2327 | C₉₇H₁₉₀N₂OS₆ | 1593.00 | 7.95 | 44.89 | 2527 | C₉₀H₁₄₉NO₈S | 1405.24 | 9.77 | 36.24 |
| 2328 | C₁₀₁H₁₉₈N₈O₆S | 1652.81 | 9.03 | 41.03 | 2528 | C₉₁H₁₅₇N₇O₄ | 1413.30 | 9.81 | 33.65 |
| 2329 | C₉₈H₁₉₃N₅O₈ | 1569.65 | 9.94 | 42.17 | 2529 | C₉₃H₁₅₈N₆O₉S | 1536.38 | 9.06 | 33.18 |
| 2330 | C₉₈H₁₉₆N₆O₂S | 1522.75 | 8.97 | 42.54 | 2530 | C₈₈H₁₅₄N₆O₂ | 1328.24 | 9.69 | 33.16 |
| 2331 | C₉₅H₁₈₇NO₈S | 1503.61 | 8.62 | 43.07 | 2531 | C₈₉H₁₅₈N₄O₇S₅ | 1556.56 | 9.19 | 36.70 |
| 2332 | C₁₀₀H₁₉₆N₄O₁₀ | 1614.69 | 8.95 | 42.32 | 2532 | C₉₁H₁₅₅N₅O₈ | 1447.27 | 9.75 | 33.92 |
| 2333 | C₉₆H₁₈₉NO₉S₂ | 1565.70 | 9.90 | 43.36 | 2533 | C₉₁H₁₅₃N₃O₁₀S | 1481.30 | 9.45 | 33.16 |
| 2334 | C₉₈H₁₈₉NO₈S₃ | 1605.79 | 7.84 | 44.98 | 2534 | C₈₈H₁₅₄N₄O₅ | 1348.22 | 9.44 | 33.71 |
| 2335 | C₁₀₄H₂₁₀N₃O₁₂ P₃ | 1787.75 | 8.95 | 40.77 | 2535 | C₈₅H₁₄₉NO₁₁S₄ | 1489.36 | 9.07 | 32.92 |
| 2336 | C₉₅H₁₉₂NO₉PS₃ | 1619.75 | 9.61 | 38.95 | 2536 | C₈₃H₁₄₅NO₂S₃ | 1285.26 | 8.63 | 34.83 |
| 2337 | C₉₆H₁₉₆N₆O₉S₃ | 1674.86 | 9.53 | 39.50 | 2537 | C₈₉H₁₅₂N₄O₂S₆ | 1502.58 | 8.69 | 33.48 |
| 2338 | C₉₆H₁₉₄N₆O₉S₄ | 1704.91 | 8.66 | 39.31 | 2538 | C₈₇H₁₅₃NO₄S₇ | 1501.60 | 9.11 | 35.87 |
| 2339 | C₉₇H₁₉₇NO₉S₅ | 1681.98 | 10.29 | 43.64 | 2539 | C₉₀H₁₅₃N₃O₂S₇ | 1533.65 | 8.67 | 35.66 |
| 2340 | C₁₀₀H₁₉₃N₅O₉S ₃ | 1705.87 | 8.02 | 43.88 | 2540 | C₉₀H₁₅₅N₃O₃S₇ | 1551.67 | 8.78 | 37.08 |
| 2341 | C₉₃H₁₈₅NO₂S₄ | 1477.78 | 8.83 | 44.69 | 2541 | C₅₀H₉₂N₄O₈ | 877.31 | 7.61 | 14.76 |
| 2342 | C₉₆H₁₉₅NS₅ | 1523.96 | 10.32 | 46.50 | 2542 | C₅₄H₁₀₁N₃O₁₀S | 984.47 | 8.61 | 17.32 |
| 2343 | C₉₆H₁₉₂N₄O₂S₇ | 1659.09 | 9.15 | 45.27 | 2543 | C₅₅H₁₀₇N₃OS₃ | 922.66 | 10.3 5 | 19.69 |
| 2344 | C₇₂H₁₃₇N₇O₆ | 1196.93 | 9.55 | 23.01 | 2544 | C₇₃H₁₃₃N₃O₇ | 1164.88 | 8.78 | 26.27 |
| 2345 | C₇₅H₁₃₆N₆O₆S | 1250.02 | 8.12 | 23.71 | 2545 | C₇₂H₁₃₉N₄O₆P | 1187.90 | 8.50 | 25.02 |
| 2346 | C₇₃H₁₃₃N₃O₃S₄ | 1229.15 | 8.60 | 25.60 | 2546 | C₇₆H₁₄₅N₉O₆ | 1281.05 | 8.95 | 25.90 |
| 2347 | C₇₂H₁₃₄N₂O₂S₆ | 1252.28 | 8.91 | 31.69 | 2547 | C₇₂H₁₃₃N₅O₁₀ | 1228.88 | 7.28 | 26.13 |
| 2348 | C₇₃H₁₃₅NO₂S₇ | 1283.36 | 10.25 | 31.89 | 2548 | C₇₅H₁₄₈N₄O₂ | 1138.04 | 10.2 5 | 29.53 |
| 2349 | C₇₄H₁₃₈N₆O₈S | 1272.02 | 8.96 | 26.06 | 2549 | C₇₂H₁₃₈N₆O₃ | 1135.93 | 8.47 | 25.31 |
| 2350 | C₇₁H₁₃₂N₆O₈ | 1197.87 | 8.73 | 24.03 | 2550 | C₇₂H₁₃₁N₃O₁₁S | 1246.91 | 7.71 | 26.16 |
| 2351 | C₇₁H₁₃₇N₅O₈S | 1220.97 | 10.21 | 27.15 | 2551 | C₇₆H₁₃₅N₃O₁₃ | 1298.92 | 8.06 | 26.23 |
| 2352 | C₇₀H₁₂₇NO₇S | 1126.85 | 8.28 | 27.40 | 2552 | C₇₃H₁₃₃N₃O₁₃S | 1292.93 | 8.53 | 24.60 |
| 2353 | C₆₆H₁₂₄N₄O₃S₂ | 1085.88 | 7.45 | 24.89 | 2553 | C₇₂H₁₃₅NO₁₃S | 1254.93 | 9.37 | 28.42 |
| 2354 | C₆₉H₁₃₃NO₃S₂ | 1088.96 | 10.01 | 27.96 | 2554 | C₇₅H₁₄₉N₂O₁₁P₃ | 1347.94 | 8.85 | 27.16 |
| 2355 | C₇₃H₁₄₁N₂O₁₃P ₃S | 1379.92 | 7.73 | 24.08 | 2555 | C₇₅H₁₄₈N₃O₁₀P₃ S | 377.00 | 9.09 | 25.62 |
| 2356 | C₇₂H₁₃₆N₄O₁₅S ₄ | 1426.16 | 8.39 | 26.10 | 2556 | C₇₂H₁₃₅N₃O₉S₄ | 1315.12 | 8.02 | 23.79 |
| 2357 | C₆₆H₁₂₇NO₈S₄ | 1191.01 | 9.56 | 23.38 | 2557 | C₆₇H₁₃₃N₅O₇S₃ | 1217.01 | 9.55 | 24.42 |
| 2358 | C₆₉H₁₃₄N₆O₈S₄ | 1304.13 | 8.63 | 23.43 | 2558 | C₆₈H₁₃₇N₇O₈S₄ | 1309.12 | 8.59 | 23.56 |
| 2359 | C₇₀H₁₃₄N₂O₁₁S ₃ | 1276.05 | 9.01 | 25.11 | 2559 | C₆₆H₁₃₀N₄O₉S₄ | 1252.03 | 7.70 | 24.69 |
| 2360 | C₇₁H₁₂₉N₃O₆S₆ | 1313.23 | 7.83 | 29.32 | 2560 | C₆₉H₁₃₁NO₁₀S₄ | 1263.04 | 9.72 | 26.82 |
| 2361 | C₇₅H₁₃₈N₃O₇PS ₆ | 1417.32 | 8.79 | 29.20 | 2561 | C₆₉H₁₃₄N₂O₁₁S₄ | 1296.07 | 9.65 | 25.47 |
| 2362 | C₇₃H₁₂₈N₆O₆S₇ | 1410.33 | 8.05 | 29.45 | 2562 | C₇₂H₁₃₇N₃O₁₄S₄ | 1397.13 | 7.96 | 24.84 |
| 2363 | C₆₉H₁₃₃N₃O₄S₃ | 1165.04 | 8.75 | 27.33 | 2563 | C₇₆H₁₃₄N₆O₅S₆ | 1404.30 | 8.44 | 29.07 |
| 2364 | C₇₀H₁₃₃N₂O₄PS ₃ | 1194.02 | 8.64 | 27.09 | 2564 | C₇₅H₁₃₈N₄O₄S₇ | 1384.37 | 7.99 | 27.97 |
| 2365 | C₆₈H₁₃₁N₅O₅ | 1098.83 | 8.96 | 24.38 | 2565 | C₇₂H₁₃₃NO₂S₇ | 1269.28 | 9.22 | 29.39 |
| 2366 | C₆₆H₁₃₀N₄O₄ | 1043.79 | 9.95 | 23.32 | 2566 | C₇₆H₁₃₉NS₈ | 1323.43 | 9.99 | 32.54 |
| 2367 | C₆₂H₁₂₄N₄O₅S | 1037.76 | 8.99 | 22.55 | 2567 | C₆₈H₁₃₂N₂OS₆ | 1186.18 | 9.61 | 29.43 |
| 2368 | C₆₇H₁₃₀N₆O₃S₂ | 1131.95 | 9.38 | 22.39 | 2568 | C₇₆H₁₄₆N₆O₈ | 1272.04 | 7.91 | 25.83 |
| 2369 | C₆₇H₁₃₁N₅O₅S₂ | 1150.95 | 8.80 | 22.94 | 2569 | C₇₄H₁₄₂N₂O₆S₃ | 1252.14 | 7.97 | 29.92 |
| 2370 | C₆₅H₁₃₀N₄O₃S₂ | 1079.91 | 10.12 | 23.75 | 2570 | C₇₆H₁₄₇N₃O₇S₄ | 1343.27 | 8.74 | 29.77 |
| 2371 | C₆₄H₁₂₁NO₅S₄ | 1112.94 | 9.72 | 26.66 | 2571 | C₆₈H₁₃₃NO₆S₄ | 1189.05 | 9.29 | 28.76 |
| 2372 | C₆₃H₁₂₇N₇O₆S | 1110.82 | 8.83 | 20.79 | 2572 | C₇₃H₁₄₅N₅O₅ | 1172.99 | 9.61 | 27.70 |
| 2373 | C₆₃H₁₂₆N₆OS₂ | 1047.88 | 8.76 | 23.90 | 2573 | C₇₁H₁₃₉N₅O₁₀S | 1254.98 | 7.51 | 27.38 |
| 2374 | C₆₁H₁₁₉NO₁₄S | 1122.68 | 7.69 | 23.72 | 2574 | C₆₉H₁₄₂N₄O₂ | 1059.92 | 10.0 5 | 27.68 |
| 2375 | C₆₆H₁₂₈N₃O₁₃P | 1202.73 | 7.46 | 24.54 | 2575 | C₇₁H₁₄₄N₆O₃S | 1162.03 | 8.83 | 25.65 |
| 2376 | C₇₃H₁₄₅N₄O₁₁P ₃ | 1347.90 | 8.61 | 22.75 | 2576 | C₇₁H₁₄₈N₄O₁₀S₄ | 1346.22 | 9.78 | 25.99 |
| 2377 | C₇₂H₁₅₀NO₁₃P₃ S | 1362.97 | 10.23 | 26.58 | 2577 | C₇₅H₁₄₁N₃O₉S₂ | 1293.09 | 9.29 | 29.12 |
| 2378 | C₅₈H₁₂₁N₅O₈S₃ | 1112.83 | 7.95 | 21.32 | 2578 | C₇₅H₁₄₆N₃O₁₀PS ₃ | 1377.16 | 8.69 | 28.51 |
| 2379 | C₆₁H₁₂₈N₄O₉S₄ | 1189.98 | 9.93 | 23.20 | 2579 | C₆₉H₁₃₈N₄O₅S | 1135.95 | 7.81 | 25.35 |
| 2380 | C₇₉H₁₅₀N₆O₅S₂ | 1328.24 | 8.02 | 27.18 | 2580 | C₈₀H₁₆₁N₃O₁₃P₄ | 1497.07 | 8.34 | 25.40 |
| 2381 | C₈₀H₁₅₆N₆O₇ | 1314.16 | 8.79 | 28.87 | 2581 | C₆₈H₁₃₈N₂O₁₃S₄ | 1320.09 | 7.81 | 24.98 |
| 2382 | C₇₇H₁₅₃N₅O₉S | 1325.16 | 8.63 | 27.52 | 2582 | C₇₃H₁₅₆N₃O₁₁P₃ | 1405.04 | 8.90 | 25.51 |
| 2383 | C₇₉H₁₅₆N₄O₅S | 1274.21 | 9.99 | 30.04 | 2583 | C₇₃H₁₄₃N₉O₇S₅ | 1419.30 | 8.50 | 27.26 |
| 2384 | C₇₈H₁₄₈N₂O₃S₇ | 1386.52 | 8.53 | 34.02 | 2584 | C₇₅H₁₄₆N₄O₄S₃ | 1264.20 | 9.75 | 28.36 |
| 2385 | C₇₇H₁₄₈N₂O₅S₇ | 1406.51 | 8.37 | 33.09 | 2585 | C₇₆H₁₄₁N₇O₅S₃ | 1329.19 | 8.44 | 6.51 |
| 2386 | C₇₈H₁₄₉N₃S₇ | 1353.54 | 9.35 | 33.71 | 2586 | C₇₃H₁₄₃N₃S₈ | 1319.45 | 9.73 | 31.55 |
| 2387 | C₈₀H₁₅₅N₇O₅ | 1295.16 | 9.05 | 28.08 | 2587 | C₆₈H₁₃₇NO₂S₇ | 1225.27 | 9.56 | 32.84 |
| 2388 | C₇₇H₁₅₅N₇O₃S₂ | 1291.27 | 9.72 | 30.97 | 2588 | C₁₀₀H₁₉₂N₂O₄S | 1518.71 | 9.01 | 41.94 |
| 2389 | C₇₇H₁₅₃N₆O₂S | 1269.20 | 8.46 | 28.74 | 2589 | C₉₈H₁₈₉NOS₅ | 1557.90 | 7.61 | 43.84 |
| 2390 | C₇₆H₁₅₆N₄O₇S₃ | 1334.31 | 8.94 | 31.72 | 2590 | C₉₈H₁₉₄NO₃PS₃ | 1561.79 | 8.48 | 42.90 |
| 2391 | C₇₆H₁₅₄N₆O₈S₃ | 1376.30 | 8.64 | 28.57 | 2591 | C₉₉H₁₉₄N₄O₇S | 1584.72 | 9.65 | 43.10 |
| 2392 | C₇₈H₁₄₆N₂O₈ | 1240.03 | 8.71 | 30.11 | 2592 | C₉₉H₁₉₄N₄O₈S₂ | 1632.78 | 7.76 | 43.54 |
| 2393 | C₇₆H₁₄₈NO₉P | 1250.99 | 8.86 | 31.56 | 2593 | C₉₆H₁₉₆N₄O₃S | 1486.71 | 9.59 | 42.11 |
| 2394 | C₇₈H₁₅₀N₆O₉S | 1348.16 | 7.69 | 29.53 | 2594 | C₉₇H₁₉₅N₅O₄S | 1527.72 | 8.65 | 40.06 |
| 2395 | C₇₇H₁₅₀N₄O₇S | 1276.13 | 9.43 | 30.93 | 2595 | C₁₀₂H₁₉₉N₅O₉ | 1639.74 | 8.75 | 41.94 |
| 2396 | C₇₇H₁₄₇NO₇S₄ | 1327.29 | 9.86 | 34.36 | 2596 | C₉₈H₁₉₇N₄O₁₀P | 1622.65 | 8.51 | 41.00 |
| 2397 | C₇₅H₁₄₉NO₁₁S₄ | 1369.28 | 9.91 | 31.06 | 2597 | C₉₉H₁₉₅NO₆S₄ | 1623.89 | 10.0 9 | 47.23 |
| 2398 | C₇₉H₁₆₂NO₁₁P₃ S | 1427.15 | 9.02 | 30.52 | 2598 | C₁₀₅H₂₁₄NO₁₀P₃ | 1743.79 | 10.1 9 | 43.28 |
| 2399 | C₇₆H₁₅₇N₆O₁₀P S₃ | 1442.30 | 8.06 | 28.96 | 2599 | C₉₈H₁₉₃N₃O₁₂S₄ | 1733.87 | 8.00 | 41.24 |
| 2400 | C₇₅H₁₄₉N₃O₁₀S ₅ | 1413.36 | 8.76 | 28.82 | 2600 | C₆₃H₁₀₇NO₆S | 1006.62 | 9.80 | 21.64 |
| 2601 | C₆₅H₁₁₂N₃O₄PS ₆ | 1223.00 | 7.95 | 21.13 | 2801 | C₉₉H₂₀₀N₆O₁₀S₄ | 1762.96 | 8.40 | 40.16 |
| 2602 | C₆₆H₁₁₉N₇O₅ | 1090.72 | 10.00 | 21.99 | 2802 | C₁₀₀H₁₉₉N₃O₁₁S ₃ | 1715.88 | 8.98 | 40.36 |
| 2603 | C₆₁H₁₁₄N₄O₇S₅ | 1175.94 | 9.86 | 19.74 | 2803 | C₉₈H₂₀₁N₄O₁₂PS ₃ | 1754.86 | 9.49 | 39.38 |
| 2604 | C₆₁H₁₁₂N₆O₁₁S ₄ | 1233.87 | 7.61 | 17.67 | 2804 | C₉₉H₁₉₅N₃OS₉ | 1732.21 | 8.95 | 48.29 |
| 2605 | C₅₈H₁₁₀N₄O₉S₄ | 1135.80 | 7.67 | 19.80 | 2805 | C₇₇H₁₂₁N₃O₈S | 1248.89 | 8.03 | 26.31 |
| 2606 | C₆₆H₁₁₆N₆O₈S | 1153.75 | 8.85 | 21.05 | 2806 | C₇₂H₁₁₇NO₃S₅ | 1205.03 | 10.0 7 | 28.49 |
| 2607 | C₆₂H₁₀₆N₂O₁₁ | 1055.53 | 9.60 | 20.49 | 2807 | C₇₄H₁₁₇N₃OS₄ | 1193.01 | 8.45 | 25.19 |
| 2608 | C₆₃H₁₀₉NO₈S₃ | 1104.76 | 9.87 | 23.85 | 2808 | C₇₅H₁₂₁NO₃S₇ | 1309.22 | 9.65 | 27.97 |
| 2609 | C₆₃H₁₀₈N₂O₁₀S ₄ | 1181.83 | 8.42 | 23.27 | 2809 | C₇₉H₁₃₄N₉O₆PS | 1369.03 | 8.95 | 25.87 |
| 2610 | C₆₅H₁₀₉N₃O₇S₅ | 1204.94 | 8.71 | 22.79 | 2810 | C₇₉H₁₃₀N₆O₉ | 1307.94 | 8.95 | 26.58 |
| 2611 | C₆₆H₁₂₂NO₁₀P₃ S | 1214.69 | 9.74 | 20.96 | 2811 | C₇₆H₁₃₅N₄O₄P | 1199.91 | 10.0 1 | 25.75 |
| 2612 | C₆₃H₁₁₁N₃O₉S₅ | 1214.93 | 9.29 | 21.07 | 2812 | C₈₁H₁₂₆N₆O₉ | 1327.93 | 8.71 | 26.39 |
| 2613 | C₆₃H₁₁₃N₃O₁₁S ₄ | 1216.87 | 8.22 | 20.49 | 2813 | C₇₇H₁₂₆N₄O₆S₂ | 1268.00 | 9.98 | 27.83 |
| 2614 | C₆₅H₁₂₂NO₁₁P₃ S | 1218.68 | 8.62 | 19.71 | 2814 | C₇₅H₁₂₄N₄O₈ | 1209.84 | 8.73 | 27.26 |
| 2615 | C₆₀H₁₁₂NO₉PS₃ | 1118.73 | 10.00 | 17.50 | 2815 | C₇₁H₁₁₉NO₆ | 1082.73 | 8.65 | 26.27 |
| 2616 | C₆₃H₁₀₈N₆O₁₁S ₄ | 1253.86 | 8.07 | 20.48 | 2816 | C₈₁H₁₃₈N₃O₁₂P₃ S | 1470.99 | 8.02 | 25.79 |
| 2617 | C₆₃H₁₁₃N₂O₅PS ₃ | 1105.78 | 7.69 | 21.48 | 2817 | C₇₈H₁₃₅N₄O₁₁P₃ S | 1429.94 | 8.22 | 25.01 |
| 2618 | C₆₁H₁₀₉N₃O₂S₄ | 1044.83 | 8.31 | 22.42 | 2818 | C₇₄H₁₂₈NO₁₁P₃S | 1332.82 | 8.72 | 26.25 |
| 2619 | C₆₀H₁₀₅N₃O₄S₆ | 1124.92 | 7.15 | 23.05 | 2819 | C₇₁H₁₂₁NO₁₆S₃ | 1244.93 | 9.53 | 26.84 |
| 2620 | C₅₆H₁₀₄N₂O₅S | 917.52 | 9.66 | 18.46 | 2820 | C₇₆H₁₃₀NO₁₂PS₃ | 1377.02 | 9.99 | 26.39 |
| 2621 | C₅₇H₁₀₀N₂O₁₀ | 973.43 | 7.44 | 19.35 | 2821 | C₆₉H₁₁₇NO₁₆S₄ | 1344.93 | 8.21 | 27.40 |
| 2622 | C₅₇H₁₀₁NOS₄ | 944.71 | 8.76 | 21.30 | 2822 | C₇₆H₁₃₀N₄O₈S₃ | 1324.08 | 10.01 | 27.21 |
| 2623 | C₆₁H₁₀₉N₃O₃S₆ | 1124.96 | 8.89 | 21.53 | 2823 | C₇₁H₁₂₁N₇O₈S₅ | 1361.09 | 7.65 | 25.06 |
| 2624 | C₇₂H₁₃₁N₃O₄S | 1134.92 | 8.90 | 25.07 | 2824 | C₇₄H₁₂₀N₂O₅S₃ | 1213.96 | 9.95 | 27.23 |
| 2625 | C₇₃H₁₃₇N₇O₆ | 1208.94 | 8.96 | 23.56 | 2825 | C₇₇H₁₂₀N₆O₇S₄ | 1370.08 | 7.80 | 26.86 |
| 2626 | C₇₂H₁₂₇N₇O₉ | 1234.85 | 8.10 | 24.19 | 2826 | C₇₃H₁₂₀N₂O₅S₄ | 1234.01 | 9.58 | 27.28 |
| 2627 | C₇₃H₁₃₅N₃O₉S | 1230.96 | 9.09 | 25.63 | 2827 | C₇₆H₁₂₄N₃O₁₀PS ₃ | 1366.99 | 7.57 | 26.82 |
| 2628 | C₆₈H₁₂₇NO₃S₅ | 1167.10 | 9.70 | 29.88 | 2828 | C₇₂H₁₂₃NO₃S₇ | 1275.20 | 9.67 | 28.90 |
| 2629 | C₆₈H₁₂₇N₃S₇ | 1211.25 | 9.60 | 27.84 | 2829 | C₇₄H₁₂₃NO₂S₇ | 1283.22 | 9.86 | 30.94 |
| 2630 | C₇₁H₁₃₄NO₁₀P | 1192.82 | 9.64 | 24.22 | 2830 | C₇₂H₁₄₂N₂O₃ | 1083.94 | 9.36 | 28.29 |
| 2631 | C₆₈H₁₂₈N₄O₆S₂ | 1161.93 | 10.07 | 26.57 | 2831 | C₆₉H₁₃₄N₂O₅S | 1103.90 | 9.68 | 26.54 |
| 2632 | C₇₀H₁₃₀N₂O₈S₃ | 1224.02 | 9.71 | 26.56 | 2832 | C₇₁H₁₃₆N₂O₇S₂ | 1194.00 | 8.99 | 28.63 |
| 2633 | C₇₂H₁₃₁N₃O₉S₃ | 1279.05 | 8.83 | 27.55 | 2833 | C₆₅H₁₂₇NO₆S₂ | 1082.85 | 9.93 | 28.44 |
| 2634 | C₆₈H₁₂₅NO₇S₅ | 1229.08 | 9.96 | 29.51 | 2834 | C₇₂H₁₃₃N₃O₆S₃ | 1233.05 | 7.96 | 27.03 |
| 2635 | C₆₉H₁₃₀N₄O₄ | 1079.82 | 8.27 | 25.32 | 2835 | C₇₃H₁₃₉N₃O₆S₃ | 1251.11 | 8.84 | 28.79 |
| 2636 | C₆₈H₁₃₂N₂O₁₄S ₃ | 1298.00 | 10.19 | 27.01 | 2836 | C₇₃H₁₃₉NO₅S₄ | 1239.16 | 10.1 3 | 31.72 |
| 2637 | C₇₂H₁₄₀N₃O₁₂P ₃ | 1332.84 | 7.62 | 23.57 | 2837 | C₆₉H₁₃₁NOS₅ | 1151.11 | 9.73 | 29.40 |
| 2638 | C₇₁H₁₃₅N₇O₉S₃ | 1327.10 | 8.42 | 23.39 | 2838 | C₆₉H₁₃₆N₂S₆ | 1186.22 | 10.2 0 | 29.92 |
| 2639 | C₆₉H₁₃₁N₃O₁₂S ₄ | 1323.08 | 8.78 | 25.51 | 2839 | C₆₈H₁₃₉N₉O₃S₂ | 1195.04 | 9.53 | 26.63 |
| 2640 | C₇₁H₁₂₇N₃O₇S₄ | 1263.08 | 7.70 | 27.37 | 2840 | C₇₀H₁₃₄N₆O₇S | 1203.94 | 8.24 | 27.19 |
| 2641 | C₇₂H₁₃₀N₄O₉S₃ | 1292.05 | 8.32 | 28.21 | 2841 | C₇₀H₁₃₃N₅O₉S | 1220.92 | 8.39 | 26.98 |
| 2642 | C₆₉H₁₂₉N₃O₆S₇ | 1321.27 | 9.35 | 27.16 | 2842 | C₆₉H₁₄₂N₆O₇S₃ | 1264.11 | 9.97 | 26.55 |
| 2643 | C₇₀H₁₂₉NS₈ | 1241.29 | 10.16 | 30.09 | 2843 | C₆₅H₁₃₇N₅O₉S₄ | 1261.08 | 8.86 | 24.06 |
| 2644 | C₅₈H₁₀₈N₄O₈S | 1021.59 | 10.16 | 30.09 | 2844 | C₇₁H₁₃₇NO₉ | 1148.88 | 10.0 5 | 28.77 |
| 2645 | C₅₆H₁₁₁N₆O₄P | 963.52 | 7.19 | 17.58 | 2845 | C₆₉H₁₃₇N₃O₆ | 1104.87 | 8.83 | 26.42 |
| 2646 | C₅₅H₁₀₆N₄O₆S₅ | 1079.81 | 9.87 | 19.57 | 2846 | C₇₂H₁₄₄N₃O₁₀P₃ | 1304.88 | 7.67 | 25.50 |
| 2647 | C₅₆H₁₀₇N₇O₈S₃ | 1102.71 | 8.21 | 17.61 | 2847 | C₇₁H₁₄₉N₂O₁₁P₃ S | 1331.96 | 8.90 | 26.60 |
| 2648 | C₅₄H₁₀₂N₆O₈S₄ | 1091.71 | 7.81 | 16.98 | 2848 | C₆₆H₁₃₄NO₁₂PS₃ | 1260.95 | 9.07 | 25.89 |
| 2649 | C₅₈H₁₀₇N₇O₈S | 1062.60 | 8.83 | 17.19 | 2849 | C₆₃H₁₃₂N₄O₁₀S₄ | 1234.01 | 8.10 | 24.36 |
| 2650 | C₅₄H₁₀₄N₂O₅S | 893.50 | 9.68 | 18.78 | 2850 | C₇₂H₃₇NO₃S₅ | 1225.19 | 9.90 | 31.49 |
| 2651 | C₆₄H₁₂₄N₃O₁₄P₃S | 1284.69 | 8.20 | 17.97 | 2851 | C₇₀H₁₃₅NO₃S₅ | 1199.15 | 9.97 | 30.96 |
| 2652 | C₅₅H₁₀₃N₃O₁₀S ₅ | 1126.77 | 8.47 | 18.72 | 2852 | C₇₁H₁₃₅N₃OS₆ | 1239.24 | 9.56 | 28.40 |
| 2653 | C₅₅H₁₀₀N₆O₁₃S ₄ | 1181.70 | 8.28 | 18.03 | 2853 | C₅₃H₁₀₀N₆O₅ | 901.42 | 8.32 | 14.69 |
| 2654 | C₅₅H₁₀₆N₄O₇S₄ | 1063.74 | 9.80 | 19.25 | 2854 | C₅₀H₁₀₃N₁₁O₅ | 938.45 | 8.48 | 15.09 |
| 2655 | C₅₉H₁₀₄N₄O₄S₃ | 1029.71 | 9.13 | 19.59 | 2855 | C₅₂H₁₀₀N₆O₉S | 985.46 | 8.31 | 17.51 |
| 2656 | C₅₆H₁₀₃NO₇S₄ | 1030.71 | 9.87 | 21.24 | 2856 | C₅₃H₁₀₁N₃O₇ | 892.40 | 9.95 | 18.39 |
| 2657 | C₅₇H₁₀₄N₂OS₆ | 1025.87 | 10.01 | 20.81 | 2857 | C₅₁H₉₇NO₁₂S | 948.39 | 9.30 | 17.75 |
| 2658 | C₇₂H₁₂₈N₂OS₃ | 1134.03 | 10.16 | 24.30 | 2858 | C₅₀H₉₅NO₁₀S₄ | 998.55 | 8.15 | 20.65 |
| 2659 | C₆₉H₁₂₈N₄OS | 1061.88 | 10.02 | 24.28 | 2859 | C₅₄H₁₁₂NO₁₁P₃S | 1076.47 | 9.21 | 18.64 |
| 2660 | C₇₀H₁₂₉N₅O₇S₅ | 1313.16 | 8.99 | 22.78 | 2860 | C₅₃H₁₀₀N₆O₁₀S₄ | 1109.65 | 8.67 | 15.63 |
| 2661 | C₇₂H₁₃₃N₄O₉P | 1229.85 | 9.96 | 23.06 | 2861 | C₅₀H₁₀₁NO₁₄S₄ | 1068.59 | 10.0 7 | 20.13 |
| 2662 | C₆₈H₁₂₀N₂O₁₁ | 1141.71 | 9.11 | 21.06 | 2862 | C₅₁H₁₀₂N₂O₁₄S₅ | 1127.68 | 8.80 | 20.04 |
| 2663 | C₇₂H₁₂₅N₃O₉S₃ | 1273.00 | 9.10 | 26.58 | 2863 | C₅₁H₁₀₀N₆O₉S₄ | 1069.63 | 8.09 | 16.22 |
| 2664 | C₇₂H₁₂₅NO₃S₃ | 1228.99 | 10.00 | 27.22 | 2864 | C₄₆H₉₆N₄O₆S₅ | 961.60 | 9.69 | 17.23 |
| 2665 | C₇₅H₁₄₁N₂O₁₄P ₃S | 1419.94 | 8.57 | 22.34 | 2865 | C₅₂H₁₀₂N₄O₁₂S₃ | 1071.58 | 8.63 | 16.45 |
| 2666 | C₆₇H₁₂₁N₃O₁₁S ₄ | 1272.95 | 7.36 | 19.18 | 2866 | C₄₇H₉₁NO₃S₅ | 878.55 | 9.65 | 20.22 |
| 2667 | C₆₉H₁₂₆N₆O₇S₄ | 1280.04 | 9.00 | 22.36 | 2867 | C₅₁H₉₆N₄OS₆ | 973.72 | 9.50 | 17.13 |
| 2668 | C₆₉H₁₃₀N₄O₆S₅ | 1272.12 | 10.21 | 24.79 | 2868 | C₄₉H₉₉NO₃S₃ | 846.51 | 9.67 | 20.47 |
| 2669 | C₆₇H₁₁₇NO₆S₄ | 1160.91 | 8.83 | 24.72 | 2869 | C₄₈H₉₃N₃OS₇ | 952.71 | 7.90 | 20.75 |
| 2670 | C₇₃H₁₂₉N₃O₄S₅ | 1273.15 | 8.98 | 26.99 | 2870 | C₆₈H₁₃₂N₂O₆S₃ | 1169.99 | 8.20 | 27.11 |
| 2671 | C₆₇H₁₁₉NO₇S₇ | 1275.11 | 7.43 | 24.74 | 2871 | C₆₉H₁₃₁N₅O₇S₄ | 1271.07 | 8.05 | 28.00 |
| 2672 | C₇₀H₁₂₁N₃O₄S₇ | 1293.17 | 8.47 | 24.89 | 2872 | C₇₄H₁₄₇N₄O₁₃P₃ S | 1425.99 | 8.26 | 21.77 |
| 2673 | C₆₇H₁₁₉N₃O₃S₄ | 1142.95 | 7.75 | 24.17 | 2873 | C₇₈H₁₅₅NO₁₃P₄S | 1471.05 | 9.23 | 26.84 |
| 2674 | C₆₆H₁₁₉NO₂S₇ | 1183.10 | 9.76 | 27.41 | 2874 | C₇₄H₁₄₇N₂O₁₂P₃ S | 1381.97 | 7.99 | 25.04 |
| 2675 | C₇₄H₁₄₂N₄O₉S | 1264.03 | 8.40 | 26.85 | 2875 | C₈₀H₁₄₁N₇O₁₂S | 1425.10 | 8.28 | 27.74 |
| 2676 | C₇₂H₁₃₅N₃O₇S | 1186.95 | 7.50 | 28.39 | 2876 | C₇₅H₁₃₇N₅O₁₂S | 1333.00 | 7.02 | 25.80 |
| 2677 | C₇₄H₁₃₈N₄O₂S₃ | 1212.12 | 7.44 | 26.73 | 2877 | C₇₅H₁₄₆N₅O₈PS | 1309.05 | 9.53 | 24.72 |
| 2678 | C₇₅H₁₃₉N₃O₄S₄ | 1275.19 | 7.91 | 28.49 | 2878 | C₅₂H₁₀₁NO₁₁S₂ | 980.50 | 7.66 | 18.55 |
| 2679 | C₇₂H₁₃₆N₂O₂S₆ | 1254.25 | 8.75 | 29.44 | 2879 | C₆₈H₁₁₅NO₇S₄ | 1186.91 | 9.71 | 24.43 |
| 2680 | C₉₁H₁₇₇NO₄S | 1381.48 | 9.69 | 39.41 | 2880 | C₆₅H₁₁₃NO₁₀S₅ | 1228.92 | 7.12 | 22.76 |
| 2681 | C₈₉H₁₇₂N₂O₇ | 1382.36 | 9.42 | 37.27 | 2881 | C₆₅H₁₂₂NO₁₂P₃S | 1234.67 | 7.76 | 19.16 |
| 2682 | C₉₁H₁₇₆N₆O₇ | 1466.44 | 8.11 | 36.76 | 2882 | C₆₈H₁₁₅N₃O₁₂S | 1198.74 | 8.37 | 22.77 |
| 2683 | C₉₂H₁₇₈N₃O₁₀P | 1517.42 | 8.01 | 36.76 | 2883 | C₇₅H₁₃₀N₃O₆PS₄ | 1329.09 | 8.51 | 25.28 |
| 2684 | C₉₁H₁₇₇N₃O₇S₄ | 1553.67 | 8.56 | 37.79 | 2884 | C₇₆H₁₃₆N₂O₁₀S₅ | 1398.23 | 9.74 | 30.25 |
| 2685 | C₉₃H₁₇₉N₃O₃S₃ | 1483.65 | 8.65 | 37.45 | 2885 | C₇₀H₁₃₀NO₁₂PS₅ | 1369.08 | 9.35 | 22.77 |
| 2686 | C₉₀H₁₇₄N₄O₂S₆ | 1536.77 | 9.13 | 38.77 | 2886 | C₆₈H₁₂₇N₃O₈S₅ | 1275.08 | 7.49 | 18.47 |
| 2687 | C₈₉H₁₇₆N₄O₄ | 1366.41 | 9.65 | 36.39 | 2887 | C₅₆H₁₀₆N₄O₅S₂ | 979.61 | 8.60 | 19.07 |
| 2688 | C₈₈H₁₇₆N₄O₇S | 1434.46 | 7.44 | 34.46 | 2888 | C₅₇H₁₀₄N₄O₇S₄ | 1085.72 | 8.19 | 20.14 |
| 2689 | C₉₀H₁₈₀N₁₀O₃S | 1482.56 | 9.39 | 37.26 | 2889 | C₆₁H₁₀₆N₄O₈S₃ | 1119.72 | 7.67 | 21.03 |
| 2690 | C₉₃H₁₈₅N₉O₆S | 1557.62 | 8.83 | 35.92 | 2890 | C₅₉H₁₁₁N₃O₁₄S₆ | 1278.90 | 8.13 | 18.51 |
| 2691 | C₉₁H₁₅₀N₄O₈ | 1458.46 | 9.68 | 39.20 | 2891 | C₅₇H₁₀₇N₃O₁₂S₅ | 1186.79 | 8.93 | 18.22 |
| 2692 | C₈₄H₁₇₄N₄O₁₀S ₄ | 1528.57 | 9.73 | 35.64 | 2892 | C₅₈H₁₀₈N₂O₄S₆ | 1089.87 | 9.71 | 23.62 |
| 2693 | C₉₁H₁₇₇NO₈ | 1413.42 | 9.74 | 39.59 | 2893 | C₇₅H₁₃₃N₃O₁₂S₃ | 1365.08 | 7.62 | 28.16 |
| 2694 | C₉₁H₁₇₇NO₈ | 1413.42 | 9.63 | 39.59 | 2894 | C₇₃H₁₄₄N₅O₇PS | 1267.02 | 8.77 | 23.15 |
| 2695 | C₉₄H₁₉₁NO₁₄S₄ | 1687.80 | 8.92 | 40.68 | 2895 | C₇₀H₁₃₅NO₃S₈ | 1295.33 | 9.28 | 29.89 |
| 2696 | C₉₈H₂₀₀N₄O₉S₃ | 1674.88 | 10.05 | 42.36 | 2896 | C₇₁H₁₄₅N₃OS₄ | 1185.20 | 10.1 3 | 32.12 |
| 2697 | C₁₀₁H₁₉₄N₂O₆S ₄ | 1660.91 | 8.50 | 44.51 | 2897 | C₆₉H₁₃₈NO₁₃PS₅ | 1381.13 | 8.17 | 26.27 |
| 2698 | C₉₅H₁₈₇NO₂S₇ | 1599.97 | 7.87 | 43.73 | 2898 | C₇₆H₁₂₈N₄O₇S | 1241.94 | 9.76 | 26.18 |
| 2699 | C₉₆H₁₉₄N₂O₃S₇ | 1649.04 | 8.91 | 43.81 | 2899 | C₇₇H₁₂₈N₄O₁₀S₃ | 1366.07 | 9.13 | 28.18 |
| 2700 | C₇₃H₁₃₅N₃O₇S | 1198.96 | 8.83 | 27.72 | 2900 | C₇₈H₁₂₉N₇O₁₁S₄ | 1469.17 | 7.08 | 24.13 |
| 2701 | C₇₃H₁₃₂N₄O₂S₃ | 1194.07 | 8.26 | 26.95 | 2901 | C₇₆H₁₃₀N₆O₁₀S₆ | 1480.27 | 8.32 | 26.25 |
| 2702 | C₇₄H₁₄₃N₇O₆ | 1227.00 | 10.05 | 27.15 | 2902 | C₇₉H₁₂₈N₆O₁₂S₄ | 1482.16 | 8.21 | 26.97 |
| 2703 | C₇₂H₁₃₈N₄O₈S | 1219.98 | 9.16 | 26.82 | 2903 | C₇₈H₁₃₅N₅O₄ | 1206.97 | 9.64 | 29.41 |
| 2704 | C₇₀H₁₃₃N₅OS₂ | 1124.99 | 8.67 | 26.08 | 2904 | C₈₀H₁₂₉N₇O₇ | 1300.95 | 8.42 | 24.11 |
| 2705 | C₇₀H₁₃₀N₄O₆S | 1155.89 | 9.03 | 26.72 | 2905 | C₉₂H₁₅₆N₂O₅S₅ | 1530.57 | 8.87 | 38.21 |
| 2706 | C₇₁H₁₃₃N₅O₉S | 1232.93 | 7.56 | 25.84 | 2906 | C₉₅H₁₆₀N₃O₁₂PS ₃ | 1663.49 | 7.57 | 35.94 |
| 2707 | C₇₂H₁₃₄N₄O₁₀S | 1247.94 | 9.32 | 26.54 | 2907 | C₉₁H₁₅₇NO₁₇S₅ | 1697.55 | 9.77 | 37.07 |
| 2708 | C₇₂H₁₃₂N₂O₁₂S | 1249.91 | 8.50 | 26.64 | 2908 | C₉₂H₁₅₉NOS₉ | 1583.83 | 9.44 | 41.50 |
| 2709 | C₆₈H₁₃₁NO₁₅S₃ | 1298.97 | 8.73 | 27.84 | 2909 | C₈₀H₁₅₇N₃O₁₁S₂ | 1401.27 | 7.36 | 30.07 |
| 2710 | C₇₀H₁₃₃N₃O₁₅S ₄ | 1385.08 | 8.44 | 27.06 | 2910 | C₈₀H₁₆₁N₇O₉S | 1397.27 | 9.05 | 31.05 |
| 2711 | C₈₀H₁₅₇N₃O₁₄P ₄ | 1509.04 | 8.07 | 24.70 | 2911 | C₈₆H₁₇₂N₃O₁₄P₃ S₂ | 1629.37 | 7.98 | 30.43 |
| 2712 | C₆₉H₁₂₉N₃O₉S₄ | 1273.04 | 8.14 | 23.02 | 2912 | C₈₂H₁₅₉NO₈S | 1319.23 | 9.17 | 32.07 |
| 2713 | C₇₁H₁₃₉N₃O₁₀S ₃ | 1291.08 | 10.20 | 26.84 | 2913 | C₈₂H₁₅₇NO₉S₂ | 1365.28 | 8.94 | 34.05 |
| 2714 | C₇₁H₁₃₀N₆O₉S₅ | 1372.15 | 7.66 | 25.94 | 2914 | C₈₂H₁₅₅NO₁₀S₃ | 1411.32 | 9.46 | 33.89 |
| 2715 | C₇₁H₁₃₁NO₄S₃ | 1159.01 | 9.05 | 28.83 | 2915 | C₈₂H₁₅₆N₆O₈ | 1354.18 | 7.58 | 27.67 |
| 2716 | C₇₄H₁₃₆N₄O₅S₃ | 1258.11 | 9.28 | 26.98 | 2916 | C₁₀₂H₂₀₀N₄O₆S | 1610.80 | 9.30 | 41.11 |
| 2717 | C₇₄H₁₄₀NO₆PS₆ | 1363.27 | 10.18 | 30.16 | 2917 | C₉₉H₁₉₉N₅O₁₂S₃ | 1747.88 | 8.74 | 39.52 |
| 2718 | C₇₂H₁₄₁NO₃S₄ | 1197.16 | 10.22 | 30.35 | 2918 | C₉₈H₂₀₁N₅O₁₂S₅ | 1802.01 | 7.78 | 38.29 |
| 2719 | C₇₄H₁₂₀N₂O₃S₆ | 1298.30 | 9.07 | 30.62 | 2919 | C₁₀₄H₂₀₂N₃O₁₃P S₂ | 1797.86 | 8.16 | 42.89 |
| 2720 | C₆₈H₁₃₃N₃O₅S | 1104.89 | 9.56 | 23.55 | 2920 | C₇₂H₁₄₃N₅O₆ | 1174.96 | 7.02 | 24.11 |
| 2721 | C₆₈H₁₃₁NO₂S₃ | 1090.98 | 10.20 | 27.08 | 2921 | C₇₅H₁₄₃NOS₄ | 1203.22 | 9.90 | 30.72 |
| 2722 | C₆₆H₁₂₇N₅O₇ | 1102.77 | 7.40 | 22.13 | 2922 | C₇₆H₁₄₆N₄O₈S₅ | 1404.32 | 7.57 | 29.76 |
| 2723 | C₆₇H₁₃₄N₆O₄S | 1119.91 | 8.03 | 22.12 | 2923 | C₇₆H₁₄₆N₄O₉S₃ | 1356.20 | 8.27 | 30.39 |
| 2724 | C₆₅H₁₃₅N₅O₉S₃ | 1227.00 | 9.81 | 23.41 | 2924 | C₇₈H₁₆₁N₂O₁₁P₃ S | 1428.13 | 9.86 | 28.23 |
| 2725 | C₆₇H₁₂₇N₃O₇ | 1086.77 | 9.14 | 23.56 | 2925 | C₇₇H₁₅₆NO₁₁P₃S ₂ | 1429.13 | 9.80 | 27.74 |
| 2726 | C₆₅H₁₂₆N₂O₁₂S | 1159.78 | 7.67 | 23.67 | 2926 | C₇₂H₁₄₆N₆O₉S₄ | 1368.23 | 7.44 | 25.66 |
| 2727 | C₆₆H₁₂₃N₃O₈S₅ | 1247.02 | 8.30 | 26.43 | 2927 | C₇₅H₁₄₇N₅O₇ | 1231.03 | 8.75 | 27.63 |
| 2728 | C₆₈H₁₄₁N₂O₁₀P ₃ | 1239.80 | 9.68 | 23.81 | 2928 | C₇₁H₁₄₃NO₅S₃ | 1187.11 | 9.63 | 29.70 |
| 2729 | C₆₃H₁₂₅N₃O₁₄S ₄ | 1276.94 | 7.77 | 24.47 | 2929 | C₇₇H₁₄₆N₂O₄S₆ | 1356.38 | 8.92 | 30.90 |
| 2730 | C₆₇H₁₃₃N₃O₇S₄ | 1221.05 | 8.93 | 22.48 | 2930 | C₇₅H₁₄₅N₃O₈S₃ | 1313.18 | 9.69 | 30.82 |
| 2731 | C₆₃H₁₂₃N₃O₃S₆ | 1163.05 | 7.51 | 26.47 | 2931 | C₇₄H₁₄₂N₆O₁₁S₅ | 1452.28 | 7.20 | 26.83 |
| 2732 | C₆₄H₁₃₁NO₃S₇ | 1187.18 | 9.65 | 28.98 | 2932 | C₁₀₇H₂₁₀N₄O₆S | 1680.94 | 9.30 | 44.75 |
| 2733 | C₆₃H₁₂₇NOS₈ | 1171.19 | 10.03 | 29.92 | 2933 | C₁₀₃H₂₀₅N₅OS₇ | 1754.23 | 7.43 | 49.14 |
| 2734 | C₈₂H₁₆₂NO₆P | 1289.17 | 10.20 | 32.78 | 2934 | C₁₀₈H₂₁₂NO₇PS | 1699.92 | 7.96 | 46.71 |
| 2735 | C₇₉H₁₅₃NO₄S₂ | 1245.22 | 9.98 | 34.17 | 2935 | C₉₉H₂₀₀N₄O₂S₃ | 1574.89 | 9.31 | 44.17 |
| 2736 | C₇₉H₁₅₁N₃O₁₀S | 1335.15 | 7.16 | 30.27 | 2936 | C₁₀₀H₁₉₉N₇O₁₁S ₄ | 1803.97 | 7.82 | 41.43 |
| 2737 | C₇₉H₁₅₁N₂O₁₀P | 1320.05 | 7.65 | 29.97 | 2937 | C₉₇H₁₉₇NO₁₉S₅ | 1841.93 | 9.29 | 43.58 |
| 2738 | C₇₉H₁₅₂N₄O₅S₃ | 1334.29 | 8.33 | 31.06 | 2938 | C₉₉H₁₉₄N₂O₈S | 1572.71 | 7.31 | 41.98 |
| 2739 | C₇₇H₁₄₉NO₄S₃ | 1249.22 | 9.44 | 32.44 | 2939 | C₉₄H₁₇₈N₄O₅S₃ | 1540.66 | 7.70 | 37.45 |
| 2740 | C₇₈H₁₅₃N₉O₃S₃ | 1361.32 | 8.05 | 31.75 | 2940 | C₇₄H₁₃₇N₃O₄S₅ | 1293.23 | 8.28 | 28.37 |
| 2741 | C₇₅H₁₅₀N₆O₉S₃ | 1376.24 | 7.11 | 28.59 | 2941 | C₇₈H₁₅₁N₂O₁₄P₃S | 1466.05 | 8.41 | 24.98 |
| 2742 | C₈₀H₁₅₄N₄O₉ | 1316.13 | 8.39 | 31.34 | 2942 | C₇₃H₁₄₁N₇O₈S₃ | 1341.15 | 9.86 | 23.70 |
| 2743 | C₇₈H₁₅₃NO₈S | 1265.14 | 9.73 | 30.75 | 2943 | C₇₆H₁₃₆N₄O₈S | 1266.00 | 7.96 | 27.68 |
| 2744 | C₇₇H₁₅₁NO₆S₂ | 1251.18 | 10.04 | 33.91 | 2944 | C₆₉H₁₃₅N₅O₈S₃ | 1259.05 | 9.93 | 26.72 |
| 2745 | C₇₈H₁₅₆N₄O₉S | 1326.18 | 9.48 | 31.83 | 2945 | C₆₉H₁₃₅NO₃S₆ | 1219.20 | 10.2 6 | 30.26 |
| 2746 | C₈₀H₁₅₂N₂O₁₀S ₄ | 1430.34 | 8.79 | 32.39 | 2946 | C₆₅H₁₂₉NO₃S₆ | 1165.11 | 10.0 3 | 29.30 |
| 2747 | C₇₉H₁₆₀N₄O₄ | 1230.17 | 9.90 | 31.06 | 2947 | C₇₀H₁₃₇N₄O₁₁PS ₄ | 1370.10 | 9.59 | 27.67 |
| 2748 | C₈₂H₁₆₈NO₁₁P₃ S | 1469.22 | 9.35 | 31.16 | 2948 | C₇₃H₁₄₉N₂O₁₃P₃ S | 1387.98 | 9.89 | 25.38 |
| 2749 | C₇₄H₁₄₉NO₉S₄ | 1325.24 | 9.52 | 31.12 | 2949 | C₆₆H₁₃₀N₆O₆S | 1135.86 | 8.58 | 21.52 |
| 2750 | C₇₆H₁₅₁N₃O₉S₃ | 1347.24 | 7.62 | 28.28 | 2950 | C₆₉H₁₃₂N₂O₈S | 1149.88 | 9.31 | 25.41 |
| 2751 | C₈₀H₁₆₃NO₉S₄ | 1411.42 | 10.02 | 31.61 | 2951 | C₆₇H₁₃₀NO₁₁P | 1156.75 | 9.20 | 24.28 |
| 2752 | C₇₇H₁₅₅N₇O₈S₃ | 1403.31 | 8.62 | 28.92 | 2952 | C₇₄H₁₄₁N₄O₇PS | 1262.00 | 10.0 7 | 25.50 |
| 2753 | C₇₇H₁₅₅NO₁₂S₄ | 1415.32 | 9.90 | 31.42 | 2953 | C₇₉H₁₃₉N₇O₂S₈ | 1475.51 | 8.66 | 30.35 |
| 2754 | C₇₉H₁₅₀N₂O₄S₄ | 1320.32 | 8.20 | 32.38 | 2954 | C₈₀H₁₄₆N₃O₇PS₆ | 1485.40 | 8.03 | 29.10 |
| 2755 | C₈₀H₁₅₂N₂O₅S₅ | 1382.40 | 8.23 | 33.60 | 2955 | C₇₂H₁₃₉NO₁₄S₅ | 1403.20 | 10.2 4 | 26.99 |
| 2756 | C₈₂H₁₅₇N₃O₉S₄ | 1457.41 | 8.95 | 34.69 | 2956 | C₆₈H₁₃₄N₂O₁₁S₅ | 1316.12 | 9.97 | 23.02 |
| 2757 | C₇₈H₁₅₃N₂O₈PS ₆ | 1470.42 | 7.17 | 32.63 | 2957 | C₇₃H₁₃₈N₄O₅S₇ | 1376.35 | 8.95 | 30.58 |
| 2758 | C₇₈H₁₄₇N₃OS₆ | 1335.41 | 8.18 | 32.47 | 2958 | C₇₆H₁₃₆N₂O₈S | 1237.99 | 8.81 | 25.65 |
| 2759 | C₇₈H₁₅₁N₇O₃S₆ | 1427.47 | 8.10 | 32.55 | 2959 | C₅₅H₁₀₅N₅O₉S | 1012.53 | 9.99 | 19.03 |
| 2760 | C₈₀H₁₅₇N₇OS₆ | 1425.54 | 9.93 | 32.94 | 2960 | C₅₅H₁₀₃N₃O₇S₈ | 1174.92 | 9.06 | 19.72 |
| 2761 | C₁₀₃H₁₉₈N₁₀O₄ | 1640.78 | 8.43 | 38.34 | 2961 | C₅₅H₉₈N₂O₇S₆ | 1091.76 | 8.02 | 19.51 |
| 2762 | C₉₉H₁₉₁NO₈S | 1555.68 | 9.65 | 43.86 | 2962 | C₅₆H₁₀₄N₂O₇S₄ | 1045.70 | 8.13 | 19.65 |
| 2763 | C₉₉H₁₉₁NO₇S₂ | 1571.74 | 9.34 | 44.32 | 2963 | C₅₅H₁₀₃N₃O₁₃S₄ | 1142.68 | 8.95 | 20.08 |
| 2764 | C₁₀₅H₂₀₅NO₅S₃ | 1657.98 | 10.13 | 47.57 | 2964 | C₄₉H₉₇NO₄S₅ | 924.62 | 7.78 | 18.16 |
| 2765 | C₁₀₀H₁₉₈NO₃PS ₃ | 1589.84 | 8.58 | 43.46 | 2965 | C₆₀H₁₁₇N₄O₁₅P₃ | 1227.53 | 8.09 | 15.71 |
| 2766 | C₁₀₂H₂₀₀N₂OS₆ | 1663.09 | 10.10 | 45.62 | 2966 | C₅₃H₁₀₅NO₁₂S₅ | 1108.72 | 10.1 3 | 18.40 |
| 2767 | C₁₀₄H₂₀₅NO₆S | 1597.85 | 7.66 | 46.47 | 2967 | C₅₅H₁₀₈N₃O₁₆PS ₅ | 1258.75 | 9.37 | 19.39 |
| 2768 | C₁₀₄H₂₀₉N₇O₃S ₂ | 1669.98 | 9.88 | 46.29 | 2968 | C₅₄H₁₀₄N₄O₁₀S | 1001.50 | 8.74 | 17.69 |
| 2769 | C₁₀₅H₂₀₈N₆O₈ | 1682.85 | 9.03 | 44.74 | 2969 | C₅₄H₁₀₆N₂O₁₀S₂ | 1007.57 | 10.0 2 | 18.69 |
| 2770 | C₁₀₄H₂₀₄N₄O₇S ₂ | 1686.92 | 9.42 | 47.15 | 2970 | C₆₆H₁₁₇N₅O₈S | 1140.75 | 9.75 | 19.41 |
| 2771 | C₁₀₉H₂₀₈N₂O₉ | 1690.87 | 8.92 | 46.22 | 2971 | C₆₈H₁₂₁N₇O₅ | 1116.76 | 8.93 | 17.67 |
| 2772 | C₁₀₅H₂₁₀N₄O₁₀ S | 1720.91 | 9.84 | 46.22 | 2972 | C₆₅H₁₁₄N₂O₅S₅ | 1163.94 | 10.0 2 | 23.50 |
| 2773 | C₁₀₅H₂₀₁N₃O₁₁ S | 1713.83 | 7.46 | 46.28 | 2973 | C₆₈H₁₁₄N₂O₉S₂ | 1167.78 | 8.32 | 21.74 |
| 2774 | C₁₀₂H₂₀₅N₃O₈S | 1633.83 | 8.04 | 44.66 | 2974 | C₇₁H₁₂₆NO₄PS₇ | 1313.19 | 10.0 9 | 25.85 |
| 2775 | C₁₀₉H₂₂₃N₂O₁₁ P₃S | 1862.97 | 9.95 | 45.08 | 2975 | C₇₄H₁₂₉N₃O₆S₉ | 1445.40 | 8.19 | 27.04 |
| 2776 | C₁₀₂H₂₀₈N₂O₉S ₃ | 1702.97 | 10.14 | 46.00 | 2976 | C₇₄H₁₂₉NO₆S₂ | 1192.97 | 10.3 5 | 24.90 |
| 2777 | C₁₀₇H₂₀₈N₄O₃S ₅ | 1759.17 | 8.34 | 49.21 | 2977 | C₇₃H₁₂₅N₃O₁₁S₂ | 1284.93 | 8.96 | 23.77 |
| 2778 | C₁₀₄H₂₀₃NO₁₀S ₄ | 1756.01 | 9.55 | 48.00 | 2978 | C₇₁H₁₂₅N₅O₁₄S₂ | 1336.92 | 8.57 | 20.70 |
| 2779 | C₁₀₅H₂₀₃NO₇S₄ | 1720.02 | 8.74 | 49.41 | 2979 | C₅₉H₁₀₈N₂O₃S₄ | 1021.76 | 10.2 0 | 20.34 |
| 2780 | C₁₀₈H₂₁₃NO₆S₇ | 1846.31 | 10.05 | 50.72 | 2980 | C₆₁H₁₁₈NO₁₄PS₄ | 1248.82 | 10.2 5 | 22.37 |
| 2781 | C₁₀₃H₂₀₃N₃O₂S ₃ | 1611.96 | 8.66 | 46.73 | 2981 | C₅₆H₁₀₆N₂O₁₁S₄ | 1111.71 | 8.96 | 17.71 |
| 2782 | C₁₀₄H₂₀₈N₂O₂S ₃ | 1615.00 | 9.88 | 47.93 | 2982 | C₅₇H₁₀₈N₂O₁₅S₃ | 1157.67 | 10.0 5 | 20.10 |
| 2783 | C₁₀₁H₂₀₄N₂OS₆ | 1655.12 | 8.86 | 49.01 | 2983 | C₆₀H₁₁₀NO₇P | 988.51 | 10.1 5 | 18.89 |
| 2784 | C₁₀₁H₂₀₅NS₈ | 1690.24 | 9.98 | 51.49 | 2984 | C₆₁H₁₀₅N₇O₅ | 1016.55 | 8.67 | 17.66 |
| 2785 | C₁₀₂H₂₀₅N₆O₆P | 1642.77 | 9.54 | 39.05 | 2985 | C₆₁H₁₁₂N₂O₉S₂ | 1081.69 | 10.1 1 | 20.51 |
| 2786 | C₁₀₃H₂₀₃N₉O₅S ₂ | 1711.93 | 8.13 | 42.14 | 2986 | C₅₉H₁₀₉N₇O₇S | 1060.62 | 8.74 | 15.29 |
| 2787 | C₉₉H₁₉₈N₈O₅ | 1580.72 | 9.68 | 41.14 | 2987 | C₇₅H₁₃₈N₆O₁₂ | 1315.96 | 8.80 | 26.03 |
| 2788 | C₁₀₀H₂₀₂N₇O₆P | 1629.73 | 9.66 | 40.59 | 2988 | C₇₃H₁₃₄N₃O₁₂PS ₅ | 1437.16 | 8.84 | 29.13 |
| 2789 | C₁₀₃H₂₀₃N₉O₅S | 1679.88 | 8.70 | 41.15 | 2989 | C₇₀H₁₃₄N₄O₄S₃ | 1192.05 | 9.54 | 26.89 |
| 2790 | C₁₀₀H₁₉₄N₁₀O₈ S | 1696.77 | 7.27 | 40.42 | 2990 | C₆₉H₁₂₉N₃O₁₀S₅ | 1321.10 | 9.31 | 24.87 |
| 2791 | C₉₈H₁₉₉N₆O₃PS | 1572.74 | 8.26 | 41.72 | 2991 | C₇₀H₁₄₁NO₄S | 1092.96 | 10.3 7 | 29.21 |
| 2792 | C₉₅H₁₉₄N₄O₈S₃ | 1616.80 | 8.77 | 39.52 | 2992 | C₇₀H₁₄₀N₁₀O₆S | 1250.01 | 9.15 | 24.15 |
| 2793 | C₉₅H₁₉₄N₆O₈S₃ | 1644.81 | 7.43 | 39.83 | 2993 | C₇₂H₁₃₇NO₄S₅ | 1241.19 | 9.95 | 30.25 |
| 2794 | C₁₀₀H₁₉₉N₅O₈ | 1599.72 | 10.02 | 42.80 | 2994 | C₇₁H₁₄₆N₆O₂ | 1115.99 | 10.2 1 | 26.99 |
| 2795 | C₁₀₄H₂₁₃N₄O₉P ₃S | 1788.85 | 9.70 | 40.93 | 2995 | C₇₇H₁₃₁N₃O₆S | 1226.97 | 9.99 | 28.80 |
| 2796 | C₉₄H₁₉₂N₂O₁₁S ₄ | 1654.81 | 9.58 | 41.21 | 2996 | C₈₄H₁₄₁N₂O₁₂P₃ S₄ | 1592.22 | 8.25 | 26.39 |
| 2797 | C₁₀₀H₂₀₂NO₁₅P S₃ | 1785.86 | 9.99 | 43.01 | 2997 | C₇₆H₁₂₇N₇O₄ | 1202.90 | 9.28 | 25.07 |
| 2798 | C₉₄H₁₉₁NO₁₇S₄ | 1735.79 | 8.03 | 41.32 | 2998 | C₇₆H₁₂₁N₃O₇S₃ | 1285.00 | 8.58 | 26.20 |
| 2799 | C₉₆H₁₉₃NO₁₄S₅ | 1745.89 | 9.20 | 43.57 | 2999 | C₇₅H₁₁₉NO₁₀S | 1226.83 | 10.0 3 | 27.76 |
| 2800 | C₉₇H₁₉₈N₄O₈S₃ | 1644.85 | 10.17 | 41.87 | 3000 | C₇H₁₂₃NO₁₁S₃ | 1335.01 | 9.89 | 27.55 |
| 3001 | C₉₀H₁₅₇N₇O₁₀S ₃ | 1593.47 | 8.81 | 31.48 | 3201 | C₇₂H₁₂₆N₂O₉ | 1163.81 | 9.97 | 23.54 |
| 3002 | C₈₉H₁₅₅NO₆S₈ | 1591.70 | 9.94 | 37.95 | 3202 | C₇₀H₁₂₄N₄O₈ | 1149.78 | 9.97 | 23.36 |
| 3003 | C₈₄H₁₆₈NO₁₀P₃ S₃ | 1541.37 | 9.83 | 31.59 | 3203 | C₇₁H₁₂₅NO₁₄S | 1248.83 | 9.73 | 22.70 |
| 3004 | C₈₀H₁₆₃N₅O₈S₄ | 1451.45 | 10.12 | 31.84 | 3204 | C₇₀H₁₁₇N₃O₁₅ | 1240.71 | 7.26 | 20.93 |
| 3005 | C₈₀H₁₅₂N₄O₇ | 1282.12 | 8.76 | 29.06 | 3205 | C₅₇H₉₉N₃O₃S₈ | 1130.92 | 8.63 | 19.99 |
| 3006 | C₁₀₁H₁₉₈N₄O₅S ₂ | 1612.84 | 9.00 | 42.07 | 3206 | C₆₃H₁₀₉N₆O₅PS₆ | 1253.94 | 8.12 | 21.93 |
| 3007 | C₁₀₃H₁₉₉N₃O₇S ₄ | 1719.98 | 8.96 | 44.17 | 3207 | C₅₈H₁₀₅N₂O₄PS₆ | 1117.82 | 9.26 | 21.79 |
| 3008 | C₁₀₄H₁₉₆N₄O₁₂ S₃ | 1790.91 | 8.40 | 43.89 | 3208 | C₅₉H₁₀₅NO₅S₄ | 1036.73 | 9.99 | 21.27 |
| 3009 | C₁₀₅H₂₀₈N₉O₁₂ PS₄ | 1948.08 | 8.72 | 40.74 | 3209 | C₅₇H₁₀₃N₃O₁₀S₄ | 1118.70 | 9.68 | 21.46 |
| 3010 | C₇₉H₁₅₂N₆O₅S | 1298.18 | 9.08 | 25.22 | 3210 | C₅₉H₁₁₄NO₁₄P₃S₂ | 1218.60 | 8.06 | 17.66 |
| 3011 | C₇₆H₁₅₂N₆O₁₀S | 1342.14 | 9.91 | 26.73 | 3211 | C₆₀H₁₁₆NO₁₂P₃S ₂ | 1200.62 | 8.76 | 19.01 |
| 3012 | C₇₃H₁₄₂N₂O₃S₆ | 1288.31 | 8.72 | 30.69 | 3212 | C₆₆H₁₂₉N₃O₁₄P₄ | 1312.66 | 8.69 | 16.22 |
| 3013 | C₇₇H₁₄₅N₅O₁₀S ₃ | 1397.21 | 8.79 | 29.01 | 3213 | C₆₂H₁₁₇N₂O₁₃P₃ | 1191.54 | 8.34 | 16.88 |
| 3014 | C₇₇H₁₄₁N₇O₆S₈ | 1517.50 | 8.62 | 30.23 | 3214 | C₅₅H₁₀₄N₆O₉S₃ | 1089.65 | 8.26 | 15.77 |
| 3015 | C₇₅H₁₅₂N₅O₁₂P S₃ | 1443.22 | 8.29 | 25.88 | 3215 | C₅₄H₁₀₂N₂O₁₂S₅ | 1131.71 | 8.76 | 17.82 |
| 3016 | C₇₄H₁₄₅N₃O₁₅S ₄ | 1445.22 | 9.05 | 25.98 | 3216 | C₅₅H₁₀₃NO₁₅S₅ | 1178.72 | 9.92 | 19.53 |
| 3017 | C₇₂H₁₄₂N₂O₉S₆ | 1372.29 | 8.96 | 24.57 | 3217 | C₅₄H₁₀₅N₅O₄S | 920.52 | 9.05 | 18.70 |
| 3018 | C₇₇H₁₅₇N₉O₇S | 1353.22 | 10.26 | 29.33 | 3218 | C₅₃H₁₀₁NO₄S₉ | 1104.93 | 8.98 | 24.27 |
| 3019 | C₇₃H₁₄₅N₃O₅S₅ | 1305.28 | 8.80 | 30.66 | 3219 | C₆₀H₁₀₅N₂O₁₂P | 1077.48 | 8.29 | 18.94 |
| 3020 | C₇₄H₁₄₉N₇O₁₀S ₄ | 1425.28 | 8.95 | 26.66 | 3220 | C₅₈H₁₀₅NO₁₆S | 1104.53 | 9.39 | 18.70 |
| 3021 | C₇₁H₁₃₉N₃O₁₆S ₅ | 1451.20 | 8.48 | 24.73 | 3221 | C₇₀H₁₂₆N₆O₆S | 1179.87 | 7.23 | 23.31 |
| 3022 | C₇₄H₁₄₅N₃O₁₁S ₆ | 1445.34 | 9.07 | 26.30 | 3222 | C₇₄H₁₃₄N₁₀O₁₁S | 1372.00 | 8.45 | 23.42 |
| 3023 | C₇₂H₁₄₅N₄O₁₀P S₃ | 1354.12 | 8.61 | 23.41 | 3223 | C₆₉H₁₂₃NS₇ | 1191.17 | 9.86 | 26.81 |
| 3024 | C₇₃H₁₄₇N₄O₉PS ₅ | 1416.27 | 8.33 | 24.01 | 3224 | C₇₁H₁₂₆N₄O₄S₄ | 1228.05 | 7.91 | 25.16 |
| 3025 | C₇₄H₁₄₁NO₁₁S₂ | 1285.06 | 7.98 | 28.99 | 3225 | C₇₁H₁₂₈N₂O₅S₆ | 1282.17 | 9.30 | 28.34 |
| 3026 | C₁₀₉H₂₁₃N₅O₈ | 1721.93 | 8.96 | 42.65 | 3226 | C₇₇H₁₃₇N₆O₇PS₅ | 1450.25 | 8.46 | 25.30 |
| 3027 | C₁₀₄H₂₀₅NOS₇ | 1710.21 | 10.17 | 49.05 | 3227 | C₇₀H₁₂₅N₃O₁₀S₄ | 1297.02 | 7.45 | 26.12 |
| 3028 | C₁₁₁H₂₁₃N₃O₃S ₈ | 1894.42 | 8.11 | 50.12 | 3228 | C₇₂H₁₃₁N₅O₁₁S₃ | 1339.04 | 8.71 | 27.24 |
| 3029 | C₁₁₀H₂₂₂N₃O₁₁ P₃S | 1887.98 | 8.96 | 42.00 | 3229 | C₇₅H₁₄₆N₇O₁₁P₃ | 1414.95 | 7.81 | 22.60 |
| 3030 | C₁₁₀H₂₂₂N₃O₁₁ P₃S | 1887.98 | 8.96 | 42.00 | 3230 | C₇₃H₁₄₀N₃O₁₄P₃ S | 1408.91 | 8.27 | 22.79 |
| 3031 | C₁₀₆H₂₁₁N₃O₁₃ S₂ | 1799.98 | 10.02 | 45.91 | 3231 | C₇₂H₁₄₀N₃O₁₄P₃ | 1364.84 | 8.46 | 23.22 |
| 3032 | C₁₀₃H₂₀₅N₆O₁₀ P | 1718.78 | 8.96 | 40.53 | 3232 | C₇₀H₁₃₁N₅O₁₃S₄ | 1379.08 | 8.01 | 22.50 |
| 3033 | C₁₀₅H₂₁₃N₄O₁₃ P₃S₂ | 1896.92 | 8.94 | 39.79 | 3233 | C₆₇H₁₃₁N₅O₈S₅ | 1295.11 | 9.77 | 25.44 |
| 3034 | C₉₈H₁₉₂N₂O₁₂S ₄ | 1718.86 | 9.02 | 40.76 | 3234 | C₆₅H₁₂₄N₂O₁₄S₅ | 1318.01 | 7.85 | 23.07 |
| 3035 | C₉₃H₁₈₀N₄O₈S | 1514.54 | 9.95 | 38.53 | 3235 | C₇₃H₁₃₉NO₉S₆ | 1367.27 | 10.0 4 | 28.32 |
| 3036 | C₉₆H₁₈₆N₂O₂S₈ | 1657.04 | 10.34 | 44.16 | 3236 | C₇₃H₁₃₀N₄O₈S₃ | 1288.04 | 8.18 | 26.62 |
| 3037 | C₉₄H₁₈₀N₄O₅S₇ | 1670.92 | 8.81 | 39.99 | 3237 | C₇₂H₁₃₂N₄O₁₀S | 1245.93 | 9.51 | 23.80 |
| 3038 | C₈₉H₁₇₇NO₁₅S₃ | 1597.57 | 10.12 | 38.26 | 3238 | C₇₀H₁₃₂NO₁₂PS₂ | 1274.91 | 9.58 | 26.32 |
| 3039 | C₉₂H₁₇₇NO₆S₃ | 1489.61 | 9.88 | 38.99 | 3239 | C₇₃H₁₃₈N₆O₁₀S | 1292.00 | 8.50 | 25.82 |
| 3040 | C₉₃H₁₈₀N₂O₁₂S ₂ | 1582.59 | 8.97 | 38.02 | 3240 | C₇₁H₁₃₇N₇O₁₂S | 1312.97 | 7.95 | 25.01 |
| 3041 | C₇₈H₁₃₉N₃O₆S₅ | 1375.28 | 9.03 | 28.75 | 3241 | C₇₀H₁₃₂N₂O₂S₅ | 1194.14 | 9.86 | 28.40 |
| 3042 | C₇₁H₁₃₈N₆O₁₃S ₅ | 1444.21 | 8.79 | 24.44 | 3242 | C₆₈H₁₂₇N₃S₉ | 1275.32 | 8.31 | 29.94 |
| 3043 | C₇₄H₁₃₇NO₁₀S₃ | 1297.09 | 10.18 | 28.33 | 3243 | C₇₀H₁₃₂N₂O₉S₄ | 1274.07 | 8.35 | 27.50 |
| 3044 | C₅₇H₁₀₉N₅O₈S₅ | 1152.83 | 8.75 | 21.16 | 3244 | C₆₉H₁₃₄N₂O₄S₄ | 1184.08 | 8.84 | 29.64 |
| 3045 | C₅₆H₁₁₂N₆O₃S₂ | 981.67 | 8.79 | 18.08 | 3245 | C₇₁H₁₃₄N₂O₅S₇ | 1320.28 | 7.60 | 30.36 |
| 3046 | C₇₀H₁₂₄N₆O₅S₃ | 1225.98 | 9.62 | 21.57 | 3246 | C₇₀H₁₄₂NO₁₂P₃S ₂ | 1346.94 | 8.97 | 25.79 |
| 3047 | C₇₅H₁₄₀N₈O₅S₃ | 1330.18 | 10.00 | 23.56 | 3247 | C₇₂H₁₄₅N₂O₁₂P₃ S₂ | 1388.00 | 8.91 | 24.47 |
| 3048 | C₇₄H₁₃₉N₅O₁₁ | 1274.95 | 9.94 | 26.43 | 3248 | C₆₅H₁₃₄N₄O₉S₅ | 1276.11 | 8.95 | 25.16 |
| 3049 | C₇₅H₁₃₇N₃O₃S₇ | 1353.36 | 8.51 | 29.95 | 3249 | C₆₈H₁₄₁N₅O₉S₆ | 1365.26 | 9.94 | 24.68 |
| 3050 | C₇₁H₁₄₇N₅O₉S₆ | 1407.34 | 9.97 | 26.49 | 3250 | C₆₅H₁₃₀N₄O₁₆S₄ | 1352.01 | 8.42 | 25.08 |
| 3051 | C₈₁H₁₃₇N₃O₁₀S ₅ | 1473.30 | 9.99 | 30.81 | 3251 | C₆₇H₁₃₅N₅OS₂ | 1090.97 | 9.62 | 24.49 |
| 3052 | C₈₂H₁₂₉N₃O₈S₇ | 1509.37 | 7.82 | 29.90 | 3252 | C₆₉H₁₃₈N₆O₅S | 1163.96 | 8.79 | 24.62 |
| 3053 | C₉₆H₁₆₅N₂O₁₁P S₄ | 1682.59 | 9.47 | 37.52 | 3253 | C₇₀H₁₃₄N₄O₁₁S₂ | 1271.98 | 7.29 | 25.51 |
| 3054 | C₉₃H₁₆₃N₃O₃S₅ | 1531.64 | 8.96 | 37.35 | 3254 | C₇₁H₁₃₇NO₆S₂ | 1165.00 | 10.0 7 | 27.92 |
| 3055 | C₉₈H₁₇₂NO₁₀P₃ S₃ | 1713.55 | 7.92 | 34.58 | 3255 | C₇₄H₁₄₀N₄O₉S₂ | 1294.07 | 8.00 | 27.90 |
| 3056 | C₉₆H₁₆₀N₄O₆S | 1498.42 | 8.16 | 36.02 | 3256 | C₇₃H₁₃₉NO₁₀S₂ | 1255.03 | 10.0 4 | 28.57 |
| 3057 | C₈₇H₁₇₄NO₁₃P₃ | 1535.26 | 9.76 | 31.67 | 3257 | C₇₂H₁₄₁NO₁₃S | 1260.97 | 10.1 1 | 29.32 |
| 3058 | C₇₉H₁₅₃N₂O₇PS ₃ | 1370.25 | 9.95 | 30.27 | 3258 | C₇₈H₁₂₆N₈O₉ | 1319.91 | 8.30 | 26.16 |
| 3059 | C₇₅H₁₄₈N₆O₈S₆ | 1454.40 | 8.59 | 27.41 | 3259 | C₇₆H₁₂₅N₅O₁₀S₂ | 1332.98 | 8.90 | 26.29 |
| 3060 | C₁₁₀H₂₂₀N₁₀O₇ | 1795.03 | 9.47 | 43.87 | 3260 | C₇₈H₁₃₄N₁₂O₄S₂ | 1368.13 | 9.28 | 25.15 |
| 3061 | C₁₁₀H₂₁₃N₃O₁₀ S₅ | 1898.22 | 8.79 | 48.01 | 3261 | C₇₇H₁₂₇N₉O₅S₂ | 1323.04 | 8.94 | 26.78 |
| 3062 | C_{11O}H₂₂₃N₂O₁₁ P₃S₂ | 1907.04 | 9.90 | 44.27 | 3262 | C₇₇H₁₂₅N₂O₆PS₈ | 1462.31 | 8.08 | 28.61 |
| 3063 | C₁₀₉H₂₁₄N₄O₆ | 1676.93 | 9.12 | 44.30 | 3263 | C₇₇H₁₂₃NO₄S₇ | 1351.25 | 9.51 | 29.37 |
| 3064 | C₁₀₇H₂₁₀N₆O₈ | 1708.89 | 10.02 | 43.55 | 3264 | C₇₇H₁₂₂N₄O₁₁S₄ | 1408.08 | 7.58 | 26.28 |
| 3065 | C₉₃H₁₈₅N₃O₁₅S ₄ | 1713.75 | 8.68 | 36.47 | 3265 | C₈₁H₁₄₁N₆O₁₂P₃ | 1483.97 | 7.90 | 24.37 |
| 3066 | C₉₁H₁₈₃NO₁₆S₅ | 1707.76 | 9.33 | 39.98 | 3266 | C₇₃H₁₂₇N₅O₈S₄ | 1331.09 | 9.94 | 26.26 |
| 3067 | C₈₀H₁₄₆N₃O₉PS ₃ | 1421.21 | 9.01 | 30.06 | 3267 | C₇₄H₁₃₂N₄O₁₂P₂ S₃ | 1428.01 | 9.34 | 25.27 |
| 3068 | C₇₄H₁₃₈N₂O₁₂S | 1279.98 | 9.95 | 27.25 | 3268 | C₇₆H₁₃₁N₃O₁₃S₄ | 1423.13 | 9.02 | 27.31 |
| 3069 | C₇₃H₁₄₈N₉O₁₁P S | 1391.07 | 8.77 | 22.93 | 3269 | C₇₅H₁₂₇N₅O₉S₃ | 1339.05 | 9.48 | 21.87 |
| 3070 | C₆₉H₁₃₄NO₈PS₄ | 1265.04 | 9.98 | 27.73 | 3270 | C₇₅H₁₂₂N₂O₈ | 1179.81 | 8.88 | 25.72 |
| 3071 | C₇₃H₁₄₂N₃O₁₁P S₃ | 1365.10 | 9.62 | 27.62 | 3271 | C₇₉H₁₂₈N₂O₁₂S₂ | 1362.01 | 9.85 | 28.34 |
| 3072 | C₅₆H₁₀₄N₄S₅ | 993.78 | 8.81 | 19.28 | 3272 | C₇₆H₁₂₄N₂O₁₁S₂ | 1305.95 | 9.98 | 27.72 |
| 3073 | C₆₈H₁₂₃N₂O₉P | 1143.71 | 8.74 | 20.97 | 3273 | C₉₁H₁₅₄N₆O₈S₂ | 1524.39 | 8.48 | 30.75 |
| 3074 | C₇₇H₁₃₇N₄O₁₂P S₃ | 1438.11 | 8.96 | 25.55 | 3274 | C₉₂H₁₅₅N₉O₈ | 1515.31 | 8.03 | 32.69 |
| 3075 | C₇₄H₁₃₄N₄O₇S | 1223.97 | 10.08 | 21.69 | 3275 | C₉₃H₁₆₁N₄O₉PS | 1542.36 | 9.40 | 34.53 |
| 3076 | C₇₄H₁₃₈N₂O₆ | 1151.93 | 10.06 | 24.52 | 3276 | C₉₀H₁₅₃NO₄S₇ | 1537.64 | 9.51 | 36.88 |
| 3077 | C₉₄H₁₆₃N₅O₇S | 1507.43 | 10.19 | 33.88 | 3277 | C₉₄H₁₅₄N₂O₁₀S₅ | 1632.57 | 8.01 | 35.49 |
| 3078 | C₉₂H₁₆₃N₇O₈S₅ | 1655.66 | 9.65 | 32.84 | 3278 | C₉₄H₁₅₅N₉O₇S₇ | 1747.75 | 8.18 | 34.85 |
| 3079 | C₈₃H₁₅₉NO₇S₅ | 1443.48 | 10.08 | 34.53 | 3279 | C₉₁H₁₆₀N₂O₁₁S₆ | 1650.64 | 9.59 | 34.80 |
| 3080 | C₈₁H₁₆₆N₅O₁₁P S₅ | 1577.52 | 8.86 | 29.78 | 3280 | C₈₈H₁₅₃N₃O₁₄S₃ | 1573.38 | 8.83 | 34.94 |
| 3081 | C₁₀₆H₂₀₅N₃O₅S ₇ | 1826.24 | 9.06 | 44.87 | 3281 | C₈₇H₁₄₇NO₁₄S₄ | 1559.37 | 9.24 | 35.58 |
| 3082 | C₁₀₁H₁₉₉NO₇S₈ | 1796.18 | 10.22 | 47.81 | 3282 | C₉₀H₁₅₀N₄O₉S | 1464.27 | 9.12 | 33.75 |
| 3083 | C₁₀₂H₂₀₅NO₁₁S₅ | 1782.06 | 10.35 | 44.92 | 3283 | C₉₂H₁₅₈NO₉PS₂ | 1517.37 | 9.60 | 37.32 |
| 3084 | C₁₀₀H₁₉₉NO₂S₈ | 1704.18 | 7.88 | 48.22 | 3284 | C₉₂H₁₅₁NO₆S₄ | 1495.46 | 9.87 | 35.38 |
| 3085 | C₇₁H₁₄₆N₂O₁₃S ₅ | 1396.25 | 9.98 | 26.51 | 3285 | C₉₀H₁₄₈N₄O₁₀S₂ | 1510.31 | 7.57 | 32.53 |
| 3086 | C₇₆H₁₄₈N₄O₁₂S ₅ | 1470.34 | 8.20 | 25.26 | 3286 | C₉₀H₁₅₅N₅O₈S | 1467.32 | 8.94 | 33.22 |
| 3087 | C₁₁₁H₂₁₉N₂O₄P S₆ | 1869.32 | 10.16 | 48.79 | 3287 | C₇₇H₁₅₅N₉O₇ | 1319.14 | 9.48 | 28.87 |
| 3088 | C₁₁₁H₂₁₃N₃O₁₀ S₅ | 1910.24 | 8.95 | 48.91 | 3288 | C₈₁H₁₅₂N₁₀O₁₀S | 1458.23 | 8.16 | 30.05 |
| 3089 | C₁₁₂H₂₁₅N₃O₁₂ S₃ | 1892.14 | 8.95 | 48.47 | 3289 | C₇₇H₁₅₃N₉O₆S₃ | 1397.31 | 7.51 | 28.31 |
| 3090 | C₁₀₅H₂₀₁N₃O₁₃ S₄ | 1842.01 | 8.05 | 44.33 | 3290 | C₇₉H₁₅₄N₈O₉ | 1360.15 | 7.29 | 28.84 |
| 3091 | C₉₃H₁₈₅N₃O₈S | 1505.58 | 8.82 | 36.48 | 3291 | C₇₇H₁₄₀NO₃S₈ | 1393.52 | 7.90 | 34.02 |
| 3092 | C₁₀₀H₁₉₉N₄O₁₃ P₃S₂ | 1822.75 | 8.21 | 37.37 | 3292 | C₇₉H₁₅₄N₆O₃S₆ | 1428.50 | 9.20 | 32.59 |
| 3093 | C₈₁H₁₄₆N₃O₁₂P S₃ | 1481.22 | 8.46 | 28.15 | 3293 | C₇₆H₁₄₈N₂O₃S₇ | 1362.45 | 9.46 | 34.16 |
| 3094 | C₆₇H₁₂₆N₆O₅S₂ | 1159.90 | 8.50 | 24.22 | 3294 | C₈₂H₁₅₉N₃O₁₀P₂ S₃ | 1505.31 | 8.51 | 30.25 |
| 3095 | C₆₈H₁₃₃N₆O₉PS | 1241.88 | 7.67 | 19.74 | 3295 | C₇₈H₁₅₂N₂O₇S₄ | 1358.32 | 8.76 | 32.96 |
| 3096 | C₆₉H₁₃₄N₈O₇S₂ | 1252.00 | 8.41 | 22.96 | 3296 | C₇₉H₁₅₄N₄O₁₀S₄ | 1448.36 | 8.70 | 31.46 |
| 3097 | C₇₀H₁₃₁N₃O₆S₈ | 1367.31 | 7.69 | 26.18 | 3297 | C₇₉H₁₆₂NO₃PS₄ | 1333.38 | 9.86 | 33.98 |
| 3098 | C₇₀H₁₃₅NO₄S₇ | 1279.27 | 10.12 | 30.32 | 3298 | C₈₀H₁₅₄N₂O₁₁S₄ | 1448.35 | 9.26 | 33.61 |
| 3099 | C₇₀H₁₃₂N₄O₁₂S ₄ | 1350.08 | 7.46 | 26.35 | 3299 | C₇₆H₁₄₅NO₆S₈ | 1425.48 | 8.81 | 34.83 |
| 3100 | C₆₆H₁₂₅N₃O₁₁S ₄ | 1264.98 | 8.20 | 24.53 | 3300 | C₈₂H₁₇₀N₃O₁₁P₃ | 1467.19 | 9.97 | 30.57 |
| 3101 | C₆₇H₁₂₉NO₁₁S₅ | 1285.06 | 9.67 | 26.84 | 3301 | C₇₇H₁₅₂N₆O₁₁S₅ | 1498.39 | 7.59 | 29.12 |
| 3102 | C₆₇H₁₂₃N₃O₆S₇ | 1291.16 | 8.47 | 27.22 | 3302 | C₇₄H₁₅₃N₇O₁₂S₅ | 1493.38 | 8.28 | 28.08 |
| 3103 | C₆₉H₁₃₈NO₁₂P₃ S₃ | 1362.96 | 9.26 | 24.34 | 3303 | C₇₆H₁₅₂N₆O₁₂S₆ | 1534.44 | 7.73 | 28.03 |
| 3104 | C₆₃H₁₂₅N₇O₉S₆ | 1317.09 | 7.80 | 22.84 | 3304 | C₇₇H₁₅₀N₂O₁₂S₆ | 1488.41 | 7.93 | 28.36 |
| 3105 | C₆₅H₁₂₈N₄O₁₃S ₅ | 1334.05 | 8.14 | 22.01 | ₃₃₀₅ | C₇₇H₁₅₁N₂O₁₀PS ₅ | 1456.33 | 8.39 | 26.01 |
| 3106 | C₆₄H₁₂₈N₃O₁₅PS₄ | 1338.95 | 7.40 | 22.22 | 3306 | C₇₉H₁₅₅N₃O₁₀S₃ | 1403.30 | 8.45 | 27.66 |
| 3107 | C₆₃H₁₂₅N₃O₁₀S ₇ | 1309.12 | 8.27 | 22.61 | 3307 | C₈₀H₁₅₂N₆O₆ | 1294.13 | 8.06 | 30.31 |
| 3108 | C₆₃H₁₂₆N₂O₇S₄ | 1151.95 | 9.53 | 20.16 | 3308 | C₈₁H₁₅₀N₆O₉S | 1384.18 | 8.07 | 31.20 |
| 3109 | C₆₈H₁₃₁N₇O₈S₂ | 1238.96 | 8.67 | 22.69 | 3309 | C₇₈H₁₅₂N₂O₁₁S₂ | 1358.20 | 8.66 | 29.10 |
| 3110 | C₇₁H₁₃₆N₂O₅S | 1129.94 | 9.90 | 27.52 | 3310 | C₈₂H₁₅₂N₈O₁₀ | 1410.16 | 7.54 | 29.00 |
| 3111 | C₇₁H₁₃₈NO₆PS₂ | 1196.98 | 9.97 | 25.57 | 3311 | C₈₀H₁₅₃N₅O₁₀ | 1345.13 | 8.37 | 30.38 |
| 3112 | C₇₅H₁₄₃N₅O₃S₂ | 1227.12 | 10.12 | 25.60 | 3312 | C₈₂H₁₅₅N₃O₁₁S₂ | 1423.27 | 8.49 | 32.30 |
| 3113 | C₇₂H₁₃₈N₈O₅S | 1228.01 | 9.88 | 25.83 | 3313 | C₉₇H₁₉₇N₂O₆P | 1518.62 | 9.48 | 42.37 |
| 3114 | C₇₆H₁₃₄N₄O₄S₈ | 1424.41 | 8.11 | 29.40 | 3314 | C₁₀₀H₂₀₃NO₆S₃ | 1611.90 | 9.02 | 44.49 |
| 3115 | C₇₁H₁₃₃N₅O₆S₄ | 1281.11 | 7.36 | 26.07 | 3315 | C₁₀₄H₂₀₇N₆O₁₂P S | 1796.86 | 8.77 | 40.97 |
| 3116 | C₇₂H₁₃₂N₄O₅S₃ | 1230.05 | 9.26 | 27.72 | 3316 | C₁₀₀H₁₉₈N₇O₁₂P S | 1753.75 | 7.59 | 39.40 |
| 3117 | C₇₃H₁₃₂N₄O₈S₆ | 1386.24 | 7.72 | 28.90 | 3317 | C₁₀₃H₂₀₄N₉O₉PS | 1775.85 | 7.96 | 38.97 |
| 3118 | C₇₇H₁₅₂N₂O₁₅P ₄S | 1502.02 | 8.23 | 24.37 | 3318 | C₁₀₀H₁₉₀N₄O₅S₆ | 1721.00 | 7.09 | 43.77 |
| 3119 | C₇₇H₁₄₈NO₁₁P3 S₃ | 1453.13 | 9.91 | 26.96 | 3319 | C₁₀₃H₁₉₉N₄O₄PS ₆ | 1781.08 | 7.23 | 43.88 |
| 3120 | C₇₁H₁₃₇N₅O₈S₅ | 1349.20 | 7.82 | 25.24 | 3320 | C₁₀₀H₁₉₇N₅O₈S₄ | 1725.94 | 9.14 | 42.88 |
| 3121 | C₇₂H₁₃₉N₆O₁₁P S₅ | 1456.21 | 7.67 | 26.24 | 3321 | C₁₀₄H₁₉₈N₄O₉S₆ | 1841.11 | 8.25 | 44.96 |
| 3122 | C₇₃H₁₄₁N₂O₁₂P S₄ | 1398.15 | 8.95 | 24.70 | 3322 | C₉₉H₁₉₂N₂O₁₄S₅ | 1794.92 | 8.35 | 43.66 |
| 3123 | C₇₃H₁₄₀NO₁₃PS ₄ | 1399.13 | 9.72 | 26.53 | 3323 | C₁₀₃H₂₀₁N₂O₁₀P S₄ | 1786.96 | 8.46 | 44.74 |
| 3124 | C₆₉H₁₂₇N₃O₁₂S ₅ | 1351.08 | 7.54 | 25.78 | 3324 | C₁₀₂H_{19S}N₇O₈S₆ | 1840.08 | 8.41 | 43.99 |
| 3125 | C₇₂H₁₃₅N₅O₁₂S ₃ | 1359.08 | 7.71 | 24.04 | 3325 | C₁₀₂H₂₀₄N₃O₁₆P ₃S | 1853.74 | 7.25 | 38.61 |
| 3126 | C₆₈H₁₃₀N₂O₉S_{S} | 1280.09 | 9.58 | 25.45 | 3326 | C₁₀₆H₂₁₄N₃O₁₁P ₃S₂ | 1863.93 | 8.94 | 40.89 |
| 3127 | C₇₁H₁₃₃NO₁₃S₄ | 1337.08 | 9.69 | 26.71 | 3327 | C₉₇H₁₉₇N₅O₁₀S₃ | 1689.85 | 9.91 | 40.33 |
| 3128 | C₆₉H₁₃₂N₂O₁₀S ₆ | 1342.18 | 8.71 | 22.55 | 3328 | C₉₇H₁₉₂N₆O₈S₇ | 1795.06 | 7.50 | 39.79 |
| 3129 | C₇₂H₁₃₃N₃O₁₃S | 1280.92 | 7.06 | 25.48 | 3329 | C₉₇H₁₉₇N₇O₆S₅ | 1717.99 | 9.70 | 39.61 |
| 3130 | C₇₈H₁₄₃NO₉S₃ | 1335.18 | 10.04 | 30.97 | 3330 | C₉₉H₁₉₃N₃O₁₀S₇ | 1810.06 | 8.90 | 42.09 |
| 3131 | C₇₆H₁₃₄N₆O₁₂S | 1356.00 | 8.01 | 25.46 | 3331 | C₉₇H₁₉₀N₄O₉S₃ | 1652.79 | 8.14 | 38.15 |
| 3132 | C₇₂H₁₃₆N₄O₈S₂ | 1250.02 | 9.77 | 23.12 | 3332 | C₉₈H₁₉₃N₃O₁₀S₆ | 1765.99 | 8.13 | 37.98 |
| 3133 | C₄₈H₉₇NO₇S₃ | 896.48 | 9.93 | 17.95 | 3333 | C₉₆H₁₉₄N₄OS₂ | 1484.76 | 9.09 | 41.92 |
| 3134 | C₅₅H₁₀₅N₇O₆S | 992.55 | 8.64 | 14.03 | 3334 | C₁₀₅H₂₀₁N₂O₉PS | 1698.80 | 7.87 | 42.52 |
| 3135 | C₅₂H₉₇N₅O₈ | 920.37 | 7.30 | 14.27 | 3335 | C₁₀₂H₁₉₃N₃O₇S₂ | 1637.80 | 7.73 | 42.08 |
| 3136 | C₅₅H₁₀₈N₄O₆S₃ | 1017.67 | 10.20 | 20.03 | 3336 | C₁₀₁H₁₉₂N₄O₁₂ | 1654.66 | 7.87 | 39.92 |
| 3137 | C₅₁H₁₀₁N₄O₁₀P S₂ | 1025.48 | 9.12 | 18.42 | 3337 | C₉₉H₁₉₁NO₁₅S | 1667.67 | 9.69 | 42.63 |
| 3138 | C₅₅H₁₀₁N₃O₄S₄ | 996.67 | 8.27 | 18.05 | 3338 | C₇₅H₁₄₃N₁₃O₆S | 1355.11 | 8.07 | 25.23 |
| 3139 | C₅₂H₁₀₀N₄O₃S₃ | 925.58 | 8.64 | 18.64 | 3339 | C₇₆H₁₄₅N₉O₅S₄ | 1393.29 | 7.57 | 26.41 |
| 3140 | C₄₈H₉₄N₂O₃S₅ | 907.59 | 7.71 | 18.31 | 3340 | C₇₈H₁₅₁N₉O₉ | 1359.12 | 7.94 | 25.79 |
| 3141 | C₅₆H₁₀₆N₂O₉S₅ | 1111.77 | 10.02 | 23.32 | 3341 | C₇₆H₁₃₉N₃O₃S₈ | 1399.45 | 7.63 | 30.64 |
| 3142 | C₅₅H₉₉N₃O₇S₇ | 1138.83 | 9.16 | 21.40 | 3342 | C₇₆H₁₄₅NO₅S₅ | 1313.30 | 9.99 | 31.73 |
| 3143 | C₅₄H₁₀₇N₂O₁₄P ₃S | 1133.43 | 7.27 | 15.46 | 3343 | C₇₂H₁₃₇N₃O₇S₃ | 1253.08 | 9.28 | 29.58 |
| 3144 | C₅₇H₁₁₆N₃O₁₀P ₃S | 1128.55 | 9.57 | 15.21 | 3344 | C₆₇H₁₃₈N₂O₅S₅ | 1212.15 | 8.77 | 28.81 |
| 3145 | C₅₈H₁₁₉N₃O₁₄P ₄ | 1206.49 | 9.37 | 15.64 | 3345 | C₇₂H₁₃₇NO₄S₉ | 1369.43 | 9.47 | 33.57 |
| 3146 | C₅₀H₁₀₂N₆O₉S₄ | 1059.64 | 8.91 | 14.11 | 3346 | C₇₄H₁₅₂N₃O₁₂P₃ S | 1401.02 | 9.40 | 26.10 |
| 3147 | C₄₆H₉₆N₆O₈S₃ | 957.49 | 9.30 | 14.92 | 3347 | C₆₆H₁₃₈N₄O₉S₅ | 1292.15 | 8.80 | 23.19 |
| 3148 | C₄₉H₉₅NO₁₀S₇ | 1082.72 | 8.89 | 17.71 | 3348 | C₇₁H₁₄₂N₂O₁₁S₅ | 1360.22 | 10.1 0 | 28.27 |
| 3149 | C₅₂H₁₀₆N₂O₈S₄ | 1015.67 | 10.20 | 15.94 | 3349 | C₆₉H₁₃₇N₃O₉S₆ | 1345.23 | 8.87 | 26.02 |
| 3150 | C₅₂H₁₀₂N₆OS₄ | 955.67 | 8.71 | 17.72 | 3350 | C₇₁H₁₃₉N₃O₁₄S₆ | 1451.26 | 7.99 | 27.43 |
| 3151 | C₄₈H₉₉N₅O₅ | 826.35 | 8.78 | 16.86 | 3351 | C₇₁H₁₄₀N₆O₂S₂ | 1174.06 | 7.56 | 26.04 |
| 3152 | C₅₀H₁₀₀N₂O₂S₇ | 985.78 | 8.94 | 20.77 | 3352 | C₇₀H₁₄₃N₅O₂ | 1086.95 | 9.65 | 26.48 |
| 3153 | C₅₀H₉₅NO₉S₂ | 918.43 | 8.74 | 17.41 | 3353 | C₆₉H₁₄₀N₈O₃ | 1129.93 | 7.47 | 24.72 |
| 3154 | C₅₃H₉₇N₃O₁₀ | 936.37 | 8.02 | 17.12 | 3354 | C₇₇H₁₄₁N₃O₈S₂ | 1301.11 | 7.55 | 28.26 |
| 3155 | C₅₂H₉₉N₃O₈S₂ | 958.50 | 9.45 | 17.41 | 3355 | C₇₂H₁₃₅N₃O₁₀S | 1234.94 | 7.57 | 26.66 |
| 3156 | C₅₂H₉₆N₃O₁₁PS | 1002.38 | 8.12 | 16.02 | 3356 | C₇₄H₁₃₈N₂O₁₀S | 1247.98 | 7.46 | 27.52 |
| 3157 | C₅₂H₉₂N₂O₁₅ | 985.31 | 7.52 | 16.32 | 3357 | C₇₀H₁₃₆N₂O₁₀S₂ | 1229.98 | 9.66 | 28.88 |
| 3158 | C₅₃H₁₀₀N₂O₁₃ | 973.38 | 8.88 | 15.78 | 3358 | C₇₅H₁₅₀N₈O₅ | 1244.08 | 9.70 | 26.96 |
| 3159 | C₅₃H₁₀₃N₆O₁₀P S | 1047.47 | 8.46 | 14.49 | 3359 | C₇₄H₁₄₄N₅O₈PS | 1295.03 | 8.00 | 25.17 |
| 3160 | C₆₀H₁₁₀N₂O₇S₂ | 1035.67 | 8.77 | 21.53 | 3360 | C₇₂H₁₄₅N₅O₉S₃ | 1321.16 | 9.35 | 26.34 |
| 3161 | C₆₁H₁₀₉NO₇S₂ | 1032.66 | 9.76 | 22.04 | 3361 | C₇₁H₁₄₄N₈O₇S | 1254.04 | 7.70 | 26.61 |
| 3162 | C₆₈H₁₂₄N₇O₁₁P S | 1278.81 | 9.49 | 18.68 | 3362 | C₇₉H₁₅₁N₉O₇S₂ | 1403.25 | 8.31 | 26.66 |
| 3163 | C₆₈H₁₁₇NO₃S₆ | 1189.05 | 9.07 | 26.17 | 3363 | C₇₂H₁₄₄N₁₀O₇S₃ | 1358.19 | 8.43 | 26.21 |
| 3164 | C₆₅H₁₁₀N₂O₃S₆ | 1159.97 | 9.44 | 24.53 | 3364 | C₇₁H₁₃₅NO₂S₇ | 1259.29 | 9.74 | 30.80 |
| 3165 | C₆₇H₁₁₄N₄O₄S₈ | 1296.15 | 8.05 | 23.10 | 3365 | C₇₃H₁₃₉NO₂S₈ | 1319.40 | 8.81 | 32.79 |
| 3166 | C₆₇H₁₁₉N₃OS₆ | 1175.07 | 9.63 | 26.04 | 3366 | C₇₁H₁₄₄NO₅PS₄ | 1251.15 | 10.0 3 | 31.16 |
| 3167 | C₆₄H₁₀₇NO₂S₆ | 1114.92 | 8.40 | 23.72 | 3367 | C₆₉H₁₃₉NO₃S₆ | 1223.23 | 9.21 | 31.23 |
| 3168 | C₆₅H₁₁₁N₅O₅S₃ | 1138.81 | 8.82 | 21.52 | 3368 | C₇₅H₁₄₁N₂O₁₁PS ₄ | 1406.17 | 8.64 | 28.77 |
| 3169 | C₅₇H₁₀₅N₃O₃S₄ | 1008.72 | 8.60 | 22.28 | 3369 | C₇₄H₁₅₃N₄O₁₁P₃ S₂ | 1432.10 | 8.31 | 25.59 |
| 3170 | C₆₄H₁₁₃N₅O₅S₇ | 1257.06 | 9.42 | 23.90 | 3370 | C₇₇H₁₅₀N₃O₁₄P₃ S | 1467.04 | 7.45 | 24.68 |
| 3171 | C₆₄H₁₁₈NO₁₂P₃ S₄ | 1314.80 | 8.70 | 19.42 | 3371 | C₇₁H₁₄₆N₈O₈S₄ | 1368.24 | 8.39 | 25.23 |
| 3172 | C₅₇H₁₀₇N₅O₈S₄ | 1118.75 | 9.82 | 18.80 | 3372 | C₆₈H₁₃₅NO₁₅S₅ | 1367.12 | 8.51 | 26.84 |
| 3173 | C₆₀H₁₁₃N₅O₈S₄ | 1160.83 | 9.93 | 19.66 | 3373 | C₇₁H₁₄₂N₆O₁₅S₃ | 1416.12 | 7.02 | 26.05 |
| 3174 | C₆₃H₁₁₈N₆O₉S₄ | 1231.91 | 9.47 | 17.86 | 3374 | C₆₈H₁₃₅NO₁₀S₃ | 1223.00 | 8.08 | 24.60 |
| 3175 | C₆₂H₁₁₁N₃O₁₃S ₄ | 1234.82 | 8.51 | 17.77 | 3375 | C₇₃H₁₄₄N₃O₁₀PS ₅ | 1415.24 | 8.25 | 22.65 |
| 3176 | C₆₃H₁₁₄NO₁₃PS ₅ | 1284.87 | 9.07 | 21.47 | 3376 | C₇₂H₁₃₇N₃O₁₁S₆ | 1413.26 | 7.91 | 23.79 |
| 3177 | C₆₂H₁₁₇N₂O₁₅P S₄ | 1289.83 | 9.18 | 18.80 | 3377 | C₇₀H₁₄₂N₂O₁₀S₄ | 1300.15 | 9.93 | 24.88 |
| 3178 | C₆₂H₁₁₁N₃O₁₄S ₆ | 1314.94 | 8.47 | 19.91 | 3378 | C₆₈H₁₃₆N₆O₇S₂ | 1213.99 | 7.07 | 26.69 |
| 3179 | C₆₃H₁₁₅N₂O₁₇P S₅ | 1363.88 | 8.72 | 20.79 | 3379 | C₇₂H₁₄₂N₆O₄ | 1155.97 | 8.79 | 26.00 |
| 3180 | C₅₈H₁₀₇N₃O₈S₃ | 1070.69 | 9.95 | 16.48 | 3380 | C₇₅H₁₄₆N₃O₇PS | 1265.04 | 9.30 | 28.09 |
| 3181 | C₆₀H₁₀₆N₂O₈S₃ | 1079.69 | 9.71 | 22.06 | 3381 | C₇₄H₁₃₉N₃O₆S₃ | 1263.12 | 8.03 | 27.70 |
| 3182 | C₆₄H₁₁₂N₄O₅S₂ | 1081.74 | 9.13 | 19.93 | 3382 | C₇₅H₁₄₀N₂O₇ | 1181.95 | 8.49 | 27.43 |
| 3183 | C₇₂H₁₂₉N₇O₆S | 1220.93 | 9.47 | 19.86 | 3383 | C₇₄H₁₃₇N₃O₇S₄ | 1309.16 | 8.31 | 28.35 |
| 3184 | C₇₄H₁₃₁N₇O₆S₂ | 1279.03 | 8.74 | 20.89 | 3384 | C₇₄H₁₃₇N₃O₁₄S₂ | 1357.04 | 8.49 | 25.65 |
| 3185 | C₆₉H₁₂₃N₅O₈S | 1182.83 | 9.47 | 23.43 | 3385 | C₁₀₇H₂₀₉N₅O₁₀S | 1757.93 | 9.69 | 46.30 |
| 3186 | C₇₁H₁₂₆N₆O₁₀S₃ | 1320.00 | 8.10 | 21.56 | 3386 | C₁₀₅H₂₀₈N₆O₁₂S | 1778.91 | 9.08 | 44.20 |
| 3187 | C₇₀H₁₂₉N₄O₁₁P S | 1265.85 | 8.87 | 23.05 | 3387 | C₁₀₇H₂₀₉N₇O₁₀S ₂ | 1818.01 | 7.78 | 44.86 |
| 3188 | C₇₁H₁₂₈N₈O₆S | 1221.91 | 9.84 | 21.09 | 3388 | C₁₀₅H₂₀₉N₇O₇S | 1713.93 | 9.76 | 43.29 |
| 3189 | C₆₅H₁₂₂N₈O₉S₂ | 1223.86 | 7.01 | 20.16 | 3389 | C₁₁₀H₂₁₄N₃O₇PS ₅ | 1882.21 | 8.72 | 46.15 |
| 3190 | C₇₂H₁₂₈N₃O₇PS ₄ | 1307.04 | 8.58 | 21.87 | 3390 | C₁₀₄H₂₀₃NO₄S₇ | 1756.19 | 7.73 | 48.33 |
| 3191 | C₆₉H₁₂₀N₂O₂S₇ | 1234.15 | 9.59 | 25.76 | 3391 | C₁₁₀H₂₁₄NO₈PS₄ | 1838.13 | 9.76 | 48.15 |
| 3192 | C₇₂H₁₂₈N₂O₃S₇ | 1294.25 | 9.98 | 27.71 | 3392 | C₁₀₅H₂₀₅NO₈S₅ | 1770.09 | 8.22 | 48.48 |
| 3193 | C₇₁H₁₂₀N₂O₇S₃ | 1209.93 | 8.72 | 23.50 | 3393 | C₁₀₆H₂₀₇N₃O₈S₃ | 1748.01 | 8.86 | 45.55 |
| 3194 | C₆₉H₁₁₉NO₇S₇ | 1299.13 | 9.31 | 26.45 | 3394 | C₁₀₂H₂₀₂N₄O₆S₄ | 1709.00 | 8.10 | 46.12 |
| 3195 | C₆₆H₁₂₀N₂O₅S₄ | 1149.93 | 7.54 | 24.03 | 3395 | C₁₁₁H₂₂₁N₂O₁₃P ₃S₂ | 1949.03 | 7.97 | 44.55 |
| 3196 | C₇₄H₁₃₇N₂O₁₂P ₃S₃ | 1436.01 | 8.52 | 22.06 | 3396 | C₁₀₀H₁₉₉NO₁₁S₇ | 1816.11 | 7.43 | 44.88 |
| 3197 | C₇₄H₁₃₆N₅O₁₂P ₃S₂ | 1444.97 | 7.61 | 19.31 | 3397 | C₁₀₅H₂₁₂N₃O₁₃P S₃ | 1852.01 | 8.82 | 42.85 |
| 3198 | C₆₆H₁₂₃N₅O₆S₅ | 1243.04 | 9.94 | 20.38 | 3398 | C₁₀₂H₂₀₃N₅O₁₂S ₅ | 1852.07 | 7.51 | 45.60 |
| 3199 | C₆₈H₁₂₃N₃O₁₂S ₄ | 1302.98 | 8.93 | 19.98 | 3399 | C₁₀₂H₂₁₀N₄OS | 1540.89 | 10.1 3 | 45.50 |
| 3200 | C₆₇H₁₁₇N₃O₉S₅ | 1268.98 | 7.82 | 18.53 | 3400 | C₁₀₆H₂₁₀N₄O₅S₇ | 1845.29 | 8.50 | 47.42 |
| 3401 | C₈₉H₁₇₄NO₇PS₄ | 1529.58 | 8.39 | 38.72 | 3413 | C₈₈H₁₇₄N₂O₃S₇ | 1532.79 | 8.91 | 42.54 |
| 3402 | C₉₀H₁₇₁N₃O₇S₃ | 1503.55 | 7.71 | 37.84 | 3414 | C₉₁H₁₇₃N₅O₆S₃ | 1529.59 | 7.96 | 34.92 |
| 3403 | C₈₉H₁₇₄N₂O₈S₅ | 1560.68 | 9.46 | 37.71 | 3415 | C₈₇H₁₇₂N₄O₅S | 1386.42 | 8.88 | 34.57 |
| 3404 | C₉₂H₁₇₇NO₆S₈ | 1649.91 | 9.21 | 43.98 | 3416 | C₇₁H₁₃₅N₅O₉S₃ | 1299.07 | 9.96 | 27.23 |
| 3405 | C₉₁H₁₇₇NO₈S₇ | 1637.84 | 8.69 | 40.23 | 3417 | C₇₈H₁₄₉N₄O₁₁P | 1350.04 | 9.88 | 28.11 |
| 3406 | C₈₈H₁₇₁NO₇S₇ | 1579.76 | 9.60 | 39.68 | 3418 | C₇₁H₁₃₁NO₃S₉ | 1335.37 | 9.68 | 29.97 |
| 3407 | C₉₄H₁₉₃N₂O₁₁P ₃S₄ | 1748.74 | 8.86 | 36.61 | 3419 | C₇₅H₁₃₇N₃O₅S₄ | 1289.18 | 9.96 | 29.92 |
| 3408 | C₉₄H₁₉₁N₂O₁₁P ₃S₂ | 1682.61 | 9.39 | 36.62 | 3420 | C₇₁H₁₃₂N₆O₁₀S₆ | 1422.23 | 7.13 | 23.90 |
| 3409 | C₉₇H₁₉₆N₃O₁₅P ₃S | 1769.62 | 9.74 | 34.98 | 3421 | C₆₉H₁₃₃N₅O₈S₅ | 1321.15 | 8.74 | 23.45 |
| 3410 | C₈₈H₁₈₀N₄O₇S₅ | 1566.73 | 9.79 | 37.89 | 3422 | C₇₀H₁₃₉N₄O₁₄PS ₄ | 1420.11 | 7.55 | 24.35 |
| 3411 | C₈₉H₁₈₂NO₁₅PS ₄ | 1665.64 | 9.36 | 36.96 | | | | | |
| 3412 | C₈₈H₁₇₆N₂O₁₄S ₄ | 1614.62 | 7.11 | 32.69 | | | | | |

The above properties of the exemplary compounds of the first ionizable lipid of the present application indicate that the exemplary compounds can serve as excellent surfactants, and are particularly suitable for preparation of lipid nanoparticles as drug delivery carriers.

### (4) Preparation of prefabricated carrier in preparation of product for in vitro gene delivery into immune cells and stem cells

### 4.1 Preparation of Prefabricated Carrier (Lipid Nanoparticle (LNP))

| Formulation number | Formulation composition | Molar percentage in formulation, % |
|---|---|---|
| 1 | Compound 1008/DSPC/Chol/ALC-0159 | 40/20/39/1 |
| 2 | Compound 6023/Compound 1115/DOPE/Chol/ALC-0159 | 5/25/15/54.5/0.5 |
| 3 | Compound 6008/Compound 1005/DSPC/Chol/PEG-DMG | 10/30/10/47/3 |
| 4 | Compound 1001/ALC-0315/DSPC/Chol/ALC-0159 | 10/40/10/38.5/1.5 |
| 5 | Compound 6011/Compound 1114/DSPC/Chol/PEG-DMG | 25/25/10/39/1 |
| 6 | Compound 1009/DOPE/Chol/ALC-0159 | 50/10/38.5/1.5 |
| 7 | Compound 1013/DOPC/Chol/PEG-DMG | 45/10/40/5 |
| 8 | Compound 6024/Compound 1008/DOPE/Chol/ALC-0159 | 2.5/30/30/37/0.5 |
| 9 | Compound 6024/Compound 1011/DOPE/Chol/PEG-DMG | 5/45/10/39/1 |
| 10 | Compound 1003/POPC/Chol/PEG-DMG | 40/20/38/2 |
| 11 | Compound 1005/Compound 6038/DOPE/Chol/ALC-0159 | 30/10/10/48.5/1.5 |
| 12 | Compound 6003/Compound 1014/ALC-0315/DSPC/Chol/PEG-DMG | 3/10/35/10/40.5/1.5 |
| 13 | Compound 1115/cKK-E12/DOPE/Chol/PEG-DMG | 30/20/15/34/1 |

The prefabricated carriers were respectively prepared according to the molar ratios described above, for each, the specific method is as follows:
The lipid raw materials were dissolved into the ethanol to obtain a lipid-ethanol solution in which a total concentration of all the lipid raw materials was 8 mg/mL. The lipid-ethanol solution and a 50 mM citrate buffer (pH 4.0) were mixed at a volume ratio of 1:3 using a nanoparticle preparation device, followed by ultrafiltration. A sample was collected which was the prefabricated carrier with a lipid concentration of 2 mg/mL.

### 4.2 Preparation of Prefabricated Carried-Based Composition

| Example number | Prefabricated carrier number | Nucleic acid type | Concentration of nucleic acid in delivery system, ng/µl | Nucleic acid amount, w/w | Prefabricated carrier amount, w/w |
|---|---|---|---|---|---|
| 1 | 1 | eGFP-pDNA | 10 | 36% | 64% |
| 2 | 13 | Luc-mRNA | 30 | 10% | 90% |
| 3 | 2 | eGFP mRNA | 40 | 5% | 95% |
| 4 | 4 | Luc pDNA | 40 | 2.7% | 97.3% |
| 5 | 7 | Luc-mRNA | 50 | 3.6% | 96.4% |
| 6 | 6 | EGFP mRNA | 50 | 1.8% | 98.2% |
| 7 | 4 | EGFP mRNA | 80 | 0.5% | 99.5% |
| 8 | 10 | Luc-mRNA | 30 | 20% | 80% |
| 9 | 3 | eGFP mRNA | 30 | 3.6% | 96.4% |
| 10 | 8 | Luc-mRNA | 80 | 7% | 93% |
| 11 | 9 | eGFP mRNA | 100 | 10% | 90% |
| 12 | 10 | siRNA-cy | 150 | 3.6% | 96.4% |
| 13 | 5 | siRNA-cy | 200 | 12% | 88% |
| 14 | 11 | eGFP mRNA | 60 | 7% | 93% |
| 15 | 8 | Luc-pDNA | 500 | 5% | 95% |
| 16 | 12 | Luc-mRNA | 20 | 5% | 95% |

The nucleic acid was used as an active ingredient and dissolved into nuclease-free water to prepare solution 1 with twice the target concentration specified in each example. The prefabricated carrier was diluted with the nuclease-free water according to a formulation ratio of each example to prepare solution 2. Equal volumes of solution 1 and solution 2 were mixed, followed by vortexing for 2-3 s to obtain the prefabricated carrier-based composition.

### (5) Determination of particle size and Zeta potential of prefabricated carrier-based composition

1. Determination of particle size and polydispersity index (PDI): an average particle size and PDI of the sample solutions in the prefabricated carrier-based compositions in examples were determined through dynamic light scattering using Malvern ZetaSizer Nano ZS90. It set a measurement angle as 90°, a refractive index of the dispersant as 1.330, and a testing temperature as 25°C.
2. A testing method for an encapsulation ratio is as follows:

An encapsulation ratio of mRNA was determined using the Quant-it Ribogreen RNA Quantitation Kit (ThermoFisher Scientific, UK) in accordance with a manufacturer's instructions.

An encapsulation ratio of pDNA in the prefabricated carrier-based composition was determined using the dsDNA HS Kit (Vazyme/EQ121) in accordance with the manufacturer's instructions.

An encapsulation ratio of siRNA in the prefabricated carrier-based composition was determined using the Qubit^{®} microRNA Reagent Kit in accordance with the manufacturer's instructions.

Data for the average particle size, PDI, and encapsulation ratio of the prefabricated carrier-based compositions prepared in examples are shown in Table 2.

**Table 2 Summary Table of Particle Size, PDI, and Encapsulation Ratio of Prefabricated Carrier-Based Composition**

| Example number | Particle size, nm | PDI | Encapsulation ratio, % |
|---|---|---|---|
| 1 | 190.6 | 0.146 | 82.1 |
| 2 | 246.7 | 0.156 | 90.5 |
| 3 | 160.4 | 0.168 | 96.2 |
| 4 | 124.3 | 0.118 | 84.6 |
| 5 | 156.1 | 0.137 | 89.4 |
| 6 | 124.8 | 0.117 | 94.6 |
| 7 | 114.3 | 0.105 | 96.7 |
| 8 | 448.7 | 0.201 | 82.3 |
| 9 | 172.6 | 0.189 | 85.8 |
| 10 | 215.6 | 0.179 | 84.2 |
| 11 | 346.7 | 0.193 | 85.6 |
| 12 | 120.7 | 0.137 | 85.1 |
| 13 | 426.7 | 0.204 | 80.8 |
| 14 | 246.7 | 0.173 | 88.7 |
| 15 | 147.4 | 0.156 | 83.7 |
| 16 | 190.6 | 0.172 | 90.7 |

It can be seen from Table 2 that the prefabricated carrier-based compositions provided in the present application have small and uniform particle sizes, and high encapsulation ratios (>80%).

### (6) Evaluation on in vitro delivery to immune cells using prefabricated carrier-based composition

### 6.1 Human-derived NK cells (NK-92)

Human-derived NK cells (NK-92) were used to evaluate in vitro delivery efficiency of the prefabricated carrier-eGFP mRNA compositions in Example 3 and Example 6. The cells were cultured in an NK-92 dedicated medium to ensure that the cells were at a logarithmic growth phase. One day prior to delivery, the cells were seeded into a 24-well culture plate at an appropriate cell density and allowed to grow overnight. During delivery, the prefabricated carrier-nucleic acid complexes prepared in Examples 3 and 6 were separately diluted with a buffer solution and then added to the 24-well cell culture plate with an amount of eGFP-mRNA per well reaching 500 ng and 1000 ng, respectively. Lipofectamine 2000-mediated delivery of eGFP mRNA was set as control. After incubation for 24 h in an incubator under conditions of 37°C and 5% CO₂ , cell viability and delivery efficiency were determined by flow cytometry. Results are shown in Table 3.

**Table 3 In Vitro Delivery Efficiency of Prefabricated Carrier-Nucleic Acid Complex to Human-Derived NK Cells**

| Example | Delivery efficiency (Q3) | | Cell viability (Q3 + Q4) | |
|---|---|---|---|---|
| | 500 ng | 1000 ng | 500 ng | 1000 ng |
| Example 3 | 63.3% | 69.5% | 99.4% | 99.5% |
| Example 6 | 78.9% | 83.3% | 98.7% | 98.2% |
| Negative | 0.2% | | 99.7% | |
| Lipofectamine Stem | 0.6% | | 99.7% | |

The results show that all the prefabricated carrier-eGFP mRNA complexes provided in the present application have high protein expression efficiency in the NK-92 cells.

### 6.2 Use in human-derived MSC cells

Human-derived MSC cells were used to evaluate in vitro transfection efficiency of the prefabricated carrier-eGFP mRNA complex prepared in Example 9. The cells were cultured in a dedicated medium to ensure that the cells were at the logarithmic growth phase. One day prior to transfection, the cells were seeded into a 24-well culture plate at an appropriate density and allowed to grow overnight. During delivery, the prefabricated carrier-eGFP mRNA complex prepared in Example 20 was diluted with a buffer solution and then added to the 24-well cell culture plate with an amount of eGFP-mRNA per well reaching 250 ng and 500 ng, respectively. Lipofectamine 2000-mediated delivery of eGFP mRNA was set as control. After incubation for 24 h in an incubator under the conditions of 37°C and 5% CO₂ , cell viability and delivery efficiency were determined by flow cytometry. Results are shown in Table 4.

**Table 4 In Vitro Delivery Efficiency of Prefabricated Carrier-Nucleic Acid Complex to Human-Derived MSC Cells**

| Example | Delivery efficiency (Q3) | | Cell viability (Q3 + Q4) | |
|---|---|---|---|---|
| | 250 ng | 500 ng | 250 ng | 500 ng |
| Example 9 | 83.2% | 91.5% | 97.1% | 95.8% |
| Blank | 0.019% | | 96.9% | |
| Lipofectamine 2000 | / | 2.4% | / | 25.4% |

The results show that all the prefabricated carrier-eGFP mRNA complexes provided in the present application have high protein expression efficiency in the human-derived MSC cells.

### 6.3 Use in T cells

Human T lymphocytic leukemia cells (Jurkat) were used to evaluate the in vitro delivery efficiency of the prefabricated carrier-eGFP mRNA complexes prepared in Examples 7, 11 and 14. The cells were cultured in a RPMI-1640 medium to ensure that the cells were at the logarithmic growth phase. One day prior to delivery, the cells were seeded into a 24-well culture plate at an appropriate density and allowed to grow overnight. During delivery, the prefabricated carrier-eGFP mRNA complexes prepared in Examples 7, 11 and 14 were diluted with a buffer solution and then added to the 24-well cell culture plate with an amount of eGFP-mRNA per well reaching 500 ng/1000 ng, respectively. After incubation for 24 h in an incubator under the conditions of 37°C and 5% CO₂ , cell viability and delivery efficiency were determined by flow cytometry. Results are shown in Table 5.

**Table 5 In Vitro Delivery Efficiency of Prefabricated Carrier-Nucleic Acid Complex to T Cells**

| **Example** | **Delivery efficiency (Q3)** | | **Cell viability (Q3 + Q4)** | |
|---|---|---|---|---|
| | 500 ng | 1000 ng | 500 ng | 1000 ng |
| Example 7 | 96.8% | 97.6% | 99.5% | 99.3% |
| Example 11 | 94.4% | 99.5% | 96.1% | 99.3% |
| Example 14 | 87.8% | 99.4% | 100.0% | 99.9% |
| Negative | 0% | | 98.8% | |

The results show that all the prefabricated carrier-eGFP mRNA complexes provided in the present application have high protein expression efficiency in the T cells, and, therefore, can be used for delivery into the T cells.

### 6.4 Primary T cells

Primary T cells isolated from human peripheral blood were used to evaluate in vitro delivery efficiency of the prefabricated carrier-eGFP mRNA complex prepared in Example 11. The cells were cultured in a dedicated medium and activated with CD3/CD28 to ensure that the cells were at the logarithmic growth phase. The cells were seeded into a 24-well culture plate at an appropriate density and allowed to grow overnight. Prior to delivery, it required a CD25 positive rate of the cells to be greater than 60%. During delivery, the prefabricated carrier-eGFP mRNA complex in Example 11 was separately diluted with a buffer solution and then added to the 24-well cell culture plate with an amount of eGFP-mRNA per well reaching 500 ng/1000 ng, respectively. After incubation for 24 h in an incubator under the conditions of 37°C and 5% CO₂, cell viability and delivery efficiency were determined by flow cytometry. Results are shown in Table 6.

**Table 6 In Vitro Delivery Efficiency of Prefabricated Carrier-Nucleic Acid Complex to Primary T Cells**

| Example | Delivery efficiency (Q3) | Cell viability (Q3 + Q4) |
|---|---|---|
| | 500 ng | 500 ng |
| Example 11 | 84.8% | 96.4% |
| Negative | 0.4% | 98.7% |

The results show that all the prefabricated carrier-eGFP mRNA complexes provided in the present application have high protein expression efficiency in the primary T cells, and, therefore, can be used for delivery into the primary T cells.

The solutions in the present application are not only limited to the technical means disclosed in the aforementioned technical means, but also include technical solutions formed by any combination of the aforementioned technical features. The aforementioned descriptions are specific implementations of the present application. It should be noted that for those of ordinary skill in the art, several improvements and modifications can also be made without departing the principle of the present application. These improvements and modifications are also deemed as the protection scope of the present application.

## Claims

1. Use of a prefabricated carrier in preparation of a product for in vitro gene delivery into immune cells and stem cells, comprising a step of mixing the prefabricated carrier with a nucleic acid in a solvent to obtain a prefabricated carrier-based composition, wherein the prefabricated carrier comprises the following components: 10-60 mol% of a first ionizable lipid, 0-30 mol% of a second ionizable lipid, 5-30 mol% of a phospholipid, 15-70 mol% of a steroid or a derivative thereof, and 0-10 mol% of a PEG-conjugated lipid.

2. The use according to claim 1, wherein the use further comprises a step of contacting the prefabricated carrier-based composition with the immune cells or the stem cells.

3. The use according to claim 2, wherein an adjuvant is also added to the product for in vitro gene delivery into the immune cells and the stem cells.

4. The use according to claim 3, wherein the adjuvant comprises at least one of a lipid, a polypeptide, lipopolyamine or a synthetic polymer.

5. The use according to claim 1, wherein the nucleic acid is DNA and/or RNA.

6. The use according to claim 5, wherein the nucleic acid comprises at least one of siRNA, miRNA, saRNA, sgRNA, dsRNA, shRNA, smRNA, ssRNA, mRNA, circRNA, snRNA, crRNA, IncRNA, snoRNA, piRNA, pDNA, ssDNA, circular or linear DNA, a DNA minicircle and msDNA.

7. The use according to claim 1, wherein an amount of the nucleic acid is 0.5%-50% (w/w) of a total amount of the prefabricated carrier and the nucleic acid; and in the prefabricated carrier-based composition, a concentration of the nucleic acid is 5-1000 ng/µl.

8. The use according to claim 1, wherein the first ionizable lipid is selected from a compound of General formula (1), or a pharmaceutically acceptable salt, stereoisomer or tautomer thereof, 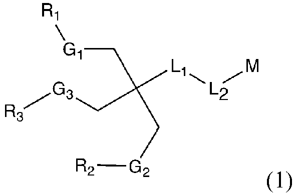
wherein R₁, R₂, and R₃ are each independently H, C₅₋₄₀ straight-chain or branched alkyl, C₅₋₄₀ straight-chain or branched alkenyl, C₅₋₄₀ straight-chain or branched alkynyl, 3-6 membered saturated or partially unsaturated cyclic hydrocarbyl containing 1-3 side chains, or a 6-10 membered aromatic group containing 1-3 side chains; the side chain is independently selected from C₁₀₋₃₀ straight-chain or branched alkyl, C₁₀₋₃₀ straight-chain or branched alkenyl, and C₁₀₋₃₀ straight-chain or branched alkynyl; provided that at most one of R₁, R₂, and R₃ is H;
M is selected from -NR₄R₅, saturated or partially unsaturated 3-6 membered heterocyclyl containing at least one nitrogen atom and 6-10 membered heteroaryl containing at least one nitrogen atom, and the heterocyclyl and the heteroaryl are unsubstituted or substituted with one or more -OH, carboxyl, amino, oxo or halogen;
R₄ and R₅ are each independently H, C₁₋₆ straight-chain or branched alkyl, C₂₋₆ straight-chain or branched alkenyl, or C₂₋₆ straight-chain or branched alkynyl, and the C₁₋₆ straight-chain or branched alkyl, the C₂₋₆ straight-chain or branched alkenyl, or the C₂₋₆ straight-chain or branched alkynyl is unsubstituted or substituted with one or more -OH, carboxyl, amido, amidino, guanidino or halogen;
G₁, G₂, and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)-, or -OP(=O)(OR₆)O-; wherein R₆ and R₁₃ are each independently selected from H, hydroxyl, C₁₋₃₀ straight-chain or branched alkyl or cycloalkyl, and C₂₋₃₀ straight-chain or branched alkenyl;
L₁ is selected from -X₁- or -(CR₇R₈)m-X₁-, wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, - SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)-, or -OP(=O)(OR₁₄)O-; wherein m is an integer of 2-6, R₇ and R₈ are each independently H, hydroxyl, halogen, C₁₋₆ straight-chain or branched alkyl or cycloalkyl, or C₂₋₆ straight-chain or branched alkenyl, and R₁₄ and R₁₅ are each independently selected from H, the C₁₋₃₀ straight-chain or branched alkyl or cycloalkyl, and the C₂₋₃₀ straight-chain or branched alkenyl;
L₂ is -(CR₉R₁₀)ₙ- or-(CR₉R₁₀)ₙ-X₂-(CR₁₁R₁₂)ₖ-, wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, - SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, - P(=O)(OR₁₆)O-, - OP(=O)(OR₁₆)-, or -OP(=O)(OR₁₆)O-; n is an integer of 1-6; k is an integer of 1-6; R₉, R₁₀, R₁₁, and R₁₂ are each independently H, the hydroxyl, the halogen, the C₁₋₆ straight-chain or branched alkyl or cycloalkyl, or the C₂₋₆ straight-chain or branched alkenyl; and R₁₆ and R₁₇ are each independently selected from H, the C₁₋₃₀ straight-chain or branched alkyl or cycloalkyl, and the C₂₋₃₀ straight-chain or branched alkenyl;
wherein the alkyl, the cycloalkyl, and the alkenyl in R₄-R₁₇ are unsubstituted or substituted with one or more groups selected from the hydroxyl, sulfydryl, the amino, substituted amino, and the halogen; and
the salt does not comprise quaternary ammonium salts.

9. The use according to claim 8, wherein R₁, R₂, and R₃ are each independently the following groups: 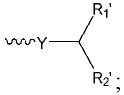 wherein Y is absent, or the C₁₋₃₀ straight-chain or branched alkyl or cycloalkyl, the C₂₋₂₀ straight-chain or branched alkenyl, or the C₂₋₂₀ straight-chain or branched alkynyl; R₁' and R₂' are each independently H, the C₁₋₃₀ straight-chain or branched alkyl, the C₂₋₃₀ straight-chain or branched alkenyl, or the C₂₋₃₀ straight-chain or branched alkynyl, and a total carbon chain length of Y, R₁', and R₂' is 8-40.

10. The use according to claim 8, wherein R₁, R₂, and R₃ are each independently selected from the following groups: 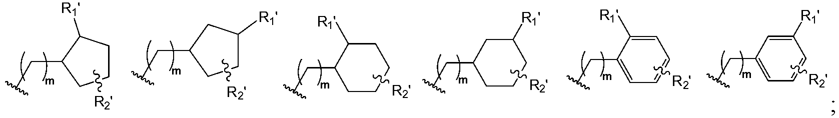 wherein R₁' and R₂' are each independently H, the C₁₋₃₀ straight-chain or branched alkyl, the C₂₋₃₀ straight-chain or branched alkenyl, or the C₂₋₃₀ straight-chain or branched alkynyl, and a total carbon chain length of R₁' and R₂' is 8-30.

11. The use according to claim 8, wherein R₁, R₂, and R₃ are each independently selected from any one of the following groups:

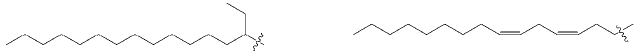

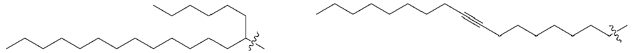

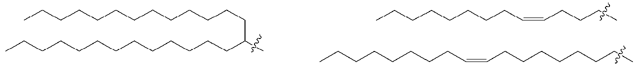

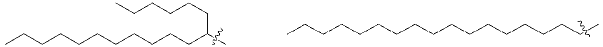

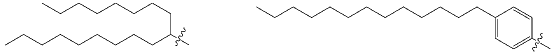

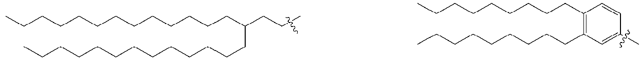

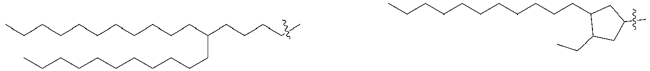

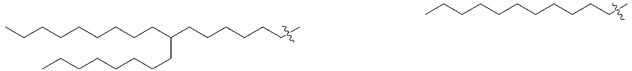

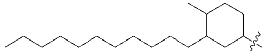

12. The use according to claim 8, wherein G₁, G₂, and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)-, or -OP(=O)(OR₆)O-.

13. The use according to claim 8, wherein L₁ is selected from-(CR₇R₈)ₘ-X₁-, wherein X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)-, or -OP(=O)(OR₁₄)O-.

14. The use according to claim 8, wherein L₂ is -(CR₉R₁₀)ₙ-X₂-(CR₁₁R₁₂)ₖ-, wherein X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)-, or -OP(=O)(OR₁₆)O-.

15. The use according to claim 8, wherein M is selected from the following structures: 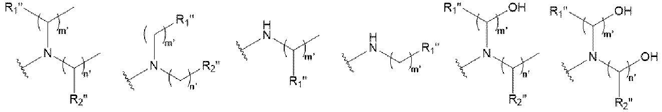 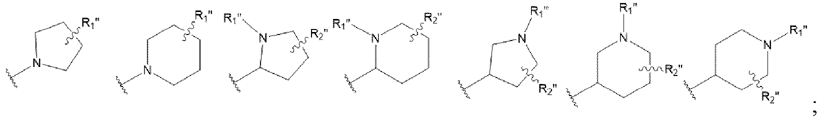 wherein m' and n' are each independently integers of 0-6; and R₁" and R₂" are each independently H, C₁₋₆ alkyl, C₂₋₆ alkenyl, the guanidino, the amidino, the amido, aliphatic amino or 3-10 membered nitrogen-containing heterocyclyl; the nitrogen-containing heterocyclyl is selected from pyrrolyl, imidazolyl, pyridyl, pyrazolyl, triazolyl, oxazolyl, isoxazolyl, thienyl, isothiazolyl, pyridazinyl, pyrazinyl, piperazinyl, indolyl, benzimidazolyl, carbazolyl, quinolyl, isoquinolyl, purinyl, pyrimidinyl, and tautomeric forms thereof, and are unsubstituted or optionally substituted with one or more organic groups selected from the hydroxyl, the sulfydryl, the amino, the substituted amino, the halogen, the C₁₋₆ alkyl, the C₂₋₆ alkenyl, C₂₋₆ alkynyl, and C₆₋₁₄ aryl.

16. The use according to claim 8, wherein the compound of Formula (1) is selected from compounds of Formula (1A):

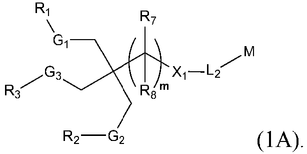

17. The use according to claim 8, wherein the compound of Formula (1) is selected from compounds of Formula (1B):

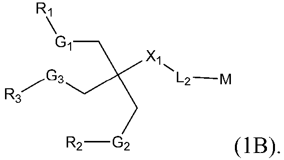

18. The use according to claim 8, wherein the compound of Formula (1) is selected from compounds of Formula (1C):

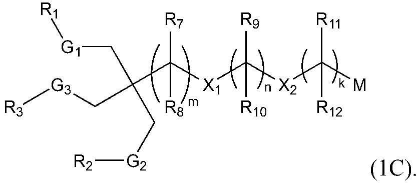

19. The use according to claim 17, wherein the compound of Formula (1) is selected from compounds of Formula (1D):

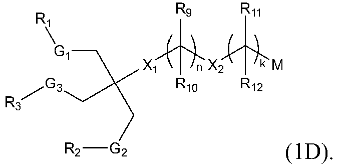

20. The use according to claim 19, wherein the compound of Formula (1) is selected from compounds of Formula (1E):

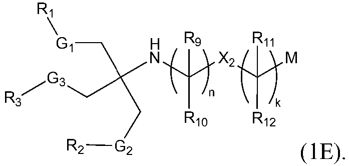

21. The use according to claim 20, wherein the compound of Formula (1) is selected from compounds of Formula (1F):

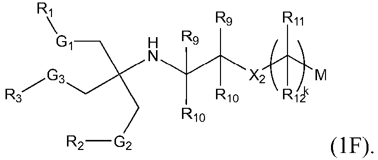

22. The use according to claim 21, wherein the compound of Formula (1) is selected from compounds of Formula (1G):

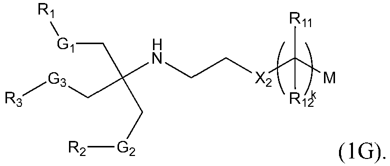

23. The use according to claim 21, wherein the compound of Formula (1) is selected from compounds of Formula (1H):

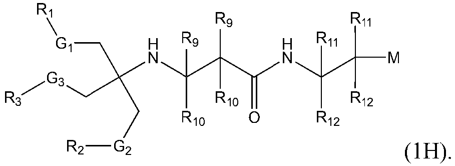

24. The use according to claim 8, wherein the compound of Formula (1) is selected from compounds of Formula (11):

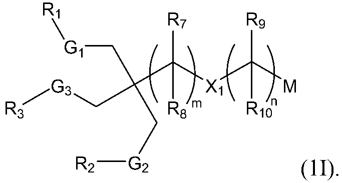

25. The use according to claim 17, wherein the compound of Formula (1) is selected from compounds of Formula (1J):

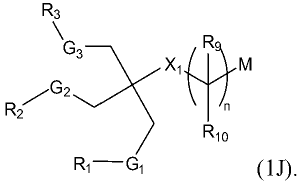

26. The use according to claim 24, wherein the compound of Formula (1) is selected from compounds of Formula (1K):

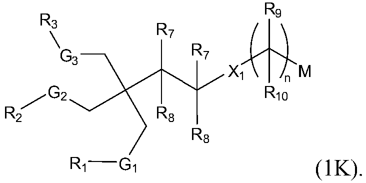

27. The use according to claim 9, wherein Y is absent, and the compound of Formula (1) is selected from compounds of Formula (1L): 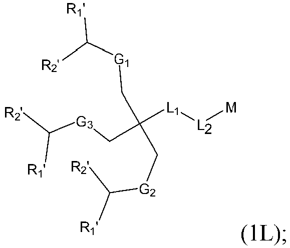 wherein R₁' and R₂' are independently selected from H, the C₁₋₃₀ straight-chain or branched alkyl, the C₂₋₃₀ straight-chain or branched alkenyl, and the C₂₋₃₀ straight-chain or branched alkynyl, and the total carbon chain length of R₁' and R₂' is 8-40.

28. The use according to claim 9, wherein the compound of Formula (1) is selected from compounds of Formula (1M):

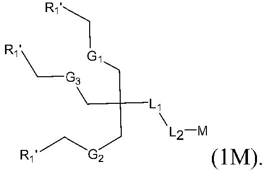

29. The use according to any one of claims 1 to 28, wherein the compound of Formula (1) is selected from:

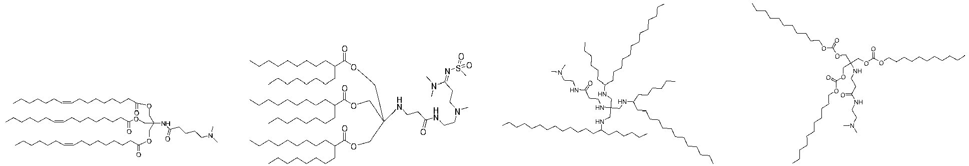

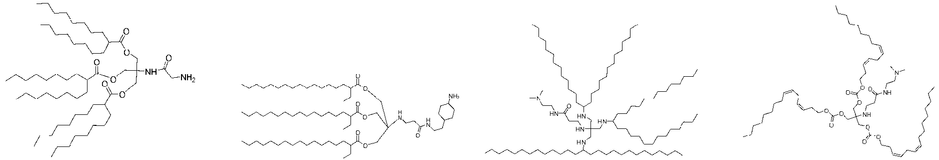

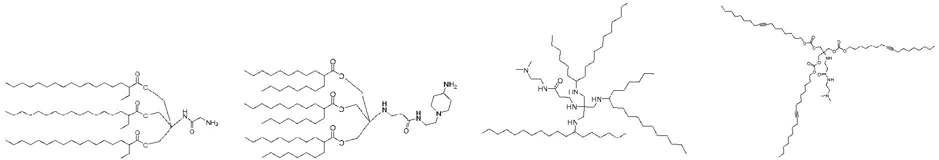

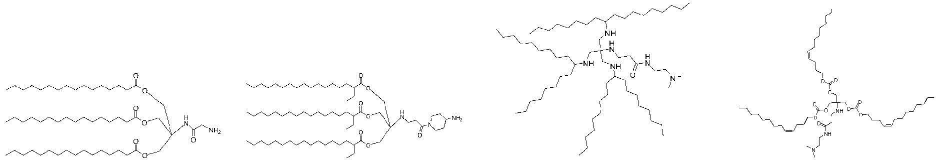

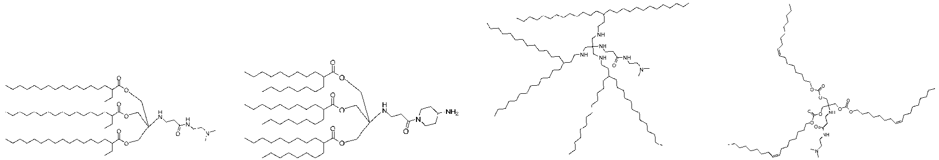

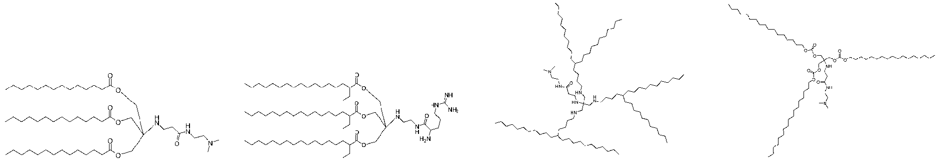

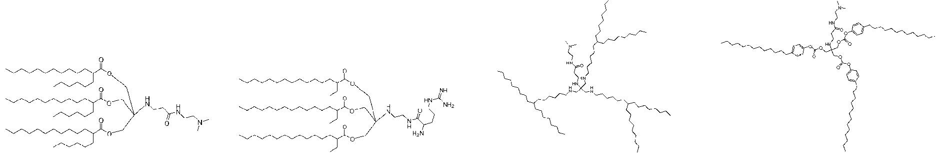

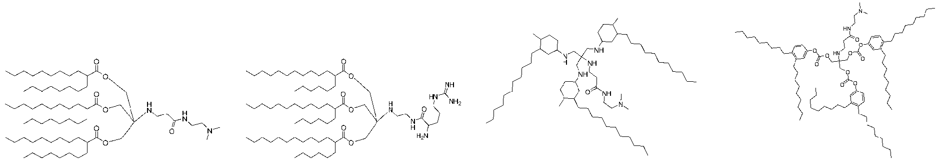

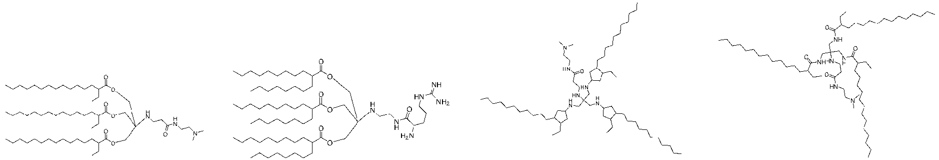

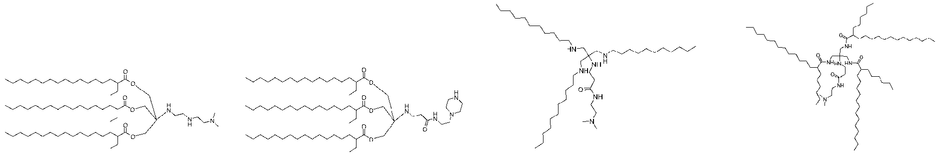

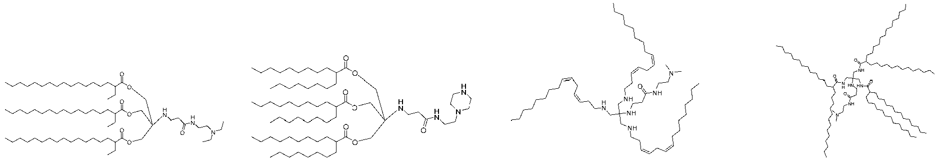

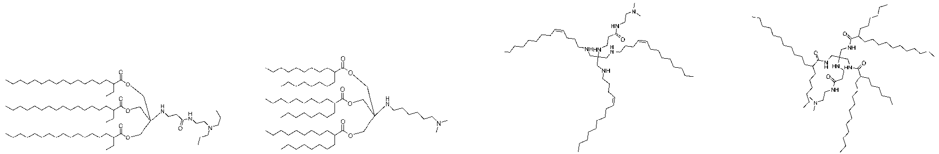

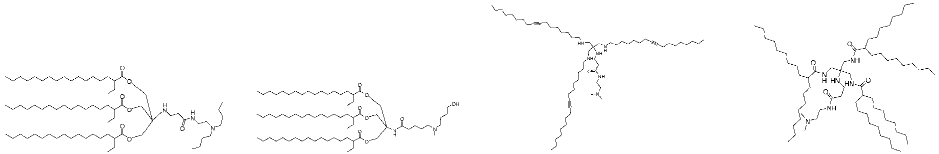

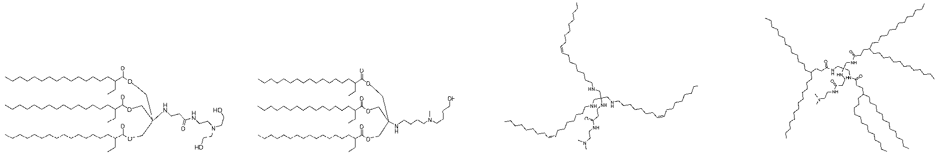

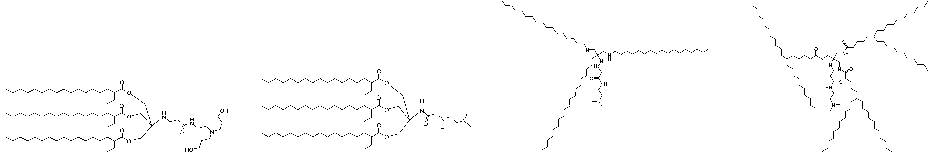

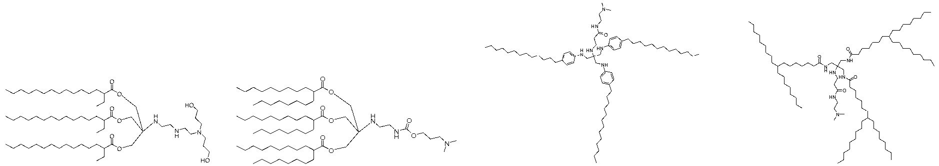

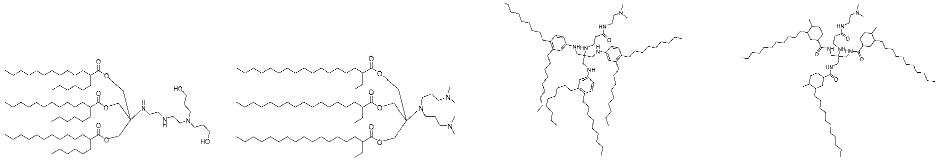

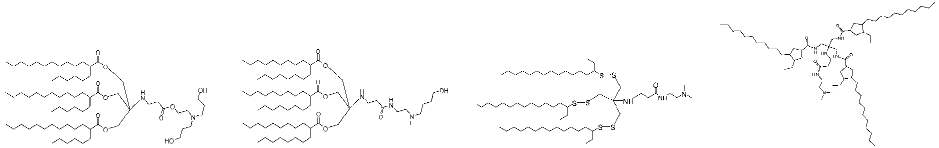

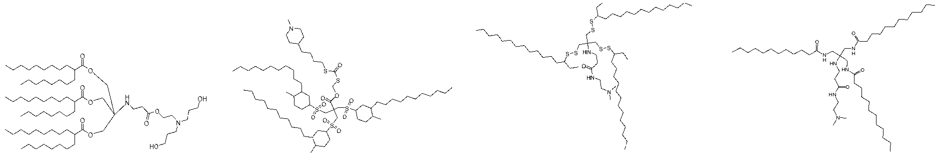

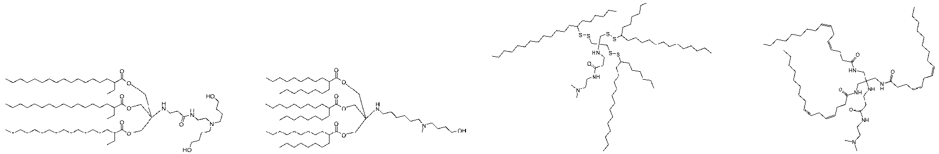

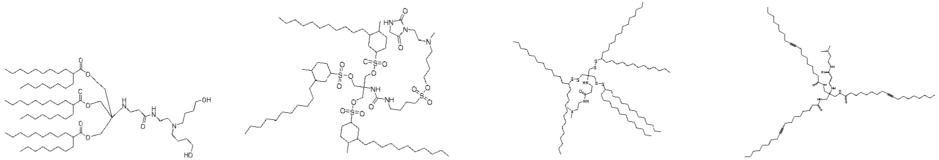

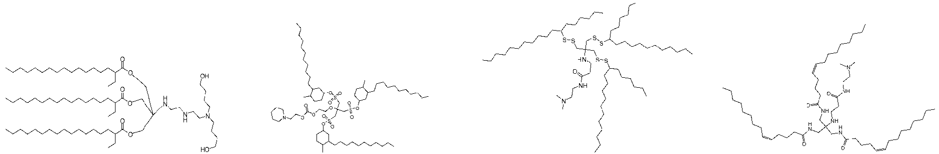

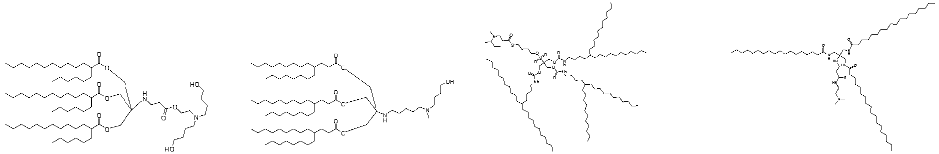

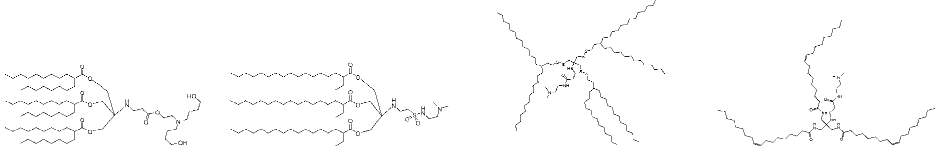

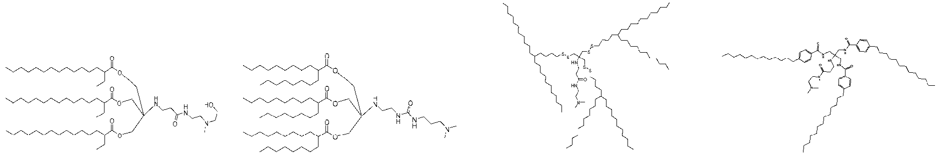

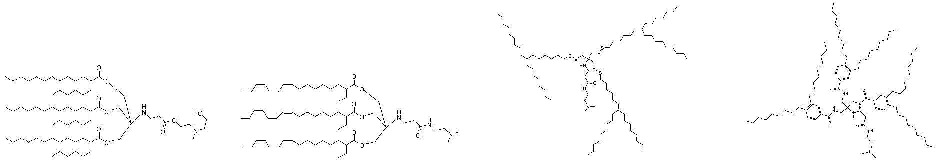

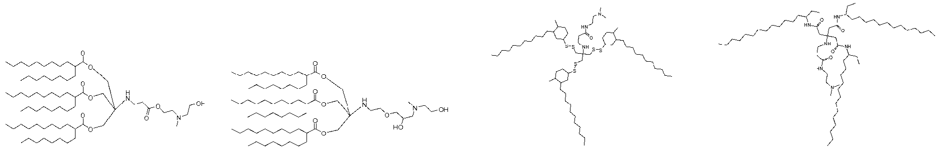

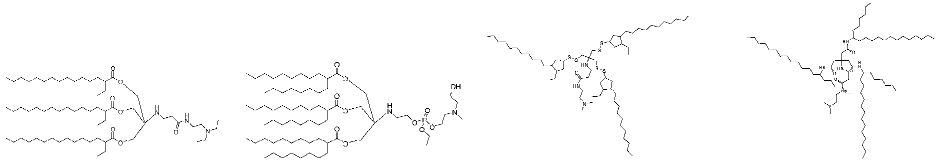

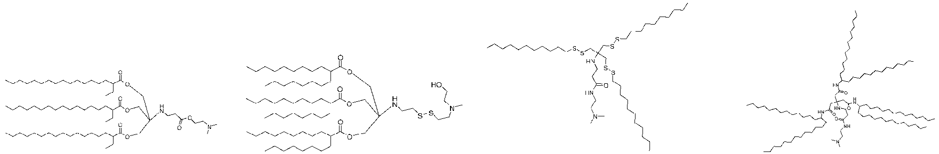

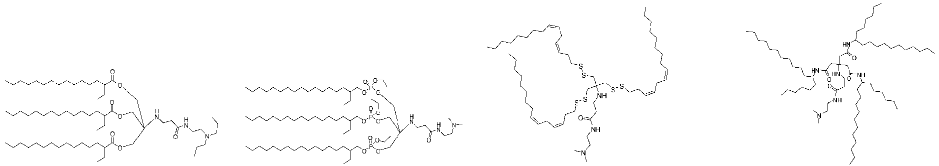

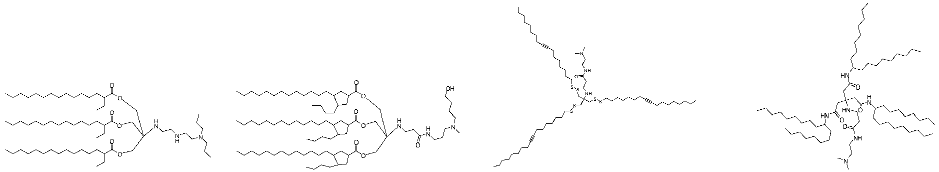

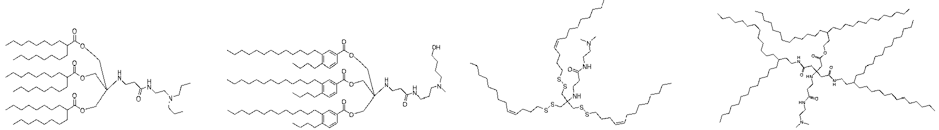

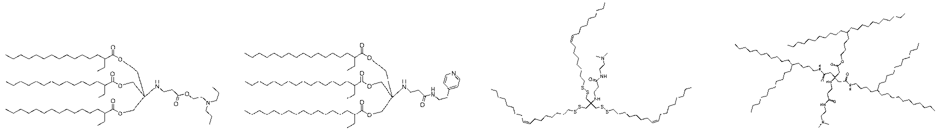

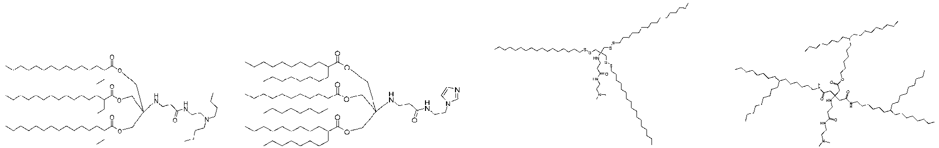

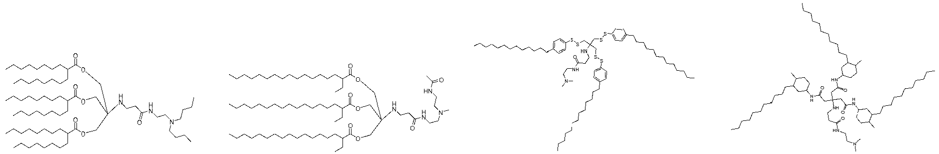

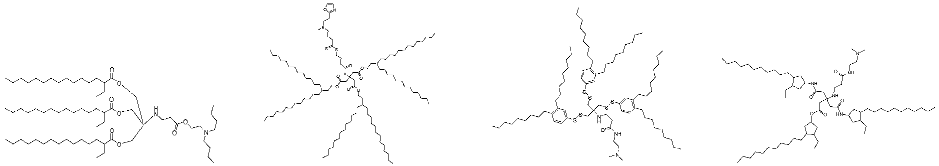

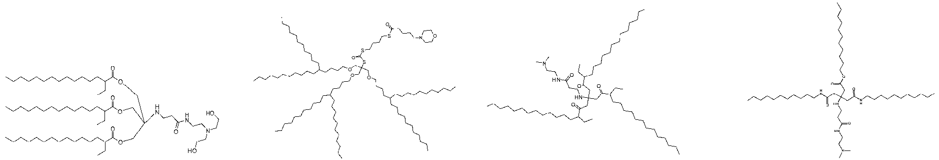

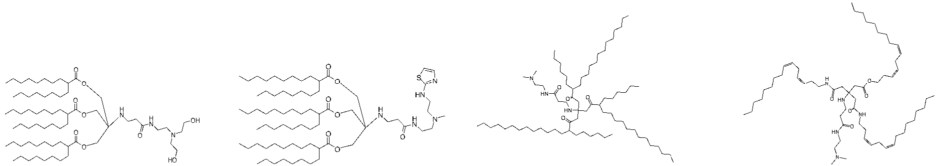

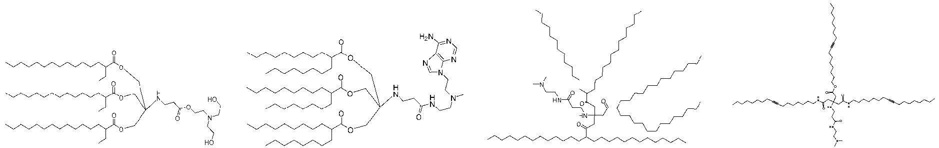

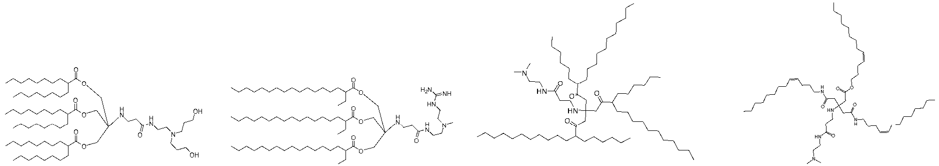

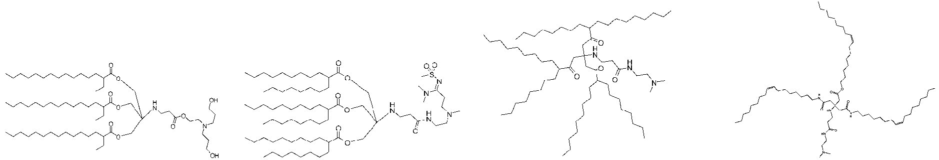

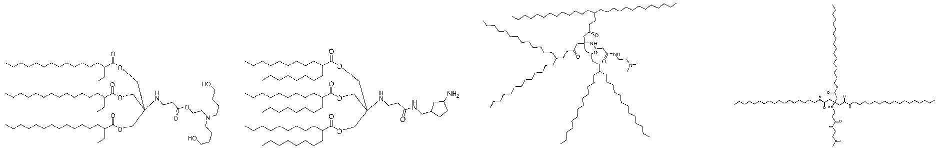

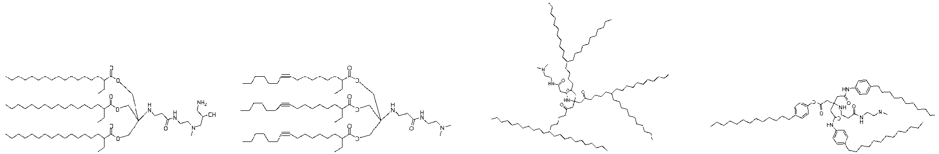

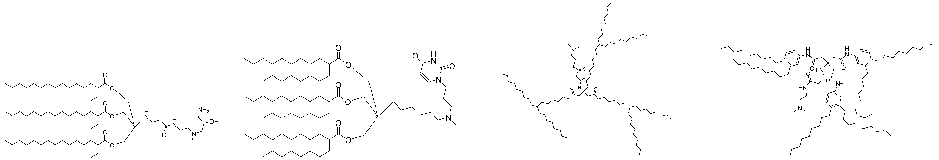

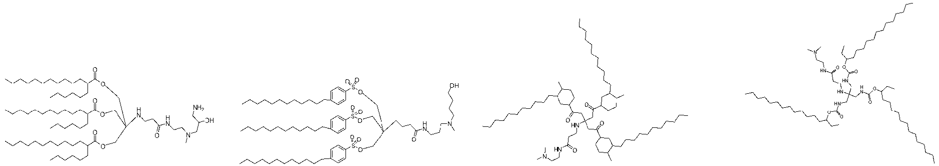

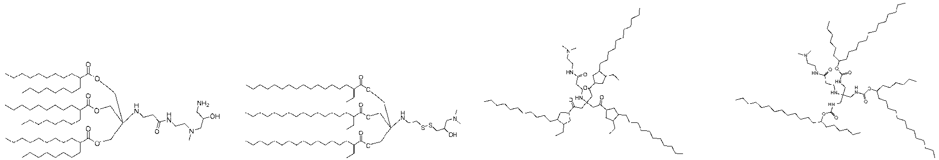

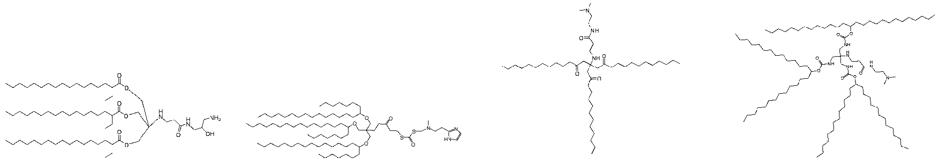

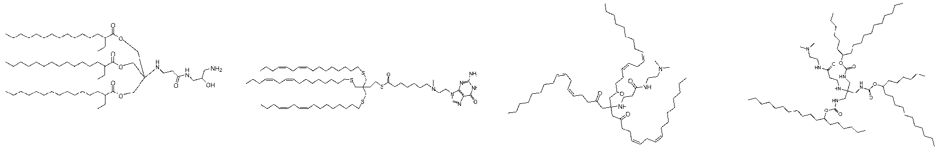

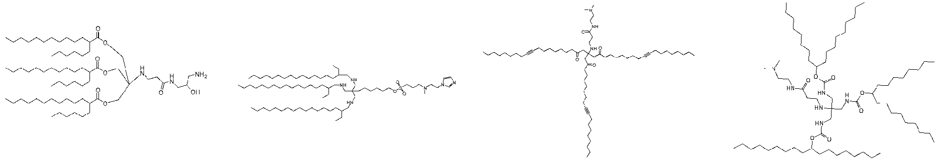

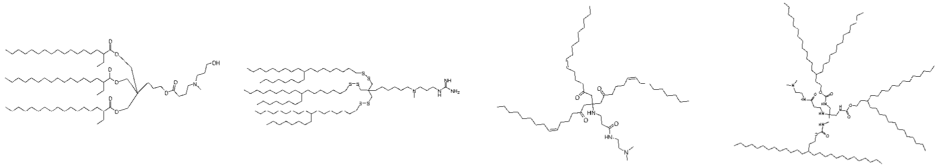

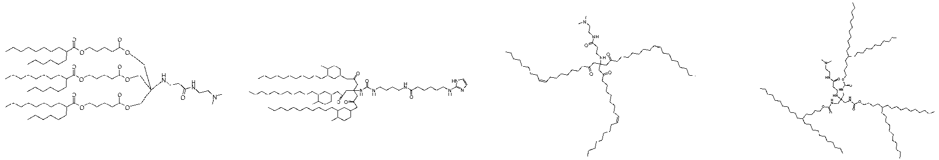

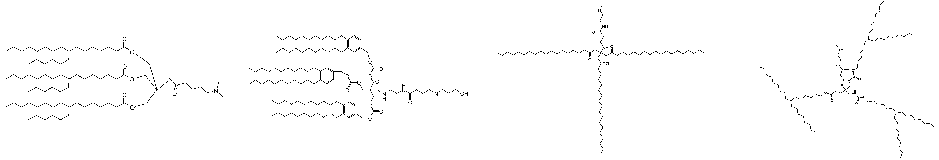

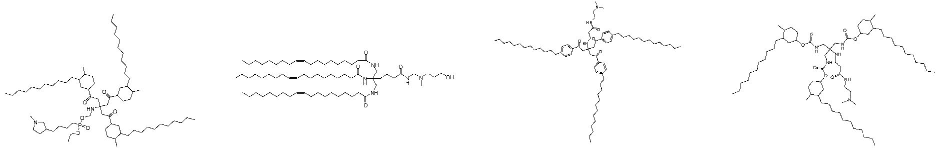

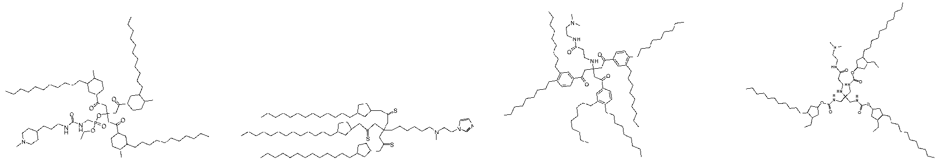

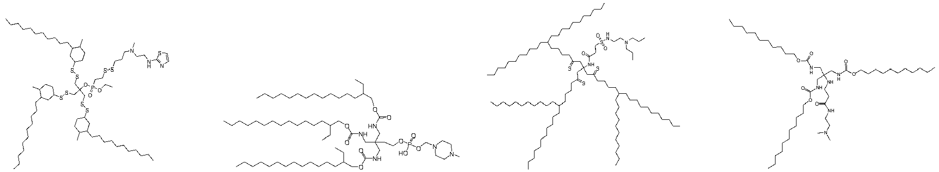

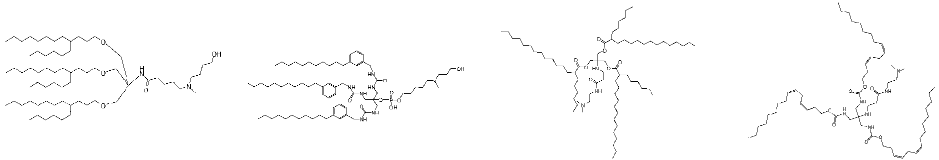

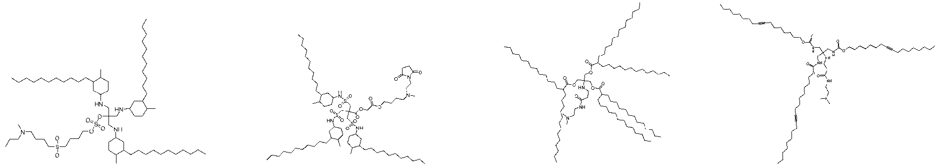

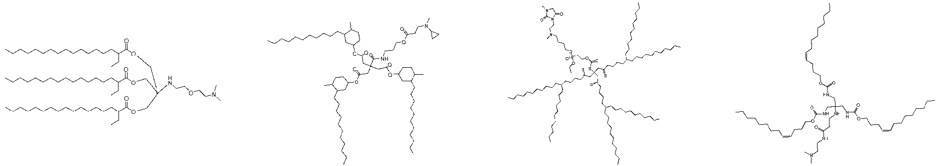

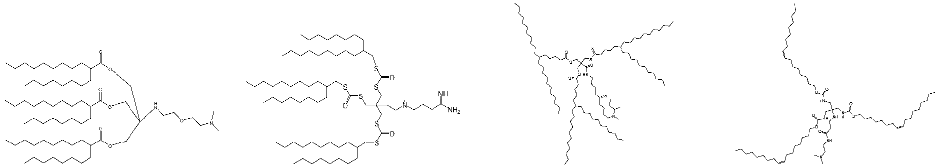

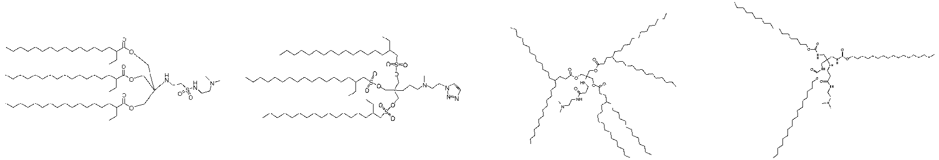

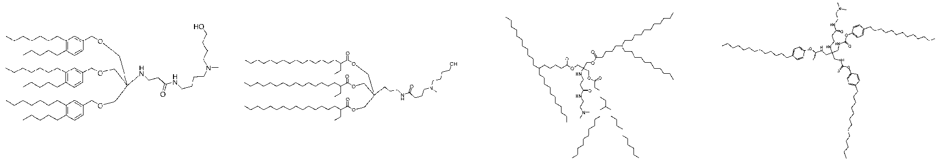

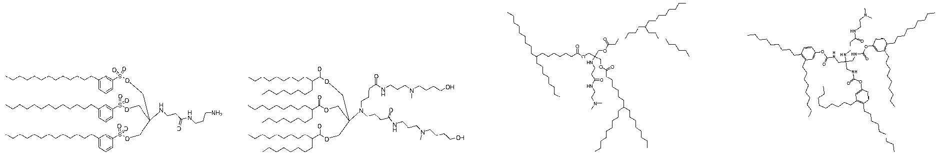

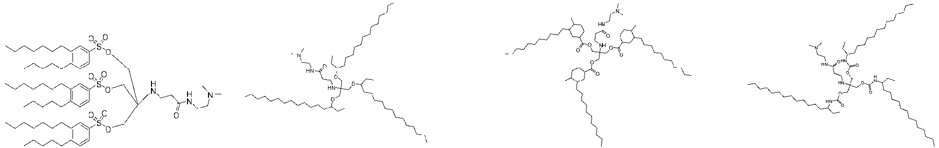

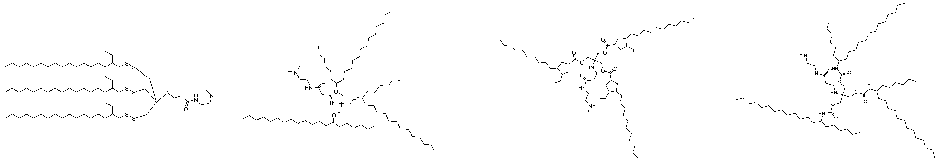

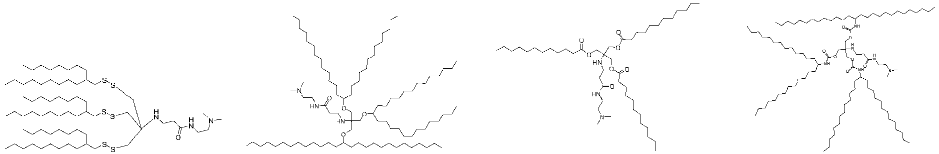

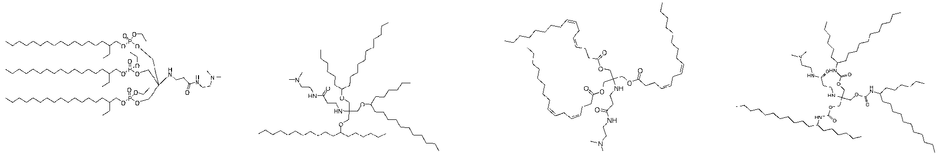

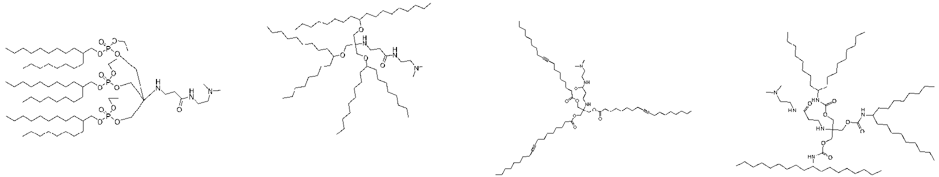

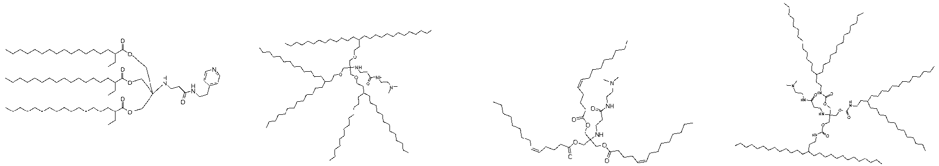

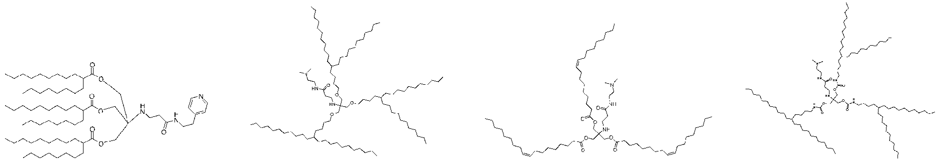

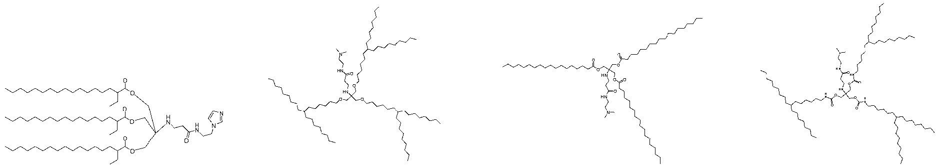

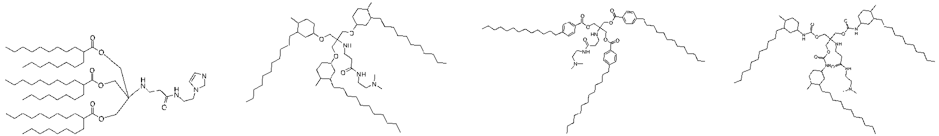

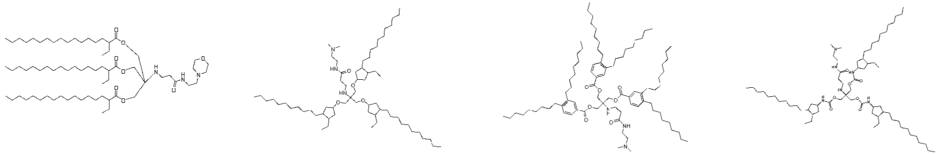

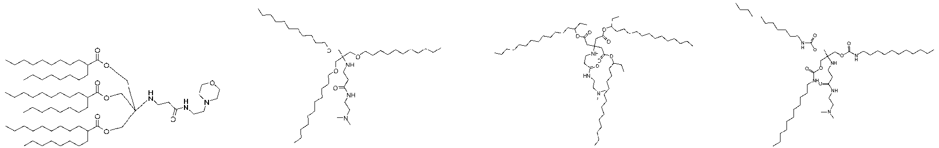

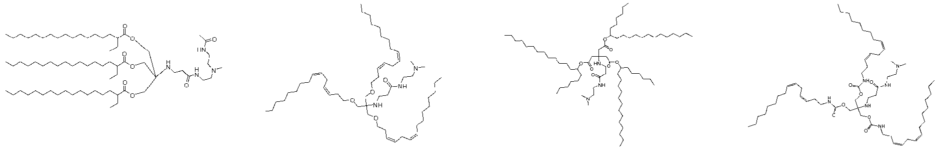

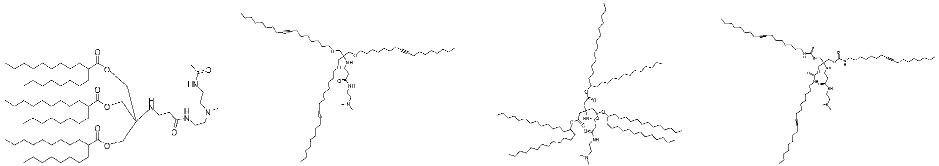

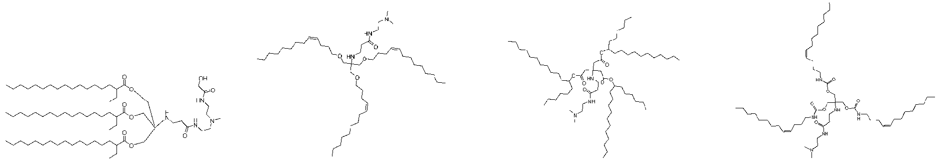

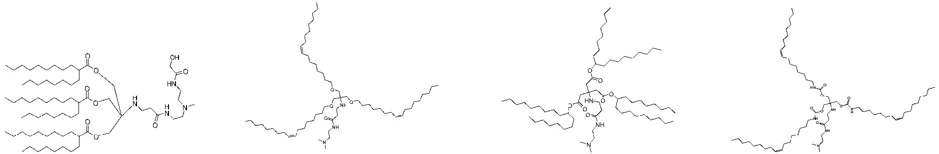

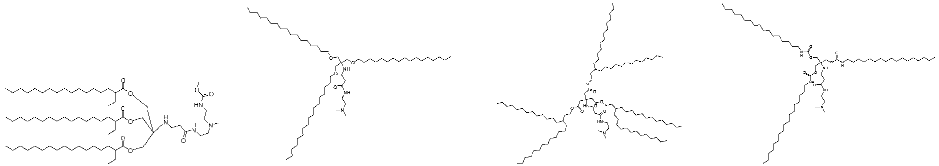

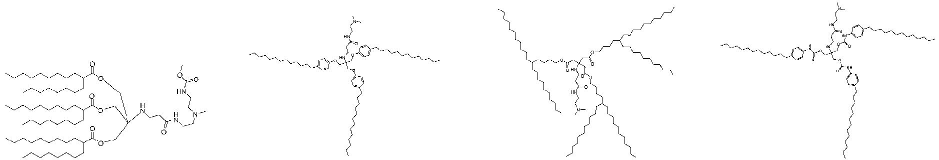

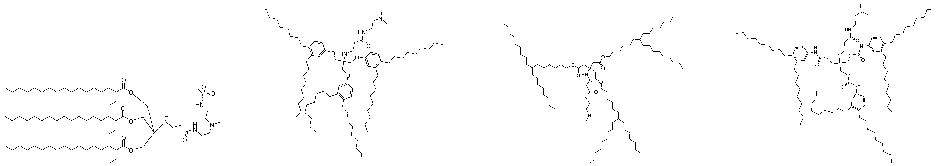

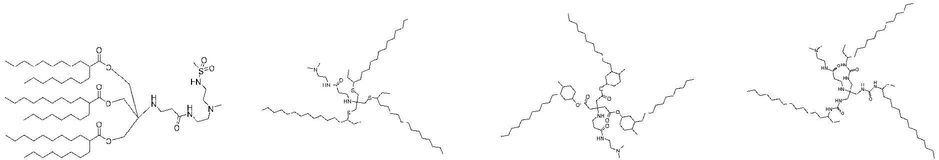

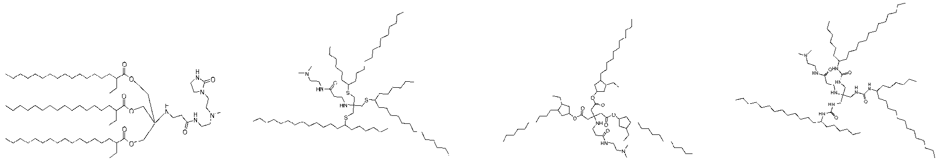

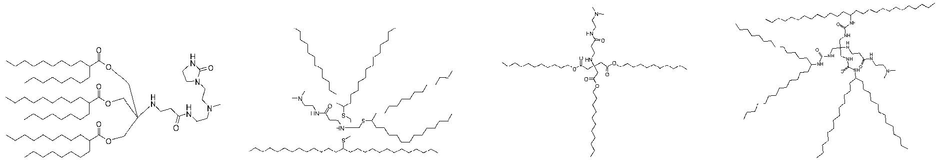

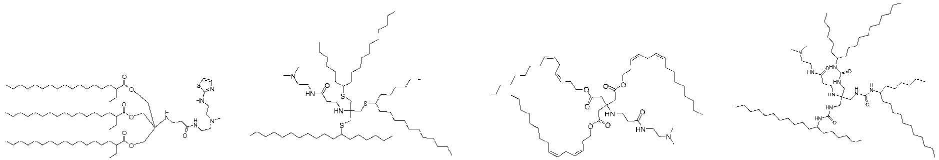

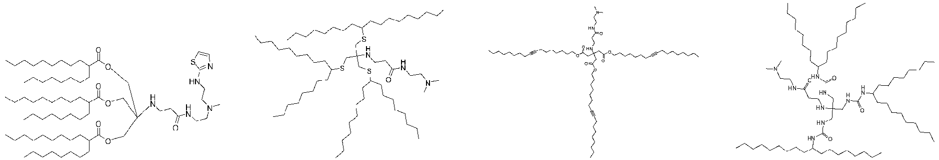

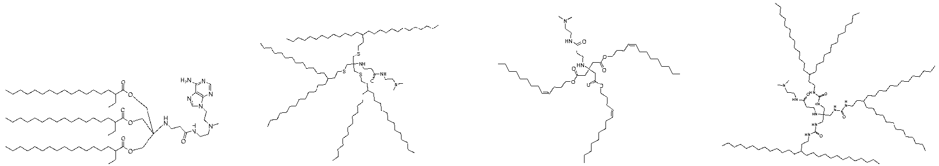

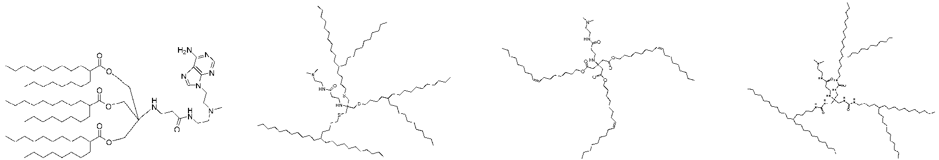

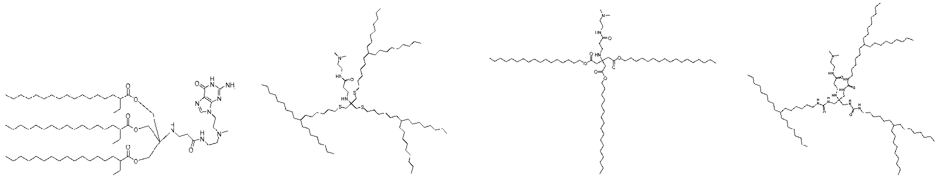

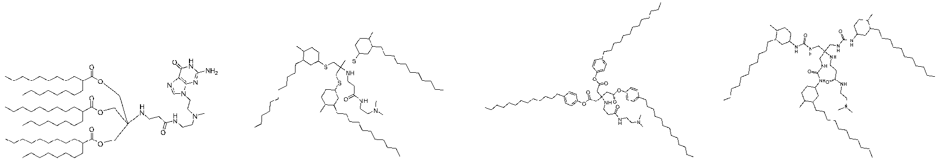

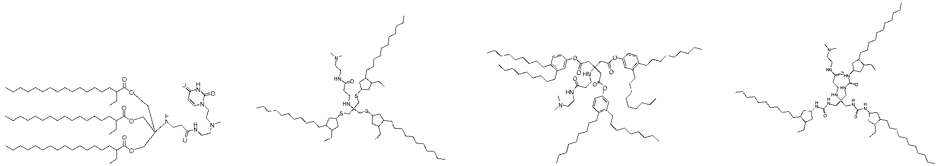

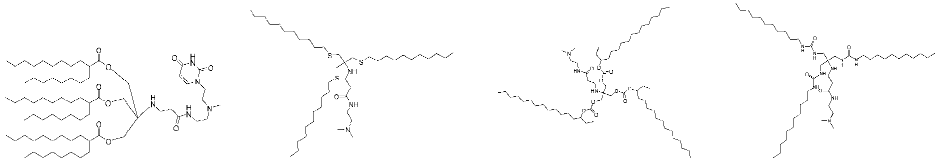

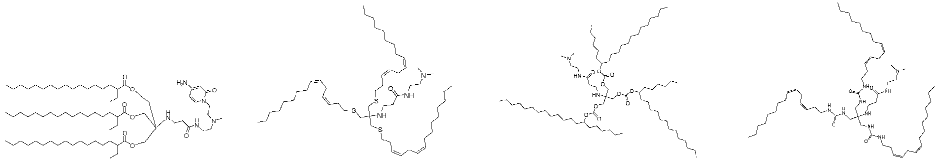

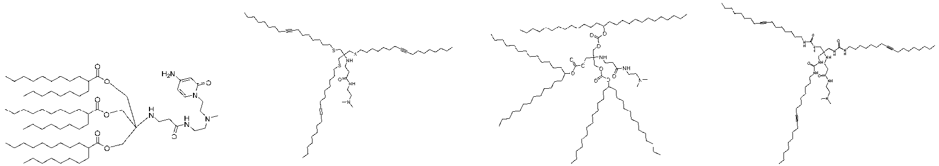

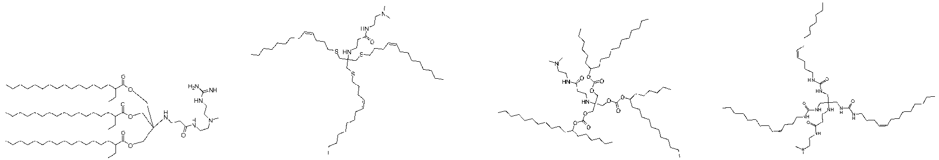

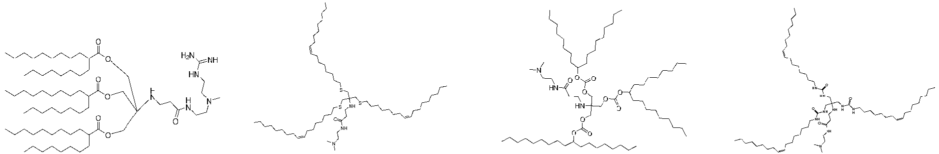

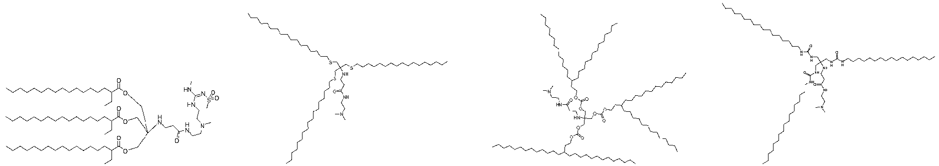

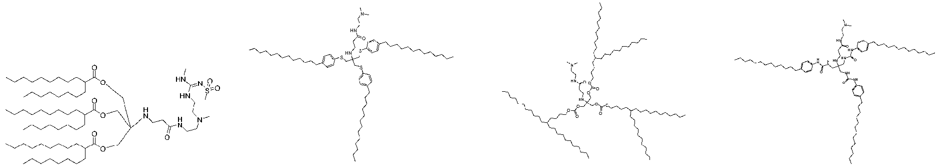

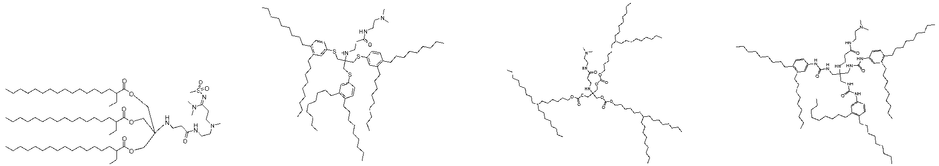

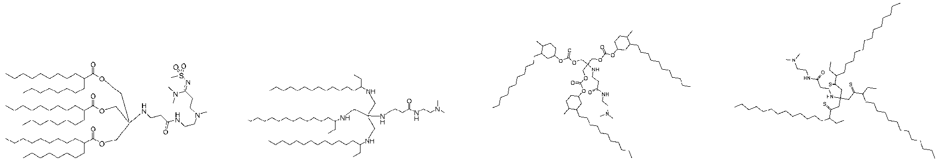

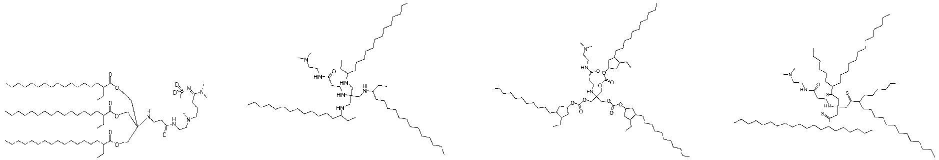

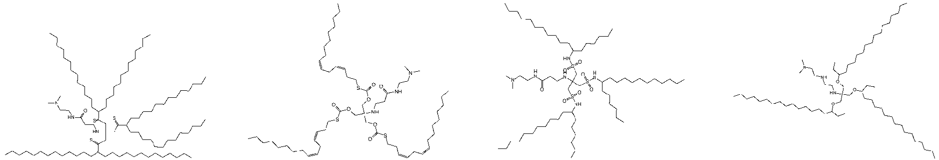

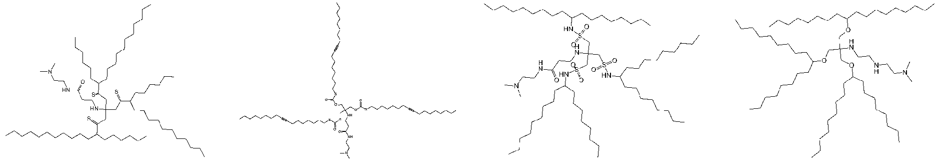

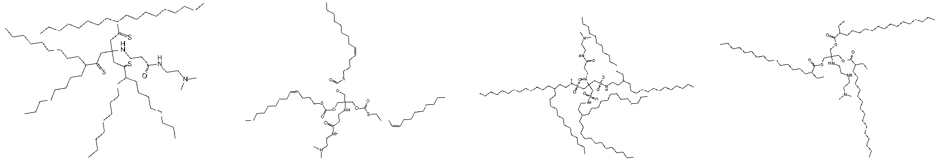

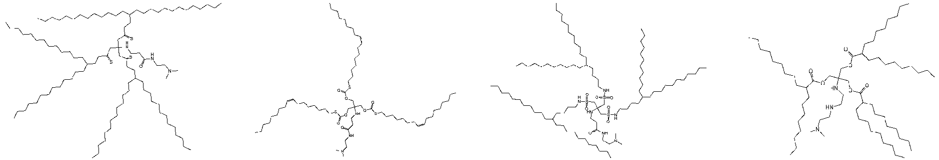

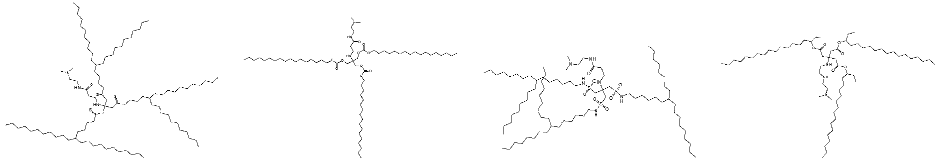

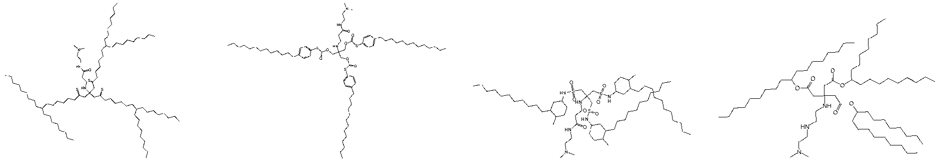

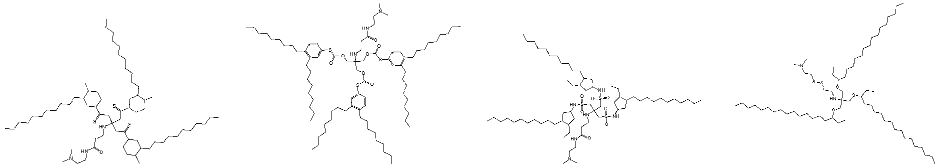

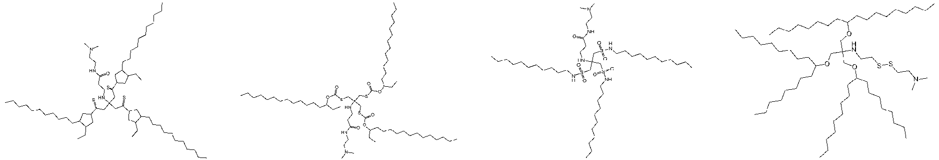

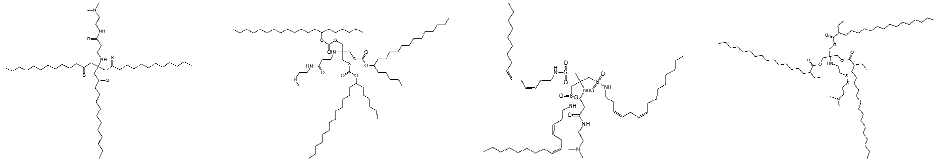

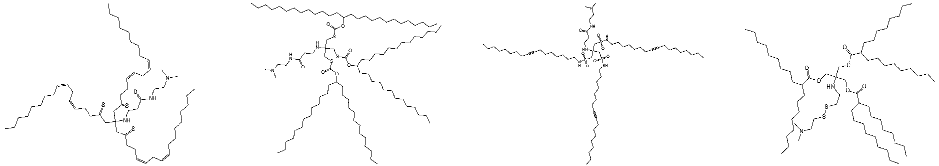

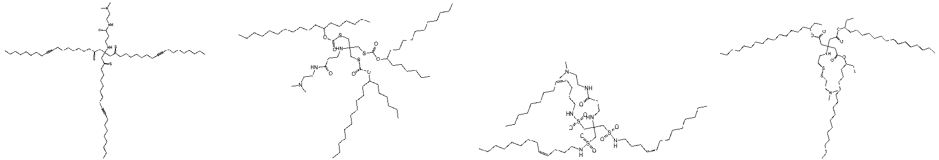

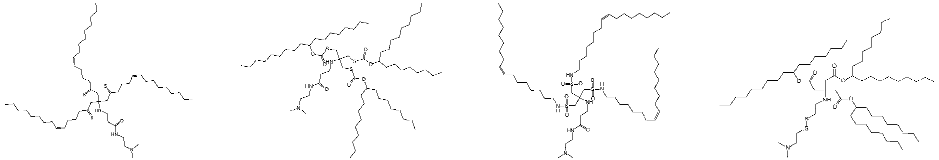

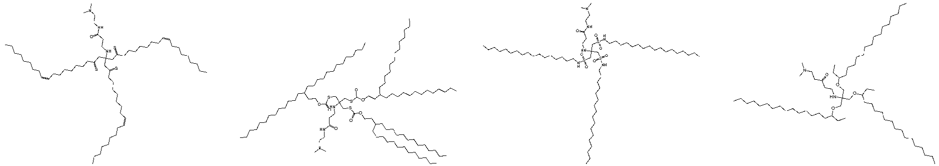

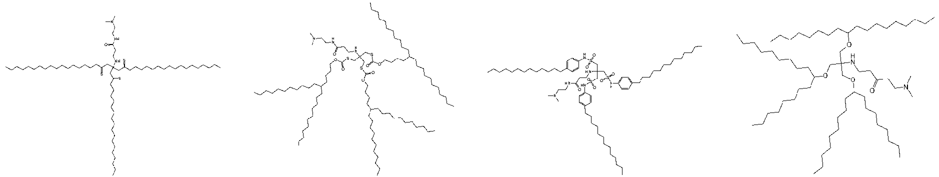

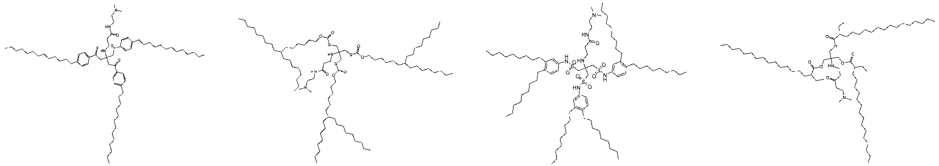

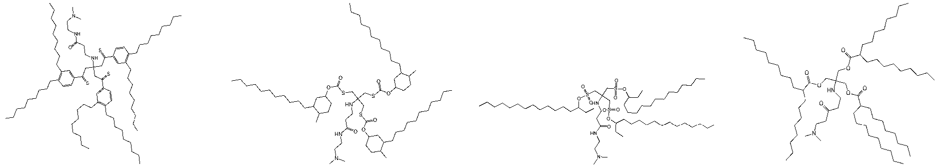

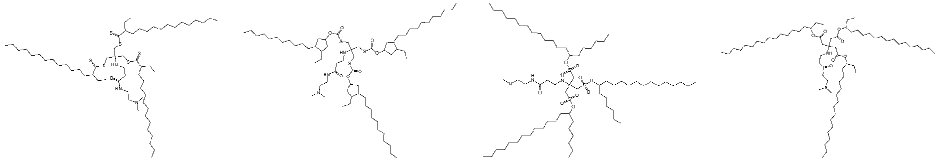

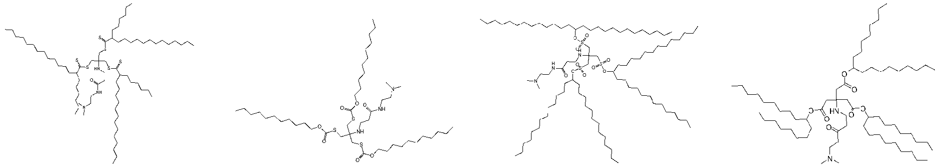

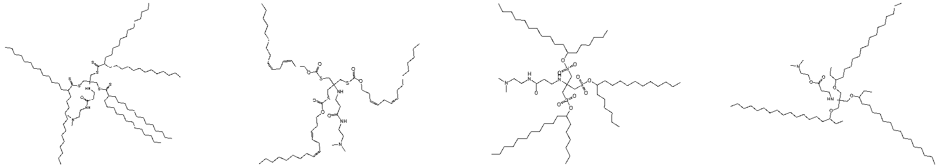

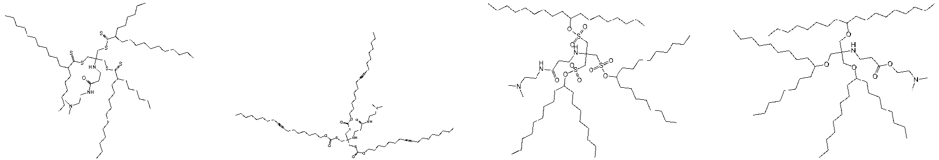

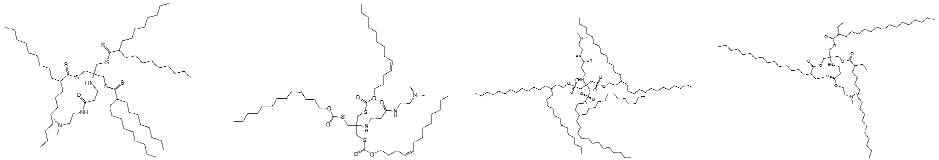

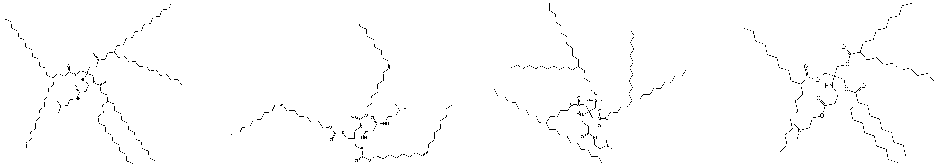

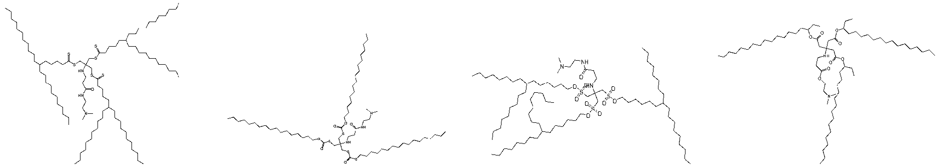

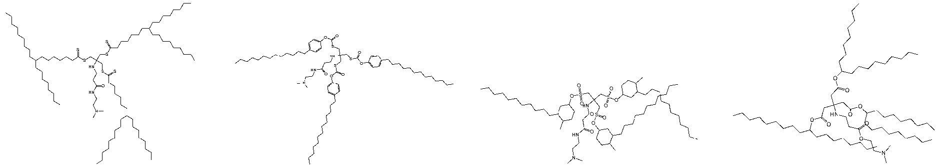

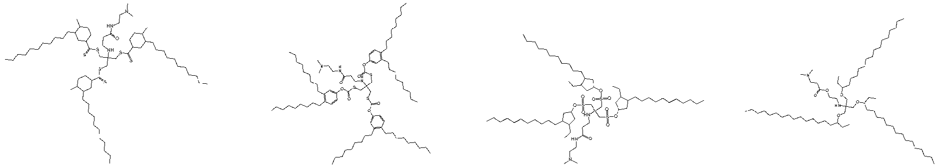

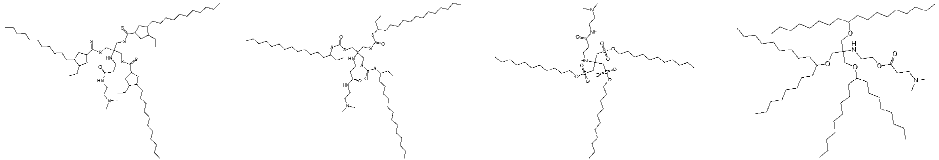

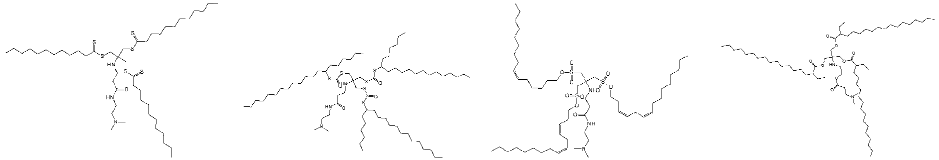

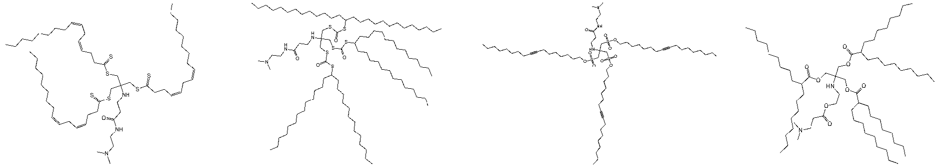

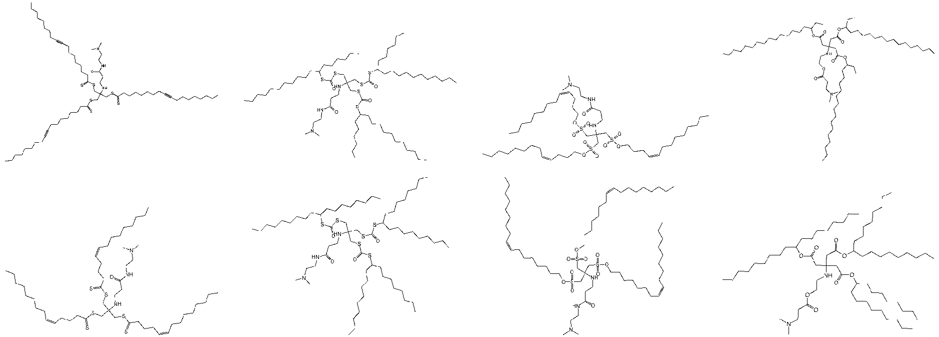

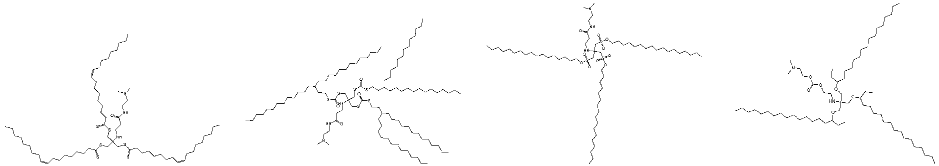

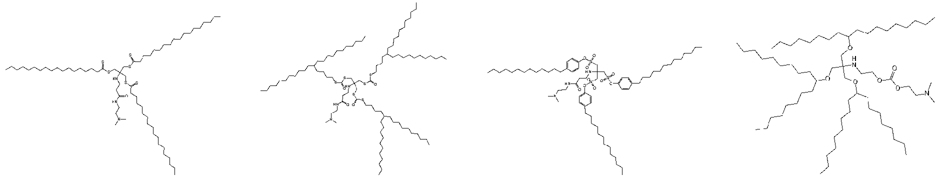

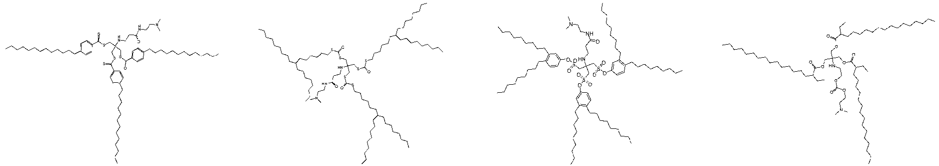

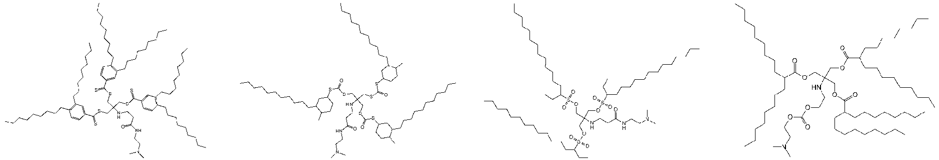

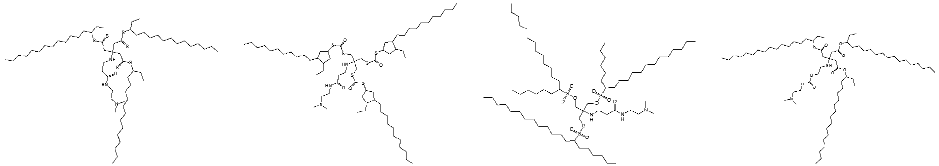

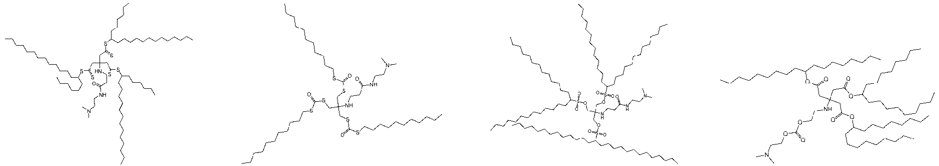

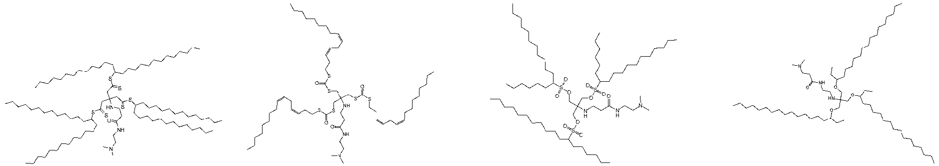

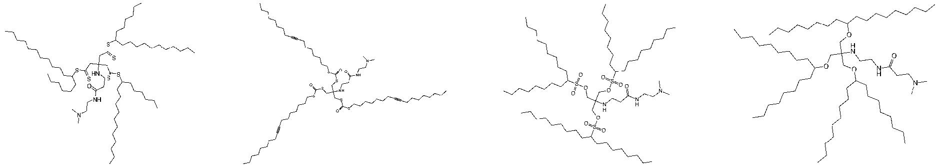

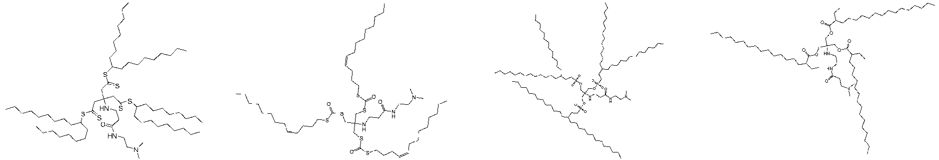

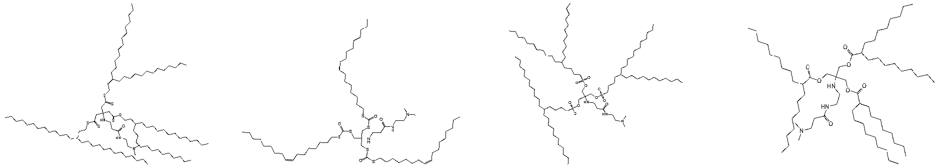

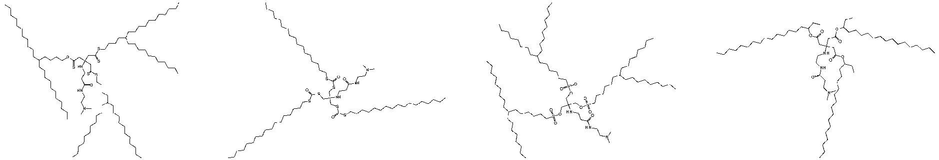

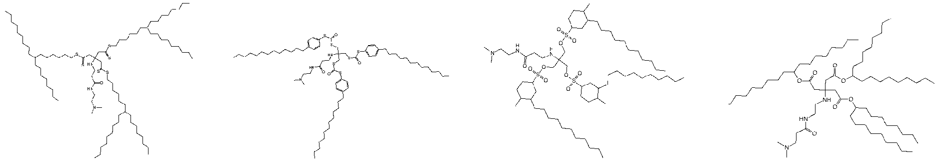

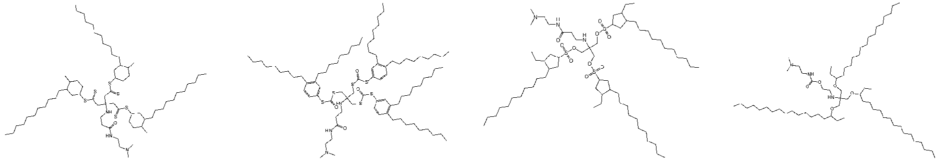

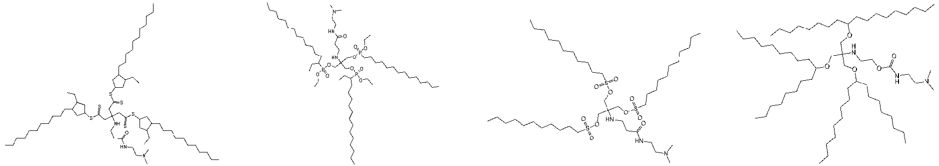

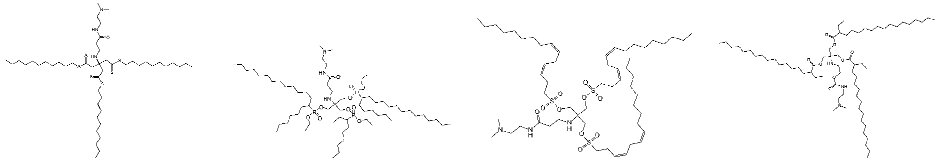

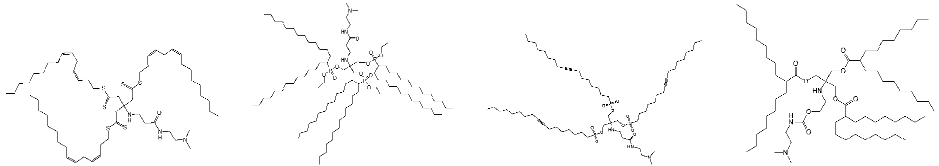

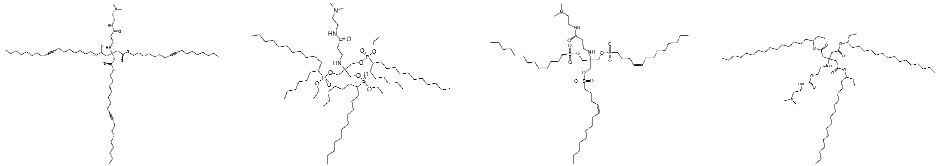

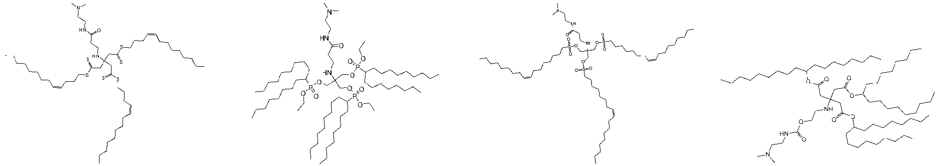

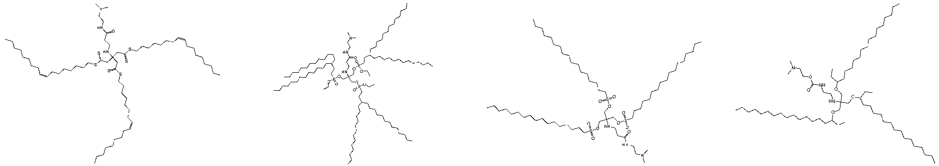

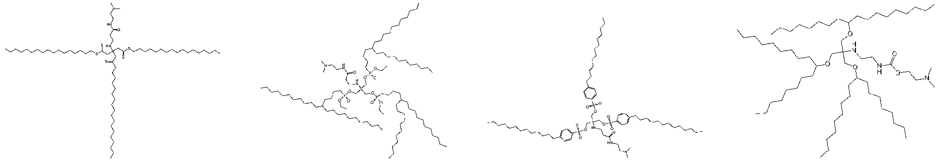

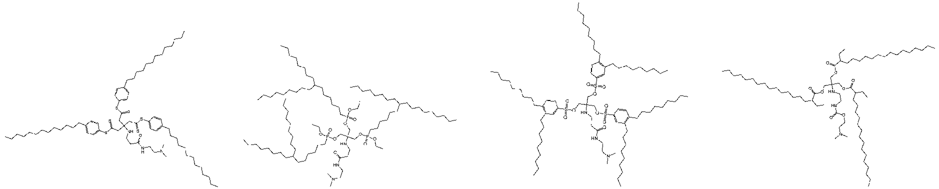

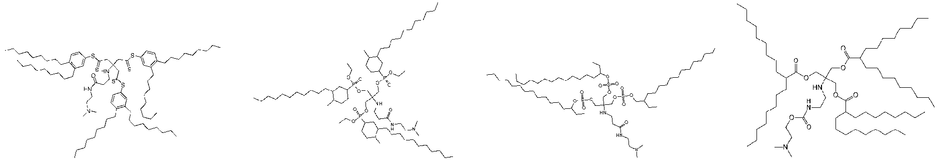

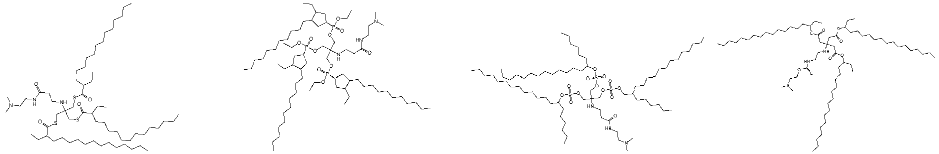

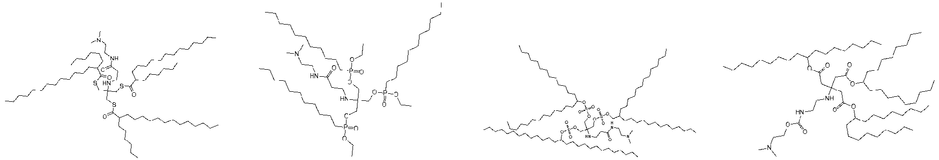

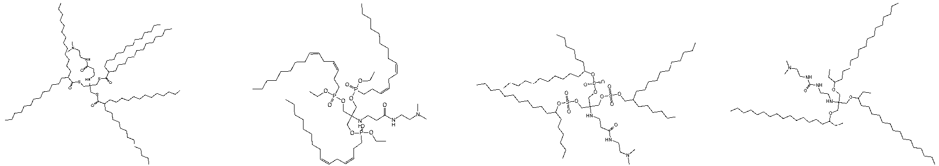

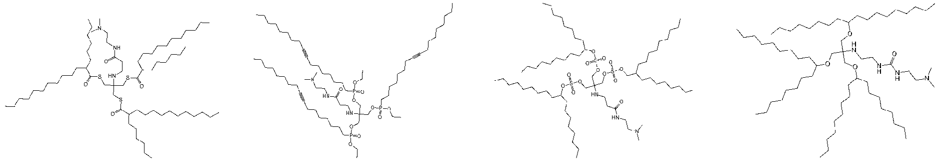

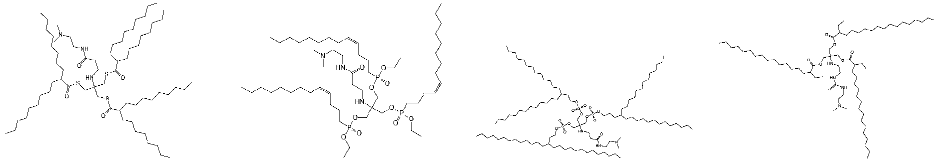

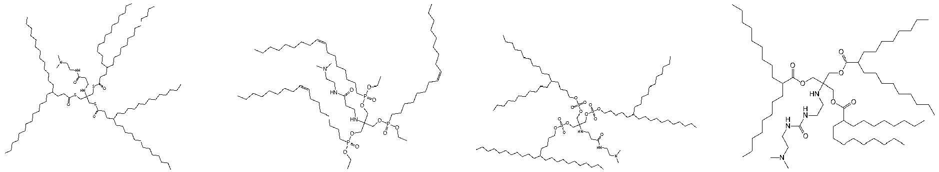

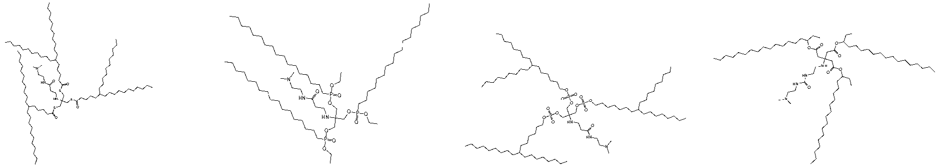

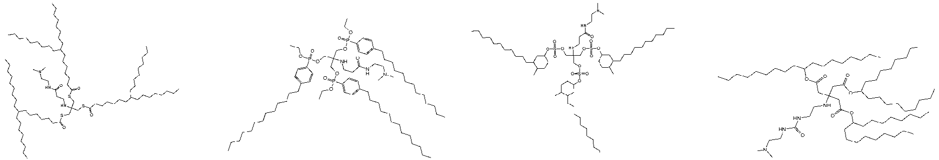

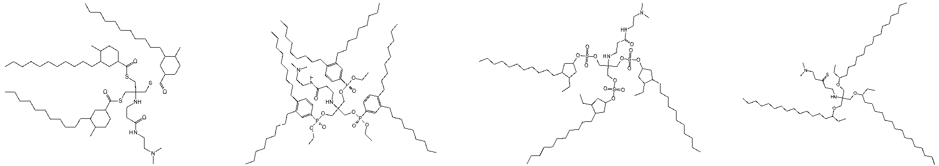

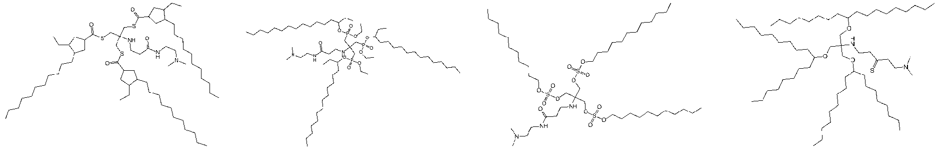

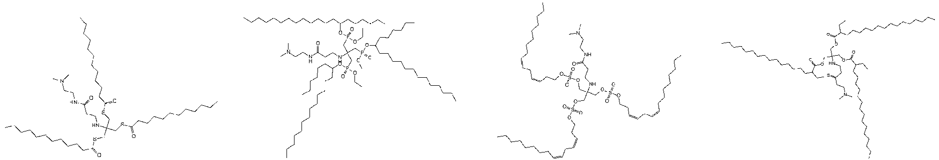

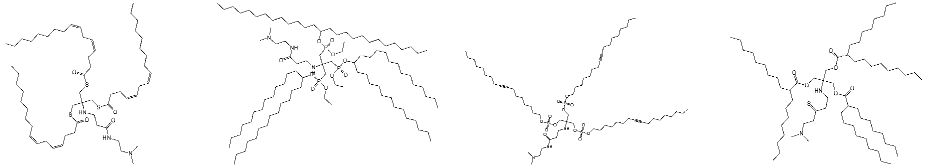

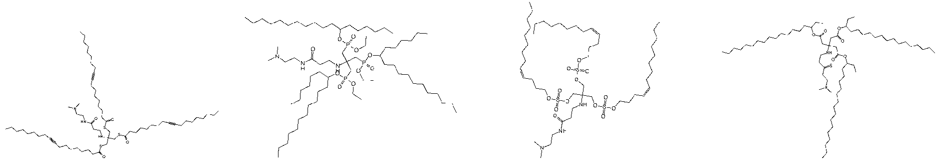

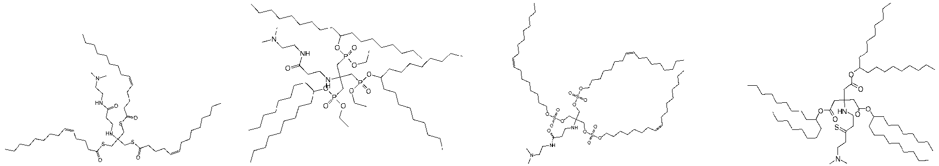

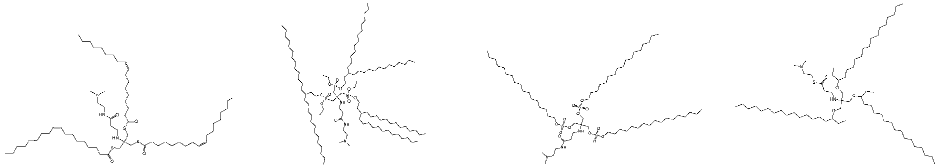

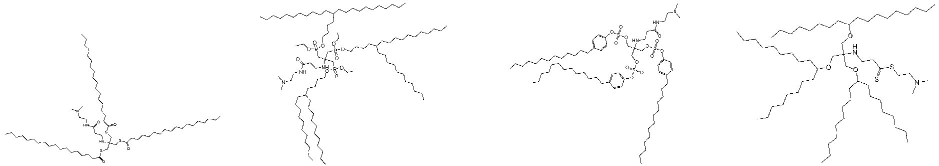

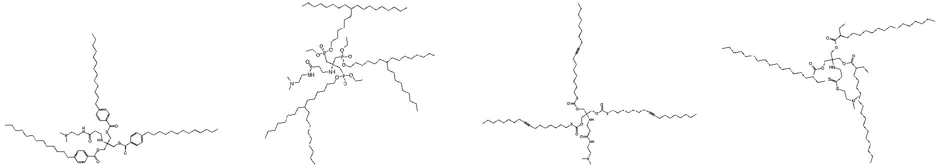

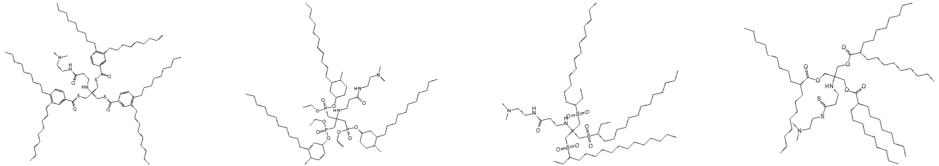

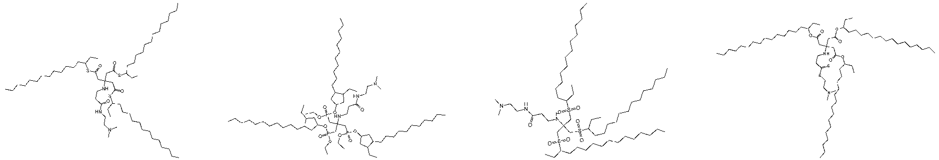

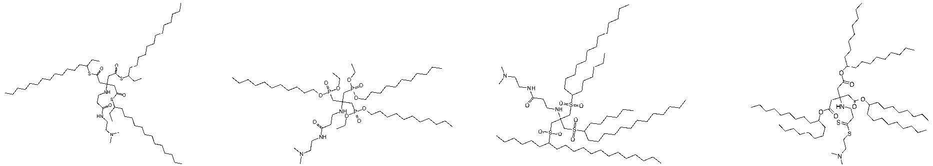

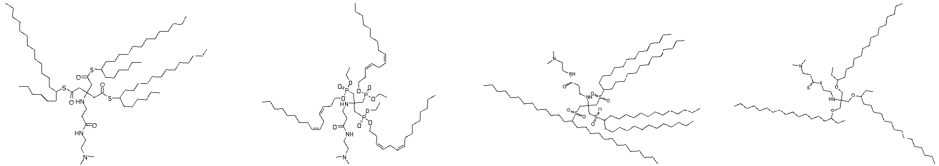

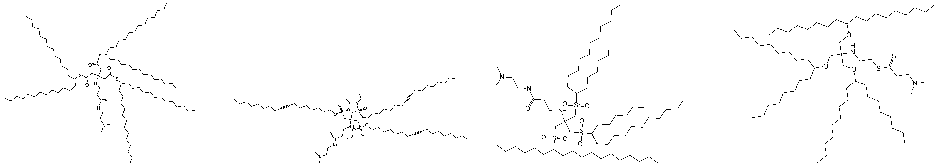

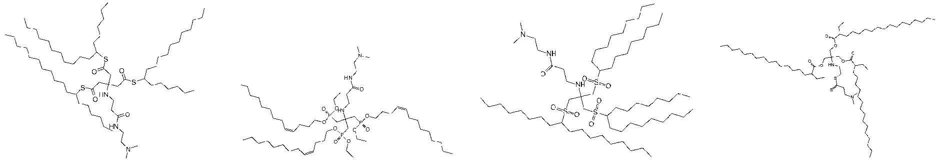

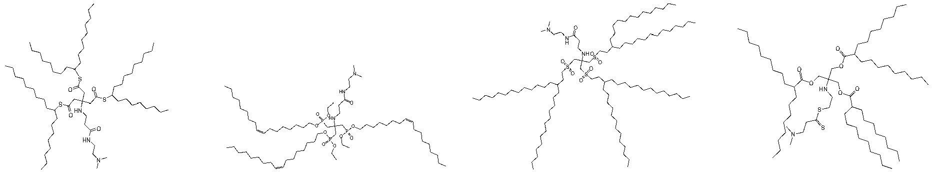

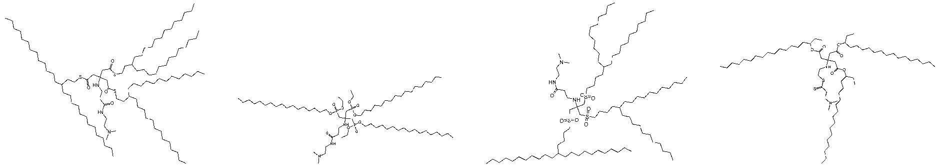

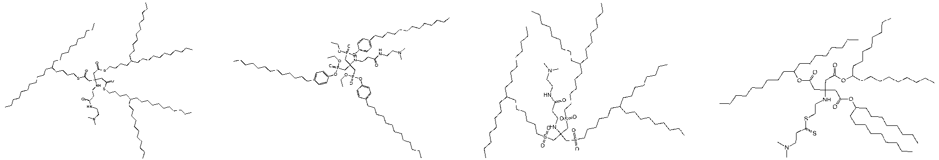

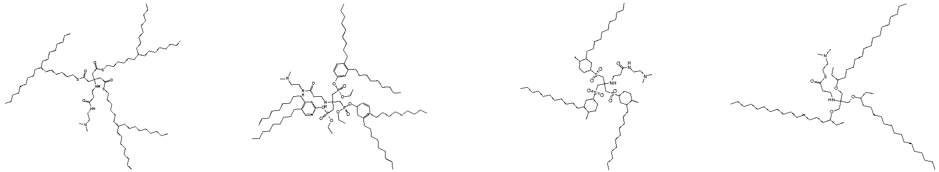

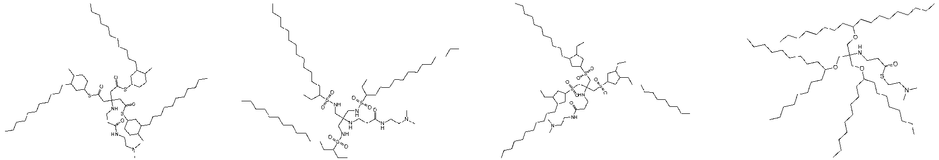

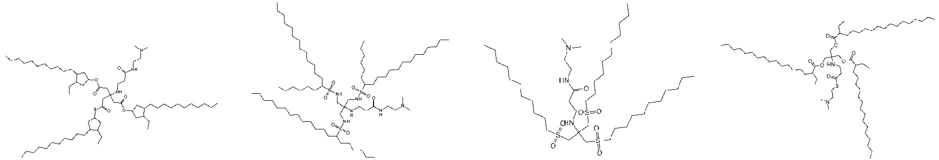

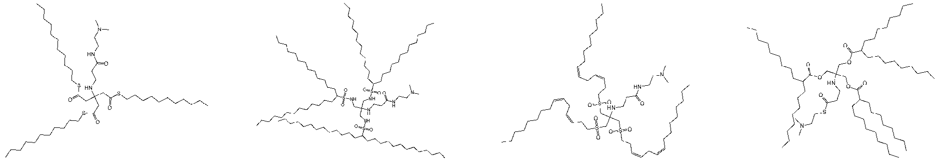

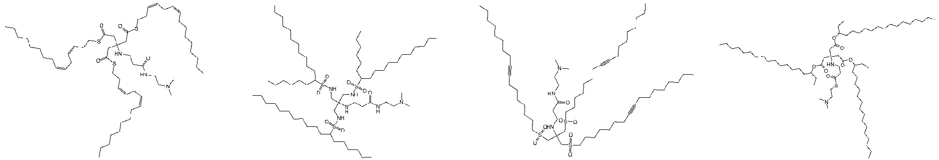

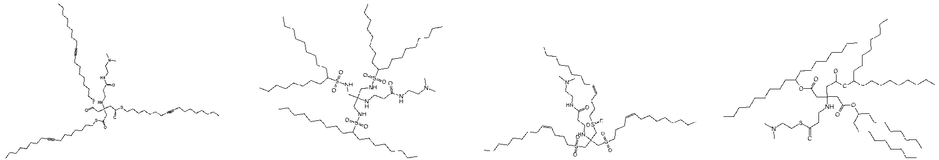

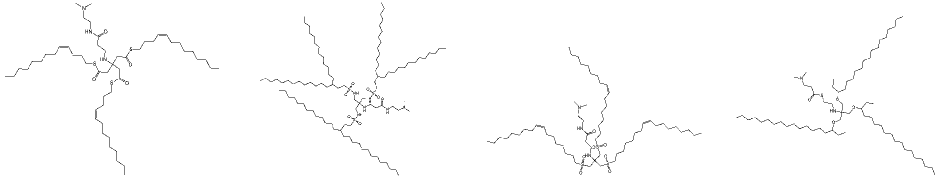

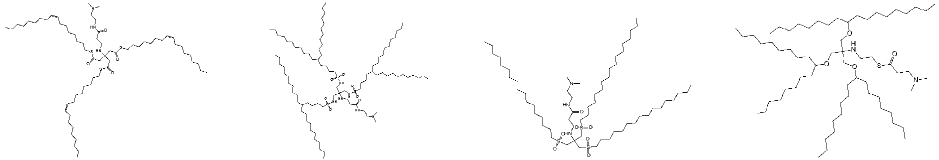

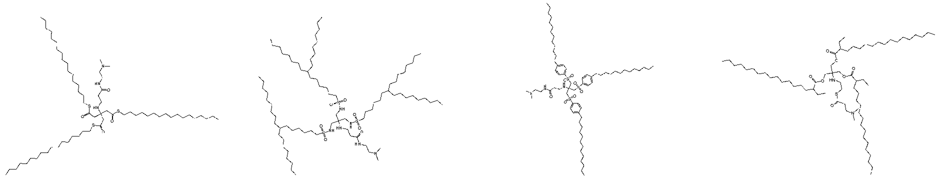

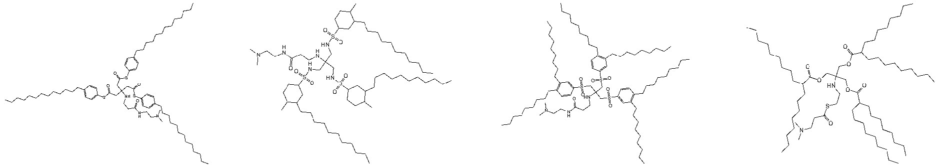

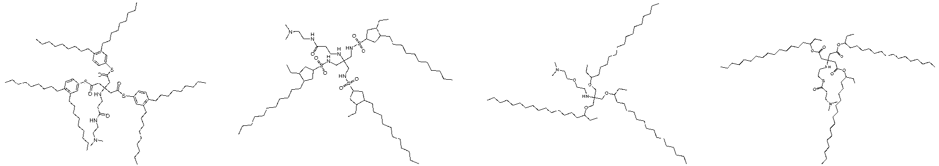

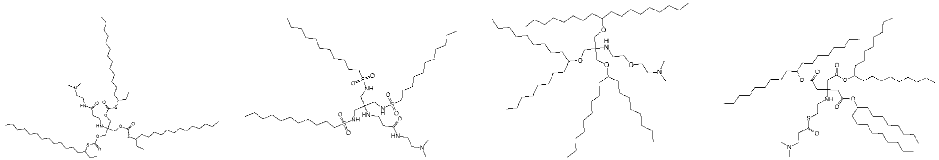

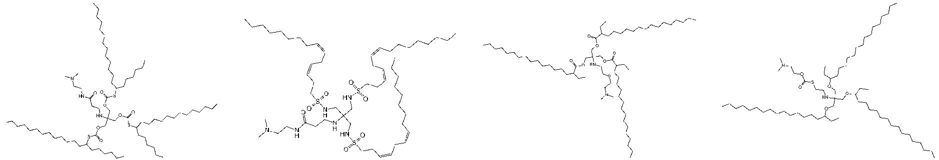

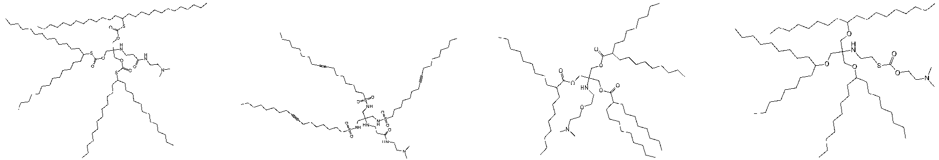

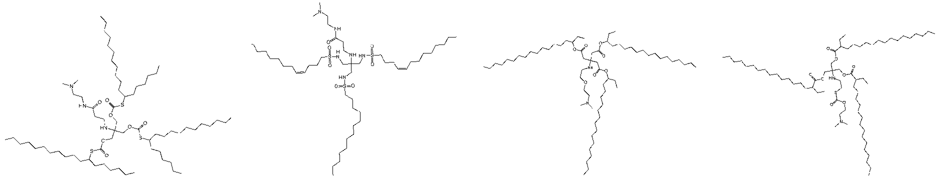

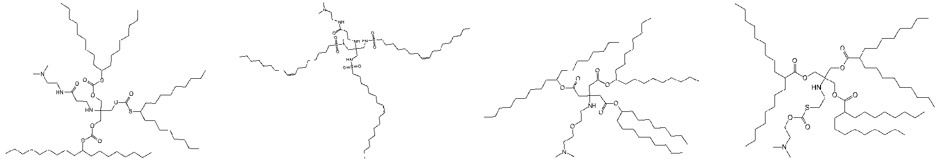

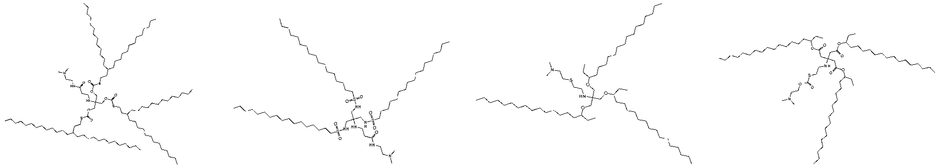

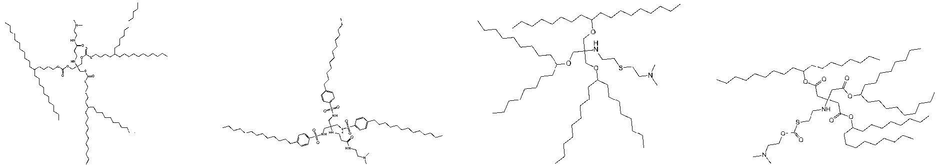

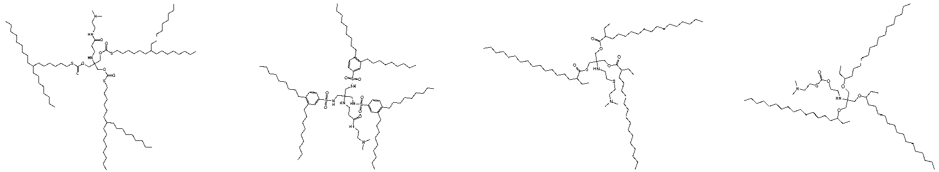

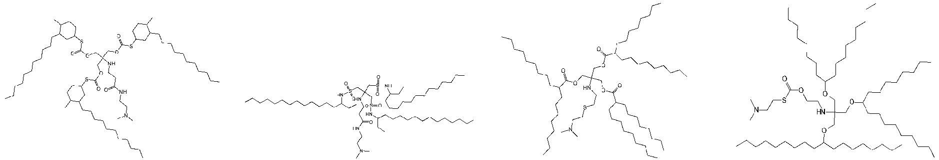

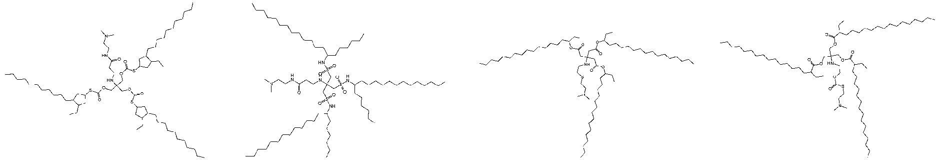

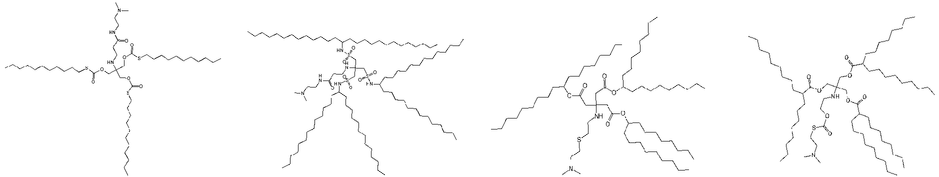

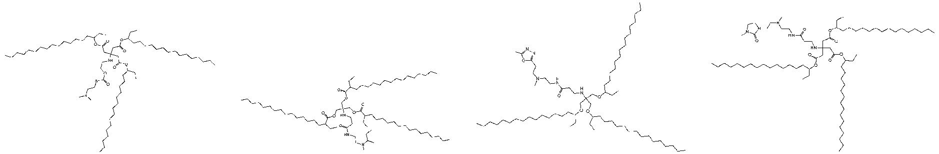

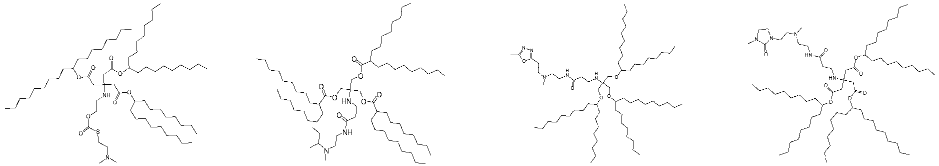

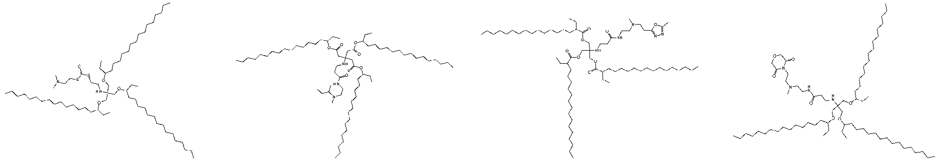

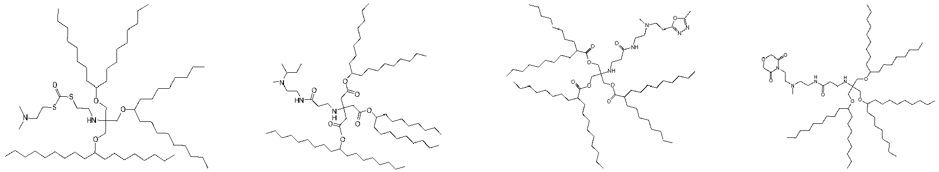

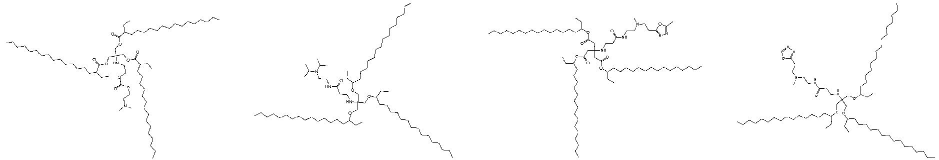

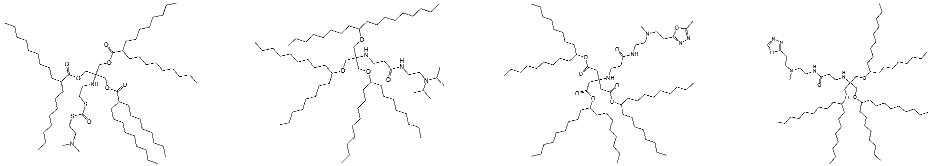

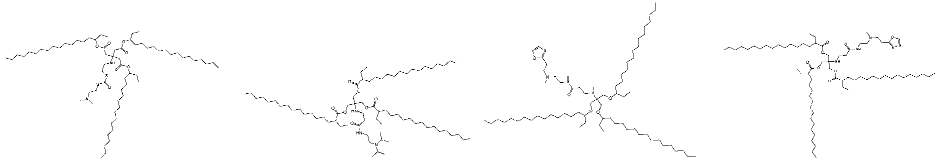

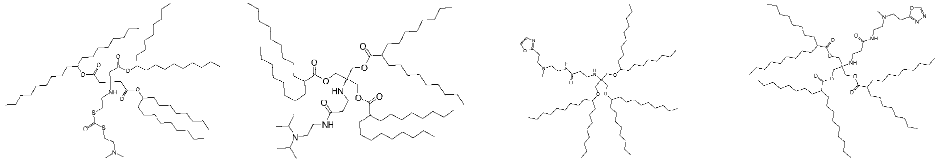

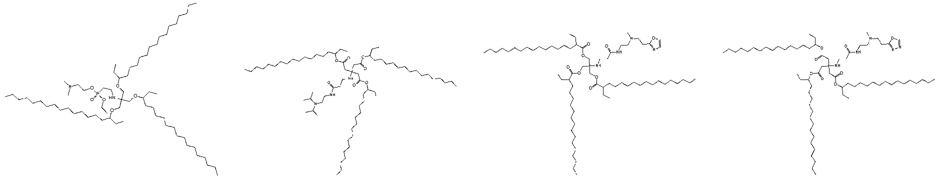

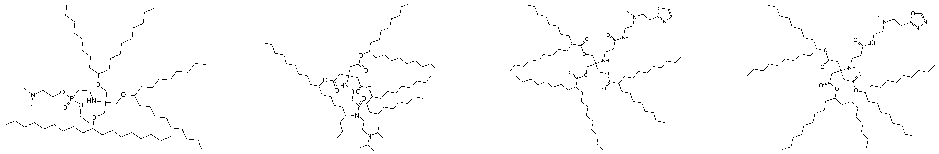

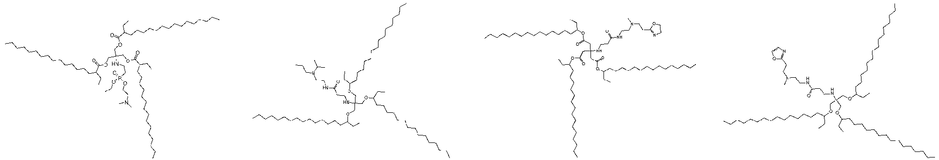

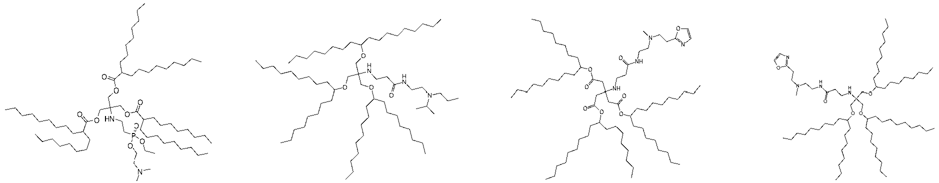

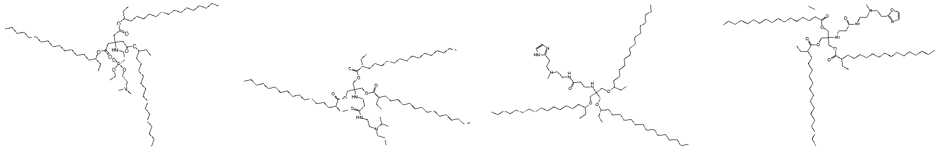

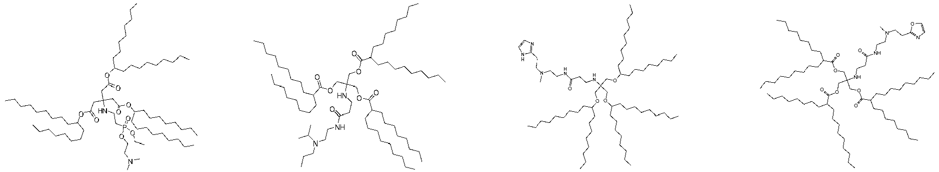

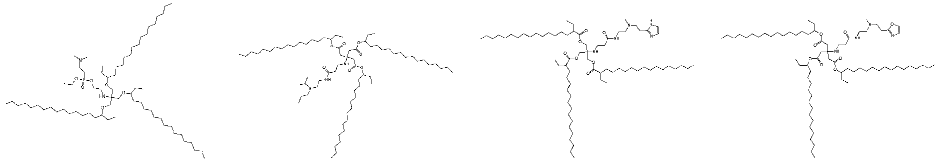

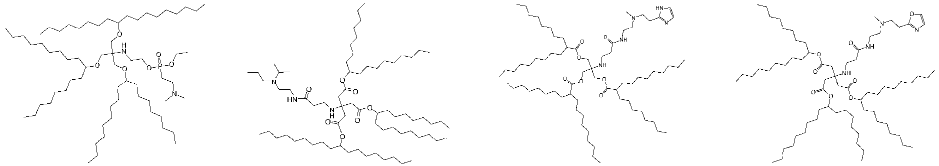

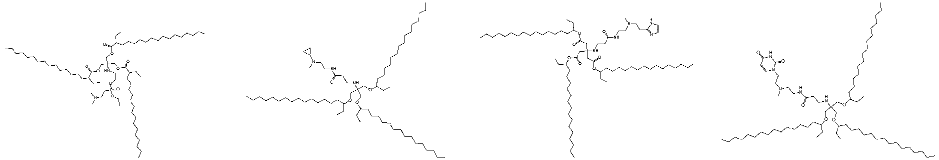

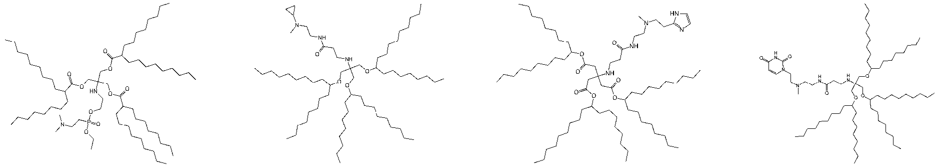

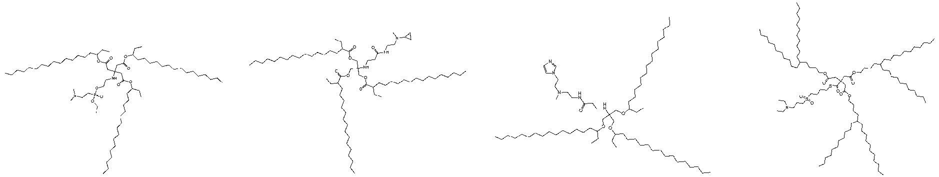

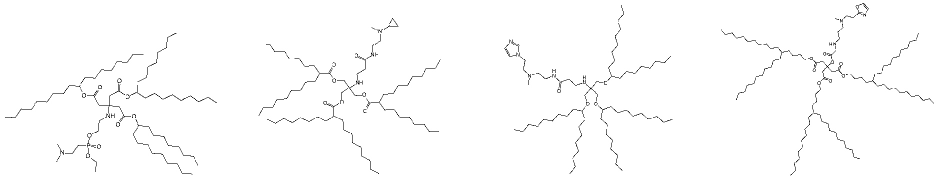

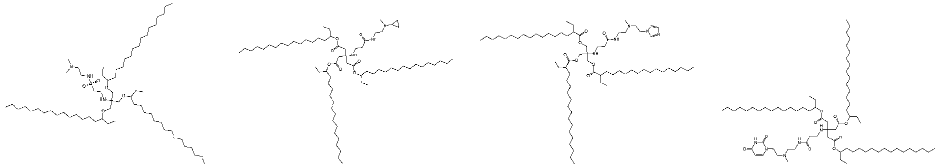

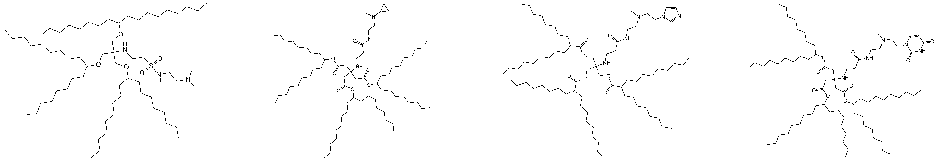

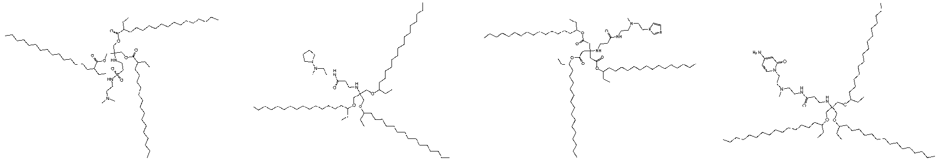

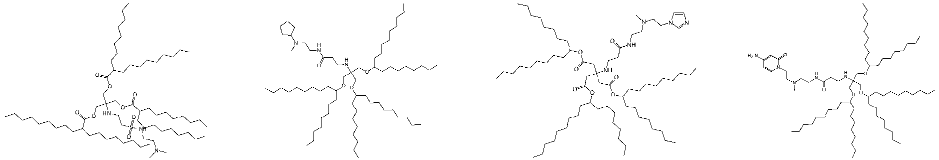

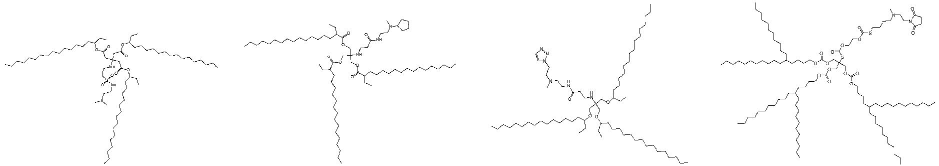

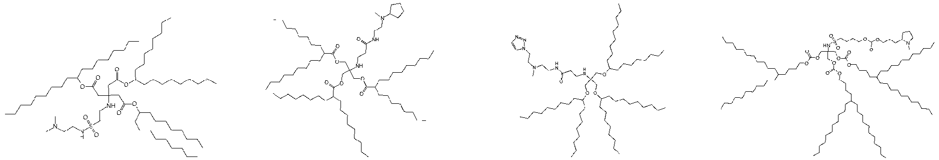

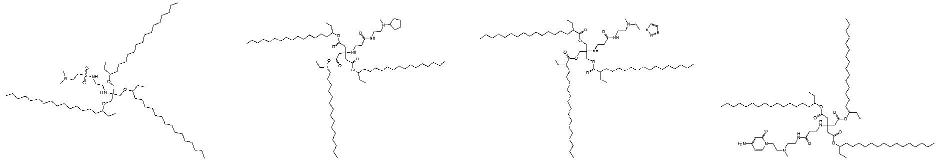

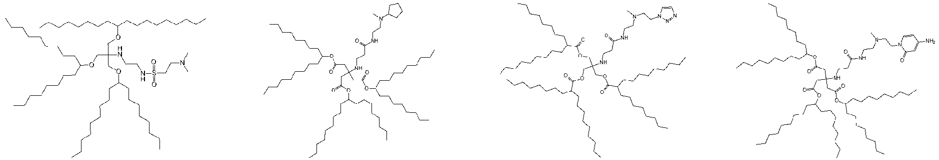

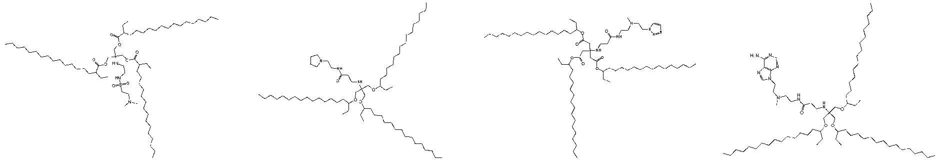

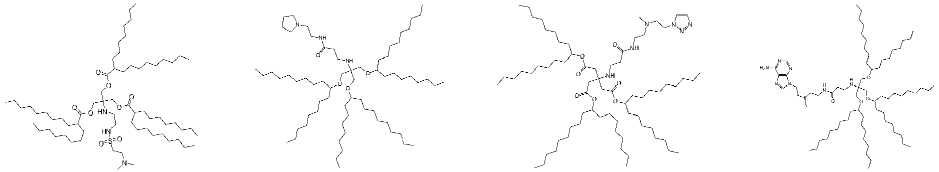

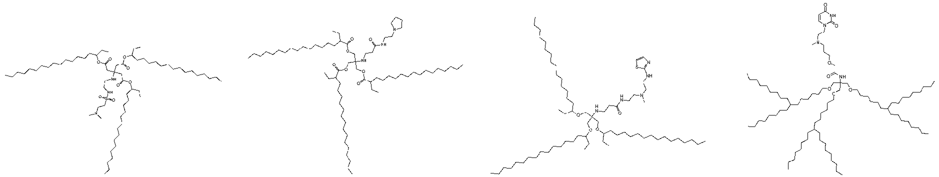

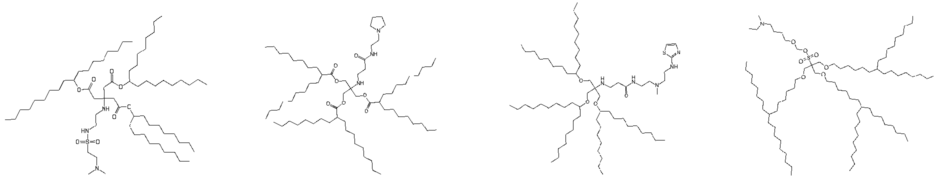

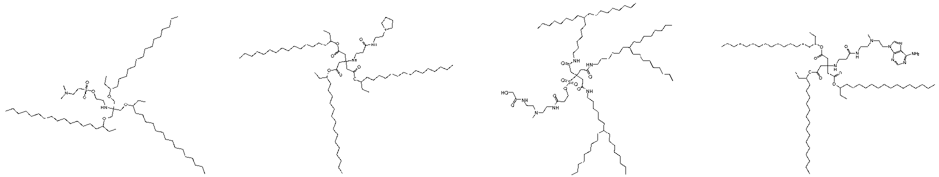

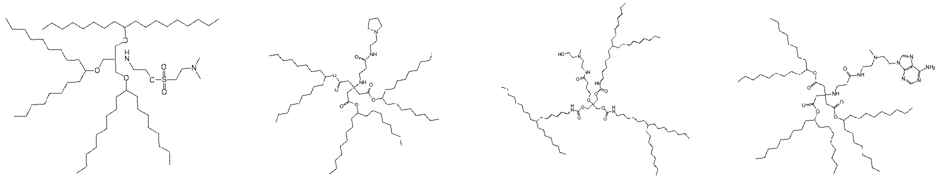

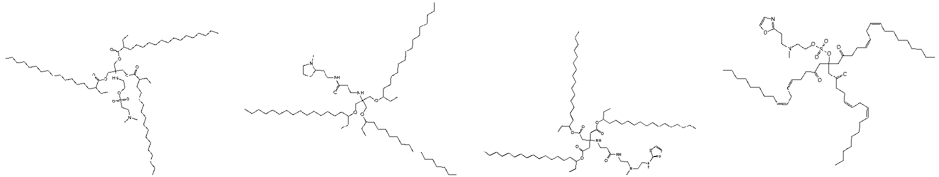

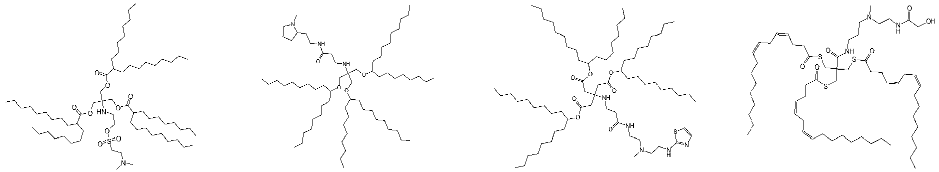

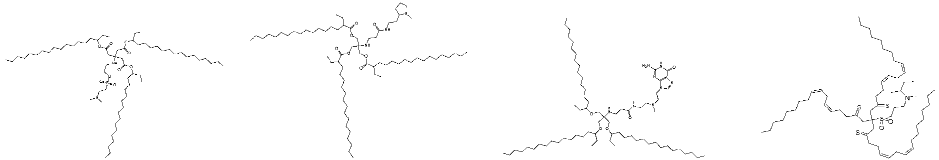

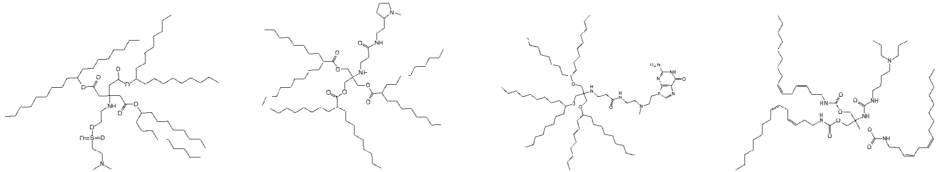

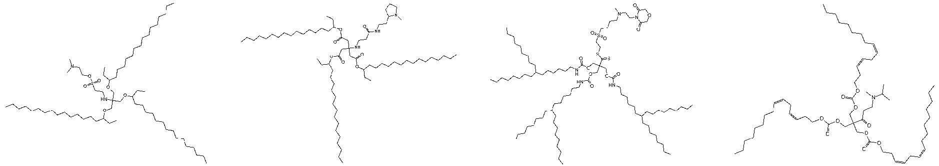

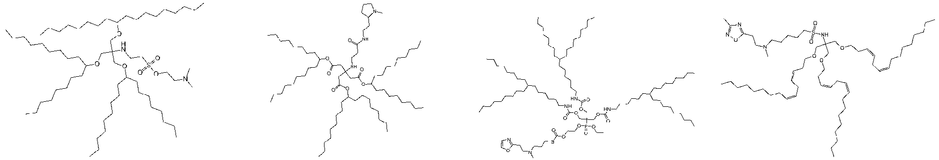

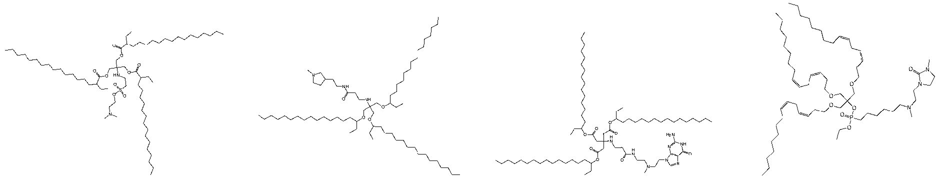

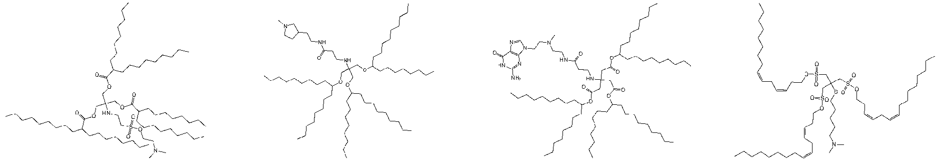

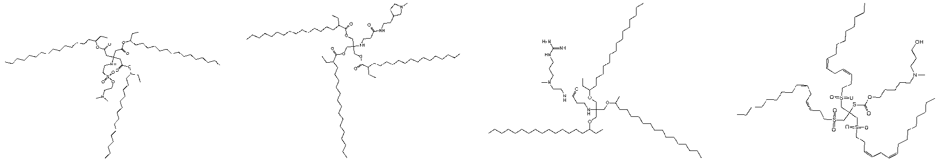

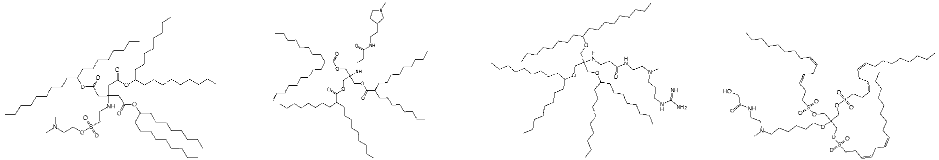

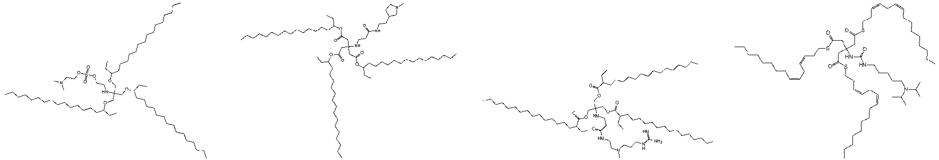

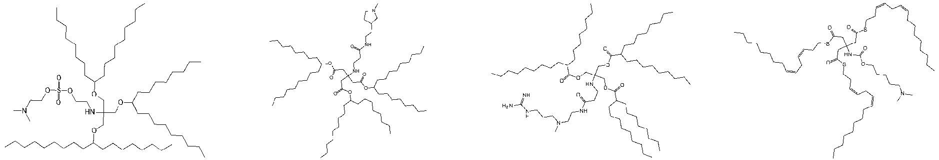

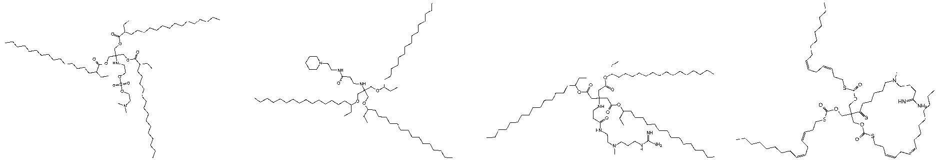

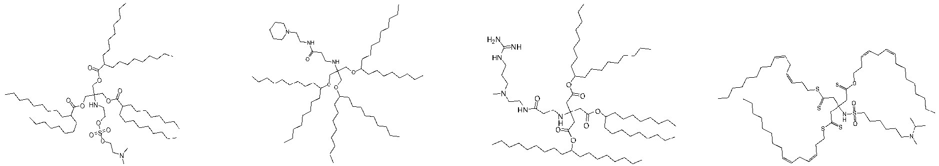

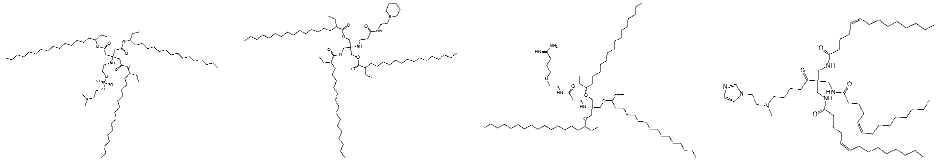

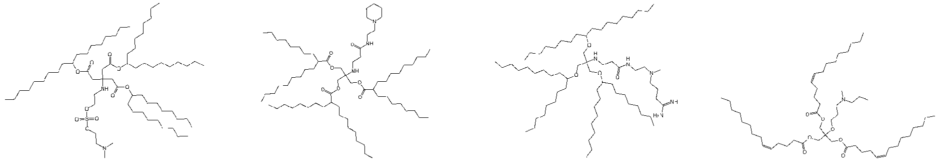

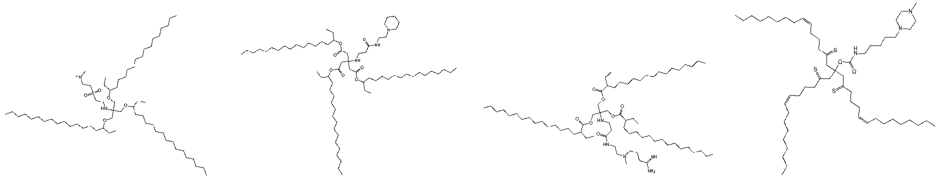

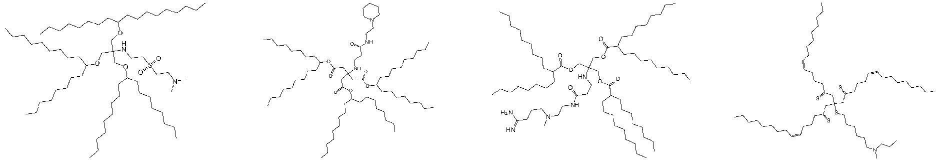

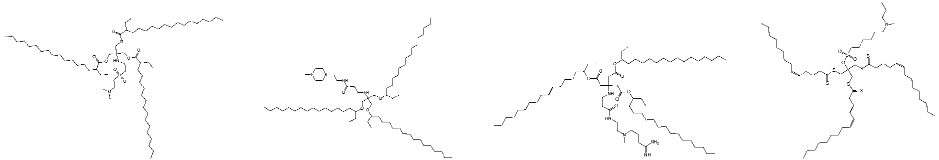

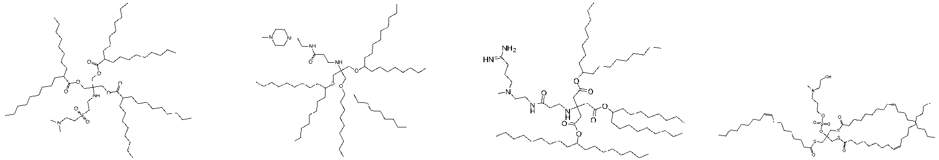

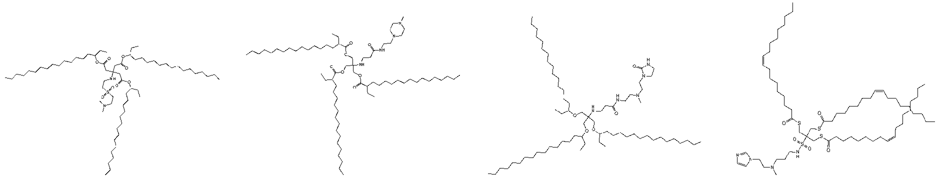

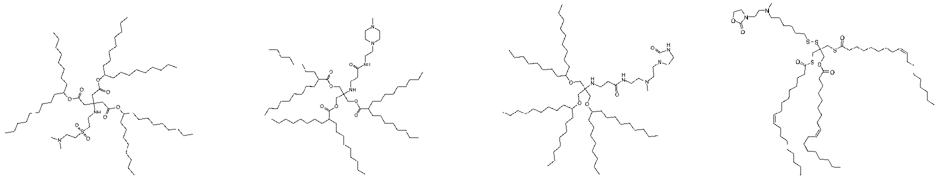

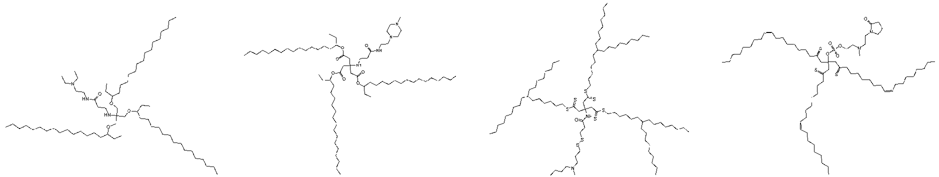

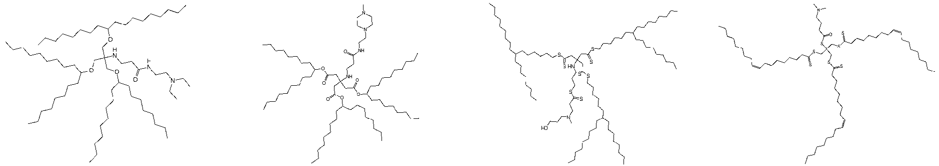

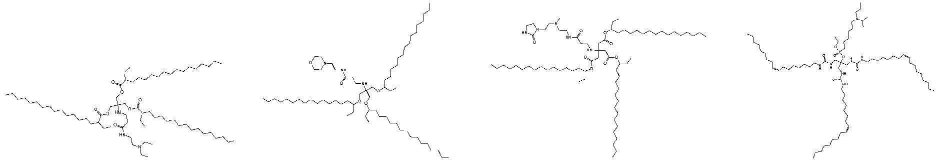

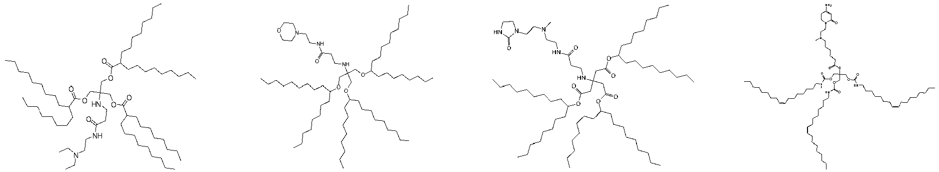

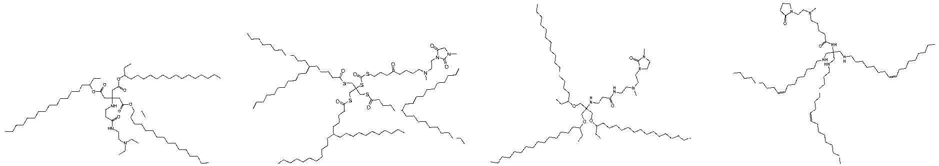

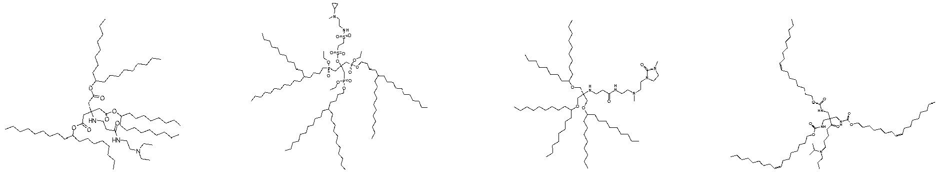

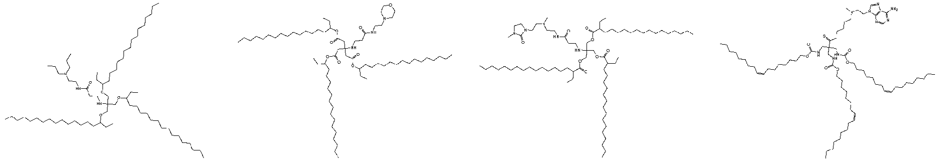

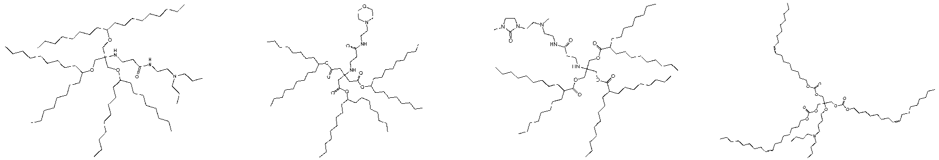

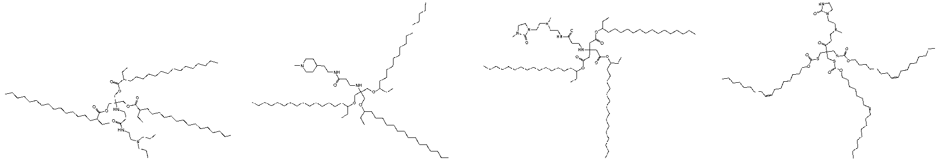

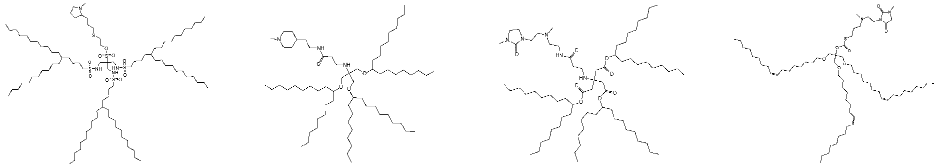

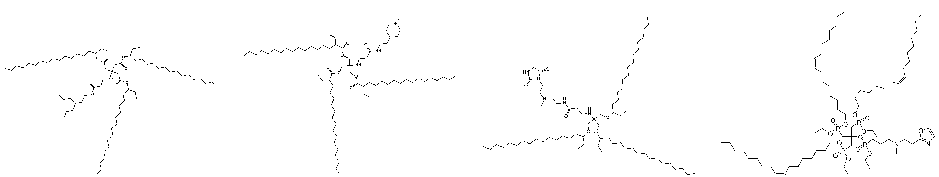

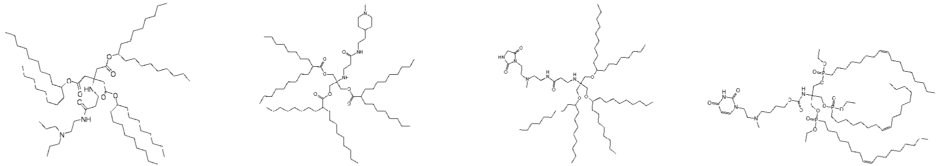

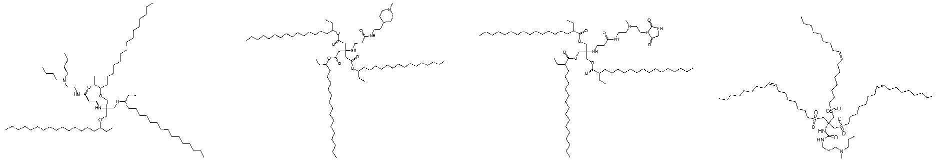

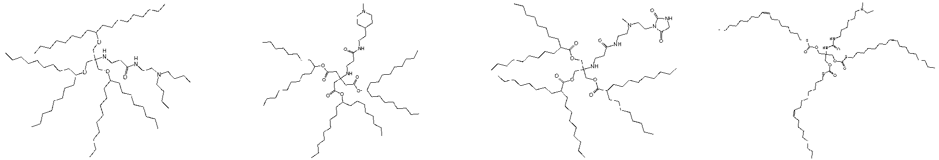

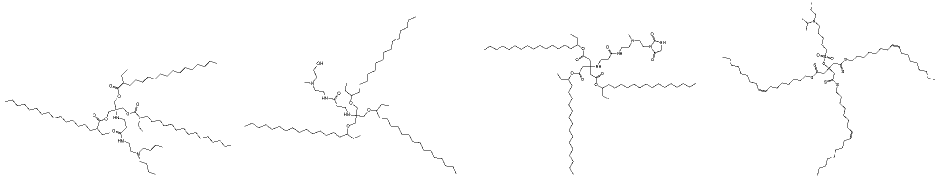

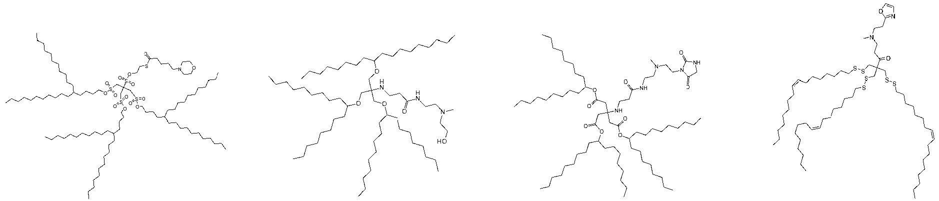

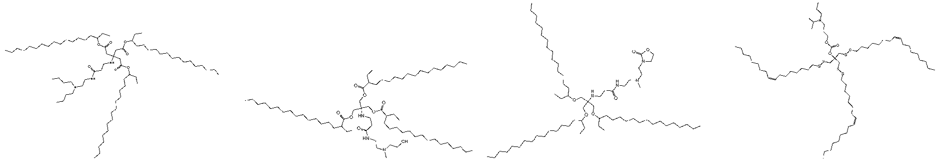

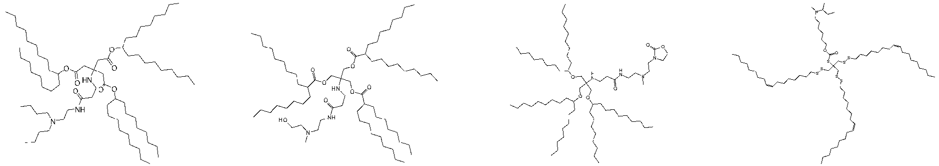

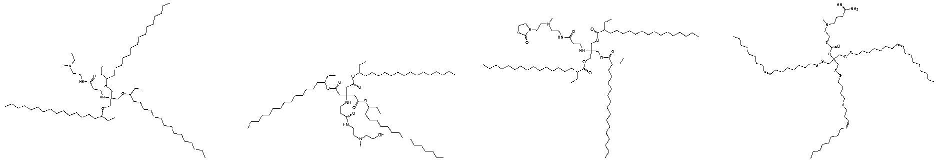

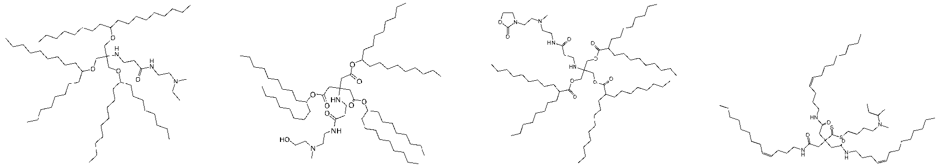

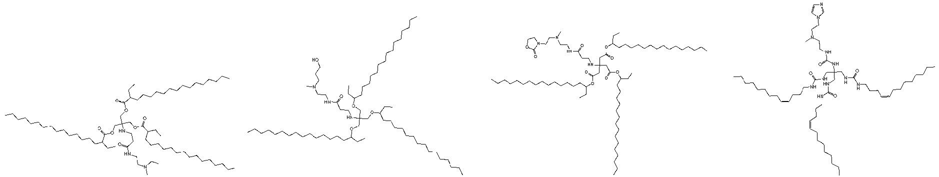

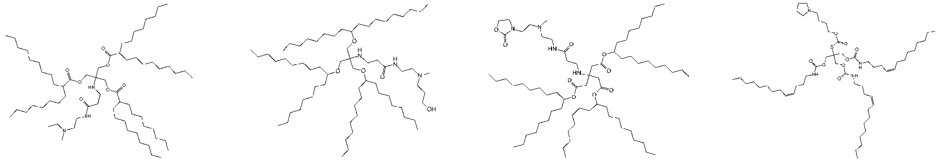

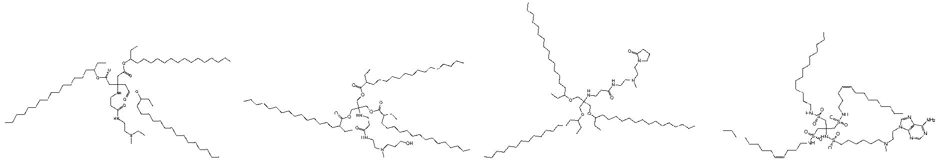

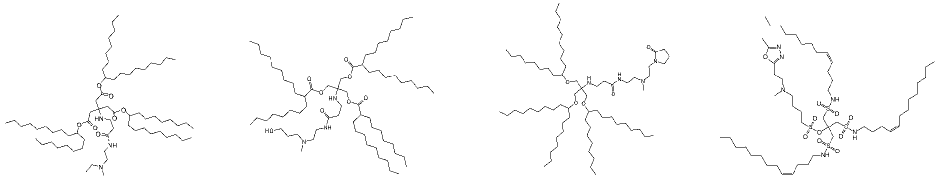

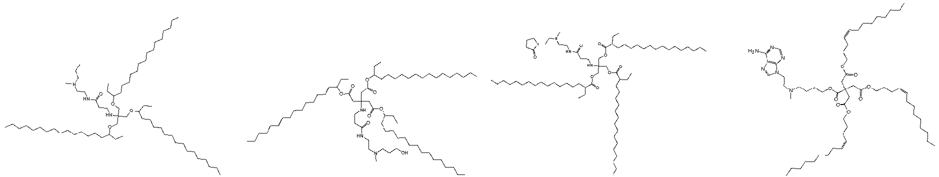

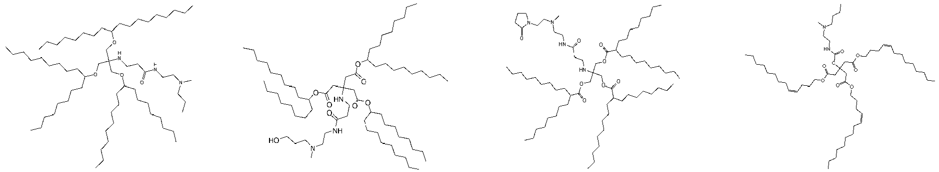

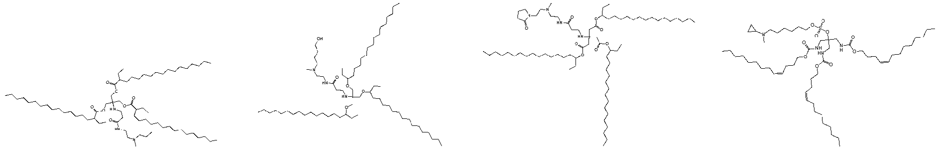

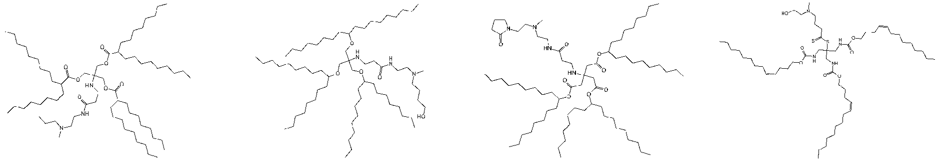

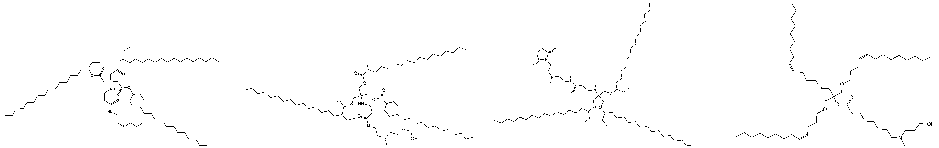

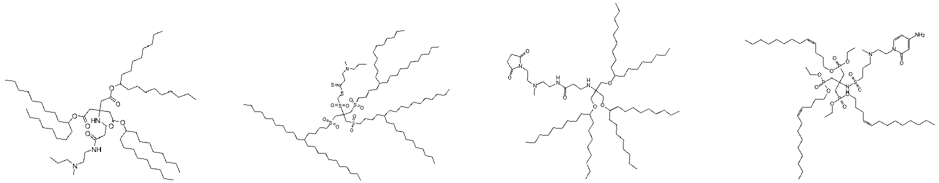

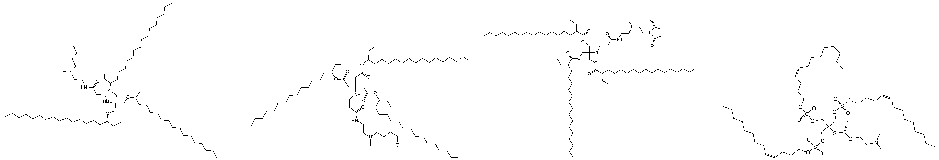

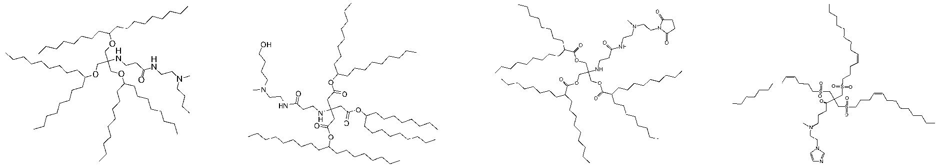

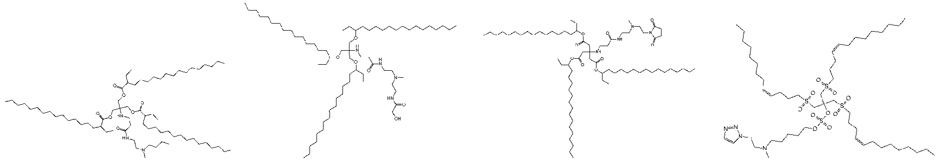

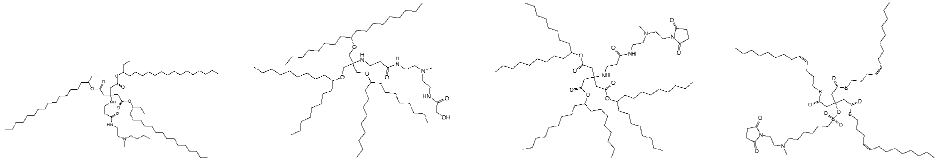

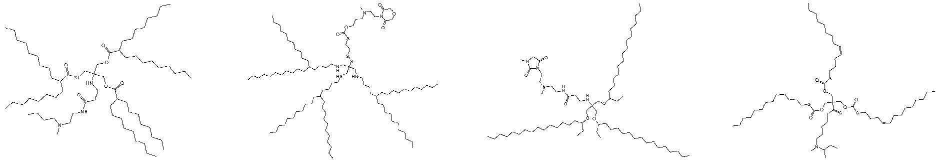

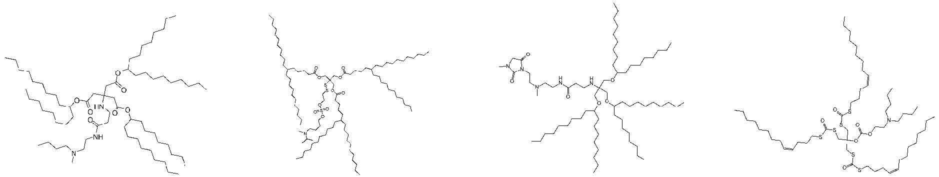

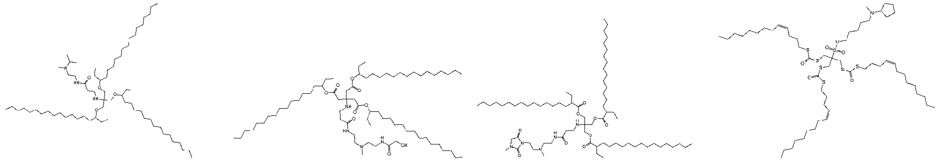

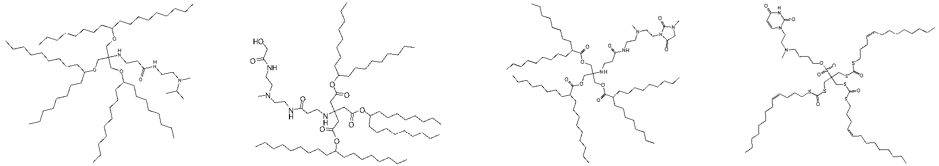

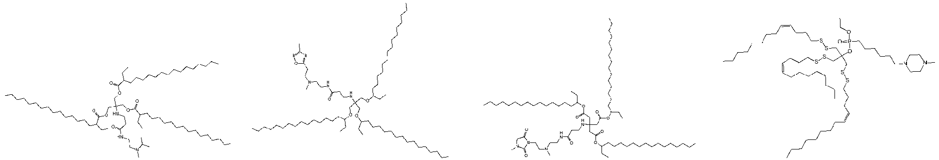

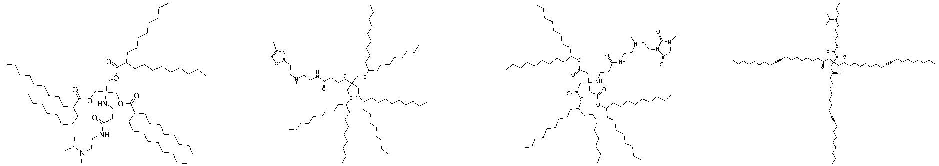

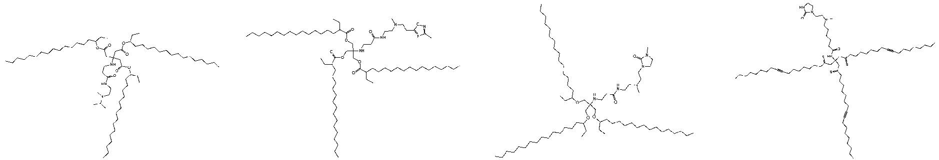

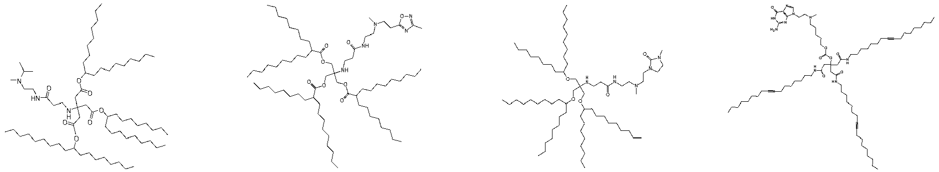

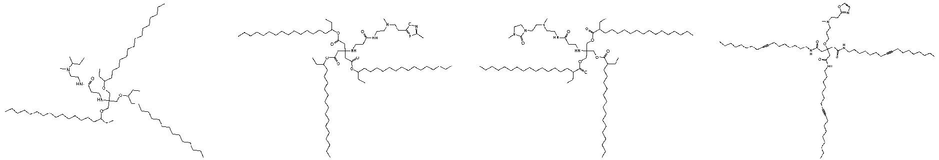

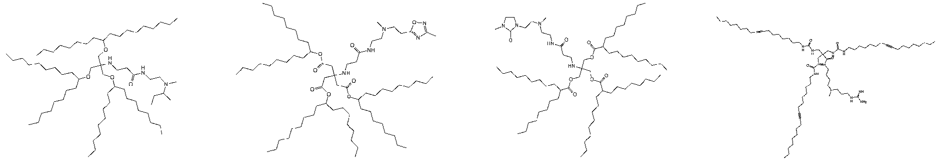

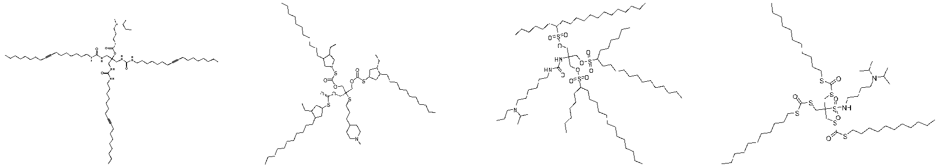

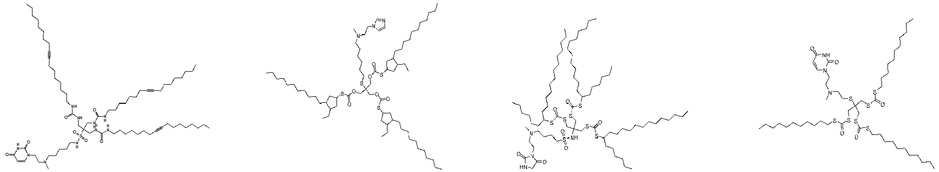

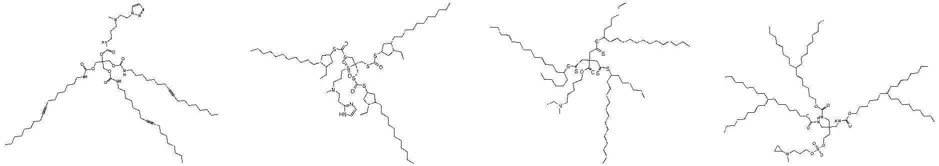

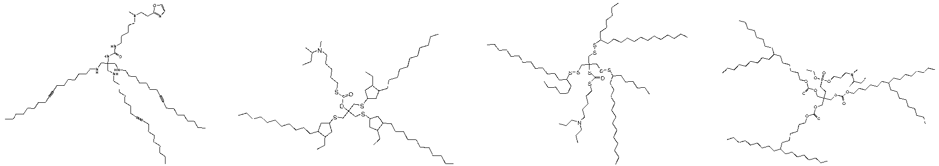

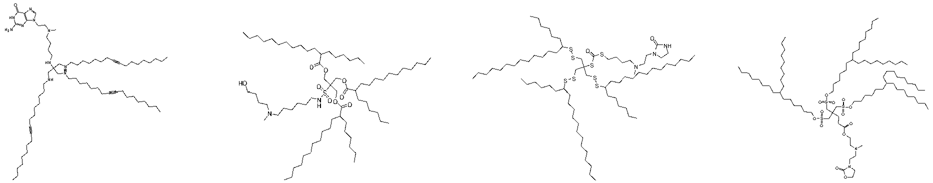

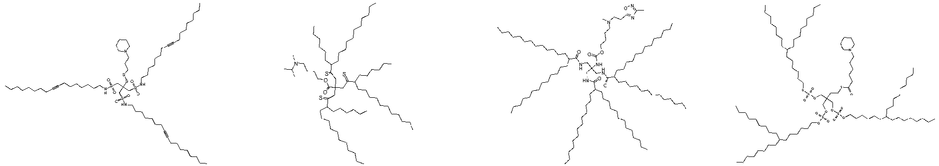

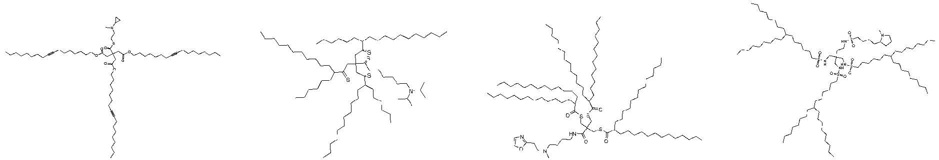

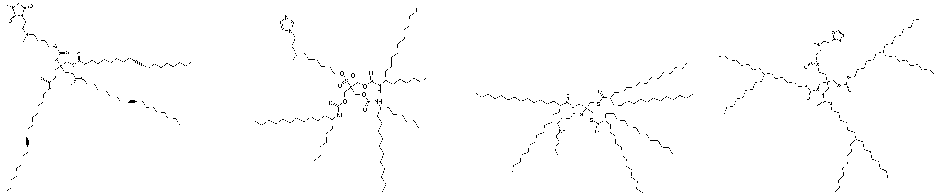

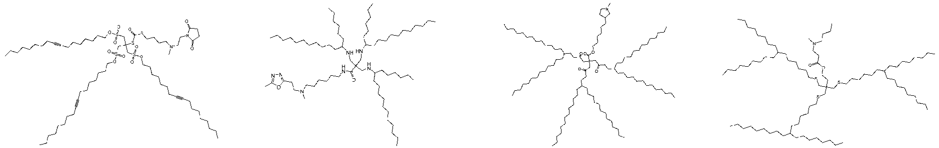

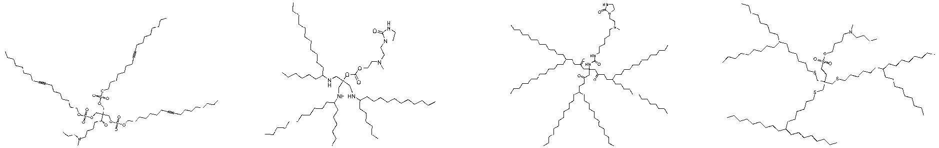

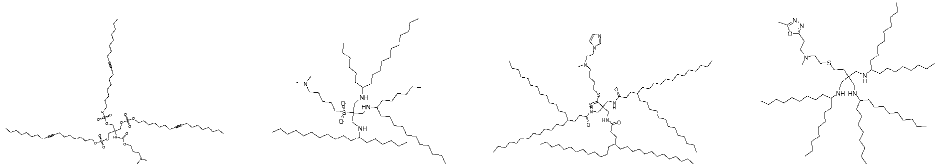

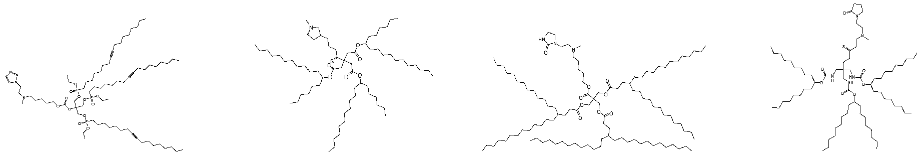

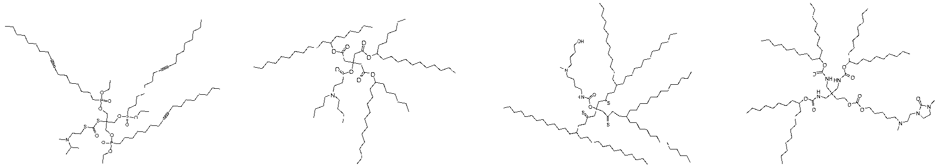

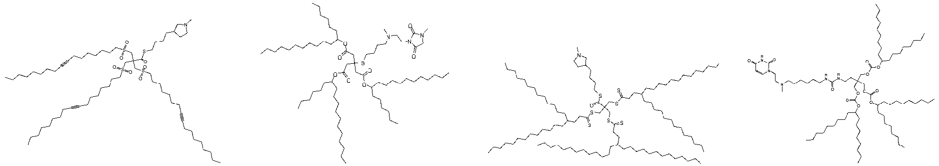

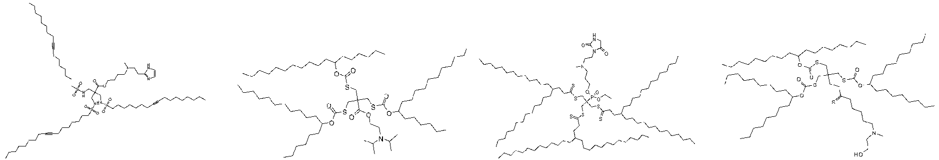

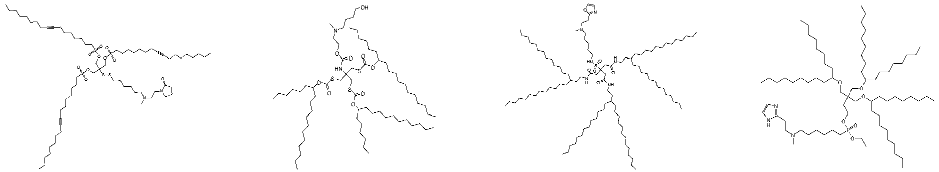

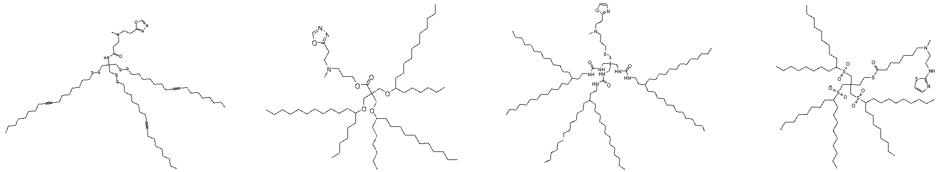

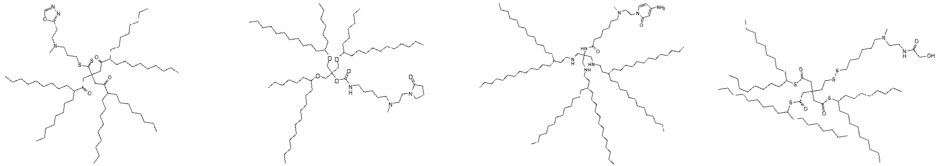

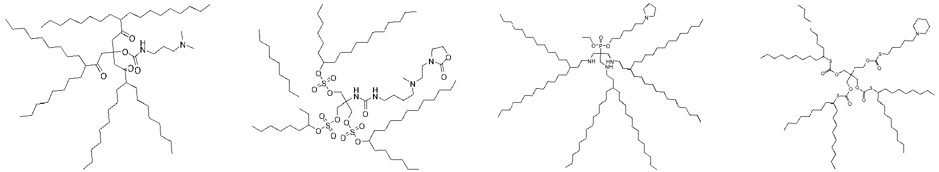

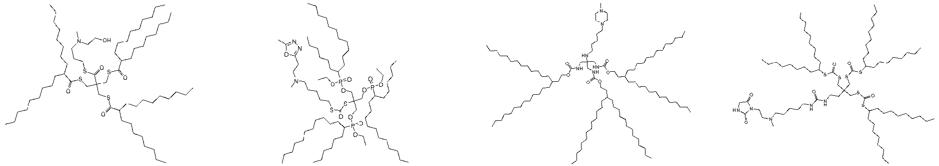

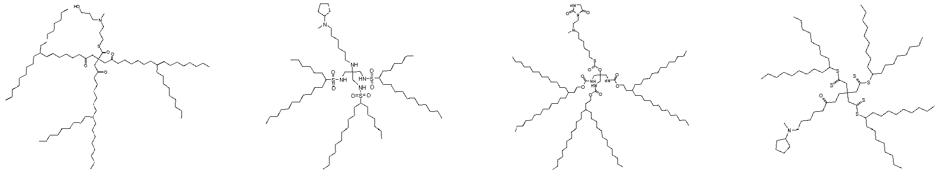

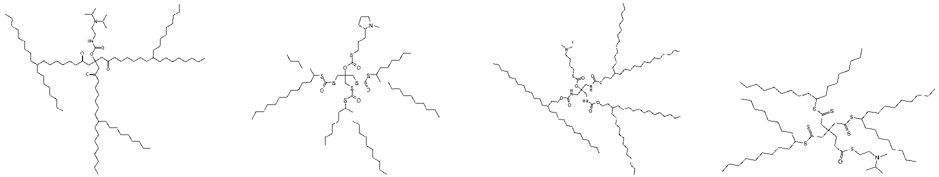

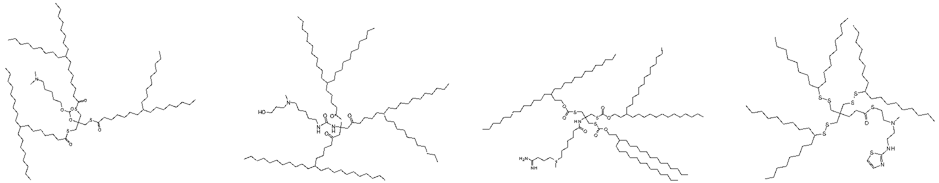

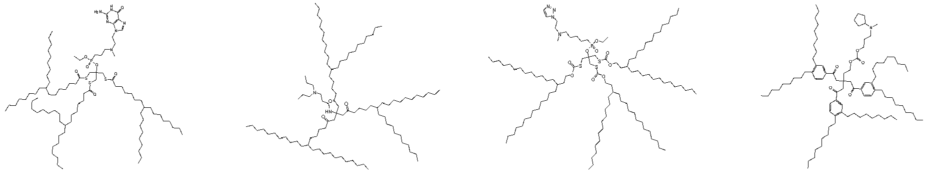

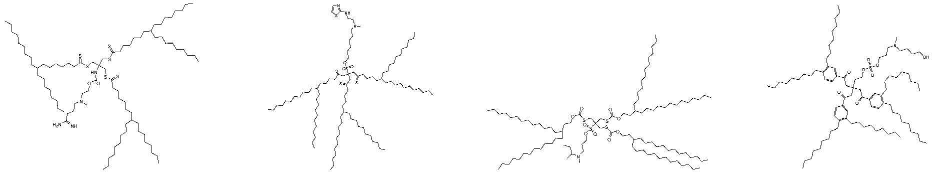

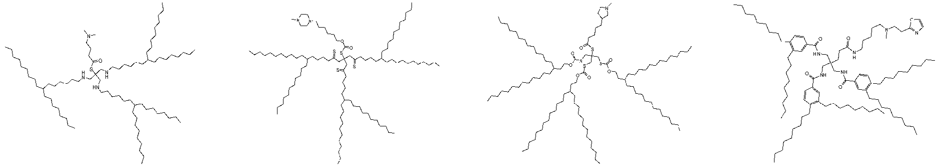

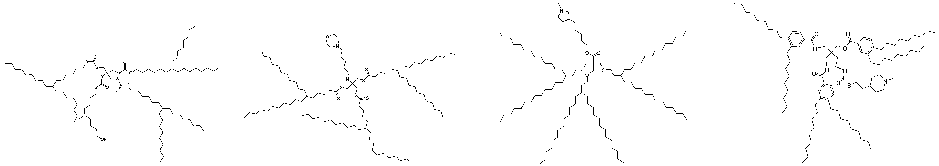

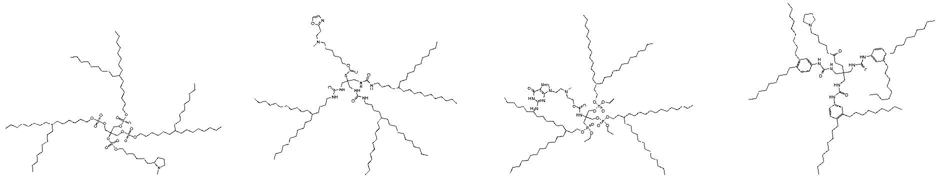

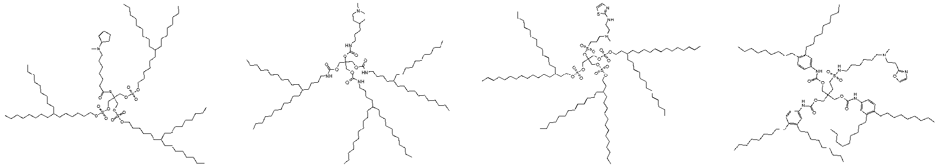

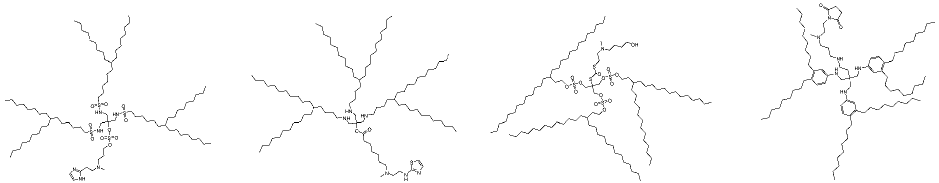

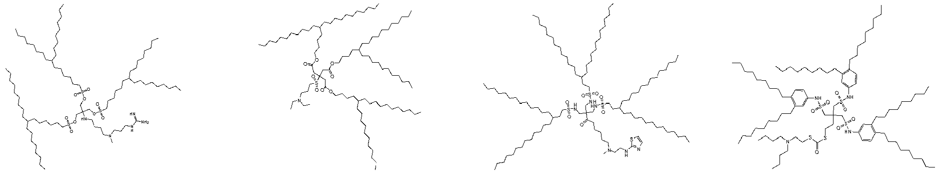

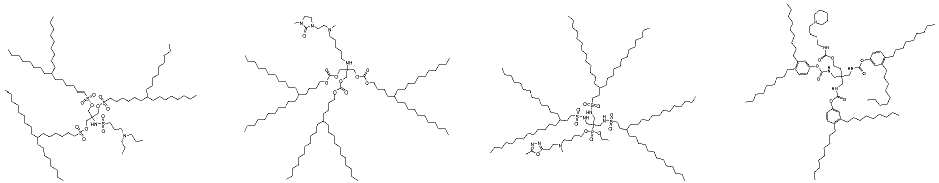

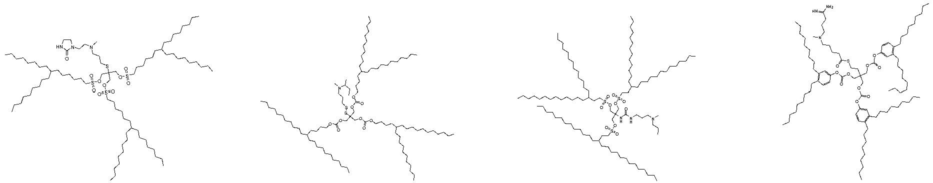

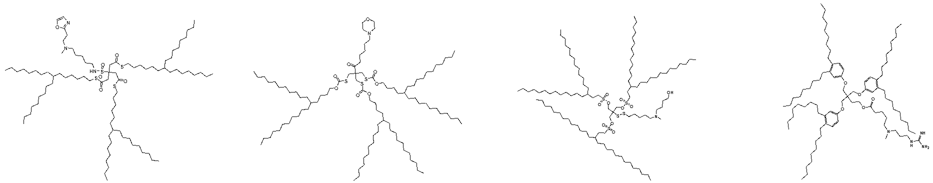

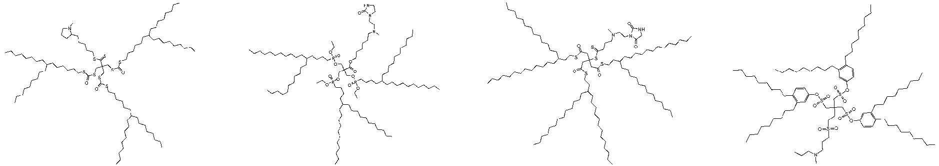

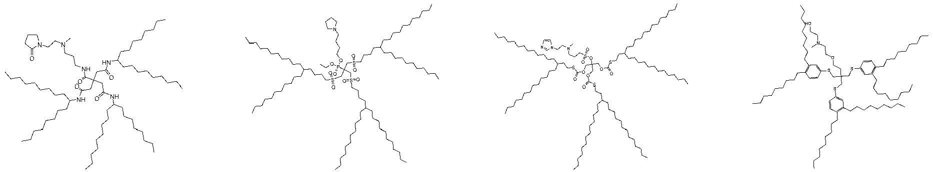

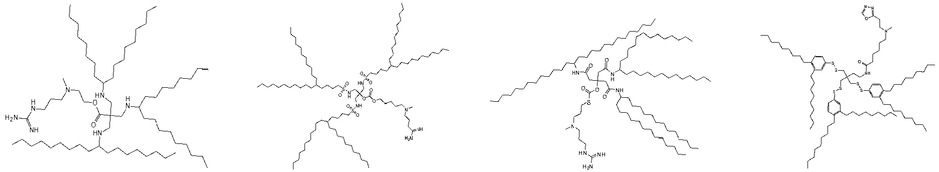

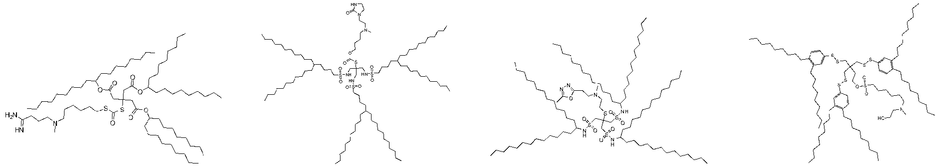

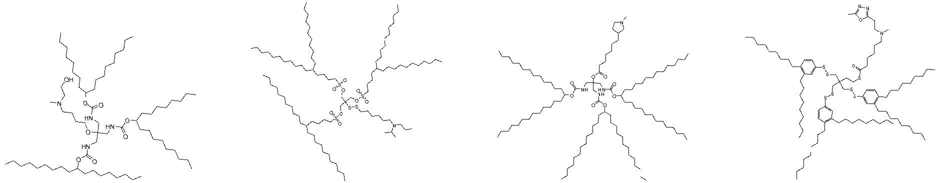

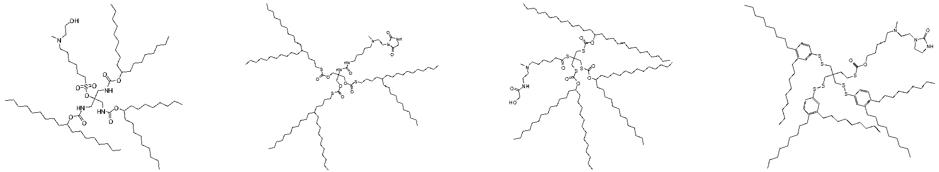

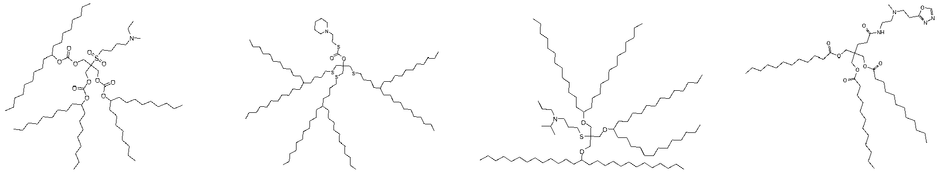

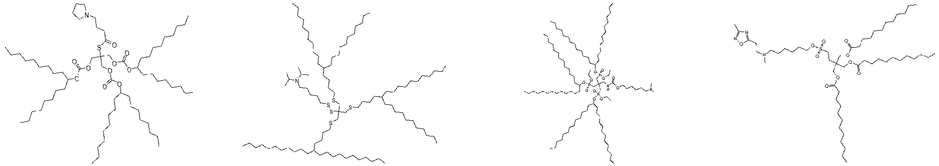

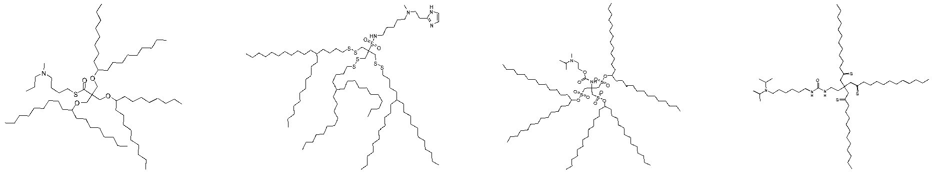

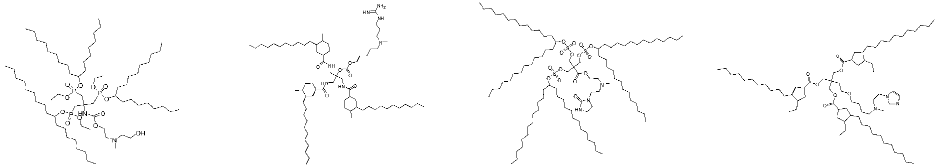

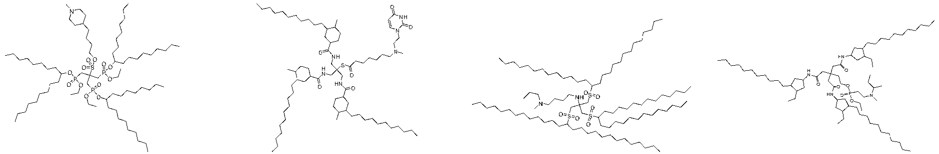

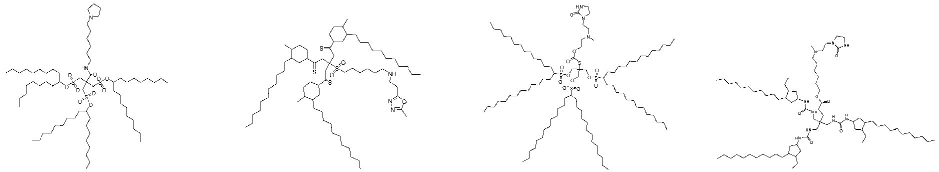

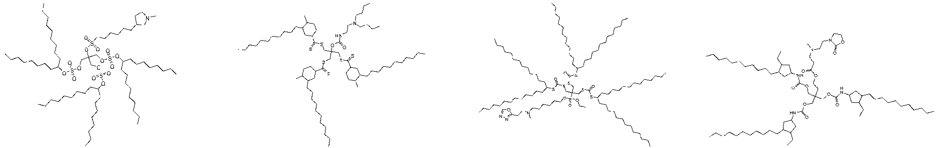

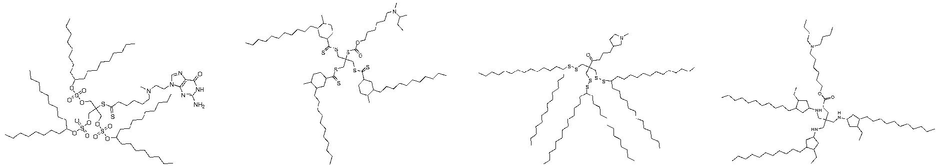

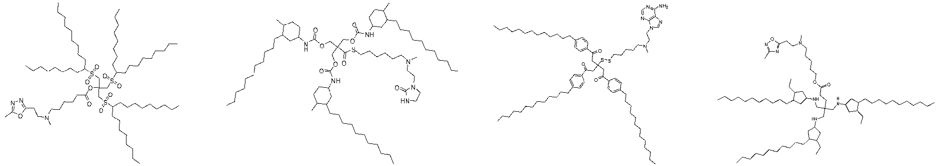

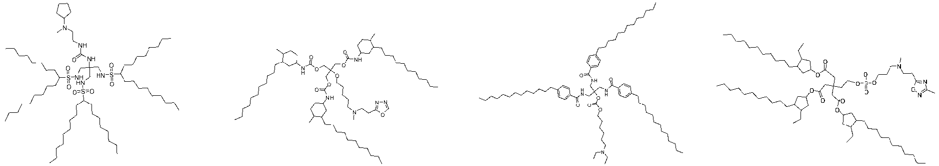

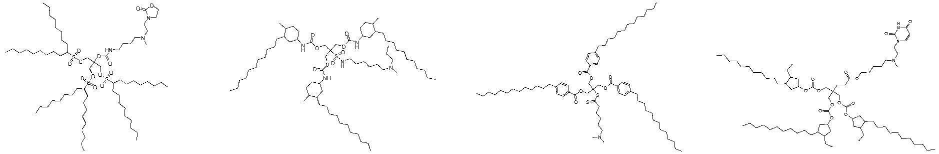

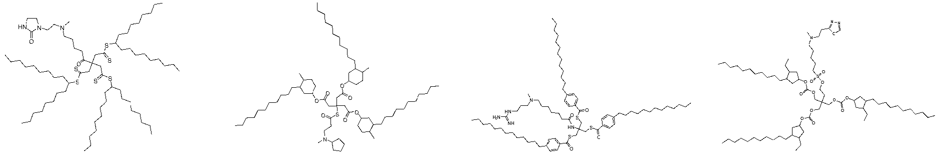

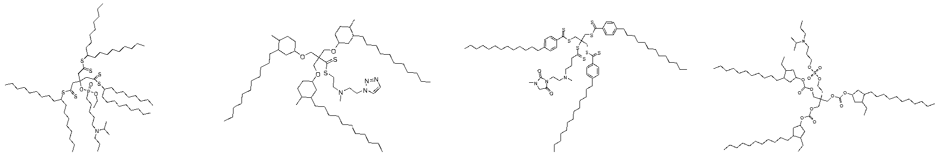

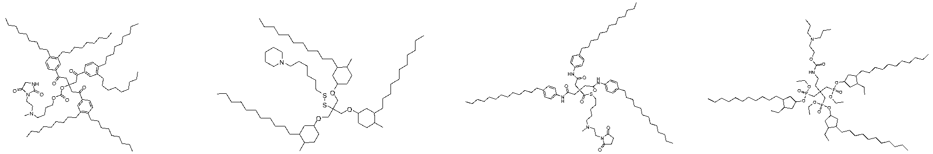

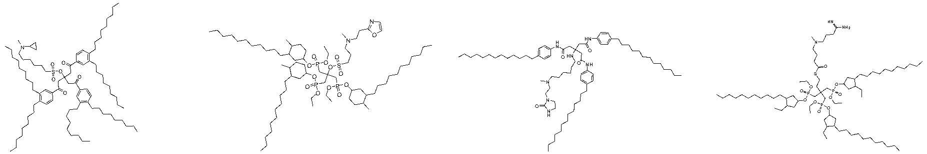

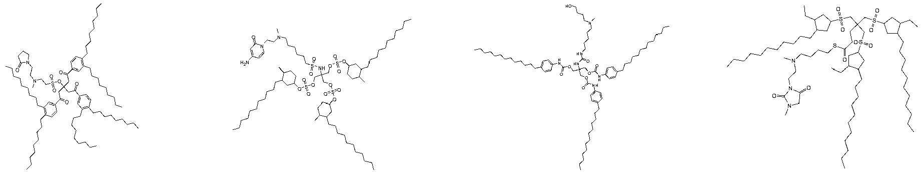

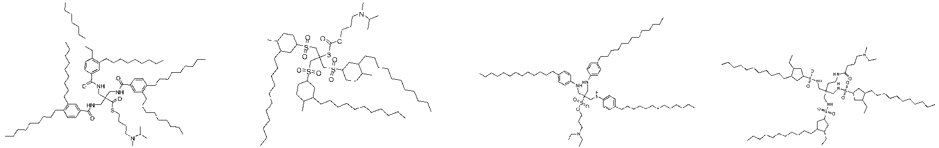

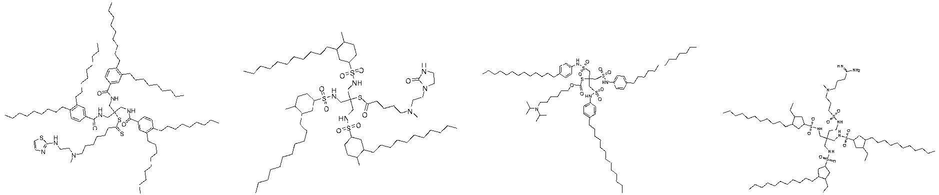

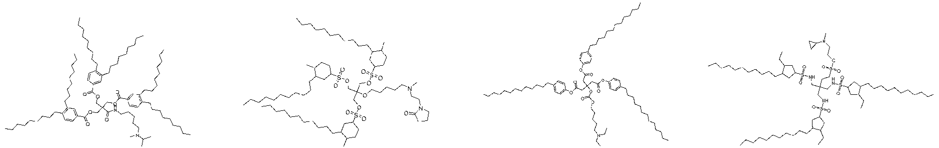

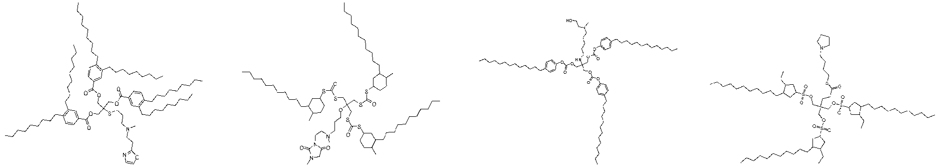

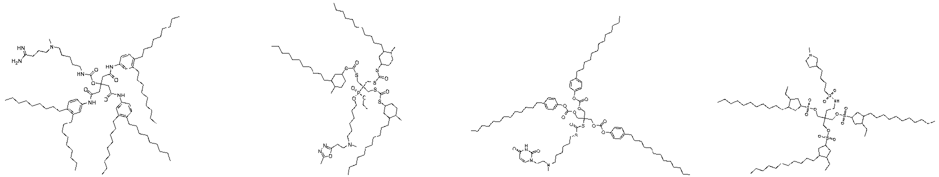

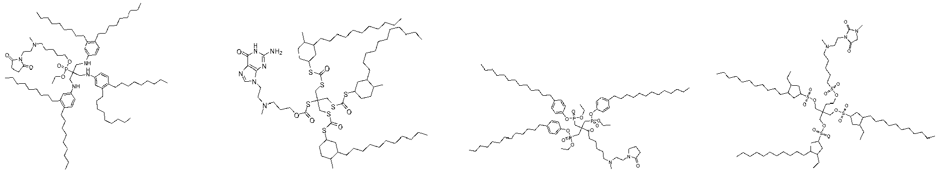

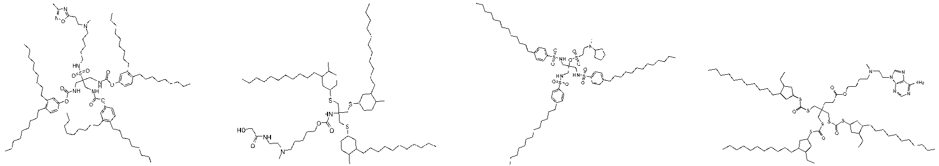

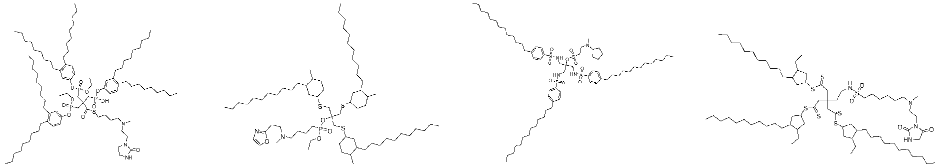

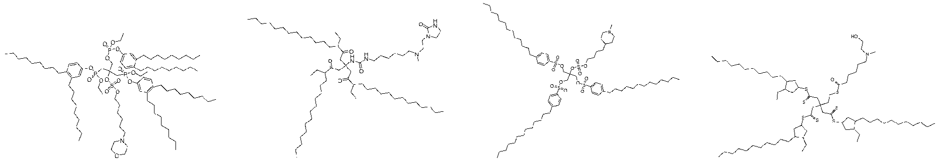

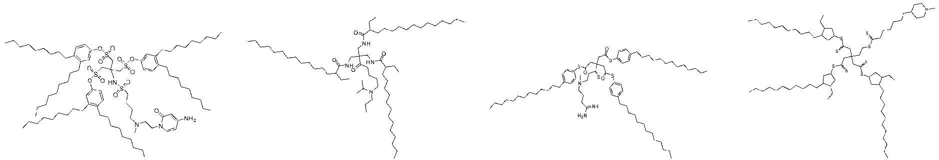

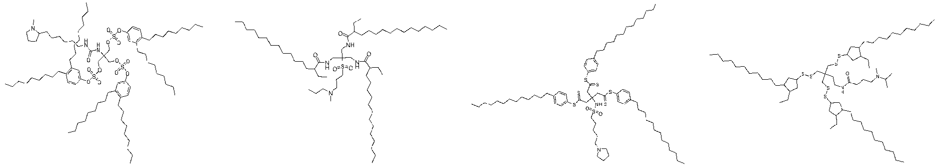

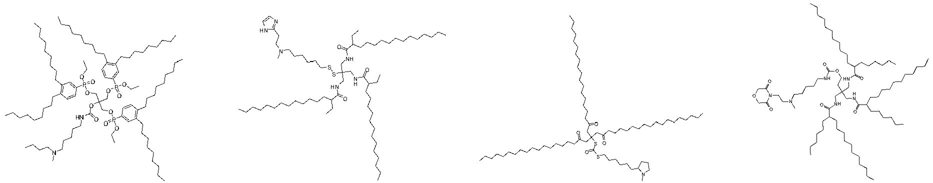

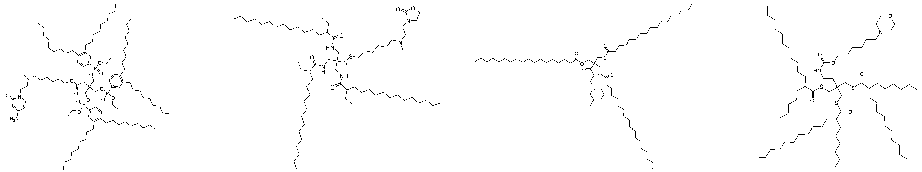

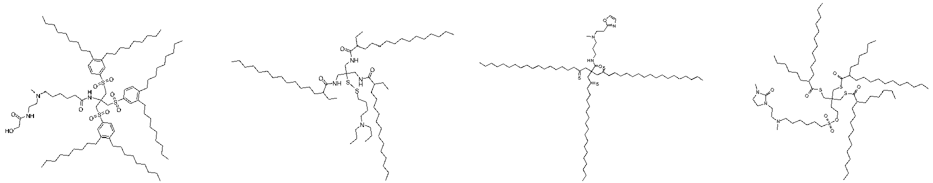

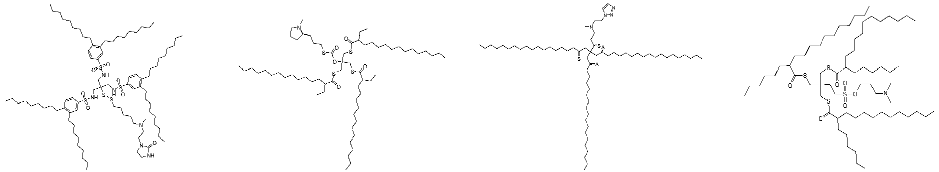

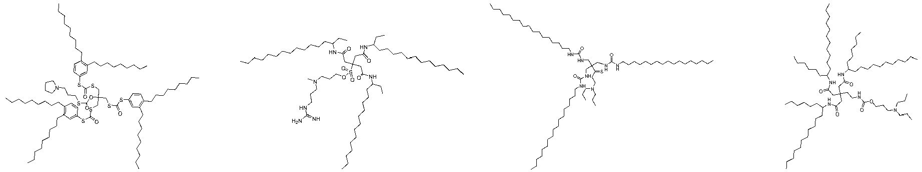

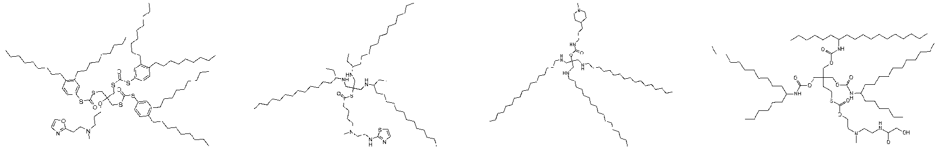

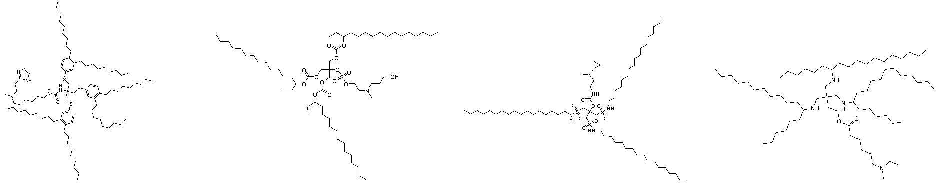

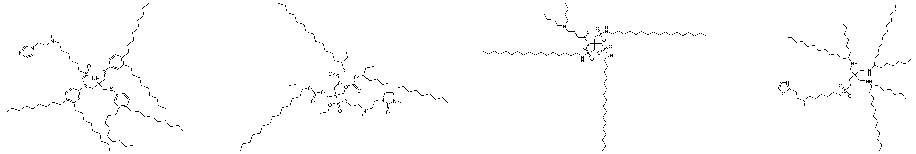

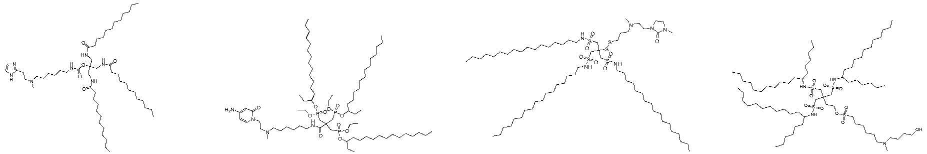

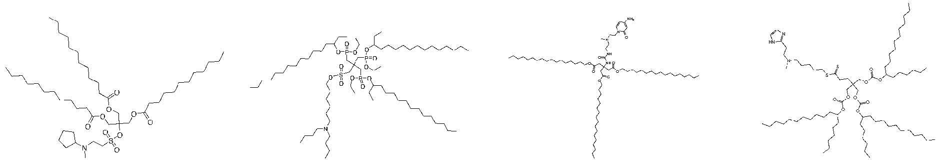

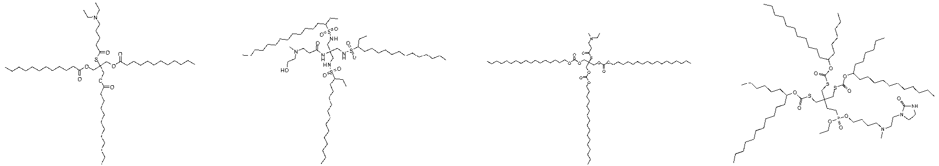

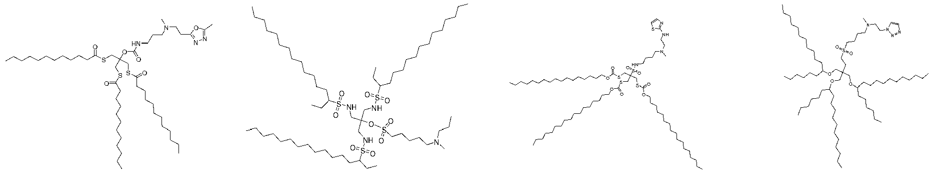

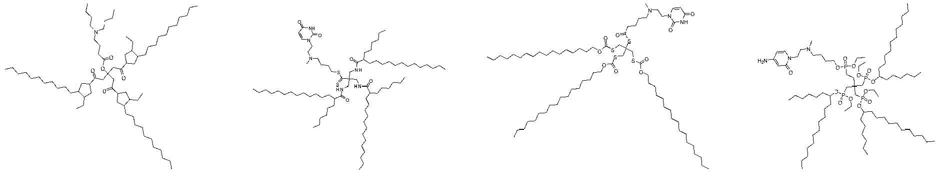

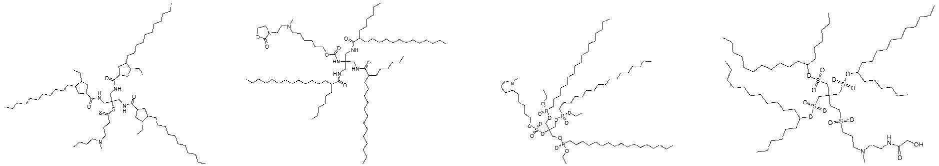

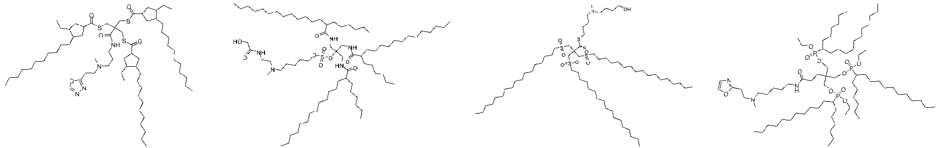

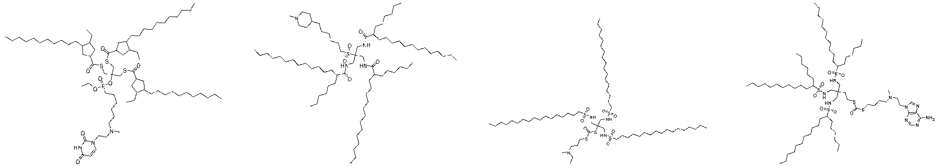

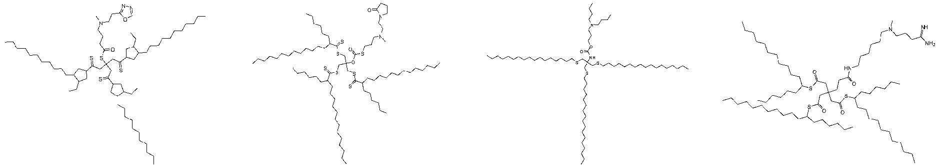

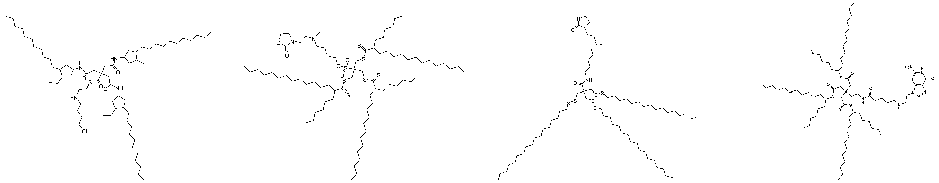

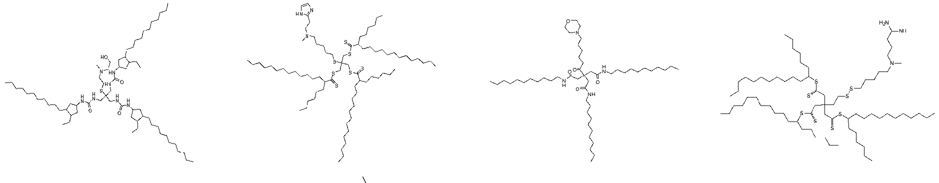

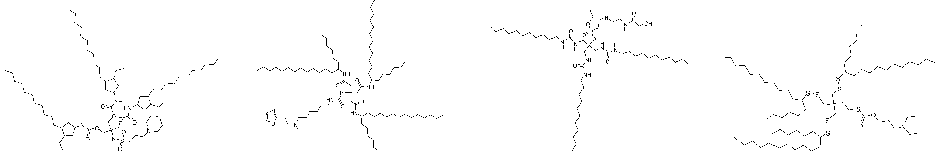

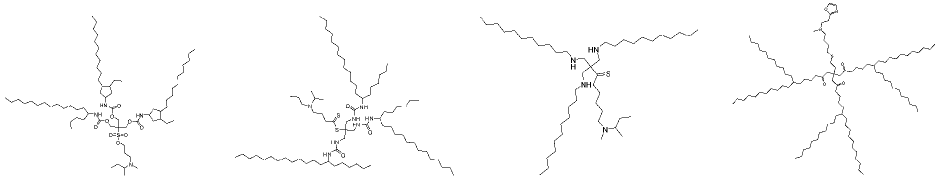

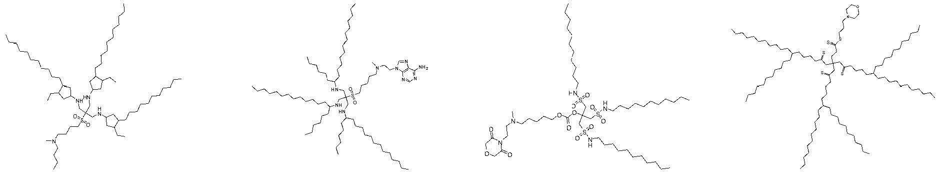

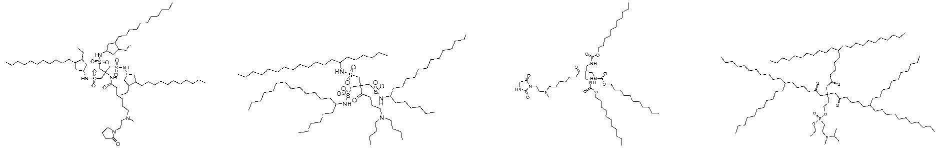

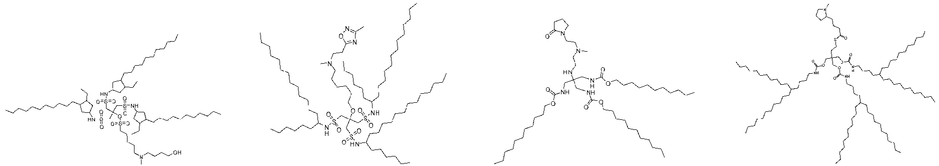

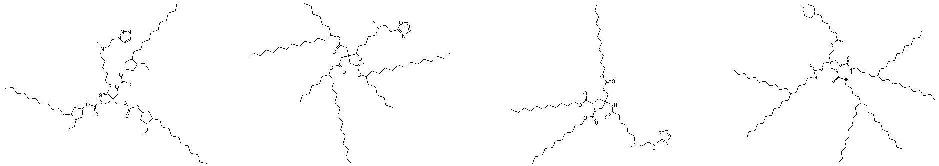

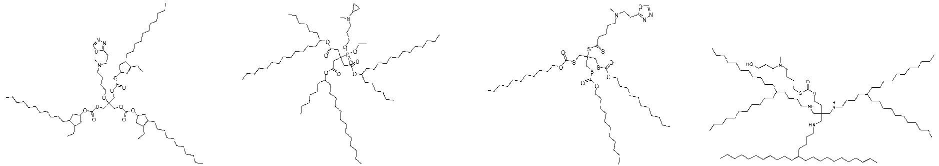

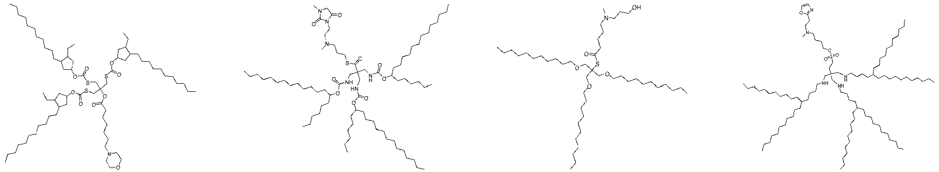

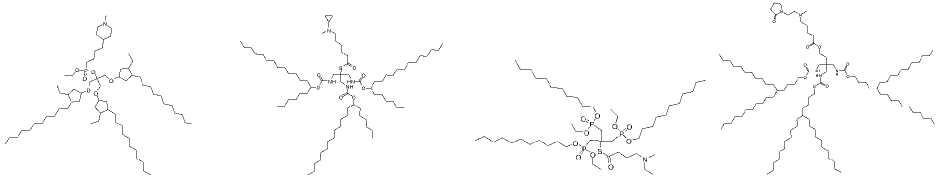

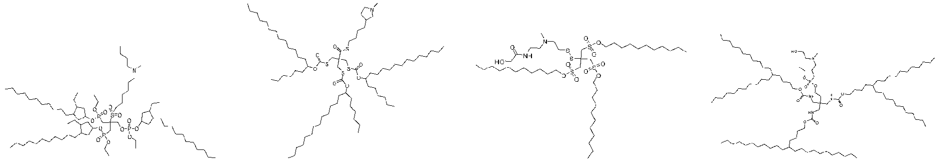

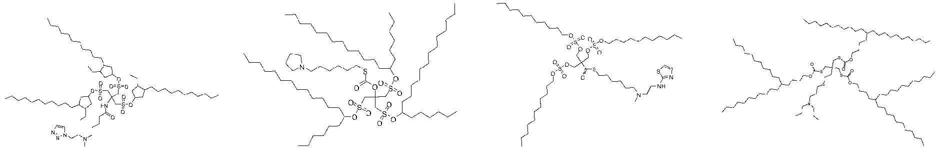

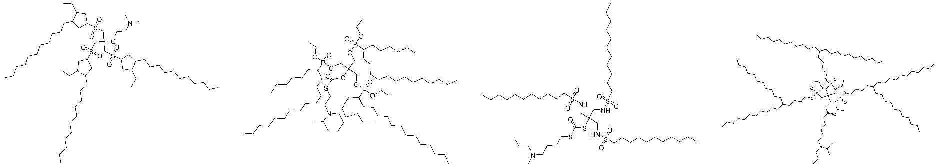

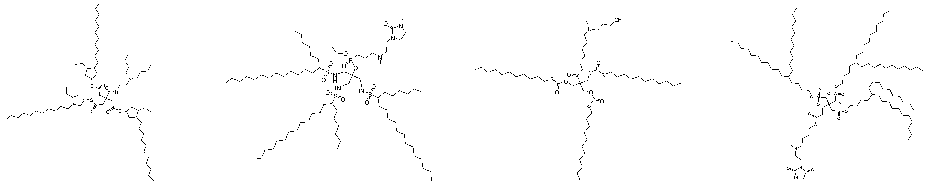

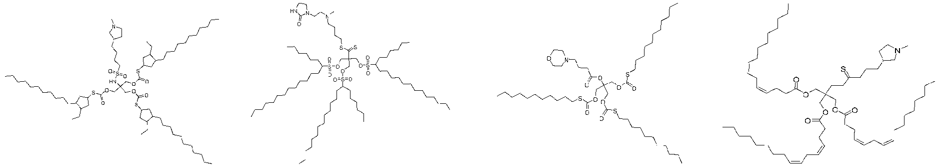

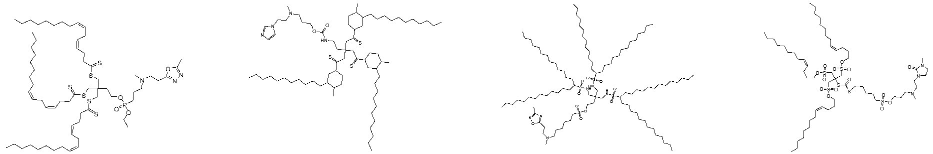

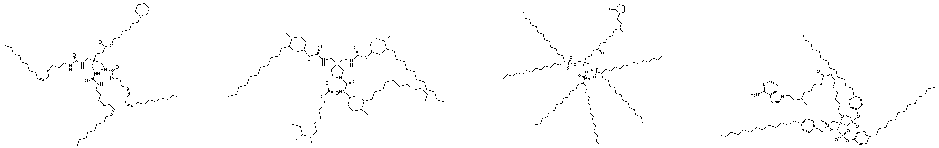

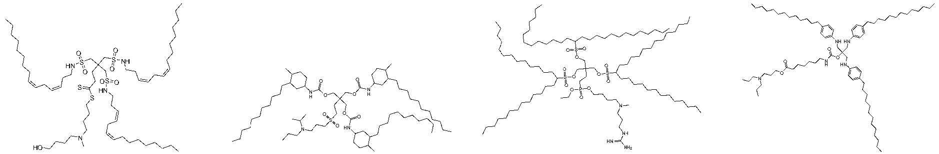

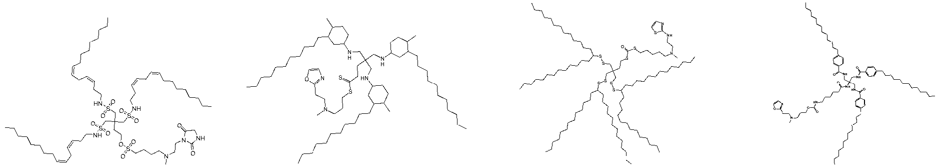

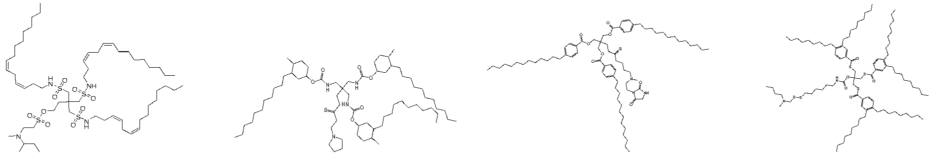

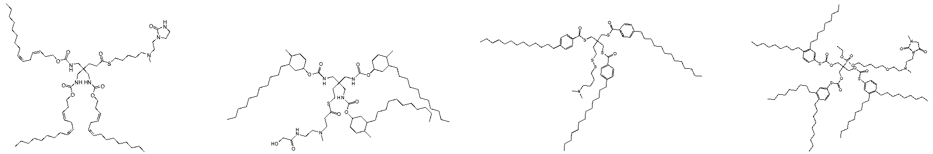

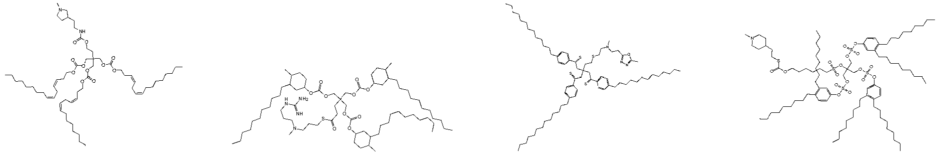

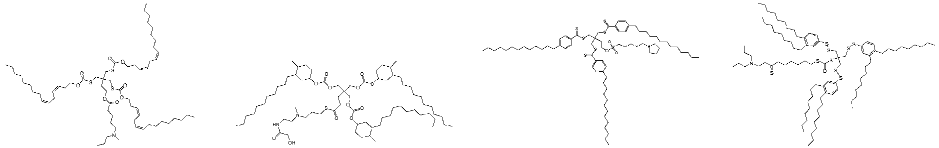

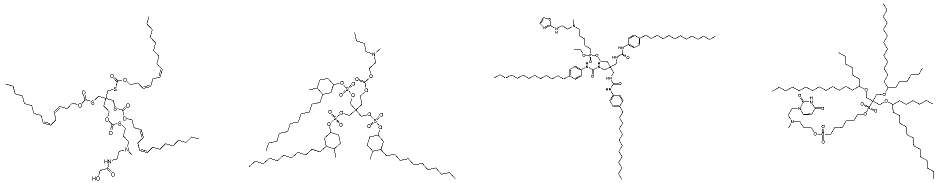

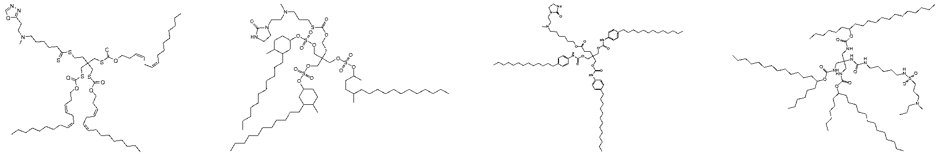

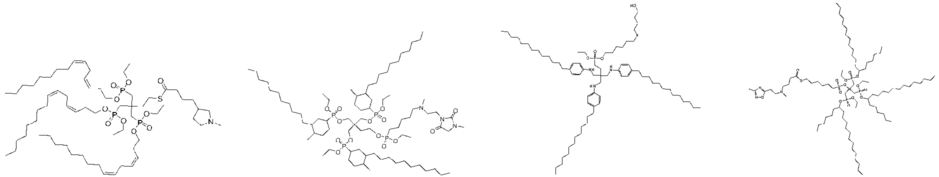

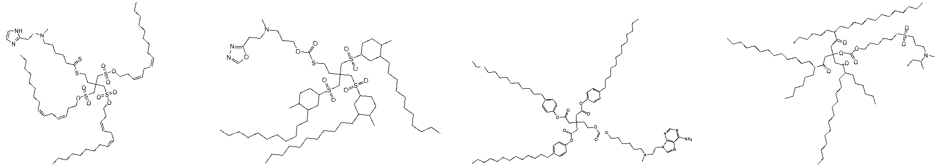

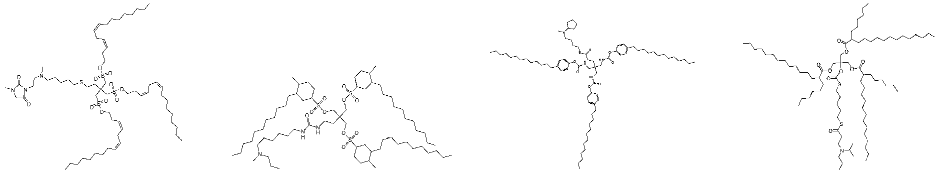

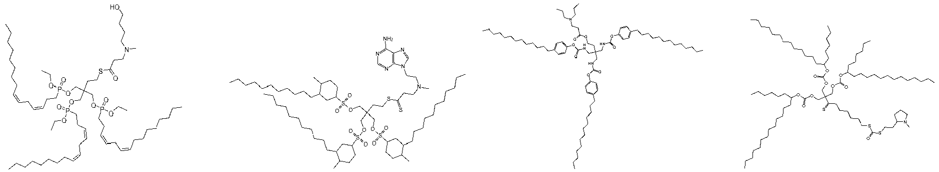

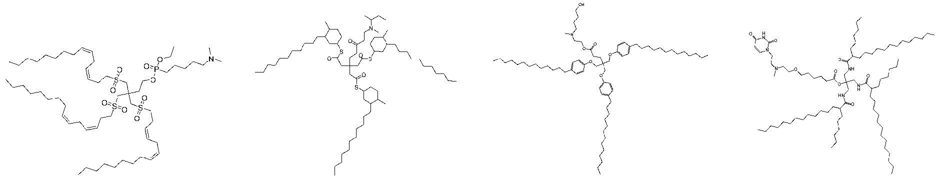

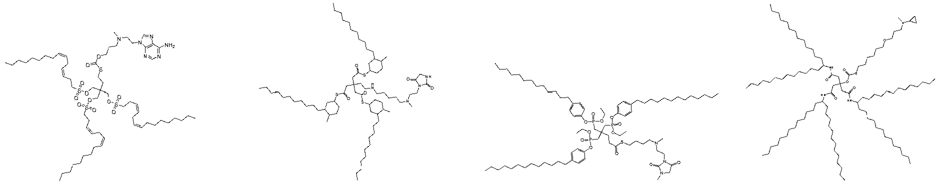

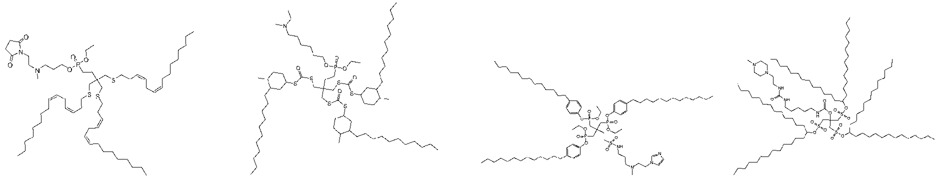

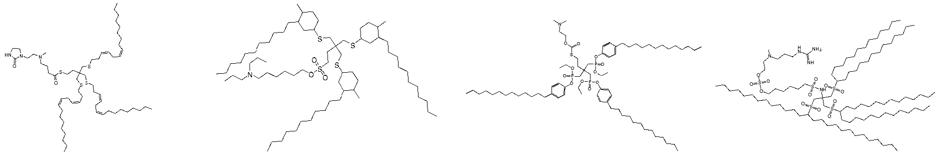

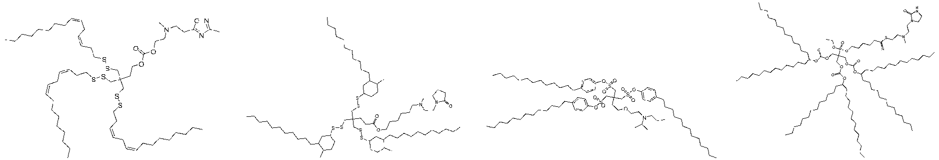

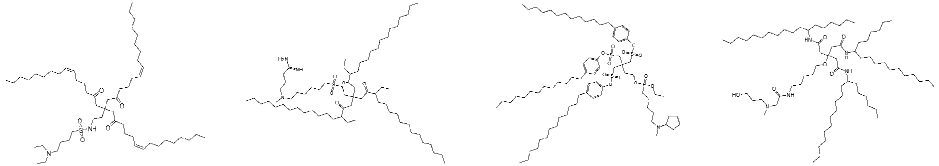

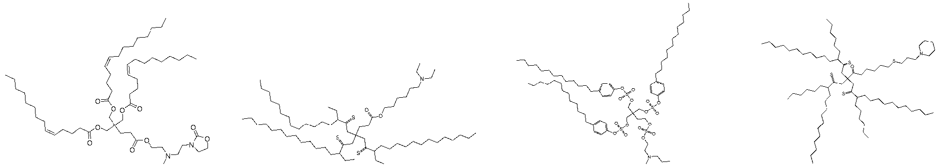

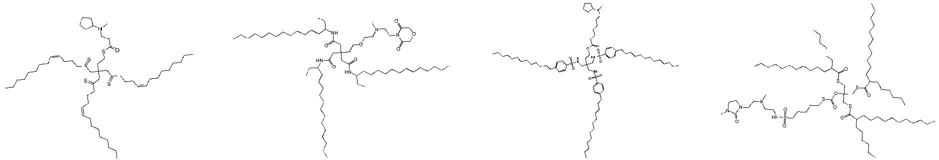

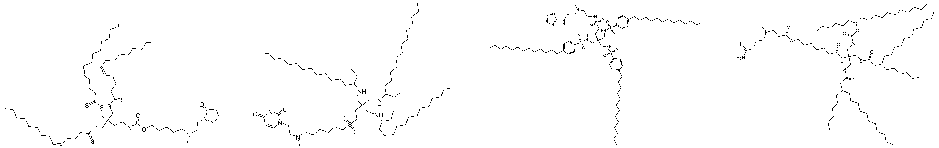

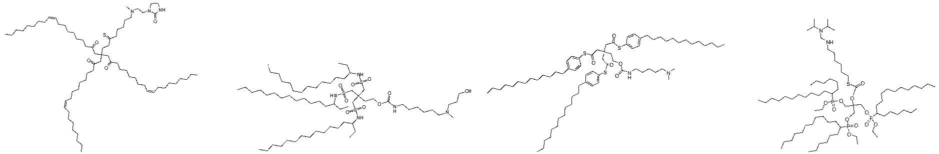

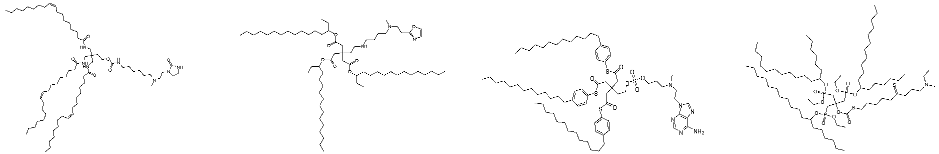

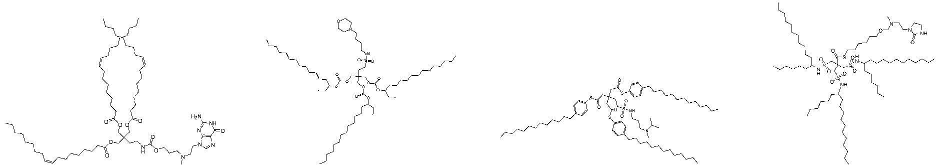

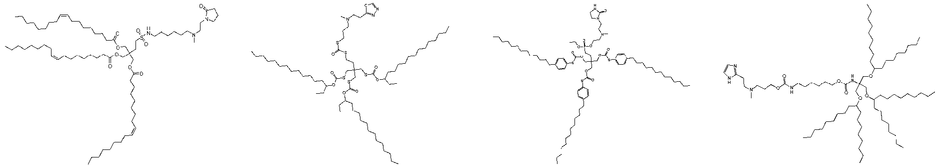

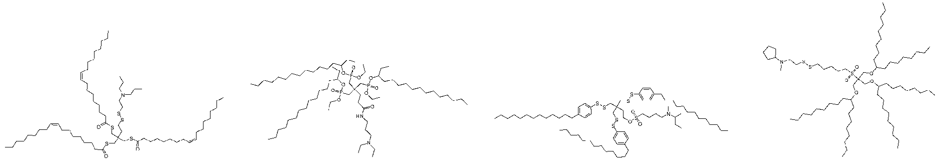

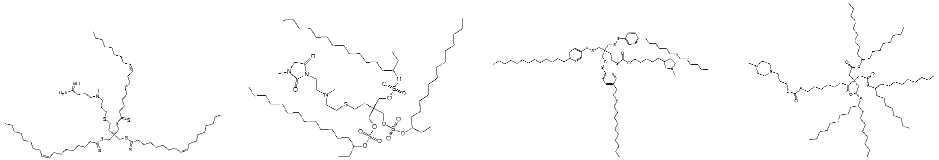

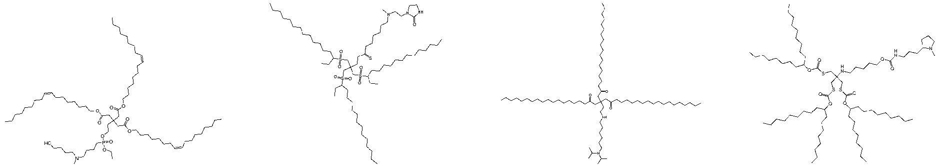

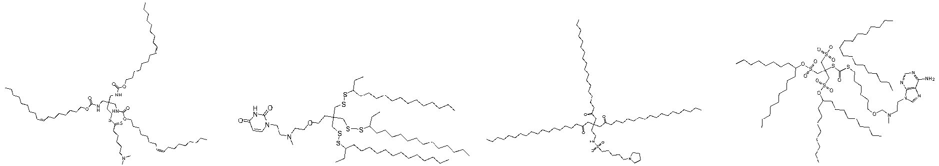

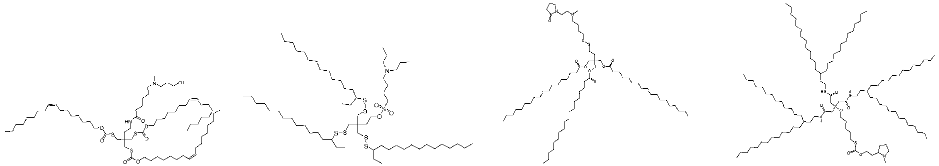

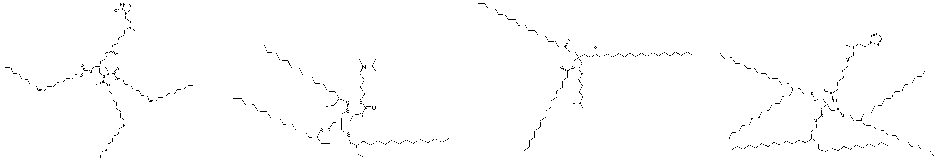

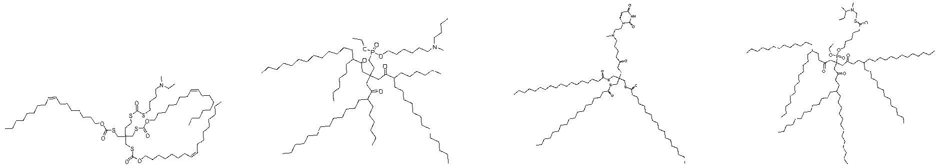

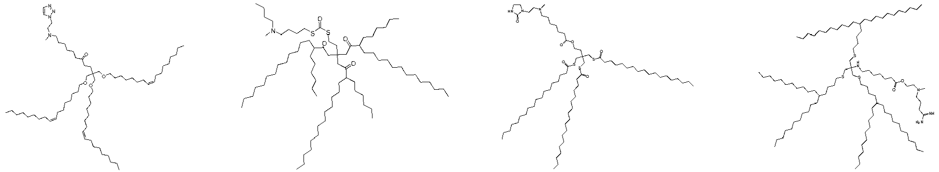

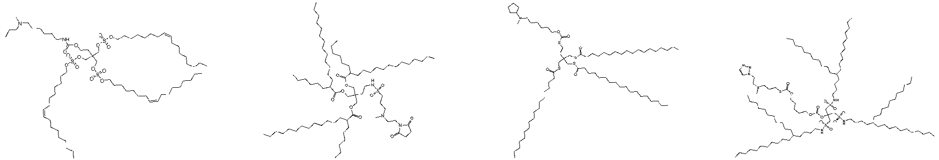

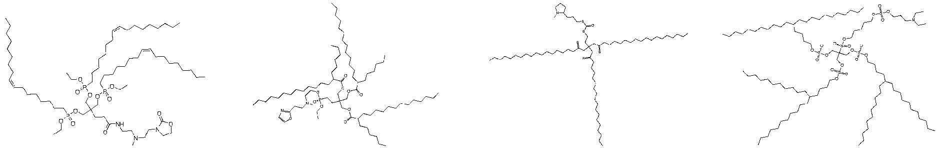

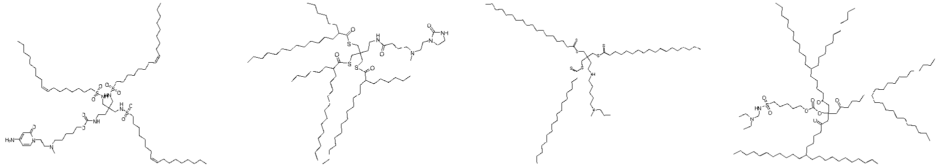

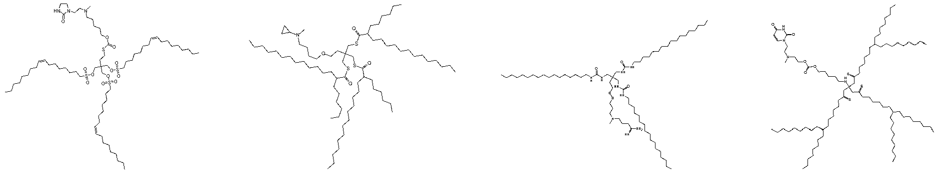

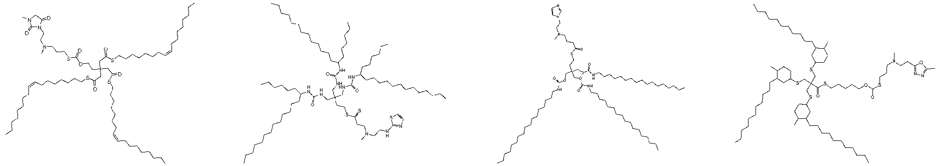

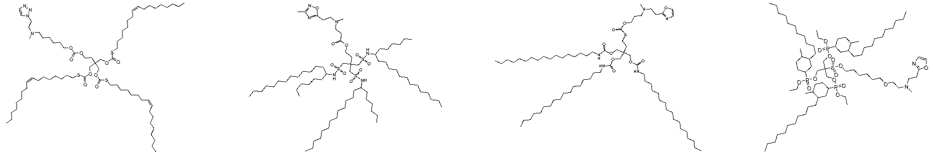

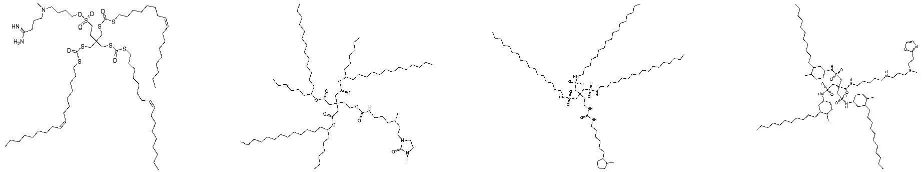

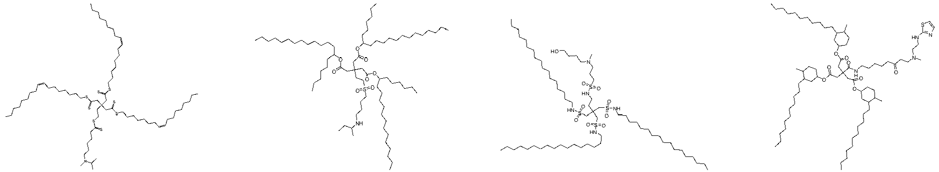

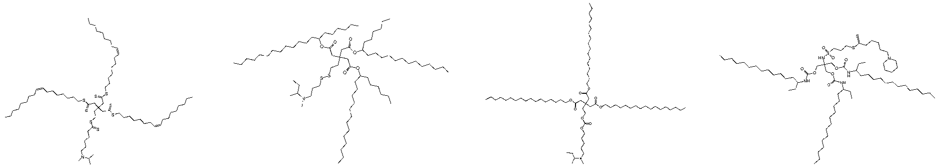

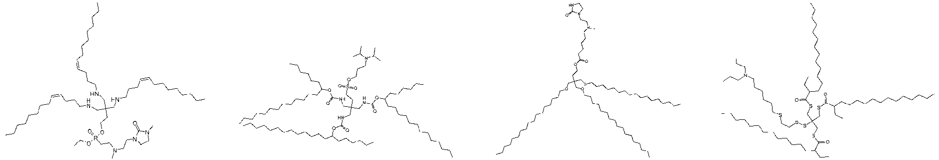

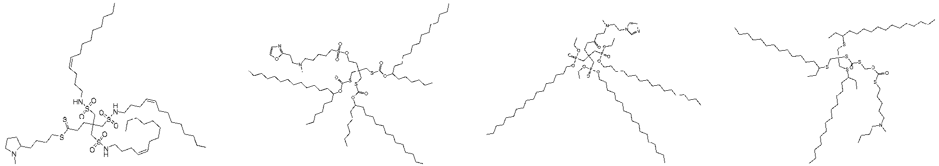

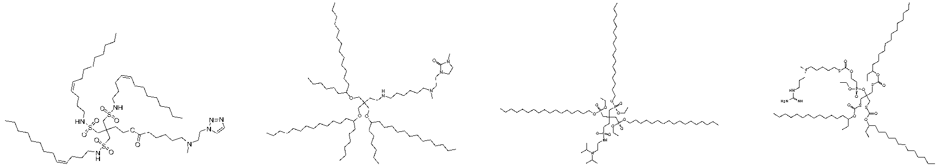

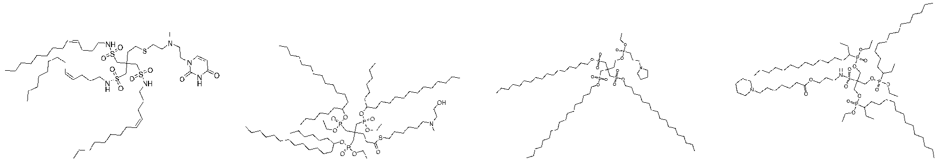

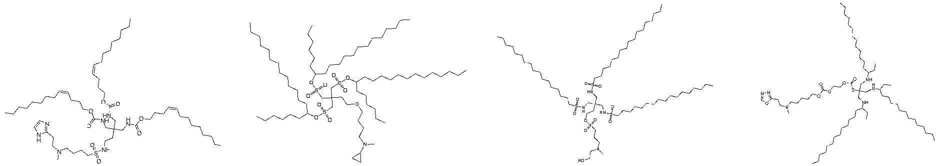

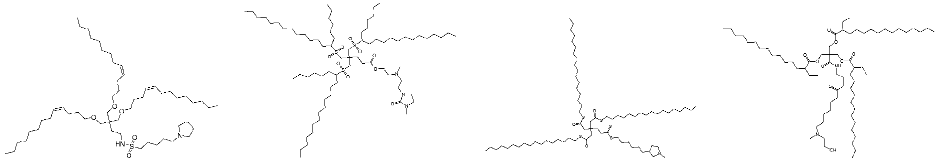

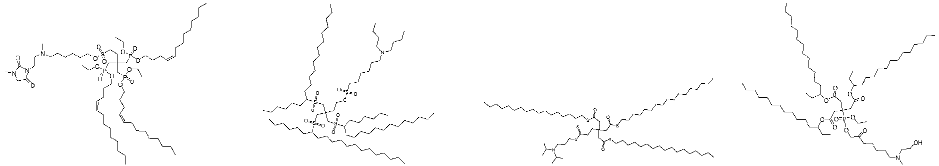

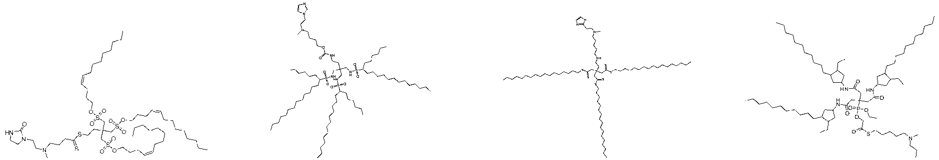

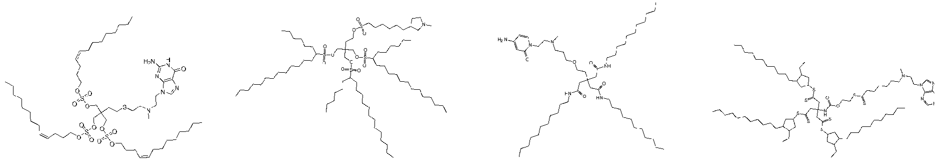

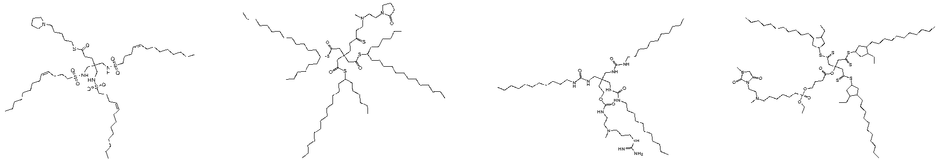

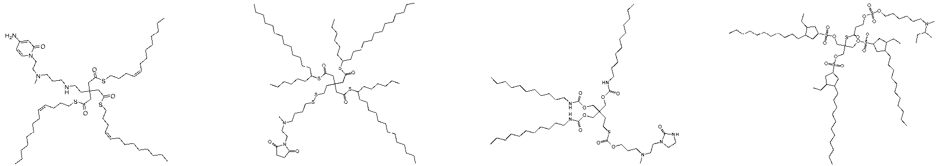

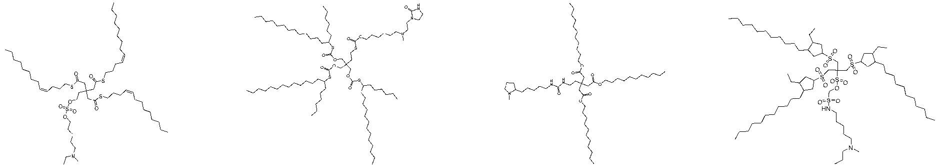

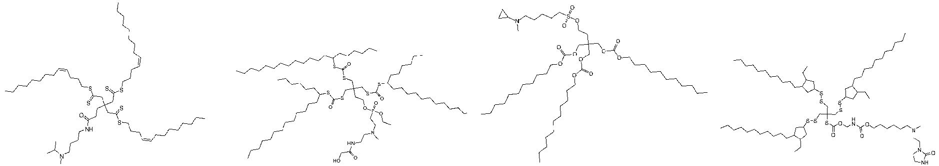

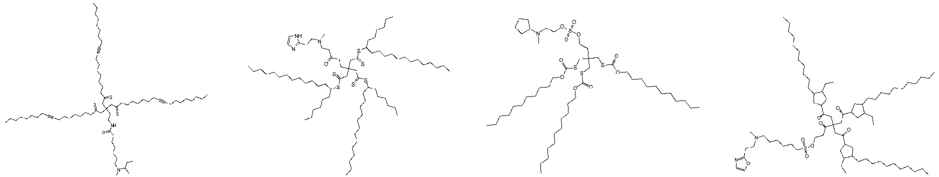

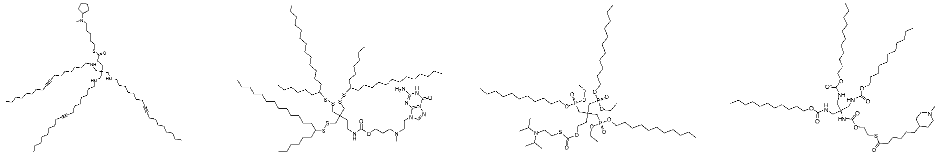

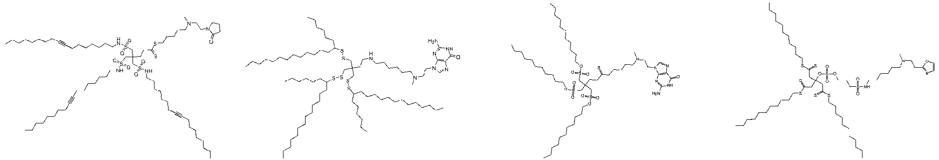

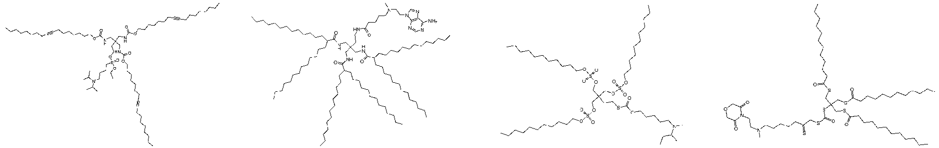

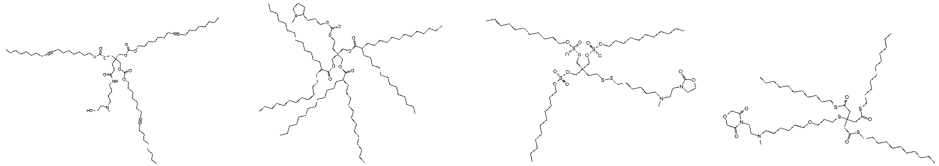

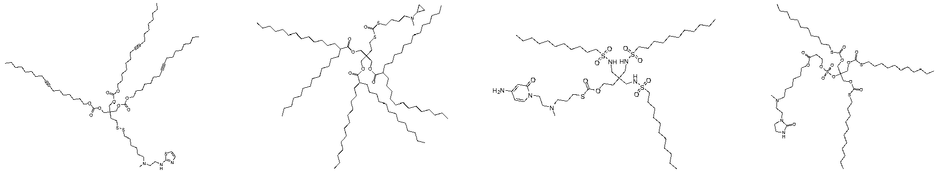

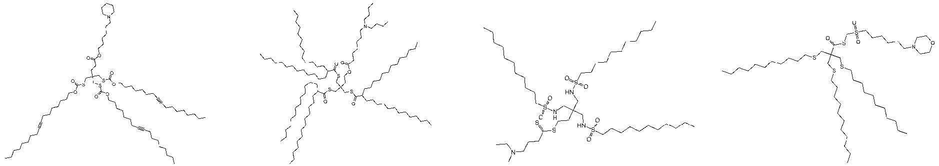

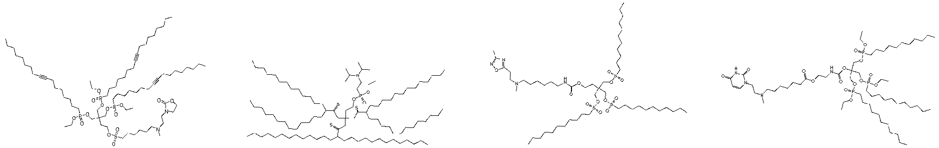

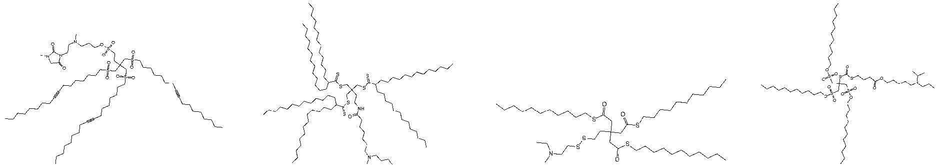

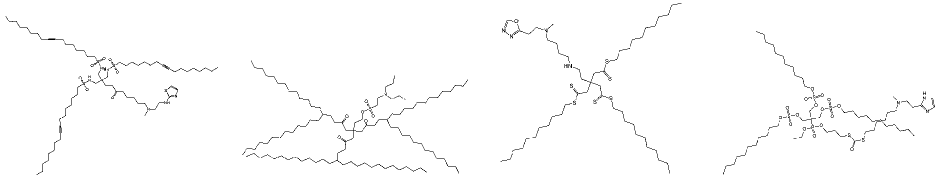

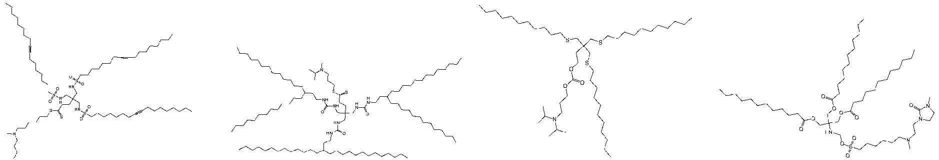

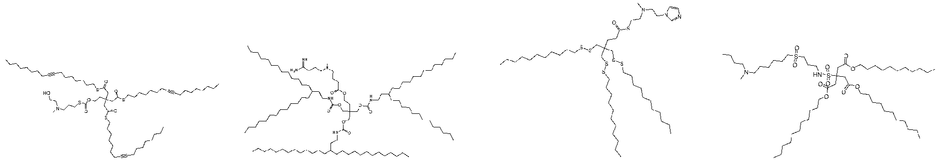

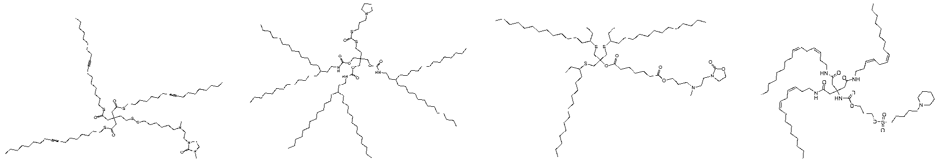

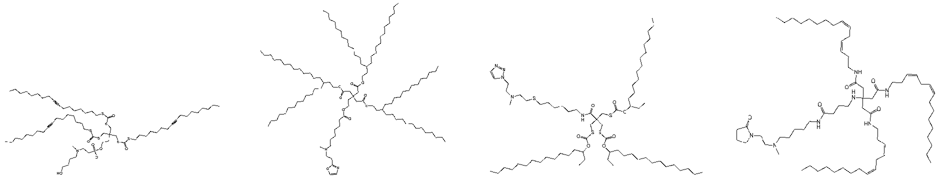

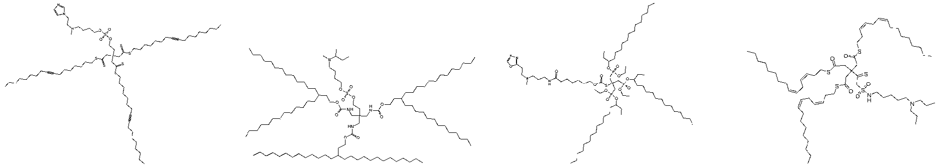

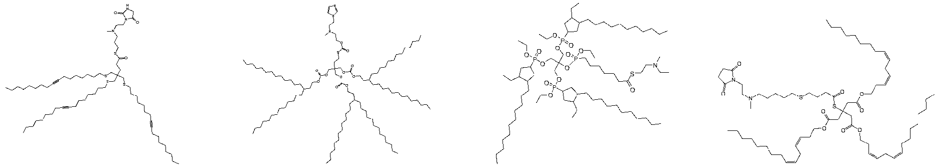

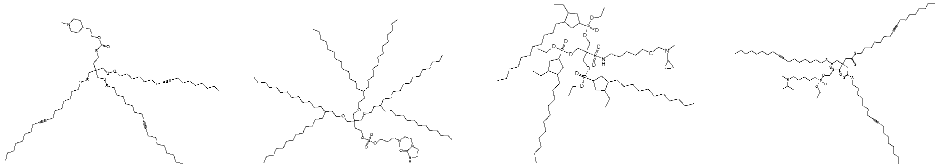

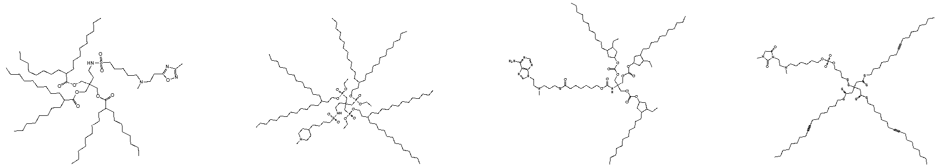

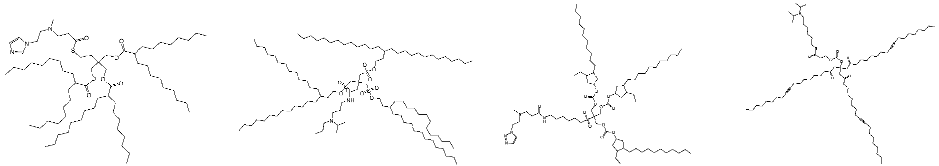

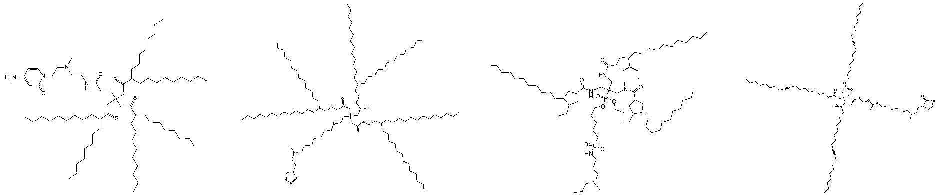

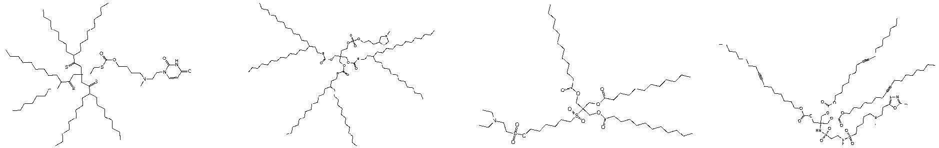

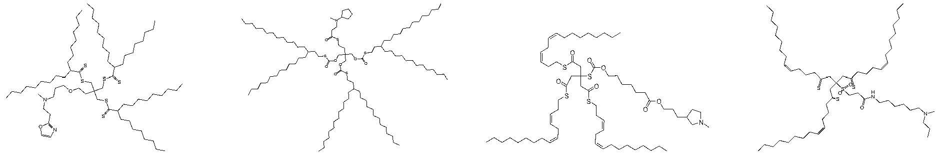

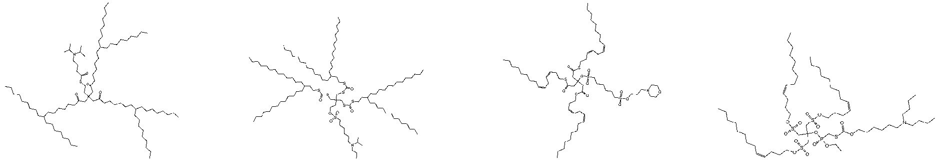

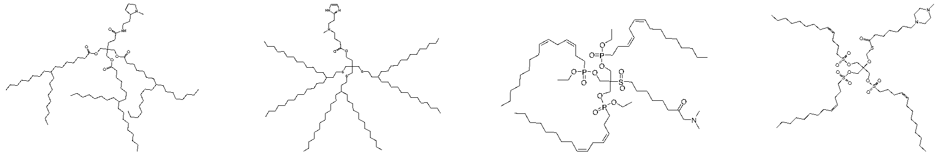

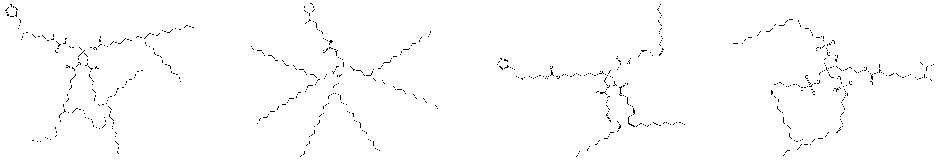

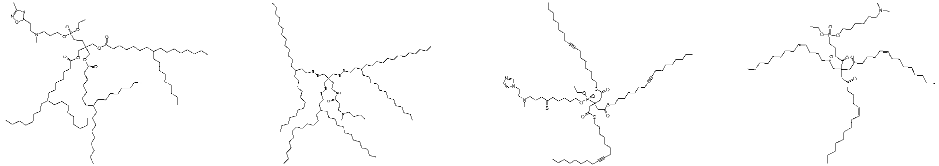

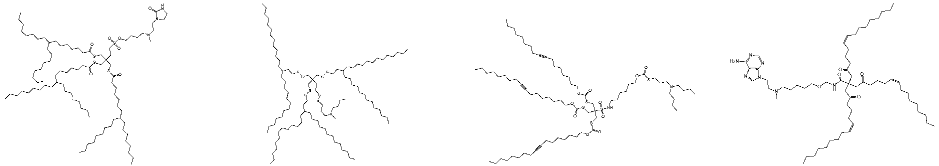

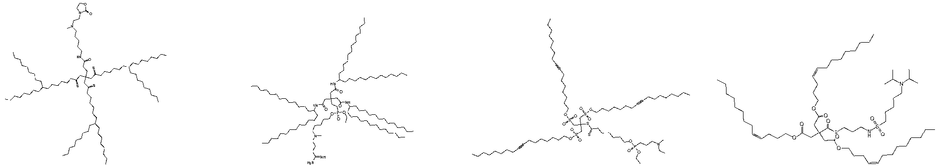

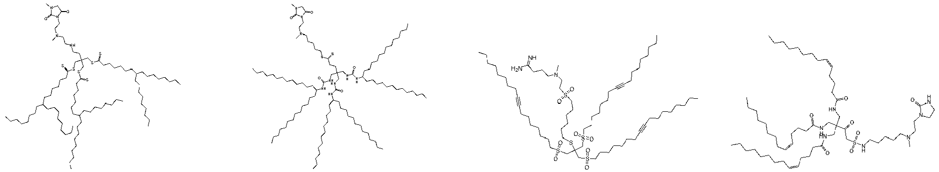

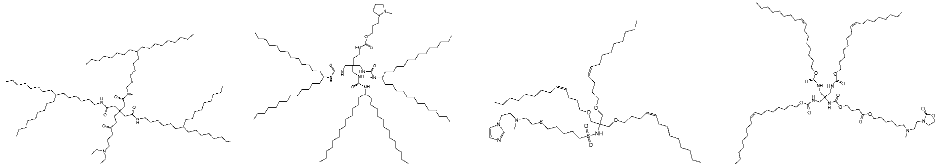

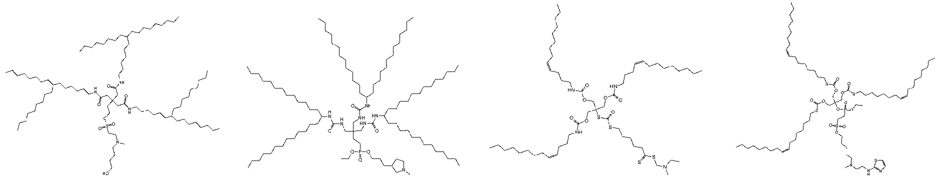

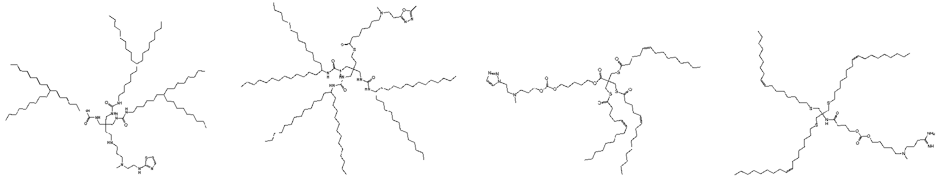

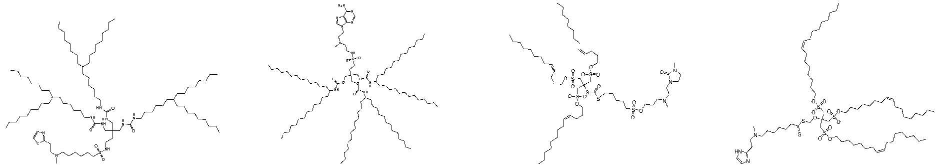

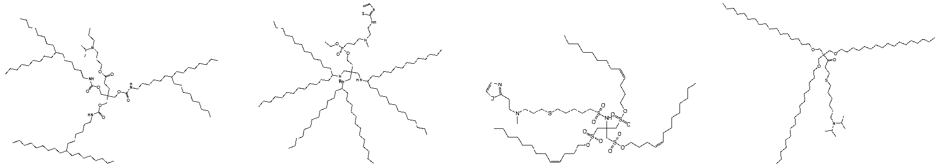

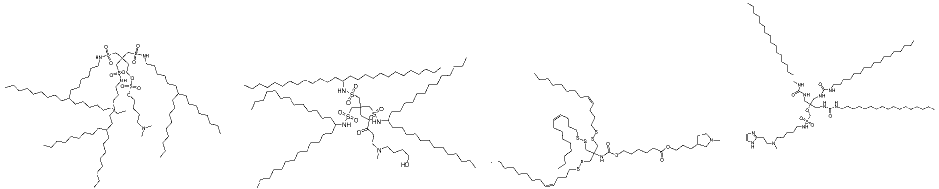

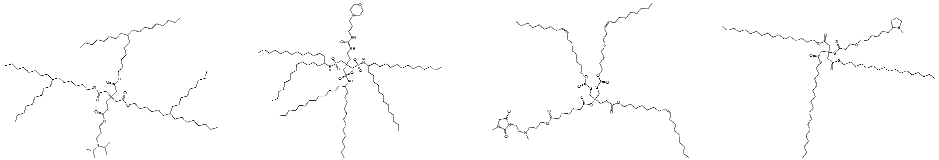

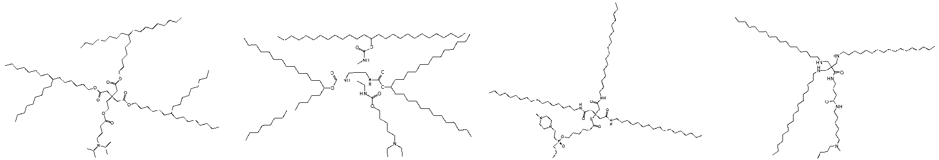

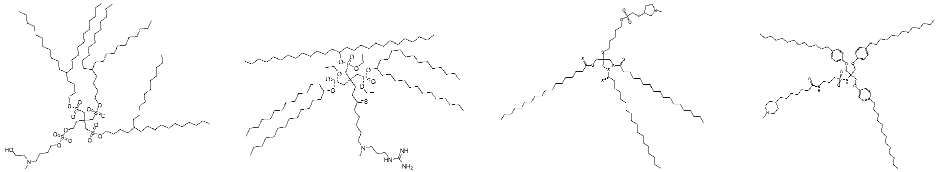

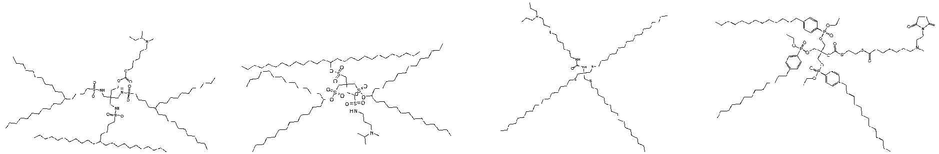

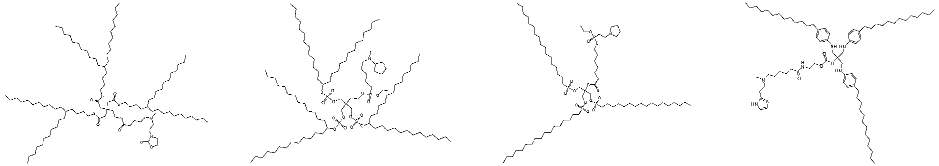

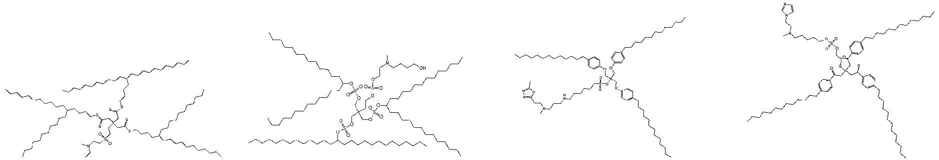

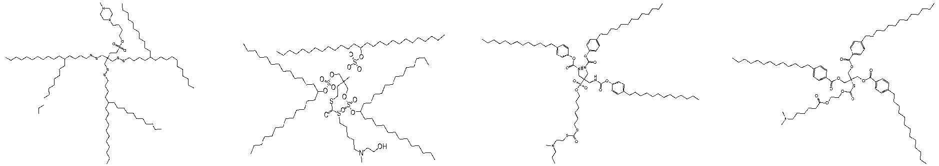

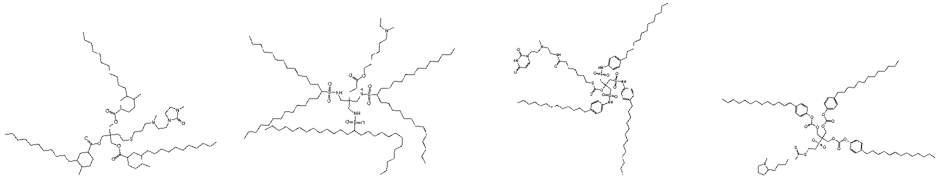

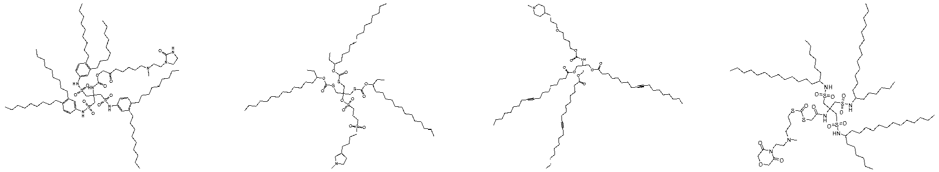

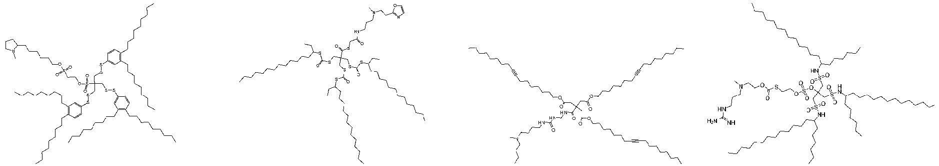

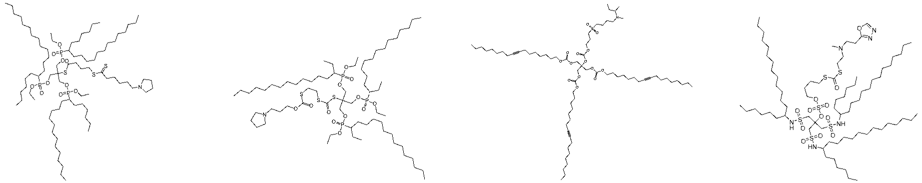

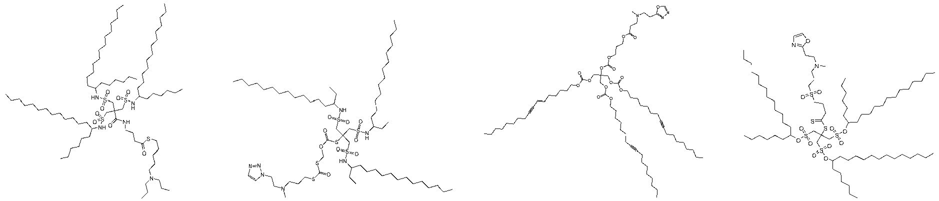

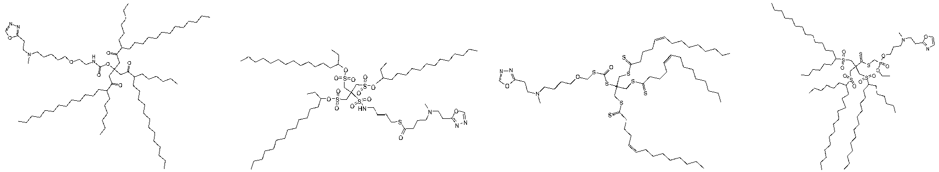

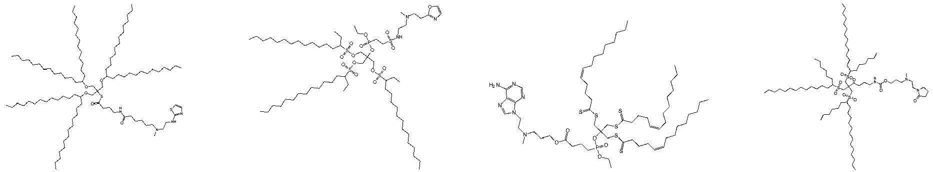

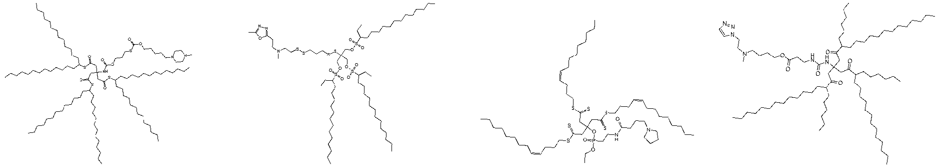

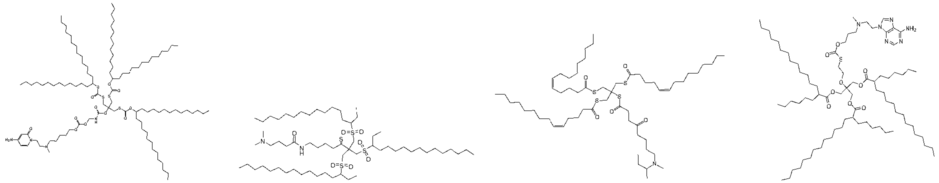

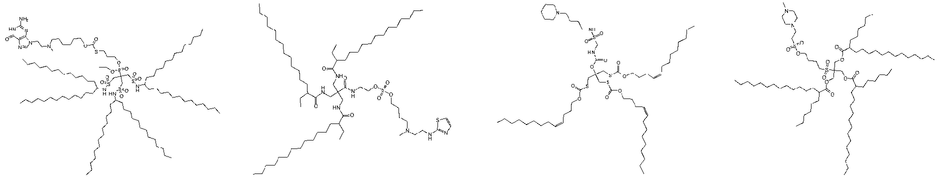

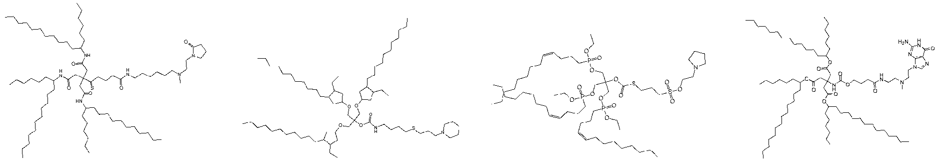

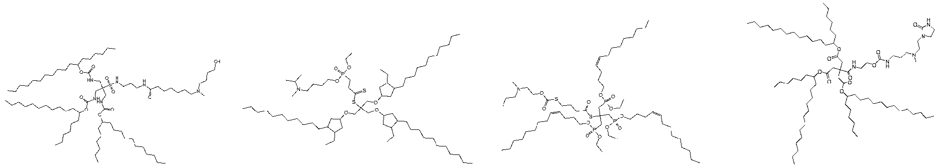

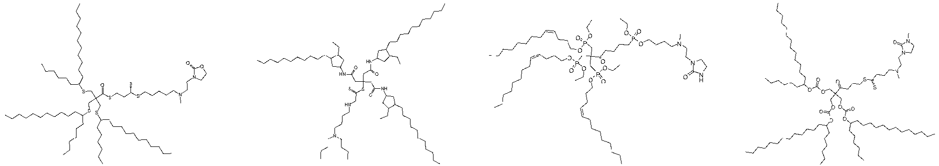

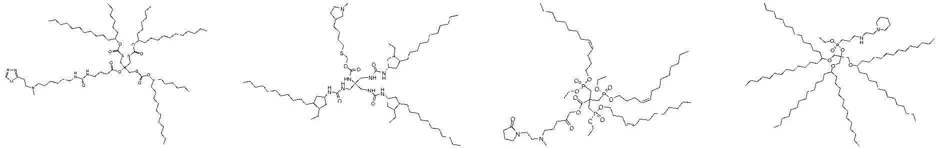

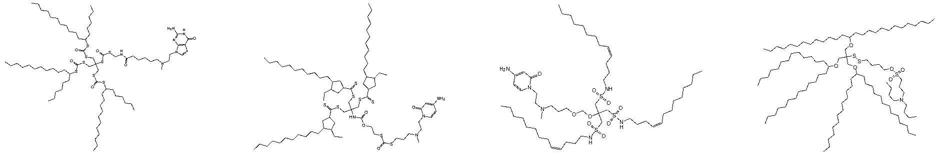

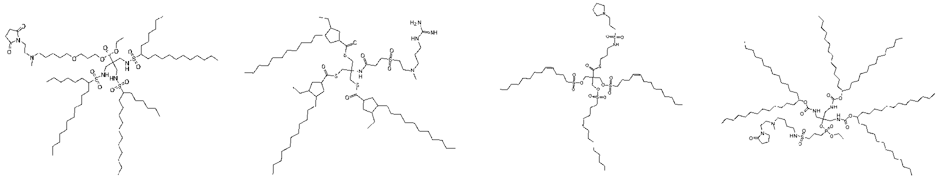

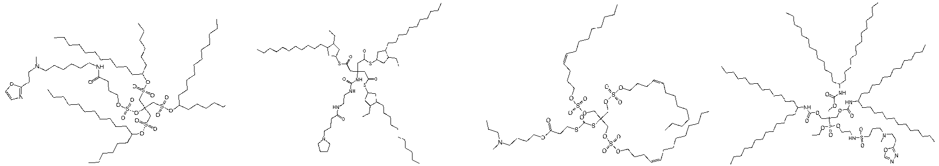

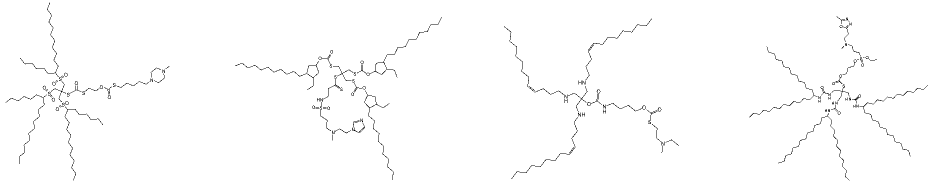

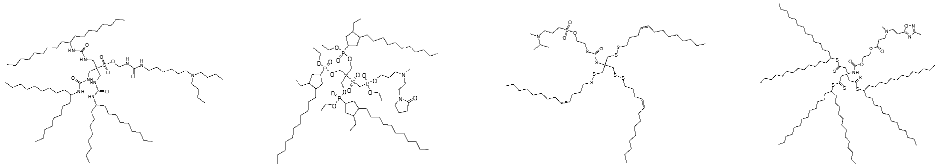

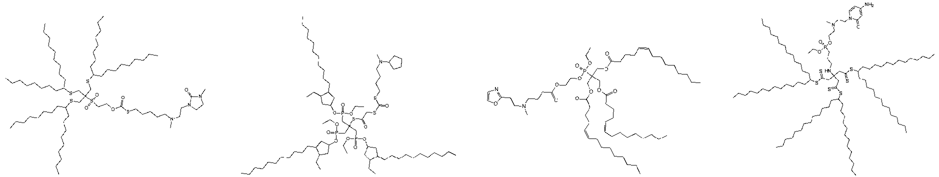

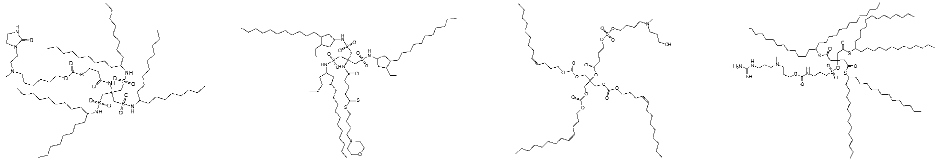

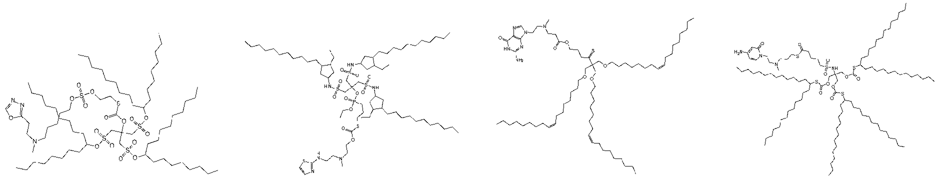

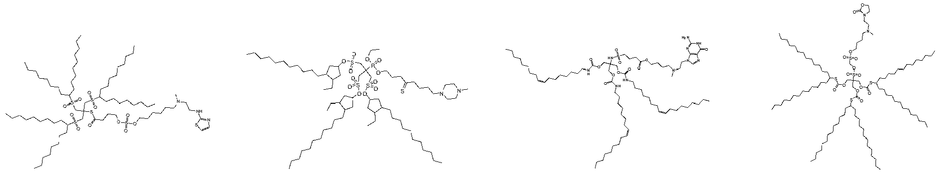

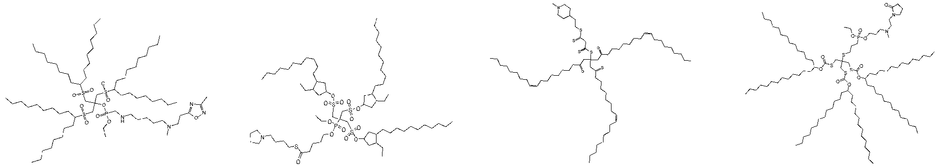

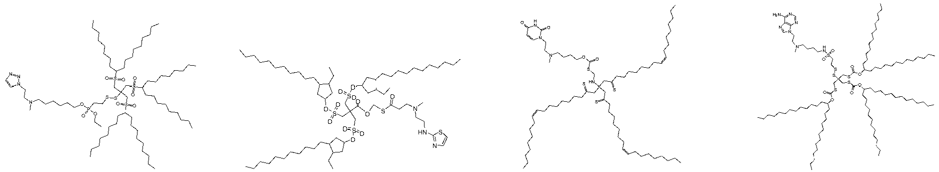

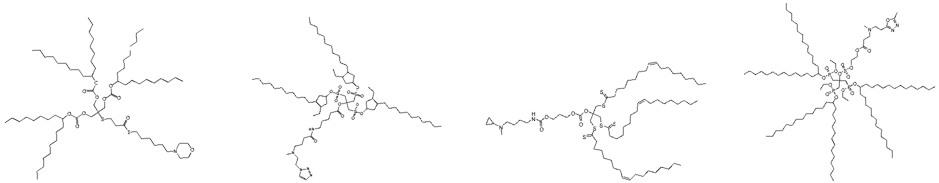

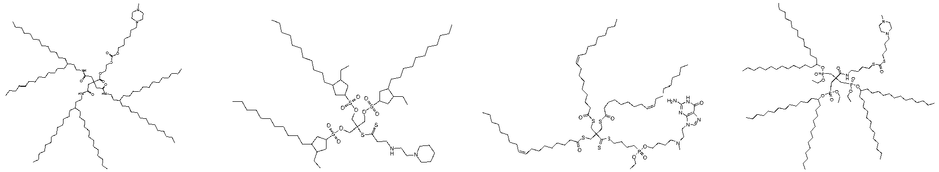

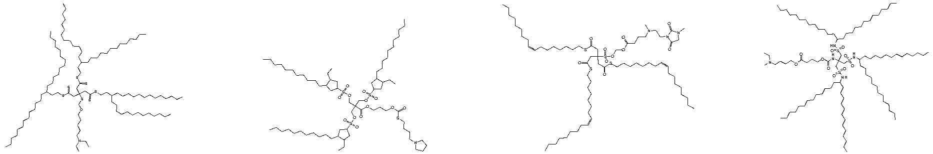

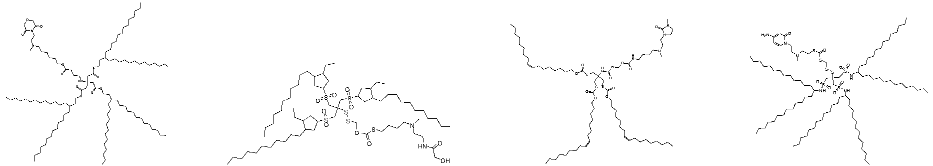

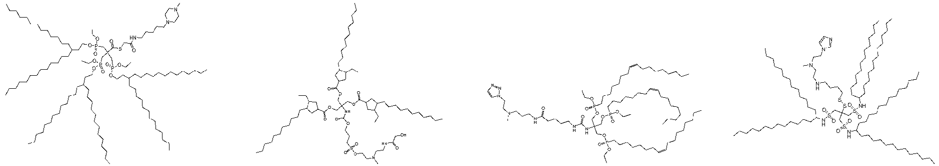

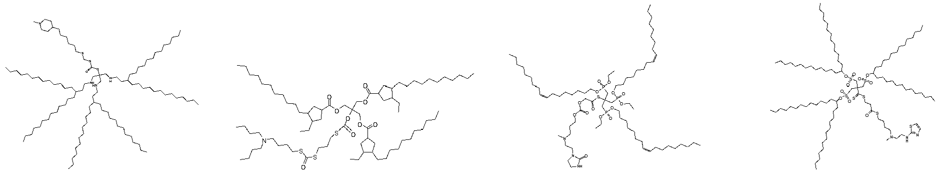

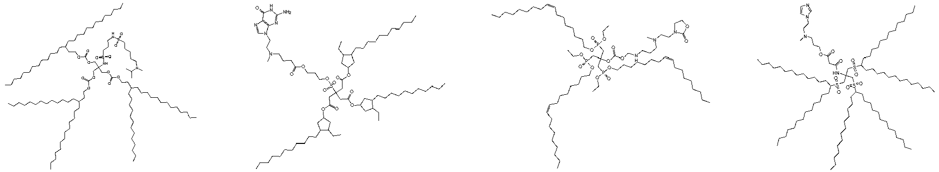

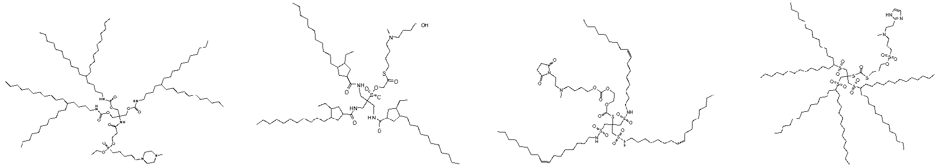

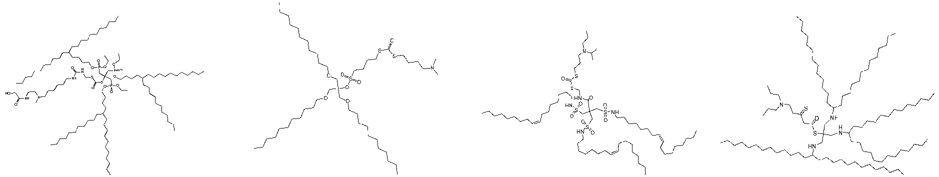

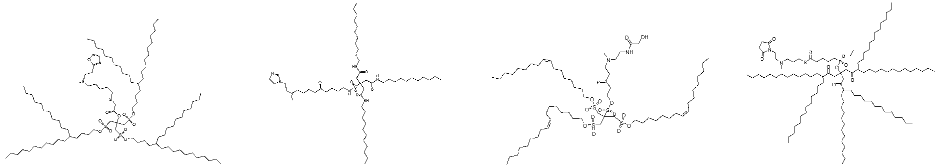

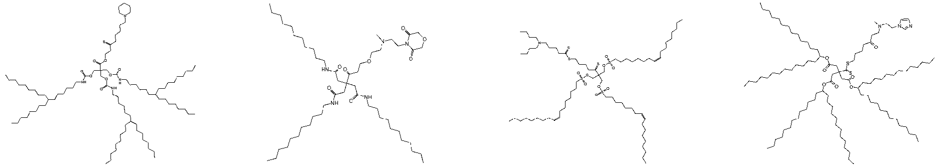

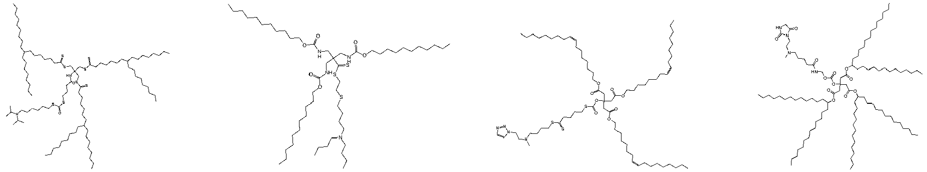

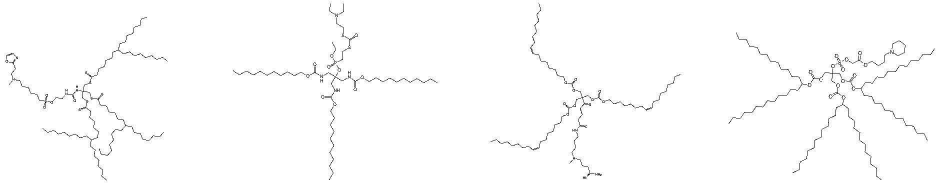

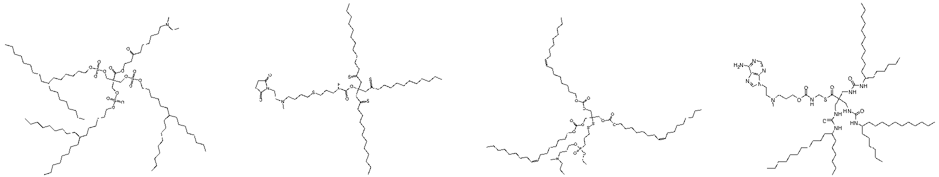

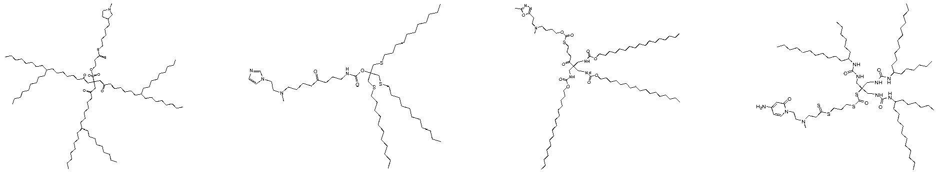

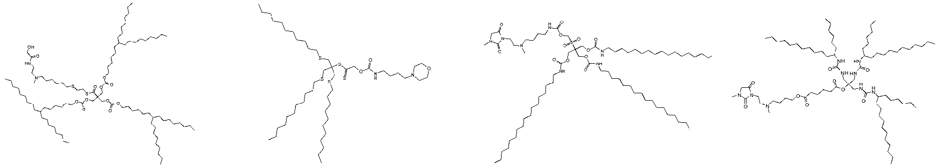

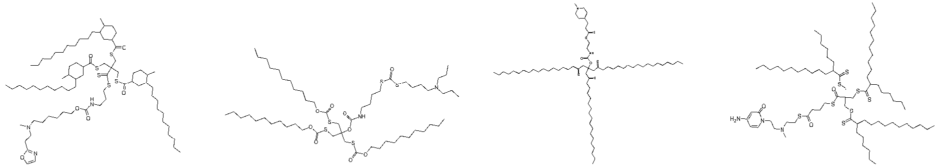

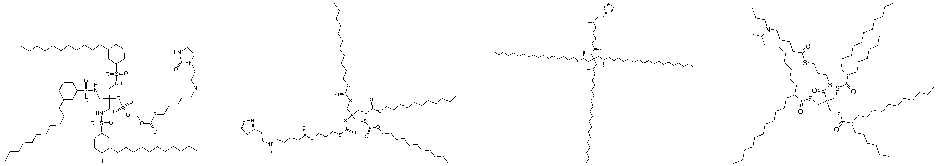

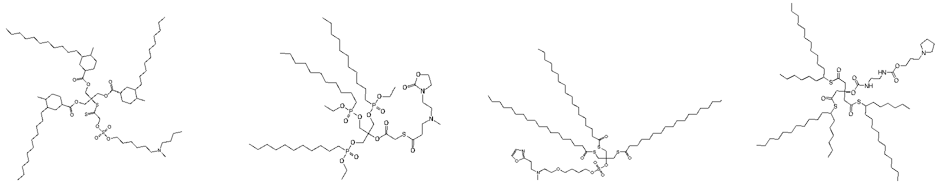

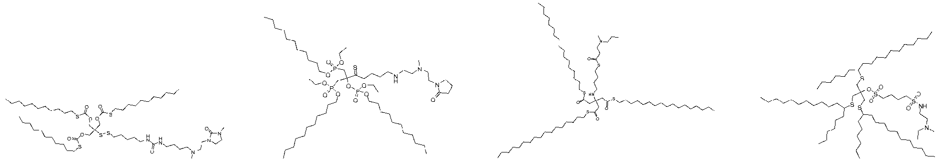

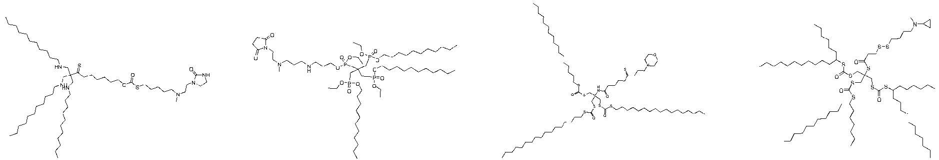

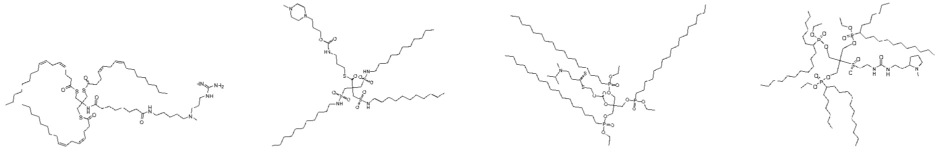

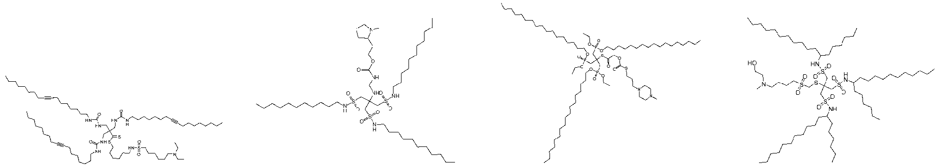

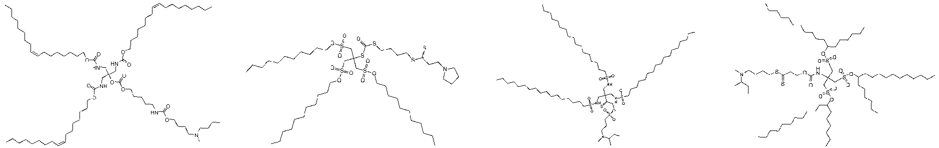

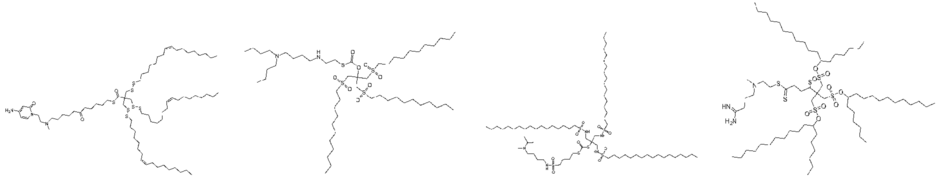

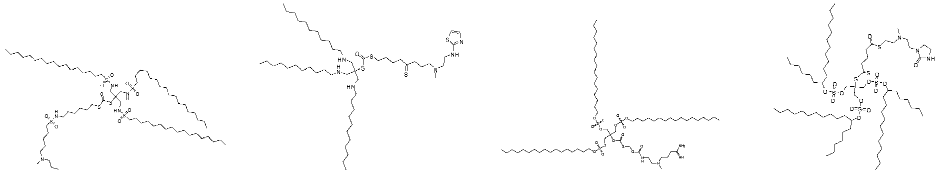

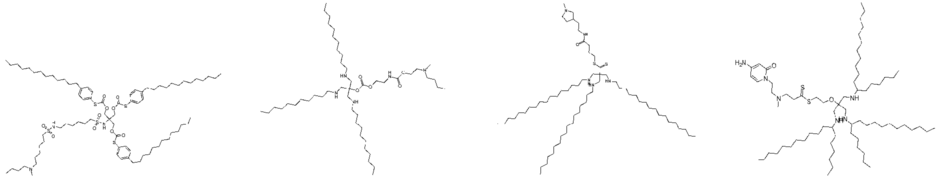

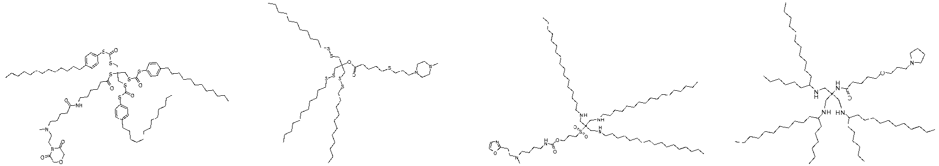

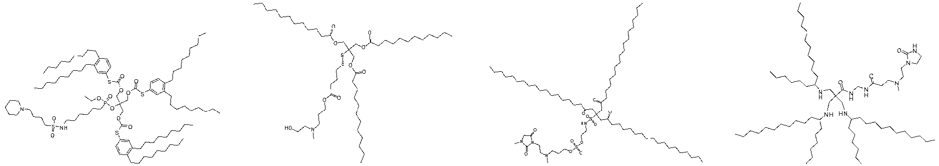

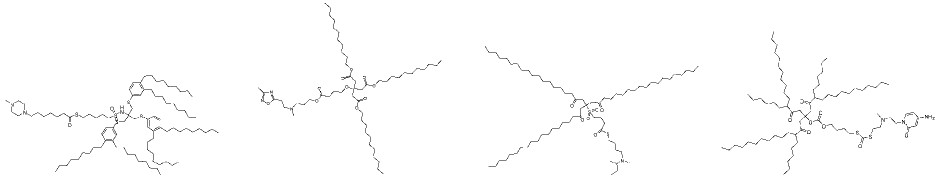

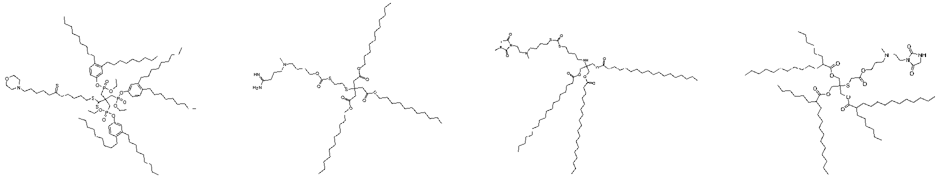

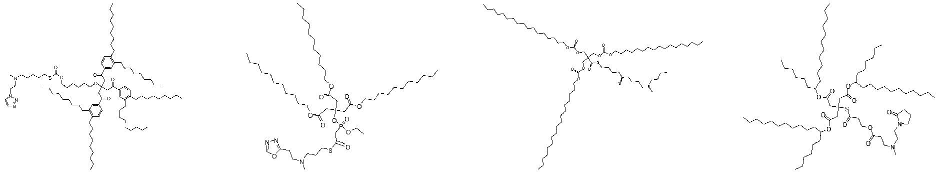

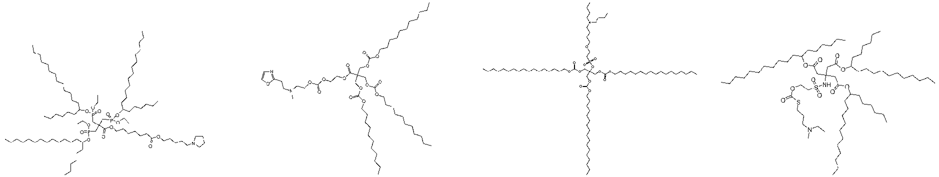

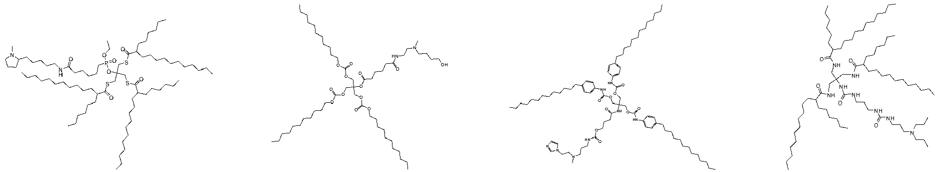

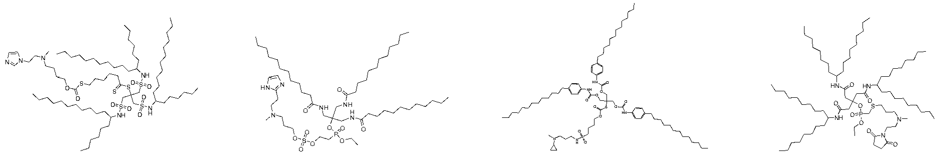

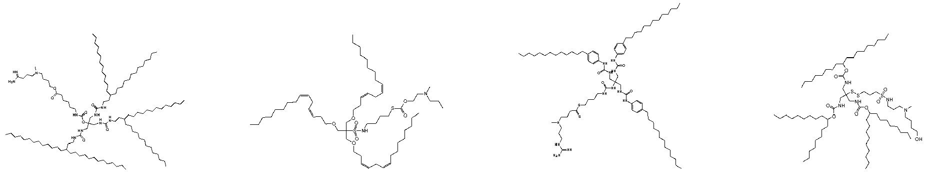

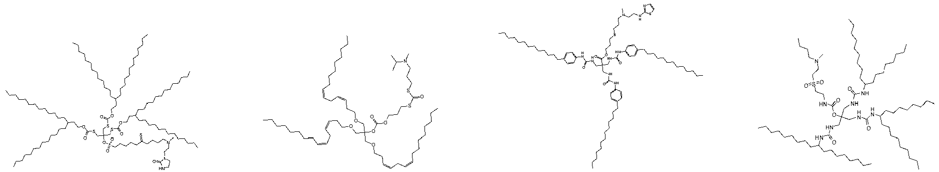

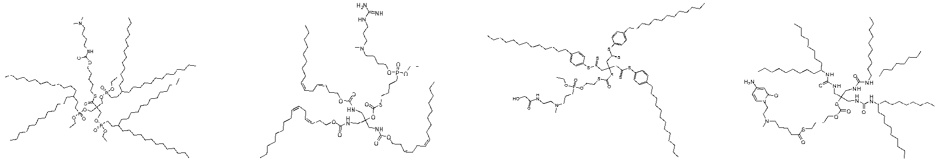

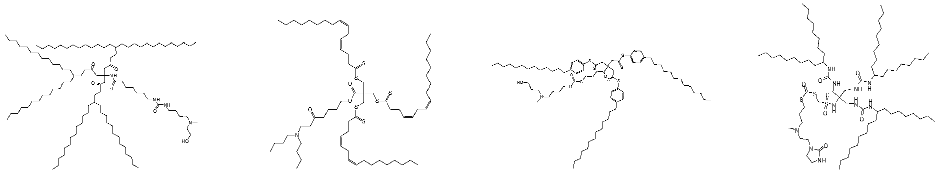

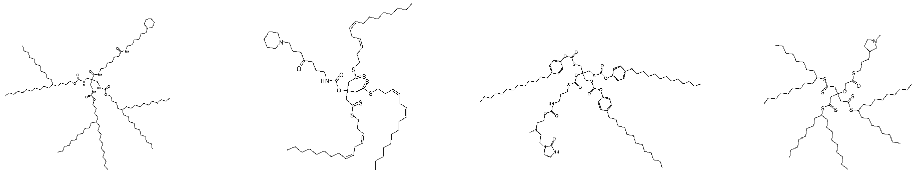

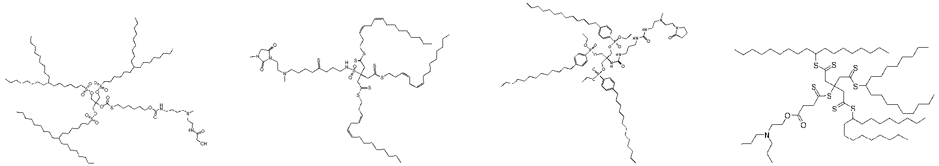

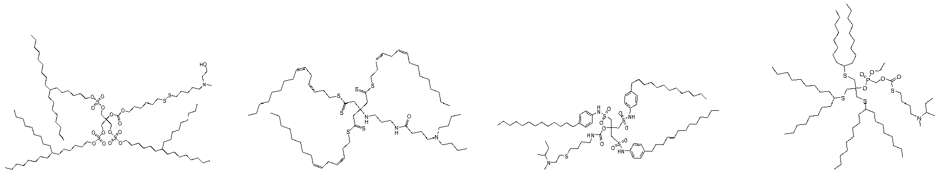

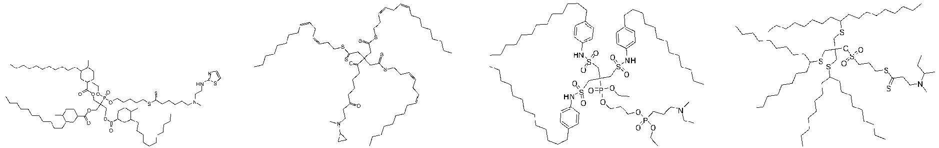

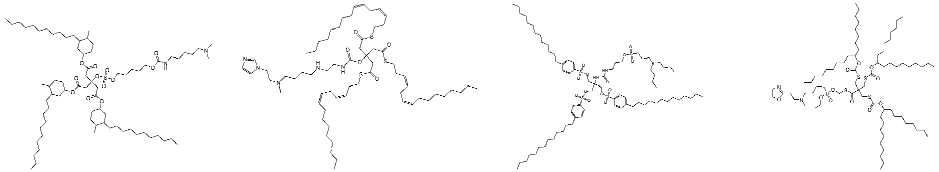

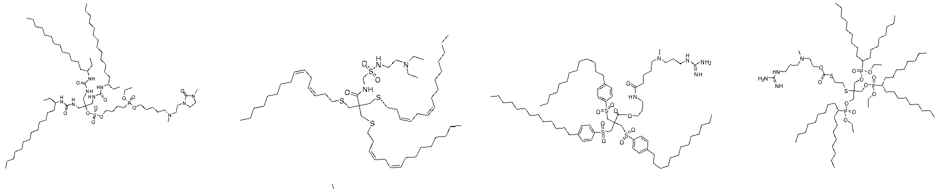

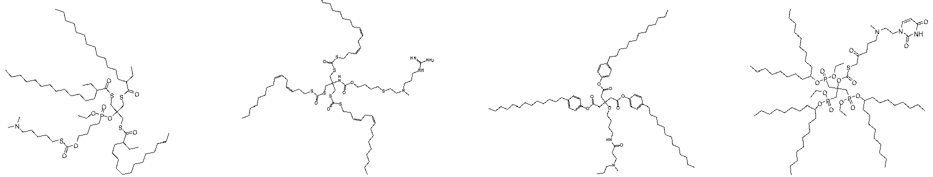

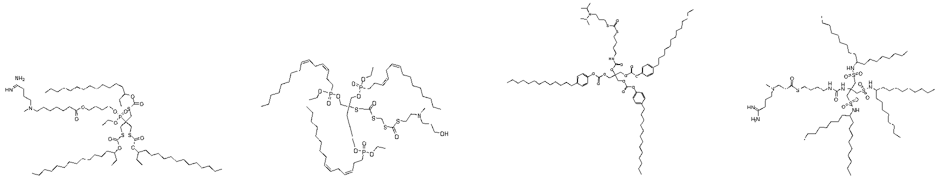

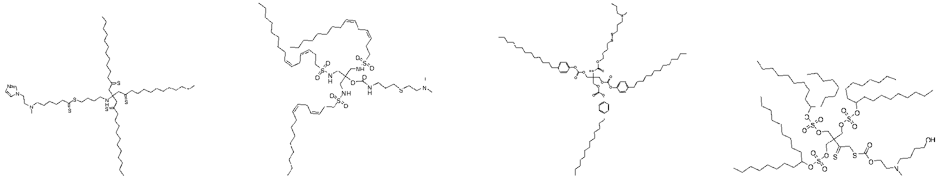

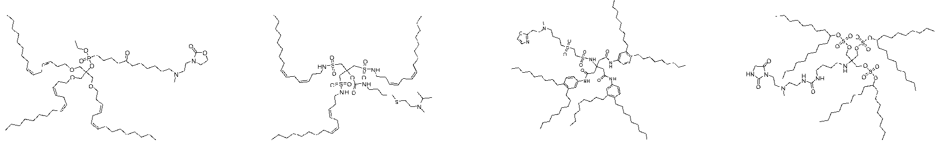

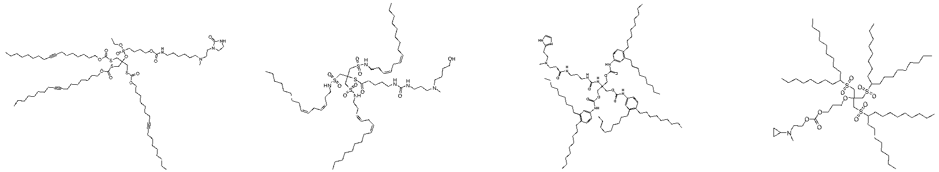

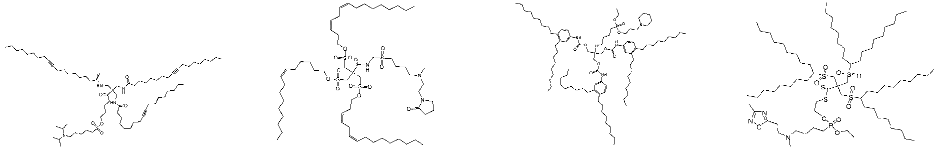

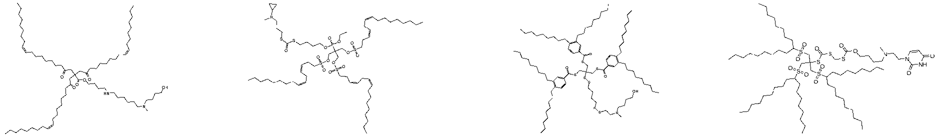

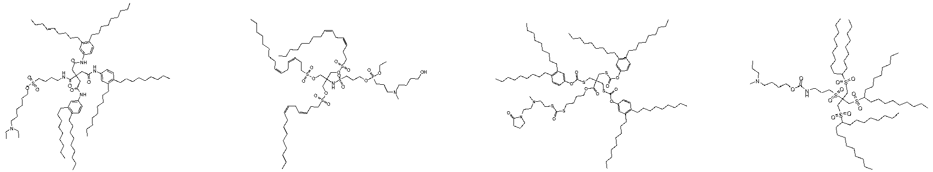

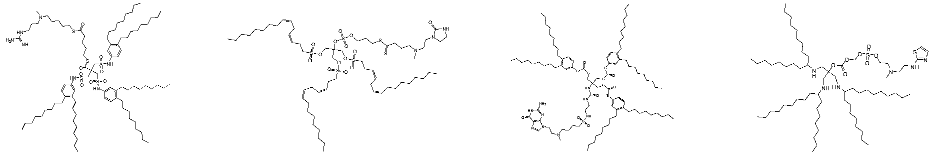

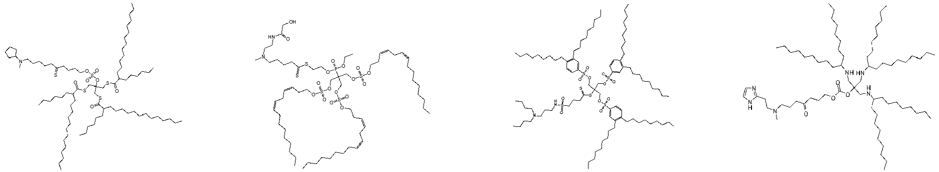

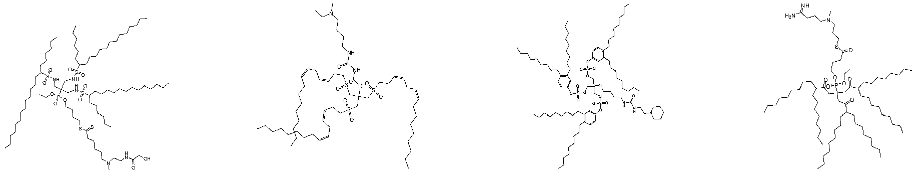

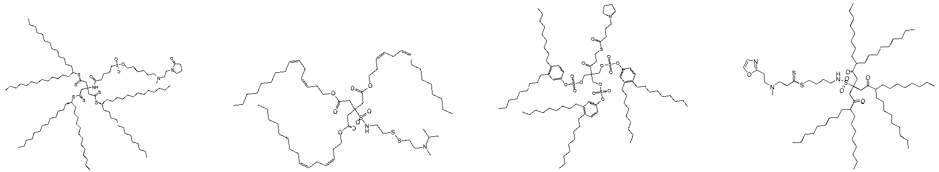

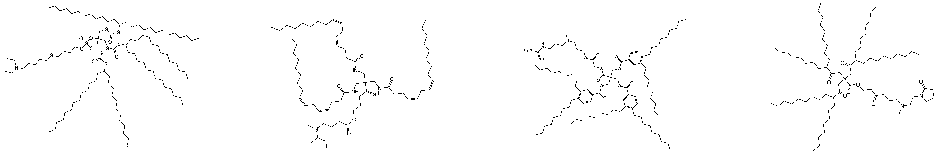

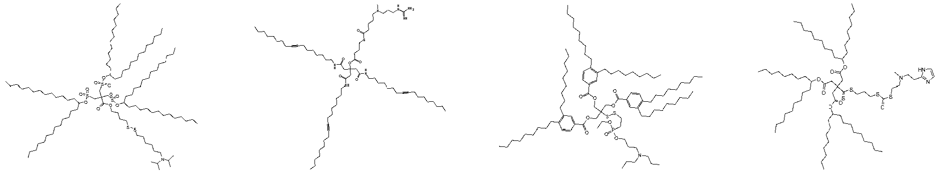

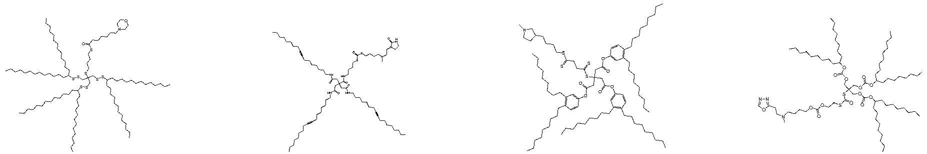

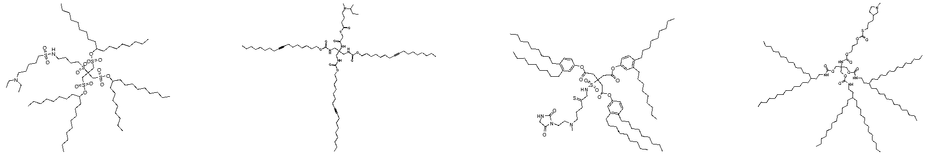

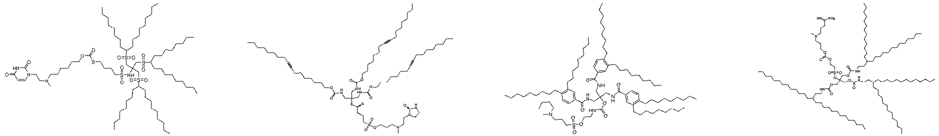

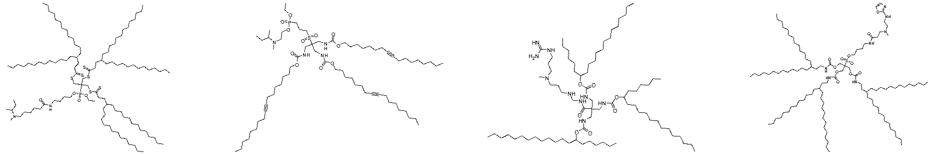

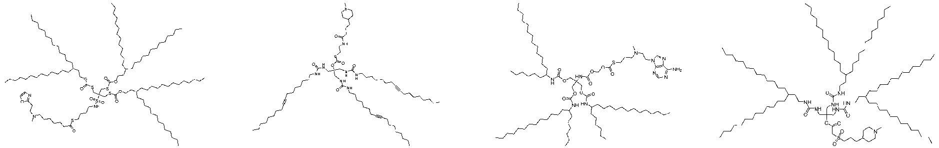

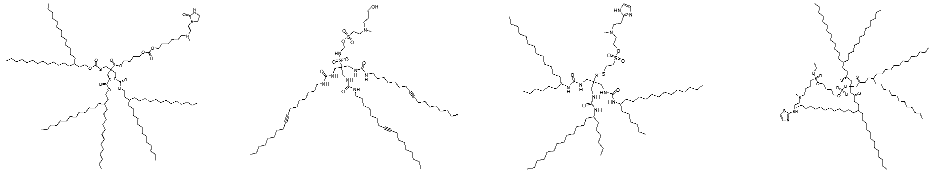

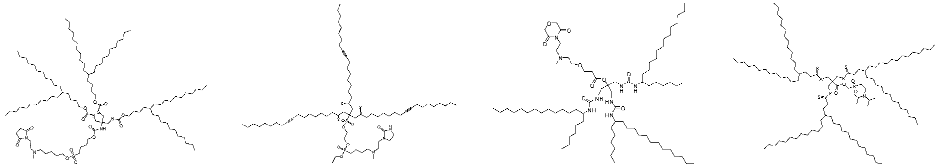

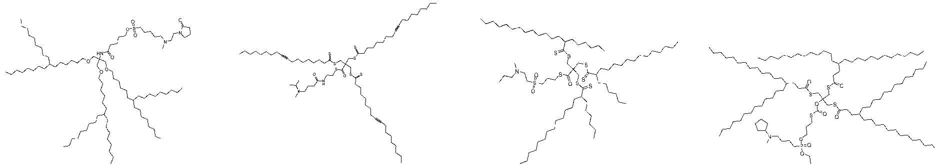

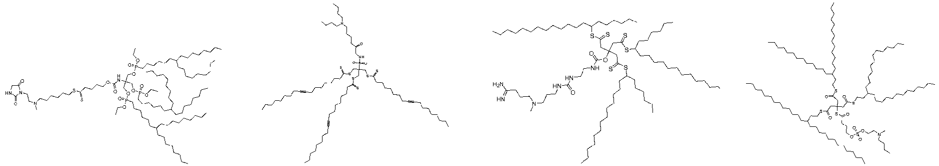

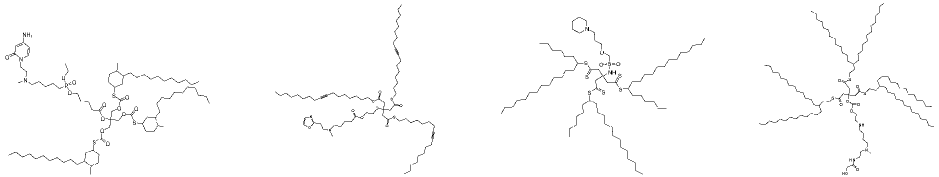

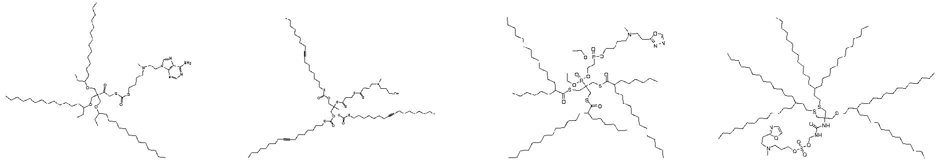

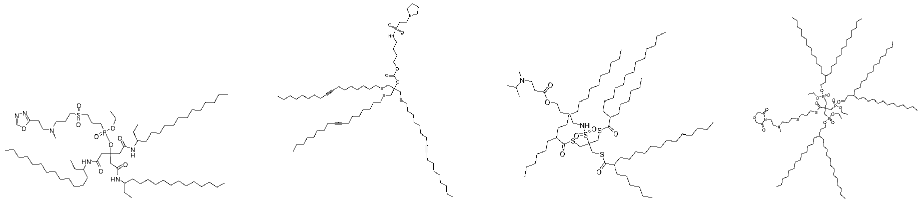

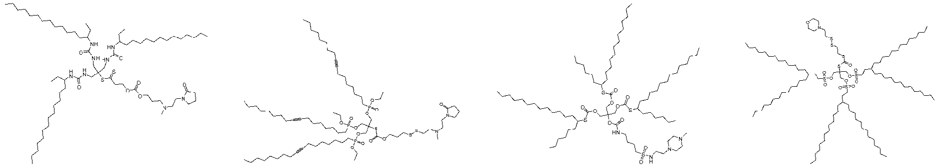

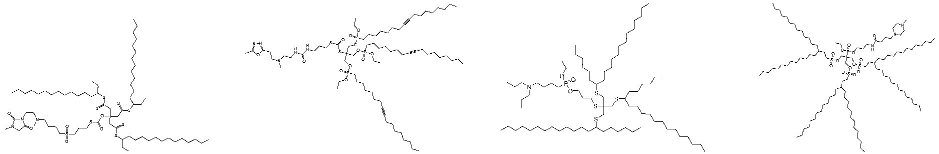

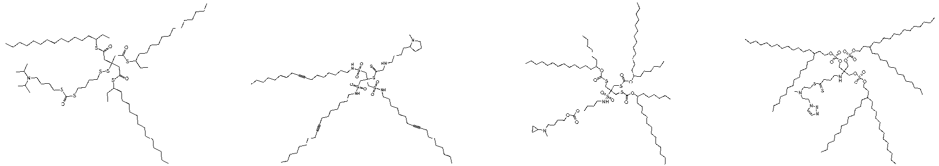

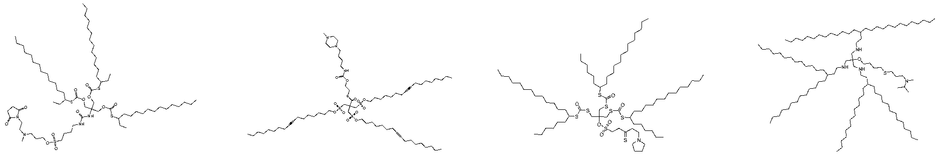

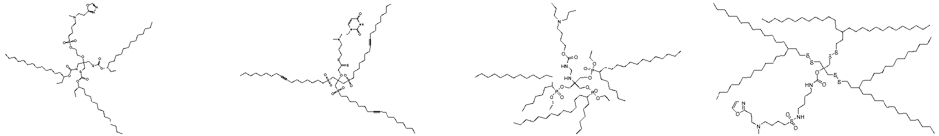

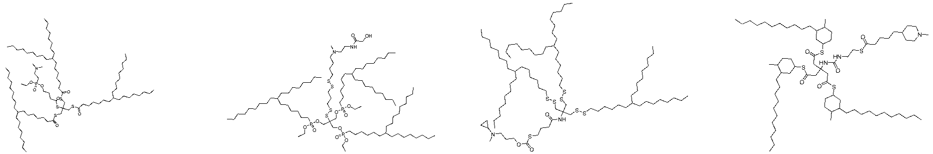

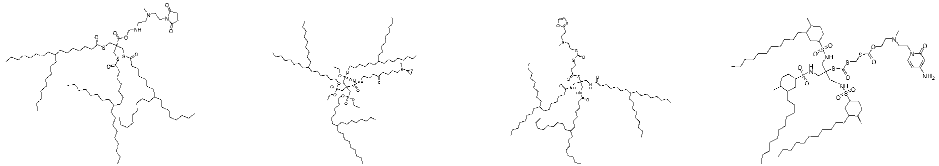

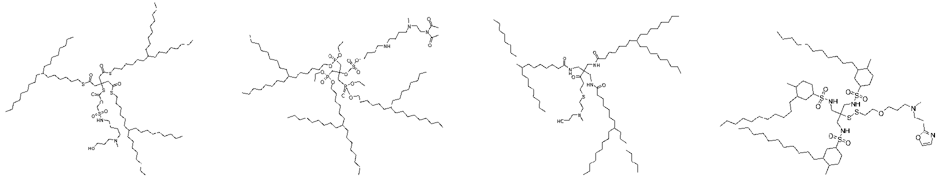

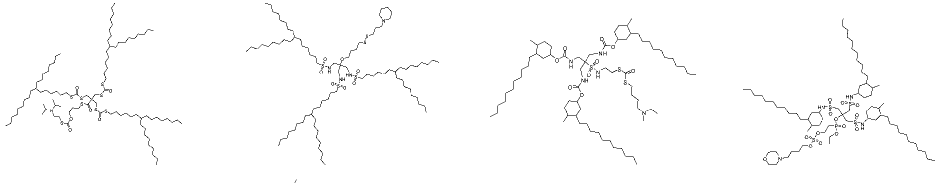

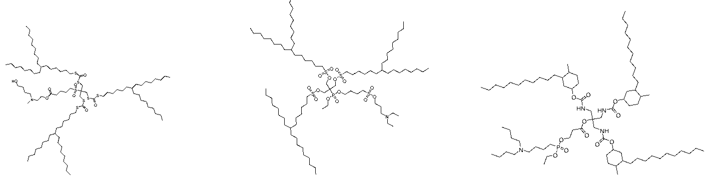

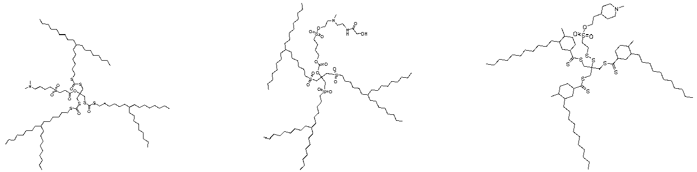

30. The use according to any one of claims 1 to 28, wherein the second ionizable lipid is a compound of Formula (2), or a salt, stereoisomer and tautomer thereof: 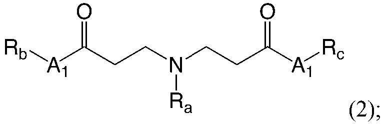
wherein A₁ is NH or O;
Rₐ is selected from C₆-C₂₄ alkyl, C₆-C₂₄ alkenyl, C₆-C₂₄ cycloalkyl, hydroxyl-substituted C₆-C₂₄ hydrocarbyl, and C₆-C₂₄ short-chain polyoxyethylene; the C₆-C₂₄ alkyl, the C₆-C₂₄ alkenyl, C₆-C₂₄ cycloalkyl, the hydroxyl-substituted C₆-C₂₄ hydrocarbyl and the C₆-C₂₄ short-chain polyoxyethylene are of straight-chain or branched structures; R_{b} and R_{c} are each independently selected from C₁-C₁₂ alkyl, C₂-C₁₂ alkenyl, C₁-C₁₂ alkynyl, C₃-C₁₂ cycloalkyl, C₆-C₁₂ aryl, C₁-C₁₂ hydroxyalkyl, C₁-C₁₂ heterocyclyl, and alkylamino;
the alkylamino is 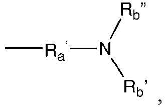
wherein Rₐ' is the C₁-C₁₂ alkyl, R_{b}' and R_{b}" are each independently selected from H, C₁-C₆ alkylamino, , or 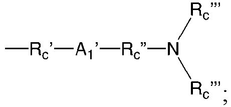 R_{c}" is selected from unsubstituted or amino-substituted C₁-C₆ alkyl, and Rc‴ is H, 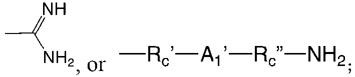
provided that when A₁' is -CO-NH-, -NH-CO- or -CO-O-, R_{c}' is the C₁-C₆ alkyl; and when A₁' is -CO-, R_{c}' is absent.

31. The use according to claim 30, wherein the compound of Formula (2) is selected from at least one of the following compounds:

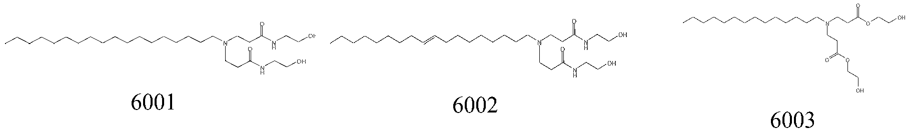

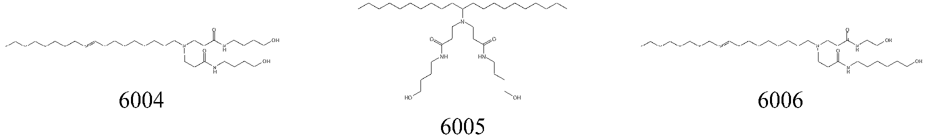

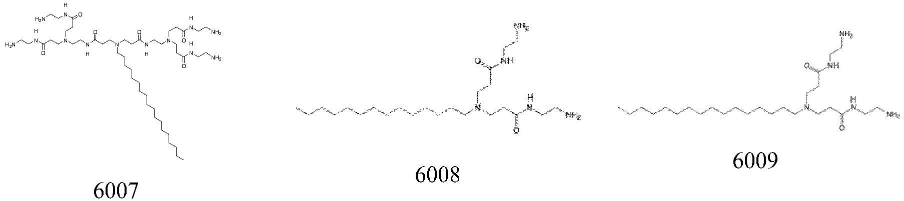

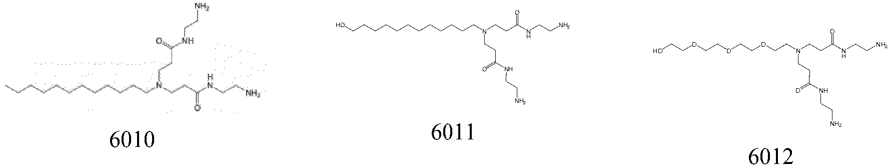

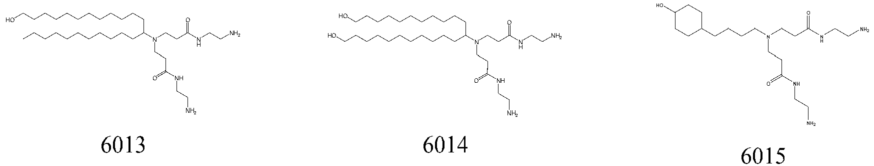

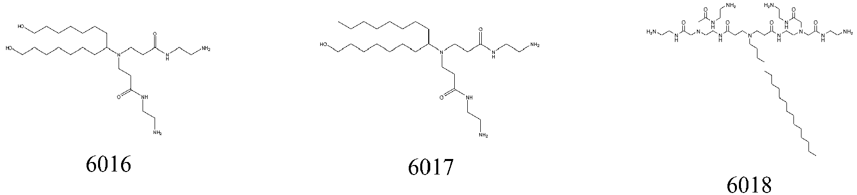

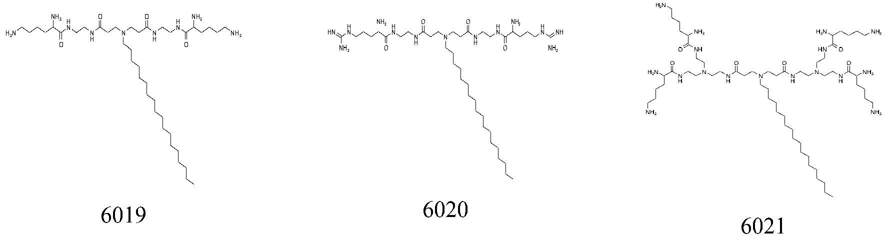

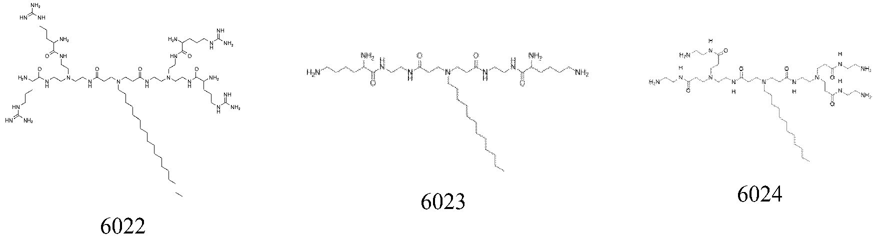

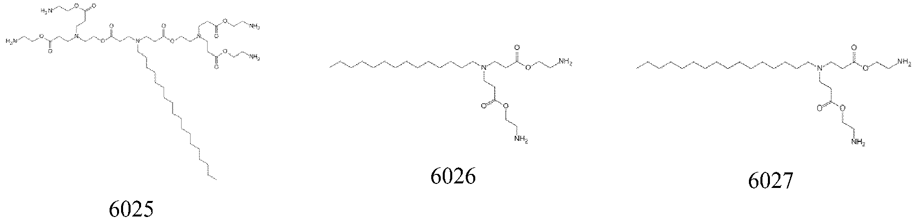

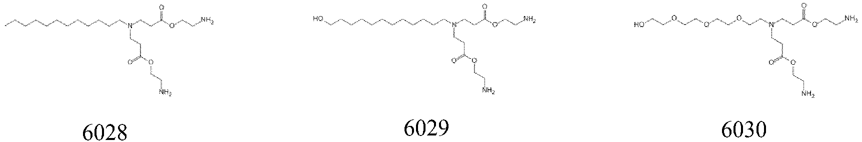

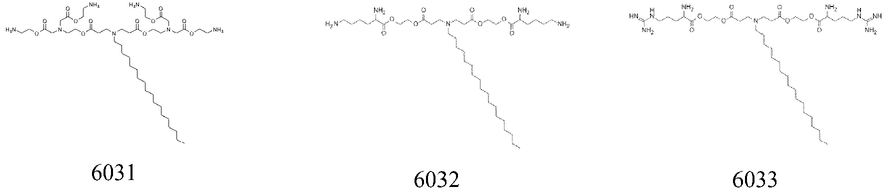

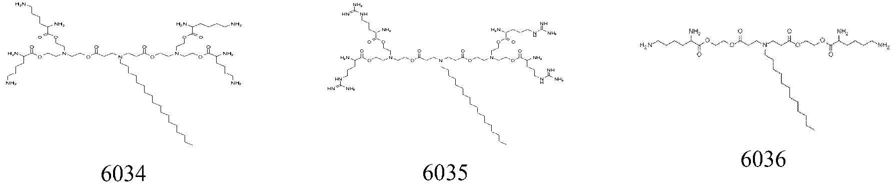

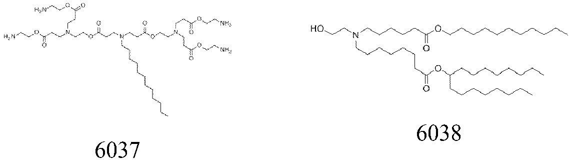

32. The use according to any one of claims 1 to 28, wherein the prefabricated carrier-based composition further comprises 0-60 mol% of other ionizable lipids selected from at least one of the following compounds:

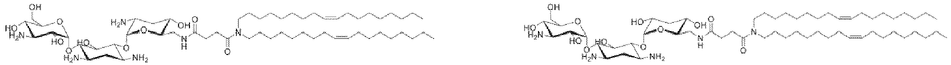

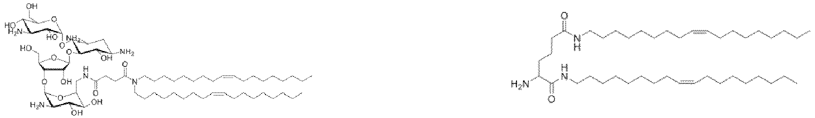

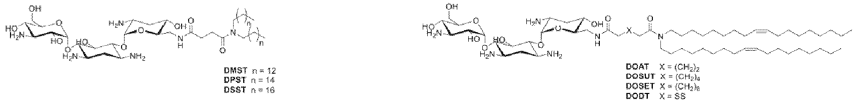

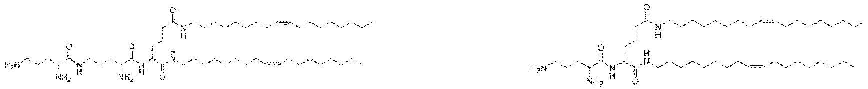

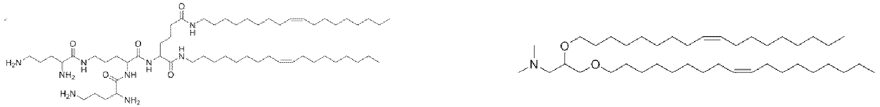

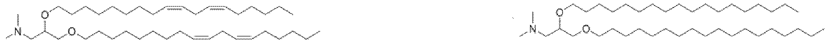

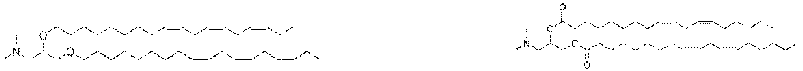

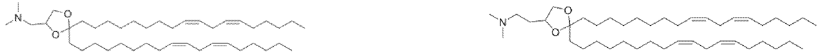

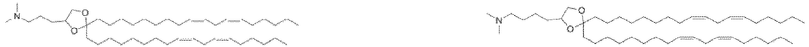

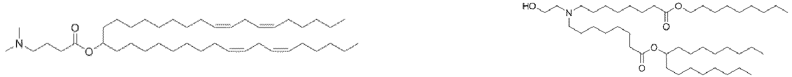

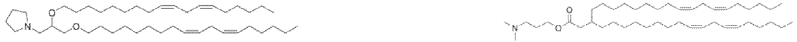

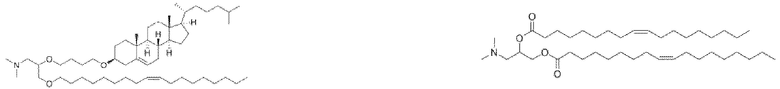

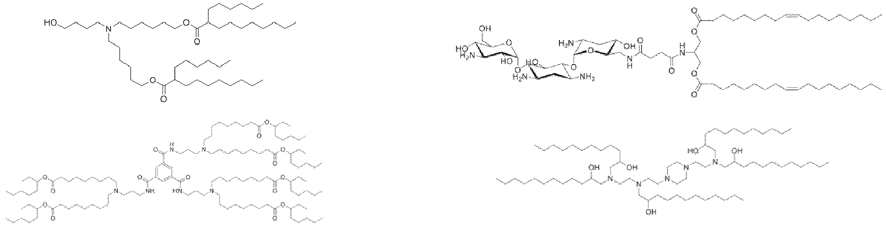

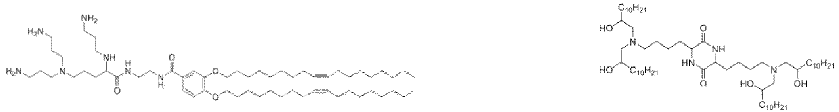

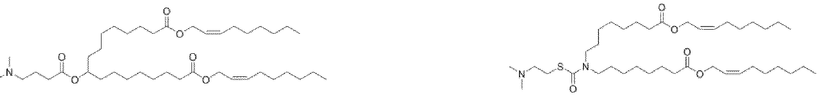

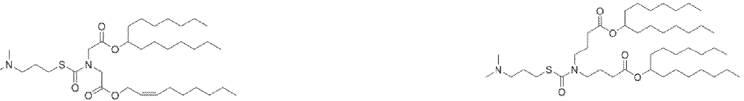

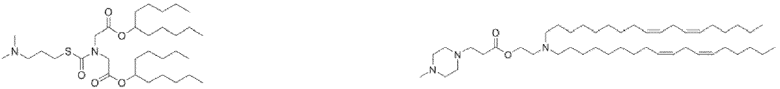

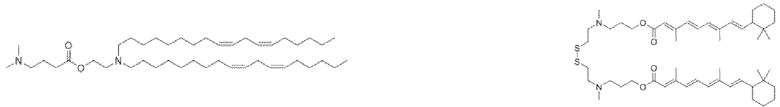

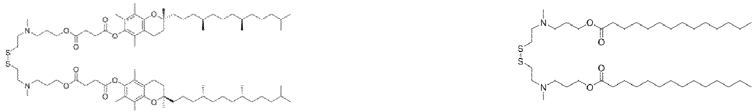

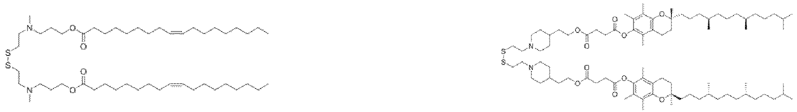

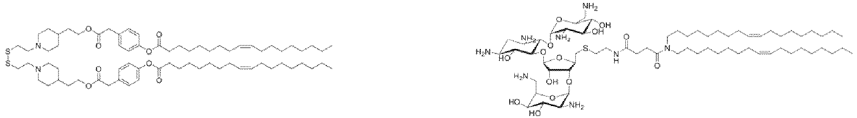

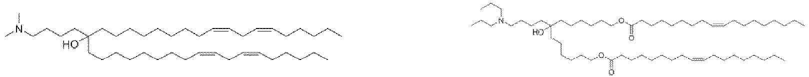

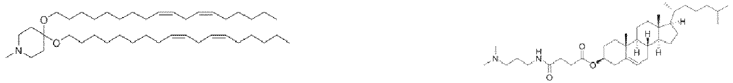

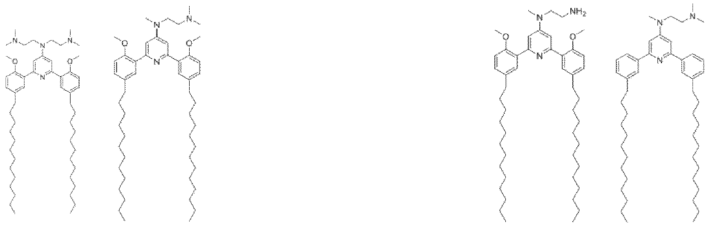

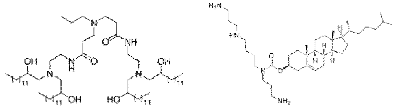

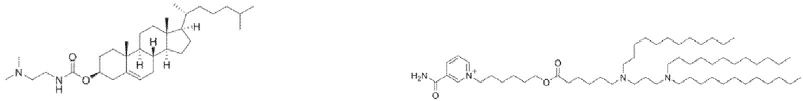

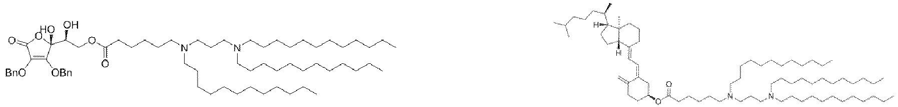

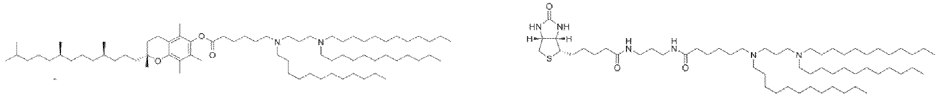

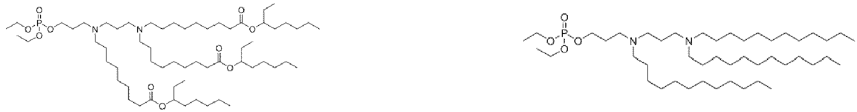

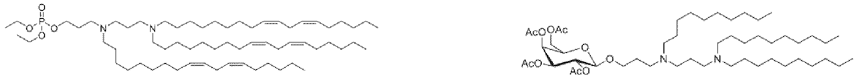

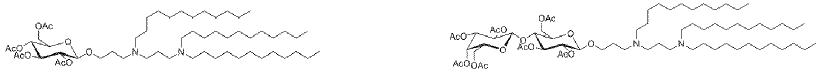

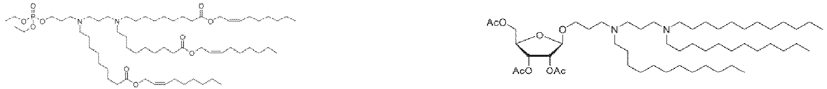

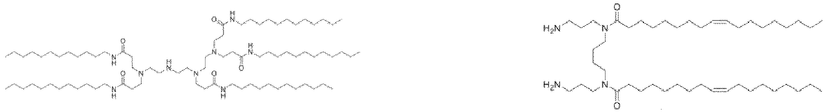

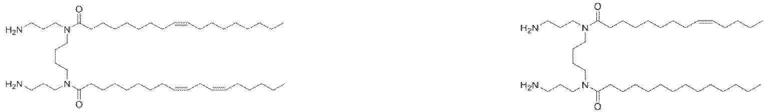

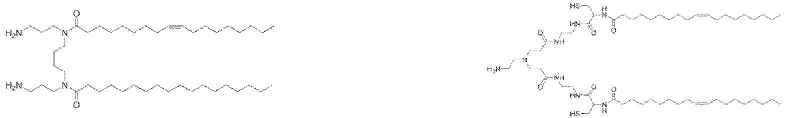

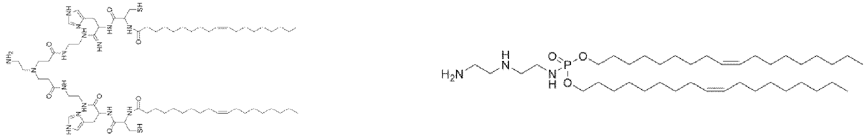

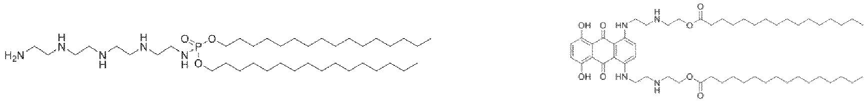

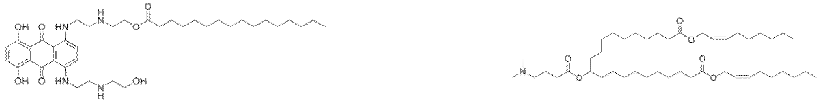

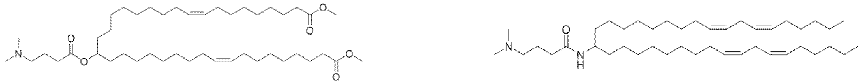

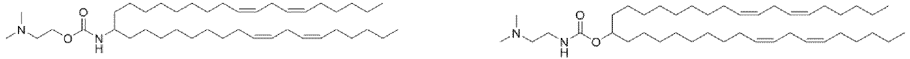

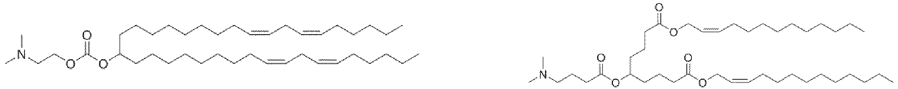

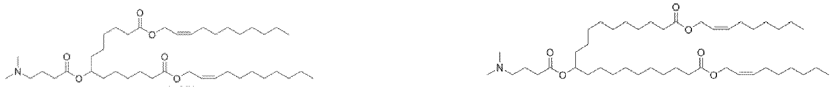

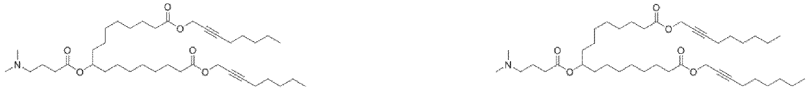

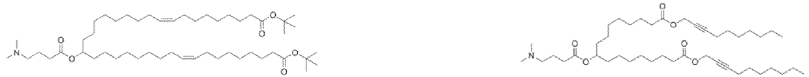

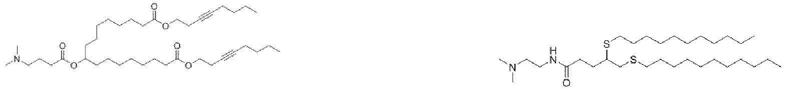

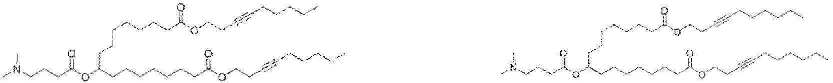

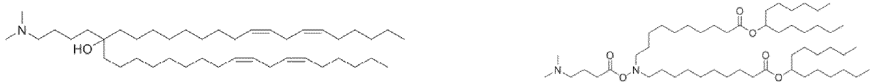

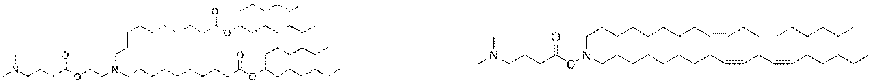

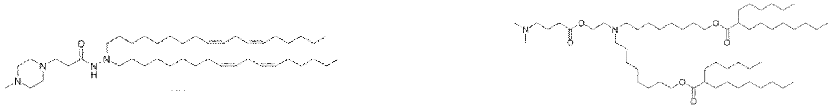

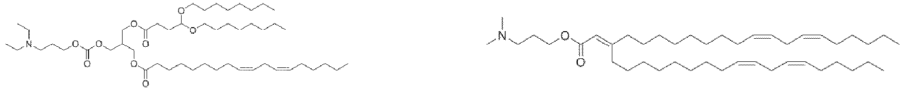

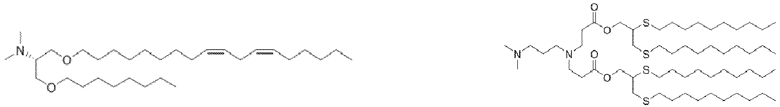

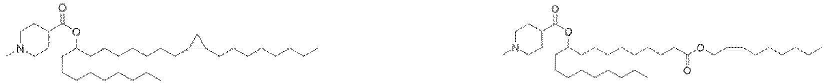

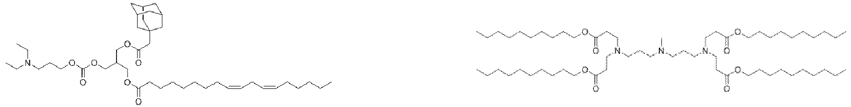

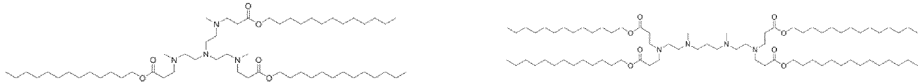

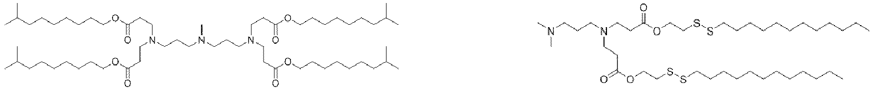

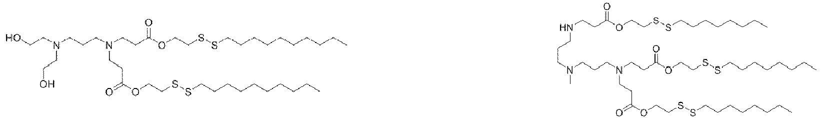

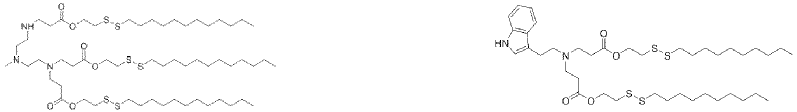

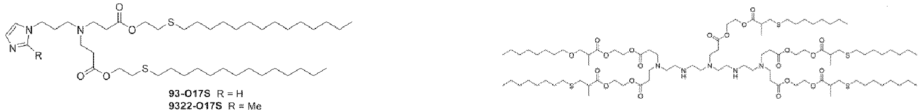

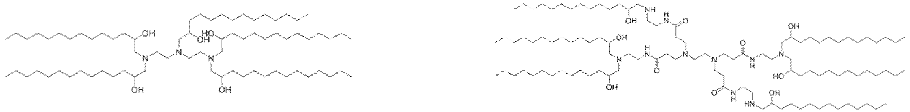

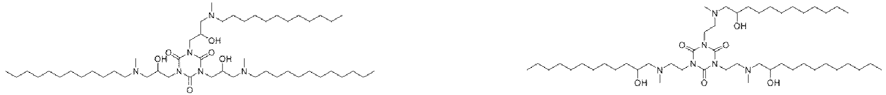

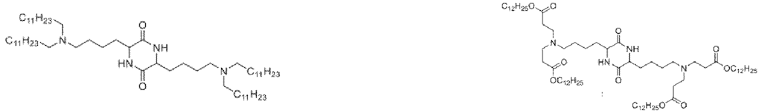

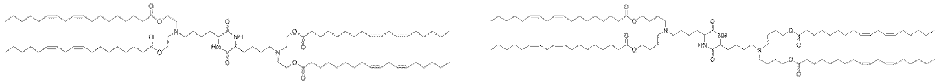

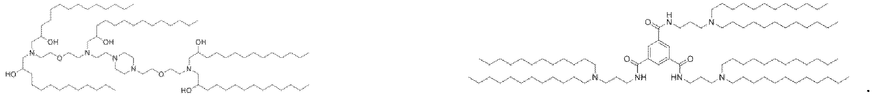

33. The use according to claim 1, wherein the phospholipid comprises at least one of 1,2-dilinoleoyl-sn-glycero-3-phosphocholine, 1,2-dimyristoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-docosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-docosahexaenoyl-sn-glycero-3-phosphoethanolamine, sodium 1,2-dioleoyl-sn-glycero-3-phospho-rac-(1-glycerol), dipalmitoylphosphatidylglycerol, palmitoyloleoylphosphatidylethanolamine, distearoylphosphatidylethanolamine, dipalmitoylphosphatidylethanolamine, dimyristoylphosphatidylethanolamine, 1-stearoyl-2-oleoylphosphatidylethanolamine, 1-stearoyl-2-oleoylphosphatidylcholine, sphingomyelin, phosphatidylcholine, phosphatidylethanolamine, phosphatidylserine, phosphatidylinositol, phosphatidic acid, palmitoyloleoylphosphatidylcholine, lysophosphatidylcholine, and lysophosphatidylethanolamine.

34. The use according to claim 1, wherein the steroid or the derivative thereof comprises at least one of cholesterol, cholesteryl stearate, coprostanol, sitosterol, ergosterol, campesterol, stigmasterol, brassicasterol, dunaliasterol, tomatine, ursolic acid, and α-tocopherol.

35. The use according to claim 1, wherein the PEG-conjugated lipid comprises at least one of PEGylated phosphatidylethanolamine, PEGylated phosphatidic acid, PEGylated ceramide, PEGylated dialkylamine, PEGylated diacylglycerol, and PEGylated dialkylglycerol.

36. The use according to claim 35, wherein the PEG-conjugated lipid comprises at least one of PEG-distearyloxypropyl, PEG-conjugated dimyristoyl glycerol (PEG-c-DOMG), PEG-dipalmitoylphosphatidylcholine (PEG-DPPC), polyethylene glycol dimethacrylate, 1,2-dimethylstyryl-rac-glycero-3-methoxypolyethylene glycol, dipalmitoylglycerol-polyethylene glycol, 1,2-distearoyl-rac-glycero-3-methoxypolyethylene glycol, 2-[(polyethylene glycol)-2000]-N,N-ditetracosylacetamide, dipalmitoylphosphatidylethanolamine-polyethylene glycol, distearoylphosphatidylethanolamine-polyethylene glycol, dilauroylphosphatidylethanolamine-polyethylene glycol and dimyristoylphosphatidylethanolamine-polyethylene glycol lipids.

37. The use according to claim 1, wherein in the prefabricated carrier-based composition, the nucleic acid is encapsulated inside the prefabricated carrier and/or adsorbed onto a surface of the prefabricated carrier to form a complex.

38. The use according to any one of claims 1 to 28, wherein the solvent is at least one of water, an aqueous solution of an organic solvent, and a buffered salt solution.

39. The use according to claim 38, wherein a pH value of the buffered salt solution is 1-9, a concentration of the buffered salt solution is 0.1-200 mM, and the aqueous solution of the organic solvent is an alcohol solution with a volume concentration smaller than 50%.

40. The use according to claim 38, wherein the buffered salt solution is selected from at least one of a citrate solution, an acetate solution, a tartrate solution, a phosphate solution, a carbonate solution, a Tris-HCl solution, and a sodium chloride solution.

41. The use according to claim 1, wherein at least one of sugar, glycerol, dimethyl sulfoxide (DMSO), a salt, an antibiotic, and a surfactant is further added to the prefabricated carrier-based composition.

42. A method for in vitro gene delivery into immune cells and stem cells, comprising a step of mixing a prefabricated carrier with a nucleic acid in a solvent to obtain a prefabricated carrier-based composition, wherein the prefabricated carrier comprises the following components: 10-60 mol% of a first ionizable lipid, 0-30 mol% of a second ionizable lipid, 5-30 mol% of a phospholipid, 15-70 mol% of a steroid or a derivative thereof, and 0-10 mol% of a PEG-conjugated lipid.
